# EUROPEAN PATENT APPLICATION

(11) **EP 4 567 031 A1**
(43) Date of publication of application: **11.06.2025**
(21) Application number: 23867630.8
(22) Date of filing: 22.09.2023
(51) Int. Cl.: C07D 401/10, C07D 519/06, A61K 31/166, A61K 31/438, A61P 31/12, A61P 35/00

(54) **HPK1 INHIBITOR, PREPARATION METHOD THEREFOR, AND USE THEREOF**

(30) Priority: 22.09.2022 CN 202211160306; 06.04.2023 CN 202310363790; 26.07.2023 CN 202310928425; 06.09.2023 CN 202311147856
(71) Applicant: Beijing Earthwise Technology Co., Ltd., Beijing 100080 (CN)
(72) Inventor: SUN, Guanglong, Beijing 100080 (CN); WANG, Huting, Beijing 100080 (CN); FANG, Shengyang, Beijing 100080 (CN); WANG, Ruina, Beijing 100080 (CN); SHI, Lei, Beijing 100080 (CN); DU, Haolin, Beijing 100080 (CN); MENG, Qinghua, Beijing 100080 (CN); LIU, Lei, Beijing 100080 (CN); GE, Yuyang, Beijing 100080 (CN); KONG, Desheng, Beijing 100080 (CN); WANG, Jianhao, Beijing 100080 (CN); WANG, Jingjing, Beijing 100080 (CN)
(74) Representative: Cabinet Becker et Associés
(86) International application number: PCT/CN2023/120707
(87) International publication number: WO 2024/061345

(57) **Abstract**

The present application belongs to the field of medicines, and provides an HPK1 inhibitor, a preparation method therefor, and use thereof. Particularly, disclosed are a compound of formula (I'), a preparation method therefor, a pharmaceutical composition comprising the compound, and use thereof in the preparation of drugs for preventing and/or treating HPK1-mediated related diseases.

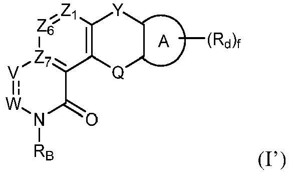

## Description

This application claims the priority of Chinese application 202211160306.4, filed on September 22, 2022, Chinese application 202310363790.9, filed on April 6, 2023, Chinese application 202310928425.8, filed on July 26, 2023, and Chinese application 202311147856.7, filed on September 06, 2023, which are incorporated herein by reference in their entirety.

### TECHNICAL FIELD

The present disclosure belongs to the field of medicine, and specifically relates to a compound of formula (I'), a preparation method thereof, a pharmaceutical composition comprising the compound, and their use in the manufacture of a medicament for the prevention and/or treatment of an HPK1-mediated related disease.

### BACKGROUND

HPK1 (Hematopoietic progenitor kinase 1), also known as MAP4K1, is a serine/threonine kinase and a member of MAP4K family. HPK1 is expressed by hematopoietic cells, and can activate the JNK/MAPK signaling pathway by binding to adapter proteins such as SLP76, thereby negatively regulating the TCR (T cell receptor) pathway and playing an important role in immune regulation. HPK1 has also become a popular target for tumor immunity.

MAP4K3 (also known as GLK) is a serine/threonine kinase, which belongs to the mammalian Ste20-like kinase family and is a homologous enzyme of HPK1. In T cells, GLK directly interacts with and activates PKCθ by phosphorylating PKCθ at residue Ser-538, leading to the activation of IKK/NF-κB. Accordingly, GLK-deficient mice exhibit T cell-mediated impaired immune responses and reduced inflammatory phenotypes in autoimmune disease models. Consistently, the percentage of GLK-overexpressing T cells is increased in the peripheral blood of patients with autoimmune diseases.

Given that HPK1 plays an important role in the occurrence and development of tumors and immune-related diseases (solid tumors, hematologic malignancies, autoimmune diseases, inflammation, etc.), and there is no marketed medicament for this target yet, it is particularly important to develop an HPK1 inhibitor with high activity, high selectivity, and good druggability.

### SUMMARY

One purpose of the present disclosure is to provide a class of HPK1 inhibitor compounds and a preparation method thereof; another purpose of the present disclosure is to provide use of a class of HPK1 inhibitors in tumor immunotherapy; the compounds of the present disclosure have good inhibitory activity on HPK1, kinase selectivity, and good druggability, and can be used to prevent and/or treat an HPK1-mediated related disease.

The present disclosure provides a compound of formula (I'), or a stereoisomer, an N-oxide, a hydrate, a solvate, a metabolite, a pharmaceutically acceptable salt, a polymorph, or a prodrug thereof: 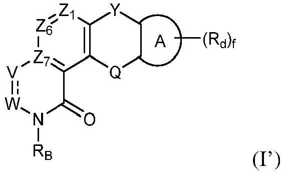 wherein each group is as defined herein.

Some embodiments of the present disclosure relate to a salt of the above compound, alternatively a hydrochloride.

In some embodiments, the present disclosure provides a pharmaceutical composition, comprising a compound of the present disclosure and a pharmaceutically acceptable carrier, adjuvant or vehicle.

In some embodiments, the present disclosure provides use of a compound of the present disclosure, or a pharmaceutical composition thereof, in the manufacture of a medicament for the treatment and/or prevention of an HPK1-mediated disorder, disease or condition.

In some embodiments, the present disclosure provides a method of treating and/or preventing an HPK1-mediated disorder, disease or condition with a compound of the present disclosure or a pharmaceutical composition thereof, comprising administering the compound of the present disclosure or the pharmaceutical composition thereof to a subject.

In some embodiments, the present disclosure provides a compound of the present disclosure, or a pharmaceutical composition thereof, for use in the treatment and/or prevention of an HPK-mediated disorder, disease or condition.

In some embodiments, the present disclosure is for use in the treatment and/or prevention of a proliferative disorder.

In some embodiments, the proliferative disorder is associated with one or more activating mutations in HPK1.

In some embodiments, the proliferative disorder is a cancer.

In some embodiments, the HPK1-mediated disorder, disease or condition is a chronic viral infection.

In some embodiments, the present disclosure relates to use of a compound of the present disclosure, or a pharmaceutical composition thereof, in the manufacture of a medicament for increasing the efficacy of a vaccination.

In some embodiments, the present disclosure relates to a compound of the present disclosure, or a pharmaceutical composition thereof, for use in increasing the efficacy of a vaccination.

In some embodiments, the present disclosure relates to a method of increasing the efficacy of a vaccination with a compound of the present disclosure or a pharmaceutical composition thereof, comprising administering the compound or the pharmaceutical composition to a subject.

### Definition and description

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

### Definition

### Chemical definition

Definitions of specific functional groups and chemical terms are described in more detail below.

When a range of values is listed, it is intended to encompass each value and sub-range within the range. For example, "C₁₋₈ alkyl" is intended to include C₁, C₂, C₃, C₄, C₅, C₆, C₇, C₈, C₁₋₈, C₁₋₇, C₁₋₆, C₁₋₅, C₁₋₄, C₁₋₃, C₁₋₂, C₂₋₈, C₂₋₇, C₂₋₆, C₂₋₅, C₂₋₄, C₂₋₃, C₃₋₈, C₃₋₇, C₃₋₆, C₃₋₅, C₃₋₄, C₄₋₈, C₄₋₇, C₄₋₆, C₄₋₅, C₅₋₈, C₅₋₇, C₅₋₆, C₆₋₈, C₆₋₇, and C₇₋₈ alkyl.

"C₁₋₈ alkyl" refers to a radical of a straight or branched, saturated hydrocarbon group having 1 to 8 carbon atoms. In some embodiments, C₁₋₆ alkyl, C₁₋₄ alkyl, C₁₋₃ alkyl and C₁₋₂ alkyl are alternative. Examples of C₁₋₆ alkyl include methyl (C₁), ethyl (C₂), n-propyl (C₃), iso-propyl (C₃), n-butyl (C₄), tert-butyl (C₄), sec-butyl (C₄), iso-butyl (C₄), n-pentyl (C₅), 3-pentyl (C₅), pentyl (C₅), neopentyl (C₅), 3-methyl-2-butyl (C₅), tert-pentyl (C₅) and n-hexyl (C₆). The term "C₁₋₆ alkyl" also includes heteroalkyl, wherein one or more (e.g., 1, 2, 3 or 4) carbon atoms are subsituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me (-CH₃), Et (-CH₂CH₃), iPr (-CH(CH₃)₂), nPr (-CH₂CH₂CH₃), n-Bu (-CH₂CH₂CH₂CH₃), i-Bu (-CH₂CH(CH₃)₂) and t-Bu (-C(CH₃)₃).

"C₂₋₈ alkenyl" refers to a radical of a straight or branched hydrocarbon group having 2 to 8 carbon atoms and at least one carbon-carbon double bond. In some embodiments, C₂₋₆ alkenyl and C₂₋₄ alkenyl are alternative. In some embodiments, C₂₋₆ alkenyl is alternative. In some embodiments, C₂₋₄ alkenyl is alternative. Examples of C₂₋₆ alkenyl include vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. The term "C₂₋₆ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"C₂₋₈ alkynyl" refers to a radical of a straight or branched hydrocarbon group having 2 to 8 carbon atoms, at least one carbon-carbon triple bond and optionally one or more carbon-carbon double bonds. In some embodiments, C₂₋₆ alkynyl is alternative. In some embodiments, C₂₋₄ alkynyl is alternative. Examples of C₂₋₆ alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), etc. The term "C₂₋₆ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be optionally substituted with one or more substituents, for example, with 1 to 5 substituents, 1 to 3 substituents or 1 substituent.

"C₁₋₈ alkylene" refers to a divalent group formed by removing another hydrogen of the C₁₋₈ alkyl, and can be substituted or unsubstituted. In some embodiments, C₁₋₆ alkylene, C₁₋₄ alkylene, C₂₋₄ alkylene and C₁₋₃ alkylene are alternative. In some embodiments, C₁₋₂ alkylene is alternative. The unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Examples of the substituted alkylene groups, such as those substituted with one or more alkyl (methyl) groups, include, but are not limited to, substituted methylene (-CH(CH₃)-, -C(CH₃)₂-), substituted ethylene (-CH(CH₃)CH₂-, -CH₂CH(CH₃)-, -C(CH₃)₂CH₂-, -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, -CH₂C(CH₃)₂CH₂-, -CH₂CH₂C(CH₃)₂-), etc.

"C₀₋₈ alkylene" refers to a chemical bond and the above "C₁₋₈ alkylene", "C₀₋₆ alkylene" refers to a chemical bond and the above "C₁₋₆ alkylene", "C₀₋₄ alkylene" refers to a chemical bond and the above "C₁₋₄ alkylene", and "C₀₋₃ alkylene" refers to a chemical bond and the above "C₁₋₃ alkylene".

"Halo" or "halogen" refers to fluorine (F), chlorine (Cl), bromine (Br) and iodine (I).

"C₁₋₈ haloalkyl" refers to the above "C₁₋₈ alkyl", which is substituted with one or more halogen. In some embodiments, C₁₋₆ haloalkyl, C₁₋₄ haloalkyl, C₁₋₃ haloalkyl and C₁₋₂ haloalkyl are yet alternative. Exemplary haloalkyl groups include, but are not limited to, -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, and the like. The haloalkyl can be substituted at any available point of attachment, for example, with 1 to 5 substituents, 1 to 3 substituents or 1 substituent.

"C₆₋₁₀ aryl" refers to a radical of monocyclic or polycyclic (e.g., bicyclic) 4n+2 aromatic ring system having 6-10 ring carbon atoms and zero heteroatoms (e.g., having 6 or 10 shared π electrons in a cyclic array). In some embodiments, the aryl group has six ring carbon atoms ("C₆ aryl"; for example, phenyl). In some embodiments, the aryl group has ten ring carbon atoms ("C₁₀ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. The aryl can be optionally substituted with one or more substituents, for example, with 1 to 5 substituents, 1 to 3 substituents or 1 substituent.

"3- to 14-membered carbocyclyl" or "3- to 14-membered cycloalkyl" refers to a non-aromatic cyclic hydrocarbon group having from 3 to 14 ring carbon atoms and zero heteroatoms, including monocyclic carbocyclyl, bridged carbocyclyl, spiro carbocyclyl and fused carbocyclyl, optionally containing 1, 2 or 3 double or triple bonds. More specifically, "3- to 14-membered carbocyclyl" or "3- to 14-membered cycloalkyl" includes any stable 3-, 4-, 5-, 6- or 7-membered monocyclic or bicyclic ring, or 7-, 8-, 9-, 10-, 11-, 12- or 13-membered bicyclic or tricyclic ring. In some embodiments, C₅₋₁₀ cycloalkyl, C₃₋₇ cycloalkyl and C₃₋₆ cycloalkyl are yet alternative, and still alternatively C₅₋₇ cycloalkyl and C₅₋₆ cycloalkyl. In some embodiments, C₃₋₇ cycloalkyl and C₃₋₆ cycloalkyl are yet alternative, and still alternatively C₅₋₆ cycloalkyl. The cycloalkyl also includes a ring system in which the cycloalkyl ring described above is fused with one or more aryl or heteroaryl groups, wherein the point of attachment is on the cycloalkyl ring, and in such case, the number of carbon atoms continues to represent the number of carbon atoms in the cycloalkyl system. Examples of the carbocyclyl include, but are not limited to, cyclopropyl, cyclobutyl, cyclobutenyl, cyclopentyl, cyclopentenyl, cyclohexyl, cyclohexenyl, cyclohexadienyl (C₆), cycloheptyl, cycloheptenyl, cycloheptadienyl (C₇), cycloheptatrienyl (C₇), adamantyl, cyclooctyl, cyclooctenyl, cyclooctadienyl, bicyclo[3.3.0]octane, bicyclo[4.3.0]nonane, bicyclo[4.4.0]decane, bicyclo[2.2.2]octane, fluorenyl, indanyl, adamantyl and tetrahydronaphthyl. The cycloalkyl can be optionally substituted with one or more substituents, for example, with 1 to 5 substituents, 1 to 3 substituents or 1 substituent.

"3- to 14-membered heterocyclyl" refers to a radical of 3- to 14-membered non-aromatic ring system having a ring carbon atom and 1 to 5 ring heteroatoms, wherein each of the heteroatoms is independently selected from nitrogen, oxygen, sulfur, boron, phosphorus and silicon, and 1, 2 or 3 double or triple bonds are optionally contained. Optionally, a nitrogen atom in it is oxidized to form an N-oxide; a sulfur atom is oxidized to form a sulfone or sulfoxide. 3- to 14-membered heterocyclyl includes monocyclic heterocyclyl, bridged heterocyclyl, spiro heterocyclyl and fused heterocyclyl. More specifically, the 3- to 14-membered heterocyclyl includes any stable 3-, 4-, 5-, 6-, 7- or 8-membered monocyclic or bicyclic ring, or 7-, 8-, 9-, 10-, 11-, 12- or 13-membered bicyclic or tricyclic ring. Yet alternatively, the 3- to 14-membered heterocyclyl includes 3- to 8-membered or 3- to 7-membered monocyclic heterocyclyl, 6- to 10-membered bridged bicyclic heterocyclyl, 6- to 10-membered spiro bicyclic heterocyclyl and 6- to 10-membered fused bicyclic heterocyclyl. In the heterocyclyl containing one or more nitrogen atoms, the point of attachment can be a carbon or nitrogen atom as long as the valence permits. In some embodiments, 4- to 12-membered heterocyclyl is alternative, which is a 4- to 12-membered non-aromatic ring system having a ring carbon atom and 1 to 5 ring heteroatoms selected from nitrogen, oxygen and sulfur; in some embodiments, 3- to 10-membered heterocyclyl is alternative, which is a 3- to 10-membered non-aromatic ring system having a ring carbon atom and 1 to 5 ring heteroatoms selected from nitrogen, oxygen and sulfur; in some embodiments, 3- to 8-membered heterocyclyl or 3- to 7-membered heterocyclyl is alternative, which is a 3- to 8-membered or 3- to 7-membered non-aromatic ring system having a ring carbon atom and 1 to 4 ring heteroatoms selected from nitrogen, oxygen and sulfur; 3- to 6-membered heterocyclyl is alternative, which is a 3- to 6-membered non-aromatic ring system having a ring carbon atom and 1 to 3 ring heteroatoms selected from nitrogen, oxygen and sulfur; 4- to 8-membered heterocyclyl is alternative, which is a 4- to 8-membered non-aromatic ring system having a ring carbon atom and 1 to 3 ring heteroatoms selected from nitrogen, oxygen and sulfur; 5- to 6-membered heterocyclyl is yet alternative, which is a 5- to 6-membered non-aromatic ring system having a ring carbon atom and 1 to 3 ring heteroatoms selected from nitrogen, oxygen and sulfur; 4- to 5-membered heterocyclyl is yet alternative, which is a 4- to 5-membered non-aromatic ring system having a ring carbon atom and 1 to 2 ring heteroatoms selected from nitrogen, oxygen and sulfur. In some embodiments, the heterocyclyl has one oxygen ring heteroatom and one nitrogen ring heteroatom. In some embodiments, the heterocyclyl has one oxygen ring heteroatom. The heterocyclyl also includes a ring system wherein the heterocyclyl ring described above is fused with one or more cycloalkyl groups, wherein the point of attachment is on the cycloalkyl ring, or a ring system wherein the heterocyclyl ring described above is fused with one or more aryl or heteroaryl groups, wherein the point of attachment is on the heterocyclyl ring; and in such cases, the number of ring members continues to represent the number of ring members in the heterocyclyl ring system. Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, oxazolidinyl and oxazolidin-2-one. Exemplary 5-membered heterocyclyl groups containing three heteroatoms include, but are not limited to, triazolinyl, oxadiazolinyl and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocyclyl groups containing one heteroatom include, but are not limited to, azepanyl, oxepanyl, thiepanyl, azepinyl, oxepinyl, and thiepinyl. Exemplary 7-membered heterocyclyl groups containing two heteroatoms include, but are not limited to, diazepanyl, oxathiepanyl, and thiazepanyl. Exemplary 5-membered heterocyclyl groups fused with a C₆ aryl ring (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a C₆ aryl ring (also referred as 6,6-bicyclic heterocyclyl herein) include, but are not limited to, tetrahydroquinolinyl, tetrahydroisoquinolinyl, etc. The heterocyclyl can be optionally substituted with one or more substituents, for example, with 1 to 5 substituents, 1 to 3 substituents or 1 substituent.

"5- to 14-membered heteroaryl" refers to a radical of 5- to 14-membered monocyclic or bicyclic 4n+2 aromatic ring system (e.g., having 6, 10 or 14 shared π electrons in a cyclic array) having a ring carbon atom and 1-4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5- to 10-membered heteroaryl groups are alternative, which are radicals of 5- to 10-membered monocyclic or bicyclic 4n+2 aromatic ring systems having a ring carbon atom and 1-4 ring heteroatoms. In other embodiments, 5- to 6-membered heteroaryl groups are yet alternative, which are radicals of 5- to 6-membered monocyclic or bicyclic 4n+2 aromatic ring systems having a ring carbon atom and 1-4 ring heteroatoms. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,3-oxadiazoly, 1,2,4-oxadiazoly, 1,2,5-oxadiazoly, 1,3,4-oxadiazoly), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, pyridonyl, and pyrazinyl. Exemplary 6-membered heteroaryl groups containing three or four heteroatoms include, but are not limited to, triazinyl and tetrazinyl, respectively. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. The heteroaryl can be optionally substituted with one or more substituents, for example, with 1 to 5 substituents, 1 to 3 substituents or 1 substituent.

The divalent groups formed by removing another hydrogen from the alkyl, alkenyl, alkynyl, cycloalkyl, heterocyclyl, aryl and heteroaryl groups defined above are collectively referred to as "-ylene" or "-ylidene". Ring-forming groups such as cycloalkyl, heterocyclyl, aryl and heteroaryl are collectively referred to as "cyclyl".

Alkyl, alkenyl, alkynyl, cycloalkyl, heterocyclyl, aryl and heteroaryl as defined herein are optionally substituted groups.

Exemplary substituents on carbon atoms include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, -OP(=O)(NR^{bb})₂, -Nk^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 R^{dd} groups;
or two geminal hydrogen on a carbon atom are replaced with =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc} groups;
each of the R^{aa} is independently selected from alkyl, haloalkyl, alkenyl, alkynyl, cycloalkyl, heterocyclyl, aryl and heteroaryl, or two of the R^{aa} groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{bb} is independently selected from hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{bb} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{cc} is independently selected from hydrogen, alkyl, haloalkyl, alkenyl, alkynyl, cycloalkyl, heterocyclyl, aryl and heteroaryl, or two R^{cc} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{dd} is independently selected from halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee} -SO₂N(R^{ff})₂, -SO₂R^{cc}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl, and heteroaryl, wherein each of the alkyl, alkenyl, alkynyl, cycloalkyl, heterocyclyl, aryl and heteroaryl is independently substituted with 0, 1, 2, 3, 4 or 5 R^{gg} groups, or two geminal R^{dd} substituents can be combined to form=O or =S;
each of the R^{ee} is independently selected from alkyl, haloalkyl, alkenyl, alkynyl, cycloalkyl, aryl, heterocyclyl, and heteroaryl, wherein each of the alkyl, alkenyl, alkynyl, cycloalkyl, heterocyclyl, aryl and heteroaryl is independently substituted with 0, 1, 2, 3, 4 or 5 R^{gg} groups;
each of the R^{ff} is independently selected from hydrogen, alkyl, haloalkyl, alkenyl, alkynyl, cycloalkyl, heterocyclyl, aryl and heteroaryl, or two R^{ff} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{gg} groups;
each of the R^{gg} is independently halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂₋₆ alkenyl, C₂₋₆ alkynyl, C₃₋₇ cycloalkyl, C₆₋₁₀ aryl, C₃₋₇ heterocyclyl, or C₅₋₁₀ heteroaryl; or two geminal R^{gg} substituents may combine to form =O or =S; wherein X⁻ is a counter-ion.

Exemplary substituents on nitrogen atoms include, but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{cc} groups attached to a nitrogen atom combine to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups, and wherein R^{aa}, R^{bb}, R^{cc} and R^{dd} are as described above.

### Other definitions

The term "pharmaceutically acceptable salt" as used herein refers to those carboxylate and amino acid addition salts of the compounds of the present disclosure, which are suitable for the contact with patients' tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N'-dibenzylethylenediamine, chloroprocaine, choline, diethanolamine, ethylenediamine, N-methylglucamine and procaine.

The base addition salt of the acidic compound can be prepared by contacting the free acid form with a sufficient amount of the required base to form a salt in a conventional manner. The free acid can be regenerated by contacting the salt form with an acid in a conventional manner and then isolating the free acid. The free acid forms are somewhat different from their respective salt forms in their physical properties, such as solubility in polar solvents. But for the purposes of the present disclosure, the salts are still equivalent to their respective free acids.

The salts can be prepared from the inorganic acids, which include sulfates, pyrosulfates, bisulfates, sulfites, bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, such as aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methane sulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts," J. Pharm. Sci., 1977; 66: 1-19 for reference).

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults)) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "human", "patient" and "subject" can be used interchangeably herein.

"Disease," "disorder," and "condition" can be used interchangeably herein.

Unless indicated, otherwise the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment").

Unless indicated, otherwise the "therapeutically effective amount" of the compound as used herein is an amount sufficient to provide therapeutic benefits in the course of treating a disease, disorder or condition, or to delay or minimize one or more symptoms associated with the disease, disorder or condition. The therapeutically effective amount of a compound refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless indicated, otherwise the "prophylactically effective amount" of the compound as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a compound refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that enhances the prophylactic effect of other preventive agents.

"Combination" and related terms refer to the simultaneous or sequential administration of the compounds of the present disclosure and other therapeutic agents. For example, the compounds of the present disclosure can be administered simultaneously or sequentially in separate unit dosage with other therapeutic agents, or simultaneously in a single unit dosage with other therapeutic agents.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

The compounds of the present disclosure may exist in tautomeric forms. Tautomers are functional group isomers produced by the rapid movement of an atom between two positions in a molecule. The tautomers are special functional group isomers. A pair of tautomers can be converted into each other, but usually one relatively stable isomer is the main form of existence. The most important examples are the enol and keto tautomers.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula R·x H₂O, wherein R is the compound, and x is a number greater than 0. Given compounds can form more than one type of hydrates, including, for example, monohydrates (x is 1), lower hydrates (x is a number greater than 0 and smaller than 1, for example, hemihydrates (R·0.5 H₂O)) and polyhydrates (x is a number greater than 1, for example, dihydrates (R·2 H₂O) and hexahydrates (R·6 H₂O)).

The term "metabolite" refers to a product produced during the metabolism of a substance including the compound of the present disclosure in the body, including intermediate metabolites and final metabolites.

Compounds of the present disclosure may be in an amorphous or a crystalline form (polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes (isotopic variants), which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., ³H and ¹⁴C), can be used for the measurement of the distribution of drug and/or substrate in tissue. Tritium, which is ³H and carbon-14, which is ¹⁴C isotope, are yet alternative, because they are easy to prepare and detect. Furthermore, replaced by heavier isotopes, such as deuterium, which is ²H, may provide therapeutic benefits due to the higher metabolic stability, such as prolonging the half-life *in vivo* or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects *in vivo* by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which is incorporated herein by reference.

The prodrugs are any covalently bonded compounds of the present disclosure, which release the parent compound *in vivo* when the prodrug is administered to a patient. Prodrugs are typically prepared by modifying functional groups in such a way that the modifications can be cleaved either by routine manipulation or decompose *in vivo* to yield the parent compound. Prodrugs include, for example, compounds of the present disclosure wherein the hydroxyl, amino or sulfhydryl groups are bonded to any group that, when administered to a patient, cleaves to form the hydroxyl, amino or sulfhydryl groups. Thus, representative examples of prodrugs include (but are not limited to) the acetate/acetamide, formate/formamide and benzoate/benzamide derivatives of the hydroxyl, sulfhydryl or amino functional groups of the compounds of formula (I). Furthermore, in the case of carboxylic acid (-COOH), esters such as methyl esters and ethyl esters, etc. can be employed. The ester itself may be active in their own and/or hydrolyzable under *in vivo* conditions in the human body. Suitable pharmaceutically acceptable *in vivo* hydrolysable ester groups include those groups that can readily break down in the human body to release the parent acids or salts thereof.

The present disclosure also provides a pharmaceutical formulation comprising a therapeutically effective amount of a compound of formula (I), or a therapeutically acceptable salt thereof, and pharmaceutically acceptable carriers, diluents or excipients thereof. All of these forms belong to the present disclosure.

### DETAILED DESCRIPTION

As used herein, the term "compound of the present disclosure" refers to a compound of the following formula (I'), formula (I), formula (II), etc., or a pharmaceutically acceptable salt, an isotopic variant, a tautomer, a stereoisomer, a prodrug, a polymorph, a hydrate, or a solvate thereof.

In the present disclosure, compounds are named using standard nomenclature. For compounds having an asymmetric center, it should be understood, unless otherwise stated, that all optical isomers and mixtures thereof are included. Furthermore, unless otherwise specified, all isomer compounds and carbon-carbon double bonds included in the present disclosure may occur in the form of Z and E. For compounds that can exist in different tautomeric forms, a described compound is not limited to any specific tautomer but is intended to encompass all tautomeric forms.

In one embodiment, the present disclosure relates to a compound of formula (I'), or a stereoisomer, an N-oxide, a hydrate, a solvate, a metabolite, a pharmaceutically acceptable salt, a polymorph, or a prodrug thereof: 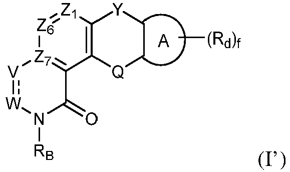
V is selected from covalent bond, O, S, NR', S(O), S(O)₂, C(O), CR_{C} and CR_{C}R_{C}';
W is selected from NR', S(O), S(O)₂, C(O), CR_{C} and CR_{C}R_{C}';
Z₇ is C;
or V is not connected to W, Z₇ is C or N, W is selected from H, C₁₋₆ alkyl and C₁₋₆ haloalkyl, alternatively selected from H and C₁₋₆ alkyl, V is selected from absent, H, D, halogen, C₁₋₈ alkyl and C₁₋₈ haloalkyl, alternatively selected from absent, H, D and halogen;
Q and Y are independently selected from covalent bond, -O-, -S-, -NR_{A}-, -S(O)-, -S(O)₂-, -C(O)-, -C(O)O-, -C(O)NR_{A}-, -OC(O)-, -OC(O)NR_{A}-, -N(R_{A})C(O)O-, -N(R_{A})C(O)- and -N(R_{A})S(O)₂-; and Q and Y are not covalent bonds at the same time;
or Q and Y are independently C₁₋₈ alkylene, in which one or more methylene units are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
R_{A} is selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L''-5- to 14-membered heteroaryl;
R_{B} is selected from H, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
R_{C}, R'_{C}, R₁ₐ and R_{1b} are independently selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
one of Z₁ and Z₆ is CR_{E}, and the other one is CR_{d1} or N;
R_{E} is selected from -L-C₆₋₁₀ aryl, -L-3- to 14-membered carbocyclyl, -L-3- to 14-membered heterocyclyl and -L-5- to 14-membered heteroaryl, wherein each of the C₆₋₁₀ aryl, 3- to 14-membered carbocyclyl, 3- to 14-membered heterocyclyl and 5- to 14-membered heteroaryl is optionally substituted with 1, 2, 3, 4, 5, 6, 7, 8, 9, or 10 Rₑ groups;
Rₑ is independently selected from H, D, halogen, -CN, oxo, C₁₋₈ alkyl, C₁₋₈ haloalkyl, C₂₋₈ alkenyl, C₂₋₈ alkynyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-C₆₋₁₀ aryl, -L'-3- to 14-membered carbocyclyl, -L'-3-to 14-membered heterocyclyl and -L'-5- to 14-membered heteroaryl, each of which is optionally substituted with 1, 2 or 3 Rₑₛ;
or two geminal Rₑ are taken together with the atom to which they are jointly attached to form a 3- to 7-membered carbocyclyl or a 3- to 7-membered heterocyclyl, which is optionally substituted with 1, 2 or 3 Rₑₛ;
Rₑₛ is independently selected from H, D, halogen, CN, C₁₋₆ alkyl, C₁₋₆ haloalkyl, -L'-OR' and -L'-NR'R";
R_{d1} is selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
ring A is selected from C₆₋₁₀ aromatic ring, 3- to 14-membered carbocycle, 3- to 14-membered heterocycle and 5- to 14-membered heteroaromatic ring;
R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or R_{d} is selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₈ alkyl, C₂₋₈ alkenyl, C₂₋₈ alkynyl, C₁₋₈ haloalkyl, -L"-OR', -L"-NR'R", -L"-C(O)OR', -L"-C(O)NR'R", -L"-S(O)₀₋₂R', -L"-S(O)₂NR'R", -L"-S(O)NR'R", -L"-OC(O)R', -L"-OC(O)NR'R", -L"-C₆₋₁₀ aryl, -L"-3- to 14-membered carbocyclyl, -L"-3- to 14-membered heterocyclyl and -L"-5- to 14-membered heteroaryl;
or CR_{dd}R_{dd} forms a 3- to 7-membered carbocyclylene or a 3- to 7-membered heterocyclylene, which is optionally substituted with 1, 2 or 3 groups selected from: H, D, halogen, C₁₋₆ alkyl, C₁₋₆ haloalkyl, -OR' and -NR'R";
or two non-adjacent R_{dd} are taken together to form a C₁₋₈ alkylene, which is optionally substituted with one or more of the following substituents: H, D, halogen, CN, C₁₋₈ alkyl, C₁₋₈ alkoxy, C₁₋₈ haloalkyl, -L"-C₆₋₁₀ aryl, -L"-3- to 10-membered carbocyclyl, -L"-3- to 10-membered heterocyclyl and -L"-5- to 10-membered heteroaryl;
or two adjacent R_{dd} are taken together with the atoms to which they are attached to form a 3- to 7-membered carbocyclylene, a 3- to 7-membered heterocyclylene, a phenylene, or a 5- to 6-membered heteroarylene, which is optionally substituted with 1, 2, 3 or 4 substituents selected from: H, D, halogen, CN, C₁₋₆ alkyl, C₁₋₆ haloalkyl, -OR' and -NR'R";
L and L' are independently selected from covalent bond, -O-, -S-, -NR'-, -S(O)₂-, -C(O)-, -C(O)O-, -C(O)NR'-, -OC(O)-, -OC(O)NR'-, -N(R')C(O)O-, -N(R')C(O)- and -N(R')S(O)₂-; or, L or L' is C₁₋₈ alkylene, in which one or more methylene units are optionally and independently replaced by -C(R')₂-, -N(R')-, -N(R')C(O)-, -C(O)N(R')-, -N(R')S(O)₂-, -S(O)₂N(R')-, -O-, -C(O)-, -OC(O)-, -C(O)O-, -S-, -S(O)-, or -S(O)₂-;
R' and R" are independently selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl; or, R' and R" are taken together with the N atom to which they are attached to form a 3- to 14-membered heterocyclyl;
L" is selected from covalent bond, -O- and -S-; or, L" is C₁₋₈ alkylene, which is optionally substituted with one or more R, and one or more methylene units in the C₁₋₈ alkylene are optionally and independently replaced by -O-, -C(O)-, -OC(O)-, -C(O)O-, -S-, -S(O)- or -S(O)₂-;
R is independently selected from H, D, C₁₋₈ alkyl, C₁₋₈ haloalkyl, -L_{d}-3- to 10-membered carbocyclyl and -L_{d}-3- to 10-membered heterocyclyl;
or two R are taken together with the C atom to which they are jointly attached to form a 3- to 10-membered carbocyclylene or a 3- to 10-membered heterocyclylene;
L_{d} is independently selected from covalent bond and C₁₋₈ alkylene;
f=0, 1, 2, 3, 4, 5, 6, 7, 8, 9, or 10;
wherein each of the above groups is optionally deuterated, up to completely deuterated.

In one embodiment, the present disclosure relates to a compound of formula (I), or a stereoisomer, an N-oxide, a hydrate, a solvate, a metabolite, a pharmaceutically acceptable salt, a polymorph, or a prodrug thereof: 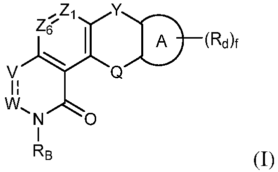
V is selected from covalent bond, O, S, NR', S(O), S(O)₂, C(O), CR_{C} and CR_{C}R_{C}';
W is selected from NR', S(O), S(O)₂, C(O), CR_{C} and CR_{C}R_{C}';
Q and Y are independently selected from covalent bond, -O-, -S-, -NR_{A}-, -S(O)-, -S(O)₂-, -C(O)-, -C(O)O-, -C(O)NR_{A}-, -OC(O)-, -OC(O)NR_{A}-, -N(R_{A})C(O)O-, -N(R_{A})C(O)- and -N(R_{A})S(O)₂-; and Q and Y are not covalent bonds at the same time;
or Q and Y are independently C₁₋₈ alkylene, in which one or more methylene units are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
R_{A} is selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl;
R_{B} is selected from H, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
R_{C}, R'_{C}, R₁ₐ and R_{1b} are independently selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
one of Z₁ and Z₆ is CR_{E}, and the other one is CR_{d1} or N;
R_{E} is selected from -L-C₆₋₁₀ aryl, -L-3- to 14-membered carbocyclyl, -L-3- to 14-membered heterocyclyl and -L-5- to 14-membered heteroaryl, wherein each of the C₆₋₁₀ aryl, 3- to 14-membered carbocyclyl, 3- to 14-membered heterocyclyl and 5- to 14-membered heteroaryl is optionally substituted with 1, 2, 3, 4, 5, 6, 7, 8, 9 or 10 Rₑ groups;
Rₑ is independently selected from H, D, halogen, -CN, oxo, C₁₋₈ alkyl, C₁₋₈ haloalkyl, C₂₋₈ alkenyl, C₂₋₈ alkynyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-C₆₋₁₀ aryl, -L'-3- to 14-membered carbocyclyl, -L'-3-to 14-membered heterocyclyl and -L'-5- to 14-membered heteroaryl;
R_{d1} is selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
ring A is selected from C₆₋₁₀ aromatic ring, 3- to 14-membered carbocycle, 3- to 14-membered heterocycle and 5- to 14-membered heteroaromatic ring;
R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or, R_{d} is selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₈ alkyl, C₂₋₈ alkenyl, C₂₋₈ alkynyl, C₁₋₈ haloalkyl, -L"-OR', -L"-NR'R", -L"-C(O)OR', -L"-C(O)NR'R", -L"-S(O)₀₋₂R', -L"-S(O)₂NR'R", -L"-S(O)NR'R", -L"-OC(O)R', -L"-OC(O)NR'R", -L"-C₆₋₁₀ aryl, -L"-3- to 14-membered carbocyclyl, -L"-3- to 14-membered heterocyclyl and -L"-5- to 14-membered heteroaryl;
or two R_{dd} are taken together to form a C₁₋₈ alkylene, which is optionally substituted with one or more of the following substituents: H, D, halogen, CN, C₁₋₈ alkyl, C₁₋₈ alkoxy, C₁₋₈ haloalkyl, -L"-C₆₋₁₀ aryl, -L"-3- to 10-membered carbocyclyl, -L"-3- to 10-membered heterocyclyl and -L"-5- to 10-membered heteroaryl;
L and L' are independently selected from covalent bond, -O-, -S-, -NR'-, -S(O)₂-, -C(O)-, -C(O)O-, -C(O)NR'-, -OC(O)-, -OC(O)NR'-, -N(R')C(O)O-, -N(R')C(O)- and -N(R')S(O)₂-; or, L or L' is C₁₋₈ alkylene, in which one or more methylene units are optionally and independently replaced by -C(R')₂-, -N(R')-, -N(R')C(O)-, -C(O)N(R')-, -N(R')S(O)₂-, -S(O)₂N(R')-, -O-, -C(O)-, -OC(O)-, -C(O)O-, -S-, -S(O)-, or -S(O)₂-;
R' and R" are independently selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl; or, R' and R" are taken together with the N atom to which they are attached to form a 3- to 14-membered heterocyclyl;
L" is selected from covalent bond, -O- and -S-; or, L" is C₁₋₈ alkylene, which is optionally substituted with one or more R, and one or more methylene units in the C₁₋₈ alkylene are optionally and independently replaced by -O-, -C(O)-, -OC(O)-, -C(O)O-, -S-, -S(O)-, or -S(O)₂-;
R is independently selected from H, D, C₁₋₈ alkyl, C₁₋₈ haloalkyl, -L_{d}-3- to 10-membered carbocyclyl and -L_{d}-3- to 10-membered heterocyclyl;
or two R are taken together with the C atom to which they are jointly attached to form a 3- to 10-membered carbocyclylene or a 3- to 10-membered heterocyclylene;
L_{d} is independently selected from covalent bond and C₁₋₈ alkylene;
f=0, 1, 2, 3, 4, 5, 6, 7, 8, 9 or 10;
wherein each of the above groups is optionally deuterated, up to completely deuterated.

In another embodiment, the present disclosure relates to a compound of formula (I), or a stereoisomer, an N-oxide, a hydrate, a solvate, a metabolite, a pharmaceutically acceptable salt, a polymorph, or a prodrug thereof: 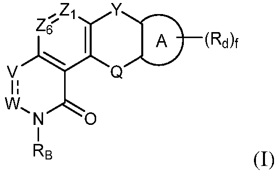
V is selected from covalent bond, O, S, NR', S(O), S(O)₂, C(O), CR_{C} and CR_{C}R_{C}';
W is selected from NR', S(O), S(O)₂, C(O), CR_{C} and CR_{C}R_{C}';
Q and Y are independently selected from covalent bond, -O-, -S-, -NR_{A}-, -S(O)-, -S(O)₂-, -C(O)-, -C(O)O-, -C(O)NR_{A}-, -OC(O)-, -OC(O)NR_{A}-, -N(R_{A})C(O)O-, -N(R_{A})C(O)- and -N(R_{A})S(O)₂-; and Q and Y are not covalent bonds at the same time;
or Q and Y are independently C₁₋₈ alkylene, in which one or more methylene units are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
R_{A} is selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl;
R_{B} is selected from H, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
R_{C}, R'_{C}, R₁ₐ and R_{1b} are independently selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
one of Z₁ and Z₆ is CR_{E}, and the other one is CR_{d1} or N;
R_{E} is selected from -L-C₆₋₁₀ aryl, -L-3- to 14-membered carbocyclyl, -L-3- to 14-membered heterocyclyl and -L-5- to 14-membered heteroaryl, wherein each of the C₆₋₁₀ aryl, 3- to 14-membered carbocyclyl, 3- to 14-membered heterocyclyl and 5- to 14-membered heteroaryl is optionally substituted with 1, 2, 3, 4, 5, 6, 7, 8, 9 or 10 Rₑ groups;
Rₑ is independently selected from H, D, halogen, -CN, oxo, C₁₋₈ alkyl, C₁₋₈ haloalkyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-C₆₋₁₀ aryl, -L'-3- to 14-membered carbocyclyl, -L'-3- to 14-membered heterocyclyl and -L'-5- to 14-membered heteroaryl;
R_{d1} is selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
ring A is selected from C₆₋₁₀ aromatic ring, 3- to 14-membered carbocycle, 3- to 14-membered heterocycle and 5- to 14-membered heteroaromatic ring;
R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or, R_{d} is selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₈ alkyl, C₂₋₈ alkenyl, C₂₋₈ alkynyl, C₁₋₈ haloalkyl, -L"-OR', -L"-NR'R", -L"-C(O)OR', -L"-C(O)NR'R", -L"-S(O)₀₋₂R', -L"-S(O)₂NR'R", -L"-S(O)NR'R", -L"-OC(O)R', -L"-OC(O)NR'R", -L"-C₆₋₁₀ aryl, -L"-3- to 14-membered carbocyclyl, -L"-3- to 14-membered heterocyclyl and -L"-5- to 14-membered heteroaryl;
L and L' are independently selected from covalent bond, -O-, -S-, -NR'-, -S(O)₂-, -C(O)-, -C(O)O-, -C(O)NR'-, -OC(O)-, -OC(O)NR'-, -N(R')C(O)O-, -N(R')C(O)- and -N(R')S(O)₂-; or, L or L' is C₁₋₈ alkylene, in which one or more methylene units are optionally and independently replaced by -C(R')₂-, -N(R')-, -N(R')C(O)-, -C(O)N(R')-, -N(R')S(O)₂-, -S(O)₂N(R')-, -O-, -C(O)-, -OC(O)-, -C(O)O-, -S-, -S(O)-, or -S(O)₂-;
R' and R" are independently selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl; or, R' and R" are taken together with the N atom to which they are attached to form a 3- to 14-membered heterocyclyl;
L" is selected from covalent bond, -O- and -S-; or, L" is C₁₋₈ alkylene, in which one or more methylene units are optionally and independently replaced by -O-, -C(O)-, -OC(O)-, -C(O)O-, -S-, -S(O)-, or -S(O)₂-;
f=0, 1, 2, 3, 4, 5, 6, 7, 8, 9 or 10;
wherein each of the above groups is optionally deuterated, up to completely deuterated.

### V

In one embodiment, V is a covalent bond; in another embodiment, V is O; in another embodiment, V is S; in another embodiment, V is NR'; in another embodiment, V is S(O); in another embodiment, V is S(O)₂; in another embodiment, V is C(O); in another embodiment, V is CR_{C}; in another embodiment, V is CR_{C}R_{C}'.

In a more specific embodiment, V is selected from covalent bond, CR_{C} and CR_{C}R_{C}'; in a more specific embodiment, V is selected from covalent bond, CH, CH₂, CD, CHD and CD₂; in a more specific embodiment, V is a covalent bond.

In one embodiment, V is not connected to W; in another embodiment, V is absent; in another embodiment, V is H; in another embodiment, V is D; in another embodiment, V is halogen, such as F; in another embodiment, V is C₁₋₈ alkyl, alternatively C₁₋₆ alkyl; in another embodiment, V is C₁₋₈ haloalkyl, alternatively C₁₋₆ haloalkyl.

In a more specific embodiment, V is selected from absent, H, D, halogen, C₁₋₈ alkyl and C₁₋₈ haloalkyl; in another more specific embodiment, V is selected from absent, H, D and halogen.

### Z₇

In one embodiment, Z₇ is C.

In one embodiment, V is not connected to W, V is absent, and Z₇ is N.

### W

In one embodiment, W is NR'; in another embodiment, W is S(O); in another embodiment, W is S(O)₂; in another embodiment, W is C(O); in another embodiment, W is CR_{C}; in another embodiment, W is CR_{C}R_{C}'.

In a more specific embodiment, W is CR_{C} or CR_{C}R_{C}'; in another more specific embodiment, W is CR_{C}R_{C}', for example, CH₂, CD, CHD or CD₂, for example, CH₂.

In one embodiment, V is not connected to W: in one embodiment, W is H; in another embodiment, W is C₁₋₆ alkyl, such as methyl; in another embodiment, W is C₁₋₆ haloalkyl.

In a more specific embodiment, W is selected from H and C₁₋₆ alkyl.

### Q and Y

In one embodiment, Q is a covalent bond; in another embodiment, Q is -O-; in another embodiment, Q is -S-; in another embodiment, Q is -NR_{A}-; in another embodiment, Q is -S(O)-; in another embodiment; in another embodiment, Q is -S(O)₂-; in another embodiment, Q is -C(O)-; in another embodiment, Q is -C(O)O-; in another embodiment, Q is -C(O)NR_{A}-; in another embodiment, Q is -OC(O)-; in another embodiment, Q is -OC(O)NR_{A}-; in another embodiment, Q is -N(R_{A})C(O)O-; in another embodiment, Q is -N(R_{A})C(O)-; in another embodiment, Q is -N(R_{A})S(O)₂-; in another embodiment, Q is CR₁ₐR_{1b};
or Q is C₁₋₈ alkylene, and in one embodiment, one or more methylene units in the C₁₋₈ alkylene are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
in one embodiment, Y is a covalent bond; in another embodiment, Y is -O-; in another embodiment, Y is -S-; in another embodiment, Y is -NR_{A}-; in another embodiment, Y is -S(O)-; in another embodiment; in another embodiment, Y is -S(O)₂-; in another embodiment, Y is -C(O)-; in another embodiment, Y is -C(O)O-; in another embodiment, Y is -C(O)NR_{A}-; in another embodiment, Y is -OC(O)-; in another embodiment, Y is -OC(O)NR_{A}-; in another embodiment, Y is -N(R_{A})C(O)O-; in another embodiment, Y is -N(R_{A})C(O)-; in another embodiment, Y is -N(R_{A})S(O)₂-; in another embodiment, Y is CR₁ₐR_{1b};
or Y is C₁₋₈ alkylene, and in one embodiment, one or more methylene units in the C₁₋₈ alkylene are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
and Q and Y are not covalent bonds at the same time.

In a more specific embodiment, Q and Y are independently selected from covalent bond, O, S, NR_{A}, S(O), S(O)₂, C(O) and CR₁ₐR_{1b}; and Q and Y are not covalent bonds at the same time.

In another more specific embodiment, Q is selected from O, S and NR_{A}; in another more specific embodiment, Q is S or NR_{A}; in another more specific embodiment, Q is NR_{A}, such as NH.

In another more specific embodiment, Y is selected from covalent bond, O, S and CR₁ₐR_{1b}; in another more specific embodiment, Y is selected from covalent bond, O and CR₁ₐR_{1b}; in another more specific embodiment, Y is selected from covalent bond, O, CH₂, CF₂ and C(CH₃)₂; in another more specific embodiment, Y is C(CH₃)₂; in another more specific embodiment, Y is

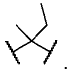

### R_{A}

In one embodiment, R_{A} is H; in another embodiment, R_{A} is C₁₋₈ alkyl; in another embodiment, R_{A} is C₁₋₈ alkoxy; in another embodiment, R_{A} is C₁₋₈ haloalkyl; in another embodiment, R_{A} is -L"-C₆₋₁₀ aryl; in another embodiment, R_{A} is -L"-3- to 14-membered carbocyclyl; in another embodiment, R_{A} is -L"-3- to 14-membered heterocyclyl or -L"-5- to 14-membered heteroaryl; in another embodiment, R_{A} is C₁₋₆ alkyl; in another embodiment, R_{A} is C₁₋₆ haloalkyl; in another embodiment, R_{A} is -L"-3- to 10-membered carbocyclyl; in another embodiment, R_{A} is -L"-3- to 10-membered heterocyclyl.

In a more specific embodiment, R_{A} is selected from H, C₁₋₆ alkyl, C₁₋₆ haloalkyl, -L"-3- to 10-membered carbocyclyl and -L"-3- to 10-membered heterocyclyl; in another more specific embodiment, R_{A} is selected from H, C₁₋₆ alkyl and C₁₋₆ haloalkyl; in another more specific embodiment, R_{A} is H.

### R_{B}

In one embodiment, R_{B} is H; in another embodiment, R_{B} is C₁₋₈ alkyl; in another embodiment, R_{B} is C₂₋₈ alkenyl; in another embodiment, R_{B} is C₂₋₈ alkynyl; in another embodiment, R_{B} is C₁₋₈ haloalkyl; in another embodiment, R_{B} is -L-OR'; in another embodiment, R_{B} is -L-NR'R"; in another embodiment, R_{B} is -L-C(O)NR'R"; in another embodiment, R_{B} is -L-C₆₋₁₀ aryl; in another embodiment, R_{B} is -L-3- to 14-membered carbocyclyl; in another embodiment, R_{B} is -L-3- to 14-membered heterocyclyl; in another embodiment, R_{B} is -L-5- to 14-membered heteroaryl; in another embodiment, R_{B} is C₁₋₆ alkyl; in another embodiment, R_{B} is C₁₋₆ haloalkyl; in another embodiment, R_{B} is -L-3- to 10-membered carbocyclyl; in another embodiment, R_{B} is -L-3- to 10-membered heterocyclyl.

In a more specific embodiment, R_{B} is selected from H, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl; in another more specific embodiment, R_{B} is selected from H, C₁₋₆ alkyl and C₁₋₆ haloalkyl; in another more specific embodiment, R_{B} is H.

### R_{C} and R'_{C}

In one embodiment, R_{C} is H; in another embodiment, R_{C} is D; in another embodiment, R_{C} is halogen; in another embodiment, R_{C} is -CN; in another embodiment, R_{C} is C₁₋₈ alkyl; in another embodiment, R_{C} is C₂₋₈ alkenyl; in another embodiment, R_{C} is C₂₋₈ alkynyl; in another embodiment, R_{C} is C₁₋₈ haloalkyl; in another embodiment, R_{C} is -L-OR'; in another embodiment, R_{C} is -L-NR'R"; in another embodiment, R_{C} is -L-C(O)NR'R"; in another embodiment, R_{C} is -L-C₆₋₁₀ aryl; in another embodiment, R_{C} is -L-3- to 14-membered carbocyclyl; in another embodiment, R_{C} is -L-3- to 14-membered heterocyclyl; in another embodiment, R_{C} is -L-5- to 14-membered heteroaryl; in another embodiment, R_{C} is C₁₋₆ alkyl; in another embodiment, R_{C} is C₁₋₆ haloalkyl; in another embodiment, R_{C} is -L-3- to 10-membered carbocyclyl; in another embodiment, R_{C} is -L-3- to 10-membered heterocyclyl.

In one embodiment, R'_{C} is H; in another embodiment, R'_{C} is D; in another embodiment, R'_{C} is halogen; in another embodiment, R'_{C} is -CN; in another embodiment, R'_{C} is C₁₋₈ alkyl; in another embodiment, R'_{C} is C₂₋₈ alkenyl; in another embodiment, R'_{C} is C₂₋₈ alkynyl; in another embodiment, R'_{C} is C₁₋₈ haloalkyl; in another embodiment, R'_{C} is -L-OR'; in another embodiment, R'_{C} is -L-NR'R"; in another embodiment, R'_{C} is -L-C(O)NR'R"; in another embodiment, R'_{C} is -L-C₆₋₁₀ aryl; in another embodiment, R'_{C} is -L-3- to 14-membered carbocyclyl; in another embodiment, R'_{C} is -L-3- to 14-membered heterocyclyl; in another embodiment, R'_{C} is -L-5- to 14-membered heteroaryl; in another embodiment, R'_{C} is C₁₋₆ alkyl; in another embodiment, R'_{C} is C₁₋₆ haloalkyl; in another embodiment, R'_{C} is -L-3- to 10-membered carbocyclyl; in another embodiment, R'_{C} is -L-3- to 10-membered heterocyclyl.

In a more specific embodiment, R_{C} and R'_{C} are independently selected from H, D, halogen, -CN, C₁₋₆ alkyl, C₁₋₈ haloalkyl, -L-OR', -L-NR'R", -L-C(O)NR'R", -L-3- to 10-membered carbocyclyl and -L-3- to 10-membered heterocyclyl; in another more specific embodiment, R_{C} and R'_{C} are independently selected from H, D, halogen, C₁₋₆ alkyl and C₁₋₆ haloalkyl; in another more specific embodiment, R_{C} and R'_{C} are independently H or D.

### R₁ₐ and R_{1b}

In one embodiment, R₁ₐ is H; in another embodiment, R₁ₐ is D; in another embodiment, R₁ₐ is halogen; in another embodiment, R₁ₐ is -CN; in another embodiment, R₁ₐ is C₁₋₈ alkyl; in another embodiment, R₁ₐ is C₂₋₈ alkenyl; in another embodiment, R₁ₐ is C₂₋₈ alkynyl; in another embodiment, R₁ₐ is C₁₋₈ haloalkyl; in another embodiment, R₁ₐ is -L-OR'; in another embodiment, Rc is -L-NR'R"; in another embodiment, R₁ₐ is -L-C(O)NR'R"; in another embodiment, R₁ₐ is -L-C₆₋₁₀ aryl; in another embodiment, R₁ₐ is -L-3- to 14-membered carbocyclyl; in another embodiment, R_{C} is -L-3- to 14-membered heterocyclyl; in another embodiment, R₁ₐ is -L-5- to 14-membered heteroaryl; in another embodiment, R₁ₐ is C₁₋₆ alkyl; in another embodiment, R₁ₐ is C₁₋₆ haloalkyl; in another embodiment, R₁ₐ is -L-3- to 10-membered carbocyclyl; in another embodiment, R₁ₐ is -L-3- to 10-membered heterocyclyl; in another embodiment, R₁ₐ is F; in another embodiment, R₁ₐ is Me; in another embodiment, R₁ₐ is Et.

In one embodiment, R_{1b} is H; in another embodiment, R_{1b} is D; in another embodiment, R_{1b} is halogen; in another embodiment, R_{1b} is -CN; in another embodiment, R_{1b} is C₁₋₈ alkyl; in another embodiment, R_{1b} is C₂₋₈ alkenyl; in another embodiment, R_{1b} is C₂₋₈ alkynyl; in another embodiment, R_{1b} is C₁₋₈ haloalkyl; in another embodiment, R_{1b} is -L-OR'; in another embodiment, R_{1b} is -L-NR'R"; in another embodiment, R_{1b} is -L-C(O)NR'R"; in another embodiment, R_{1b} is -L-C₆₋₁₀ aryl; in another embodiment, R_{1b} is -L-3- to 14-membered carbocyclyl; in another embodiment, R_{1b} is -L-3- to 14-membered heterocyclyl; in another embodiment, R_{1b} is -L-5 to 14-membered heteroaryl; in another embodiment, R_{1b} is C₁₋₆ alkyl; in another embodiment, R_{1b} is C₁₋₆ haloalkyl; in another embodiment, R_{1b} is -L-3- to 10-membered carbocyclyl; in another embodiment, R_{1b} is -L-3- to 10-membered heterocyclyl; in another embodiment, R_{1b} is F; in another embodiment, R_{1b} is Me; in another embodiment, R_{1b} is Et.

In a more specific embodiment, R₁ₐ and R_{1b} are independently selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl; in another more specific embodiment, R₁ₐ and R_{1b} are independently selected from H, D, halogen, CN, C₁₋₆ alkyl and C₁₋₆ haloalkyl; in another more specific embodiment, R₁ₐ and R_{1b} are independently selected from H, D, halogen, C₁₋₆ alkyl and C₁₋₆ haloalkyl; in another more specific embodiment, R₁ₐ and R_{1b} are independently selected from H, D, halogen, C₁₋₃ alkyl and C₁₋₃ haloalkyl; in another more specific embodiment, R₁ₐ and R_{1b} are independently selected from H, D, F and Me; in another more specific embodiment, R₁ₐ and R_{1b} are Me.

In a more specific embodiment, R₁ₐ and R_{1b} are independently selected from C₁₋₆ alkyl and C₁₋₆ haloalkyl; in another more specific embodiment, R₁ₐ and R_{1b} are independently selected from C₁₋₃ alkyl and C₁₋₃ haloalkyl; in another more specific embodiment, R₁ₐ and R_{1b} are independently selected from Me and Et.

### Z₁ and Z₆

In one embodiment, Z₁ is CR_{E}, and Z₆ is CR_{d1}; in another embodiment, Z₁ is CR_{E}, and Z₆ is N; in another embodiment, Z₆ is CR_{E}, and Z₁ is CR_{d1}; in another embodiment, Z₆ is CR_{E}, and Z₁ is N.

In a more specific embodiment, Z₆ is CR_{E}, and Z₁ is CH or CD; in another more specific embodiment, Z₁ is CR_{E}, and Z₆ is CH or CD.

### R_{E}

In one embodiment, R_{E} is -L-C₆₋₁₀ aryl; in another embodiment, R_{E} is -L-3- to 14-membered carbocyclyl; in another embodiment, R_{E} is -L-3- to 14-membered heterocyclyl; in another embodiment, R_{E} is -L-5- to 14-membered heteroaryl; in another embodiment, R_{E} is phenyl; in another embodiment, R_{E} is pyridyl; in another embodiment, R_{E} is pyridonyl; in another embodiment, R_{E} is pyrimidinyl; in another embodiment, R_{E} is pyrazinyl; in another embodiment, R_{E} is pyrrolyl; in another embodiment, R_{E} is pyrazolyl; in another embodiment, R_{E} is imidazolyl; in another embodiment, R_{E} is oxazolyl; in another embodiment, R_{E} is triazolyl; in another embodiment, R_{E} is pyrrolopyridyl; in another embodiment, R_{E} is pyrrolopyridazinyl; in another embodiment, R_{E} is pyrrolopyrimidinyl; in another embodiment, R_{E} is pyrrolopyrazinyl; in another embodiment, R_{E} is imidazopyridyl; in another embodiment, R_{E} is imidazopyridazinyl; in another embodiment, R_{E} is imidazopyrimidinyl; in another embodiment, R_{E} is imidazopyrazinyl; in another embodiment, R_{E} is pyrazolopyridyl; in another embodiment, R_{E} is pyrazolopyridazinyl; in another embodiment, R_{E} is pyrazolopyrimidinyl; in another embodiment, R_{E} is pyrazolopyrazinyl; in another embodiment, R_{E} is pyridopyridyl; in another embodiment, R_{E} is dihydropyridooxazinyl; in another embodiment, R_{E} is indolyl; in another embodiment, R_{E} is triazolopyridyl; in another embodiment, R_{E} is triazolopyridyl; in another embodiment, R_{E} is tetrahydroimidazopyrazinyl; in another embodiment, R_{E} is dihydropyrrolopyridyl; in another embodiment, R_{E} is thienopyridyl; in another embodiment, R_{E} is benzimidazolyl; in another embodiment, R_{E} is dihydroimidazooxazinyl; in another embodiment, R_{E} is tetrahydropyrazolopyridyl; in another embodiment, R_{E} is tetrahydropyrrolopyridyl; in another embodiment, R_{E} is dihydropyrazolooxazinyl; in another embodiment, R_{E} is dihydropyrroloimidazolyl; in another embodiment, R_{E} is oxazolopyridyl; in another embodiment, R_{E} is imidazocyclohexyl; in another embodiment, R_{E} is pyrazoloazepanyl; in another embodiment, R_{E} is pyrazolopiperidyl; in another embodiment, R_{E} is pyrazolopyrrolidinyl; in another embodiment, R_{E} is imidazoazepanyl; in another embodiment, R_{E} is imidazopiperidyl; in another embodiment, R_{E} is imidazopyrrolidinyl; in another embodiment, R_{E} is imidazopyrazinonyl; in another embodiment, R_{E} is imidazodiazacyclohexyl; in another embodiment, R_{E} is imidazopiperazinonyl; in another embodiment, R_{E} is pyrazolodiazepanyl; in another embodiment, R_{E} is pyrazolodiazacyclohexyl; in another embodiment, R_{E} is pyrazolodiazacyclohexyl; in another embodiment, R_{E} is dihydropyrazolooxazinyl; in another embodiment, R_{E} is triazolopiperidyl; in another embodiment, R_{E} is thienopyridyl; in another embodiment, R_{E} is optionally substituted with 1, 2, 3, 4, 5, 6, 7, 8, 9 or 10 Rₑ groups.

In a more specific embodiment, R_{E} is 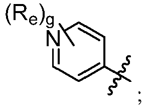 in another more specific embodiment, R_{E} is 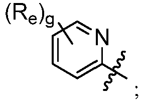 in another more specific embodiment, R_{E} is 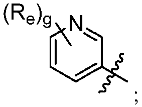 in another more specific embodiment, R_{E} is 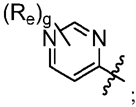 in another more specific embodiment, R_{E} is 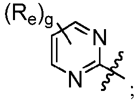 in another more specific embodiment, R_{E} is 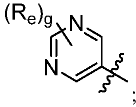 in another more specific embodiment, R_{E} is 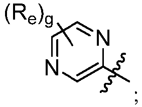 in another more specific embodiment, R_{E} is 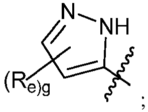 in another more specific embodiment, R_{E} is 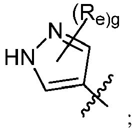 in another more specific embodiment, R_{E} is 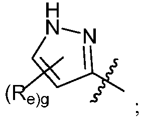 in another more specific embodiment, R_{E} is 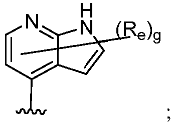 in another more specific embodiment, R_{E} is 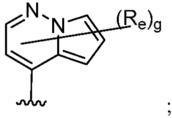 in another more specific embodiment, R_{E} is 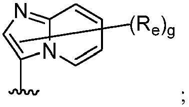 in another more specific embodiment, R_{E} is 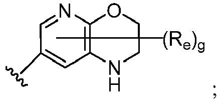 in another more specific embodiment, R_{E} is 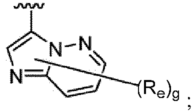 in another more specific embodiment, R_{E} is 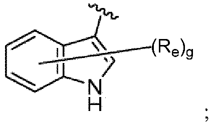 in another more specific embodiment, R_{E} is 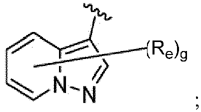 in another more specific embodiment, R_{E} is 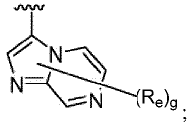 in another more specific embodiment, R_{E} is 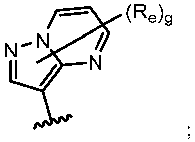 in another more specific embodiment, R_{E} is 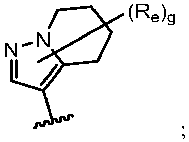 in another more specific embodiment, R_{E} is 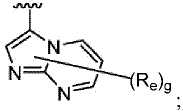 in another more specific embodiment, R_{E} is 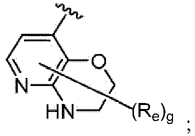 in another more specific embodiment, R_{E} is 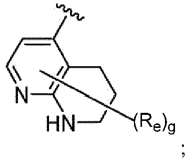 wherein g=0, 1, 2, 3, 4, 5, 6, 7, 8, 9, or 10; in another more specific embodiment, g=0, 1, 2, 3, 4, 5, or 6; in another more specific embodiment, g=0, 1, 2, 3, or 4; in another more specific embodiment, g=0, 1, or 2.

In a more specific embodiment, R_{E} and a substituent Rₑ thereon are taken together to form 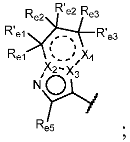 in another more specific embodiment, R_{E} and a substituent Rₑ thereon are taken together to form 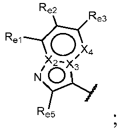 in another more specific embodiment, R_{E} and a substituent Rₑ thereon are taken together to form 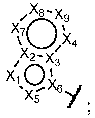 in another more specific embodiment, R_{E} and a substituent Rₑ thereon are taken together to form 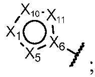 in another more specific embodiment, R_{E} and a substituent Rₑ thereon are taken together to form 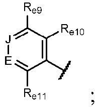 wherein X₁ is CRₑ₆, N, O, S or NR'ₑ₆, alternatively CRₑ₆, N or NR'ₑ₆, alternatively N, alternatively CRₑ₆, alternatively O, alternatively S, alternatively NR'ₑ₆; X₂ is C or N, alternatively C, alternatively N; X₃ is C or N, alternatively C, alternatively N; X₄ is CRₑ₄ or N, alternatively CRₑ₄, alternatively N; X₅ is CRₑ₅, N, O, S or NR'ₑ₅, alternatively CRₑ₅, O, N or NR'ₑ₅, alternatively CRₑ₅, N or NR'ₑ₅, alternatively CRₑ₅ or N, alternatively CRₑ₅, alternatively N, alternatively O, alternatively S, alternatively NR'ₑ₅; X₆ is C or N, alternatively C, alternatively N; X₇ is CRₑ₁ or N, alternatively CRₑ₁, alternatively N; X₈ is CRₑ₂ or N, alternatively CRₑ₂, alternatively N; X₉ is CRₑ₃ or N, alternatively CRₑ₃, alternatively N; X₁₀ is selected from CRₑ₇, O, S, N and NR'ₑ₇, alternatively CRₑ₇, N or NR'ₑ₇, alternatively CRₑ₇ or NR'ₑ₇, alternatively CRₑ₇, alternatively NR'ₑ₇, alternatively O, alternatively S, alternatively N; X₁₁ is CRₑ₈, O, S, N, or NR'ₑ₈, alternatively CRₑ₈, O, N, or NR'ₑ₈, alternatively CRₑ₈, O, or NR'ₑ₈, alternatively CRₑ₈ or NR'ₑ₈, alternatively CRₑ₈, alternatively NR'ₑ₈, alternatively O, alternatively S, alternatively N; E is selected from CRₑ₁₂ and N, alternatively CRₑ₁₂, alternatively N; J is selected from CRₑ₁₃ and N, alternatively CRₑ₁₃, alternatively N.

In a more specific embodiment, the substituents on X₁₀ and X₁₁ are taken together to form a C₃₋₅ alkylene, which optionally contains one double bond, and the C₃₋₅ alkylene is optionally substituted with 1, 2, 3 or 4 Rₑ, and one or more methylene units in the C₃₋₅ alkylene are optionally and independently replaced by NR"-, -N(R")C(O)-, -C(O)N(R")-, -N(R")S(O)₂-, -S(O)₂N(R")-, -O-, -C(O)-, -OC(O)-, -C(O)O-, -S-, -S(O)-, or -S(O)₂-, alternatively, 1, 2 or 3 methylene units in the C₃₋₅ alkylene are optionally and independently replaced by -NR"-, -N(R")C(O)-, -C(O)N(R")-, -O-, -C(O)-, -OC(O)-, -C(O)O-, -S-, or -S(O)-; in another more specific embodiment, the substituents on X₁₀ and X₁₁ are taken together to form a C₃₋₅ alkylene, alternatively form -(CH₂)₄-, which optionally contains one double bond, and the C₃₋₅ alkylene or -(CH₂)₄- is optionally substituted with 1, 2, 3 or 4 Rₑ, and 1, 2, or 3 methylene units in the C₃₋₅ alkylene or -(CH₂)₄- are optionally and independently replaced by -NR"-, -C(O)N(R")-, -C(O)-, or -O-, alternatively, one methylene unit in the C₃₋₅ alkylene or -(CH₂)₄- is optionally and independently replaced by -O-; in another more specific embodiment, the substituents on X₁₀ and X₁₁ are taken together to form 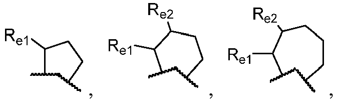 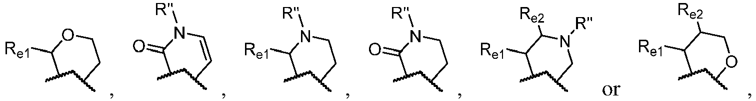 alternatively not form 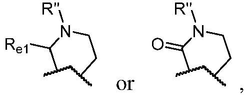 alternatively form a structure selected from: 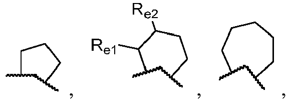 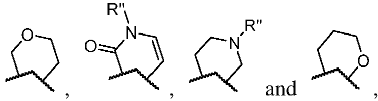 alternatively form

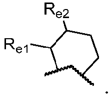

In another more specific embodiment, the substituents on X₁₀ and X₁₁ are taken together to form 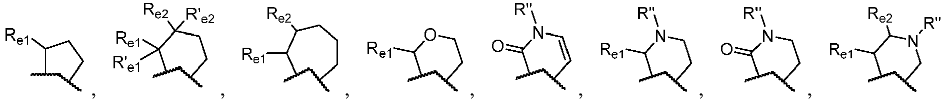 or 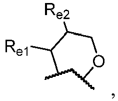 alternatively not form 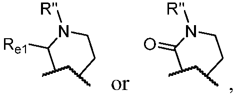 alternatively form a structure selected from:

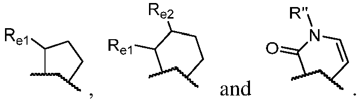

In a more specific embodiment, Rₑ₁, Rₑ₂, Rₑ₃, Rₑ₄, Rₑ₅ and Rₑ₆ are independently selected from H, D, halogen, -CN, C₁₋₈ alkyl, C₁₋₈ haloalkyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-C₆₋₁₀ aryl, -L'-3- to 14-membered carbocyclyl, -L'-3- to 14-membered heterocyclyl and -L'-5- to 14-membered heteroaryl; in another more specific embodiment, Rₑ₁ and Rₑ₂ are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl; in another more specific embodiment, Rₑ₁ and Rₑ₂ are independently selected from H, D, halogen, CN, C₁₋₆ alkyl, C₁₋₆ haloalkyl, -OR', -NR'R", 3- to 7-membered carbocyclyl and 3- to 7-membered heterocyclyl, alternatively selected from H, D, halogen, CN, C₁₋₆ alkyl, C₁₋₆ haloalkyl, -OR' and -NR'R"; in another more specific embodiment, Rₑ₁ and Rₑ₂ are independently selected from H, D, halogen, CN, C₁₋₆ alkyl, C₁₋₆ haloalkyl, -OR', -O-3- to 7-membered carbocyclyl and 3- to 7-membered heterocyclyl, alternatively selected from H, D, halogen, CN, C₁₋₆ alkyl, C₁₋₆ haloalkyl and -OR'; in another more specific embodiment, Rₑ₁ and Rₑ₂ are independently selected from H, D, halogen, CN, C₁₋₆ alkyl, C₁₋₆ haloalkyl, -OR' and -O-3- to 7-membered carbocyclyl, alternatively selected from H, D, C₁₋₆ alkyl, C₁₋₆ haloalkyl and -OR'; in another more specific embodiment, Rₑ₁ and Rₑ₂ are independently selected from H, F, Cl, CN, Me, Et, CHF₂, OCH₃, OEt, OCHF₂, -O-cyclopropyl and 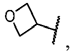 alternatively selected from H, F, Cl, CN, Me, CHF₂, OCH₃ and OEt, alternatively Rₑ₂ is not OEt; in another more specific embodiment, Rₑ₁ and Rₑ₂ are independently selected from H, F, CN, Me, OMe, OEt, OCHF₂ and -O-cyclopropyl, alternatively selected from H, F, CN, Me, OCH₃, OEt, OCHF₂ and -O-cyclopropyl, alternatively selected from H, Me, OCH₃ and OEt;

in a more specific embodiment, Rₑ₃, Rₑ₄, Rₑ₅ and Rₑ₆ are independently selected from H, D, halogen, CN, C₁₋₆ alkyl and C₁₋₆ haloalkyl; in another more specific embodiment, R'ₑ₅ and R'ₑ₆ are independently selected from H, C₁₋₆ alkyl and C₁₋₆ haloalkyl; in another more specific embodiment, Rₑ₃, Rₑ₄, Rₑ₅ and Rₑ₆ are independently H, D, halogen, C₁₋₆ alkyl or C₁₋₆ haloalkyl; in another more specific embodiment, Rₑ₃, Rₑ₄ and Rₑ₅ are independently H or D; in another more specific embodiment, Rₑ₃, Rₑ₄ and Rₑ₅ are H; in a more specific embodiment, R'ₑ₅ and R'ₑ₆ are independently selected from H, C₁₋₈ alkyl and C₁₋₈ haloalkyl; in another more specific embodiment, R'ₑ₅ and R'ₑ₆ are independently selected from H, C₁₋₆ alkyl and C₁₋₆ haloalkyl; in another more specific embodiment, R'ₑ₅ is selected from H, C₁₋₃ alkyl and C₁₋₃ haloalkyl; in another more specific embodiment, R'ₑ₅ is Me; in another more specific embodiment, Rₑ₅ is H.

In a more specific embodiment, Rₑ₅ is selected from H, D, halogen, CN, C₁₋₆ alkyl and C₁₋₆ haloalkyl; in another more specific embodiment, Rₑ₅ is selected from H, D, halogen, C₁₋₆ alkyl and C₁₋₆ haloalkyl; in another more specific embodiment, Rₑ₅ is selected from H, D, halogen, C₁₋₄ alkyl and C₁₋₄ haloalkyl; in another more specific embodiment, Rₑ₅ is H or D; in another more specific embodiment, Rₑ₅ is selected from H, D, F, Br and Me; in another more specific embodiment, Rₑ₅ is H.

In one embodiment, Rₑ₁/Rₑ₂ is H; in another embodiment, Rₑ₁/Rₑ₂ is D; in another embodiment, Rₑ₁/Rₑ₂ is halogen; in another embodiment, Rₑ₁/Rₑ₂ is CN; in another embodiment, Rₑ₁/Rₑ₂ is C₁₋₆ alkyl; in another embodiment, Rₑ₁/Rₑ₂ is C₁₋₆ haloalkyl; in another embodiment, Rₑ₁/Rₑ₂ is C₂₋₆ alkenyl; in another embodiment, Rₑ₁/Rₑ₂ is C₂₋₆ alkynyl; in another embodiment, Rₑ₁/Rₑ₂ is -L'-OR'; in another embodiment, Rₑ₁/Rₑ₂ is -L'-SR'; in another embodiment, Rₑ₁/Rₑ₂ is -L'-NR'R"; in another embodiment, Rₑ₁/Rₑ₂ is -L'-3- to 7-membered carbocyclyl; in another embodiment, Rₑ₁/Rₑ₂ is -L'-3- to 7-membered heterocyclyl; in another embodiment, Rₑ₁/Rₑ₂ is optionally substituted with 1, 2 or 3 Rₑₛ; in another embodiment, Rₑ₁/Rₑ₂ is unsubstituted.

In one embodiment, the configuration of Rₑ₁ in the general formula is 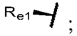 in another embodiment, the configuration of Rₑ₁ in the general formula is 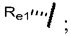 in another embodiment, the configuration of Rₑ₂ in the general formula is 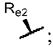 in another embodiment, the configuration of Rₑ₂ in the general formula is

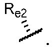

In a more specific embodiment, Rₑ₁ and Rₑ₂ are independently selected from H, D, halogen, CN, C₁₋₆ alkyl, C₁₋₆ haloalkyl, C₂₋₆ alkenyl, C₂₋₆ alkynyl, -L'-OR', -L'-SR', -L'-NR'R'', -L'-3- to 7-membered carbocyclyl and -L'-3- to 7-membered heterocyclyl; in another more specific embodiment, Rₑ₁ and Rₑ₂ are independently selected from H, D, halogen, CN, C₁₋₆ alkyl, C₁₋₆ haloalkyl, C₂₋₆ alkenyl, C₂₋₆ alkynyl, -OR', -NR'R", 3- to 7-membered carbocyclyl and 3- to 7-membered heterocyclyl; in another more specific embodiment, Rₑ₁ and Rₑ₂ are independently selected from H, D, halogen, CN, C₁₋₆ alkyl, C₁₋₆ haloalkyl, C₂₋₆ alkynyl, -OR', -NR'R", -O-3- to 7-membered carbocyclyl, -O-3- to 7-membered heterocyclyl, 3- to 7-membered carbocyclyl and 3- to 7-membered heterocyclyl; in another more specific embodiment, Rₑ₁ and Rₑ₂ are independently not -NR'R"; in another more specific embodiment, Rₑ₁ and Rₑ₂ are independently selected from H, D, halogen, CN, C₁₋₆ alkyl, C₁₋₆ haloalkyl, C₂₋₆ alkynyl, -OR', -O-3- to 7-membered carbocyclyl, 3- to 7-membered carbocyclyl and 3- to 7-membered heterocyclyl; in another more specific embodiment, Rₑ₁ is not heterocyclyl.

In one embodiment, Rₑ₁/Rₑ₂ is H; in another embodiment, Rₑ₁/Rₑ₂ is F; in another embodiment, Rₑ₁/Rₑ₂ is Cl; in another embodiment, Rₑ₁/Rₑ₂ is CN; in another embodiment, Rₑ₁/Rₑ₂ is Me; in another embodiment, Rₑ₁/Rₑ₂ is Et; in another embodiment, Rₑ₁/Rₑ₂ is n-Bu; in another embodiment, Rₑ₁/Rₑ₂ is CHF₂; in another embodiment, Rₑ₁/Rₑ₂ is 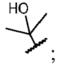 in another embodiment, Rₑ₁/Rₑ₂ is 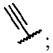 in another embodiment, Rₑ₁/Rₑ₂ is OH; in another embodiment, Rₑ₁/Rₑ₂ is OCH₃; in another embodiment, Rₑ₁/Rₑ₂ is OEt; in another embodiment, Rₑ₁/Rₑ₂ is OCHF₂; in another embodiment, Rₑ₁/Rₑ₂ is -NHCH₃; in another embodiment, Rₑ₁/Rₑ₂ is -O-cyclopropyl; in another embodiment, Rₑ₁/Rₑ₂ is 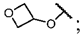 in another embodiment, Rₑ₁/Rₑ₂ is 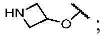 in another embodiment, Rₑ₁/Rₑ₂ is cyclopropyl; in another embodiment, Rₑ₁/Rₑ₂ is 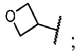 in another embodiment, Rₑ₁/Rₑ₂ is 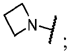 in another embodiment, Rₑ₁/Rₑ₂ is 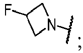 in another embodiment, Rₑ₁/Rₑ₂ is 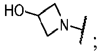 in another embodiment, Rₑ₁/Rₑ₂ is 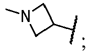 in another embodiment, Rₑ₁/Rₑ₂ is

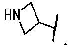

In a more specific embodiment, Rₑ₁ and Rₑ₂ are independently selected from H, F, Cl, CN, Me, Et, n-Bu, CHF₂, 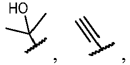 OH, OCH₃, OEt, OCHF₂, -NHCH₃, -O-cyclopropyl, 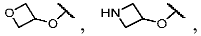 cyclopropyl, 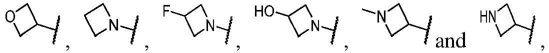 alternatively selected from H, F, Cl, CN, Me, Et, n-Bu, CHF₂, 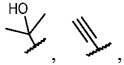 OCH₃, OEt, OCHF₂, -NHCH₃, -O-cyclopropyl, 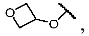 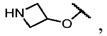 cyclopropyl, 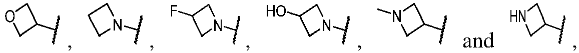 alternatively not 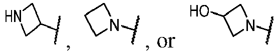 , alternatively selected from H, F, Cl, CN, Me, Et, CHF₂, 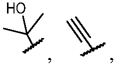 OCH₃, OEt, OCHF₂, -O-cyclopropyl, cyclopropyl,

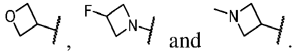

In a more specific embodiment, Rₑ₁ is not 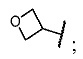 in another more specific embodiment, Rₑ₂ is not OEt.

In a more specific embodiment, Rₑ₁ is selected from H, D, halogen, CN, C₁₋₆ alkyl, C₁₋₆ haloalkyl, -OR', -NR'R'', -O-3- to 7-membered carbocyclyl, -O-3- to 7-membered heterocyclyl, 3- to 7-membered carbocyclyl and 3- to 7-membered heterocyclyl; in another more specific embodiment, Rₑ₁ is not -NR'R'' or 3-to 7-membered heterocyclyl; in another more specific embodiment, Rₑ₁ is selected from H, D, halogen, CN, C₁₋₆ alkyl, C₁₋₆ haloalkyl, -OR', -O-3- to 7-membered carbocyclyl and 3- to 7-membered carbocyclyl; in another more specific embodiment, Rₑ₁ is selected from H, F, Cl, CN, Me, Et, n-Bu, CHF₂, OCH₃, OEt, OCHF₂, -NHCH₃, -O-cyclopropyl, 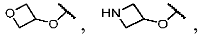 cyclopropyl, 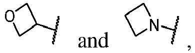 alternatively selected from H, F, Cl, CN, Me, Et, CHF₂, OCH₃, OEt, OCHF₂, -O-cyclopropyl, 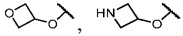 and cyclopropyl, alternatively selected from H, F, CN, Me, CHF₂, OCH₃, OEt, OCHF₂, -O-cyclopropyl and cyclopropyl.

In a more specific embodiment, Rₑ₂ is selected from H, D, halogen, CN, C₁₋₆ alkyl, C₁₋₆ haloalkyl, C₂₋₆ alkynyl, -OR', -O-3- to 7-membered heterocyclyl, 3- to 7-membered carbocyclyl and 3- to 7-membered heterocyclyl; in another more specific embodiment, Rₑ₂ is selected from H, D, halogen, CN, C₁₋₆ alkyl, C₁₋₆ haloalkyl, C₂₋₆ alkynyl, -OMe, -O-halomethyl, 3- to 7-membered carbocyclyl and 3- to 7-membered heterocyclyl; in another more specific embodiment, Rₑ₂ is selected from H, F, Cl, CN, Me, CHF₂, 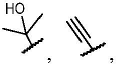 OCH₃, OEt, OCHF₂, 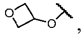 cyclopropyl, 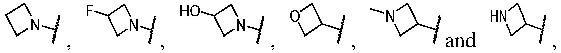 alternatively selected from H, F, Cl, CN, Me, CHF₂, 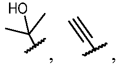 OCH₃, OCHF₂, cyclopropyl,

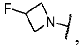

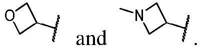

In a more specific embodiment, when Rₑ₁/Rₑ₂ is heterocyclyl, Rₑₛ is independently selected from H, D, halogen (F), C₁₋₆ alkyl and C₁₋₆ haloalkyl, alternatively selected from H, D, F and Me, alternatively halogen (F).

In a more specific embodiment, Rₑ₁ and Rₑ₂ are independently selected from H, D, halogen, CN, C₁₋₆ alkyl, C₁₋₆ haloalkyl, -OR', -O-3- to 7-membered carbocyclyl and -O-3- to 7-membered heterocyclyl, alternatively not halogen, alternatively selected from H, D, C₁₋₆ alkyl, C₁₋₆ haloalkyl, -OR' and -O-3- to 7-membered carbocyclyl; in another more specific embodiment, Rₑ₂ is selected from H, D, halogen, CN, C₁₋₆ alkyl, C₁₋₆ haloalkyl and -OR'; in another more specific embodiment, alternatively Rₑ₂ is H, D or C₁₋₆ haloalkyl;
in a more specific embodiment, Rₑ₁ and Rₑ₂ are independently selected from H, F, CN, Me, CHF₂, OH, OMe, OEt, OCHF₂, -O-cyclopropyl and 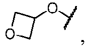 alternatively selected from H, CN, Me, CHF₂, OH, OCH₃, OEt, -O-cyclopropyl and 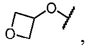 alternatively selected from H, Me, CHF₂, OCH₃, OEt and -O-cyclopropyl; in another more specific embodiment, Rₑ₂ is selected from H, F, CN, Me, CHF₂, OH and OMe; in another more specific embodiment, Rₑ₂ is H or CHF₂.

In a more specific embodiment, R'ₑ₁ and R'ₑ₂ are independently selected from H, D, halogen, C₁₋₆ alkyl and C₁₋₆ haloalkyl, alternatively selected from H, D, halogen and C₁₋₆ alkyl; in another more specific embodiment, R'ₑ₁ and R'ₑ₂ are independently selected from H, F and Me.

In a more specific embodiment, CRₑ₁R'ₑ₁ forms a 3- to 7-membered carbocyclyl or a 3- to 7-membered heterocyclyl, alternatively forms a 3- to 7-membered heterocyclyl (e.g., 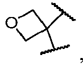 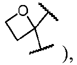 alternatively forms a 3- to 7-membered heterocyclyl or a 3- to 7-membered carbocyclyl; in another more specific embodiment, CRₑ₁R'ₑ₁ forms 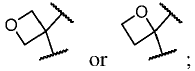 in another more specific embodiment, CRₑ₁R'ₑ₁ does not form a ring.

In a more specific embodiment, Rₑ₇ and Rₑ₈ are independently selected from H, D, halogen, CN, C₁₋₈ alkyl, C₁₋₈ haloalkyl, -L'-OR', -L'-SR', -L'-NR'R", -L'-3- to 10-membered carbocyclyl and -L'-3- to 10-membered heterocyclyl; in another more specific embodiment, R'ₑ₇ and R'ₑ₈ are independently selected from H, D, C₁₋₈ alkyl, C₁₋₈ haloalkyl, -L'-3- to 10-membered carbocyclyl and -L'-3- to 10-membered heterocyclyl; in another more specific embodiment, Rₑ₇ and Rₑ₈ are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl; in another more specific embodiment, R'ₑ₇ and R'ₑ₈ are independently selected from H, D, C₁₋₆ alkyl, C₁₋₆ haloalkyl, -L'-3- to 7-membered carbocyclyl and -L'-3- to 7-membered heterocyclyl; in another more specific embodiment, Rₑ₇ is selected from H, D, C₁₋₆ alkyl, C₁₋₆ haloalkyl, -OR', -NR'R", 3- to 7-membered carbocyclyl and 3- to 7-membered heterocyclyl; in another more specific embodiment, R'ₑ₇ is selected from H, D, C₁₋₆ alkyl, C₁₋₆ haloalkyl, 3- to 7-membered carbocyclyl and 3- to 7-membered heterocyclyl; in another more specific embodiment, Rₑ₈ is selected from H, D, C₁₋₆ alkyl and C₁₋₆ haloalkyl; in another more specific embodiment, R'ₑ₈ is selected from H, C₁₋₆ alkyl and C₁₋₆ haloalkyl; in another more specific embodiment, Rₑ₇ is selected from C₁₋₆ alkyl, C₁₋₆ haloalkyl, 3- to 7-membered carbocyclyl, 3- to 7-membered heterocyclyl and -O-3- to 7-membered heterocyclyl; in another more specific embodiment, R'ₑ₇ is selected from C₁₋₆ alkyl, C₁₋₆ haloalkyl, 3- to 7-membered carbocyclyl and 3- to 7-membered heterocyclyl, alternatively 3- to 7-membered carbocyclyl; in another more specific embodiment, when Rₑ₇ or R'ₑ₇ is heterocyclyl, Rₑ₇ or R'ₑ₇ is 3- to 7-membered oxacyclyl, alternatively 4- to 6-membered oxacyclyl, alternatively 5-membered oxacyclyl; in another more specific embodiment, Rₑ₈ is selected from H, D, C₁₋₆ alkyl and C₁₋₆ haloalkyl, alternatively C₁₋₆ alkyl or C₁₋₆ haloalkyl; in another more specific embodiment, R'ₑ₈ is selected from C₁₋₆ alkyl and C₁₋₆ haloalkyl; in another more specific embodiment, Rₑ₇ is selected from Me, Et, cyclopropyl, tetrahydrofuranyl, tetrahydropyranyl, pyrrolidinyl, morpholinyl and -O-tetrahydrofuranyl, alternatively selected from Me, Et, cyclopropyl, tetrahydrofuranyl, tetrahydropyranyl and -O-tetrahydrofuranyl, alternatively selected from Me, Et, cyclopropyl, tetrahydrofuranyl and -O-tetrahydrofuranyl, alternatively selected from Me, Et, cyclopropyl, 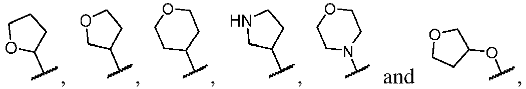 alternatively selected from Me, Et, cyclopropyl, 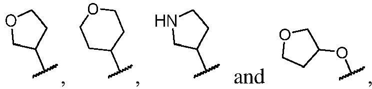 alternatively selected from Me, Et, cyclopropyl, 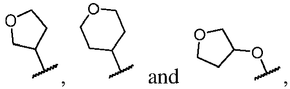 alternatively selected from Me, Et, cyclopropyl, 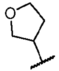 and 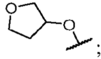 in another more specific embodiment, R'ₑ₇ is selected from Me, Et, cyclopropyl, tetrahydrofuranyl, tetrahydropyranyl, pyrrolidinyl and morpholinyl, alternatively selected from Me, Et, cyclopropyl, tetrahydrofuranyl and tetrahydropyranyl, alternatively selected from Me, Et, cyclopropyl and tetrahydrofuranyl, alternatively selected from Me, Et, cyclopropyl, 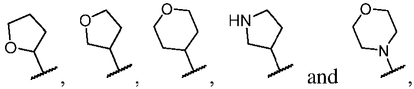 alternatively selected from Me, Et, cyclopropyl, 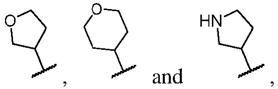 alternatively selected from Me, Et, cyclopropyl, 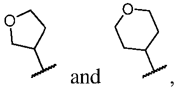 alternatively selected from Me, Et, cyclopropyl and 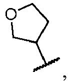 alternatively cyclopropyl; in another more specific embodiment, in another more specific embodiment, Rₑ₈ is selected from H, Me and Et, alternatively H or Me, alternatively Me; in another more specific embodiment, R'ₑ₈ is Me or Et, alternatively Me.

In a more specific embodiment, Rₑ₇ is selected from C₁₋₆ alkyl, C₁₋₆ haloalkyl, 3- to 7-membered carbocyclyl, tetrahydrofuranyl, morpholinyl and -O-3- to 7-membered heterocyclyl; in another more specific embodiment, Rₑ₇ is selected from Me, Et, cyclopropyl, 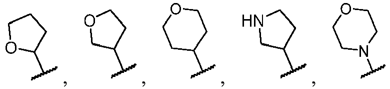 and 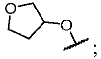 in another more specific embodiment, Rₑ₇ is selected from Me, Et, cyclopropyl, 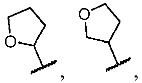 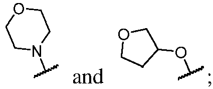 in another more specific embodiment, Rₑ₇ is 3- to 7-membered carbocyclyl; in another more specific embodiment, Rₑ₇ is cyclopropyl.

In a more specific embodiment, R'ₑ₇ is selected from Me, Et and cyclopropyl; in another more specific embodiment, R'ₑ₇ is cyclopropyl.

In a more specific embodiment, Rₑ₉, Rₑ₁₀, Rₑ₁₁, Rₑ₁₂ and Rₑ₁₃ are selected from H, D, halogen, CN, C₁₋₈ alkyl, C₁₋₈ haloalkyl, C₂₋₈ alkenyl, C₂₋₈ alkynyl, -L'-OR', -L'-SR', -L'-NR'R'', -L'-3- to 10-membered carbocyclyl, -L'-3- to 10-membered heterocyclyl and -Y₅-C₀₋₈ alkylene-R₆; in a more specific embodiment, Rₑ₉, Rₑ₁₀, Rₑ₁₁, Rₑ₁₂ and Rₑ₁₃ are selected from H, D, halogen, CN, C₁₋₆ alkyl, C₁₋₆ haloalkyl, C₂₋₆ alkenyl, C₂₋₆ alkynyl, -L'-OR', -L'-SR', -L'-NR'R'', -L'-3- to 7-membered carbocyclyl, -L'-3- to 7-membered heterocyclyl and -Y₅-C₀₋₆ alkylene-R₆; in a more specific embodiment, Rₑ₉ is selected from H, D, halogen, C₁₋₆ alkyl, C₁₋₆ haloalkyl, C₂₋₆ alkenyl, C₂₋₆ alkynyl, -OR', -NR'R'', 3- to 7-membered carbocyclyl, 3- to 7-membered heterocyclyl and -Y₅-C₀₋₆ alkylene-R₆, alternatively selected from H, D, halogen, C₁₋₆ alkyl, C₁₋₆ haloalkyl, C₂₋₆ alkenyl, C₂₋₆ alkynyl, -OR' and -NR'R''; in a more specific embodiment, Rₑ₁₀ is selected from H, D, halogen, C₁₋₆ alkyl and C₁₋₆ haloalkyl; in a more specific embodiment, Rₑ₁₁, Rₑ₁₂ and Rₑ₁₃ are H, D, C₁₋₆ alkyl or C₁₋₆ haloalkyl; in a more specific embodiment, Rₑ₉ is selected from H, D, C₁₋₆ alkyl, C₁₋₆ haloalkyl, C₂₋₄ alkynyl, -NR'R", 3- to 7-membered heterocyclyl and -O-C₀₋₄ alkylene-3- to 7-membered heterocyclyl, alternatively selected from H, D, C₁₋₆ alkyl, C₁₋₆ haloalkyl, C₂₋₄ alkynyl and -NR'R"; in a more specific embodiment, Rₑ₁₀ is selected from H, D, C₁₋₆ alkyl and C₁₋₆ haloalkyl; in a more specific embodiment, Rₑ₁₁, Rₑ₁₂ and Rₑ₁₃ are H or D; in a more specific embodiment, Rₑ₉ is selected from H, -NHMe, 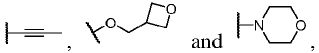 alternatively selected from H, -NHMe and 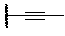 ; in a more specific embodiment, Rₑ₁₀ is selected from H and Me; in a more specific embodiment, Rₑ₁₁, Rₑ₁₂ and Rₑ₁₃ are H.

In a more specific embodiment, Rₑ₉ and Rₑ₁₃ are taken together with the carbon atoms to which they are attached to form a 5- to 10-membered carbocyclyl, a 5- to 10-membered heterocyclyl, a C₆₋₁₀ aryl or a 5- to 10-membered heteroaryl, which is optionally substituted with 1, 2, 3 or 4 Rₑ; in another more specific embodiment, Rₑ₉ and Rₑ₁₀ are taken together with the carbon atoms to which they are attached to form a 5- to 10-membered carbocyclyl, a 5- to 10-membered heterocyclyl, a C₆₋₁₀ aryl or a 5- to 10-membered heteroaryl, which is optionally substituted with 1, 2, 3 or 4 Rₑ; in another more specific embodiment, Rₑ₉ and Rₑ₁₃ are taken together with the carbon atoms to which they are attached to form a 5- to 7-membered carbocyclyl, a 5-to 7-membered heterocyclyl, a phenyl or a 5- to 6-membered heteroaryl, which is optionally substituted with 1, 2, 3 or 4 Rₑ; in another more specific embodiment, Rₑ₉ and Rₑ₁₀ are taken together with the carbon atoms to which they are attached to form a 5- to 7-membered carbocyclyl, a 5- to 7-membered heterocyclyl, a phenyl or a 5- to 6-membered heteroaryl, which is optionally substituted with 1, 2, 3 or 4 Rₑ; in another more specific embodiment, Rₑ₉ and Rₑ₁₃ are taken together with the carbon atoms to which they are attached to form a 5- to 7-membered carbocyclyl or a 5- to 7-membered heterocyclyl, which is optionally substituted with 1, 2, 3 or 4 Rₑ; in another more specific embodiment, Rₑ₉ and Rₑ₁₃ are taken together with the carbon atoms to which they are attached to form a 5- to 7-membered heterocyclyl, alternatively form dihydrooxazinyl, which is optionally substituted with 1, 2, 3 or 4 Rₑ; in another more specific embodiment, Rₑ₉ and Rₑ₁₀ are taken together with the carbon atoms to which they are attached to form a 5- to 7-membered heterocyclyl or a 5- to 6-membered heteroaryl, alternatively form dihydrooxazinyl or 5-membered azaaryl, which is optionally substituted with 1, 2, 3 or 4 Rₑ; in another more specific embodiment, Rₑ₉ and Rₑ₁₃ are taken together with the carbon atoms to which they are attached to form 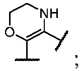 in another more specific embodiment, Rₑ₉ and Rₑ₁₀ are taken together with the carbon atoms to which they are attached to form 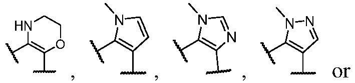 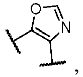 alternatively form

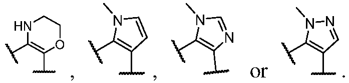

In a more specific embodiment, in another more specific embodiment, Rₑ₉ and Rₑ₁₀ are taken together with the carbon atoms to which they are attached to form a 5- to 7-membered carbocyclyl, a 5- to 7-membered heterocyclyl or a 5- to 6-membered heteroaryl, which is optionally substituted with 1, 2, 3 or 4 Rₑ.

In a more specific embodiment, Rₑ₉ and Rₑ₁₀ are taken together with the carbon atoms to which they are attached to form 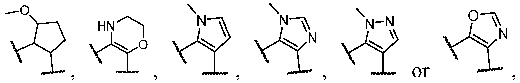 alternatively form

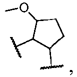

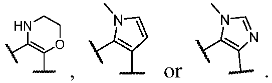

In a more specific embodiment, 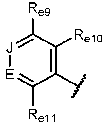 is selected from: 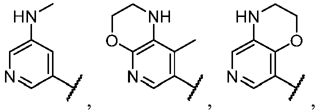 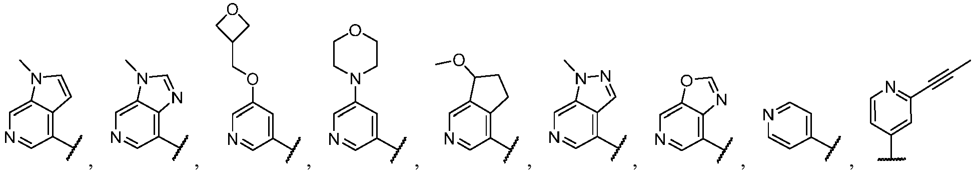 and 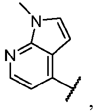 alternatively selected from:

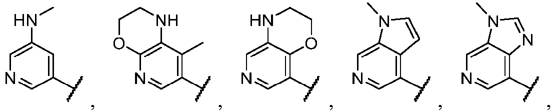

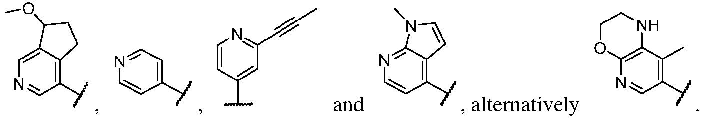

In a more specific embodiment, Y₅ is selected from O, S and NR₅, alternatively O or NR₅, alternatively O; in another more specific embodiment, R₅ is selected from H, C₁₋₈ alkyl and C₁₋₈ haloalkyl, alternatively H, C₁₋₆ alkyl or C₁₋₆ haloalkyl; in another more specific embodiment, R₆ is selected from 3- to 10-membered carbocyclyl and 3- to 10-membered heterocyclyl, alternatively 3- to 7-membered carbocyclyl or 3- to 7-membered heterocyclyl, alternatively 3- to 7-membered heterocyclyl.

In a more specific embodiment, R_{E} and a substituent Rₑ thereon are taken together to form 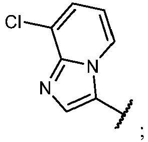 in another more specific embodiment, R_{E} is 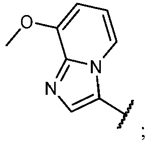 in another more specific embodiment, R_{E} is 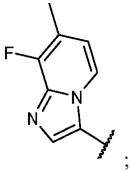 in another more specific embodiment, R_{E} is 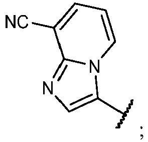 in another more specific embodiment, R_{E} is 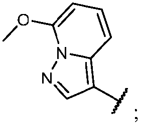 in another more specific embodiment, R_{E} is 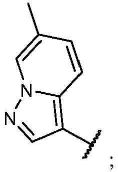 in another more specific embodiment, R_{E} is 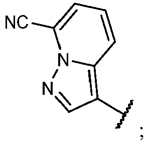 in another more specific embodiment, R_{E} is 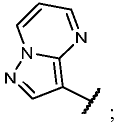 in another more specific embodiment, R_{E} is 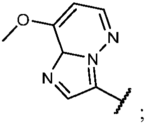 in another more specific embodiment, R_{E} is 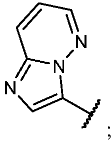 in another more specific embodiment, R_{E} is 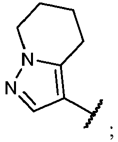 in another more specific embodiment, R_{E} is 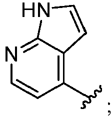 in another more specific embodiment, R_{E} is 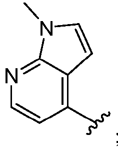 in another more specific embodiment, R_{E} is 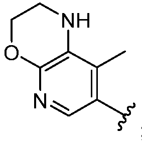 ; in another more specific embodiment, R_{E} is 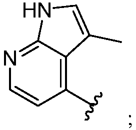 in another more specific embodiment, R_{E} is 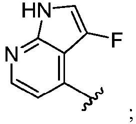 in another more specific embodiment, R_{E} is 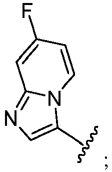 in another more specific embodiment, R_{E} is 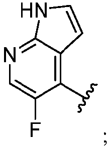 in another more specific embodiment, R_{E} is 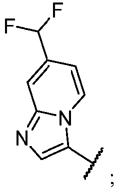 in another more specific embodiment, R_{E} is 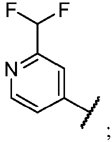 in another more specific embodiment, R_{E} is 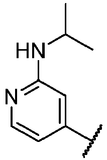 ; in another more specific embodiment, R_{E} is

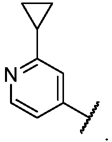

In a more specific embodiment, R_{E} is selected from -L-3- to 10-membered carbocyclyl, -L-3- to 10-membered heterocyclyl, -L-C₆₋₁₀ aryl and -L-5- to 10-membered heteroaryl, alternatively -L-C₆₋₁₀ aryl or -L-5- to 10-membered heteroaryl, alternatively -L-5- to 10-membered heteroaryl, alternatively 5- to 10-membered heteroaryl, each of which is optionally substituted with 1, 2, 3, 4 or 5 Rₑ.

In a more specific embodiment, R_{E} and a substituent Rₑ thereon are taken together to form 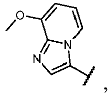 alternatively form 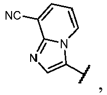 alternatively form 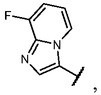 alternatively form 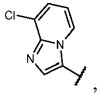 alternatively form 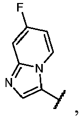 alternatively form 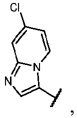 alternatively form 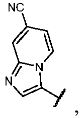 alternatively form 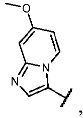 alternatively form 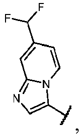 alternatively form 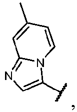 alternatively form 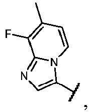 alternatively form 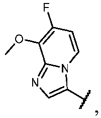 alternatively form 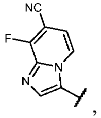 alternatively form 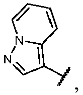 alternatively form 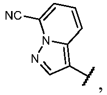 alternatively form 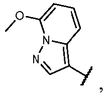 alternatively form 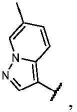 alternatively form 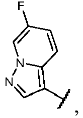 alternatively form 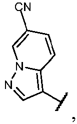 alternatively form 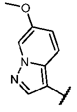 , alternatively form 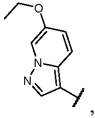 alternatively form 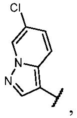 alternatively form 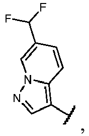 alternatively form 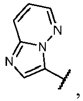 alternatively form 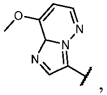 alternatively form 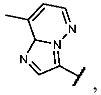 alternatively form 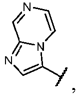 alternatively form 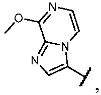 alternatively form 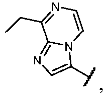 alternatively form 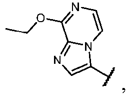 alternatively form 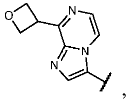 alternatively form 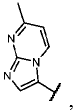 alternatively form 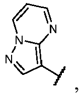 alternatively form 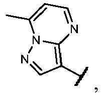 alternatively form 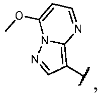 alternatively form 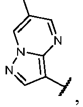 alternatively form 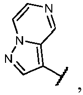 alternatively form 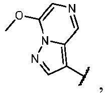 alternatively form 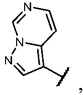 alternatively form 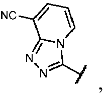 alternatively form 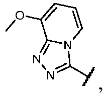 alternatively form 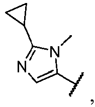 alternatively form 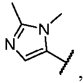 alternatively form 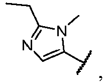 alternatively form 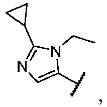 alternatively form 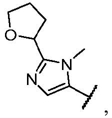 alternatively form 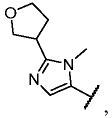 alternatively form 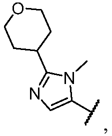 alternatively form 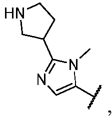 alternatively form 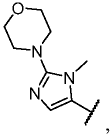 alternatively form 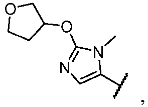 alternatively form 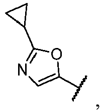 alternatively form 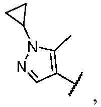 alternatively form 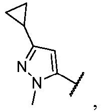 alternatively form 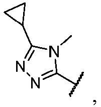 alternatively form 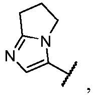 alternatively form 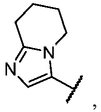 alternatively form 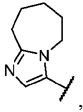 alternatively form 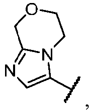 alternatively form 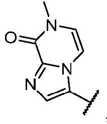 , alternatively form 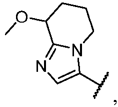 alternatively form 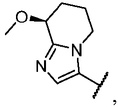 alternatively form 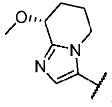 , alternatively form 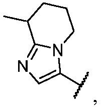 alternatively form 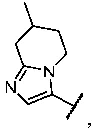 alternatively form 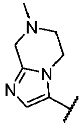 , alternatively form 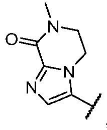 , alternatively form 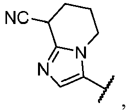 alternatively form 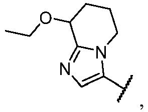 alternatively form 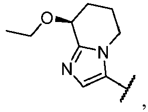 alternatively form 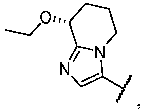 alternatively form 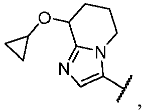 alternatively form 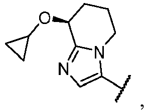 alternatively form 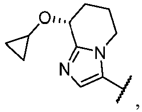 alternatively form 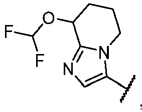 , alternatively form 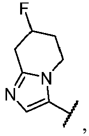 alternatively form 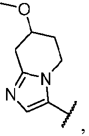 alternatively form 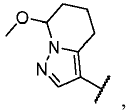 alternatively form 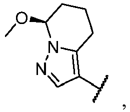 alternatively form 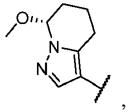 alternatively form 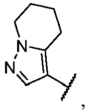 alternatively form 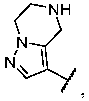 alternatively form 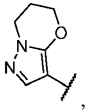 alternatively form 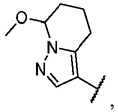 alternatively form 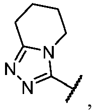 alternatively form 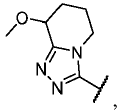 alternatively form 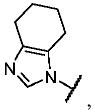 alternatively form 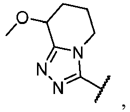 alternatively form 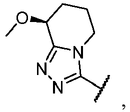 alternatively form 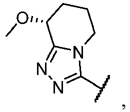 alternatively form 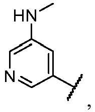 alternatively form 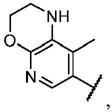 alternatively form 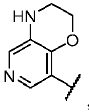 , alternatively form 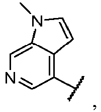 alternatively form 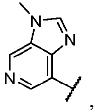 alternatively form 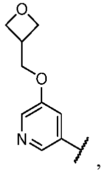 alternatively form 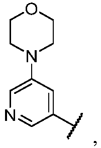 alternatively form 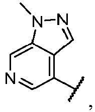 alternatively form 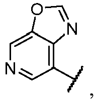 alternatively form 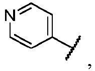 alternatively form 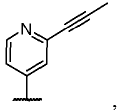 alternatively form 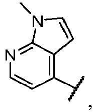 alternatively form 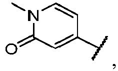 alternatively form 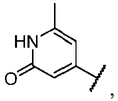 alternatively form 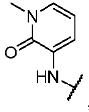 , alternatively form

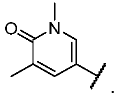

In a more specific embodiment, R_{E} and a substituent Rₑ thereon are taken together to form 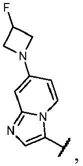 alternatively form 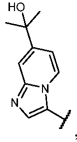 , alternatively form 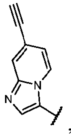 , alternatively form 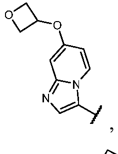 alternatively form 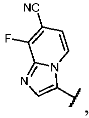 alternatively form 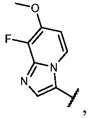 alternatively form 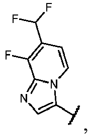 alternatively form 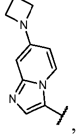 alternatively form 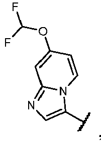 , alternatively form 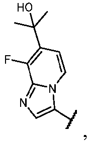 alternatively form 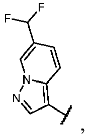 alternatively form 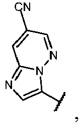 alternatively form 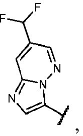 alternatively form 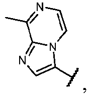 alternatively form 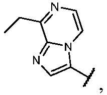 alternatively form 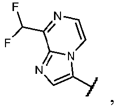 alternatively form 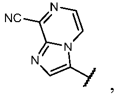 alternatively form 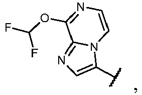 alternatively form 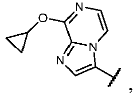 alternatively form 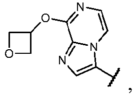 alternatively form 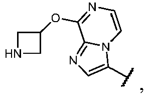 alternatively form 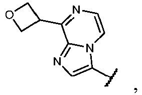 alternatively form 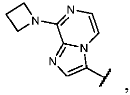 alternatively form 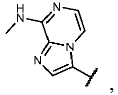 alternatively form 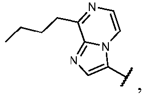 alternatively form 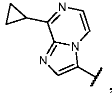 , alternatively form 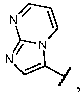 alternatively form 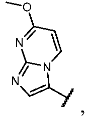 alternatively form 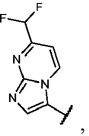 alternatively form 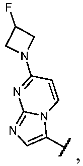 alternatively form 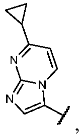 alternatively form 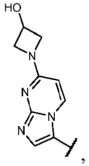 alternatively form 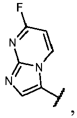 alternatively form 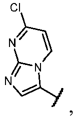 alternatively form 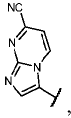 alternatively form 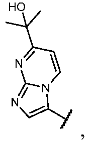 alternatively form 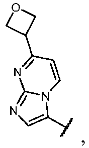 alternatively form 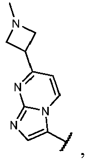 alternatively form 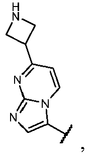 alternatively form 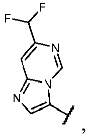 alternatively form 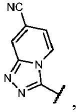 alternatively form 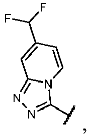 alternatively form 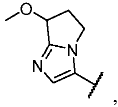 alternatively form 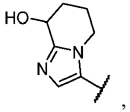 alternatively form 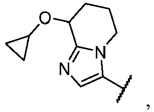 alternatively form 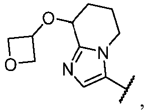 alternatively form 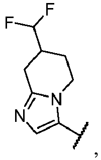 alternatively form 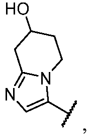 alternatively form 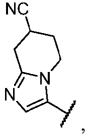 alternatively form 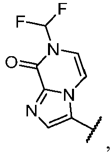 alternatively form 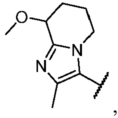 alternatively form 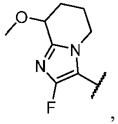 alternatively form 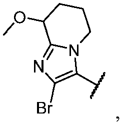 alternatively form 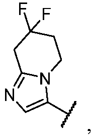 alternatively form 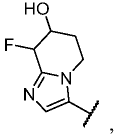 alternatively form 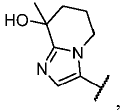 alternatively form 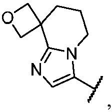 alternatively form 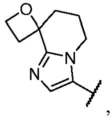 alternatively form 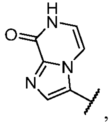 alternatively form 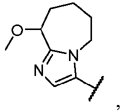 alternatively form 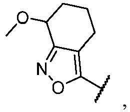 alternatively form 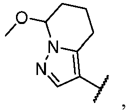 alternatively form 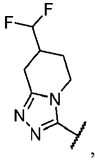 alternatively form

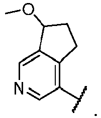

### Rₑ

In one embodiment, Rₑ is H; in another embodiment, Rₑ is D; in another embodiment, Rₑ is halogen; in another embodiment, Rₑ is -CN; in another embodiment, Rₑ is oxo; in another embodiment, Rₑ is C₁₋₈ alkyl; in another embodiment, Rₑ is C₁₋₈ haloalkyl; in another embodiment, Rₑ is C₂₋₈ alkenyl, alternatively C₂₋₆ alkenyl, alternatively C₂₋₄ alkenyl; in one embodiment, Rₑ is C₂₋₈ alkynyl, alternatively C₂₋₆ alkynyl, alternatively C₂₋₄ alkynyl; in another embodiment, Rₑ is -L'-OR'; in another embodiment, Rₑ is -L'-NR'R"; in another embodiment, Rₑ is -NO₂; in another embodiment, Rₑ is -L'-SR'; in another embodiment, Rₑ is -L'-C(O)OR'; in another embodiment, Rₑ is -L'-C(O)NR'R''; in another embodiment, Rₑ is -L'-S(O)₂R'; in another embodiment, Rₑ is -L'-S(O)₂NR'R"; in another embodiment, Rₑ is -L'-OC(O)R'; in another embodiment, Rₑ is -L'-OC(O)NR'R''; in another embodiment, Rₑ is -L'-C₆₋₁₀ aryl; in another embodiment, Rₑ is -L'-3- to 14-membered carbocyclyl; in another embodiment, Rₑ is -L'-3- to 14-membered heterocyclyl; in another embodiment, Rₑ is -L'-5- to 14-membered heteroaryl; in another embodiment, Rₑ is C₁₋₆ alkyl; in another embodiment, Rₑ is C₁₋₆ haloalkyl; in another embodiment, Rₑ is -L'-3- to 10-membered carbocyclyl, alternatively -L'-3- to 7-membered carbocyclyl, alternatively 3- to 7-membered carbocyclyl; in another embodiment, Rₑ is -L'-3- to 10-membered heterocyclyl, alternatively -L'-3- to 7-membered heterocyclyl, alternatively 3- to 7-membered heterocyclyl; in another embodiment, Rₑ is -OR'; in another embodiment, Rₑ is -SR'; in another embodiment, Rₑ is -NR'R"; in another embodiment, Rₑ is F; in another embodiment, Rₑ is Cl; in another embodiment, Rₑ is -CN; in another embodiment, Rₑ is -OCH₃; in another embodiment, Rₑ is Me; in another embodiment, Rₑ is optionally substituted with 1, 2 or 3 independent Rₑₛ; in another embodiment, Rₑ is unsubstituted; in another embodiment, Rₑ is as defined for Rₑ₁; in another embodiment, Rₑ is as defined for Rₑ₂.

In one embodiment, two geminal Rₑ are taken together with the atom to which they are jointly attached to form a 3- to 7-membered carbocyclyl; in another embodiment, two geminal Rₑ are taken together with the atom to which they are jointly attached to form a 3- to 7-membered heterocyclyl; in another embodiment, the ring group formed by the two geminal Rₑ and the atom to which they are jointly attached is optionally substituted with 1, 2 or 3 independent Rₑₛ; in another embodiment, the ring group formed by the two geminal Rₑ and the atom to which they are jointly attached is unsubstituted; in another embodiment, two geminal Rₑ and the atom to which they are jointly attached do not form a ring.

In a more specific embodiment, Rₑ is independently selected from H, D, halogen, -CN, oxo, C₁₋₆ alkyl, C₁₋₆ haloalkyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-3- to 10-membered carbocyclyl and -L'-3- to 10-membered heterocyclyl; in another more specific embodiment, Rₑ is independently selected from H, D, halogen, -CN, -OR', -SR', -NR'R", C₁₋₆ alkyl and C₁₋₆ haloalkyl; in another more specific embodiment, Rₑ is independently selected from H, D, halogen, -CN, -OR', C₁₋₆ alkyl and C₁₋₆ haloalkyl; in another more specific embodiment, Rₑ is independently selected from H, D, Me, F, Cl, -CN and -OCH₃.

In a more specific embodiment, Rₑ is independently selected from H, D, halogen, CN, oxo, C₁₋₆ alkyl, C₁₋₆ haloalkyl, C₂₋₆ alkenyl, C₂₋₆ alkynyl, -L'-OR', -L'-SR', -L'-NR'R", -L'-3- to 7-membered carbocyclyl and -L'-3- to 7-membered heterocyclyl; in another more specific embodiment, Rₑ is independently selected from H, D, halogen, CN, oxo, C₁₋₆ alkyl, C₁₋₆ haloalkyl, C₂₋₆ alkynyl, -OR', -NR'R", 3- to 7-membered carbocyclyl and 3- to 7-membered heterocyclyl; in another more specific embodiment, Rₑ is independently selected from H, D, halogen, CN, oxo, C₁₋₆ alkyl, C₁₋₆ haloalkyl, C₂₋₆ alkynyl, -OR' and -NR'R"; in another more specific embodiment, Rₑ is independently selected from H, D, C₁₋₆ alkyl, C₁₋₆ haloalkyl, alternatively H or Me.

In a more specific embodiment, Rₑ is independently selected from H, D, halogen, CN, oxo, C₁₋₆ alkyl, C₁₋₆ haloalkyl, C₂₋₆ alkenyl, C₂₋₆ alkynyl, -L'-OR', -L'-SR', -L'-NR'R", -L'-3- to 7-membered carbocyclyl and -L'-3- to 7-membered heterocyclyl; in another more specific embodiment, Rₑ is independently selected from H, D, halogen, CN, oxo, C₁₋₆ alkyl, C₁₋₆ haloalkyl, C₂₋₆ alkynyl, -OR', -NR'R", 3- to 7-membered carbocyclyl and 3- to 7-membered heterocyclyl; in another more specific embodiment, Rₑ is independently selected from H, D, halogen, CN, oxo, C₁₋₆ alkyl, C₁₋₆ haloalkyl, C₂₋₆ alkynyl, -OR' and -NR'R".

In a more specific embodiment, Rₑ is independently selected from H, D, halogen, C₁₋₆ alkyl, C₁₋₆ haloalkyl, -OR' and -NR'R"; in another more specific embodiment, Rₑ is independently selected from H, D, C₁₋₆ alkyl, C₁₋₆ haloalkyl and -OR'; in another more specific embodiment, Rₑ is independently selected from Me and OMe.

In a more specific embodiment, two geminal Rₑ are taken together with the atom to which they are jointly attached to form a 3- to 7-membered carbocyclyl or a 3- to 7-membered heterocyclyl.

### Rₑₛ

In one embodiment, Rₑₛ is H; in another embodiment, Rₑₛ is D; in another embodiment, Rₑₛ is halogen; in another embodiment, Rₑₛ is CN; in another embodiment, Rₑₛ is C₁₋₆ alkyl; in another embodiment, Rₑₛ is C₁₋₆ haloalkyl; in another embodiment, Rₑₛ is -L'-OR'; in another embodiment, Rₑₛ is -L'-NR'R".

In a more specific embodiment, Rₑₛ is independently selected from H, D, halogen, CN, C₁₋₆ alkyl, C₁₋₆ haloalkyl, -L'-OR' and -L'-NR'R"; in another more specific embodiment, Rₑₛ is independently selected from H, D, halogen, C₁₋₆ alkyl, C₁₋₆ haloalkyl, -OR' and -NR'R"; in another more specific embodiment, Rₑₛ is independently selected from H, D, halogen, C₁₋₆ alkyl, C₁₋₆ haloalkyl and -OR'; in another more specific embodiment, Rₑₛ is independently selected from H, D, F, Me and OH; in another more specific embodiment, Rₑₛ is independently selected from H, D, halogen (F), C₁₋₆ alkyl and C₁₋₆ haloalkyl; in another more specific embodiment, Rₑₛ is independently selected from H, D, F and Me; in another more specific embodiment, Rₑₛ is halogen (F).

### R_{d1}

In one embodiment, R_{d1} is H; in another embodiment, R_{d1} is D; in another embodiment, R_{d1} is halogen; in another embodiment, R_{d1} is -CN; in another embodiment, R_{d1} is C₁₋₈ alkyl; in another embodiment, R_{d1} is C₂₋₈ alkenyl; in another embodiment, R_{d1} is C₂₋₈ alkynyl; in another embodiment, R_{d1} is C₁₋₈ haloalkyl; in another embodiment, R_{d1} is -L-OR'; in another embodiment, R_{d1} is -L-NR'R"; in another embodiment, R_{d1} is -L-C(O)NR'R"; in another embodiment, R_{d1} is -L-C₆₋₁₀ aryl; in another embodiment, R_{d1} is -L-3- to 14-membered carbocyclyl; in another embodiment, R_{d1} is -L-3- to 14-membered heterocyclyl; in another embodiment, R_{d1} is -L-5- to 14-membered heteroaryl; in another embodiment, R_{d1} is C₁₋₆ alkyl; in another embodiment, R_{d1} is C₁₋₆ haloalkyl; in another embodiment, R_{d1} is -L-3- to 10-membered carbocyclyl; in another embodiment, R_{d1} is -L-3- to 10-membered heterocyclyl.

In a more specific embodiment, R_{d1} is selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl; in another more specific embodiment, R_{d1} is selected from H, D, halogen, C₁₋₆ alkyl and C₁₋₆ haloalkyl; in another more specific embodiment, R_{d1} is H or D.

### Ring A

In one embodiment, ring A is a C₆₋₁₀ aromatic ring; in another embodiment, ring A is a 3- to 14-membered carbocycle; in another embodiment, ring A is a 3- to 14-membered heterocycle; in another embodiment, ring A is a 5- to 14-membered heteroaromatic ring; in another embodiment, ring A is a benzene ring; in another embodiment, ring A is a furan ring; in another embodiment, ring A is a furazan ring; in another embodiment, ring A is an imidazolidine ring; in another embodiment, ring A is an imidazoline ring; in another embodiment, ring A is an imidazole ring; in another embodiment, ring A is an isothiazole ring; in another embodiment, ring A is an isoxazole ring; in another embodiment, ring A is a morpholine ring; in another embodiment, ring A is an oxadiazole ring; in another embodiment, ring A is a 1,2,3-oxadiazole ring; in another embodiment, ring A is a 1,2,4-oxadiazole ring; in another embodiment, ring A is a 1,2,5-oxadiazole ring; in another embodiment, ring A is a 1,3,4-oxadiazole ring; in another embodiment, ring A is an oxazolidine ring; in another embodiment, ring A is an oxazole ring; in another embodiment, ring A is an oxetane ring; in another embodiment, ring A is an azetidine ring; in another embodiment, ring A is a pyrimidine ring; in another embodiment, ring A is a piperazine ring; in another embodiment, ring A is a piperidine ring; in another embodiment, ring A is a pyran ring; in another embodiment, ring A is a pyrazine ring; in another embodiment, ring A is a pyrazolidine ring; in another embodiment, ring A is a pyrazoline ring; in another embodiment, ring A is a pyrazole ring; in another embodiment, ring A is a pyridazine ring; in another embodiment, ring A is a pyridine ring; in another embodiment, ring A is a pyrrolidine ring; in another embodiment, ring A is a pyrroline ring; in another embodiment, ring A is a 2H-pyrrole ring; in another embodiment, ring A is a pyrrole ring; in another embodiment, ring A is a tetrahydrofuran ring; in another embodiment, ring A is a tetrahydropyran ring; in another embodiment, ring A is a thiazole ring; in another embodiment, ring A is a thiophene ring; in another embodiment, ring A is a triazine ring; in another embodiment, ring A is a 1,2,3-triazole ring; in another embodiment, ring A is a 1,2,4-triazole ring; in another embodiment, ring A is a 1,2,5-triazole ring; in another embodiment, ring A is a 1,3,4-triazole ring; in another embodiment, ring A is a xanthene ring; in another embodiment, ring A is a tetrahydroquinoline ring; in another embodiment, ring A is a tetrahydroisoquinoline ring or a hexahydropyrazinoquinoline ring; alternatively a benzene ring; in another embodiment, ring A is a pyridine ring; in another embodiment, ring A is a pyrazole ring; in another embodiment, ring A is an imidazole ring.

In a more specific embodiment, ring A together with R_{d} is 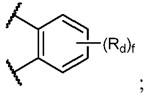 in another more specific embodiment, ring A together with R_{d} is 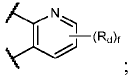 in another more specific embodiment, ring A together with R_{d} is 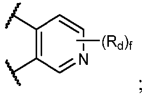 in another more specific embodiment, ring A together with R_{d} is 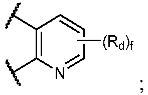 in another more specific embodiment, ring A together with R_{d} is 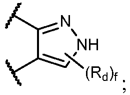 in another more specific embodiment, ring A together with R_{d} is 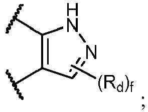 in another more specific embodiment, ring A together with R_{d} is 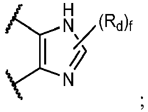 in another more specific embodiment, ring A together with R_{d} is 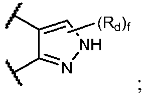 in another more specific embodiment, ring A together with R_{d} is 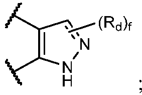 in another more specific embodiment, ring A together with R_{d} is 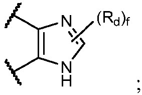 in another more specific embodiment, ring A together with R_{d} is 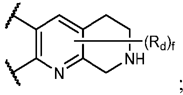 in another more specific embodiment, ring A together with R_{d} is 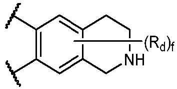 ; in another more specific embodiment, ring A together with R_{d} is 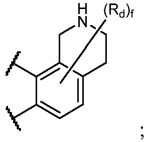 in another more specific embodiment, ring A together with R_{d} is 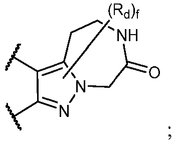 in another more specific embodiment, ring A together with R_{d} is 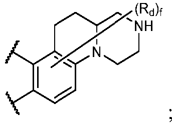 in another more specific embodiment, ring A together with R_{d} is 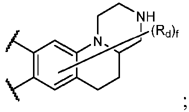 in another more specific embodiment, ring A together with R_{d} is 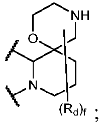 in another more specific embodiment, ring A together with R_{d} is 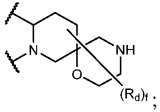 in another more specific embodiment, ring A together with R_{d} is 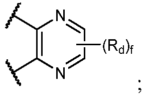 in another more specific embodiment, ring A together with R_{d} is 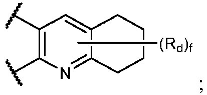 in another more specific embodiment, ring A together with R_{d} is 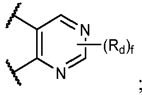 wherein f=0, 1, 2, 3, 4, 5, 6, 7, 8, 9, or 10; in another more specific embodiment, f=0, 1, 2, 3, 4, 5, or 6; in another more specific embodiment, f=0, 1, 2, 3, or 4; in another more specific embodiment, f=0, 1, or 2.

In a more specific embodiment, ring A together with R_{d} is 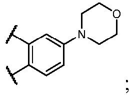 in another more specific embodiment, ring A together with R_{d} is 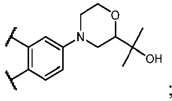 in another more specific embodiment, ring A together with R_{d} is 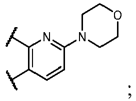 in another more specific embodiment, ring A together with R_{d} is 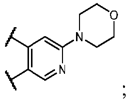 in another more specific embodiment, ring A together with R_{d} is 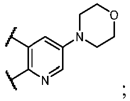 in another more specific embodiment, ring A together with R_{d} is 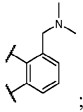 in another more specific embodiment, ring A together with R_{d} is 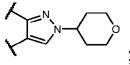 ; in another more specific embodiment, ring A together with R_{d} is 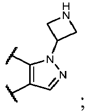 in another more specific embodiment, ring A together with R_{d} is 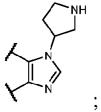 in another more specific embodiment, ring A together with R_{d} is 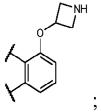 in another more specific embodiment, ring A together with R_{d} is 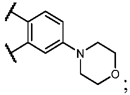 in another more specific embodiment, ring A together with R_{d} is 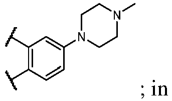 another more specific embodiment, ring A together with R_{d} is 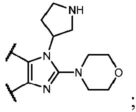 in another more specific embodiment, ring A together with R_{d} is 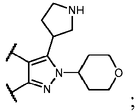 in another more specific embodiment, ring A together with R_{d} is 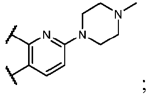 in another more specific embodiment, ring A together with R_{d} is 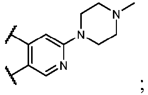 in another more specific embodiment, ring A together with R_{d} is 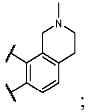 in another more specific embodiment, ring A together with R_{d} is 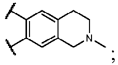 in another more specific embodiment, ring A together with R_{d} is 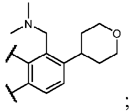 in another more specific embodiment, ring A together with R_{d} is 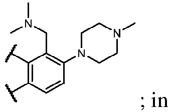 another more specific embodiment, ring A together with R_{d} is 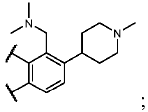 in another more specific embodiment, ring A together with R_{d} is 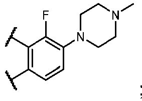 in another more specific embodiment, ring A together with R_{d} is 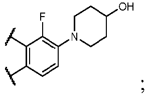 in another more specific embodiment, ring A together with R_{d} is 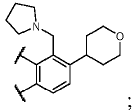 in another more specific embodiment, ring A together with R_{d} is 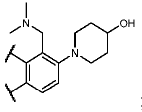 ; in another more specific embodiment, ring A together with R_{d} is 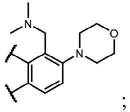 in another more specific embodiment, ring A together with R_{d}; is 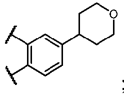 in another more specific embodiment, ring A together with R_{d} is 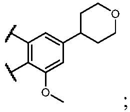 in another more specific embodiment, ring A together with R_{d} is 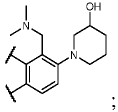 in another more specific embodiment, ring A together with R_{d} is 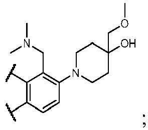 in another more specific embodiment, ring A together with R_{d} is 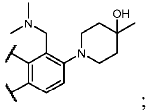 in another more specific embodiment, ring A together with R_{d} is 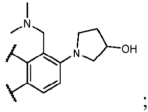 in another more specific embodiment, ring A together with R_{d} is 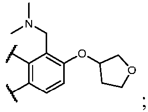 in another more specific embodiment, ring A together with R_{d} is 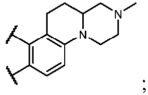 in another more specific embodiment, ring A together with R_{d} is 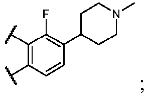 in another more specific embodiment, ring A together with R_{d} is 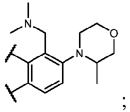 in another more specific embodiment, ring A together with R_{d} is 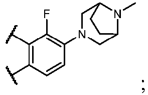 in another more specific embodiment, ring A together with R_{d} is 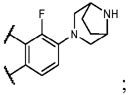 in another more specific embodiment, ring A together with R_{d} is 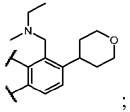 in another more specific embodiment, ring A together with R_{d} is 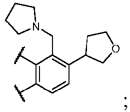 in another more specific embodiment, ring A together with R_{d} is 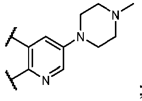 ; in another more specific embodiment, ring A together with R_{d} is 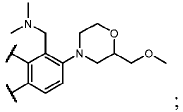 in another more specific embodiment, ring A together with R_{d} is 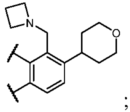 in another more specific embodiment, ring A together with R_{d} is 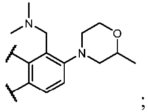 in another more specific embodiment, ring A together with R_{d} is 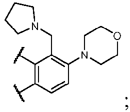 in another more specific embodiment, ring A together with R_{d} is 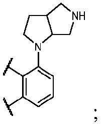 in another more specific embodiment, ring A together with R_{d} is 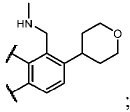 in another more specific embodiment, ring A together with R_{d} is 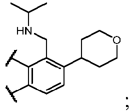 in another more specific embodiment, ring A together with R_{d} is 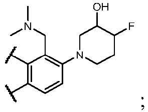 in another more specific embodiment, ring A together with R_{d} is 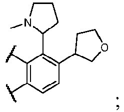 in another more specific embodiment, ring A together with R_{d} is 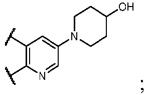 in another more specific embodiment, ring A together with R_{d} is 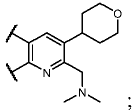 in another more specific embodiment, ring A together with R_{d} is 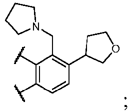 in another more specific embodiment, ring A together with R_{d} is 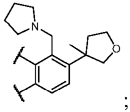 in another more specific embodiment, ring A together with R_{d} is 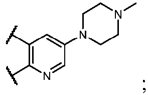 in another more specific embodiment, ring A together with R_{d} is 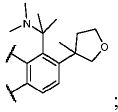 in another more specific embodiment, ring A together with R_{d} is 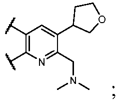 in another more specific embodiment, ring A together with R_{d} is 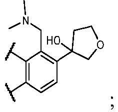 in another more specific embodiment, ring A together with R_{d} is 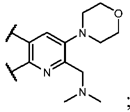 in another more specific embodiment, ring A together with R_{d} is 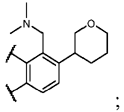 in another more specific embodiment, ring A together with R_{d} is 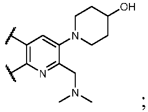 in another more specific embodiment, ring A together with R_{d} is 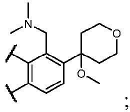 in another more specific embodiment, ring A together with R_{d} is 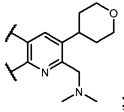 ; in another more specific embodiment, ring A together with R_{d} is 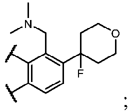 in another more specific embodiment, ring A together with R_{d} is 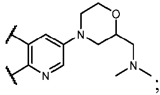 in another more specific embodiment, ring A together with R_{d} is 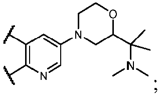 in another more specific embodiment, ring A together with R_{d} is 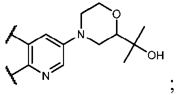 in another more specific embodiment, ring A together with R_{d} is 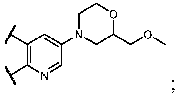 in another more specific embodiment, ring A together with R_{d} is 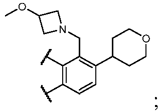 in another more specific embodiment, ring A together with R_{d} is 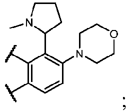 in another more specific embodiment, ring A together with R_{d} is 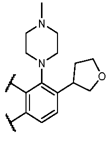 ; in another more specific embodiment, ring A together with R_{d} is 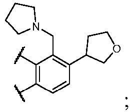 in another more specific embodiment, ring A together with R_{d} is 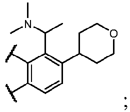 in another more specific embodiment, ring A together with R_{d} is 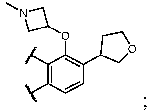 in another more specific embodiment, ring A together with R_{d} is 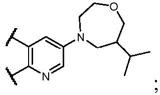 in another more specific embodiment, ring A together with R_{d} is 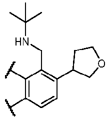 ; in another more specific embodiment, ring A together with R_{d} is 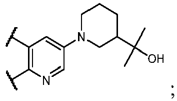 in another more specific embodiment, ring A together with R_{d} is 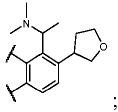 in another more specific embodiment, ring A together with R_{d} is 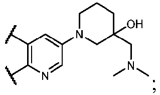 in another more specific embodiment, ring A together with R_{d} is 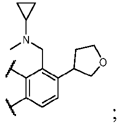 in another more specific embodiment, ring A together with R_{d} is 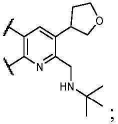 in another more specific embodiment, ring A together with R_{d} is 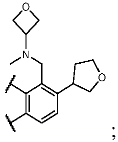 in another more specific embodiment, ring A together with R_{d} is 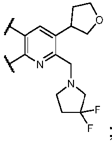 in another more specific embodiment, ring A together with R_{d} is 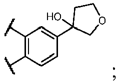 in another more specific embodiment, ring A together with R_{d} is 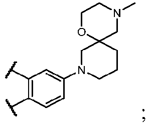 in another more specific embodiment, ring A together with R_{d} is 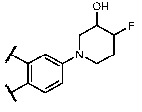 ; in another more specific embodiment, ring A together with R_{d} is 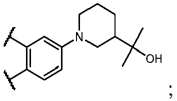 in another more specific embodiment, ring A together with R_{d} is 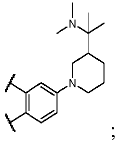 in another more specific embodiment, ring A together with R_{d} is 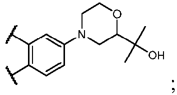 in another more specific embodiment, ring A together with R_{d} is 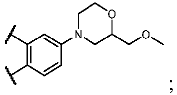 in another more specific embodiment, ring A together with R_{d} is 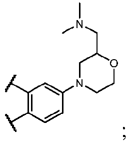 in another more specific embodiment, ring A together with R_{d} is 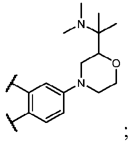 in another more specific embodiment, ring A together with R_{d} is 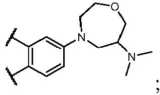 in another more specific embodiment, ring A together with R_{d} is 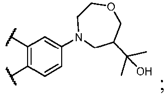 in another more specific embodiment, ring A together with R_{d} is 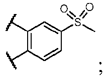 in another more specific embodiment, ring A together with R_{d} is 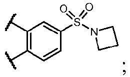 in another more specific embodiment, ring A together with R_{d} is 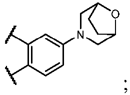 in another more specific embodiment, ring A together with R_{d} is 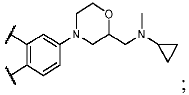 in another more specific embodiment, ring A together with R_{d} is 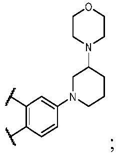 in another more specific embodiment, ring A together with R_{d} is 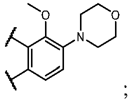 in another more specific embodiment, ring A together with R_{d} is 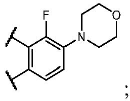 in another more specific embodiment, ring A together with R_{d} is 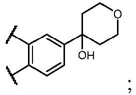 in another more specific embodiment, ring A together with R_{d} is 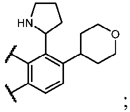 in another more specific embodiment, ring A together with R_{d} is 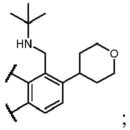 in another more specific embodiment, ring A together with R_{d} is 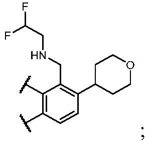 in another more specific embodiment, ring A together with R_{d} is 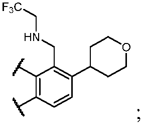 in another more specific embodiment, ring A together with R_{d} is 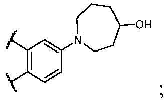 in another more specific embodiment, ring A together with R_{d} is 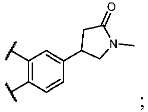 in another more specific embodiment, ring A together with R_{d} is 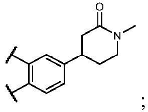 in another more specific embodiment, ring A together with R_{d} is 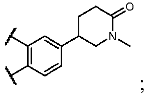 in another more specific embodiment, ring A together with R_{d} is

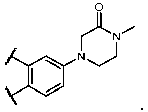

In a more specific embodiment, ring A together with R_{d} is 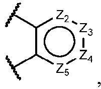 alternatively 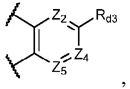 alternatively 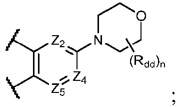 wherein Z₂ is selected from CR_{d2} and N, alternatively CR_{d2}, alternatively N; Z₃ is selected from CR_{d3} and N, alternatively CR_{d3}, alternatively N; Z₄ is selected from CR_{d4} and N, alternatively CR_{d4}, alternatively N; Z₅ is selected from covalent bond, CR_{d5} and N, alternatively CR_{d5} or N, alternatively CR_{d5}, alternatively N.

In a more specific embodiment, one of R_{d3} and R_{d4} is -NR'_{d6}C(O)R_{d6}, -L'-3- to 7-membered carbocyclyl or -L'-3- to 7-membered heterocyclyl, and the other one, R_{d2} and R_{d5} are independently selected from H, D, halogen, CN, C₁₋₆ alkyl, C₁₋₆ haloalkyl, -L'-OR', -L'-SR', -L'-NR'R'', -L'-3- to 7-membered carbocyclyl and -L'-3- to 7-membered heterocyclyl; in another more specific embodiment, R_{d2}, R_{d3}, R_{d4} and R_{d5} are optionally substituted with 1, 2, 3 or 4 R_{dd}; in another more specific embodiment, one of R_{d3} and R_{d4} is -NR'_{d6}C(O)R_{d6} or -L'-3- to 7-membered heterocyclyl, alternatively -L'-3- to 7-membered heterocyclyl, and the other one, R_{d2} and R_{d5} are independently selected from H, D, halogen, CN, C₁₋₆ alkyl and C₁₋₆ haloalkyl; in another more specific embodiment, R_{d2}, R_{d4} and R_{d5} are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl; in another more specific embodiment, R_{d2}, R_{d4} and R_{d5} are independently selected from H, D, halogen, CN, C₁₋₆ alkyl and C₁₋₆ haloalkyl; in another more specific embodiment, R_{d2}, R_{d4} and R_{d5} are independently selected from H, D, halogen, C₁₋₃ alkyl and C₁₋₃ haloalkyl; in another more specific embodiment, R_{d2} and R_{d5} are independently selected from H, D and halogen; in another more specific embodiment, R_{d5} is H or D; in another more specific embodiment, R_{d2}, R_{d4} and R_{d5} are independently selected from H, F, Me and CHF₂; in another more specific embodiment, R_{d2} and R_{d5} are independently selected from H and F; in another more specific embodiment, R_{d5} is H.

In a more specific embodiment, one of R_{d3} and R_{d4} is -NR'_{d6}C(O)R_{d6}, -C(O)NR_{d7}R'_{d7}, -L'-3- to 7-membered carbocyclyl, or -L'-3- to 7-membered heterocyclyl, alternatively -NR'_{d6}C(O)R_{d6}, -C(O)NR_{d7}R'_{d7} or -L'-3- to 7-membered heterocyclyl, and the other one, R_{d2} and R_{d5} are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, alternatively selected from H, D, halogen, CN, C₁₋₆ alkyl, C₁₋₆ haloalkyl, OR' and NR'R".

In one embodiment, R_{d2}/R_{d4}/R_{d5} is H; in another embodiment, R_{d2}/R_{d4}/R_{d5} is D; in another embodiment, R_{d2}/R_{d4}/R_{d5} is halogen, such as F; in another embodiment, R_{d2}/R_{d4}/R_{d5} is CN; in another embodiment, R_{d2}/R_{d4}/R_{d5} is C₁₋₆ alkyl; in another embodiment, R_{d2}/R_{d4}/R_{d5} is C₁₋₆ haloalkyl, such as CHF₂; in another embodiment, R_{d2}/R_{d4}/R_{d5} is -L'-OR', alternatively OR', such as OCH₃; in another embodiment, R_{d2}/R_{d4}/R_{d5} is -L'-SR', alternatively SR'; in another embodiment, R_{d2}/R_{d4}/R_{d5} is -L'-NR'R", alternatively NR'R"; in another embodiment, R_{d2}/R_{d4}/R_{d5} is -L'-3- to 7-membered carbocyclyl, alternatively 3- to 7-membered carbocyclyl; in another embodiment, R_{d2}/R_{d4}/R_{d5} is -L'-3- to 7-membered heterocyclyl, alternatively 3- to 7-membered heterocyclyl.

In a more specific embodiment, R_{d2}, R_{d4} and R_{d5} are independently selected from H, D, halogen, CN, C₁₋₆ alkyl, C₁₋₆ haloalkyl, -OR' and -NR'R"; in another more specific embodiment, R_{d2}, R_{d4} and R_{d5} are independently selected from H, D, halogen, CN, C₁₋₄ alkyl, C₁₋₄ haloalkyl and -OR'; in another more specific embodiment, R_{d2}, R_{d4} and R_{d5} are independently selected from H and D; in another more specific embodiment, R_{d2}, R_{d4} and R_{d5} are independently selected from H, F, CN, Me, CHF₂ and OCH₃; in another more specific embodiment, R_{d2}, R_{d4} and R_{d5} are H.

In a more specific embodiment, R_{d2} and R_{d5} are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl; in another more specific embodiment, R_{d2} and R_{d5} are independently selected from H, D, halogen, CN, C₁₋₆ alkyl, C₁₋₆ haloalkyl, OR' and NR'R"; in another more specific embodiment, R_{d2} and R_{d5} are independently selected from H, D, halogen, C₁₋₆ alkyl and C₁₋₆ haloalkyl; in another more specific embodiment, R_{d2} and R_{d5} are independently selected from H, D and halogen; in another more specific embodiment, R_{d2} and R_{d5} are independently selected from H and F.

In one embodiment, R_{d3}/R_{d4} is -NR'_{d6}C(O)R_{d6}; in another embodiment, R_{d3}/R_{d4} is -C(O)NR_{d7}R'_{d7}; in another embodiment, R_{d3}/R_{d4} is -L'-3- to 7-membered carbocyclyl; in another embodiment, R_{d3}/R_{d4} is -L'-3- to 7-membered heterocyclyl; in another embodiment, R_{d3}/R_{d4} is 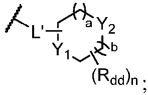 in another embodiment, R_{d3}/R_{d4} is 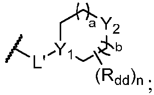 in another embodiment, R_{d3}/R_{d4} is

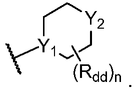

In one embodiment, R_{d7} and R'_{d7} are independently selected from H, C₁₋₆ alkyl and C₁₋₆ haloalkyl, alternatively independently H or C₁₋₆ alkyl, alternatively Me.

In a more specific embodiment, R_{d3} is selected from 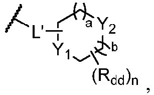 -NR'_{d6}C(O)R_{d6} and -C(O)NR_{d7}R'_{d7}; in another more specific embodiment, R_{d3} is selected from 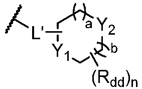 and -NR'_{d6}C(O)R_{d6}, alternatively 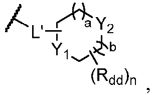 alternatively -NR'_{d6}C(O)R_{d6}; in another more specific embodiment, 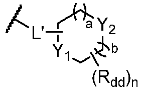 is 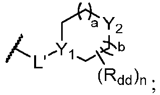 in another more specific embodiment,

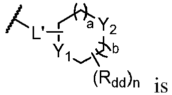

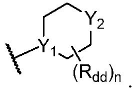

In one embodiment, a/b is 0; in another embodiment, a/b is 1; in another embodiment, a/b is 2; in another embodiment, a/b is 3.

In a more specific embodiment, a and b are independently 0, 1, 2, or 3, alternatively independently 0, 1, or 2, alternatively, a is 1 or 2, and b is 1, alternatively, a and b are 1; in another more specific embodiment, a+b≤5, alternatively a+b≤3; in another more specific embodiment, n=0, 1, 2, 3, or 4, alternatively n=0, 1, or 2. In a more specific embodiment, a is 0 or 1; in another more specific embodiment, b is 0 or 1.

In a more specific embodiment, Y₁ and Y₂ are independently selected from CR_{dd}R_{dd}, O, S and NR'_{dd}, or when Y₁ is connected to L', Y₁ is CR_{dd} or N; in another more specific embodiment, Y₁ is CR_{dd}R_{dd} or NR'_{dd}; or when Y₁ is connected to L', Y₁ is CH, CD or N; in another more specific embodiment, Y₂ is CR_{dd}R_{dd}, O or NR'_{dd}; in another more specific embodiment, at least one of Y₁ and Y₂ is a heteroatom group; in another more specific embodiment, Y₁ is NR'_{dd}, alternatively NH; or when Y₁ is connected to L', Y₁ is CH, CD or N; in another more specific embodiment, Y₂ is CR_{dd}R_{dd}, O or NR'_{dd}; in another more specific embodiment, Y₁ is NH, CH or N, alternatively CH or N, alternatively N; in another more specific embodiment, Y₂ is CR_{dd}R_{dd}, O or NR'_{dd}, alternatively O.

In a more specific embodiment, Y₂ is selected from CR_{dd}R_{dd}, O, S, S(O), NR'_{dd} and S(O)₂; in another more specific embodiment, Y₂ is selected from CR_{dd}R_{dd}, O, NR'_{dd} and S(O)₂; in another more specific embodiment, Y₂ is selected from CR_{dd}R_{dd} and O.

In a more specific embodiment, when Y₁ is connected to L', Y₁ is selected from CH, C(OH) and N, alternatively selected from CH and N, alternatively N.

In a more specific embodiment, ring A together with R_{d} is 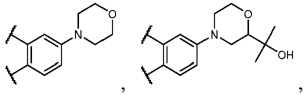 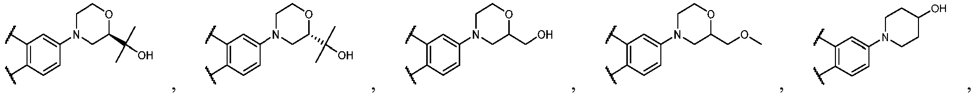 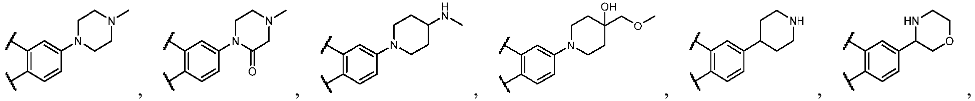 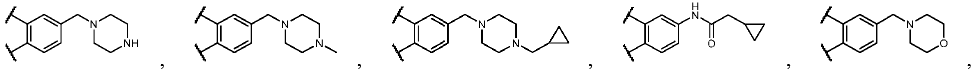 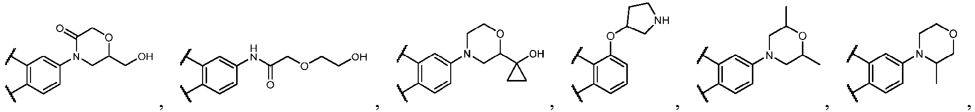 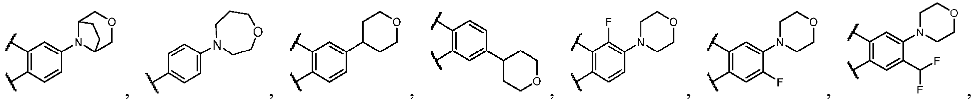 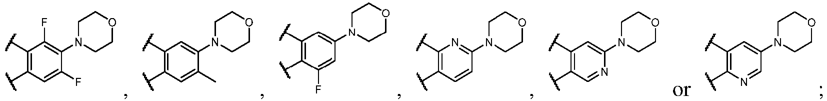 in another more specific embodiment, ring A together with R_{d} is 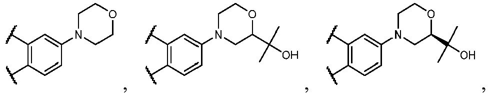 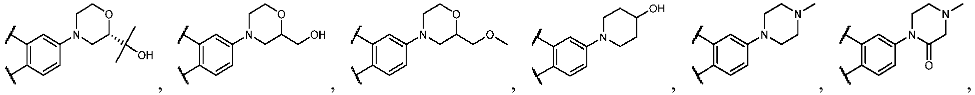 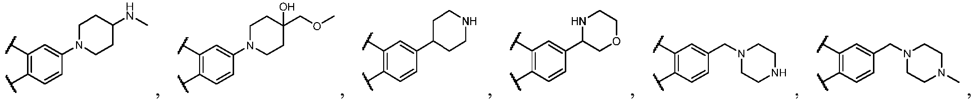 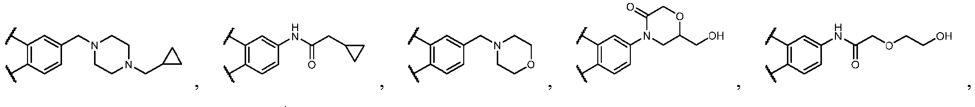 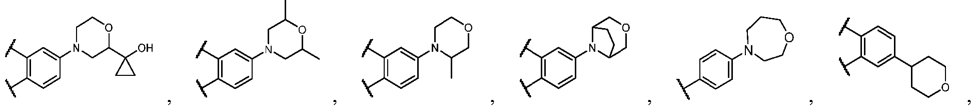 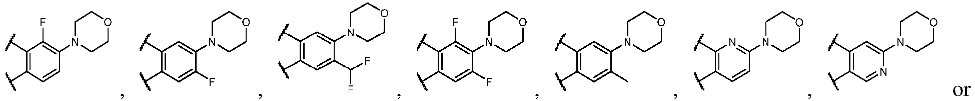 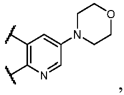 alternatively not 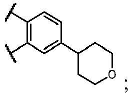 in another more specific embodiment, ring A together with R_{d} is

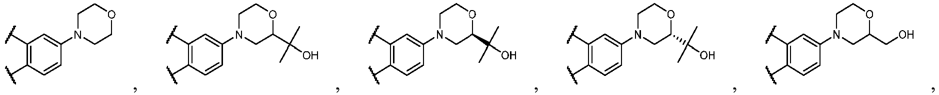

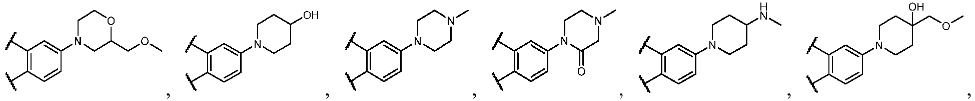

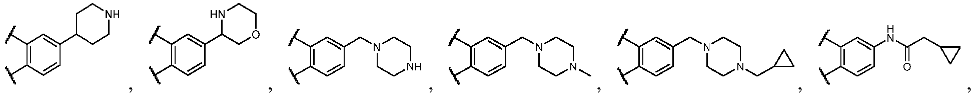

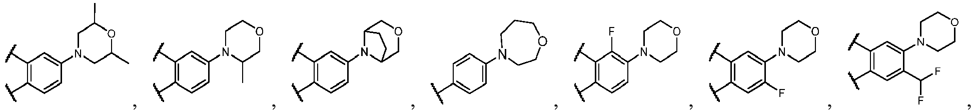

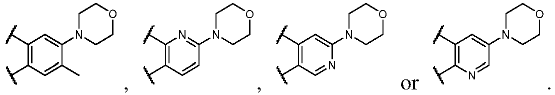

In a more specific embodiment, ring A together with R_{d} is 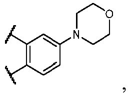 alternatively 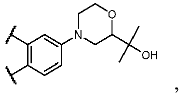 alternatively 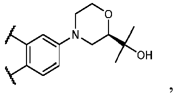 alternatively 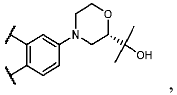 alternatively 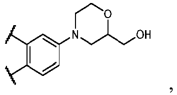 alternatively 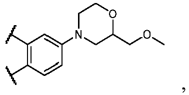 alternatively 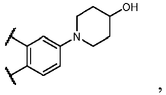 alternatively 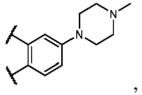 alternatively 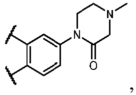 alternatively 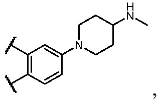 alternatively 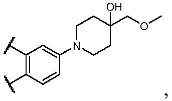 alternatively 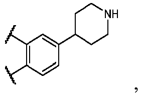 alternatively 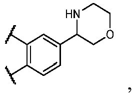 alternatively 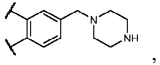 alternatively 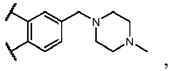 alternatively 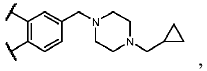 alternatively 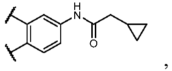 alternatively 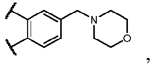 alternatively 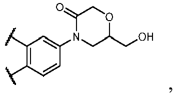 alternatively 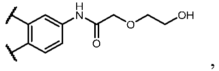 alternatively 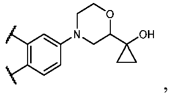 alternatively 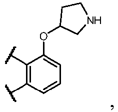 alternatively 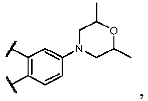 alternatively 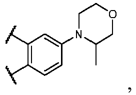 alternatively 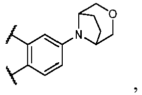 alternatively 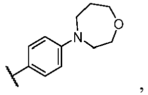 alternatively 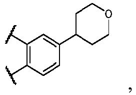 alternatively 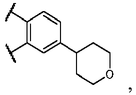 alternatively 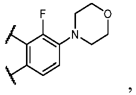 alternatively 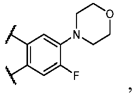 alternatively 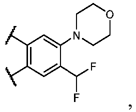 alternatively 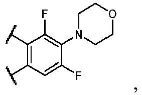 alternatively 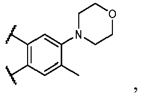 alternatively 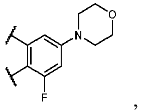 alternatively 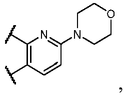 alternatively 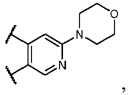 alternatively

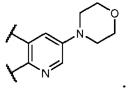

In one embodiment, R_{d3} is 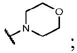 in another embodiment, R_{d3} is 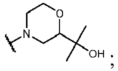 in another embodiment, R_{d3} is 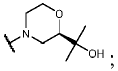 in another embodiment, R_{d3} is 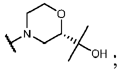 in another embodiment, R_{d3} is 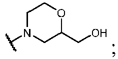 in another embodiment, R_{d3} is 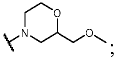 in another embodiment, R_{d3} is 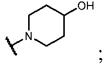 in another embodiment, R_{d3} is 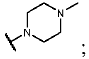 in another embodiment, R_{d3} is 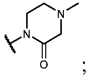 in another embodiment, R_{d3} is 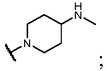 in another embodiment, R_{d3} is 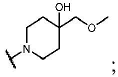 in another embodiment, R_{d3} is 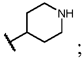 in another embodiment, R_{d3} is 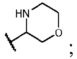 in another embodiment, R_{d3} is 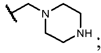 in another embodiment, R_{d3} is 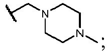 in another embodiment, R_{d3} is 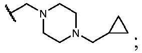 in another embodiment, R_{d3} is 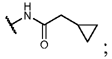 in another embodiment, R_{d3} is 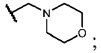 in another embodiment, R_{d3} is 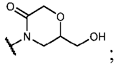 in another embodiment, R_{d3} is 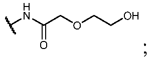 in another embodiment, R_{d3} is 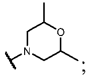 in another embodiment, R_{d3} is 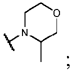 in another embodiment, R_{d3} is 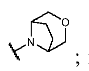 ; in another embodiment, R_{d3} is 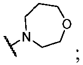 in another embodiment, R_{d3} is 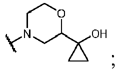 in another embodiment, R_{d3} is 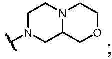 in another embodiment, R_{d3} is 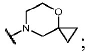 in another embodiment, R_{d3} is 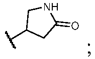 in another embodiment, R_{d3} is 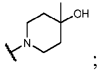 in another embodiment, R_{d3} is 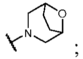 in another embodiment, R_{d3} is 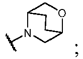 in another embodiment, R_{d3} is 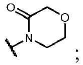 in another embodiment, R_{d3} is 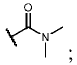 in another embodiment, R_{d3} is 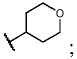 in another embodiment, R_{d3} is 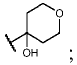 in another embodiment, R_{d3} is 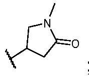 in another embodiment, R_{d3} is 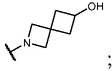 in another embodiment, R_{d3} is 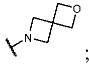 in another embodiment, R_{d3} is 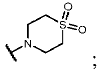 in another embodiment, R_{d3} is 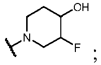 in another embodiment, R_{d3} is

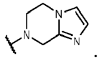

### R_{d}

In one embodiment, R_{d} is H; in another embodiment, R_{d} is D; in another embodiment, R_{d} is halogen; in another embodiment, R_{d} is -CN; in another embodiment, R_{d} is -L'-S(O)₀₋₂R'; in another embodiment, R_{d} is -L'-S(O)₂NR'R"; in another embodiment, R_{d} is -L'-S(O)NR'R"; in another embodiment, R_{d} is -L'-OR'; in another embodiment, R_{d} is -L'-NR'R"; in another embodiment, R_{d} is -L'-C(O)OR'; in another embodiment, R_{d} is -L'-C(O)NR'R''; in another embodiment, R_{d} is C₁₋₈ alkyl; in another embodiment, R_{d} is C₂₋₈ alkenyl; in another embodiment, R_{d} is C₂₋₈ alkynyl; in another embodiment, R_{d} is C₁₋₈ haloalkyl; in another embodiment, R_{d} is -L' -C₆₋₁₀ aryl; in another embodiment, R_{d} is -L'-3- to 14-membered carbocyclyl; in another embodiment, R_{d} is -L'-3- to 14-membered heterocyclyl; in another embodiment, R_{d} is -L'-5- to 14-membered heteroaryl; in another embodiment, R_{d} is C₁₋₆ alkyl; in another embodiment, R_{d} is C₁₋₆ haloalkyl; in another embodiment, R_{d} is -L'-3- to 10-membered carbocyclyl or -L'-3- to 10-membered heterocyclyl; in another embodiment, two adjacent R_{d} are taken together with the atoms to which they are attached to form a 3- to 7-membered monocyclic heterocyclyl; in another embodiment, two adjacent R_{d} are taken together with the atoms to which they are attached to form a 6- to 10-membered bridged bicyclic heterocyclyl; in another embodiment, two adjacent R_{d} are taken together with the atoms to which they are attached to form a 6- to 10-membered spiro bicyclic heterocyclyl; in another embodiment, two adjacent R_{d} are taken together with the atoms to which they are attached to form a 6- to 10-membered fused bicyclic heterocyclyl; in another embodiment, R_{d} is optionally substituted with 1, 2, 3, 4, 5, 6, 7 or 8 R_{dd}; in another embodiment, R_{d} is unsubstituted; in another embodiment, R_{d} is as defined for R_{d2}; in another embodiment, R_{d} is as defined for Rₐ₃; in another embodiment, R_{d} is as defined for R_{d4}; in another embodiment, R_{d} is as defined for R_{d5}.

In a more specific embodiment, R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or, R_{d} is independently selected from C₁₋₆ alkyl, C₁₋₆ haloalkyl, -L'-3- to 10-membered carbocyclyl and -L'-3- to 10-membered heterocyclyl, each of which is optionally substituted with 1, 2, 3, 4, 5, 6, 7 or 8 R_{dd}.

In another more specific embodiment, R_{d} is independently selected from H, D, halogen, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, and the C₁₋₆ alkyl, C₁₋₆ haloalkyl, 3- to 7-membered carbocyclyl or 3- to 7-membered heterocyclyl is optionally substituted with 1, 2, 3, 4, 5, 6, 7 or 8 R_{dd};
in another more specific embodiment, R_{d} is independently selected from H, D, C₁₋₆ alkyl, C₁₋₆ haloalkyl, -L'-NR'R" and -L'-4- to 6-membered heterocyclyl, and the C₁₋₆ alkyl, C₁₋₆ haloalkyl or 4- to 6-membered heterocyclyl is optionally substituted with 1 or 2 R_{dd};
in another more specific embodiment, R_{d} is 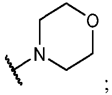 in another more specific embodiment, R_{d} is 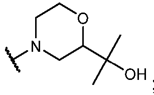 ; in another more specific embodiment, R_{d} is 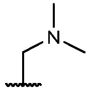 or 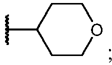 in another more specific embodiment, R_{d} is 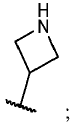 in another more specific embodiment, R_{d} is 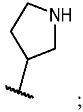 in another more specific embodiment, R_{d} is 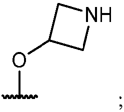 in another more specific embodiment, R_{d} is 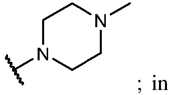 another more specific embodiment, R_{d} is F; in another more specific embodiment, R_{d} is -OH; in another more specific embodiment, R_{d} is -OCH₃; in another more specific embodiment, R_{d} is 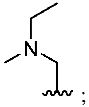 in another more specific embodiment, R_{d} is 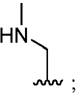 in another more specific embodiment, R_{d} is 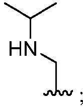 in another more specific embodiment, R_{d} is 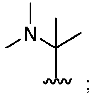 ; in another more specific embodiment, R_{d} is 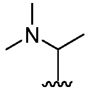 ; in another more specific embodiment, R_{d} is 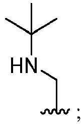 in another more specific embodiment, R_{d} is 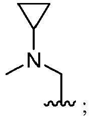 in another more specific embodiment, R_{d} is 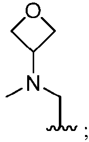 in another more specific embodiment, R_{d} is 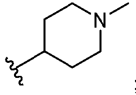 , in another more specific embodiment, R_{d} is 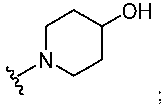 in another more specific embodiment, R_{d} is 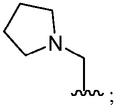 in another more specific embodiment, R_{d} is 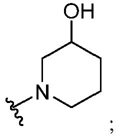 in another more specific embodiment, R_{d} is 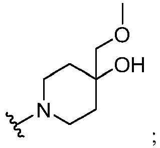 in another more specific embodiment, R_{d} is 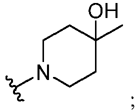 in another more specific embodiment, R_{d} is 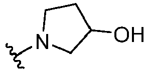 ; in another more specific embodiment, R_{d} is 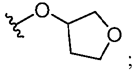 in another more specific embodiment, R_{d} is 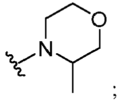 in another more specific embodiment, R_{d} is 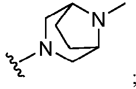 ; in another more specific embodiment, R_{d} is 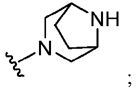 in another more specific embodiment, R_{d} is 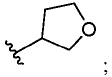 in another more specific embodiment, R_{d} is 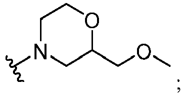 in another more specific embodiment, R_{d} is 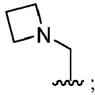 in another more specific embodiment, R_{d} is 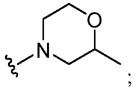 in another more specific embodiment, R_{d} is 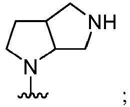 in another more specific embodiment, R_{d} is 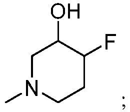 in another more specific embodiment, R_{d} is 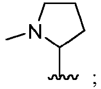 in another more specific embodiment, R_{d} is 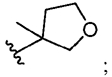 in another more specific embodiment, R_{d} is 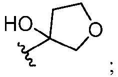 in another more specific embodiment, R_{d} is 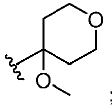 in another more specific embodiment, R_{d} is 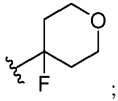 in another more specific embodiment, R_{d} is 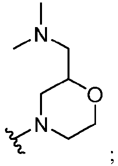 in another more specific embodiment, R_{d} is 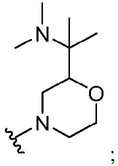 in another more specific embodiment, R_{d} is 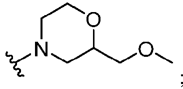 ; in another more specific embodiment, R_{d} is 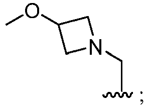 in another more specific embodiment, R_{d} is 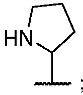 ; in another more specific embodiment, R_{d} is 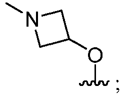 in another more specific embodiment, R_{d} is 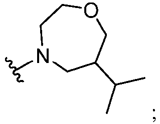 in another more specific embodiment, R_{d} is 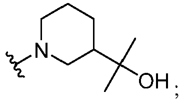 in another more specific embodiment, R_{d} is 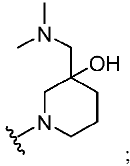 in another more specific embodiment, R_{d} is 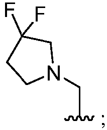 in another more specific embodiment, R_{d} is 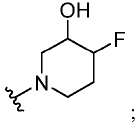 in another more specific embodiment, R_{d} is 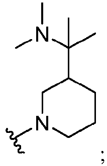 in another more specific embodiment, R_{d} is 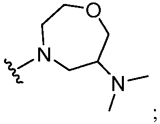 in another more specific embodiment, R_{d} is 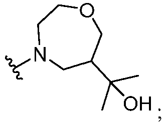 in another more specific embodiment, R_{d} is 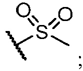 in another more specific embodiment, R_{d} is 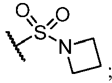 in another more specific embodiment, R_{d} is 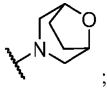 in another more specific embodiment, R_{d} is 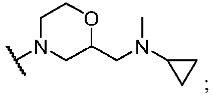 in another more specific embodiment, R_{d} is 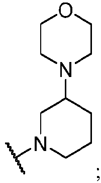 in another more specific embodiment, R_{d} is 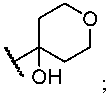 in another more specific embodiment, R_{d} is 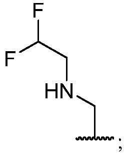 in another more specific embodiment, R_{d} is 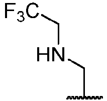 ; in another more specific embodiment, R_{d} is 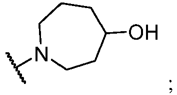 in another more specific embodiment, R_{d} is 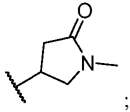 in another more specific embodiment, R_{d} is 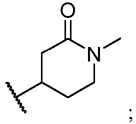 in another more specific embodiment, R_{d} is

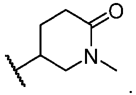

### R_{dd}

In one embodiment, R_{dd} is H; in another embodiment, R_{dd} is D; in another embodiment, R_{dd} is halogen, such as F; in another embodiment, R_{dd} is -CN; in another embodiment, R_{dd} is -NO₂; in another embodiment, R_{dd} is oxo; in another embodiment, R_{dd} is C₁₋₈ alkyl; in another embodiment, R_{dd} is C₂₋₈ alkenyl; in another embodiment, R_{dd} is C₂₋₈ alkynyl; in another embodiment, R_{dd} is C₁₋₈ haloalkyl; in another embodiment, R_{dd} is -L''-OR', alternatively OR', such as OH; in another embodiment, R_{dd} is -L"-NR'R", alternatively NR'R"; in another embodiment, R_{dd} is -L"-C(O)OR'; in another embodiment, R_{dd} is -L"-C(O)NR'R"; in another embodiment, R_{dd} is -L"-S(O)₀₋₂R'; in another embodiment, R_{dd} is -L"-S(O)₂NR'R"; in another embodiment, R_{dd} is -L''-S(O)NR'R''; in another embodiment, R_{dd} is -L"-OC(O)R'; in another embodiment, R_{dd} is -L"-OC(O)NR'R"; in another embodiment, R_{dd} is -L"-C₆₋₁₀ aryl; in another embodiment, R_{dd} is -L" -3- to 14-membered carbocyclyl; in another embodiment, R_{dd} is -L" -3- to 14-membered heterocyclyl; in another embodiment, R_{dd} is -L''-5- to 14-membered heteroaryl; in another embodiment, R_{dd} is C₁₋₆ alkyl; in another embodiment, R_{dd} is C₁₋₆ haloalkyl; in another embodiment, R_{dd} is -L" -3- to 10-membered carbocyclyl, alternatively -L"-3- to 7-membered carbocyclyl; in another embodiment, R_{dd} is -L"-3- to 10-membered heterocyclyl, alternatively -L"-3- to 7-membered heterocyclyl, alternatively -L" -3- to 7-membered carbocyclyl; in another embodiment, R_{dd} is Me; in another embodiment, R_{dd} is N(CH₃)₂ in another embodiment, R_{dd} is C(CH₃)₂OH.

In another embodiment, R_{dd} is -CH₂OH; in another embodiment, R_{dd} is -CH₂OMe; in another embodiment, R_{dd} is 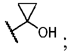 in another embodiment, R_{dd} is -NHMe; in another embodiment, R_{dd} is -CH₂-cyclopropyl.

In a more specific embodiment, R_{dd} is independently selected from H, D, oxo, halogen, CN, C₁₋₆ alkyl, C₁₋₆ haloalkyl, -L"-OR', -L"-SR', -L"-NR'R", -L"-3- to 7-membered carbocyclyl and -L"-3- to 7-membered heterocyclyl; in another more specific embodiment, R_{dd} is independently selected from H, D, oxo, halogen, CN, C₁₋₆ alkyl, C₁₋₆ haloalkyl, -L"-OR', -L"-SR', -L"-NR'R", -L"-3- to 7-membered carbocyclyl, -L"-3- to 7-membered heterocyclyl; in another more specific embodiment, R_{dd} is independently selected from H, D, oxo, C₁₋₆ alkyl, C₁₋₆ haloalkyl, -L"-OR', -L"-NR'R", -L"-3- to 7-membered carbocyclyl and -L"-3- to 7-membered heterocyclyl; in another more specific embodiment, R_{dd} is independently selected from H, D, halogen, oxo, C₁₋₆ alkyl, C₁₋₆ haloalkyl, -L''-OR', -L"-NR'R", -L"-3- to 7-membered carbocyclyl and -L''-3-to 7-membered heterocyclyl; in another more specific embodiment, R_{dd} is independently selected from H, D, halogen, oxo, C₁₋₆ alkyl, C₁₋₆ haloalkyl, -L"-OR', -L"-NR'R" and -L"-3- to 7-membered carbocyclyl; in another more specific embodiment, R_{dd} is independently selected from H, D, oxo, C₁₋₆ alkyl, C₁₋₆ haloalkyl, -L"-OR' and NR'R"; in another more specific embodiment, R_{dd} is independently selected from H, D, C₁₋₆ alkyl, C₁₋₆ haloalkyl and -L"-OR'; in another more specific embodiment, R_{dd} is independently selected from H, F, oxo, OH, Me, -C(CH₃)₂OH, -CH₂OH, -CH₂OMe, 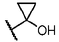 and -NHMe; in another more specific embodiment, R_{dd} is independently selected from H, oxo, OH, Me, -C(CH₃)₂OH, -CH₂OH, -CH₂OMe, 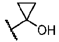 and -NHMe; in another more specific embodiment, R_{dd} is independently selected from H, OH, Me, -C(CH₃)₂OH, -CH₂OMe and 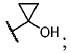 in another more specific embodiment, R_{dd} is independently selected from H, OH, Me, -CH₂OMe and -C(CH₃)₂OH.

In one embodiment, two R_{dd} are taken together to form a C₁₋₈ alkylene; in another embodiment, two non-adjacent R_{dd} are taken together to form a C₁₋₆ alkylene; in another embodiment, two non-adjacent R_{dd} are taken together to form a C₁₋₄ alkylene; in another embodiment, two non-adjacent R_{dd} are taken together to form a C₁₋₃ alkylene; in another embodiment, two non-adjacent R_{dd} are taken together to form a C₁₋₂ alkylene; in another embodiment, two non-adjacent R_{dd} are taken together to form -CH₂CH₂-; in another embodiment, two non-adjacent R_{dd} are taken together to form -CH₂CH₂-; in another embodiment, two non-adjacent R_{dd} are taken together to form a C₁₋₄ alkylene, or R'_{dd} and a non-adjacent R_{dd} are taken together to form a C₁₋₄ alkylene, or two R'_{dd} are taken together to form a C₁₋₄ alkylene; in another embodiment, the alkylene formed by two R_{dd} together is optionally substituted with one or more of the following substituents: H, D, halogen, CN, C₁₋₈ alkyl, C₁₋₈ alkoxy, C₁₋₈ haloalkyl, -L" -C₆₋₁₀ aryl, -L''-3- to 10-membered carbocyclyl, and -L''-3- to 10-membered heterocyclyl, alternatively optionally substituted with 1, 2, 3 or 4 substituents selected from: H, D, halogen, CN, C₁₋₆ alkyl and C₁₋₆ haloalkyl; in another embodiment, two non-adjacent R_{dd} are taken together to form -CH₂CH₂-, making 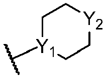 has the following structure: 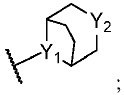 in another embodiment, the ring group formed by two non-adjacent R_{dd} is optionally substituted with 1, 2, 3 or 4 substituents selected from: H, D, halogen, CN, C₁₋₆ alkyl and C₁₋₆ haloalkyl; in another embodiment, the ring group formed by two non-adjacent R_{dd} is unsubstituted; in another embodiment, two non-adjacent R_{dd} do not form a ring.

In one embodiment, CR_{dd}R_{dd} forms a 3- to 7-membered carbocyclylene, such as cyclopropylene; in another embodiment, CR_{dd}R_{dd} forms a 3- to 7-membered heterocyclylene, for example, 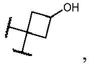 for example, 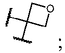 in another embodiment, the ring group formed by CR_{dd}R_{dd} is optionally substituted with 1, 2 or 3 groups selected from: H, D, halogen, C₁₋₆ alkyl, C₁₋₆ haloalkyl, -OR' and -NR'R'', alternatively optionally substituted with one -OR', alternatively optionally substituted with one -OH; in another embodiment, the ring group formed by CR_{dd}R_{dd} is unsubstituted; in another embodiment, CR_{dd}R_{dd} does not form a ring.

In a more specific embodiment, CR_{dd}R_{dd} forms a 3- to 7-membered carbocyclylene or a 3- to 7-membered heterocyclylene; in another more specific embodiment, CR_{dd}R_{dd} forms cyclopropylene, 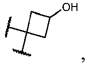 or 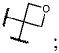 in another more specific embodiment, CR_{dd}R_{dd} forms cyclopropylene or 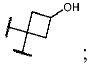 in another more specific embodiment, CR_{dd}R_{dd} forms cyclopropylene.

In one embodiment, two adjacent R_{dd} are taken together with the atoms to which they are attached to form a 3- to 7-membered carbocyclylene; in another embodiment, two adjacent R_{dd} are taken together with the atoms to which they are attached to form a 3- to 7-membered heterocyclylene; in another embodiment, two adjacent R_{dd} are taken together with the atoms to which they are attached to form a phenylene; in another embodiment, two adjacent R_{dd} are taken together with the atoms to which they are attached to form a 5- to 6-membered heteroarylene; in another embodiment, the ring formed by two adjacent R_{dd} and the atoms to which they are attached together is optionally substituted with 1, 2, 3 or 4 substituents selected from: H, D, halogen, CN, C₁₋₆ alkyl, C₁₋₆ haloalkyl, -OR' and -NR'R"; in another embodiment, the ring formed by two adjacent R_{dd} and the atoms to which they are attached together is optionally substituted with 1, 2, 3 or 4 substituents selected from: H, D, halogen, C₁₋₆ alkyl and C₁₋₆ haloalkyl; in another embodiment, the ring formed by two adjacent R_{dd} and the atoms to which they are attached together is unsubstituted; in another embodiment, two adjacent R_{dd} and the atoms to which they are attached do not form a ring.

In a more specific embodiment, two adjacent R_{dd} are taken together with the atoms to which they are attached to form a 3- to 7-membered carbocyclylene, a 3- to 7-membered heterocyclylene, a phenylene, or a 5-to 6-membered heteroarylene; in another more specific embodiment, two adjacent R_{dd} are taken together with the atoms to which they are attached to form a 3- to 7-membered heterocyclylene or a 5- to 6-membered heteroarylene; in another more specific embodiment, two adjacent R_{dd} are taken together with the atoms to which they are attached to form a 3- to 7-membered heterocyclylene.

In one embodiment, R'_{dd} is H; in another embodiment, R'_{dd} is C₁₋₆ alkyl; in another embodiment, R'_{dd} is C₁₋₆ haloalkyl; in another embodiment, R'_{dd} is -L"-OR'; in another embodiment, R'_{dd} is -L"-NR'R"; in another embodiment, R'_{dd} is -L"-3- to 7-membered carbocyclyl; in another embodiment, R'_{dd} is -L"-3- to 7-membered heterocyclyl.

In a more specific embodiment, R'_{dd} is independently selected from H, C₁₋₆ alkyl, C₁₋₆ haloalkyl, -L"-OR', -L"-NR'R", -L"-3- to 7-membered carbocyclyl and -L"-3- to 7-membered heterocyclyl; in another more specific embodiment, R'_{dd} is independently selected from H, C₁₋₆ alkyl, C₁₋₆ haloalkyl, -L"-3- to 7-membered carbocyclyl and -L"-3- to 7-membered heterocyclyl; in another more specific embodiment, R'_{dd} is independently selected from H, C₁₋₆ alkyl, C₁₋₆ haloalkyl and -L"-3- to 7-membered carbocyclyl; in another more specific embodiment, R'_{dd} is independently selected from H, Me and -CH₂-cyclopropyl.

In one embodiment, R'_{dd} and an adjacent R_{dd} are taken together with the atoms to which they are attached to form a 3- to 7-membered heterocyclylene, such as 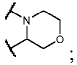 in another embodiment, R'_{dd} and an adjacent R_{dd} are taken together with the atoms to which they are attached to form a 5- to 6-membered heteroarylene, such as 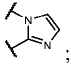 in another embodiment, the ring formed by R'_{dd}, an adjacent R_{dd} and the atoms to which they are attached together is optionally substituted with 1, 2, 3 or 4 substituents selected from: H, D, halogen, CN, C₁₋₆ alkyl, C₁₋₆ haloalkyl, -OR' and -NR'R"; in another embodiment, the ring formed by R'_{dd}, an adjacent R_{dd} and the atoms to which they are attached together is optionally substituted with 1, 2, 3 or 4 substituents selected from: H, D, halogen, C₁₋₆ alkyl and C₁₋₆ haloalkyl; in another embodiment, the ring formed by R'_{dd}, an adjacent R_{dd} and the atoms to which they are attached together is unsubstituted; in another embodiment, R'_{dd}, an adjacent R_{dd} and the atoms to which they are attached do not form a ring.

In a more specific embodiment, R'_{dd} and an adjacent R_{dd} are taken together with the atoms to which they are attached to form a 3- to 7-membered heterocyclylene or a 5- to 6-membered heteroarylene; in another more specific embodiment, R'_{dd} and an adjacent R_{dd} are taken together with the atoms to which they are attached to form a 3- to 7-membered heterocyclylene; in another more specific embodiment, R'_{dd} and an adjacent R_{dd} are taken together with the atoms to which they are attached to form 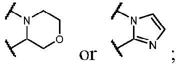 in another more specific embodiment, R'_{dd} and an adjacent R_{dd} are taken together with the atoms to which they are attached to form

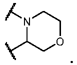

In a more specific embodiment, R_{dd} is independently selected from H, D, halogen, -CN, -NO₂, oxo, C₁₋₆ alkyl, C₁₋₆ haloalkyl, -L"-OR', -L"-NR'R", -L"-C(O)OR', -L"-C(O)NR'R", -L"-S(O)₀₋₂R', -L"-S(O)₂NR'R", -L"-S(O)NR'R", -L"-OC(O)R', -L"-OC(O)NR'R", -L''-3- to 10-membered carbocyclyl and -L''-3- to 10-membered heterocyclyl; in another more specific embodiment, R_{dd} is independently selected from H, D, halogen, C₁₋₆ alkyl, C₁₋₆ haloalkyl, -L"-OR', -L"-SR' and -L''-NR'R''; in another more specific embodiment, R_{dd} is independently selected from H, D, C₁₋₆ alkyl, C₁₋₆ haloalkyl, -L"-OR' and -L"-NR'R"; in another more specific embodiment, R_{dd} is independently selected from H, D, Me, N(CH₃)₂ and C(CH₃)₂OH.

In a more specific embodiment, R_{dd} is independently selected from H, D, oxo, halogen, CN, C₁₋₆ alkyl, C₁₋₆ haloalkyl, -L"-OR', -L"-SR', -L"-NR'R", -L"-3- to 7-membered carbocyclyl and -L"-3- to 7-membered heterocyclyl; in another more specific embodiment, R_{dd} is independently selected from H, D, oxo, C₁₋₆ alkyl, C₁₋₆ haloalkyl, -L"-OR', -L"-NR'R", -L"-3- to 7-membered carbocyclyl and -L"-3- to 7-membered heterocyclyl; in another more specific embodiment, R_{dd} is independently selected from H, D, oxo, C₁₋₆ alkyl, C₁₋₆ haloalkyl, -L"-OR', -L"-NR'R" and -L"-3- to 7-membered carbocyclyl; in another more specific embodiment, R_{dd} is independently selected from H, D, oxo, C₁₋₆ alkyl, C₁₋₆ haloalkyl, -L" -OR' and -L"-NR'R"; in another more specific embodiment, R_{dd} is independently selected from H, D, C₁₋₆ alkyl, C₁₋₆ haloalkyl and -L"-OR'; in another more specific embodiment, R_{dd} is independently selected from H, oxo, OH, Me, -C(CH₃)₂OH, -CH₂OH, -CH₂OMe, 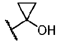 and -NHMe, alternatively H, oxo, OH, Me, -C(CH₃)₂OH, -CH₂OH, -CH₂OMe or -NHMe; in another more specific embodiment, R_{dd} is independently selected from H, Me and -C(CH₃)₂OH.

In a more specific embodiment, R'_{dd} is independently selected from H, C₁₋₆ alkyl, C₁₋₆ haloalkyl, -L"-OR', -L" -SR', -L"-NR'R", -L" -3- to 7-membered carbocyclyl and -L" -3- to 7-membered heterocyclyl; in another more specific embodiment, R'_{dd} is independently selected from H, C₁₋₆ alkyl, C₁₋₆ haloalkyl, -C₁₋₆ alkylene-OR', -C₁₋₆ alkylene-NR'R", -L"-3- to 7-membered carbocyclyl and -L"-3- to 7-membered heterocyclyl; in another more specific embodiment, R'_{dd} is independently selected from H, C₁₋₆ alkyl, C₁₋₆ haloalkyl, -L"-3- to 7-membered carbocyclyl and -L"-3- to 7-membered heterocyclyl; in another more specific embodiment, R'_{dd} is independently selected from H, C₁₋₆ alkyl, C₁₋₆ haloalkyl, -C₁₋₄ alkylene-OR', -C₁₋₄ alkylene-NR'R" and -L"-3- to 7-membered carbocyclyl; in another more specific embodiment, R'_{dd} is independently selected from H, C₁₋₆ alkyl, C₁₋₆ haloalkyl and -L"-3- to 7-membered carbocyclyl; in another more specific embodiment, R'_{dd} is independently selected from H, Me, -CH₂-cyclopropyl.

In a more specific embodiment, two non-adjacent R_{dd} are taken together to form a C₁₋₄ alkylene, or R'_{dd} and a non-adjacent R_{dd} are taken together to form a C₁₋₄ alkylene, or two R'_{dd} are taken together to form a C₁₋₄ alkylene, wherein the C₁₋₄ alkylene is optionally substituted with 1, 2, 3 or 4 substituents selected from: H, D, halogen, CN, C₁₋₆ alkyl and C₁₋₆ haloalkyl.

### L and L'

In one embodiment, L is a covalent bond; in another embodiment, L is -O-; in another embodiment, L is -S-; in another embodiment, L is -NR'-, such as -NH-; in another embodiment, L is -S(O)₂-; in another embodiment, L is -C(O)-; in another embodiment, L is -C(O)O-; in another embodiment, L is -C(O)NR'-; in another embodiment, L is -OC(O)-; in another embodiment, L is -OC(O)NR'-; in another embodiment, L is -N(R')C(O)O-; in another embodiment, L is -N(R')C(O)-; in another embodiment, L is -N(R')S(O)₂; in another embodiment, L is C₁₋₈ alkylene, alternatively C₁₋₆ alkylene, yet alternatively C₁₋₄ alkylene, in which one or more methylene units are optionally and independently replaced by -C(R')₂-, -N(R')-, -N(R')C(O)-, -C(O)N(R')-, -N(R')S(O)₂-, -S(O)₂N(R')-, -O-, -C(O)-, -OC(O)-, -C(O)O-, -S-, -S(O)-, or -S(O)₂-.

In one embodiment, L' is a covalent bond; in another embodiment, L' is -O-; in another embodiment, L' is -S-; in another embodiment, L' is -NR'-; in another embodiment, L' is -S(O)₂-; in another embodiment, L' is -C(O)-; in another embodiment, L' is -C(O)O-; in another embodiment, L' is -C(O)NR'-; in another embodiment, L' is -OC(O)-; in another embodiment, L' is -OC(O)NR'-; in another embodiment, L' is -N(R')C(O)O-; in another embodiment, L' is -N(R')C(O)-; in another embodiment, L' is -N(R')S(O)₂; in another embodiment, L' is C₁₋₈ alkylene, alternatively C₁₋₆ alkylene, yet alternatively C₁₋₄ alkylene, in which one or more methylene units are optionally and independently replaced by -C(R')₂-, -N(R')-, -N(R')C(O)-, -C(O)N(R')-, -N(R*)S(O)₂-, -S(O)₂N(R')-, -O-, -C(O)-, -OC(O)-, -C(O)O-, -S-, -S(O)-, or -S(O)₂-.

In another embodiment, L' is C₁₋₈ alkylene, alternatively C₁₋₆ alkylene, yet alternatively C₁₋₄ alkylene, in which 1, 2, 3 or 4 methylene units are optionally and independently replaced by -C(R')₂-, -N(R')-, -N(R')C(O)-, -C(O)N(R')-, -O-, -C(O)-, -OC(O)-, or -C(O)O-, alternatively optionally and independently replaced by -N(R')C(O)- or -O-.

In a more specific embodiment, L and L' are independently selected from covalent bond, -O-, -S-, -NR'-, -S(O)₂- and -C(O)-; or, L or L' is C₁₋₆ alkylene, in which one or more methylene units are optionally and independently replaced by -C(R')₂-, -N(R')-, -O-, -C(O)-, -S-, -S(O)-, or -S(O)₂-; in another more specific embodiment, L or L' is independently selected from covalent bond, O and C₁₋₆ alkylene; in another more specific embodiment, L or L' is independently selected from covalent bond, O and C₁₋₄ alkylene. In a more specific embodiment, L or L' is independently selected from covalent bond and C₁₋₆ alkylene; in another more specific embodiment, L or L' is independently selected from covalent bond and C₁₋₄ alkylene; in another more specific embodiment, L is independently selected from covalent bond, -O-, -S-, -NR'- and C₁₋₆ alkylene; in another more specific embodiment, L is independently selected from covalent bond, -NR'- and C₁₋₄ alkylene; in another more specific embodiment, L is independently selected from covalent bond and -NR'-; in another more specific embodiment, L is independently selected from covalent bond, -NH- and -CH(CH₃)-; in another more specific embodiment, L is independently selected from covalent bond and -NH-; in another more specific embodiment, L' is independently selected from covalent bond and C₁₋₂ alkylene; in another more specific embodiment, L' is independently selected from covalent bond and methylene; in another more specific embodiment, each of L and L' is optionally substituted with 1, 2, 3 or 4 groups selected from: C₁₋₆ alkyl, C₁₋₆ haloalkyl, -L_{d}-3- to 7-membered carbocyclyl and -L_{d}-3- to 7-membered heterocyclyl, alternatively optionally substituted with 1, 2, 3 or 4 groups selected from: C₁₋₆ alkyl and C₁₋₆ haloalkyl.

### R' and R"

In one embodiment, R' is H; in another embodiment, R' is C₁₋₈ alkyl; in another embodiment, R' is C₁₋₈ alkoxy; in another embodiment, R' is C₁₋₈ haloalkyl; in another embodiment, R' is -L" -C₆₋₁₀ aryl; in another embodiment, R' is -L" -3- to 14-membered carbocyclyl; in another embodiment, R' is -L"-3- to 14-membered heterocyclyl; in another embodiment, R' is -L"-5- to 14-membered heteroaryl; in another embodiment, R' is C₁₋₆ alkyl; in another embodiment, R' is C₁₋₆ haloalkyl; in another embodiment, R' is Me; in another embodiment, R' is Et; in another embodiment, R' is CHF₂; in another embodiment, R' is cyclopropyl.

In one embodiment, R" is H; in another embodiment, R" is C₁₋₈ alkyl; in another embodiment, R" is C₁₋₈ alkoxy; in another embodiment, R" is C₁₋₈ haloalkyl; in another embodiment, R" is -L" -C₆₋₁₀ aryl; in another embodiment, R" is -L" -3- to 14-membered carbocyclyl; in another embodiment, R" is -L" -3- to 14-membered heterocyclyl; in another embodiment, R" is -L"-5- to 14-membered heteroaryl; in another embodiment, R" is C₁₋₆ alkyl, alternatively C₁₋₄ alkyl; in another embodiment, R" is C₁₋₆ haloalkyl, alternatively C₁₋₄ haloalkyl; in another embodiment, R" is Me; in another embodiment, R" is Et; in another embodiment, R" is CHF₂; in another embodiment, R" is cyclopropyl.

In a more specific embodiment, each of R' and R" is independently selected from H, C₁₋₆ alkyl, C₁₋₆ haloalkyl, -L_{d}-3- to 7-membered carbocyclyl and -L_{d}-3- to 7-membered heterocyclyl; in another more specific embodiment, each of R' and R" is independently selected from H, C₁₋₆ alkyl, C₁₋₆ haloalkyl, 3- to 7-membered carbocyclyl and 3- to 7-membered heterocyclyl; in another more specific embodiment, each of R' and R" is independently selected from H, C₁₋₆ alkyl and C₁₋₆ haloalkyl; in another more specific embodiment, each of R' and R" is independently selected from C₁₋₆ alkyl and C₁₋₆ haloalkyl; in another more specific embodiment, R" is H or Me, alternatively Me.

In a more specific embodiment, R" is selected from H, C₁₋₆ alkyl and C₁₋₆ haloalkyl; in another more specific embodiment, R" is C₁₋₄ haloalkyl; in another more specific embodiment, R" is selected from H, methyl and CHF₂; in another more specific embodiment, R" is CHF₂.

### L''

In one embodiment, L'' is a covalent bond; in another embodiment, L" is -O-; in another embodiment, L" is -S-; in another embodiment, L" is C₁₋₈ alkylene, alternatively C₁₋₆ alkylene, alternatively C₁₋₄ alkylene, in which one or more methylene units are optionally and independently replaced by -O-, -C(O)-, -OC(O)-, -C(O)O-, -S-, -S(O)-, or -S(O)₂-; in another embodiment, L" is optionally substituted with one or more R, alternatively optionally substituted with 1, 2, 3 or 4 R; in another embodiment, L" is -C(R)₂-, alternatively 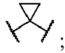 in another embodiment, L" is unsubstituted.

In a more specific embodiment, L" is independently selected from covalent bond and C₁₋₆ alkylene, each of which is optionally substituted with 1, 2, 3 or 4 R; in another more specific embodiment, L" is independently selected from covalent bond and C₁₋₆ alkylene, each of which is optionally substituted with 1, 2, 3 or 4 R; in another more specific embodiment, L" is independently selected from covalent bond, C₁₋₄ alkylene and -C(R)₂-; in another more specific embodiment, L" is independently selected from covalent bond and C₁₋₄ alkylene.

In a more specific embodiment, L" is independently selected from covalent bond and C₁₋₄ alkylene, each of which is optionally substituted with 1, 2, 3 or 4 R, and R is independently selected from H, D, C₁₋₆ alkyl and C₁₋₆ haloalkyl, or two R are taken together with the C atom to which they are jointly attached to form a 3- to 7-membered carbocyclylene, alternatively form a 3- to 5-membered carbocyclylene, alternatively form

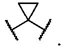

### R

In one embodiment, R is H; in another embodiment, R is D; in another embodiment, R is C₁₋₈ alkyl, alternatively C₁₋₆ alkyl; in another embodiment, R is C₁₋₈ haloalkyl, alternatively C₁₋₆ haloalkyl; in another embodiment, R is -L_{d}-3- to 10-membered carbocyclyl, alternatively -L_{d}-3- to 7-membered carbocyclyl; in another embodiment, R is -L_{d}-3- to 10-membered heterocyclyl, alternatively -L_{d}-3- to 7-membered heterocyclyl;

in one embodiment, two R are taken together with the C atom to which they are jointly attached to form a 3- to 10-membered carbocyclylene, alternatively form a 3- to 10-membered heterocyclylene, alternatively form a 3- to 7-membered carbocyclylene, alternatively form a 3- to 7-membered heterocyclylene, alternatively form a 3- to 5-membered carbocyclylene, alternatively form a 3- to 5-membered heterocyclylene, alternatively form

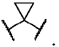

In a more specific embodiment, R is independently selected from H, D, C₁₋₆ alkyl, C₁₋₆ haloalkyl, -L_{d}-3- to 7-membered carbocyclyl and -L_{d}-3- to 7-membered heterocyclyl; in another more specific embodiment, R is independently selected from H, D, C₁₋₆ alkyl and C₁₋₆ haloalkyl.

In a more specific embodiment, two R are taken together with the C atom to which they are jointly attached to form a 3- to 7-membered carbocyclylene or a 3- to 7-membered heterocyclylene, alternatively form a 3- to 7-membered carbocyclylene, alternatively form a 3- to 5-membered carbocyclylene, alternatively form

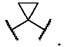

### L_{d}

In one embodiment, L_{d} is a covalent bond; in another embodiment, L_{d} is C₁₋₈ alkylene; in another embodiment, L_{d} is C₁₋₆ alkylene; in another embodiment, L_{d} is C₁₋₄ alkylene.

In a more specific embodiment, L_{d} is independently selected from covalent bond and C₁₋₆ alkylene; in another more specific embodiment, L_{d} is independently selected from covalent bond and C₁₋₄ alkylene.

Any technical solution in any one of the above specific embodiments, or any combination thereof, may be combined with any technical solution in other specific embodiments or any combination thereof. For example, any technical solution of ring A or any combination thereof may be combined with any technical solution of V, W, Q, Y, R_{A}, R_{B}, R_{C}, R'_{C}, R₁ₐ, R_{1b}, Z₁, Z₆, R_{E}, Rₑ, R_{d1}, R_{d}, R_{dd}, L, L', R', R", and L", etc., or any combination thereof. The present disclosure is intended to include all combination of such technical solutions, which are not exhaustively listed here to save space.

In a more specific embodiment, the present disclosure provides a compound of formula (I), or a stereoisomer, an N-oxide, a hydrate, a solvate, a metabolite, a pharmaceutically acceptable salt, a polymorph, or a prodrug thereof: 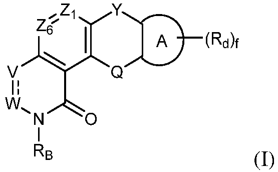
V is selected from covalent bond, O, S, NR', S(O), S(O)₂, C(O), CR_{C} and CR_{C}R_{C}';
W is selected from NR', S(O), S(O)₂, C(O), CR_{C} and CR_{C}R_{C}';
Q and Y are independently selected from covalent bond, -O-, -S-, -NR_{A}-, -S(O)-, -S(O)₂-, -C(O)-, -C(O)O-, -C(O)NR_{A}-, -OC(O)-, -OC(O)NR_{A}-, -N(R_{A})C(O)O-, -N(R_{A})C(O)- and -N(R_{A})S(O)₂-; and Q and Y are not covalent bonds at the same time;
or Q and Y are independently C₁₋₈ alkylene, in which one or more methylene units are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
R_{A} is selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl;
R_{B} is selected from H, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
R_{C}, R'_{C}, R₁ₐ and R_{1b} are independently selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
one of Z₁ and Z₆ is CR_{E}, and the other one is CR_{d1} or N;
R_{E} is selected from -L-C₆₋₁₀ aryl, -L-3- to 14-membered carbocyclyl, -L-3- to 14-membered heterocyclyl and -L-5- to 14-membered heteroaryl, wherein each of the C₆₋₁₀ aryl, 3- to 14-membered carbocyclyl, 3- to 14-membered heterocyclyl and 5- to 14-membered heteroaryl is optionally substituted with 1, 2, 3, 4, 5, 6, 7, 8, 9 or 10 Rₑ groups;
Rₑ is independently selected from H, D, halogen, -CN, oxo, C₁₋₈ alkyl, C₁₋₈ haloalkyl, C₂₋₈ alkenyl, C₂₋₈ alkynyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-C₆₋₁₀ aryl, -L'-3- to 14-membered carbocyclyl, -L'-3-to 14-membered heterocyclyl and -L'-5- to 14-membered heteroaryl;
R_{d1} is selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
ring A is selected from C₆₋₁₀ aromatic ring, 3- to 14-membered carbocycle, 3- to 14-membered heterocycle and 5- to 14-membered heteroaromatic ring;
R_{d} is independently selected from H, D, halogen, -CN, -L'-S(O)₀₋₂R', -L'-S(O)₂NR'R'', -L'-S(O)NR'R", -L'-OR', -L'-NR'R", -L'-C(O)OR' and -L'-C(O)NR'R"; or, R_{d} is selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₈ alkyl, C₂₋₈ alkenyl, C₂₋₈ alkynyl, C₁₋₈ haloalkyl, -L"-OR', -L"-NR'R", -L"-C(O)OR', -L"-C(O)NR'R", -L"-S(O)₀₋₂R', -L"-S(O)₂NR'R", -L"-S(O)NR'R", -L"-OC(O)R', -L"-OC(O)NR'R", -L"-C₆₋₁₀ aryl, -L"-3- to 14-membered carbocyclyl, -L"-3- to 14-membered heterocyclyl and -L"-5- to 14-membered heteroaryl;
or two R_{dd} are taken together to form a C₁₋₈ alkylene, which is optionally substituted with one or more of the following substituents: H, D, halogen, CN, C₁₋₈ alkyl, C₁₋₈ alkoxy, C₁₋₈ haloalkyl, -L"-C₆₋₁₀ aryl, -L"-3- to 10-membered carbocyclyl, -L"-3- to 10-membered heterocyclyl and -L"-5- to 10-membered heteroaryl;
L and L' are independently selected from covalent bond, -O-, -S-, -NR'-, -S(O)₂-, -C(O)-, -C(O)O-, -C(O)NR'-, -OC(O)-, -OC(O)NR'-, -N(R')C(O)O-, -N(R')C(O)- and -N(R')S(O)₂-; or, L or L' is C₁₋₈ alkylene, in which one or more methylene units are optionally and independently replaced by -C(R')₂-, -N(R')-, -N(R')C(O)-, -C(O)N(R')-, -N(R')S(O)₂-, -S(O)₂N(R')-, -O-, -C(O)-, -OC(O)-, -C(O)O-, -S-, -S(O)-, or -S(O)₂-;
R' and R" are independently selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl; or, R' and R" are taken together with the N atom to which they are attached to form a 3- to 14-membered heterocyclyl;
L" is selected from covalent bond, -O- and -S-; or, L" is C₁₋₈ alkylene, which is optionally substituted with one or more R, and one or more methylene units in the C₁₋₈ alkylene are optionally and independently replaced by -O-, -C(O)-, -OC(O)-, -C(O)O-, -S-, -S(O)-, or -S(O)₂-;
R is independently selected from H, D, C₁₋₈ alkyl, C₁₋₈ haloalkyl, -L_{d}-3- to 10-membered carbocyclyl and -L_{d}-3- to 10-membered heterocyclyl;
or two R are taken together with the C atom to which they are jointly attached to form a 3- to 10-membered carbocyclylene or a 3- to 10-membered heterocyclylene;
L_{d} is independently selected from covalent bond and C₁₋₈ alkylene;
f=0, 1, 2, 3, 4, 5, 6, 7, 8, 9 or 10;
wherein each of the above groups is optionally deuterated, up to completely deuterated.

In a more specific embodiment, the present disclosure provides a compound of formula (I), or a stereoisomer, an N-oxide, a hydrate, a solvate, a metabolite, a pharmaceutically acceptable salt, a polymorph, or a prodrug thereof: 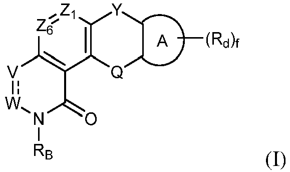
V is selected from covalent bond, O, S, NR', S(O), S(O)₂, C(O), CR_{C} and CR_{C}R_{C}';
W is selected from NR', S(O), S(O)₂, C(O), CR_{C} and CR_{C}R_{C}';
Q and Y are independently selected from covalent bond, -O-, -S-, -NR_{A}-, -S(O)-, -S(O)₂-, -C(O)-, -C(O)O-, -C(O)NR_{A}-, -OC(O)-, -OC(O)NR_{A}-, -N(R_{A})C(O)O-, -N(R_{A})C(O)- and -N(R_{A})S(O)₂-; and Q and Y are not covalent bonds at the same time;
or Q and Y are independently C₁₋₈ alkylene, in which one or more methylene units are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
R_{A} is selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl;
R_{B} is selected from H, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
R_{C}, R'_{C}, R₁ₐ and R_{1b} are independently selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
one of Z₁ and Z₆ is CR_{E}, and the other one is CR_{d1} or N;
R_{E} is selected from -L-C₆₋₁₀ aryl, -L-3- to 14-membered carbocyclyl, -L-3- to 14-membered heterocyclyl and -L-5- to 14-membered heteroaryl, wherein each of the C₆₋₁₀ aryl, 3- to 14-membered carbocyclyl, 3- to 14-membered heterocyclyl and 5- to 14-membered heteroaryl is optionally substituted with 1, 2, 3, 4, 5, 6, 7, 8, 9 or 10 Rₑ groups;
Rₑ is independently selected from H, D, halogen, -CN, oxo, C₁₋₈ alkyl, C₁₋₈ haloalkyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-C₆₋₁₀ aryl, -L'-3- to 14-membered carbocyclyl, -L'-3- to 14-membered heterocyclyl and -L'-5- to 14-membered heteroaryl;
R_{d1} is selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
ring A is selected from C₆₋₁₀ aromatic ring, 3- to 14-membered carbocycle, 3- to 14-membered heterocycle and 5- to 14-membered heteroaromatic ring;
R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or, R_{d} is selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₈ alkyl, C₂₋₈ alkenyl, C₂₋₈ alkynyl, C₁₋₈ haloalkyl, -L"-OR', -L"-NR'R", -L"-C(O)OR', -L"-C(O)NR'R", -L"-S(O)₀₋₂R', -L"-S(O)₂NR'R", -L"-S(O)NR'R", -L"-OC(O)R', -L"-OC(O)NR'R", -L"-C₆₋₁₀ aryl, -L"-3- to 14-membered carbocyclyl, -L"-3- to 14-membered heterocyclyl and -L"-5- to 14-membered heteroaryl;
L and L' are independently selected from covalent bond, -O-, -S-, -NR'-, -S(O)₂-, -C(O)-, -C(O)O-, -C(O)NR'-, -OC(O)-, -OC(O)NR'-, -N(R')C(O)O-, -N(R')C(O)- and -N(R')S(O)₂-; or, L or L' is C₁₋₈ alkylene, in which one or more methylene units are optionally and independently replaced by -C(R')₂-, -N(R')-, -N(R')C(O)-, -C(O)N(R')-, -N(R")S(O)₂-, -S(O)₂N(R')-, -O-, -C(O)-, -OC(O)-, -C(O)O-, -S-, -S(O)-, or -S(O)₂-;
R' and R" are independently selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl; or, R' and R" are taken together with the N atom to which they are attached to form a 3- to 14-membered heterocyclyl;
L" is selected from covalent bond, -O- and -S-; or, L" is C₁₋₈ alkylene, in which one or more methylene units are optionally and independently replaced by -O-, -C(O)-, -OC(O)-, -C(O)O-, -S-, -S(O)-, or -S(O)₂-;
f=0, 1, 2, 3, 4, 5, 6, 7, 8, 9 or 10;
wherein each of the above groups is optionally deuterated, up to completely deuterated.

The structure of ring A in formula (I) should be understood as two adjacent atoms on the ring A being connected to Q and Y, respectively. The two adjacent atoms may be carbon atoms or heteroatoms such as N.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein V is selected from covalent bond, CR_{C} and CR_{C}R_{C}'; alternatively selected from covalent bond, CH, CH₂, CD, CHD and CD₂, yet alternatively a covalent bond.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein W is CR_{C} or CR_{C}R_{C}'; alternatively CR_{C}R_{C}', yet alternatively CH₂, CD, CHD, or CD₂.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein the compound of formula (I) is represented by one of the following formulas:

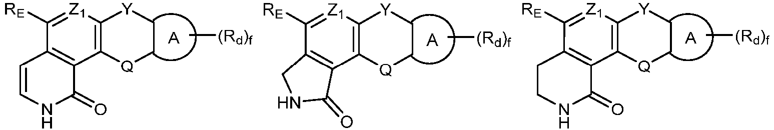

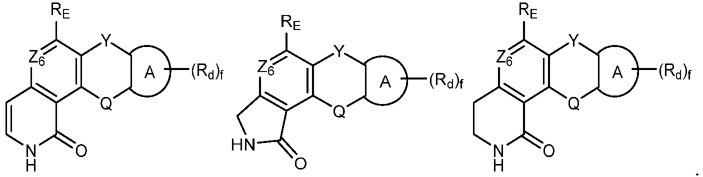

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein Q and Y are independently selected from covalent bond, O, S, NR_{A}, S(O), S(O)₂, C(O) and CR₁ₐR_{1b}; and Q and Y are not covalent bonds at the same time.

Alternatively, Q is selected from O, S and NR_{A}, alternatively S or NR_{A}, alternatively NR_{A}, yet alternatively NH.

Alternatively, Y is selected from covalent bond, O, S and CR₁ₐR_{1b}, alternatively selected from covalent bond, O and CR₁ₐR_{1b}, alternatively selected from covalent bond, O, CH₂, CF₂ and C(CH₃)₂, yet alternatively C(CH₃)₂.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein R_{A} is selected from H, C₁₋₆ alkyl, C₁₋₆ haloalkyl, -L"-3- to 10-membered carbocyclyl and -L"-3- to 10-membered heterocyclyl; alternatively selected from H, C₁₋₆ alkyl and C₁₋₆ haloalkyl; alternatively H.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein R_{B} is selected from H, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl; alternatively selected from H, C₁₋₆ alkyl and C₁₋₆ haloalkyl; alternatively H.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein R_{C} and R'_{C} are independently selected from H, D, halogen, -CN, C₁₋₆ alkyl, C₁₋₈ haloalkyl, -L-OR', -L-NR'R", -L-C(O)NR'R", -L-3- to 10-membered carbocyclyl and -L-3- to 10-membered heterocyclyl; alternatively independently selected from H, D, halogen, C₁₋₆ alkyl and C₁₋₆ haloalkyl; yet alternatively independently H or D.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein R₁ₐ and R_{1b} are independently selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl; alternatively independently selected from H, D, halogen, CN, C₁₋₆ alkyl and C₁₋₆ haloalkyl; alternatively independently selected from H, D, halogen, C₁₋₆ alkyl and C₁₋₆ haloalkyl; alternatively independently selected from H, D, halogen, C₁₋₃ alkyl and C₁₋₃ haloalkyl; alternatively independently selected from H, D, F and Me; yet alternatively Me.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein R_{E} is selected from phenyl, pyridyl, pyrimidinyl, pyrazinyl, pyrrolyl, pyrazolyl, imidazolyl, pyrrolopyridyl, pyrrolopyridazinyl, pyrrolopyrimidinyl, pyrrolopyrazinyl, imidazopyridyl, imidazopyridazinyl, imidazopyrimidinyl, imidazopyrazinyl, pyrazolopyridyl, pyrazolopyridazinyl, pyrazolopyrimidinyl, pyrazolopyrazinyl, pyridopyridyl, dihydropyridooxazinyl, indolyl, triazolopyridyl, tetrahydroimidazopyrazinyl, dihydropyrrolopyridyl, thienopyridyl, benzimidazolyl, dihydroimidazooxazinyl, tetrahydropyrazolopyridyl, tetrahydropyrrolopyridyl, dihydropyrazolooxazinyl, dihydropyrroloimidazolyl, oxazolopyridyl, pyrazoloazepanyl, pyrazolopiperidyl, imidazoazepanyl, imidazopiperidyl, pyrazolodiazepanyl, pyrazolodiazacyclohexyl and thienopyridyl, each of which is optionally substituted with 1, 2, 3, 4, 5, 6, 7, 8, 9 or 10 Rₑ groups;
optionally, R_{E} and a substituent Rₑ thereon are taken together to form a group selected from:

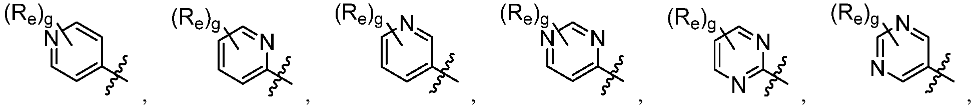

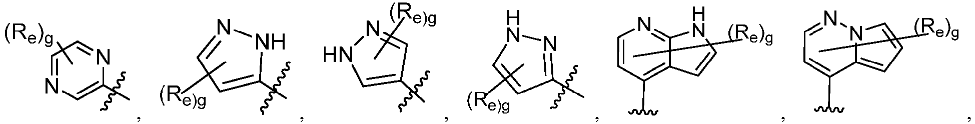

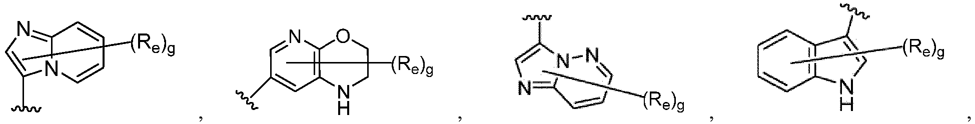

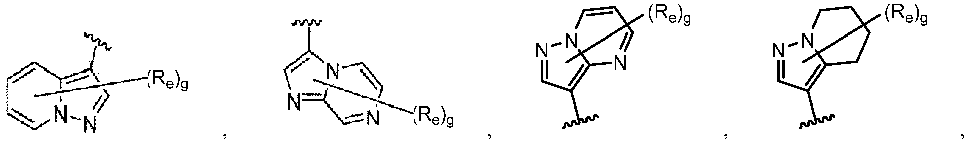

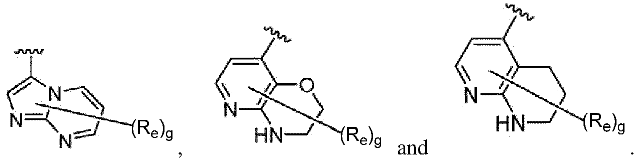

In some embodiments of the present disclosure, R_{E} and a substituent Rₑ thereon are taken together to form a group selected from:

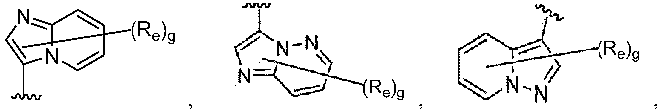

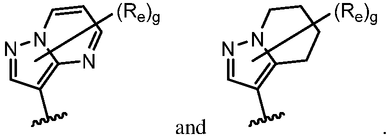

Wherein g=0, 1, 2, 3, 4, 5, 6, 7, 8, 9 or 10.

Alternatively, g=0, 1, 2, 3, 4, 5 or 6, alternatively g=0, 1, 2, 3 or 4, alternatively g=0, 1 or 2.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein Rₑ is independently selected from H, D, halogen, -CN, oxo, C₁₋₆ alkyl, C₁₋₆ haloalkyl, -L'-OR', -L'-NR'R'', -NO₂, -L'-SR', -L'-C(O)OR', -L'-C(O)NR'R", -L'-S(O)₂R', -L'-S(O)₂NR'R'', -L'-OC(O)R', -L'-OC(O)NR'R", -L'-3- to 10-membered carbocyclyl and -L'-3- to 10-membered heterocyclyl; alternatively independently selected from H, D, halogen, -CN, -OR', -SR', -NR'R", C₁₋₆ alkyl and C₁₋₆ haloalkyl; alternatively independently selected from H, D, halogen, -CN, -OR', C₁₋₆ alkyl and C₁₋₆ haloalkyl; alternatively independently selected from H, D, Me, F, Cl, -CN and -OCH₃.

Optionally, R_{E} and a substituent Rₑ thereon are taken together to form a group selected from: 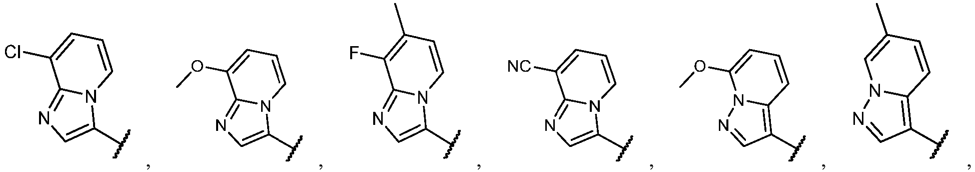 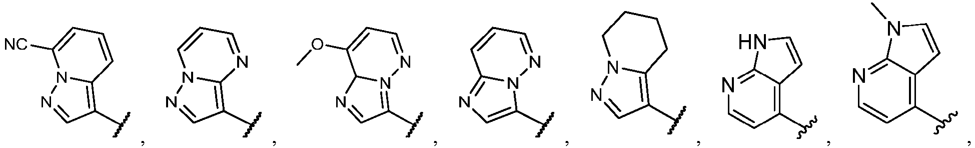 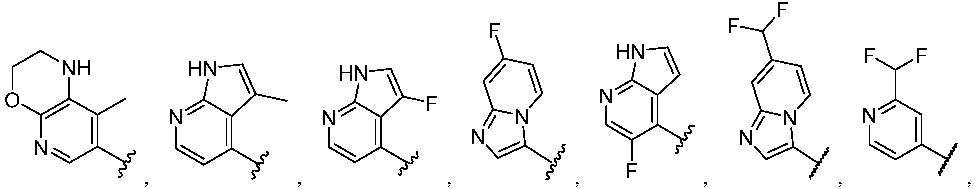 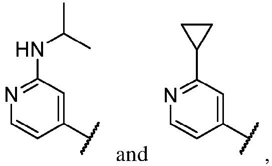 alternatively selected from: 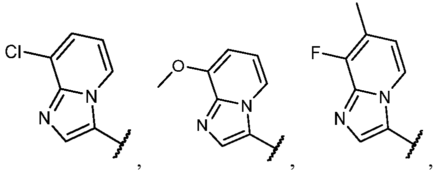 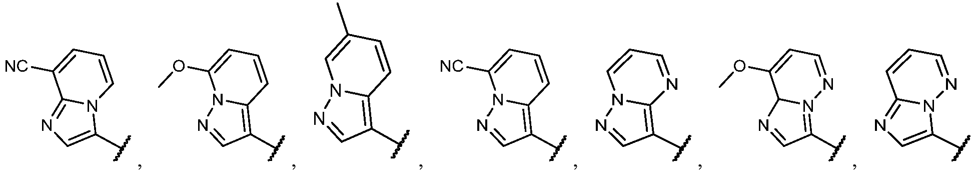 and 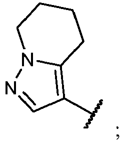 yet alternatively selected from:

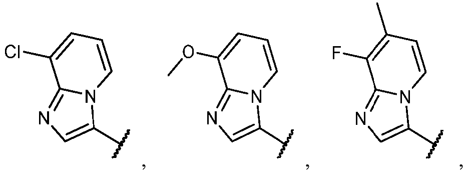

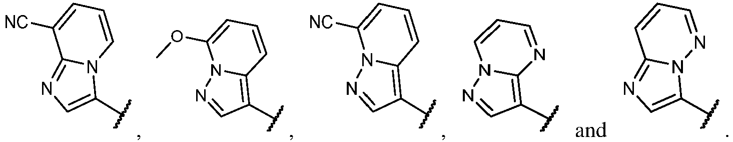

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein Z₆ is CR_{E}, and Z₁ is CR_{d1} or N, or Z₁ is CR_{E}, and Z₆ is N; alternatively Z₆ is CR_{E}, and Z₁ is CR_{d1} or N; alternatively Z₆ is CR_{E}, and Z₁ is CR_{d1}; yet alternatively, Z₆ is CR_{E}, and Z₁ is CH or CD.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein R_{d1} is selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl; alternatively selected from H, D, halogen, C₁₋₆ alkyl and C₁₋₆ haloalkyl; yet alternatively H or D.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein ring A is selected from benzene ring, furan ring, furazan ring, imidazolidine ring, imidazoline ring, imidazole ring, isothiazole ring, isoxazole ring, morpholine ring, oxadiazole ring, 1,2,3-oxadiazole ring, 1,2,4-oxadiazole ring, 1,2,5-oxadiazole ring, 1,3,4-oxadiazole ring, oxazolidine ring, oxazole ring, oxetane ring, azetidine ring, pyrimidine ring, piperazine ring, piperidine ring, pyran ring, pyrazine ring, pyrazolidine ring, pyrazoline ring, pyrazole ring, pyridazine ring, pyridine ring, pyrrolidine ring, pyrroline ring, 2H-pyrrole ring, pyrrole ring, tetrahydrofuran ring, tetrahydropyran ring, thiazole ring, thiophene ring, triazine ring, 1,2,3-triazole ring, 1,2,4-triazole ring, 1,2,5-triazole ring, 1,3,4-triazole ring, xanthene ring, tetrahydroquinoline ring, tetrahydroisoquinoline ring and hexahydropyrazinoquinoline ring; alternatively selected from benzene ring, pyridine ring, pyrazole ring and imidazole ring; yet alternatively benzene ring.

Optionally, ring A and a substituent R_{d} thereon are taken together to form a group selected from: 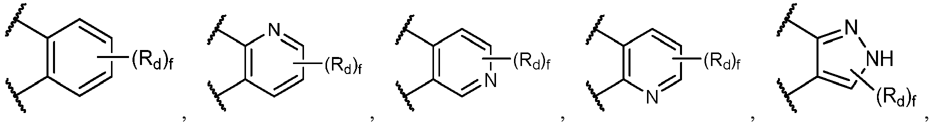 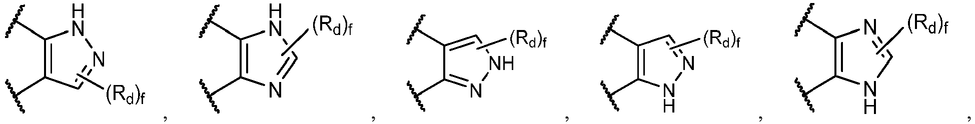 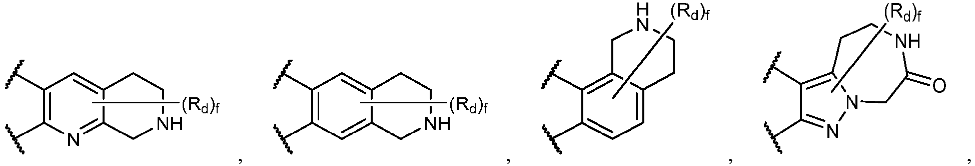 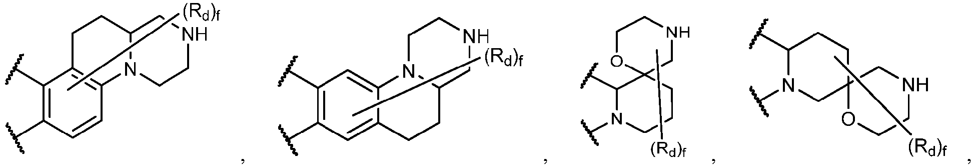 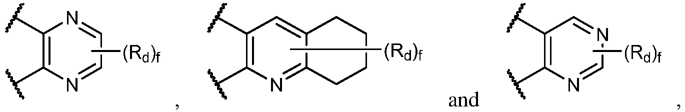 alternatively selected from: 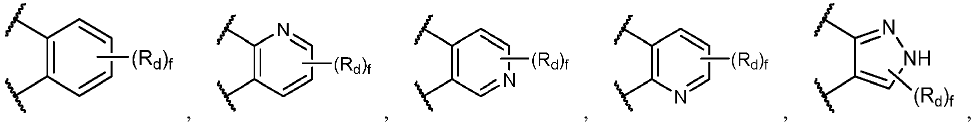 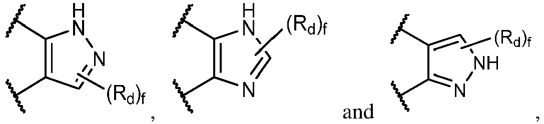 wherein f=0, 1, 2, 3, 4, 5, 6, 7, 8, 9 or 10.

Optionally, f=0, 1, 2, 3, 4, 5 or 6, alternatively f=0, 1, 2, 3 or 4, alternatively f=0, 1 or 2.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or, R_{d} is independently selected from C₁₋₆ alkyl, C₁₋₆ haloalkyl, -L'-3- to 10-membered carbocyclyl and -L'-3-to 10-membered heterocyclyl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd}.

Optionally, R_{d} is independently selected from H, D, halogen, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, and the C₁₋₆ alkyl, C₁₋₆ haloalkyl, 3- to 7-membered carbocyclyl or 3- to 7-membered heterocyclyl is optionally substituted with 1, 2, 3, 4, 5, 6, 7 or 8 R_{dd}.

Optionally, R_{d} is independently selected from H, D, C₁₋₆ alkyl, C₁₋₆ haloalkyl, -L'-NR'R" and -L'-4-to 6-membered heterocyclyl, and the C₁₋₆ alkyl, C₁₋₆ haloalkyl or 4- to 6-membered heterocyclyl is optionally substituted with 1 or 2 R_{dd}.

In some specific embodiments of the present disclosure, R_{d} is independently selected from H, D, 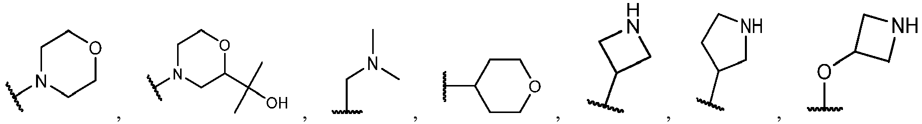 and 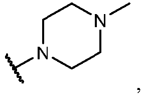 alternatively independently selected from H, D, 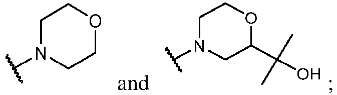 in some specific embodiments of the present disclosure, ring A and a substituent R_{d} thereon are taken together to form a group selected from: 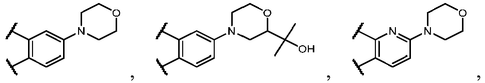 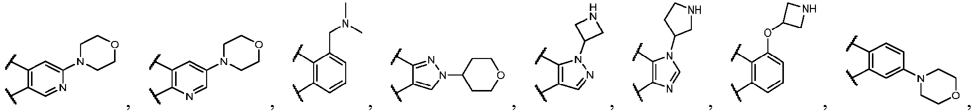 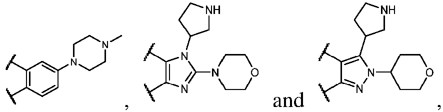 alternatively the group is 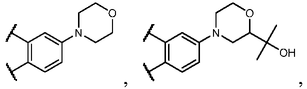 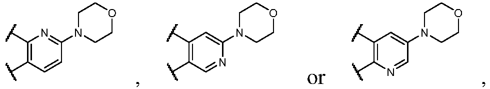 alternatively 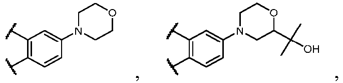 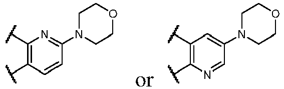 , yet alternatively

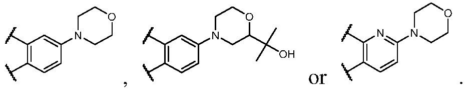

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein R_{d} is independently selected from H, F, -OH, -OCH₃,

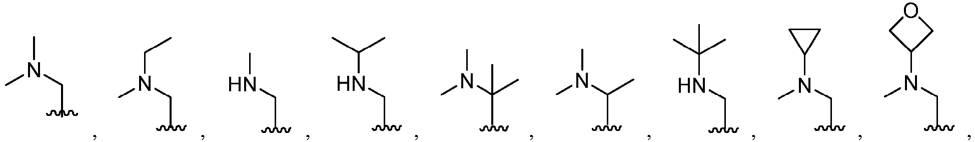

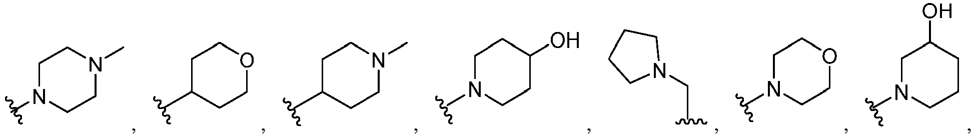

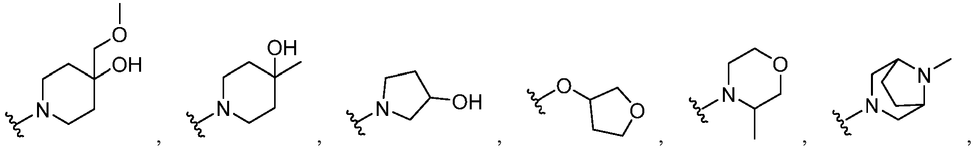

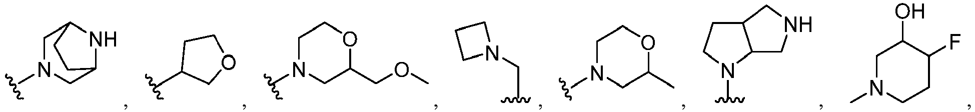

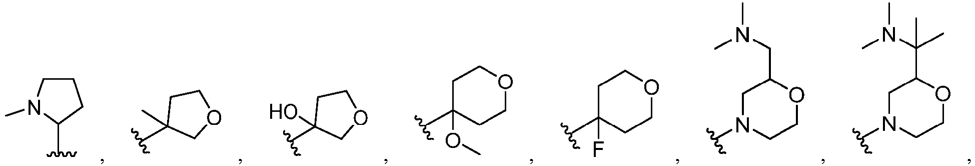

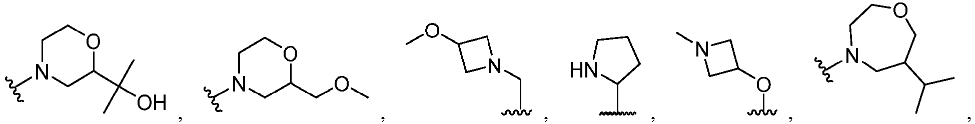

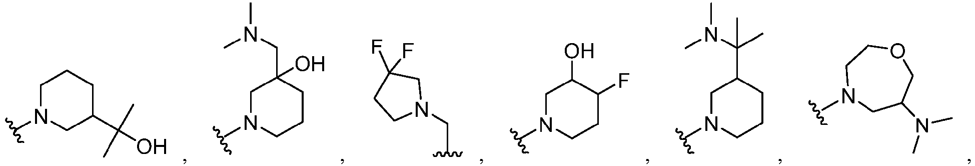

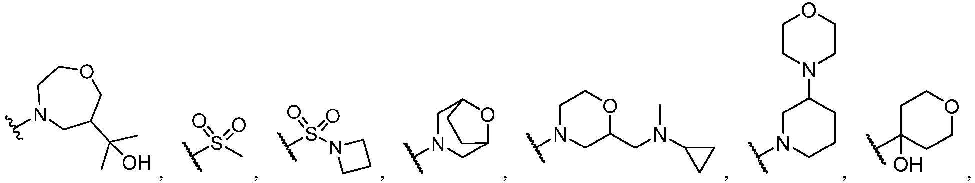

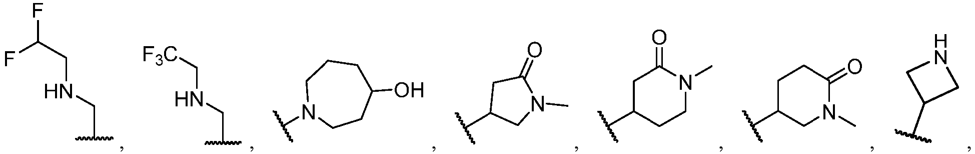

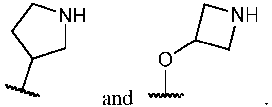

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein R_{dd} is independently selected from H, D, halogen, -CN, -NO₂, oxo, C₁₋₆ alkyl, C₁₋₆ haloalkyl, -L"-OR', -L"-NR'R", -L"-C(O)OR', -L"-C(O)NR'R", -L"-S(O)₀₋₂R', -L"-S(O)₂NR'R", -L"-S(O)NR'R", -L"-OC(O)R', -L"-OC(O)NR'R", -L"-3- to 10-membered carbocyclyl and -L"-3- to 10-membered heterocyclyl; alternatively independently selected from H, D, halogen, C₁₋₆ alkyl, C₁₋₆ haloalkyl, -L"-OR', -L"-SR' and -L"-NR'R"; alternatively independently selected from H, D, C₁₋₆ alkyl, C₁₋₆ haloalkyl, -L"-OR' and -L"-NR'R", alternatively independently selected from H, D, Me, N(CH₃)₂ and C(CH₃)₂OH.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein ring A and a substituent R_{d} thereon are taken together to form a group selected from:

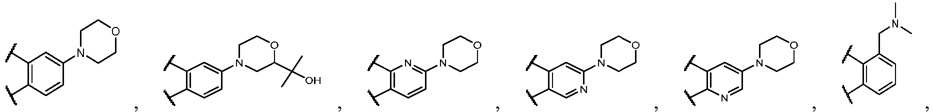

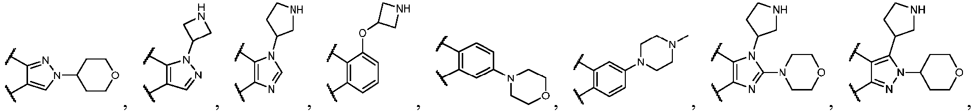

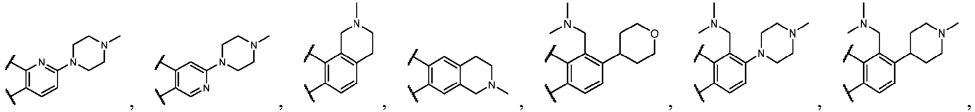

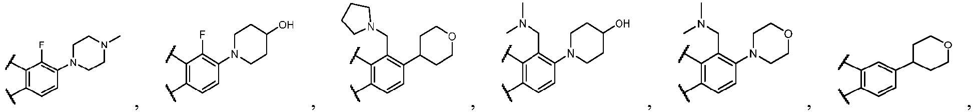

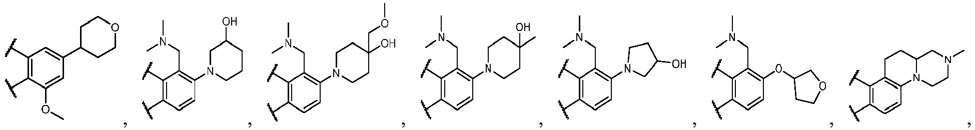

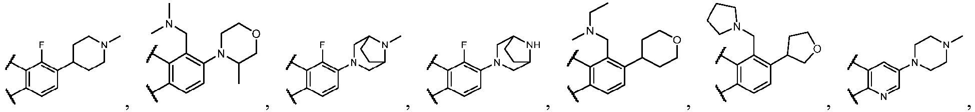

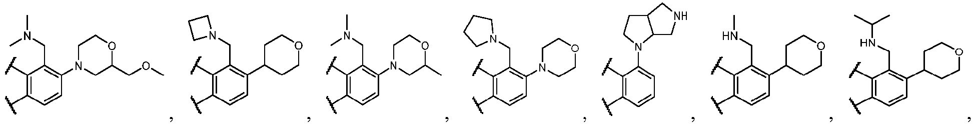

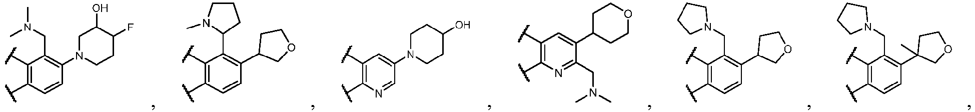

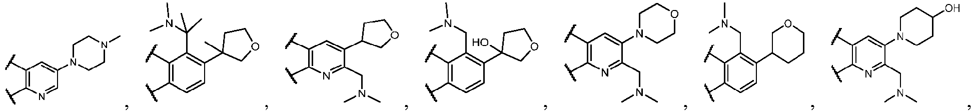

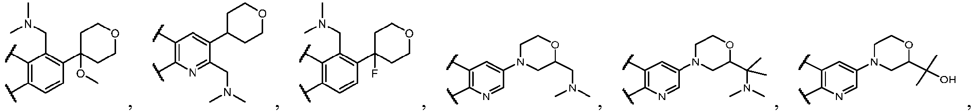

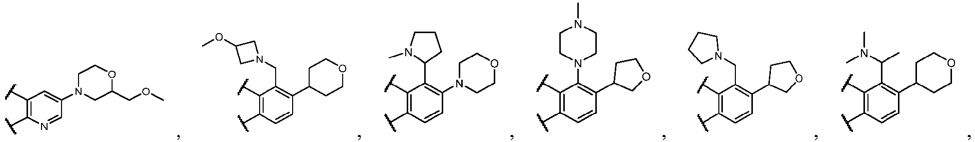

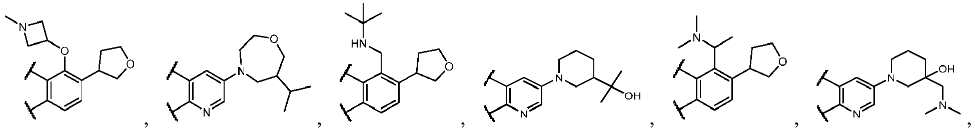

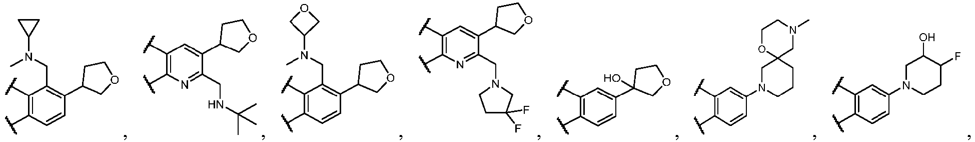

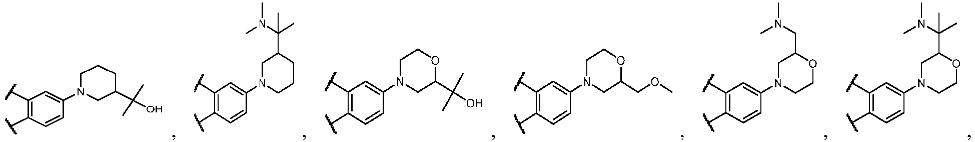

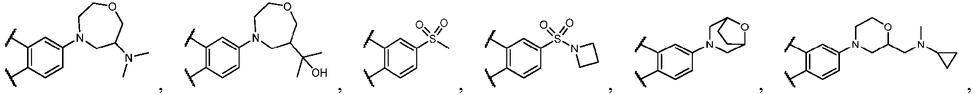

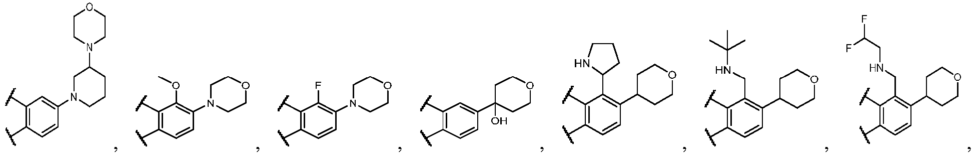

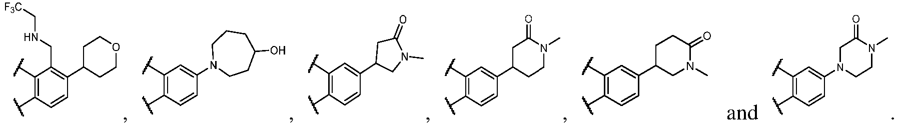

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein L and L' are independently selected from chemical bond, -O-, -S-, -NR'-, -S(O)₂- and -C(O)-; or, L or L' is C₁₋₆ alkylene, in which one or more methylene units are optionally and independently replaced by -C(R')₂-, -N(R')-, -O-, -C(O)-, -S-, -S(O)-, or -S(O)₂-.

Alternatively, L or L' is independently selected from covalent bond, O and C₁₋₆ alkylene, alternatively independently selected from covalent bond, O and C₁₋₄ alkylene.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, wherein L" is selected from covalent bond, -O- and -S-; or, L" is C₁₋₆ alkylene, in which one or more methylene units are optionally and independently replaced by -O-, -C(O)-, -S-, -S(O)-, or -S(O)₂-; alternatively L" is selected from covalent bond and C₁₋₆ alkylene; yet alternatively covalent bond or C₁₋₄ alkylene.

In a more specific embodiment, the present disclosure provides a compound of the above formula (I') or formula (I), or a stereoisomer, an N-oxide, a hydrate, a solvate, a metabolite, a pharmaceutically acceptable salt, a polymorph, or a prodrug thereof, and the compound has the following structural formula: 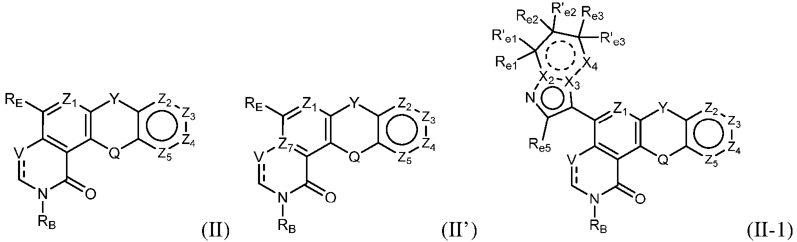 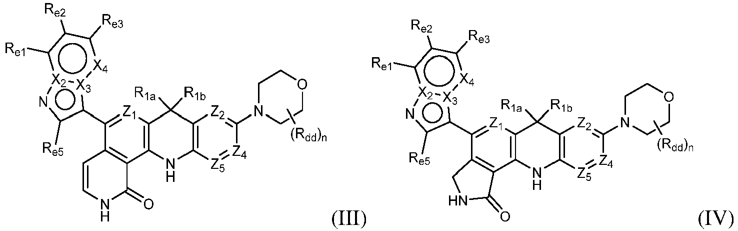 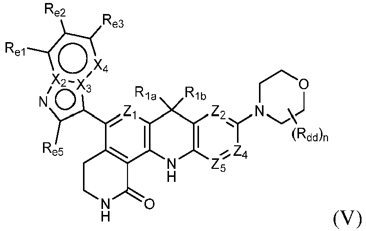 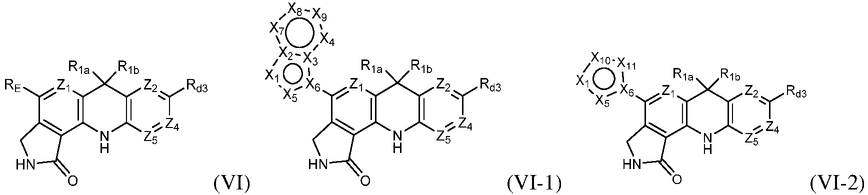 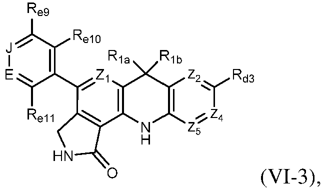 wherein
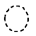 indicates that a double bond may be present or absent; when the double bond is present, it means that the ring where it is located is an aromatic ring, and R'ₑ₁, R'ₑ₂, R'ₑ₃ and R'ₑ₄ are absent; when the double bond is absent, it means that the ring where it is located is not aromatic;
X₁ is CRₑ₆, N, O, S or NR'ₑ₆;
X₂ is C or N;
X₃ is C or N;
X₄ is CRₑ₄, CRₑ₄R'ₑ₄ or N;
X₅ is CRₑ₅, N, O, S or NR'ₑ₅;
X₆ is C or N;
X₇ is CRₑ₁ or N;
X₈ is CRₑ₂ or N;
X₉ is CRₑ₃ or N;
X₁₀ is selected from CRₑ₇, O, S, N and NR'ₑ₇;
X₁₁ is selected from CRₑ₈, O, S, N and NR'ₑ₈;
Rₑ₁, Rₑ₂, Rₑ₃, Rₑ₄, Rₑ₅ and Rₑ₆ are independently selected from H, D, halogen, -CN, C₁₋₈ alkyl, C₁₋₈ haloalkyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-C₆₋₁₀ aryl, -L'-3- to 14-membered carbocyclyl, -L'-3-to 14-membered heterocyclyl and -L'-5- to 14-membered heteroaryl;
R'ₑ₁, R'ₑ₂, R'ₑ₃ and R'ₑ₄ are independently selected from absent, H, D, halogen, -CN, C₁₋₈ alkyl and C₁₋₈ haloalkyl; or CRₑ₁R'ₑ₁ forms -C(O)-, or CRₑ₂R'ₑ₂ forms -C(O)-, or CRₑ₃R'ₑ₃ forms -C(O)-, or CRₑ₄R'ₑ₄ forms -C(O)-;
R'ₑ₅ and R'ₑ₆ are independently selected from H, C₁₋₈ alkyl and C₁₋₈ haloalkyl;
Rₑ₇ and Rₑ₈ are independently selected from H, D, halogen, CN, C₁₋₈ alkyl, C₁₋₈ haloalkyl, -L'-OR', -L'-SR', -L'-NR'R", -L'-3- to 10-membered carbocyclyl and -L'-3- to 10-membered heterocyclyl;
R'ₑ₇ and R'ₑ₈ are independently selected from H, D, C₁₋₈ alkyl, C₁₋₈ haloalkyl, -L'-3- to 10-membered carbocyclyl and -L'-3- to 10-membered heterocyclyl;
or the substituents on X₁₀ and X₁₁ are taken together to form a C₃₋₅ alkylene, which optionally contains one double bond; the C₃₋₅ alkylene is optionally substituted with 1, 2, 3 or 4 Rₑ, and one or more methylene units in the C₃₋₅ alkylene are optionally and independently replaced by NR"-, -N(R")C(O)-, -C(O)N(R")-, -N(R")S(O)₂-, -S(O)₂N(R")-, -O-, -C(O)-, -OC(O)-, -C(O)O-, -S-, -S(O)-, or -S(O)₂-;
E is selected from CRₑ₁₂ and N;
J is selected from CRₑ₁₃ and N;
Rₑ₉, Rₑ₁₀, Rₑ₁₁, Rₑ₁₂ and Rₑ₁₃ are selected from H, D, halogen, CN, C₁₋₈ alkyl, C₁₋₈ haloalkyl, C₂₋₈ alkenyl, C₂₋₈ alkynyl, -L'-OR', -L'-SR', -L'-NR'R", -L'-3- to 10-membered carbocyclyl, -L'-3- to 10-membered heterocyclyl and -Y₅-C₀₋₈ alkylene-R₆;
Y₅ is selected from O, S and NR₅;
R₅ is selected from H, C₁₋₈ alkyl and C₁₋₈ haloalkyl;
R₆ is selected from 3- to 10-membered carbocyclyl and 3- to 10-membered heterocyclyl;
or Rₑ₉ and Rₑ₁₃ are taken together with the carbon atoms to which they are attached to form a 5- to 10-membered carbocyclyl, a 5- to 10-membered heterocyclyl, a C₆₋₁₀ aryl or a 5- to 10-membered heteroaryl, which is optionally substituted with 1, 2, 3 or 4 Rₑ;
or Rₑ₉ and Rₑ₁₀ are taken together with the carbon atoms to which they are attached to form a 5- to 10-membered carbocyclyl, a 5- to 10-membered heterocyclyl, a C₆₋₁₀ aryl or a 5- to 10-membered heteroaryl, which is optionally substituted with 1, 2, 3 or 4 Rₑ;
Z₂ is C atom or N atom, which is optionally substituted with R_{d2};
Z₃ is C atom or N atom, which is optionally substituted with R_{d3};
Z₄ is C atom or N atom, which is optionally substituted with R_{d4};
Z₅ is selected from covalent bond, CR_{d5} and N;
R_{d2}, R_{d3}, R_{d4} and R_{d5} are independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R", or R_{d2}, R_{d3}, R_{d4} and R_{d5} are independently selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or R_{d2} and R_{d3} are taken together with the atoms to which they are attached, or R_{d3} and R_{d4} are taken together with the atoms to which they are attached, or R_{d4} and R_{d5} are taken together with the atoms to which they are attached, to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl or 6- to 10-membered fused bicyclic heterocyclyl is optionally substituted with 1, 2, 3, 4 or 5 R_{dd};
n=0, 1, 2, 3, 4, 5, 6, 7 or 8;
other groups are as defined herein;
each of the above groups is optionally deuterated, up to completely deuterated.
In a more specific embodiment, the present disclosure provides a compound of the above formula (II-1), or a stereoisomer, an N-oxide, a hydrate, a solvate, a metabolite, a pharmaceutically acceptable salt, a polymorph, or a prodrug thereof, wherein
V is selected from covalent bond, CR_{C} and CR_{C}R_{C}';
Q and Y are independently selected from covalent bond, O, S, NR_{A}, S(O), S(O)₂, C(O) and CR₁ₐR_{1b}; and Q and Y are not covalent bonds at the same time;
R_{A} is selected from H, C₁₋₆ alkyl, C₁₋₆ haloalkyl, -L"-3- to 10-membered carbocyclyl and -L"-3- to 10-membered heterocyclyl;
R_{B} is selected from H, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl;
R_{C}, R'c, R₁ₐ and R_{1b} are independently selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl;
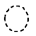 indicates that a double bond may be present or absent; when the double bond is present, it means that the ring where it is located is an aromatic ring, and R'ₑ₁, R'ₑ₂, R'ₑ₃ and R'ₑ₄ are absent; when the double bond is absent, it means that the ring where it is located is not aromatic;
X₂ is C or N;
X₃ is C or N;
X₄ is CRₑ₄, CRₑ₄R'ₑ₄ or N;
Rₑ₁, Rₑ₂, Rₑ₃, Rₑ₄ and Rₑ₅ are independently selected from H, D, halogen, -CN, C₁₋₆ alkyl, C₁₋₆ haloalkyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-3- to 10-membered carbocyclyl and -L'-3- to 10-membered heterocyclyl;
R'ₑ₁, R'ₑ₂, R'ₑ₃ and R'ₑ₄ are independently selected from H, D, halogen, -CN, C₁₋₆ alkyl and C₁₋₆ haloalkyl; or CRₑ₁R'ₑ₁ forms -C(O)-, or CRₑ₂R'ₑ₂ forms -C(O)-, or CRₑ₃R'ₑ₃ forms -C(O)-, or CRₑ₄R'ₑ₄ forms -C(O)-;
Z₁ is CR_{d1} or N;
Z₂ is C atom or N atom, which is optionally substituted with R_{d2};
Z₃ is C atom or N atom, which is optionally substituted with R_{d3};
Z₄ is C atom or N atom, which is optionally substituted with R_{d4};
Z₅ is selected from covalent bond, CR_{d5} and N;
R_{d1} is selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl;
R_{d2}, R_{d3}, R_{d4} and R_{d5} are independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R", or R_{d2}, R_{d3}, R_{d4} and R_{d5} are independently selected from C₁₋₆ alkyl, C₁₋₆ haloalkyl, -L'-3- to 10-membered carbocyclyl and -L'-3- to 10-membered heterocyclyl, each of which is optionally substituted with 1, 2, 3, 4, 5, 6, 7 or 8 R_{dd};
or R_{d2} and R_{d3} are taken together with the atoms to which they are attached, or R_{d3} and R_{d4} are taken together with the atoms to which they are attached, or R_{d4} and R_{d5} are taken together with the atoms to which they are attached, to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₆ alkyl, C₁₋₆ haloalkyl, -L"-OR', -L"-NR'R", -L"-C(O)OR', -L"-C(O)NR'R", -L"-S(O)₀₋₂R', -L"-S(O)₂NR'R", -L"-S(O)NR'R", -L"-OC(O)R', -L"-OC(O)NR'R", -L"-3- to 10-membered carbocyclyl and -L"-3- to 10-membered heterocyclyl;
L and L' are independently selected from covalent bond, -O-, -S-, -NR'-, -S(O)₂- and -C(O)-; or, L or L' is C₁₋₆ alkylene, in which one or more methylene units are optionally and independently replaced by -C(R')₂-, -N(R')-, -O-, -C(O)-, -S-, -S(O)-, or -S(O)₂-;
R' and R" are independently selected from H, C₁₋₆ alkyl, C₁₋₆ haloalkyl, -L"-3- to 10-membered carbocyclyl and -L"-3- to 10-membered heterocyclyl; or, R' and R" are taken together with the N atom to which they are attached to form a 3- to 10-membered heterocyclyl;
L" is selected from covalent bond, -O- and -S-; or L" is C₁₋₆ alkylene, in which one or more methylene units are optionally and independently replaced by -O-, -C(O)-, -S-, -S(O)-, or -S(O)₂-;
wherein each of the above groups is optionally deuterated, up to completely deuterated.

In a more specific embodiment, the present disclosure provides a compound of the above formula (II-1), or a stereoisomer, an N-oxide, a hydrate, a solvate, a metabolite, a pharmaceutically acceptable salt, a polymorph, or a prodrug thereof, wherein
V is selected from covalent bond, CR_{C} and CR_{C}R'_{C};
Q is selected from O, S and NR_{A};
Y is selected from covalent bond, O, S and CR₁ₐR_{1b};
R_{A} and R_{B} are independently selected from H, C₁₋₆ alkyl and C₁₋₆ haloalkyl;
R_{C} and R'_{C} are independently selected from H, D, halogen, C₁₋₆ alkyl and C₁₋₆ haloalkyl;
R₁ₐ and R_{1b} are independently selected from H, D, halogen, CN, C₁₋₆ alkyl and C₁₋₆ haloalkyl;
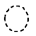 indicates that a double bond may be present or absent; when the double bond is present, it means that the ring where it is located is an aromatic ring, and R'ₑ₁, R'ₑ₂, R'ₑ₃ and R'ₑ₄ are absent; when the double bond is absent, it means that the ring where it is located is not aromatic;
X₂ is C or N;
X₃ is C or N;
X₄ is CRₑ₄, CRₑ₄R'ₑ₄ or N;
Rₑ₁ is selected from H, D, halogen, -CN, -OR', -SR', -NR'R", C₁₋₆ alkyl and C₁₋₆ haloalkyl;
Rₑ₂, Rₑ₃, Rₑ₄ and Rₑ₅ are independently selected from H, D, halogen, C₁₋₆ alkyl and C₁₋₆ haloalkyl;
R'ₑ₁, R'ₑ₂, R'ₑ₃ and R'ₑ₄ are independently selected from H, D, halogen, C₁₋₆ alkyl and C₁₋₆ haloalkyl;
Z₁ is CR_{d1} or N;
Z₂ is C atom or N atom, which is optionally substituted with R_{d2};
Z₃ is C atom or N atom, which is optionally substituted with R_{d3};
Z₄ is C atom or N atom, which is optionally substituted with R_{d4};
Z₅ is selected from covalent bond, CR_{d5} and N;
R_{d1} and R_{d5} are independently selected from H, D, halogen, C₁₋₆ alkyl and C₁₋₆ haloalkyl;
R_{d2}, R_{d3} and R_{d4} are independently selected from H, D, halogen, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, and the C₁₋₆ alkyl, C₁₋₆ haloalkyl, 3- to 7-membered carbocyclyl or 3- to 7-membered heterocyclyl is optionally substituted with 1, 2, 3, 4, 5, 6, 7 or 8 R_{dd};
R_{dd} is independently selected from H, D, halogen, C₁₋₆ alkyl, C₁₋₆ haloalkyl, -L"-OR', -L"-SR' and -L"-NR'R";
R' and R" are independently selected from H, C₁₋₆ alkyl, C₁₋₆ haloalkyl, -L"-3- to 7-membered carbocyclyl and -L"-3- to 7-membered heterocyclyl; or, R' and R" are taken together with the N atom to which they are attached to form a 3- to 7-membered heterocyclyl;
L' is selected from covalent bond, O and C₁₋₆ alkylene;
L" is selected from covalent bond and C₁₋₆ alkylene;
each of the above groups is optionally deuterated, up to completely deuterated.

In a more specific embodiment, the present disclosure provides a compound of the above formula (II-1), or a stereoisomer, an N-oxide, a hydrate, a solvate, a metabolite, a pharmaceutically acceptable salt, a polymorph, or a prodrug thereof, wherein
V is selected from covalent bond, CH, CH₂, CD, CHD and CD₂, alternatively covalent bond;
Q is selected from S and NR_{A}, alternatively NR_{A};
Y is selected from covalent bond, O and CR₁ₐR_{1b}, alternatively selected from covalent bond, O, CH₂, CF₂ and C(CH₃)₂, yet alternatively C(CH₃)₂;
R_{A} and R_{B} are H;
R₁ₐ and R_{1b} are independently selected from H, D, halogen, C₁₋₆ alkyl and C₁₋₆ haloalkyl, alternatively independently selected from H, D, F and Me;
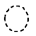 indicates that a double bond may be present or absent; when the double bond is present, it means that the ring where it is located is an aromatic ring, and R'ₑ₁, R'ₑ₂, R'ₑ₃ and R'ₑ₄ are absent; when the double bond is absent, it means that the ring where it is located is not aromatic;
X₂ is C or N;
X₃ is C or N; alternatively, one of X₂ and X₃ is C, and the other one is N;
X₄ is selected from CRₑ₄, CRₑ₄R'ₑ₄ and N;
Rₑ₁ is selected from H, D, halogen, -CN and -OR', alternatively H, D, F, Cl, -CN or -OCH₃;
Rₑ₂ is selected from H, D, C₁₋₆ alkyl and C₁₋₆ haloalkyl, alternatively H, D or Me;
Rₑ₃, Rₑ₄ and Rₑ₅ are independently selected from H and D;
R'ₑ₁, R'ₑ₂, R'ₑ₃ and R'ₑ₄ are independently selected from H and D;
Z₁ is CR_{d1} or N;
Z₂ is C atom or N atom, which is optionally substituted with R_{d2};
Z₃ is C atom or N atom, which is optionally substituted with R_{d3};
Z₄ is C atom or N atom, which is optionally substituted with R_{d4};
Z₅ is selected from covalent bond, CR_{d5} and N; alternatively, the ring where Z₂, Z₃, Z₄ and Z₅ are located is selected from benzene ring, pyridine ring, pyrazole ring and imidazole ring;
R_{d1} and R_{d5} are independently H or D;
R_{d2}, R_{d3} and R_{d4} are independently selected from H, D, C₁₋₆ alkyl, C₁₋₆ haloalkyl, -L'-NR'R'' and -L'-4- to 6-membered heterocyclyl, and the C₁₋₆ alkyl, C₁₋₆ haloalkyl or 4- to 6-membered heterocyclyl is optionally substituted with 1 or 2 R_{dd}; alternatively, R_{d2}, R_{d3} and R_{d4} are independently selected from H, D, 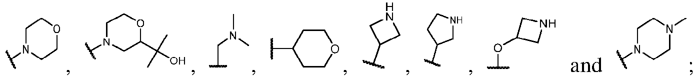 yet alternatively, R_{d2} is selected from H, D, 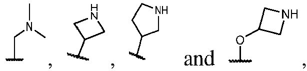 R_{d3} is selected from H, D, 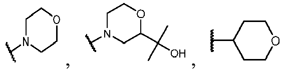 and 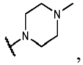 R_{d4} is selected from H, D and 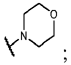
R_{dd} is independently selected from H, D, C₁₋₆ alkyl, C₁₋₆ haloalkyl, -L"-OR' and -L"-NR'R", alternatively independently selected from H, D, Me, N(CH₃)₂ and C(CH₃)₂OH;
L' is selected from covalent bond, O and C₁₋₆ alkylene, alternatively covalent bond, O, or C₁₋₄ alkylene;
L" is selected from covalent bond and C₁₋₆ alkylene, alternatively covalent bond or C₁₋₄ alkylene;
R' and R" are independently selected from H, C₁₋₆ alkyl and C₁₋₆ haloalkyl;
each of the above groups is optionally deuterated, up to completely deuterated.

In a more specific embodiment, the present disclosure provides a compound of the above formula (II), or a stereoisomer, an N-oxide, a hydrate, a solvate, a metabolite, a pharmaceutically acceptable salt, a polymorph, or a prodrug thereof, wherein 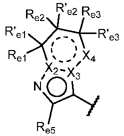 is selected from 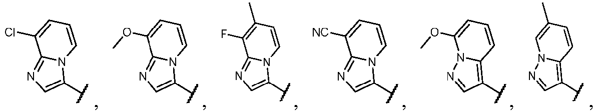 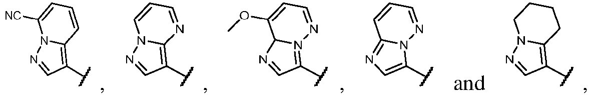 alternatively selected from 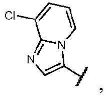 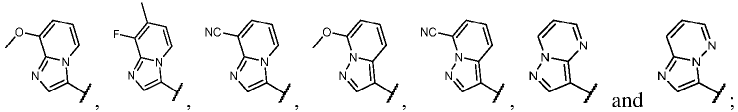 the ring where Z₂, Z₃, Z₄ and Z₅ are located and a substituent thereon are taken together to form the following groups: 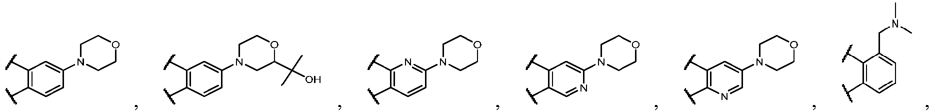 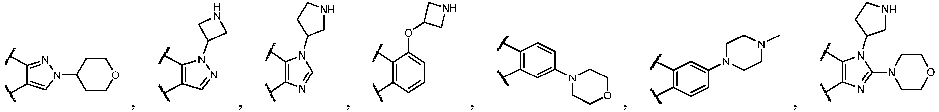 or 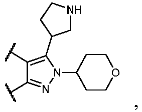 alternatively 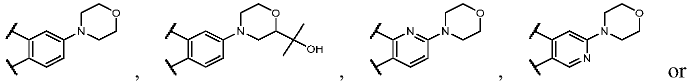 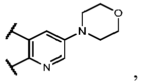 alternatively 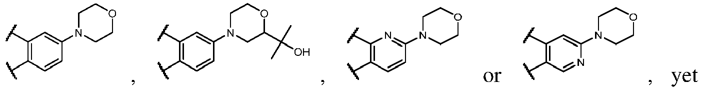 alternatively

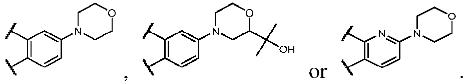

In a more specific embodiment, the present disclosure provides a compound of the above formula (III), formula (IV), or formula (V), or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N;
Rₑ₁ is selected from H, D, halogen, -CN, -OR', -SR', -NR'R", C₁₋₆ alkyl and C₁₋₆ haloalkyl;
Rₑ₂, Rₑ₃, Rₑ₄ and Rₑ₅ are independently selected from H, D, halogen, C₁₋₆ alkyl and C₁₋₆ haloalkyl;
R₁ₐ and R_{1b} are independently H, D, halogen, CN, C₁₋₆ alkyl or C₁₋₆ haloalkyl;
Z₁ is CR_{d1} or N, alternatively CR_{d1};
Z₂ is CR_{d2} or N, alternatively CR_{d2};
Z₄ is CR_{d4} or N, alternatively CR_{d4};
Z₅ is CR_{d5} or N, alternatively CR_{d5};
R_{d2} is selected from H, D, halogen, C₁₋₆ alkyl, C₁₋₆ haloalkyl, -L'-OR', -L'-SR', -L'-NR'R", -L'-3-to 7-membered carbocyclyl and -L'-3- to 7-membered heterocyclyl, and the C₁₋₆ alkyl, C₁₋₆ haloalkyl, 3- to 7-membered carbocyclyl or 3- to 7-membered heterocyclyl is optionally substituted with 1, 2, 3, 4, 5, 6 or 7 R_{dd};
R_{d1}, R_{d4} or R_{d5} is independently selected from H, D, halogen, C₁₋₆ alkyl and C₁₋₆ haloalkyl;
R_{dd} is selected from H, D, halogen, C₁₋₆ alkyl, C₁₋₆ haloalkyl, -L"-OR', -L"-SR' and -L"-NR'R";
L' is selected from covalent bond, -O- and C₁₋₆ alkylene;
L" is selected from covalent bond and C₁₋₆ alkylene;
n=0, 1, 2, 3, 4, 5, 6, 7 or 8;
R' and R" are independently selected from H, C₁₋₆ alkyl, C₁₋₆ haloalkyl, -L"-3- to 7-membered carbocyclyl and -L"-3- to 7-membered heterocyclyl; or, R' and R" are taken together with the N atom to which they are attached to form a 3- to 7-membered heterocyclyl;
wherein each of the above groups is optionally deuterated, up to completely deuterated.

In a more specific embodiment, the present disclosure provides a compound of the above formula (III), formula (IV), or formula (V), or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N;
Rₑ₁ is selected from H, D, halogen, -CN, -OR', C₁₋₆ alkyl and C₁₋₆ haloalkyl;
Rₑ₂, Rₑ₃, Rₑ₄ and Rₑ₅ are independently selected from H, D, halogen, C₁₋₆ alkyl and C₁₋₆ haloalkyl;
R₁ₐ and R_{1b} are independently C₁₋₆ alkyl or C₁₋₆ haloalkyl;
Z₁ is CR_{d1} or N, alternatively CR_{d1};
Z₂ is CR_{d2} or N, alternatively CR_{d2};
Z₄ is CR_{d4} or N, alternatively CR_{d4};
Z₅ is CR_{d5} or N, alternatively CR_{d5}; alternatively, the ring where Z₂, Z₄ and Z₅ are located is selected from benzene ring and pyridine ring, alternatively benzene ring;
R_{d1}, R_{d2}, R_{d4} or R_{d5} is independently selected from H, D, halogen, C₁₋₆ alkyl and C₁₋₆ haloalkyl;
R_{dd} is selected from H, D, halogen, C₁₋₆ alkyl, C₁₋₆ haloalkyl, -L"-OR' and -L"-NR'R";
L" is selected from covalent bond and C₁₋₆ alkylene;
n=0, 1, 2, 3, 4, 5, 6, 7 or 8;
R' and R" are independently selected from H, C₁₋₆ alkyl and C₁₋₆ haloalkyl;
each of the above groups is optionally deuterated, up to completely deuterated.

In a more specific embodiment, the present disclosure provides a compound of the above formula (III), or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N;
Rₑ₁ is selected from H, D, halogen and -OR', alternatively H, D or halogen;
Rₑ₂ is selected from H, D, C₁₋₆ alkyl and C₁₋₆ haloalkyl, alternatively H or D.
Rₑ₃, Rₑ₄ and Rₑ₅ are independently H or D;
R₁ₐ and R_{1b} are independently C₁₋₃ alkyl or C₁₋₃ haloalkyl, alternatively Me;
Z₁, Z₂, Z₄ and Z₅ are CH or CD;
R_{dd} is selected from H, D, C₁₋₆ alkyl, C₁₋₆ haloalkyl and -L" -OR';
L" is selected from covalent bond and C₁₋₆ alkylene;
n=0, 1, 2, 3, 4, 5, 6, 7 or 8;
R' is selected from H, C₁₋₆ alkyl and C₁₋₆ haloalkyl;
each of the above groups is optionally deuterated, up to completely deuterated.

In a more specific embodiment, the present disclosure provides a compound of the above formula (III), or a stereoisomer, an N-oxide, a hydrate, a solvate, a metabolite, a pharmaceutically acceptable salt, a polymorph, or a prodrug thereof, wherein
X₂ is C or N;
X₃ is C or N; alternatively, one of X₂ and X₃ is C, and the other one is N;
X₄ is CRₑ₄ or N;
Rₑ₁ is selected from H, F, Cl and OCH₃, alternatively H, F or Cl;
Rₑ₂ is selected from H and Me, alternatively H;
Rₑ₃, Rₑ₄ and Rₑ₅ are H; alternatively, 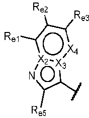 is selected from 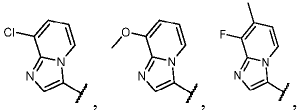 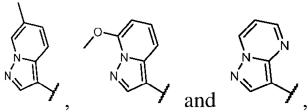 alternatively selected from 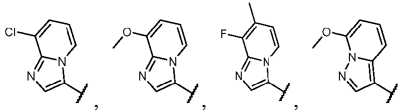 and 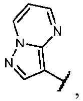 yet alternatively selected from 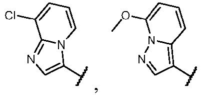 and 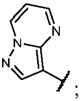
R₁ₐ and R_{1b} are Me;
Z₁, Z₂, Z₄ and Z₅ are CH;
R_{dd} is H or -L''-OH, alternatively H or C(CH₃)₂OH;
L'' is selected from covalent bond and C₁₋₄ alkylene;
n=0 or 1; alternatively 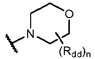 is

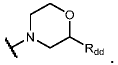

In a more specific embodiment, the present disclosure provides a compound of the above formula (IV), or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N;
Rₑ₁ is selected from H, D, halogen, CN and OR', alternatively selected from H, D, F, CN and OR';
Rₑ₂ is selected from H, D, C₁₋₆ alkyl and C₁₋₆ haloalkyl;
Rₑ₃, Rₑ₄ and Rₑ₅ are independently H or D;
R₁ₐ and R_{1b} are independently C₁₋₃ alkyl or C₁₋₃ haloalkyl, alternatively Me;
Z₁ is selected from CH, CD and N, alternatively CH or CD;
Z₂, Z₄ and Z₅ are independently selected from CH, CD and N;
R_{dd} is selected from H, D, C₁₋₆ alkyl, C₁₋₆ haloalkyl and -L''-OR';
L'' is selected from covalent bond and C₁₋₆ alkylene;
n=0, 1, 2, 3, 4, 5, 6, 7 or 8;
R' is selected from H, C₁₋₆ alkyl and C₁₋₆ haloalkyl;
each of the above groups is optionally deuterated, up to completely deuterated.

In a more specific embodiment, the present disclosure provides a compound of the above formula (IV), or a stereoisomer, an N-oxide, a hydrate, a solvate, a metabolite, a pharmaceutically acceptable salt, a polymorph, or a prodrug thereof, wherein
X₂ is C or N;
X₃ is C or N; alternatively, one of X₂ and X₃ is C, and the other one is N;
X₄ is CRₑ₄ or N;
Rₑ₁ is selected from H, F, Cl, CN and OCH₃, alternatively selected from H, F, CN and OCH₃;
Rₑ₂ is selected from H and Me;
Rₑ₃, Rₑ₄ and Rₑ₅ are H; alternatively, 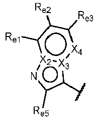 is selected from 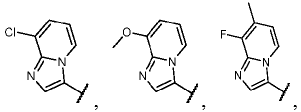 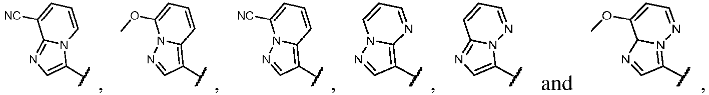 alternatively selected from 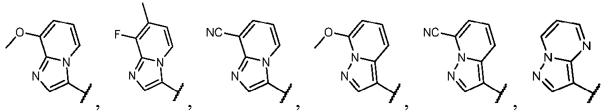 and 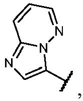 yet alternatively selected from 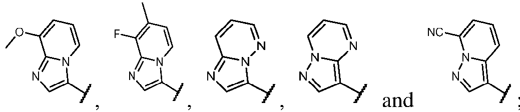 ; yet alternatively selected from 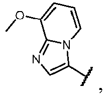 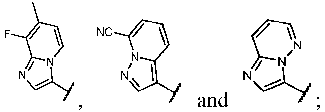
R₁ₐ and R_{1b} are Me;
Z₁ is CH;
Z₂, Z₄ and Z₅ are independently CH or N; alternatively Z₂ and Z₄ are independently CH or N, and Z₅ is CH; alternatively Z₂ is CH or N, and Z₄ and Z₅ are CH;
R_{dd} is H or -L" -OH, alternatively H or C(CH₃)₂OH;
L'' is selected from covalent bond and C₁₋₄ alkylene;
n=0 or 1; alternatively 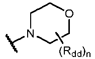 is

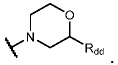

In a more specific embodiment, the present disclosure provides a compound of the above formula (V), or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N, alternatively CRₑ₄;
Rₑ₁ is selected from H, D, halogen and OR', alternatively OR';
Rₑ₂ is selected from H, D, C₁₋₆ alkyl and C₁₋₆ haloalkyl, alternatively H or D;
Rₑ₃, Rₑ₄ and Rₑ₅ are independently H or D;
R₁ₐ and R_{1b} are independently C₁₋₃ alkyl or C₁₋₃ haloalkyl, alternatively Me;
Z₁ is N, CH or CD, alternatively CH or CD;
Z₂, Z₄ and Z₅ are independently CH or CD;
R_{dd} is selected from H, D, C₁₋₆ alkyl, C₁₋₆ haloalkyl and -L" -OR', alternatively H or D;
L" is selected from covalent bond and C₁₋₆ alkylene;
n=0, 1, 2, 3, 4, 5, 6, 7 or 8;
R' is selected from H, C₁₋₆ alkyl and C₁₋₆ haloalkyl, alternatively C₁₋₆ alkyl or C₁₋₆ haloalkyl;
each of the above groups is optionally deuterated, up to completely deuterated.

In a more specific embodiment, the present disclosure provides a compound of the above formula (V), or a stereoisomer, an N-oxide, a hydrate, a solvate, a metabolite, a pharmaceutically acceptable salt, a polymorph, or a prodrug thereof, wherein
X₂ is C or N;
X₃ is C or N; alternatively, one of X₂ and X₃ is C, and the other one is N;
X₄ is CRₑ₄ or N, alternatively CRₑ₄;
Rₑ₁ is selected from H, F, Cl and OCH₃, alternatively OCH₃;
Rₑ₂ is H;
Rₑ₃, Rₑ₄ and Rₑ₅ are H; alternatively, 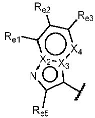 is selected from 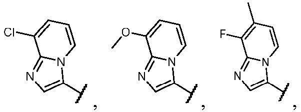 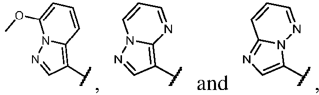 alternatively 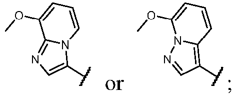
R₁ₐ and R_{1b} are Me;
Z₁ is CH or N;
Z₂, Z₄ and Z₅ are CH;
R_{dd} is H;
n=0.

In a more specific embodiment, the present disclosure provides a compound of the above formula (II), or a stereoisomer, an N-oxide, a hydrate, a solvate, a metabolite, a pharmaceutically acceptable salt, a polymorph, or a prodrug thereof, 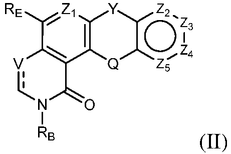 wherein
R_{E} is selected from -L-3- to 10-membered carbocyclyl, -L-3- to 10-membered heterocyclyl, -L-C₆₋₁₀ aryl and -L-5- to 10-membered heteroaryl, alternatively -L-C₆₋₁₀ aryl or -L-5- to 10-membered heteroaryl, each of which is optionally substituted with 1, 2, 3, 4 or 5 Rₑ;
Rₑ is independently selected from H, D, halogen, CN, oxo, C₁₋₆ alkyl, C₁₋₆ haloalkyl, C₂₋₆ alkenyl, C₂₋₆ alkynyl, -L'-OR', -L'-SR', -L'-NR'R", -L'-3- to 7-membered carbocyclyl and -L'-3- to 7-membered heterocyclyl;
Y is selected from O, S and CR₁ₐR_{1b};
R₁ₐ and R_{1b} are independently selected from H, D, halogen, C₁₋₆ alkyl and C₁₋₆ haloalkyl;
each of Q, V, R_{B} and Z₁ is as defined herein;
Z₂ is selected from CR_{d2} and N;
Z₃ is selected from CR_{d3} and N;
Z₄ is selected from CR_{d4} and N;
Z₅ is selected from covalent bond, CR_{d5} and N, alternatively CR_{d5} or N;
one of R_{d3} and R_{d4} is -NR'_{d6}C(O)R_{d6}, -L'-3- to 7-membered carbocyclyl or -L'-3- to 7-membered heterocyclyl, and the other one, R_{d2} and R_{d5} are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl;
R_{d2}, R_{d3}, R_{d4} and R_{d5} are optionally substituted with 1, 2, 3 or 4 R_{dd};
R_{dd} is independently selected from H, D, oxo, halogen, CN, C₁₋₆ alkyl, C₁₋₆ haloalkyl, -L" -OR', -L"-SR', -L"-NR'R", -L"-3- to 7-membered carbocyclyl and -L"-3- to 7-membered heterocyclyl;
or two non-adjacent R_{dd} are taken together to form a C₁₋₆ alkylene, alternatively form a C₁₋₄ alkylene, which is optionally substituted with 1, 2, 3 or 4 substituents selected from: H, D, halogen, CN, C₁₋₆ alkyl and C₁₋₆ haloalkyl;
R_{d6} is selected from H, C₁₋₆ alkyl, C₁₋₆ haloalkyl, -L"-OR', -L"-SR', -L"-NR'R", -L"-3- to 7-membered carbocyclyl, -L"-3- to 7-membered heterocyclyl and -L"-Y₃-C₁₋₆ alkylene-R₄;
Y₃ is selected from O, S and NR₃;
R'_{d6} and R₃ are independently selected from H, C₁₋₆ alkyl and C₁₋₆ haloalkyl;
R₄ is selected from -OR', -SR' and -NR'R";
L is independently selected from covalent bond, -O-, -S-, -NR'- and C₁₋₆ alkylene;
L' is independently selected from covalent bond and C₁₋₆ alkylene;
each of L and L' is optionally substituted with 1, 2, 3 or 4 groups selected from: C₁₋₆ alkyl, C₁₋₆ haloalkyl, -L_{d}-3- to 7-membered carbocyclyl and -L_{d}-3- to 7-membered heterocyclyl;
L" is independently selected from covalent bond and C₁₋₆ alkylene, each of which is optionally substituted with 1, 2, 3 or 4 R, and R is independently selected from H, D, C₁₋₆ alkyl, C₁₋₆ haloalkyl, -L_{d}-3- to 7-membered carbocyclyl and -L_{d}-3- to 7-membered heterocyclyl, or two R are taken together with the C atom to which they are jointly attached to form a 3- to 7-membered carbocyclylene or a 3- to 7-membered heterocyclylene;
L_{d} is independently selected from covalent bond and C₁₋₆ alkylene, alternatively selected from covalent bond and C₁₋₄ alkylene;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl;
wherein each of the above groups is optionally deuterated, up to completely deuterated.

In a more specific embodiment, the present disclosure provides a compound of the above formula (II), or a stereoisomer, an N-oxide, a hydrate, a solvate, a metabolite, a pharmaceutically acceptable salt, a polymorph, or a prodrug thereof, wherein
R_{E} is -L-5- to 10-membered heteroaryl, alternatively 5- to 10-membered heteroaryl; alternatively, the heteroaryl in R_{E} is selected from pyridyl, pyridonyl, pyrazolyl, imidazolyl, oxazolyl, triazolyl, imidazopyridyl, imidazopyridazinyl, imidazopyrazinyl, imidazopyrimidinyl, pyrazolopyridyl, pyrazolopyrimidinyl, pyrazolopyrazinyl, triazolopyridyl, imidazopyrrolidinyl, imidazocyclohexyl, imidazopiperidyl, imidazoazepanyl, dihydroimidazooxazinyl, imidazopyrazinonyl, imidazodiazacyclohexyl, imidazopiperazinonyl, pyrazolopiperidyl, pyrazolodiazacyclohexyl, dihydropyrazolooxazinyl, triazolopiperidyl, dihydropyridooxazinyl, pyrrolopyridyl and oxazolopyridyl, each of which is optionally substituted with 1, 2, 3, 4 or 5 Rₑ;
Rₑ is independently selected from H, D, halogen, CN, oxo, C₁₋₆ alkyl, C₁₋₆ haloalkyl, C₂₋₆ alkynyl, -OR', -NR'R", 3- to 7-membered carbocyclyl and 3- to 7-membered heterocyclyl, alternatively selected from H, D, halogen, CN, oxo, C₁₋₆ alkyl, C₁₋₆ haloalkyl, C₂₋₆ alkynyl, -OR' and -NR'R";
Y is O or CR₁ₐR_{1b}, alternatively CR₁ₐR_{1b};
R₁ₐ and R_{1b} are independently selected from C₁₋₆ alkyl and C₁₋₆ haloalkyl, alternatively selected from Me and Et;
each of Q, V, R_{B} and Z₁ is as defined herein;
Z₂ is selected from CR_{d2} and N;
Z₃ is selected from CR_{d3} and N;
Z₄ is selected from CR_{d4} and N;
Z₅ is selected from CR_{d5} and N;
one of Rₐ₃ and R_{d4} is -NR'_{d6}C(O)R_{d6} or -L'-3- to 7-membered heterocyclyl, alternatively -L'-3- to 7-membered heterocyclyl, and the other one, R_{d2} and R_{d5} are independently selected from H, D, halogen, CN, C₁₋₆ alkyl and C₁₋₆ haloalkyl;
R_{d2}, R_{d3}, R_{d4} and R_{d5} are optionally substituted with 1, 2, 3 or 4 R_{dd};
R_{dd} is independently selected from H, D, oxo, C₁₋₆ alkyl, C₁₋₆ haloalkyl, -L"-OR', -L"-NR'R", -L"-3- to 7-membered carbocyclyl and -L''-3- to 7-membered heterocyclyl;
or two non-adjacent R_{dd} are taken together to form a C₁₋₃ alkylene, which is optionally substituted with 1, 2, 3 or 4 substituents selected from: H, D, halogen, CN, C₁₋₆ alkyl and C₁₋₆ haloalkyl;
R_{d6} is selected from C₁₋₆ alkyl, C₁₋₆ haloalkyl, -L"-3- to 7-membered carbocyclyl, -L"-3- to 7-membered heterocyclyl and -L"- Y₃-C₁₋₄ alkylene-R₄;
Y₃ is selected from O and NR₃, alternatively O;
R'_{d6} and R₃ are independently selected from H, C₁₋₆ alkyl and C₁₋₆ haloalkyl, alternatively H, C₁₋₃ alkyl or C₁₋₃ haloalkyl;
R₄ is selected from -OR' and -NR'R", alternatively -OR';
L is independently selected from covalent bond, -NR'- and C₁₋₄ alkylene, alternatively covalent bond or -NR'-; alternatively, L is a covalent bond, -NH- or -CH(CH₃)-, alternatively covalent bond or -NH-;
L' is independently selected from covalent bond and C₁₋₄ alkylene;
each of L and L' is optionally substituted with 1, 2, 3 or 4 groups selected from: C₁₋₆ alkyl and C₁₋₆ haloalkyl;
L" is independently selected from covalent bond and C₁₋₆ alkylene, each of which is optionally substituted with 1, 2, 3 or 4 R, and R is independently selected from H, D, C₁₋₆ alkyl and C₁₋₆ haloalkyl, or two R are taken together with the C atom to which they are jointly attached to form a 3- to 7-membered carbocyclylene, alternatively form a 3- to 5-membered carbocyclylene;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl, alternatively selected from H, C₁₋₆ alkyl and C₁₋₆ haloalkyl.

In a more specific embodiment, the present disclosure provides a compound of the above formula (II) or formula (II'), or a stereoisomer, an N-oxide, a hydrate, a solvate, a metabolite, a pharmaceutically acceptable salt, a polymorph, or a prodrug thereof, 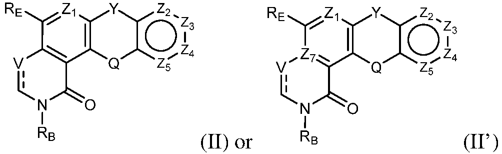
R_{E} is as defined herein;
Rₑ is independently selected from H, D, halogen, CN, oxo, C₁₋₆ alkyl, C₁₋₆ haloalkyl, C₂₋₆ alkenyl, C₂₋₆ alkynyl, -L'-OR', -L'-SR', -L'-NR'R", -L'-3- to 7-membered carbocyclyl and -L'-3- to 7-membered heterocyclyl, alternatively selected from H, D, halogen, CN, oxo, C₁₋₆ alkyl, C₁₋₆ haloalkyl, C₂₋₆ alkynyl, -OR', -NR'R", 3- to 7-membered carbocyclyl and 3- to 7-membered heterocyclyl, alternatively selected from H, D, halogen, CN, oxo, C₁₋₆ alkyl, C₁₋₆ haloalkyl, C₂₋₆ alkynyl, -OR' and -NR'R", each of which is optionally substituted with 1, 2 or 3 Rₑₛ;
or two geminal Rₑ are taken together with the atom to which they are jointly attached to form a 3- to 7-membered carbocyclyl or a 3- to 7-membered heterocyclyl, alternatively form a 3- to 7-membered heterocyclyl, which is optionally substituted with 1, 2 or 3 Rₑₛ;
Rₑₛ is independently selected from H, D, halogen, C₁₋₆ alkyl, C₁₋₆ haloalkyl, -OR' and -NR'R";
each of Q, V, R_{B}, Z₁ and Z₇ is as defined herein;
Z₂ is selected from CR_{d2} and N;
Z₃ is selected from CR_{d3} and N;
Z₄ is selected from CR_{d4} and N;
Z₅ is selected from covalent bond, CR_{d5} and N, alternatively CR_{d5} or N;
one of R_{d3} and R_{d4} is -NR'_{d6}C(O)R_{d6}, -C(O)NR_{d7}R'_{d7}, -L'-3- to 7-membered carbocyclyl, or -L'-3-to 7-membered heterocyclyl, alternatively -NR'_{d6}C(O)R_{d6}, -C(O)NR_{d7}R'_{d7} or -L'-3- to 7-membered heterocyclyl, and the other one, R_{d2} and R_{d5} are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, alternatively selected from H, D, halogen, CN, C₁₋₆ alkyl, C₁₋₆ haloalkyl, OR' and NR'R";
R_{d2}, R_{d3}, R_{d4} and R_{d5} are optionally substituted with 1, 2, 3 or 4 R_{dd};
R_{dd} is independently selected from H, D, oxo, halogen, CN, C₁₋₆ alkyl, C₁₋₆ haloalkyl, -L"-OR', -L"-SR', -L"-NR'R", -L"-3- to 7-membered carbocyclyl and -L"-3- to 7-membered heterocyclyl, alternatively selected from H, D, oxo, C₁₋₆ alkyl, C₁₋₆ haloalkyl, -L" -OR', -L"-NR'R", -L"-3- to 7-membered carbocyclyl and -L"-3- to 7-membered heterocyclyl;
or CR_{dd}R_{dd} forms a 3- to 7-membered carbocyclylene or a 3- to 7-membered heterocyclylene, which is optionally substituted with 1, 2 or 3 groups selected from: H, D, halogen, C₁₋₆ alkyl, C₁₋₆ haloalkyl, -OR' and -NR'R", alternatively optionally substituted with one -OR';
or two non-adjacent R_{dd} are taken together to form a C₁₋₆ alkylene, alternatively form a C₁₋₄ alkylene, alternatively form a C₁₋₃ alkylene, which is optionally substituted with 1, 2, 3 or 4 substituents selected from: H, D, halogen, CN, C₁₋₆ alkyl and C₁₋₆ haloalkyl;
or two adjacent R_{dd} are taken together with the atoms to which they are attached to form a 3- to 7-membered carbocyclylene, a 3- to 7-membered heterocyclylene, a phenylene, or a 5- to 6-membered heteroarylene, alternatively form a 3- to 7-membered heterocyclylene or a 5- to 6-membered heteroarylene, alternatively form a 3- to 7-membered heterocyclylene, which is optionally substituted with 1, 2, 3 or 4 substituents selected from: H, D, halogen, CN, C₁₋₆ alkyl, C₁₋₆ haloalkyl, -OR' and -NR'R";
R_{d7} and R'_{d7} are independently selected from H, C₁₋₆ alkyl and C₁₋₆ haloalkyl;
the remaining variables are as defined herein;
wherein each of the above groups is optionally deuterated, up to completely deuterated.

In a more specific embodiment, the present disclosure provides a compound of the above formula (VI) or formula (VI-1), or a stereoisomer, an N-oxide, a hydrate, a solvate, a metabolite, a pharmaceutically acceptable salt, a polymorph, or a prodrug thereof, 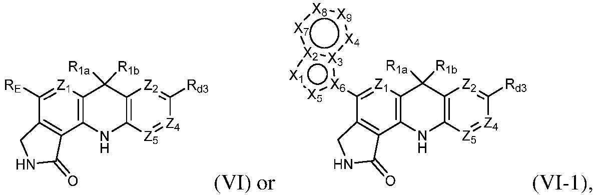 wherein
R_{E} is as defined herein;
X₁ is CRₑ₆, N, O, S or NR'ₑ₆;
X₂ is C or N;
X₃ is C or N;
X₄ is CRₑ₄ or N;
X₅ is CRₑ₅, N, O, S or NR'ₑ₅;
X₆ is C or N;
X₇ is CRₑ₁ or N;
X₅ is CRₑ₂ or N;
X₉ is CRₑ₃ or N;
Rₑ₁ and Rₑ₂ are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl;
Rₑ₃, Rₑ₄, Rₑ₅ and Rₑ₆ are independently selected from H, D, halogen, CN, C₁₋₆ alkyl and C₁₋₆ haloalkyl;
R'ₑ₅ and R'ₑ₆ are independently selected from H, C₁₋₆ alkyl and C₁₋₆ haloalkyl;
R₁ₐ and R_{1b} are independently H, D, halogen, C₁₋₆ alkyl or C₁₋₆ haloalkyl;
Z₁ is selected from CR_{d1} and N;
Z₂ is selected from CR_{d2} and N;
Z₄ is selected from CR_{d4} and N;
Z₅ is selected from CR_{d5} and N;
R_{d1} is selected from H, D, halogen, CN, C₁₋₆ alkyl and C₁₋₆ haloalkyl;
R_{d2}, R_{d4} and R_{d5} are independently selected from H, D, halogen, CN, C₁₋₆ alkyl, C₁₋₆ haloalkyl, -L'-OR', -L'-SR', -L'-NR'R'', -L'-3- to 7-membered carbocyclyl and -L'-3- to 7-membered heterocyclyl;
R_{d3} is selected from 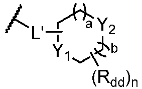 and -NR'_{d6}C(O)R_{d6};
a and b are independently 0, 1, 2 or 3, alternatively a+b≤5;
L' is independently selected from covalent bond and C₁₋₆ alkylene, each of which is optionally substituted with 1, 2, 3 or 4 groups selected from: C₁₋₆ alkyl and C₁₋₆ haloalkyl;
Y₁ and Y₂ are independently selected from CR_{dd}R_{dd}, O, S and NR'_{dd}, or when Y₁ is connected to L', Y₁ is CR_{dd} or N;
R_{dd} is independently selected from H, D, oxo, halogen, CN, C₁₋₆ alkyl, C₁₋₆ haloalkyl, -L" -OR', -L"-SR', -L"-NR'R", -L"-3- to 7-membered carbocyclyl and -L"-3- to 7-membered heterocyclyl;
or two non-adjacent R_{dd} are taken together to form a C₁₋₄ alkylene, or R'_{dd} and a non-adjacent R_{dd} are taken together to form a C₁₋₄ alkylene, or two R'_{dd} are taken together to form a C₁₋₄ alkylene, wherein the C₁₋₄ alkylene is optionally substituted with 1, 2, 3 or 4 substituents selected from: H, D, halogen, CN, C₁₋₆ alkyl and C₁₋₆ haloalkyl;
R'_{dd} is independently selected from H, C₁₋₆ alkyl, C₁₋₆ haloalkyl, -L"-OR', -L"-SR', -L"-NR'R", -L"-3- to 7-membered carbocyclyl and -L''-3- to 7-membered heterocyclyl;
n=0, 1, 2, 3 or 4;
R_{d6} is selected from H, C₁₋₆ alkyl, C₁₋₆ haloalkyl, -L"-OR', -L"-SR', -L"-NR'R", -L"-3- to 7-membered carbocyclyl, -L"-3- to 7-membered heterocyclyl and -L"-Y₃-C₁₋₆ alkylene-R₄;
L" is independently selected from covalent bond and C₁₋₆ alkylene, each of which is optionally substituted with 1, 2, 3 or 4 R, and R is independently selected from H, D, C₁₋₆ alkyl, C₁₋₆ haloalkyl, -L_{d}-3- to 7-membered carbocyclyl and -L_{d}-3- to 7-membered heterocyclyl, or two R are taken together with the C atom to which they are jointly attached to form a 3- to 7-membered carbocyclylene or a 3- to 7-membered heterocyclylene;
L_{d} is independently selected from covalent bond and C₁₋₆ alkylene, alternatively selected from covalent bond and C₁₋₄ alkylene;
Y₃ is selected from O, S and NR₃;
R'_{d6} and R₃ are independently selected from H, C₁₋₆ alkyl and C₁₋₆ haloalkyl;
R₄ is selected from -OR', -SR' and -NR'R";
each of R' and R" is independently selected from H, C₁₋₆ alkyl, C₁₋₆ haloalkyl, -L_{d}-3- to 7-membered carbocyclyl and -L_{d}-3- to 7-membered heterocyclyl;
wherein each of the above groups is optionally deuterated, up to completely deuterated.

It should be understood that, when Y₁ or Y₂ is CR_{dd}R_{dd}, and one of the R_{dd} is oxo, the other R_{dd} should be absent.

In a more specific embodiment, the present disclosure provides a compound of the above formula (VI) or formula (VI-1), or a stereoisomer, an N-oxide, a hydrate, a solvate, a metabolite, a pharmaceutically acceptable salt, a polymorph, or a prodrug thereof,
wherein R_{E} is as defined herein;
X₁ is CRₑ₆, N or NR'ₑ₆, alternatively N;
X₂ is C or N;
X₃ is C or N;
X₄ is CRₑ₄ or N;
X₅ is CRₑ₅, N or NR'ₑ₅, alternatively CRₑ₅ or N;
X₆ is C or N, alternatively C;
X₇ is CRₑ₁ or N;
X₅ is CRₑ₂ or N;
X₉ is CRₑ₃ or N;
Rₑ₁ and Rₑ₂ are independently selected from H, D, halogen, CN, C₁₋₆ alkyl, C₁₋₆ haloalkyl, -OR', -NR'R", 3- to 7-membered carbocyclyl and 3- to 7-membered heterocyclyl, alternatively selected from H, D, halogen, CN, C₁₋₆ alkyl, C₁₋₆ haloalkyl, -OR' and -NR'R";
Rₑ₃, Rₑ₄, Rₑ₅ and Rₑ₆ are independently H, D, halogen, C₁₋₆ alkyl or C₁₋₆ haloalkyl;
R'ₑ₅ and R'ₑ₆ are independently selected from H, C₁₋₆ alkyl and C₁₋₆ haloalkyl;
R₁ₐ and R_{1b} are independently C₁₋₆ alkyl or C₁₋₆ haloalkyl;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1}, R_{d2}, R_{d4} and R_{d5} are independently selected from H, D, halogen, CN, C₁₋₆ alkyl and C₁₋₆ haloalkyl;
R_{d3} is selected from 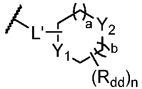 and -NR'_{d6}C(O)R_{d6};
a and b are independently 0, 1 or 2; alternatively a+b≤3;
L' is independently selected from covalent bond and C₁₋₄ alkylene;
Y₁ is CR_{dd}R_{dd} or NR'_{dd}; or when Y₁ is connected to L', Y₁ is CH, CD or N;
Y₂ is CR_{dd}R_{dd}, O or NR'_{dd}; alternatively, at least one of Y₁ and Y₂ is a heteroatom group;
R_{dd} is independently selected from H, D, oxo, C₁₋₆ alkyl, C₁₋₆ haloalkyl, -L"-OR', -L"-NR'R", -L"-3- to 7-membered carbocyclyl and -L''-3- to 7-membered heterocyclyl;
or two non-adjacent R_{dd} are taken together to form a C₁₋₃ alkylene, which is optionally substituted with 1, 2, 3 or 4 substituents selected from: H, D, halogen, CN, C₁₋₆ alkyl and C₁₋₆ haloalkyl;
R'_{dd} is independently selected from H, C₁₋₆ alkyl, C₁₋₆ haloalkyl, -C₁₋₆ alkylene-OR', -C₁₋₆ alkylene-NR'R", -L"-3- to 7-membered carbocyclyl and -L"-3- to 7-membered heterocyclyl, alternatively selected from H, C₁₋₆ alkyl, C₁₋₆ haloalkyl, -L"-3- to 7-membered carbocyclyl and -L"-3- to 7-membered heterocyclyl;
n=0, 1, 2, 3 or 4;
R_{d6} is selected from C₁₋₆ alkyl, C₁₋₆ haloalkyl, -L"-3- to 7-membered carbocyclyl, -L"-3- to 7-membered heterocyclyl and -L"- Y₃-C₁₋₄ alkylene-R₄, alternatively not -L"- Y₃-C₁₋₄ alkylene-R₄;
L" is independently selected from covalent bond and C₁₋₆ alkylene, each of which is optionally substituted with 1, 2, 3 or 4 R, and R is independently selected from H, D, C₁₋₆ alkyl and C₁₋₆ haloalkyl, or two R are taken together with the C atom to which they are jointly attached to form a 3- to 7-membered carbocyclylene;
Y₃ is selected from O and NR₃;
R'_{d6} and R₃ are independently selected from H, C₁₋₆ alkyl and C₁₋₆ haloalkyl;
R₄ is selected from -OR' and -NR'R";
each of R' and R" is independently selected from H, C₁₋₆ alkyl, C₁₋₆ haloalkyl, 3- to 7-membered carbocyclyl and 3- to 7-membered heterocyclyl.

In some embodiments of the present disclosure, at most five of X₁-X₉ are heteroatoms, alternatively at most four are heteroatoms, alternatively at most three are heteroatoms.

In some embodiments of the present disclosure, one of X₂ and X₃ is N, and the other one is C.

In some embodiments of the present disclosure, at most only one of X₄ and X₇-X₉ is N.

In some embodiments of the present disclosure, at most two of Z₂, Z₄ and Z₅ are N, alternatively at most one is N.

In a more specific embodiment, the present disclosure provides a compound of the above formula (VI) or formula (VI-1), or a stereoisomer, an N-oxide, a hydrate, a solvate, a metabolite, a pharmaceutically acceptable salt, a polymorph, or a prodrug thereof, wherein R_{E} is as defined herein;
X₁ is N;
X₂ is C or N;
X₃ is C or N;
X₄ is CRₑ₄ or N, alternatively N;
X₅ is CRₑ₅ or N, alternatively CRₑ₅;
X₆ is C;
X₇ is CRₑ₁ or N, alternatively CRₑ₁;
X₈ is CRₑ₂ or N, alternatively N;
X₉ is CRₑ₃ or N, alternatively CRₑ₃;
Rₑ₁ and Rₑ₂ are independently selected from H, D, halogen, CN, C₁₋₆ alkyl, C₁₋₆ haloalkyl, -OR', -O-3- to 7-membered carbocyclyl and 3- to 7-membered heterocyclyl, alternatively selected from H, D, halogen, CN, C₁₋₆ alkyl, C₁₋₆ haloalkyl and -OR';
Rₑ₃, Rₑ₄ and Rₑ₅ are independently H or D;
R'ₑ₅ is selected from H, C₁₋₃ alkyl and C₁₋₃ haloalkyl;
R₁ₐ and R_{1b} are independently C₁₋₃ alkyl or C₁₋₃ haloalkyl;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is H or D;
R_{d2}, R_{d4} and R_{d5} are independently selected from H, D, halogen, C₁₋₃ alkyl and C₁₋₃ haloalkyl, alternatively R_{d2} and R_{d5} are independently selected from H, D and halogen, alternatively R_{d5} is H or D;
R_{d3} is selected from 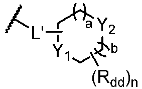 and -NR'_{d6}C(O)R_{d6}; alternatively, 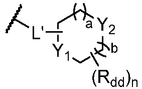 is 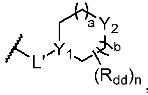 , alternatively 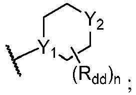
a and b are independently 0, 1 or 2, alternatively a and b are 1;
L' is independently selected from covalent bond and C₁₋₂ alkylene, alternatively selected from covalent bond and methylene;
Y₁ is NR'_{dd}, alternatively NH; or when Y₁ is connected to L', Y₁ is CH, CD or N;
Y₂ is CR_{dd}R_{dd}, O or NR'_{dd};
R_{dd} is independently selected from H, D, oxo, C₁₋₆ alkyl, C₁₋₆ haloalkyl, -L" -OR', -L''-NR'R'' and -L"-3- to 7-membered carbocyclyl, alternatively selected from H, D, oxo, C₁₋₆ alkyl, C₁₋₆ haloalkyl, -L"-OR' and -L"-NR'R", alternatively selected from H, D, C₁₋₆ alkyl, C₁₋₆ haloalkyl and -L"-OR';
or two non-adjacent R_{dd} are taken together to form a C₁₋₂ alkylene;
R'_{dd} is independently selected from H, C₁₋₆ alkyl, C₁₋₆ haloalkyl, -C₁₋₄ alkylene-OR', -C₁₋₄ alkylene-NR'R" and -L"-3- to 7-membered carbocyclyl, alternatively selected from H, C₁₋₆ alkyl, C₁₋₆ haloalkyl and -L"-3- to 7-membered carbocyclyl;
n=0, 1, 2, 3 or 4;
R_{d6} is selected from C₁₋₆ alkyl, C₁₋₆ haloalkyl, -L"-3- to 7-membered carbocyclyl and -L"-O-C₁₋₄ alkylene-OR', alternatively not -L"-O-C₁₋₄ alkylene-OR';
R'_{d6} is selected from H, C₁₋₆ alkyl and C₁₋₆ haloalkyl;
L" is independently selected from covalent bond, C₁₋₄ alkylene and -C(R)₂-, alternatively selected from covalent bond and C₁₋₄ alkylene, wherein two R are taken together with the C atom to which they are jointly attached to form a 3- to 5-membered carbocyclylene, alternatively form 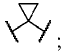
each of R' and R" is independently selected from H, C₁₋₆ alkyl and C₁₋₆ haloalkyl.

In some specific embodiments of the present disclosure, when Y₁ is N and Y₂ is O, L' is a covalent bond and R_{dd} is not oxo.

In some specific embodiments of the present disclosure, when Rₑ₂ is -O-C₁₋₆ alkyl, Rₑ₂ is -OMe, alternatively, when Rₑ₂ is -O-C₁₋₆ haloalkyl, Rₑ₂ is -O-halomethyl.

In some specific embodiments of the present disclosure, at least one of R_{d2} and R_{d4} is H or D, alternatively at least one is H.

In a more specific embodiment, the present disclosure provides a compound of the above formula (VI) or formula (VI-1), or a stereoisomer, an N-oxide, a hydrate, a solvate, a metabolite, a pharmaceutically acceptable salt, a polymorph, or a prodrug thereof, wherein R_{E} is as defined herein;
X₁ is N;
X₂ is C or N;
X₃ is C or N;
X₄ is CRₑ₄ or N, alternatively N;
X₅ is CRₑ₅ or N, alternatively CRₑ₅;
X₆ is C;
X₇ is CRₑ₁ or N, alternatively CRₑ₁;
X₈ is CRₑ₂ or N, alternatively N;
X₉ is CRₑ₃ or N, alternatively CRₑ₃;
Rₑ₁ and Rₑ₂ are independently selected from H, F, Cl, CN, Me, Et, CHF₂, OCH₃, OEt, OCHF₂, -O-cyclopropyl and 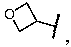 alternatively selected from H, F, Cl, CN, Me, CHF₂, OCH₃ and OEt, alternatively Rₑ₂ is not OEt;
Rₑ₃, Rₑ₄ and Rₑ₅ are H;
R'ₑ₅ is Me;
R₁ₐ and R_{1b} are independently Me or Et;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is H;
R_{d2}, R_{d4} and R_{d5} are independently selected from H, F, Me and CHF₂, alternatively R_{d2} and R_{d5} are independently selected from H and F, alternatively R_{d5} is H;
R_{d3} is selected from 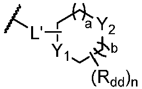 and -NR'_{d6}C(O)R_{d6}, alternatively 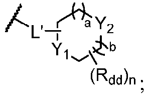
a is 1 or 2, b is 1;
L' is independently selected from covalent bond and methylene, alternatively covalent bond;
Y₁ is NH, CH or N, alternatively CH or N, alternatively N;
Y₂ is CR_{dd}R_{dd}, O or NR'_{dd}, alternatively O;
R_{dd} is independently selected from H, oxo, OH, Me, -C(CH₃)₂OH, -CH₂OH, -CH₂OMe, 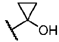 and -NHMe, alternatively H, oxo, OH, Me, -C(CH₃)₂OH, -CH₂OH, -CH₂OMe or -NHMe;
or two non-adjacent R_{dd} are taken together to form -CH₂CH₂-, alternatively make 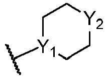 has the following structure: 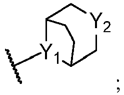
R'_{dd} is independently selected from H, Me and -CH₂-cyclopropyl;
n=0, 1 or 2;
R_{d6} is selected from -CH₂-cyclopropyl and -CH₂-O-CH₂CH₂-OH, alternatively -CH₂-cyclopropyl;
R'_{d6} is H.

In some specific embodiments of the present disclosure, R_{d3} is selected from: 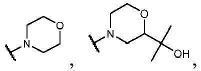 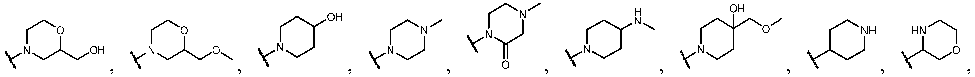 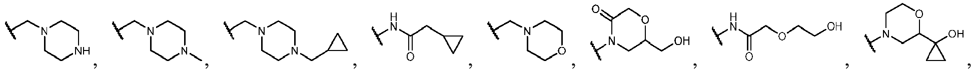 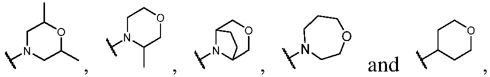 alternatively selected from: 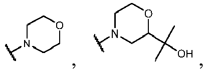 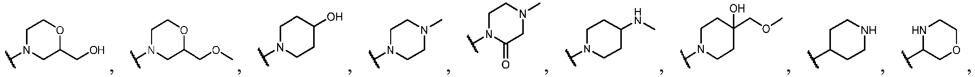 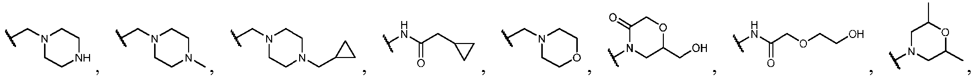 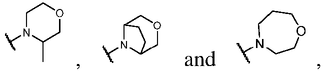 alternatively selected from: 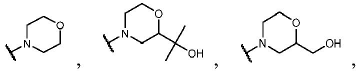 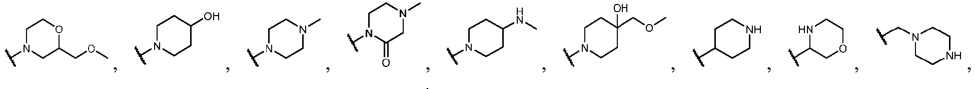 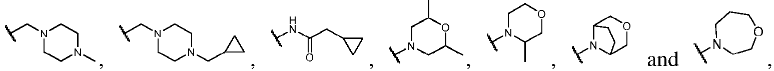 alternatively selected from: 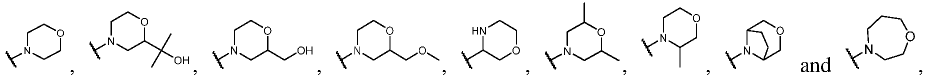 alternatively

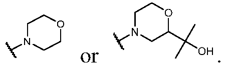

In some specific embodiments of the present disclosure, R_{d3} is selected from: 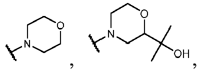 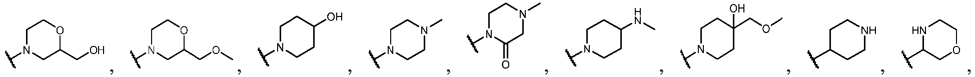 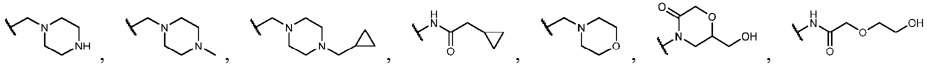 and 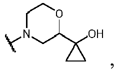 alternatively selected from: 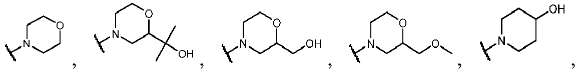 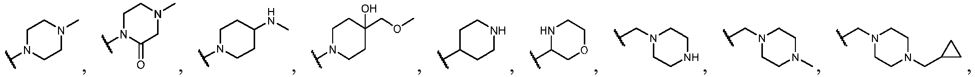 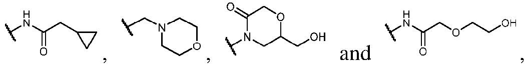 alternatively selected from: 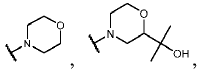 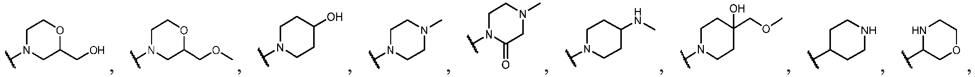 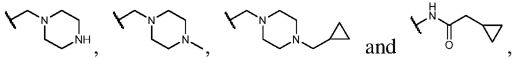 alternatively selected from:

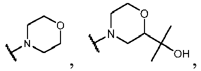

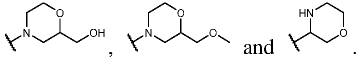

In some specific embodiments of the present disclosure, 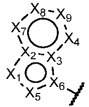 is selected from: 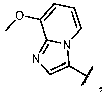 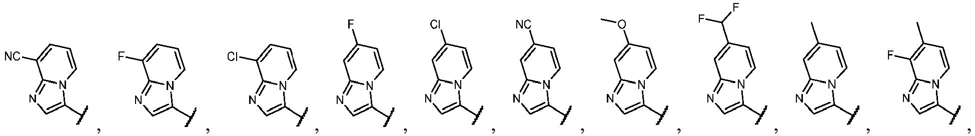 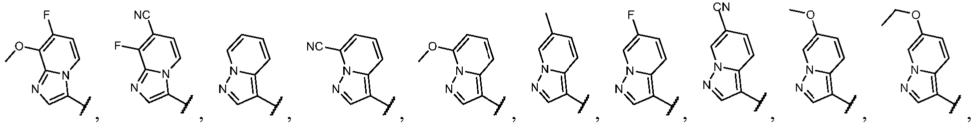 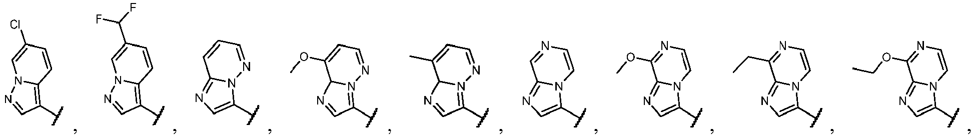 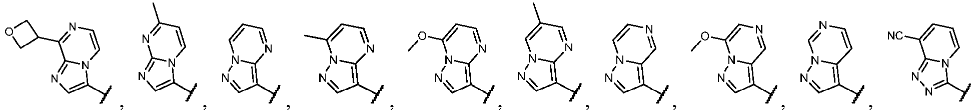 and 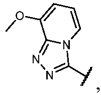 alternatively selected from: 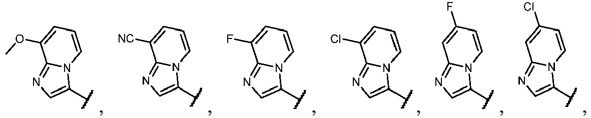 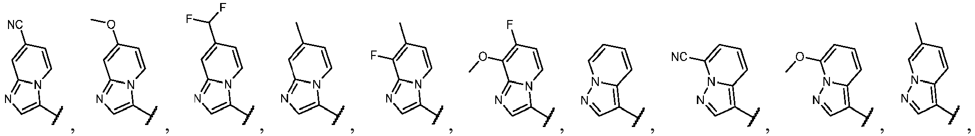 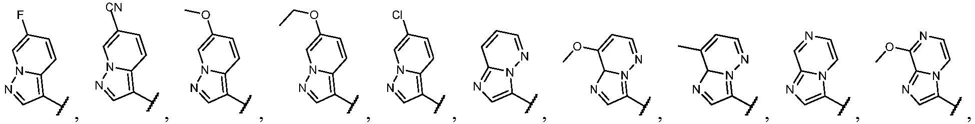 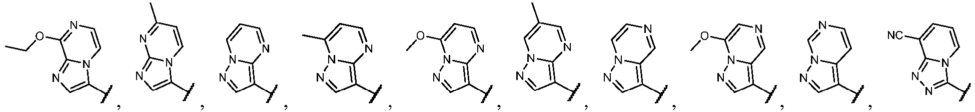 and 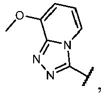 , alternatively not 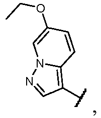 alternatively selected from:

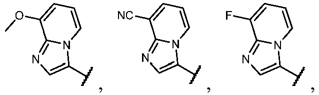

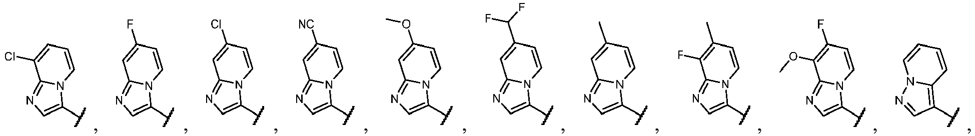

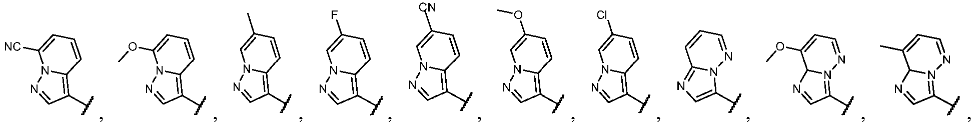

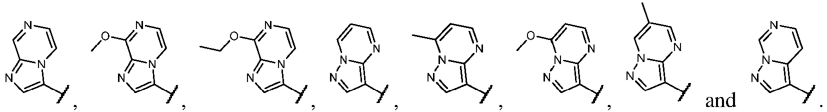

In a more specific embodiment, the present disclosure provides a compound of the above formula (VI) or formula (VI-1), or a stereoisomer, an N-oxide, a hydrate, a solvate, a metabolite, a pharmaceutically acceptable salt, a polymorph, or a prodrug thereof, 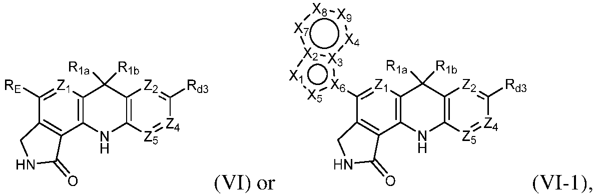 wherein
R_{E} is as defined herein;
X₅ is CRₑ₅, N, O, S or NR'ₑ₅, alternatively CRₑ₅, O, N or NR'ₑ₅, alternatively CRₑ₅ or N;
X₇ is CRₑ₁ or N;
X₈ is CRₑ₂ or N;
Rₑ₁ and Rₑ₂ are independently selected from H, D, halogen, CN, C₁₋₆ alkyl, C₁₋₆ haloalkyl, C₂₋₆ alkenyl, C₂₋₆ alkynyl, -L'-OR', -L'-SR', -L'-NR'R", -L'-3- to 7-membered carbocyclyl and -L'-3- to 7-membered heterocyclyl, alternatively selected from H, D, halogen, CN, C₁₋₆ alkyl, C₁₋₆ haloalkyl, C₂₋₆ alkenyl, C₂₋₆ alkynyl, -OR', -NR'R", 3- to 7-membered carbocyclyl and 3- to 7-membered heterocyclyl, each of which is optionally substituted with 1, 2 or 3 Rₑₛ;
Rₑₛ is independently selected from H, D, halogen, C₁₋₆ alkyl, C₁₋₆ haloalkyl, -OR' and -NR'R";
Rₑ₅ is selected from H, D, halogen, CN, C₁₋₆ alkyl and C₁₋₆ haloalkyl, alternatively selected from H, D, halogen, C₁₋₆ alkyl and C₁₋₆ haloalkyl;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is selected from H, D, halogen, CN, C₁₋₆ alkyl and C₁₋₆ haloalkyl;
R_{d2}, R_{d4} and R_{d5} are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, alternatively selected from H, D, halogen, CN, C₁₋₆ alkyl, C₁₋₆ haloalkyl, -OR' and -NR'R"; alternatively R_{d2} and R_{d5} are independently selected from H, D, halogen, C₁₋₆ alkyl and C₁₋₆ haloalkyl;
R_{d3} is selected from 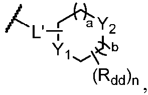 -NR'_{d6}C(O)R_{d6} and -C(O)NR_{d7}R'_{d7};
a and b are independently 0, 1, 2 or 3, alternatively 0, 1 or 2; alternatively a+b≤5, alternatively a+b≤3;
L' is independently selected from covalent bond and C₁₋₆ alkylene, alternatively selected from covalent bond and C₁₋₄ alkylene, each of which is optionally substituted with 1, 2, 3 or 4 groups selected from: C₁₋₆ alkyl and C₁₋₆ haloalkyl;
Y₁ is CR_{dd}R_{dd} or NR'_{dd}; or when Y₁ is connected to L', Y₁ is CR_{dd} or N;
Y₂ is selected from CR_{dd}R_{dd}, O, S, S(O), NR'_{dd} and S(O)₂, alternatively selected from CR_{dd}R_{dd}, O, NR'_{dd} and S(O)₂; alternatively, at least one of Y₁ and Y₂ is a heteroatom group;
R_{dd} is independently selected from H, D, oxo, halogen, CN, C₁₋₆ alkyl, C₁₋₆ haloalkyl, -L"-OR', -L"-SR', -L"-NR'R", -L"-3- to 7-membered carbocyclyl and -L"-3- to 7-membered heterocyclyl, alternatively selected from H, D, halogen, oxo, C₁₋₆ alkyl, C₁₋₆ haloalkyl, -L"-OR', -L"-NR'R", -L"-3- to 7-membered carbocyclyl and -L"-3- to 7-membered heterocyclyl;
or CR_{dd}R_{dd} forms a 3- to 7-membered carbocyclylene or a 3- to 7-membered heterocyclylene, which is optionally substituted with 1, 2 or 3 groups selected from: H, D, halogen, C₁₋₆ alkyl, C₁₋₆ haloalkyl, -OR' and -NR'R";
or two non-adjacent R_{dd} are taken together to form a C₁₋₃ alkylene, which is optionally substituted with 1, 2, 3 or 4 substituents selected from: H, D, halogen, CN, C₁₋₆ alkyl and C₁₋₆ haloalkyl;
R'_{dd} is independently selected from H, C₁₋₆ alkyl, C₁₋₆ haloalkyl, -L"-OR', -L"-NR'R", -L"-3- to 7-membered carbocyclyl and -L"-3- to 7-membered heterocyclyl, alternatively selected from H, C₁₋₆ alkyl, C₁₋₆ haloalkyl, -L"-3- to 7-membered carbocyclyl and -L"-3- to 7-membered heterocyclyl;
or R'_{dd} and an adjacent R_{dd} are taken together with the atoms to which they are attached to form a 3-to 7-membered heterocyclylene or a 5- to 6-membered heteroarylene, alternatively form a 3- to 7-membered heterocyclylene, which is optionally substituted with 1, 2, 3 or 4 substituents selected from: H, D, halogen, CN, C₁₋₆ alkyl, C₁₋₆ haloalkyl, -OR' and -NR'R";
R_{d7} and R'_{d7} are selected from H, C₁₋₆ alkyl and C₁₋₆ haloalkyl;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl, alternatively selected from H, C₁₋₆ alkyl, C₁₋₆ haloalkyl, 3- to 7-membered carbocyclyl and 3- to 7-membered heterocyclyl;
L_{d} is independently selected from covalent bond and C₁₋₆ alkylene, alternatively selected from covalent bond and C₁₋₄ alkylene;
the remaining variables are as defined herein;
wherein each of the above groups is optionally deuterated, up to completely deuterated.
In a more specific embodiment, the present disclosure provides a compound of the above formula (VI) or formula (VI-1), or a stereoisomer, an N-oxide, a hydrate, a solvate, a metabolite, a pharmaceutically acceptable salt, a polymorph, or a prodrug thereof, wherein R_{E} is as defined herein;
X₅ is CRₑ₅ or N, alternatively CRₑ₅;
X₇ is CRₑ₁ or N, alternatively CRₑ₁;
X₈ is CRₑ₂ or N, alternatively N;
Rₑ₁ and Rₑ₂ are independently selected from H, D, halogen, CN, C₁₋₆ alkyl, C₁₋₆ haloalkyl, C₂₋₆ alkynyl, -OR', -NR'R", -O-3- to 7-membered carbocyclyl, -O-3- to 7-membered heterocyclyl, 3- to 7-membered carbocyclyl and 3- to 7-membered heterocyclyl, alternatively not -NR'R", alternatively selected from H, D, halogen, CN, C₁₋₆ alkyl, C₁₋₆ haloalkyl, C₂₋₆ alkynyl, -OR', -O-3- to 7-membered carbocyclyl, 3- to 7-membered carbocyclyl and 3- to 7-membered heterocyclyl, each of which is optionally substituted with 1, 2 or 3 Rₑₛ; alternatively Rₑ₁ is not heterocyclyl;
Rₑₛ is independently selected from H, D, halogen, C₁₋₆ alkyl, C₁₋₆ haloalkyl and -OR', alternatively selected from H, D, F, Me and OH;
Rₑ₅ is selected from H, D, halogen, C₁₋₄ alkyl and C₁₋₄ haloalkyl, alternatively H or D;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is H or D;
R_{d2}, R_{d4} and R_{d5} are independently selected from H, D, halogen, CN, C₁₋₄ alkyl, C₁₋₄ haloalkyl and -OR', alternatively R_{d2} and R_{d5} are independently selected from H, D and halogen; alternatively R_{d2}, R_{d4} and R_{d5} are H or D;
R_{d3} is selected from 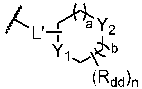 and -NR'_{d6}C(O)R_{d6}; alternatively, 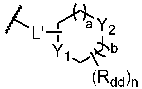 is 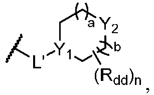 alternatively 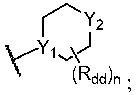
a and b are independently 0, 1 or 2, alternatively a and b are 1;
L' is independently selected from covalent bond and C₁₋₂ alkylene, alternatively selected from covalent bond and methylene;
Y₁ is NR'_{dd}; or when Y₁ is connected to L', Y₁ is CR_{dd} or N;
Y₂ is selected from CR_{dd}R_{dd}, O, NR'_{dd} and S(O)₂;
R_{dd} is independently selected from H, D, halogen, oxo, C₁₋₆ alkyl, C₁₋₆ haloalkyl, -L"-OR', -L"-NR'R" and -L"-3- to 7-membered carbocyclyl, alternatively selected from H, D, oxo, C₁₋₆ alkyl, C₁₋₆ haloalkyl, -L"-OR' and NR'R", alternatively selected from H, D, C₁₋₆ alkyl, C₁₋₆ haloalkyl and -L"-OR';
or CR_{dd}R_{dd} forms a 3- to 7-membered carbocyclylene or a 3- to 7-membered heterocyclylene, which is optionally substituted with one -OR', alternatively optionally substituted with one OH;
or two non-adjacent R_{dd} are taken together to form a C₁₋₂ alkylene;
R'_{dd} is independently selected from H, C₁₋₆ alkyl, C₁₋₆ haloalkyl and -L"-3- to 7-membered carbocyclyl;
or R'_{dd} and an adjacent R_{dd} are taken together with the atoms to which they are attached to form a 3-to 7-membered heterocyclylene or a 5- to 6-membered heteroarylene, alternatively form a 3- to 7-membered heterocyclylene, which is optionally substituted with 1, 2, 3 or 4 substituents selected from: H, D, halogen, C₁₋₆ alkyl and C₁₋₆ haloalkyl;
L" is independently selected from covalent bond and C₁₋₄ alkylene, each of which is optionally substituted with 1, 2, 3 or 4 R, and R is independently selected from H, D, C₁₋₆ alkyl and C₁₋₆ haloalkyl, or two R are taken together with the C atom to which they are jointly attached to form a 3- to 7-membered carbocyclylene, alternatively form a 3- to 5-membered carbocyclylene, alternatively form 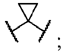
each of R' and R" is independently selected from H, C₁₋₆ alkyl and C₁₋₆ haloalkyl;
the remaining variables are as defined herein;
alternatively, when Y₁ is N and Y₂ is O, L' is a covalent bond and R_{dd} is not oxo;
alternatively, when Rₑ₂ is -O-C₁₋₆ alkyl, Rₑ₂ is -OMe, alternatively, when Rₑ₂ is -O-C₁₋₆ haloalkyl, Rₑ₂ is -O-halomethyl;
alternatively,
Rₑ₁ is selected from H, D, halogen, CN, C₁₋₆ alkyl, C₁₋₆ haloalkyl, -OR', -NR'R", -O-3- to 7-membered carbocyclyl, -O-3- to 7-membered heterocyclyl, 3- to 7-membered carbocyclyl and 3- to 7-membered heterocyclyl, alternatively not -NR'R" or 3- to 7-membered heterocyclyl, alternatively selected from H, D, halogen, CN, C₁₋₆ alkyl, C₁₋₆ haloalkyl, -OR', -O-3- to 7-membered carbocyclyl and 3- to 7-membered carbocyclyl, and Rₑ₁ is optionally substituted with 1, 2 or 3 Rₑₛ;
Rₑ₂ is selected from H, D, halogen, CN, C₁₋₆ alkyl, C₁₋₆ haloalkyl, C₂₋₆ alkynyl, -OR', -O-3- to 7-membered heterocyclyl, 3- to 7-membered carbocyclyl and 3- to 7-membered heterocyclyl, alternatively selected from H, D, halogen, CN, C₁₋₆ alkyl, C₁₋₆ haloalkyl, C₂₋₆ alkynyl, -OMe, -O-halomethyl, 3- to 7-membered carbocyclyl and 3- to 7-membered heterocyclyl, and Rₑ₂ is optionally substituted with 1, 2 or 3 Rₑₛ;
alternatively, when Rₑ₂ is heterocyclyl, Rₑₛ is independently selected from H, D, halogen (F), C₁₋₆ alkyl and C₁₋₆ haloalkyl, alternatively selected from H, D, F and Me, alternatively halogen (F).

In a more specific embodiment, the present disclosure provides a compound of the above formula (VI) or formula (VI-1), or a stereoisomer, an N-oxide, a hydrate, a solvate, a metabolite, a pharmaceutically acceptable salt, a polymorph, or a prodrug thereof, wherein R_{E} is as defined herein;
X₅ is CRₑ₅ or N, alternatively CRₑ₅;
X₇ is CRₑ₁ or N, alternatively CRₑ₁;
X₈ is CRₑ₂ or N, alternatively N;
Rₑ₁ and Rₑ₂ are independently selected from H, F, Cl, CN, Me, Et, n-Bu, CHF₂, 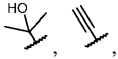 OH, OCH₃, OEt, OCHF₂, -NHCH₃, -O-cyclopropyl, 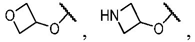 cyclopropyl, 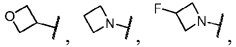 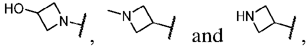 alternatively selected from H, F, Cl, CN, Me, Et, n-Bu, CHF₂, 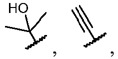 OCH₃, OEt, OCHF₂, -NHCH₃, -O-cyclopropyl, 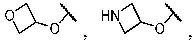 cyclopropyl, 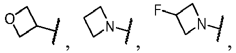 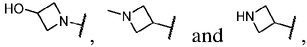 alternatively not 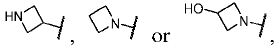 alternatively selected from H, F, Cl, CN, Me, Et, CHF₂, 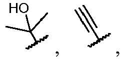 OCH₃, OEt, OCHF₂, -O-cyclopropyl, cyclopropyl, 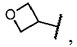 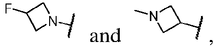 alternatively Rₑ₁ is not 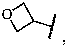 , alternatively Rₑ₂ is not OEt;
Rₑ₅ is selected from H, D, F, Br and Me, alternatively H;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is H;
R_{d2}, R_{d4} and R_{d5} are independently selected from H, F, CN, Me, CHF₂ and OCH₃, alternatively R_{d2} and R_{d5} are independently selected from H and F; alternatively, R_{d2}, R_{d4} and R_{d5} are H;
R_{d3} is selected from 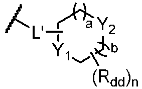 and -NR'_{d6}C(O)R_{d6}, alternatively 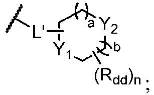
a is 0, 1 or 2, alternatively 0 or 1, b is 0 or 1;
L' is independently selected from covalent bond and methylene, alternatively covalent bond;
Y₁ is NH; or when Y₁ is connected to L', Y₁ is selected from CH, C(OH) and N, alternatively selected from CH and N, alternatively N;
Y₂ is selected from CR_{dd}R_{dd}, O, NR'_{dd} and S(O)₂, alternatively selected from CR_{dd}R_{dd}, O and NR'_{dd}, alternatively O;
R_{dd} is independently selected from H, F, oxo, OH, Me, -C(CH₃)₂OH, -CH₂OH, -CH₂OMe, 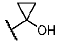 and -NHMe, alternatively selected from H, oxo, OH, Me, -C(CH₃)₂OH, -CH₂OH, -CH₂OMe, 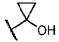 and -NHMe, alternatively selected from H, OH, Me, -C(CH₃)₂OH, -CH₂OMe and 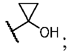
or CR_{dd}R_{dd} forms cyclopropylene, 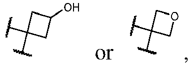 alternatively forms cyclopropylene or 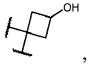 alternatively forms cyclopropylene;
or two non-adjacent R_{dd} are taken together to form -CH₂CH₂-;
R'_{dd} is independently selected from H, Me and -CH₂-cyclopropyl;
or R'_{dd} and an adjacent R_{dd} are taken together with the atoms to which they are attached to form 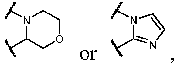 alternatively form 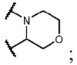
the remaining variables are as defined herein;
alternatively,
Rₑ₁ is selected from H, F, Cl, CN, Me, Et, n-Bu, CHF₂, OCH₃, OEt, OCHF₂, -NHCH₃, -O-cyclopropyl, 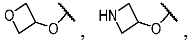 cyclopropyl, 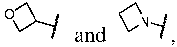 alternatively selected from H, F, Cl, CN, Me, Et, CHF₂, OCH₃, OEt, OCHF₂, -O-cyclopropyl, 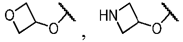 and cyclopropyl, alternatively selected from H, F, CN, Me, CHF₂, OCH₃, OEt, OCHF₂, -O-cyclopropyl and cyclopropyl;
Rₑ₂ is selected from H, F, Cl, CN, Me, CHF₂, 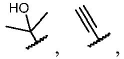 OCH₃, OEt, OCHF₂, 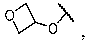 cyclopropyl, 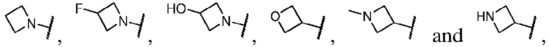 alternatively selected from H, F, Cl, CN, Me, CHF₂, 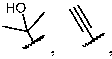 , OCH₃, OCHF₂, cyclopropyl, 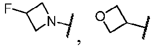 and 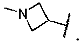
Alternatively,
R_{d3} is selected from: 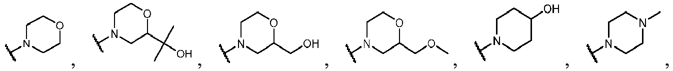 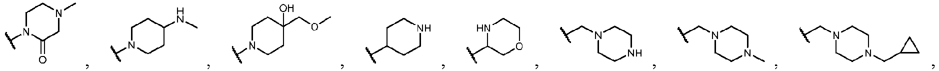 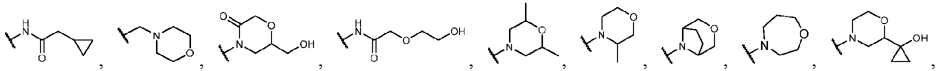 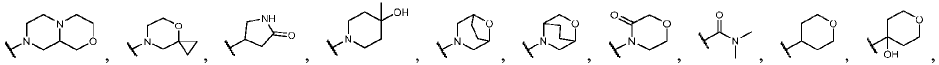 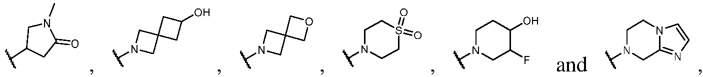 alternatively selected from: 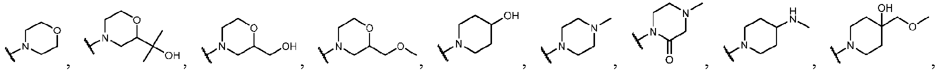 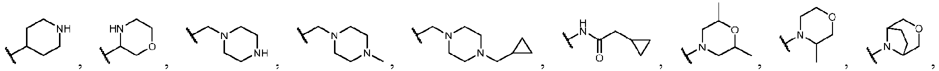 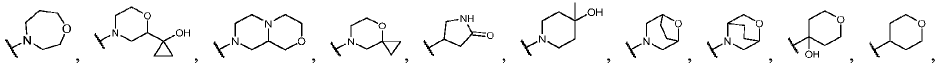 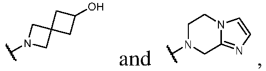 alternatively selected from: 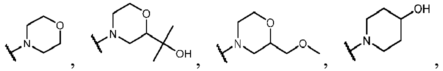 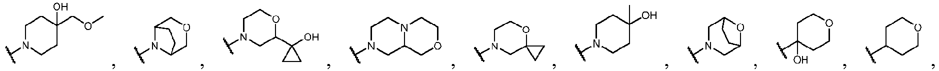 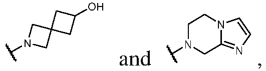 alternatively selected from: 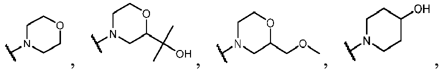 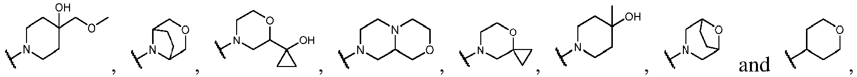 alternatively 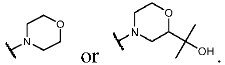
Alternatively,
R_{d3} is selected from: 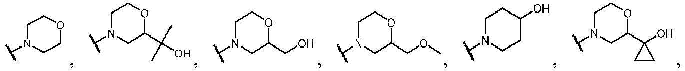 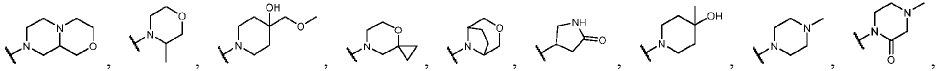 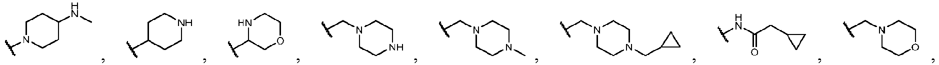 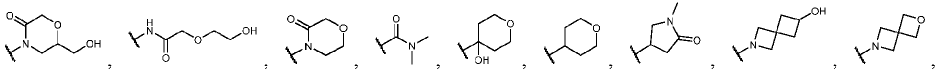 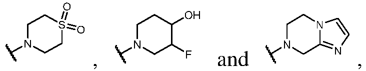 alternatively selected from: 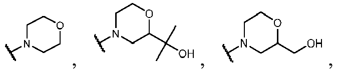 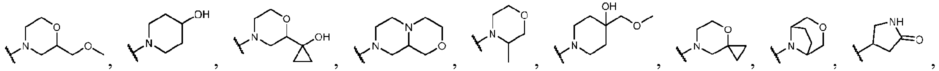 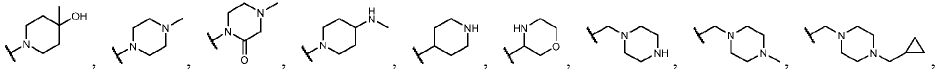 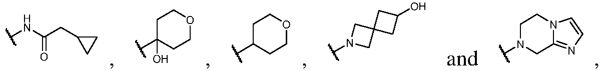 alternatively selected from: 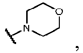 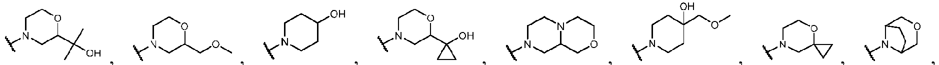 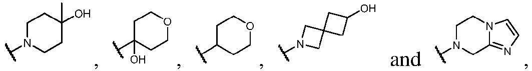 alternatively selected from: 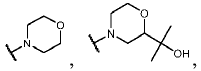 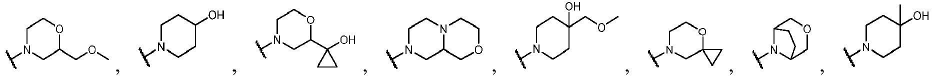 and 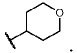
Alternatively, 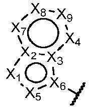 is selected from: 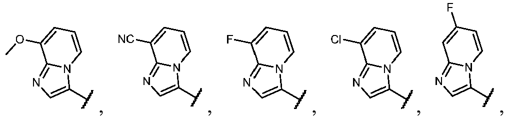 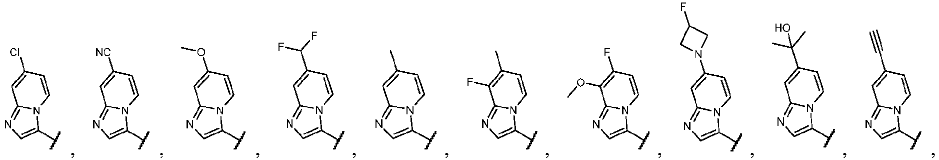 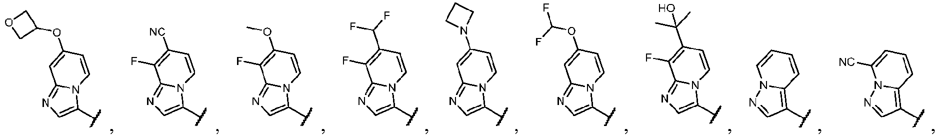 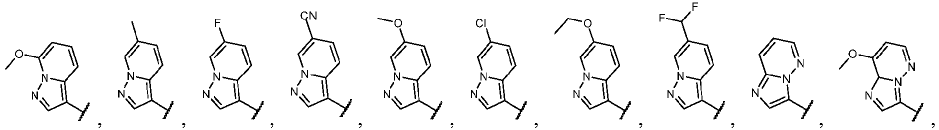 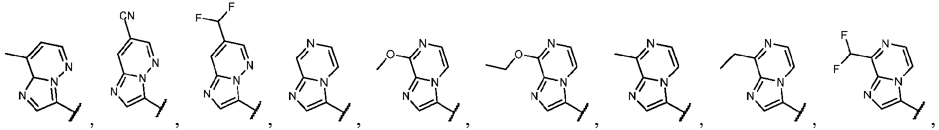 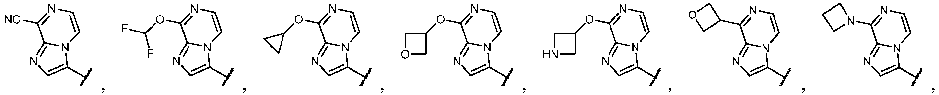 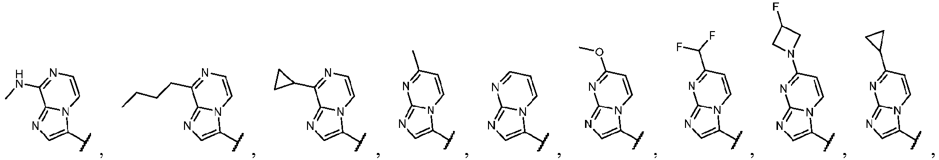 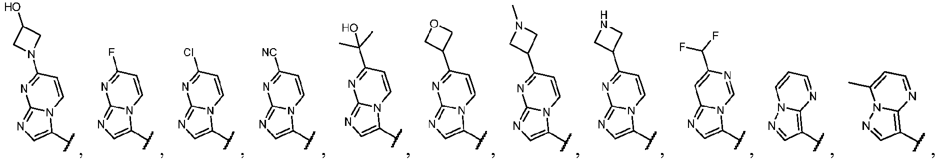 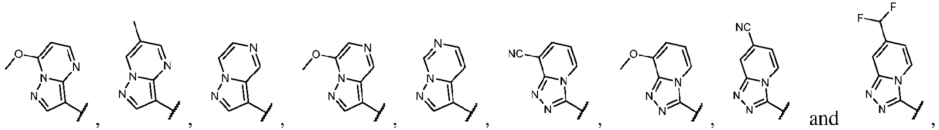 alternatively selected from: 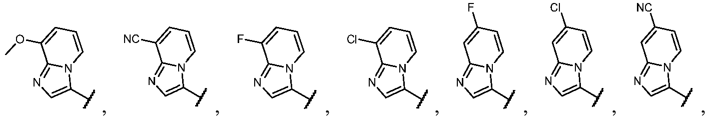 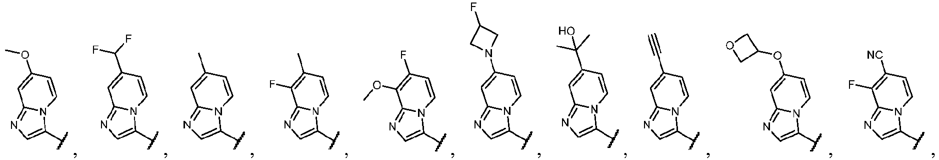 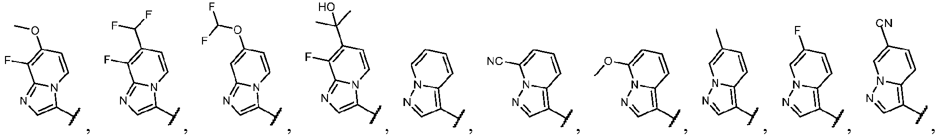 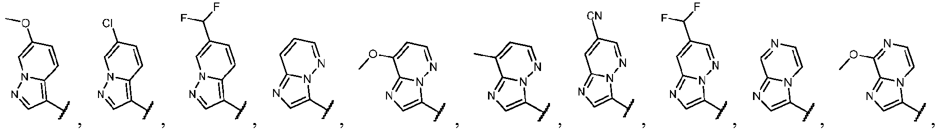 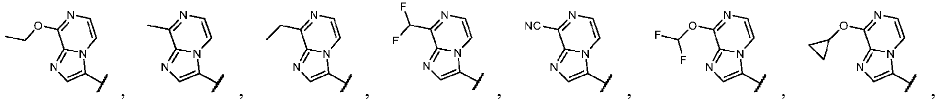 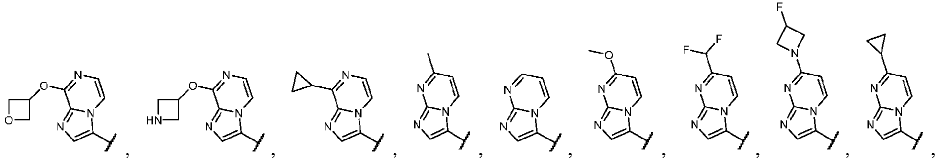 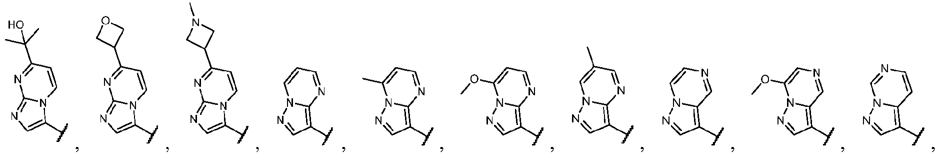 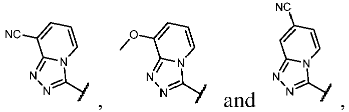 alternatively selected from:

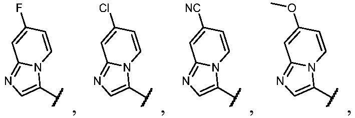

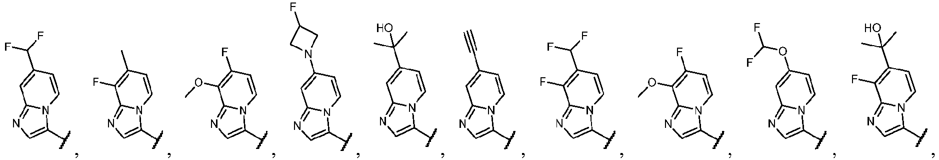

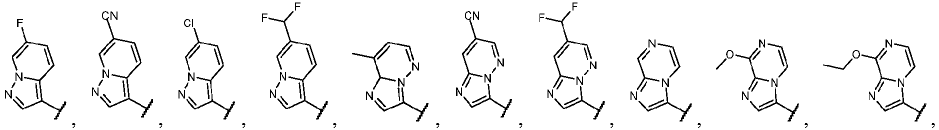

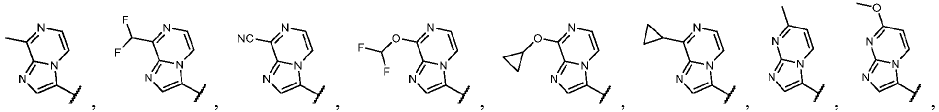

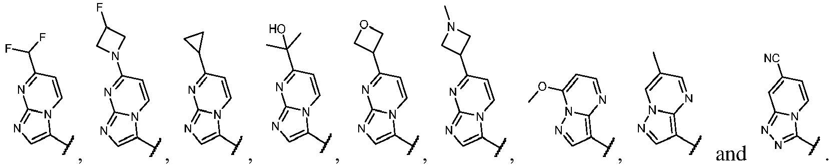

In a more specific embodiment, the present disclosure provides a compound of the above formula (VI-2), or a stereoisomer, an N-oxide, a hydrate, a solvate, a metabolite, a pharmaceutically acceptable salt, a polymorph, or a prodrug thereof, 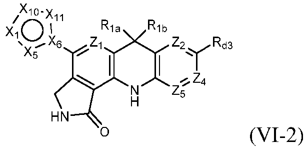 wherein
X₁ is selected from CRₑ₆, O, S, N and NR'ₑ₆;
X₅ is CRₑ₅, N, O, S or NR'ₑ₅;
X₆ is C or N;
X₁₀ is selected from CRₑ₇, O, S, N and NR'ₑ₇;
X₁₁ is selected from CRₑ₈, O, S, N and NR'ₑ₈;
Rₑ₇ and Rₑ₈ are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl;
R'ₑ₇ and R'ₑ₈ are independently selected from H, D, C₁₋₆ alkyl, C₁₋₆ haloalkyl, -L'-3- to 7-membered carbocyclyl and -L'-3- to 7-membered heterocyclyl;
or the substituents on X₁₀ and X₁₁ are taken together to form a C₃₋₅ alkylene, which optionally contains one double bond, and the C₃₋₅ alkylene is optionally substituted with 1, 2, 3 or 4 Rₑ, and 1, 2 or 3 methylene units in the C₃₋₅ alkylene are optionally and independently replaced by -NR"-, -N(R")C(O)-, -C(O)N(R")-, -O-, -C(O)-, -OC(O)-, -C(O)O-, -S-, or -S(O)-;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl;
L' is independently selected from covalent bond and C₁₋₆ alkylene, each of which is optionally substituted with 1, 2, 3 or 4 groups selected from: C₁₋₆ alkyl and C₁₋₆ haloalkyl;
L_{d} is independently selected from covalent bond and C₁₋₆ alkylene, alternatively selected from covalent bond and C₁₋₄ alkylene;
Rₑ is as defined herein, alternatively Rₑ is not alkenyl or alkynyl;
each of Rₑ₅, R'ₑ₅, Rₑ₆ and R'ₑ₆ is as defined herein;
each of R₁ₐ, R_{1b}, Z₁, Z₂, Z₄, Z₅ and R_{d3} is as defined herein;
wherein each of the above groups is optionally deuterated, up to completely deuterated.

In a more specific embodiment, the present disclosure provides a compound of the above formula (VI-2), or a stereoisomer, an N-oxide, a hydrate, a solvate, a metabolite, a pharmaceutically acceptable salt, a polymorph, or a prodrug thereof, wherein
X₁ is selected from CRₑ₆, N and NR'ₑ₆, alternatively N;
X₅ is CRₑ₅, N or NR'ₑ₅;
X₆ is C or N, alternatively C;
X₁₀ is selected from CRₑ₇, N and NR'ₑ₇;
X₁₁ is selected from CRₑ₈, O, N and NR'ₑ₈;
Rₑ₇ is selected from H, D, C₁₋₆ alkyl, C₁₋₆ haloalkyl, -OR', -NR'R", 3- to 7-membered carbocyclyl and 3- to 7-membered heterocyclyl;
R'ₑ₇ is selected from H, D, C₁₋₆ alkyl, C₁₋₆ haloalkyl, 3- to 7-membered carbocyclyl and 3- to 7-membered heterocyclyl;
Rₑ₈ is selected from H, D, C₁₋₆ alkyl and C₁₋₆ haloalkyl;
R'ₑ₈ is selected from H, C₁₋₆ alkyl and C₁₋₆ haloalkyl;
or the substituents on X₁₀ and X₁₁ are taken together to form a C₃₋₅ alkylene, alternatively form -(CH₂)₄-, which optionally contains one double bond, and the C₃₋₅ alkylene or -(CH₂)₄- is optionally substituted with 1, 2, 3 or 4 Rₑ, and 1, 2 or 3 methylene units in the C₃₋₅ alkylene or -(CH₂)₄- are optionally and independently replaced by NR"-, -C(O)N(R")-, -C(O)-, or -O-, alternatively, one methylene unit in the C₃₋₅ alkylene or -(CH₂)₄- is optionally and independently replaced by -O-;
each of R' and R" is independently selected from H, C₁₋₆ alkyl, C₁₋₆ haloalkyl, 3- to 7-membered carbocyclyl and 3- to 7-membered heterocyclyl;
Rₑ is as defined herein, alternatively Rₑ is not alkenyl or alkynyl;
each of Rₑ₅, R'ₑ₅, Rₑ₆ and R'ₑ₆ is as defined herein;
each of R₁ₐ, R_{1b}, Z₁, Z₂, Z₄, Z₅ and R_{d3} is as defined herein.

In some specific embodiments of the present disclosure, at most three of X₁, X₅, X₆, X₁₀ and X₁₁ are heteroatoms.

In a more specific embodiment, the present disclosure provides a compound of the above formula (VI-2), or a stereoisomer, an N-oxide, a hydrate, a solvate, a metabolite, a pharmaceutically acceptable salt, a polymorph, or a prodrug thereof, wherein
X₁ is N;
X₅ is CRₑ₅, N or NR'ₑ₅, alternatively CRₑ₅ or N, yet alternatively CRₑ₅;
X₆ is C or N, alternatively C;
X₁₀ is CRₑ₇ or NR'ₑ₇;
X₁₁ is selected from CRₑ₈, O and NR'ₑ₈, alternatively CRₑ₈ or NR'ₑ₈;
Rₑ₇ is selected from C₁₋₆ alkyl, C₁₋₆ haloalkyl, 3- to 7-membered carbocyclyl, 3- to 7-membered heterocyclyl and -O-3- to 7-membered heterocyclyl;
R'ₑ₇ is selected from C₁₋₆ alkyl, C₁₋₆ haloalkyl, 3- to 7-membered carbocyclyl and 3- to 7-membered heterocyclyl, alternatively 3- to 7-membered carbocyclyl;
Rₑ₈ is selected from H, D, C₁₋₆ alkyl and C₁₋₆ haloalkyl, alternatively C₁₋₆ alkyl or C₁₋₆ haloalkyl;
R'ₑ₈ is selected from C₁₋₆ alkyl and C₁₋₆ haloalkyl;
or the substituents on X₁₀ and X₁₁ are taken together to form 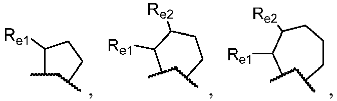 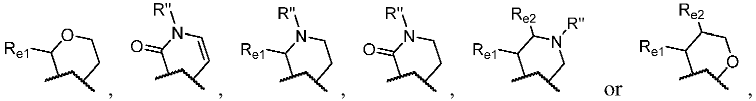 alternatively not form 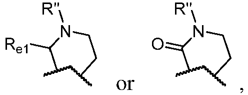 alternatively form a structure selected from: 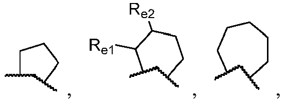 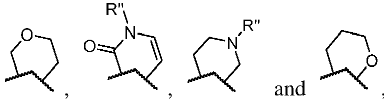 , alternatively form 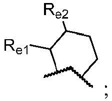
each of R' and R" is independently selected from H, C₁₋₆ alkyl and C₁₋₆ haloalkyl;
each of Rₑ₁, Rₑ₂, Rₑ₅ and R'ₑ₅ is as defined herein; alternatively, Rₑ₁ and Rₑ₂ are independently selected from H, D, halogen, CN, C₁₋₆ alkyl, C₁₋₆ haloalkyl, -OR' and -O-3- to 7-membered carbocyclyl, alternatively selected from H, D, C₁₋₆ alkyl, C₁₋₆ haloalkyl and -OR';
each of R₁ₐ, R_{1b}, Z₁, Z₂, Z₄, Z₅ and R_{d3} is as defined herein.

In some specific embodiments of the present disclosure, when Rₑ₇ or R'ₑ₇ is heterocyclyl, Rₑ₇ or R'ₑ₇ is 3- to 7-membered oxacyclyl, alternatively 4- to 6-membered oxacyclyl, alternatively 5-membered oxacyclyl.

In a more specific embodiment, the present disclosure provides a compound of the above formula (VI-2), or a stereoisomer, an N-oxide, a hydrate, a solvate, a metabolite, a pharmaceutically acceptable salt, a polymorph, or a prodrug thereof, wherein
X₁ is N;
X₅ is CRₑ₅, N or NR'ₑ₅, alternatively CRₑ₅ or N, yet alternatively CRₑ₅;
X₆ is C or N, alternatively C;
X₁₀ is selected from CRₑ₇ and NR'ₑ₇;
X₁₁ is selected from CRₑ₈, O and NR'ₑ₈, alternatively CRₑ₈ or NR'ₑ₈;
Rₑ₇ is selected from Me, Et, cyclopropyl, tetrahydrofuranyl, tetrahydropyranyl, pyrrolidinyl, morpholinyl and -O-tetrahydrofuranyl, alternatively selected from Me, Et, cyclopropyl, tetrahydrofuranyl, tetrahydropyranyl and -O-tetrahydrofuranyl, alternatively selected from Me, Et, cyclopropyl, tetrahydrofuranyl and -O-tetrahydrofuranyl, alternatively selected from Me, Et, cyclopropyl, 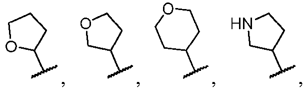 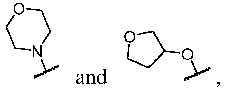 alternatively selected from Me, Et, cyclopropyl, 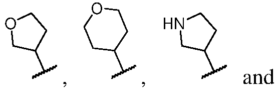 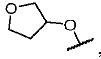 , alternatively selected from Me, Et, cyclopropyl, 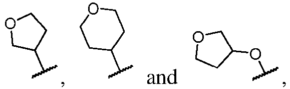 alternatively selected from Me, Et, cyclopropyl, 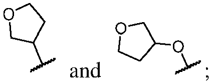
R'ₑ₇ is selected from Me, Et, cyclopropyl, tetrahydrofuranyl, tetrahydropyranyl, pyrrolidinyl and morpholinyl, alternatively selected from Me, Et, cyclopropyl, tetrahydrofuranyl and tetrahydropyranyl, alternatively selected from Me, Et, cyclopropyl and tetrahydrofuranyl, alternatively selected from Me, Et, cyclopropyl, 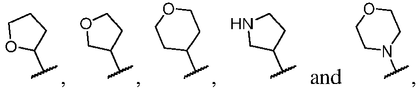 alternatively selected from Me, Et, cyclopropyl, 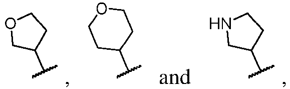 alternatively selected from Me, Et, cyclopropyl, 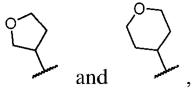 alternatively selected from Me, Et, cyclopropyl and 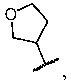 alternatively cyclopropyl;
Rₑ₈ is selected from H, Me and Et, alternatively H or Me, alternatively Me;
R'ₑ₈ is Me or Et, alternatively Me;
or the substituents on X₁₀ and X₁₁ are taken together to form 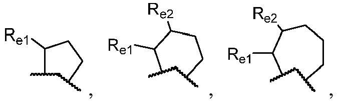 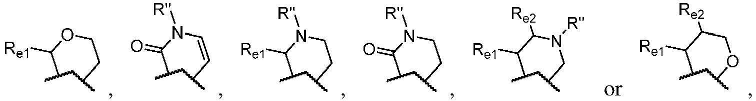 alternatively not form 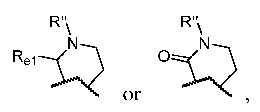 alternatively form a structure selected from: 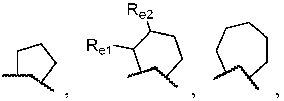 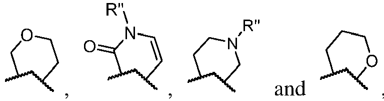 , alternatively form 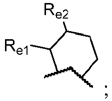
Rₑ₁ and Rₑ₂ are independently selected from H, F, Cl, CN, Me, Et, CHF₂, OCH₃, OEt, OCHF₂, -O-cyclopropyl and 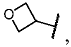 alternatively selected from H, F, CN, Me, OMe, OEt, OCHF₂ and -O-cyclopropyl, alternatively selected from H, F, CN, Me, OCH₃, OEt, OCHF₂ and -O-cyclopropyl, alternatively selected from H, Me, OCH₃ and OEt;
Rₑ₅ is H;
R'ₑ₅ is Me;
R" is H or methyl;
each of R₁ₐ, R_{1b}, Z₁, Z₂, Z₄, Z₅ and R_{d3} is as defined herein.

In some specific embodiments of the present disclosure, the substituents on X₁₀ and X₁₁ are taken together to form a structure selected from: 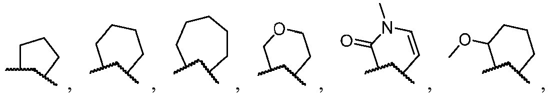 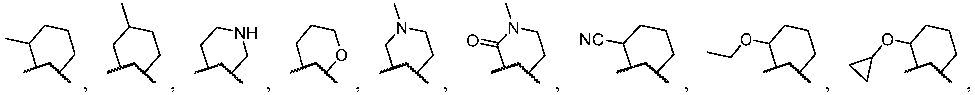 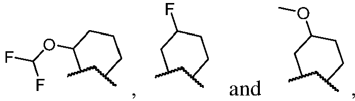 alternatively selected from: 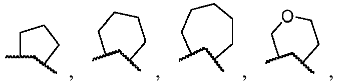 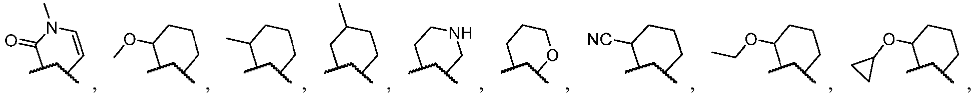 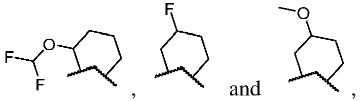 alternatively selected from: 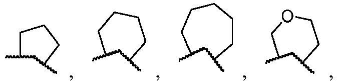 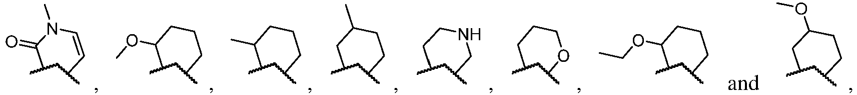 alternatively selected from:

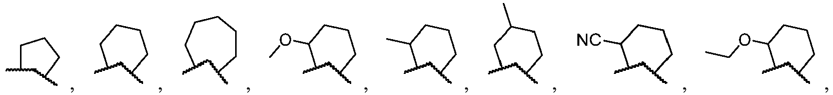

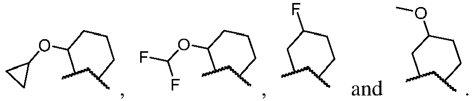

In some specific embodiments of the present disclosure, 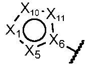 is selected from 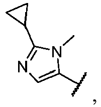 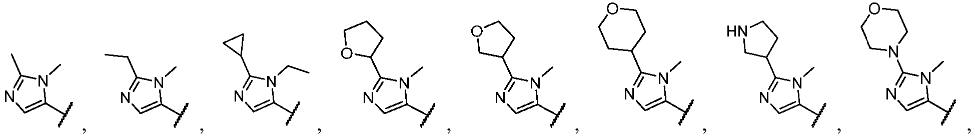 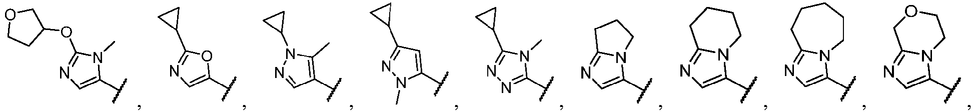 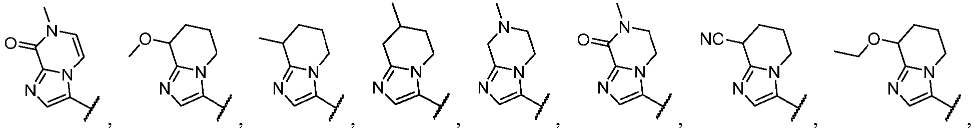 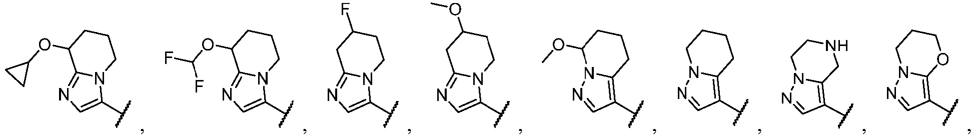 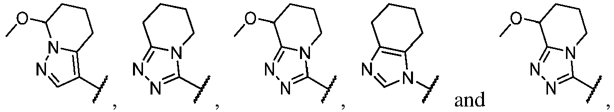 alternatively selected from: 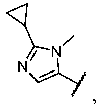 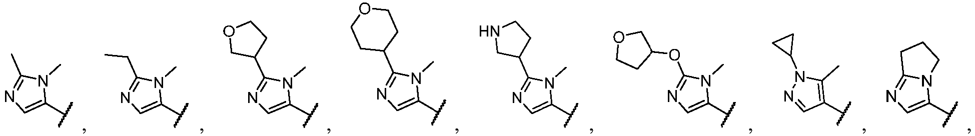 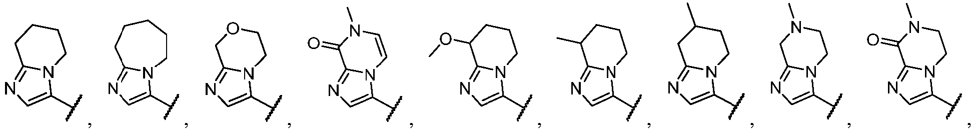 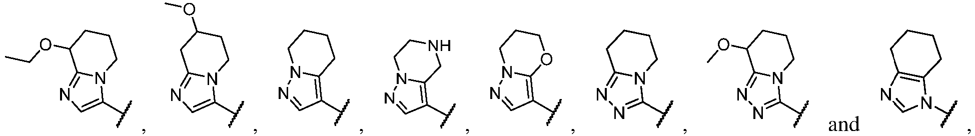 alternatively selected from:

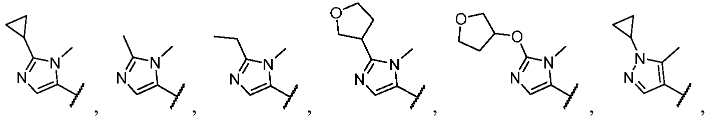

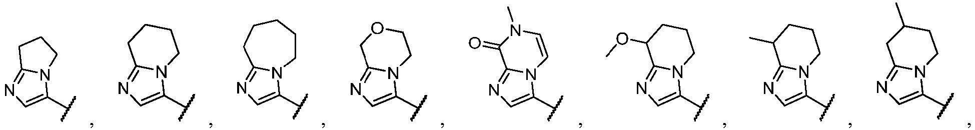

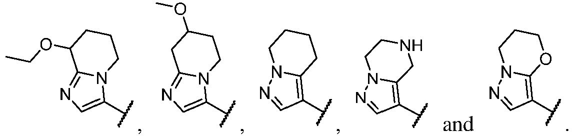

In some specific embodiments of the present disclosure, R_{dd} is independently selected from H, Me and -C(CH₃)₂OH, or two non-adjacent R_{dd} are taken together to form -CH₂CH₂-, alternatively make 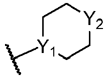 has the following structure: 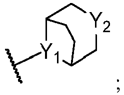 alternatively, R_{d3} is selected from: 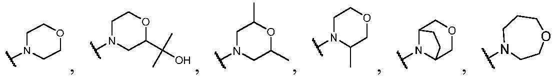 and 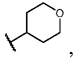 alternatively selected from: 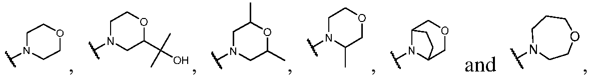 alternatively

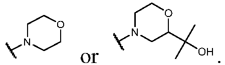

In a more specific embodiment, the present disclosure provides a compound of the above formula (VI-2), or a stereoisomer, an N-oxide, a hydrate, a solvate, a metabolite, a pharmaceutically acceptable salt, a polymorph, or a prodrug thereof, 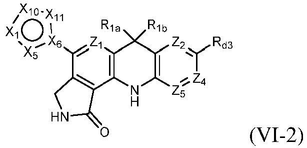 wherein
X₁₀ is CRₑ₇ or NR'ₑ₇;
X₁₁ is selected from CRₑ₈, O and NR'ₑ₈, alternatively CRₑ₈ or NR'ₑ₈;
Rₑ₇ is selected from C₁₋₆ alkyl, C₁₋₆ haloalkyl, 3- to 7-membered carbocyclyl, 3- to 7-membered heterocyclyl and -O-3- to 7-membered heterocyclyl; alternatively, Rₑ₇ is selected from C₁₋₆ alkyl, C₁₋₆ haloalkyl, 3- to 7-membered carbocyclyl, tetrahydrofuranyl, morpholinyl and -O-3- to 7-membered heterocyclyl; alternatively Rₑ₇ is 3- to 7-membered carbocyclyl;
R'ₑ₇ is selected from C₁₋₆ alkyl, C₁₋₆ haloalkyl, 3- to 7-membered carbocyclyl and 3- to 7-membered heterocyclyl, alternatively 3- to 7-membered carbocyclyl;
Rₑ₈ is selected from H, D, C₁₋₆ alkyl and C₁₋₆ haloalkyl, alternatively C₁₋₆ alkyl or C₁₋₆ haloalkyl;
R'ₑ₈ is selected from C₁₋₆ alkyl and C₁₋₆ haloalkyl;
or the substituents on X₁₀ and X₁₁ are taken together to form 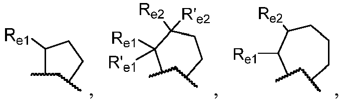 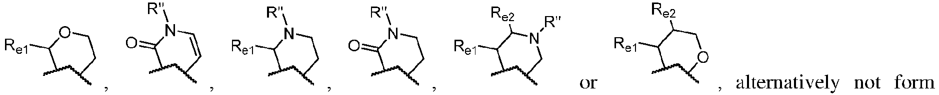 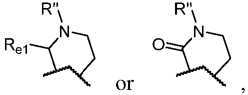 alternatively form a structure selected from: 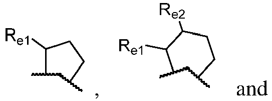 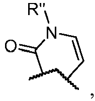 alternatively form 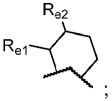
each of Rₑ₁ and Rₑ₂ is as defined herein, and each of R'ₑ₁ and R'ₑ₂ is as defined herein; alternatively, Rₑ₁ and Rₑ₂ are independently selected from H, D, halogen, CN, C₁₋₆ alkyl, C₁₋₆ haloalkyl, -OR', -O-3- to 7-membered carbocyclyl and -O-3- to 7-membered heterocyclyl, alternatively not halogen, alternatively selected from H, D, C₁₋₆ alkyl, C₁₋₆ haloalkyl, -OR' and -O-3- to 7-membered carbocyclyl; alternatively, Rₑ₂ is selected from H, D, halogen, CN, C₁₋₆ alkyl, C₁₋₆ haloalkyl and -OR', alternatively Rₑ₂ is H, D or C₁₋₆ haloalkyl;
R'ₑ₁ and R'ₑ₂ are independently selected from H, D, halogen, C₁₋₆ alkyl and C₁₋₆ haloalkyl, alternatively selected from H, D, halogen and C₁₋₆ alkyl;
or CRₑ₁R'ₑ₁ forms a 3- to 7-membered carbocyclyl or a 3- to 7-membered heterocyclyl, alternatively forms a 3- to 7-membered heterocyclyl;
each of R' and R" is independently selected from H, C₁₋₆ alkyl and C₁₋₆ haloalkyl, alternatively R" is C₁₋₄ haloalkyl;
the remaining variables are as defined herein;
wherein each of the above groups is optionally deuterated, up to completely deuterated.

In a more specific embodiment, the present disclosure provides a compound of the above formula (VI-2), or a stereoisomer, an N-oxide, a hydrate, a solvate, a metabolite, a pharmaceutically acceptable salt, a polymorph, or a prodrug thereof, wherein
X₁₀ is selected from CRₑ₇ and NR'ₑ₇;
X₁₁ is selected from CRₑ₈, O and NR'ₑ₈, alternatively CRₑ₈ or NR'ₑ₈;
Rₑ₇ is selected from Me, Et, cyclopropyl, 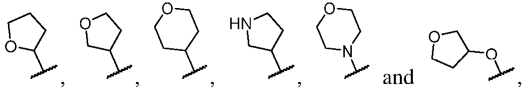 alternatively selected from Me, Et, cyclopropyl, 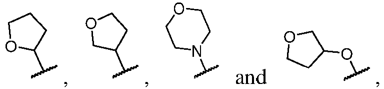 alternatively cyclopropyl;
R'ₑ₇ is selected from Me, Et and cyclopropyl, alternatively cyclopropyl;
Rₑ₈ is selected from H, Me and Et, alternatively H or Me, alternatively Me;
R'ₑ₈ is Me or Et, alternatively Me;
or the substituents on X₁₀ and X₁₁ are taken together to form 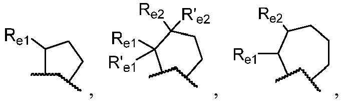 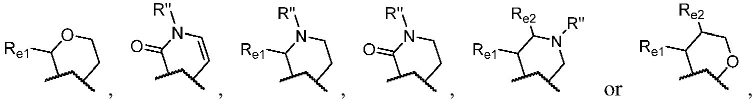 alternatively not form 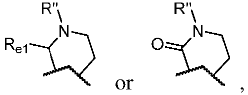 alternatively form a structure selected from: 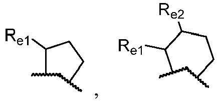 and 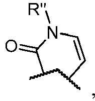 , alternatively form 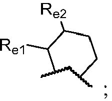
Rₑ₁ and Rₑ₂ are independently selected from H, F, CN, Me, CHF₂, OH, OMe, OEt, OCHF₂, -O-cyclopropyl and 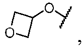 alternatively selected from H, CN, Me, CHF₂, OH, OCH₃, OEt, -O-cyclopropyl 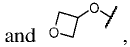 alternatively selected from H, Me, CHF₂, OCH₃, OEt and -O-cyclopropyl; alternatively Rₑ₂ is selected from H, F, CN, Me, CHF₂, OH and OMe, alternatively Rₑ₂ is H or CHF₂;
R'ₑ₁ and R'ₑ₂ are independently selected from H, F and Me;
or CRₑ₁R'ₑ₁ forms 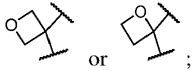
R" is selected from H, methyl and CHF₂, alternatively CHF₂;
the remaining variables are as defined herein.

Alternatively,
the substituents on X₁₀ and X₁₁ are taken together to form a structure selected from: 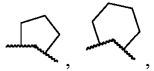 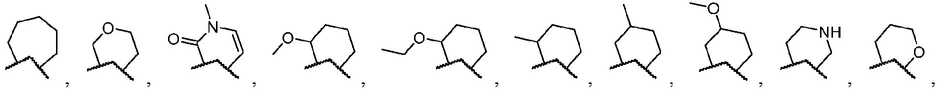 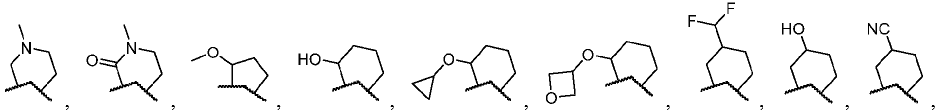 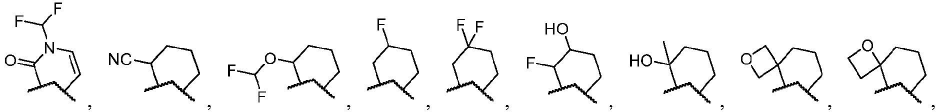 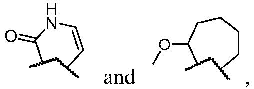 alternatively selected from: 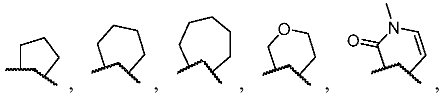 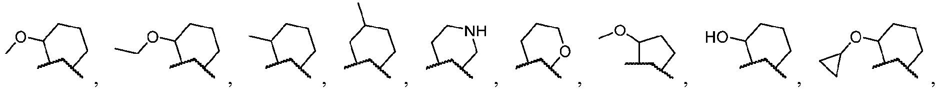 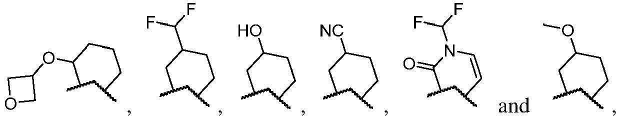 alternatively selected from: 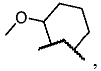 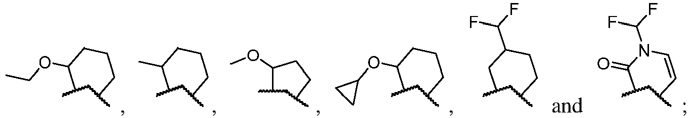
alternatively, 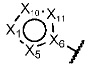 is selected from 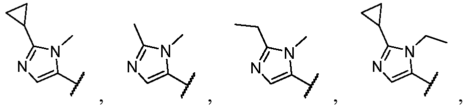 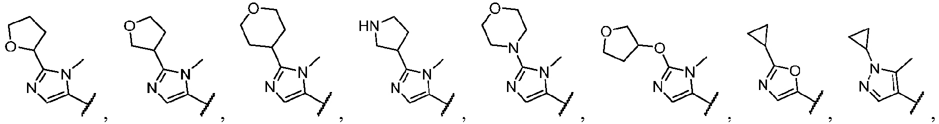 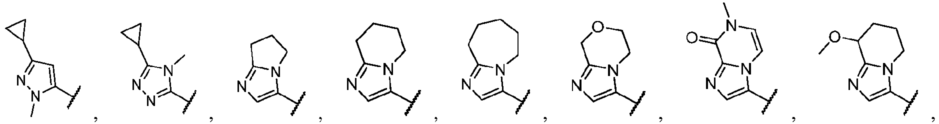 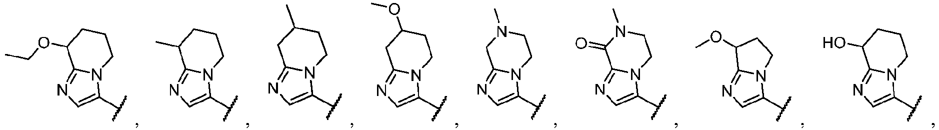 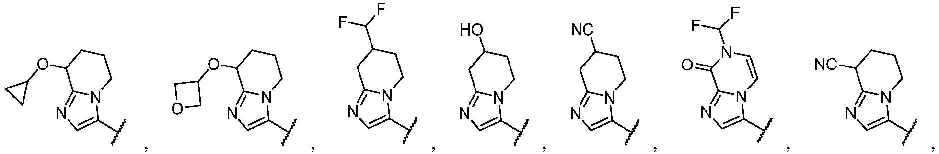 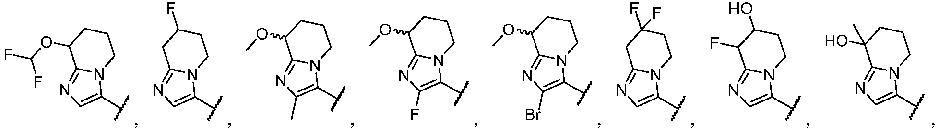 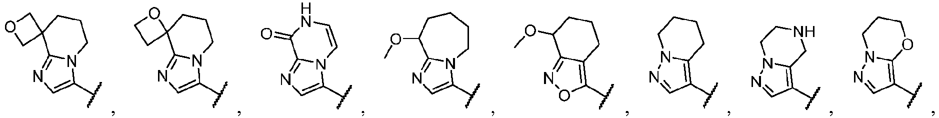 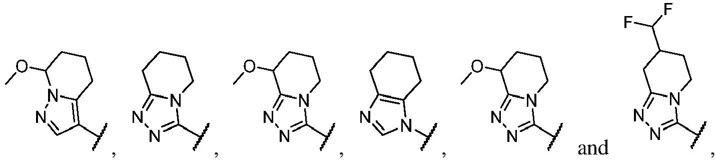 alternatively selected from: 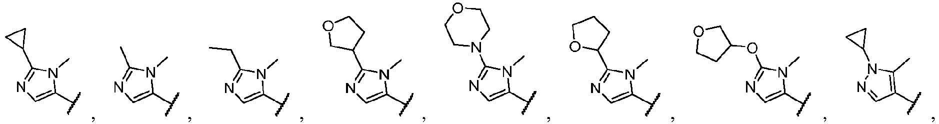 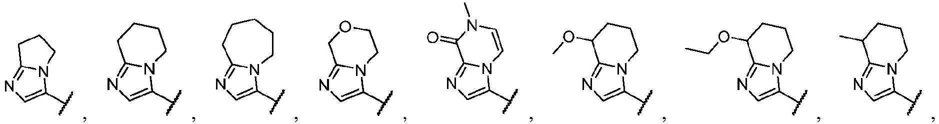 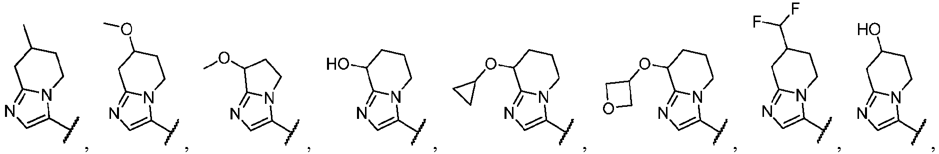 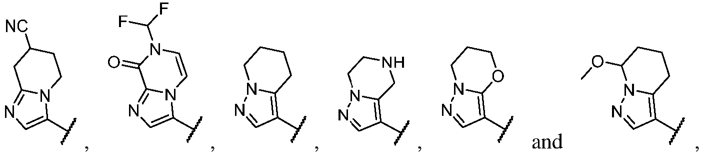 alternatively selected from: 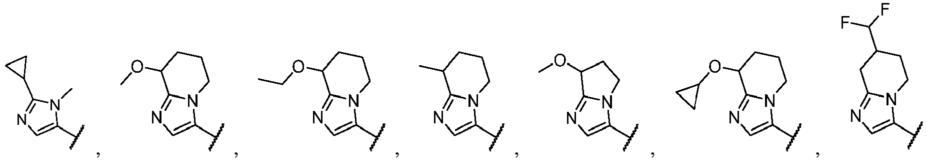 and 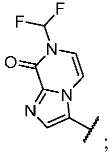
alternatively, Y₁ is CH or N, and Y₂ is selected from CR_{dd}R_{dd} and O;
R_{dd} is independently selected from H, OH, Me, -CH₂OMe and -C(CH₃)₂OH, or two non-adjacent R_{dd} are taken together to form -CH₂CH₂-;
alternatively, R_{d3} is selected from: 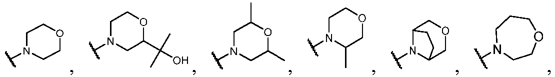 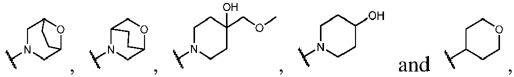 alternatively selected from: 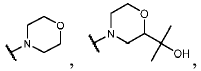 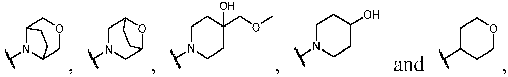 alternatively

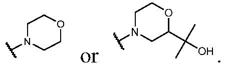

In a more specific embodiment, the present disclosure provides a compound of the above formula (VI-3), or a stereoisomer, an N-oxide, a hydrate, a solvate, a metabolite, a pharmaceutically acceptable salt, a polymorph, or a prodrug thereof, 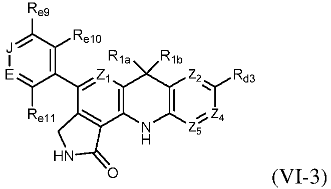 wherein
E is selected from CRₑ₁₂ and N;
J is selected from CRₑ₁₃ and N;
Rₑ₉, Rₑ₁₀, Rₑ₁₁, Rₑ₁₂ and Rₑ₁₃ are selected from H, D, halogen, CN, C₁₋₆ alkyl, C₁₋₆ haloalkyl, C₂₋₆ alkenyl, C₂₋₆ alkynyl, -L'-OR', -L'-SR', -L'-NR'R", -L'-3- to 7-membered carbocyclyl, -L'-3- to 7-membered heterocyclyl and -Y₅-C₀₋₆ alkylene-R₆;
Y₅ is selected from O, S and NR₅;
R₅ is selected from H, C₁₋₆ alkyl and C₁₋₆ haloalkyl;
R₆ is selected from 3- to 7-membered carbocyclyl and 3- to 7-membered heterocyclyl;
or Rₑ₉ and Rₑ₁₃ are taken together with the carbon atoms to which they are attached to form a 5- to 7-membered carbocyclyl, a 5- to 7-membered heterocyclyl, a phenyl, or a 5- to 6-membered heteroaryl, which is optionally substituted with 1, 2, 3 or 4 Rₑ;
or Rₑ₉ and Rₑ₁₀ are taken together with the carbon atoms to which they are attached to form a 5- to 7-membered carbocyclyl, a 5- to 7-membered heterocyclyl, a phenyl, or a 5- to 6-membered heteroaryl, which is optionally substituted with 1, 2, 3 or 4 Rₑ;
Rₑ is as defined herein, alternatively Rₑ is not alkenyl or alkynyl;
L' is independently selected from covalent bond and C₁₋₆ alkylene, each of which is optionally substituted with 1, 2, 3 or 4 groups selected from: C₁₋₆ alkyl, C₁₋₆ haloalkyl, -L_{d}-3- to 7-membered carbocyclyl and -L_{d}-3- to 7-membered heterocyclyl;
L_{d} is independently selected from covalent bond and C₁₋₆ alkylene, alternatively selected from covalent bond and C₁₋₄ alkylene;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl;
each of R₁ₐ, R_{1b}, Z₁, Z₂, Z₄, Z₅ and R_{d3} is as defined herein;
wherein each of the above groups is optionally deuterated, up to completely deuterated.

In a more specific embodiment, the present disclosure provides a compound of the above formula (VI-3), or a stereoisomer, an N-oxide, a hydrate, a solvate, a metabolite, a pharmaceutically acceptable salt, a polymorph, or a prodrug thereof, wherein
E is selected from CRₑ₁₂ and N;
J is selected from CRₑ₁₃ and N;
Rₑ₉ is selected from H, D, halogen, C₁₋₆ alkyl, C₁₋₆ haloalkyl, C₂₋₆ alkenyl, C₂₋₆ alkynyl, -OR', -NR'R", 3- to 7-membered carbocyclyl, 3- to 7-membered heterocyclyl and -Y₅-C₀₋₆ alkylene-R₆, alternatively selected from H, D, halogen, C₁₋₆ alkyl, C₁₋₆ haloalkyl, C₂₋₆ alkenyl, C₂₋₆ alkynyl, -OR' and -NR'R";
Y₅ is selected from O and NR₅;
R₅ is selected from H, C₁₋₆ alkyl and C₁₋₆ haloalkyl;
R₆ is selected from 3- to 7-membered carbocyclyl and 3- to 7-membered heterocyclyl;
Rₑ₁₀ is selected from H, D, halogen, C₁₋₆ alkyl and C₁₋₆ haloalkyl;
Rₑ₁₁, Rₑ₁₂ and Rₑ₁₃ are H, D, C₁₋₆ alkyl or C₁₋₆ haloalkyl;
or Rₑ₉ and Rₑ₁₃ are taken together with the carbon atoms to which they are attached to form a 5- to 7-membered carbocyclyl or a 5- to 7-membered heterocyclyl, which is optionally substituted with 1, 2, 3 or 4 Rₑ;
or Rₑ₉ and Rₑ₁₀ are taken together with the carbon atoms to which they are attached to form a 5- to 7-membered carbocyclyl, a 5- to 7-membered heterocyclyl, a phenyl, or a 5- to 6-membered heteroaryl, which is optionally substituted with 1, 2, 3 or 4 Rₑ;
Rₑ is as defined herein, alternatively Rₑ is not alkenyl or alkynyl;
each of R' and R" is independently selected from H, C₁₋₆ alkyl, C₁₋₆ haloalkyl, 3- to 7-membered carbocyclyl and 3- to 7-membered heterocyclyl;
each of R₁ₐ, R_{1b}, Z₁, Z₂, Z₄, Z₅ and R_{d3} is as defined herein.

In some specific embodiments of the present disclosure, at least one of E and J is N; alternatively, only one of E and J is N.

In a more specific embodiment, the present disclosure provides a compound of the above formula (VI-3), or a stereoisomer, an N-oxide, a hydrate, a solvate, a metabolite, a pharmaceutically acceptable salt, a polymorph, or a prodrug thereof, wherein
E is selected from CRₑ₁₂ and N;
J is selected from CRₑ₁₃ and N;
Rₑ₉ is selected from H, D, C₁₋₆ alkyl, C₁₋₆ haloalkyl, C₂₋₄ alkynyl, -NR'R", 3- to 7-membered heterocyclyl and -O-C₀₋₄ alkylene-3- to 7-membered heterocyclyl, alternatively selected from H, D, C₁₋₆ alkyl, C₁₋₆ haloalkyl, C₂₋₄ alkynyl and -NR'R";
Rₑ₁₀ is selected from H, D, C₁₋₆ alkyl and C₁₋₆ haloalkyl;
Rₑ₁₁, Rₑ₁₂ and Rₑ₁₃ are H or D;
or Rₑ₉ and Rₑ₁₃ are taken together with the carbon atoms to which they are attached to form a 5- to 7-membered heterocyclyl, alternatively form dihydrooxazinyl, which is optionally substituted with 1, 2, 3 or 4 Rₑ;
or Rₑ₉ and Rₑ₁₀ are taken together with the carbon atoms to which they are attached to form a 5- to 7-membered carbocyclyl, a 5- to 7-membered heterocyclyl, or a 5- to 6-membered heteroaryl, alternatively form a 5- to 7-membered heterocyclyl or a 5- to 6-membered heteroaryl, alternatively form dihydrooxazinyl or 5-membered azaaryl, which is optionally substituted with 1, 2, 3 or 4 Rₑ;
Rₑ is as defined herein, alternatively Rₑ is not alkenyl or alkynyl; alternatively, Rₑ is independently selected from H, D, halogen, C₁₋₆ alkyl, C₁₋₆ haloalkyl, -OR' and -NR'R", alternatively selected from H, D, C₁₋₆ alkyl, C₁₋₆ haloalkyl and -OR', alternatively selected from Me and OMe;
each of R' and R" is independently selected from H, C₁₋₆ alkyl and C₁₋₆ haloalkyl;
each of R₁ₐ, R_{1b}, Z₁, Z₂, Z₄, Z₅ and R_{d3} is as defined herein.

In some specific embodiments of the present disclosure, Rₑ is independently selected from H, D, C₁₋₆ alkyl and C₁₋₆ haloalkyl, alternatively H or Me.

In a more specific embodiment, the present disclosure provides a compound of the above formula (VI-3), or a stereoisomer, an N-oxide, a hydrate, a solvate, a metabolite, a pharmaceutically acceptable salt, a polymorph, or a prodrug thereof, wherein
E is selected from CRₑ₁₂ and N;
J is selected from CRₑ₁₃ and N;
Rₑ₉ is selected from H, -NHMe, 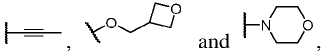 alternatively selected from H, -NHMe and 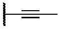 ;
Rₑ₁₀ is selected from H and Me;
Rₑ₁₁, Rₑ₁₂ and Rₑ₁₃ are H;
or Rₑ₉ and Rₑ₁₃ are taken together with the carbon atoms to which they are attached to form 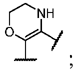
or Rₑ₉ and Rₑ₁₀ are taken together with the carbon atoms to which they are attached to form 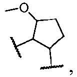 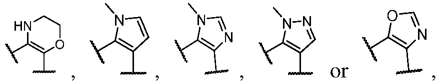 alternatively form 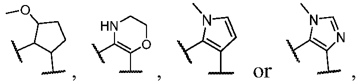 alternatively form 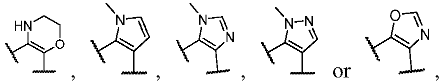 alternatively form 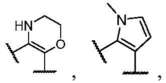 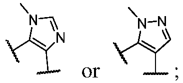
each of R₁ₐ, R_{1b}, Z₁, Z₂, Z₄, Z₅ and R_{d3} is as defined herein.

In some specific embodiments of the present disclosure, 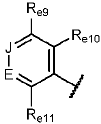 is selected from: 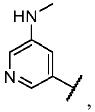 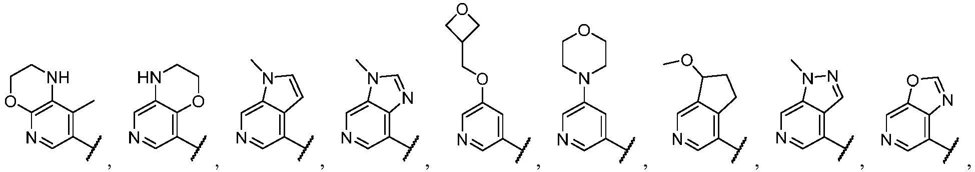 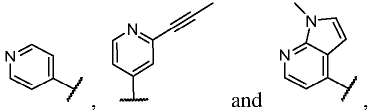 alternatively selected from: 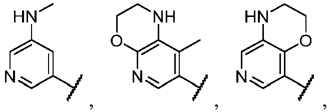 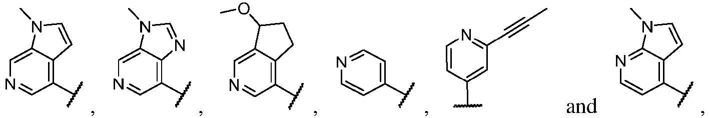 alternatively selected from: 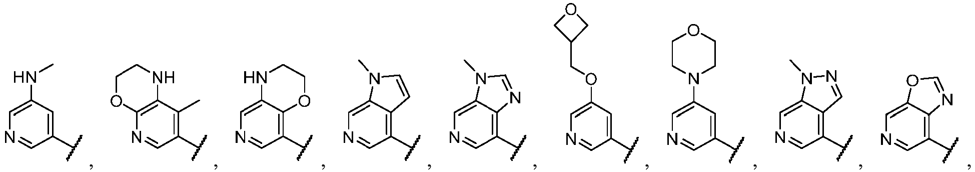 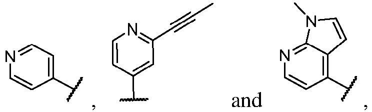 alternatively selected from: 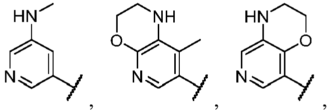 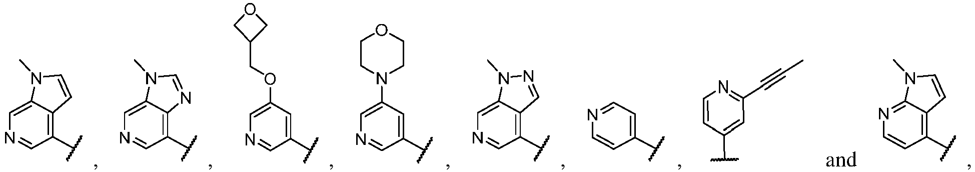 alternatively selected from: 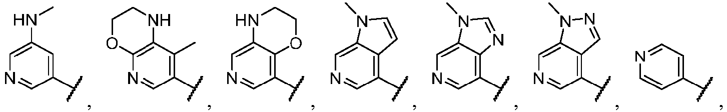 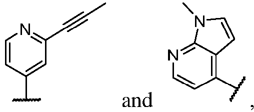 alternatively

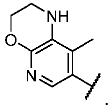

In a more specific embodiment, the present disclosure provides a compound of the above formula (I), or a stereoisomer, an N-oxide, a hydrate, a solvate, a metabolite, a pharmaceutically acceptable salt, a polymorph, or a prodrug thereof, and the compound is selected from:

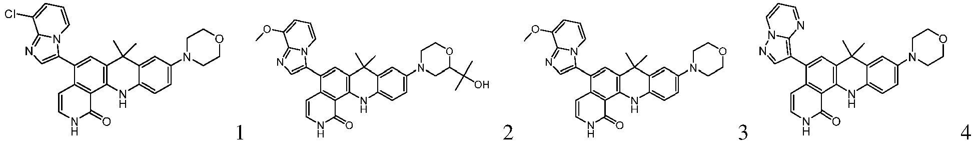

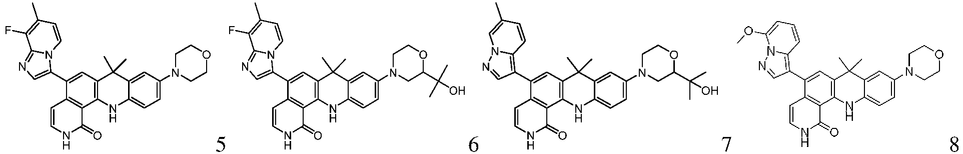

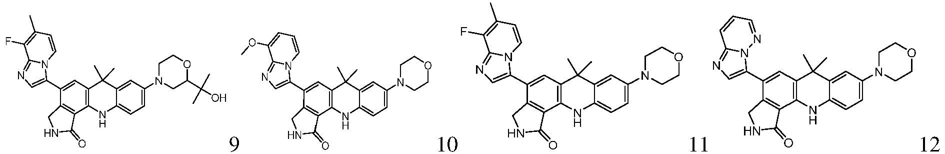

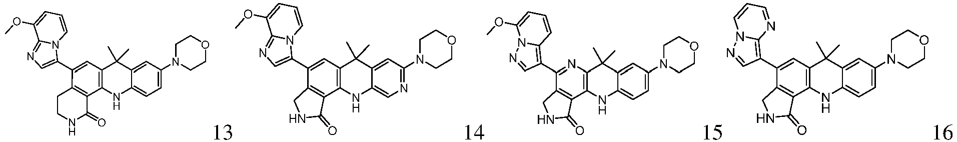

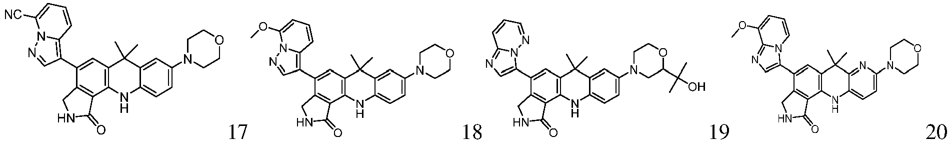

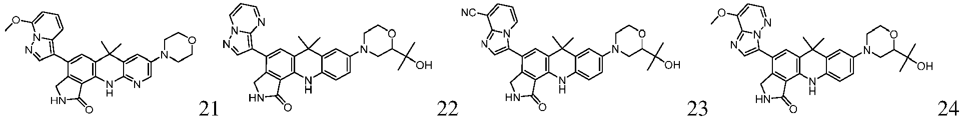

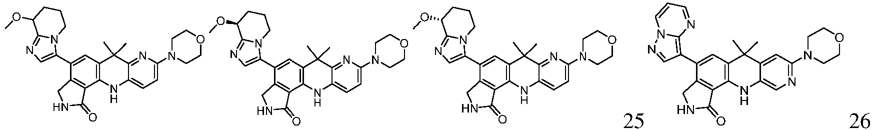

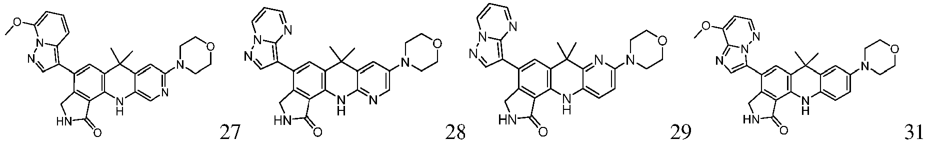

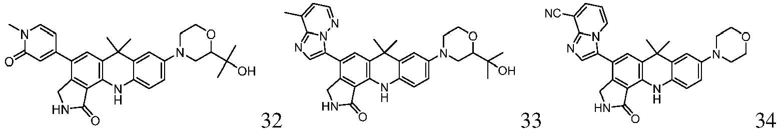

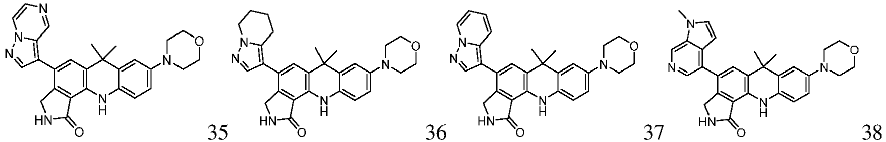

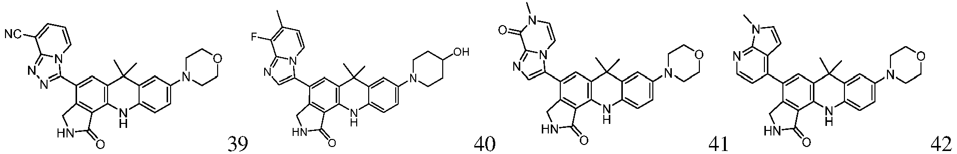

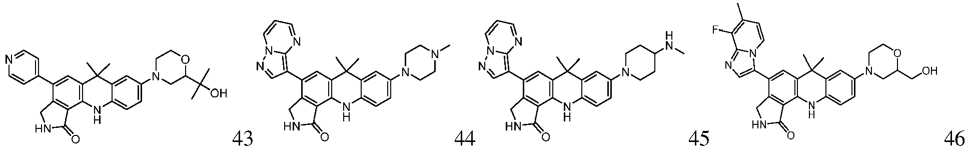

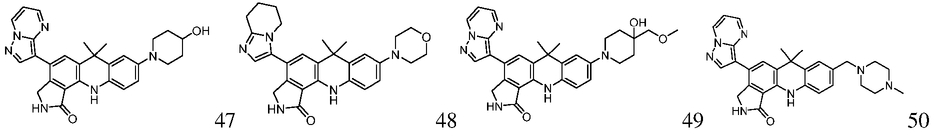

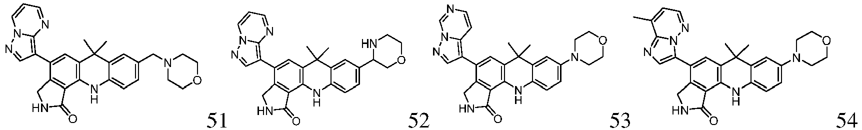

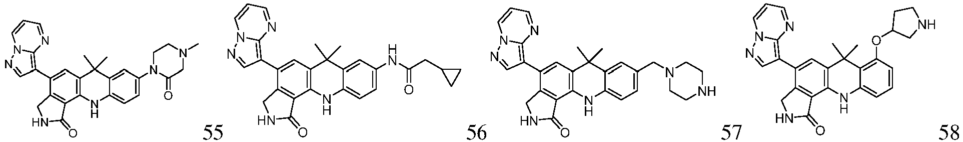

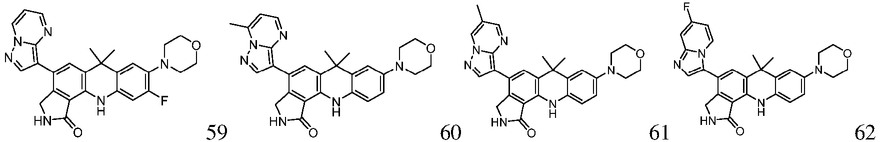

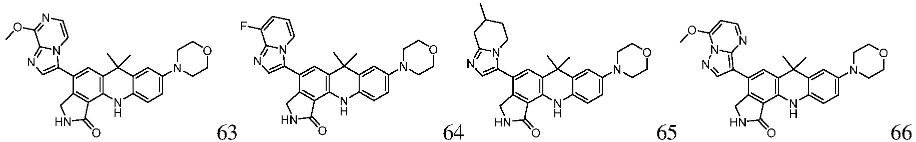

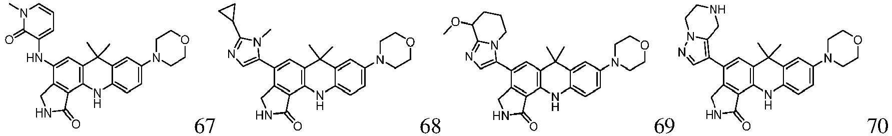

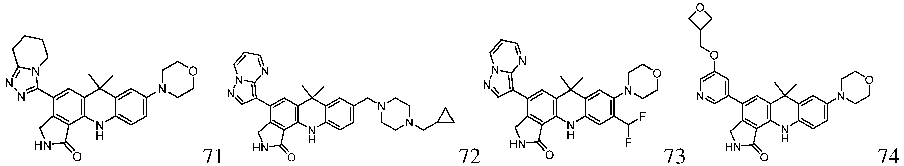

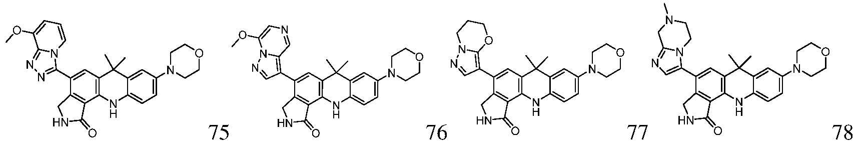

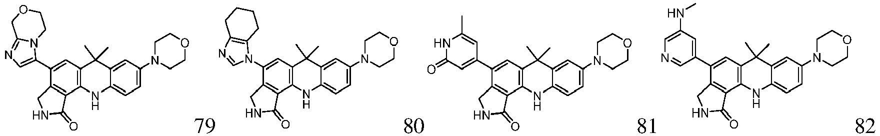

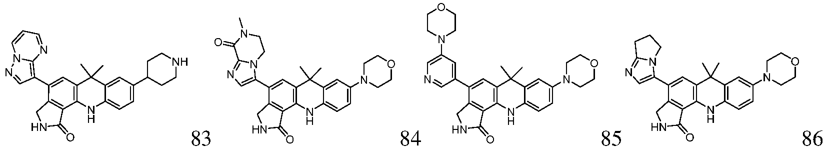

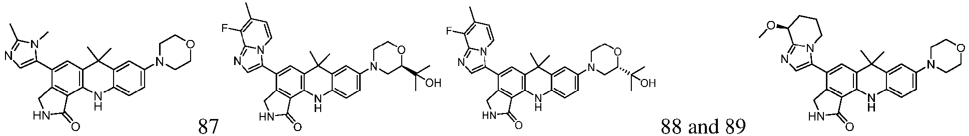

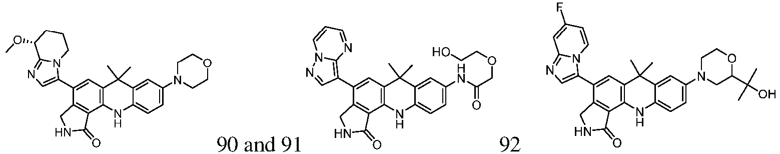

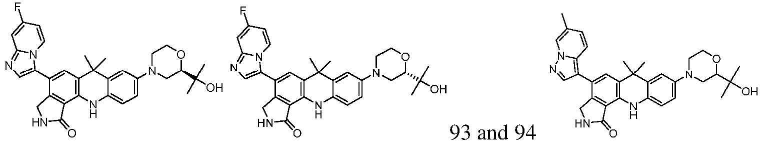

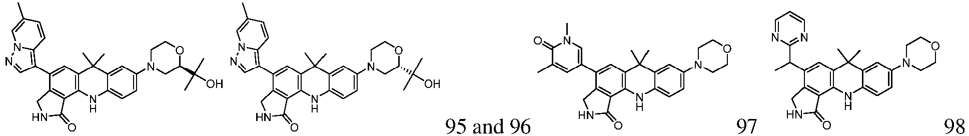

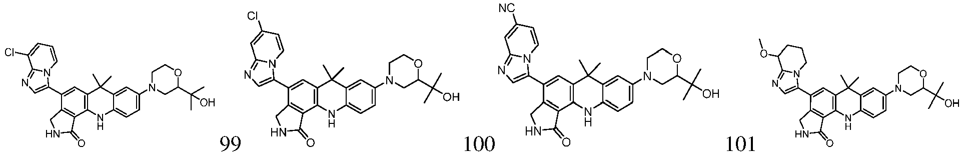

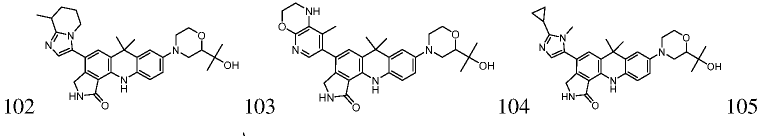

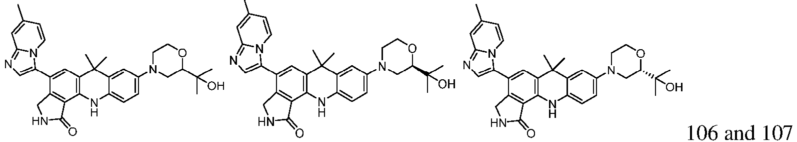

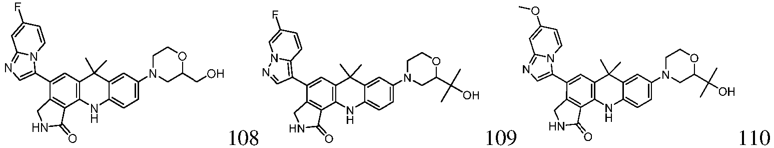

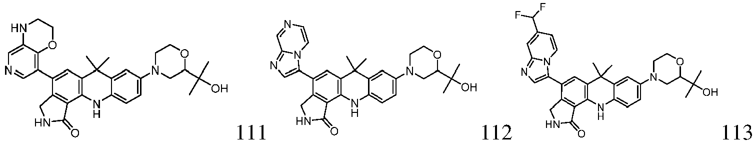

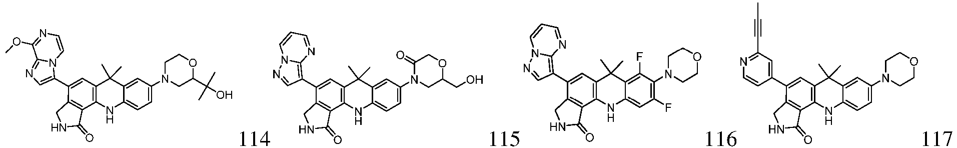

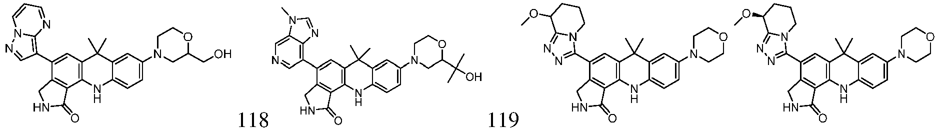

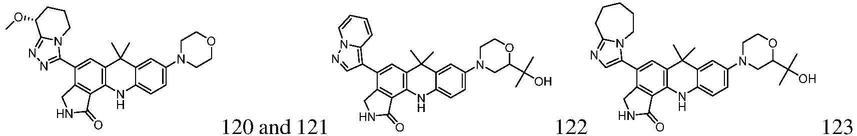

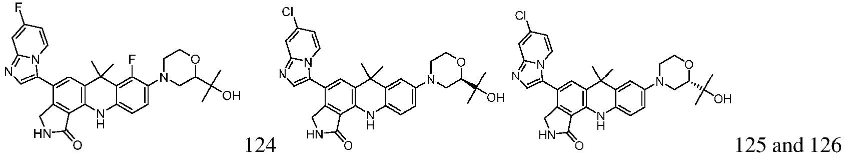

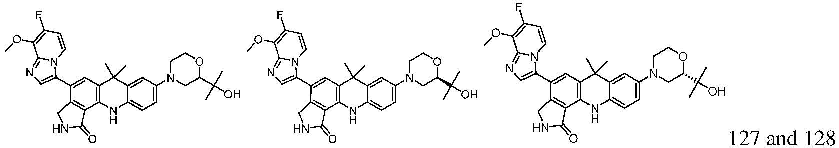

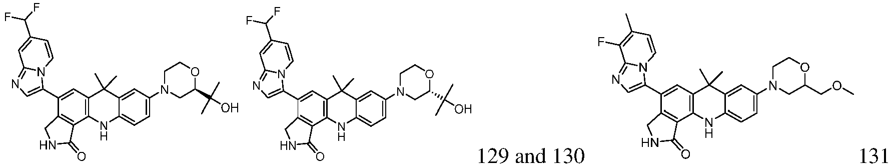

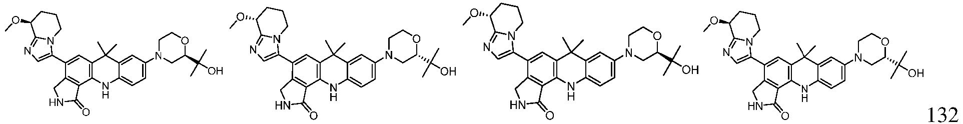

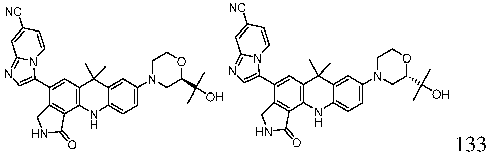

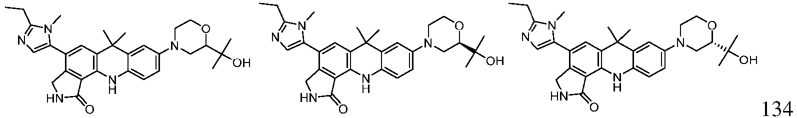

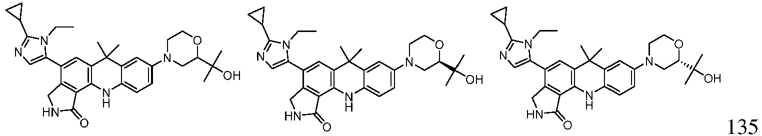

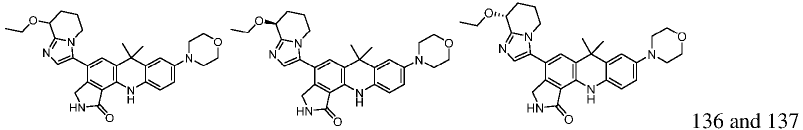

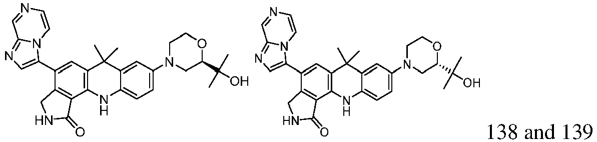

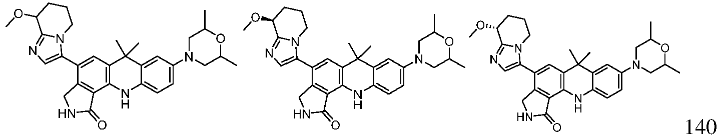

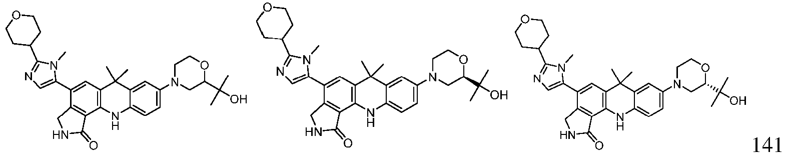

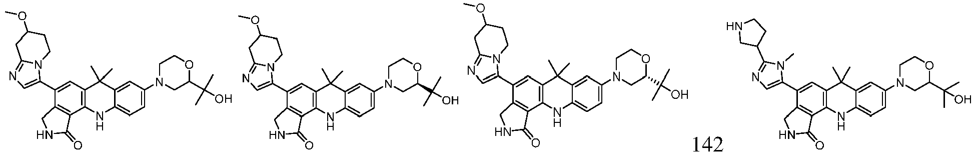

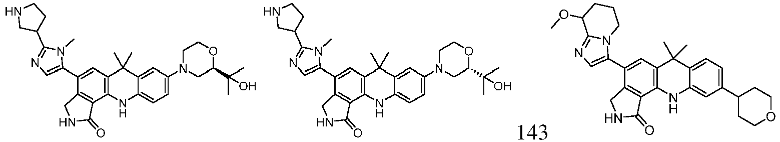

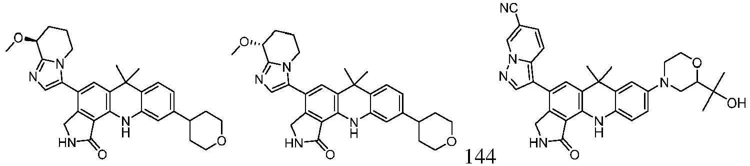

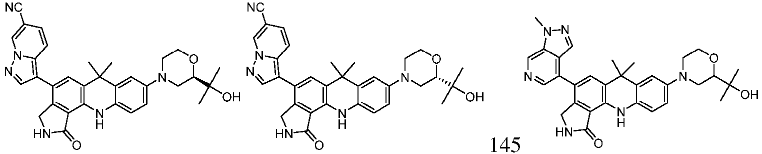

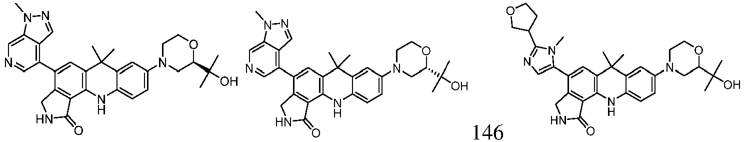

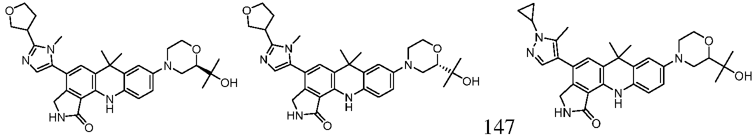

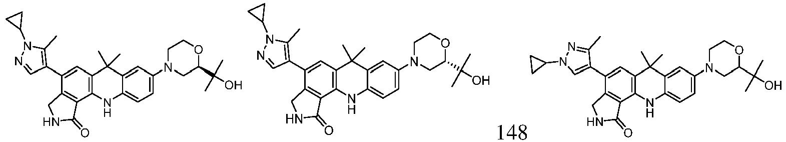

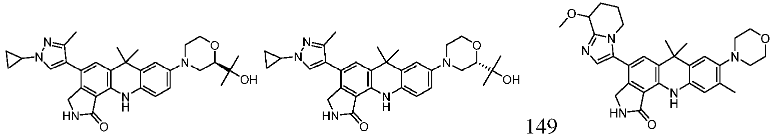

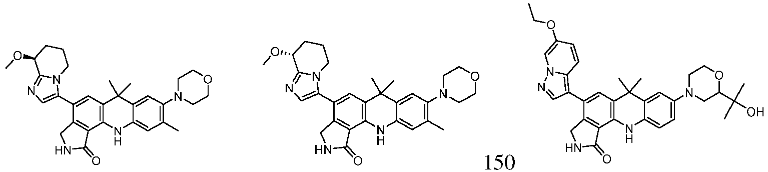

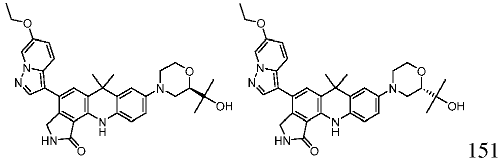

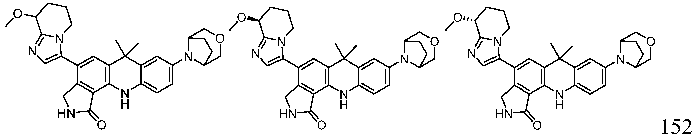

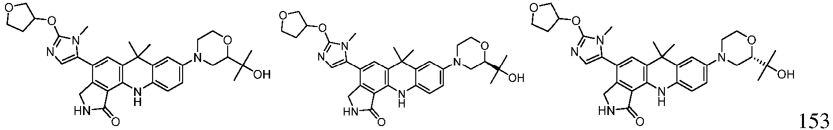

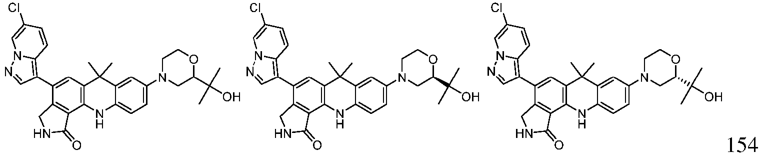

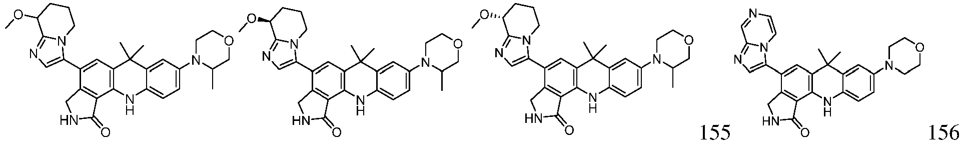

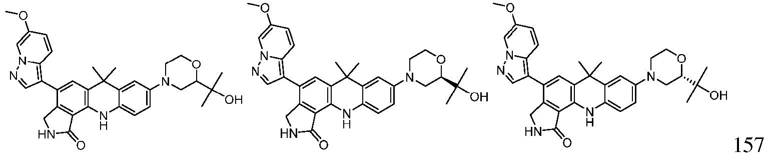

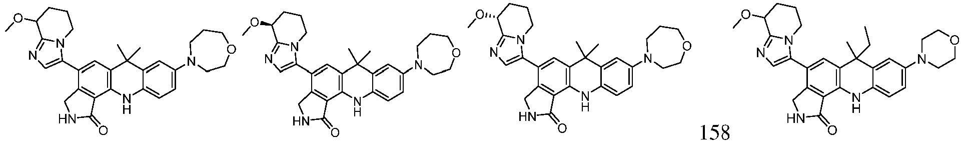

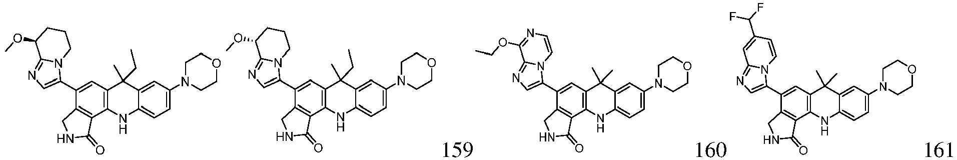

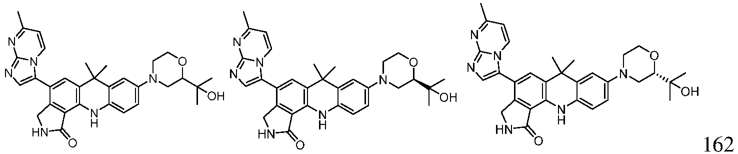

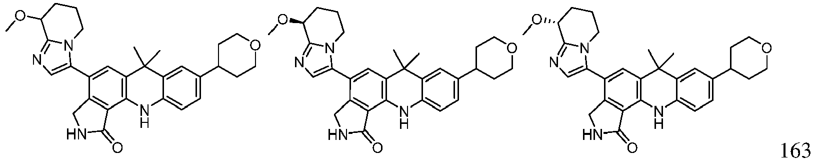

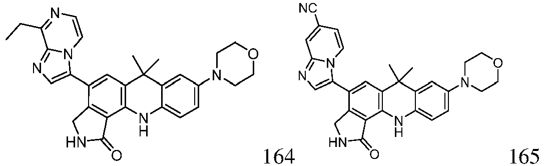

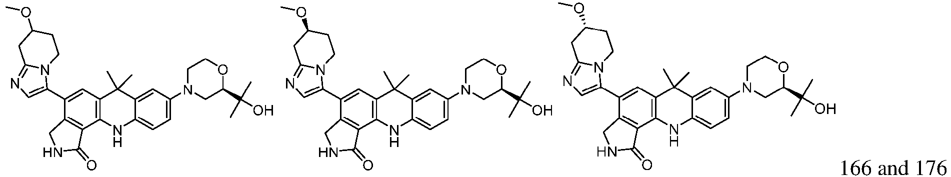

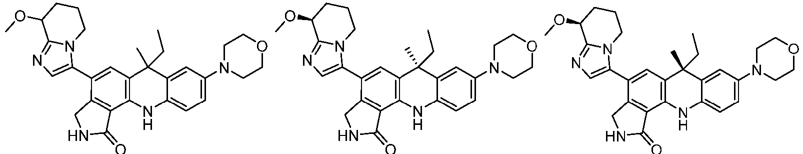

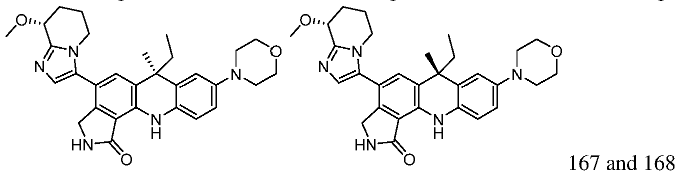

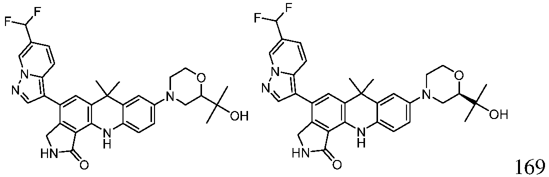

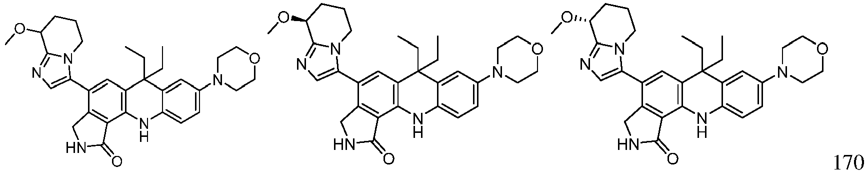

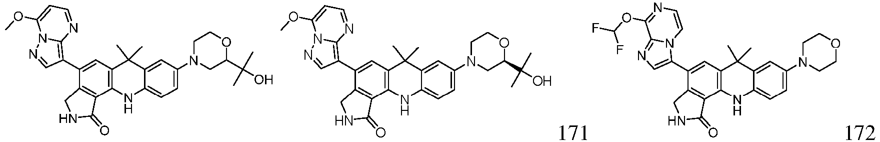

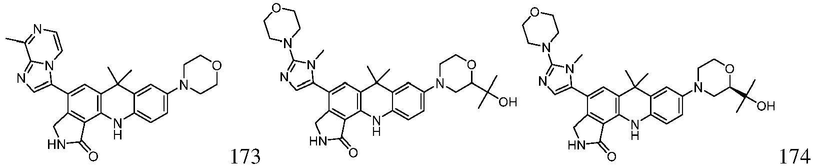

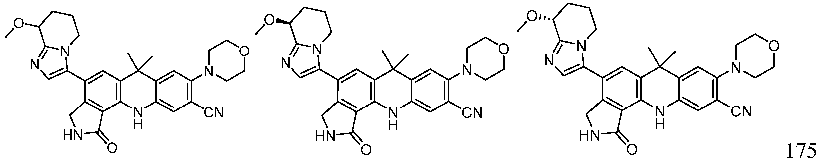

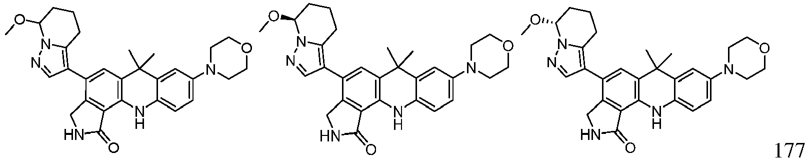

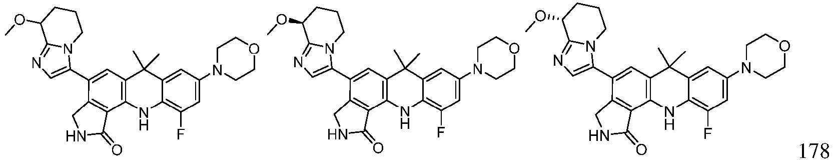

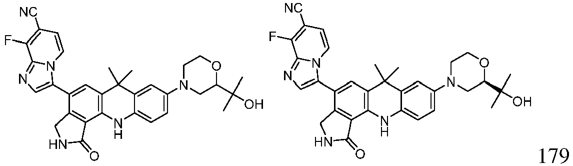

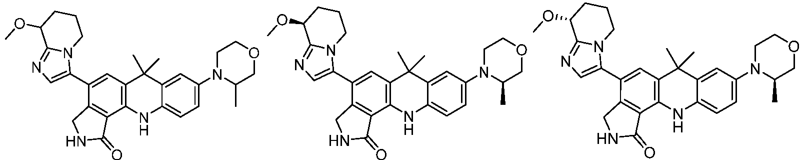

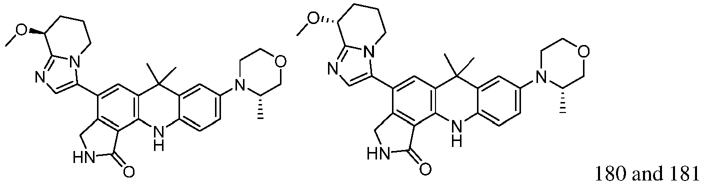

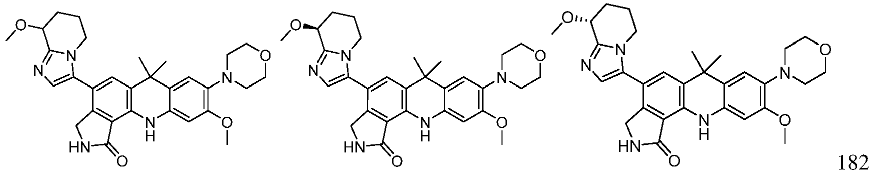

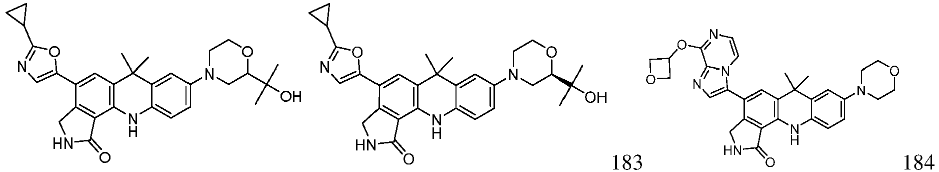

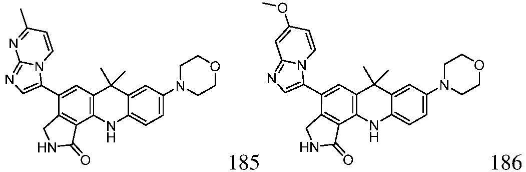

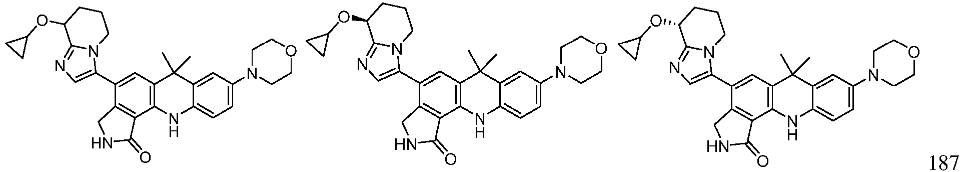

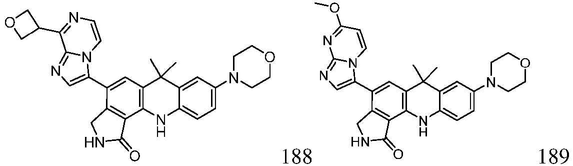

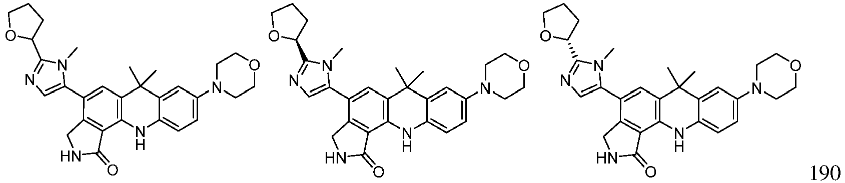

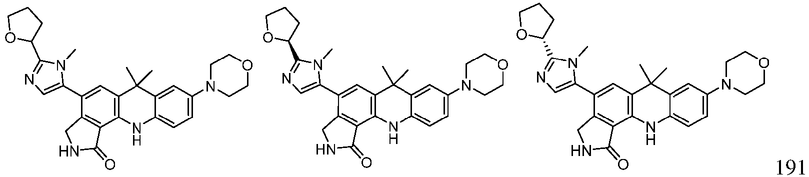

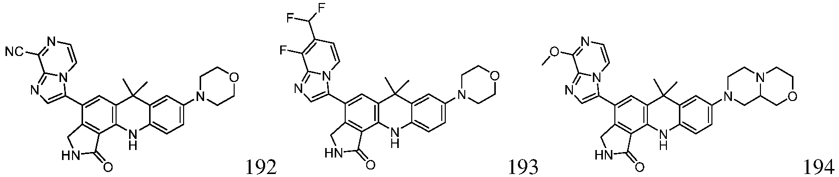

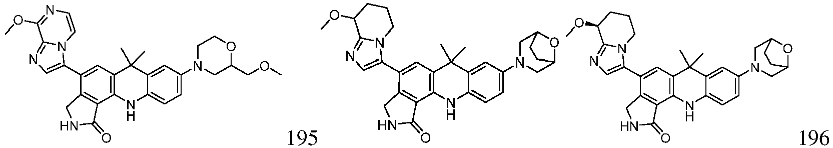

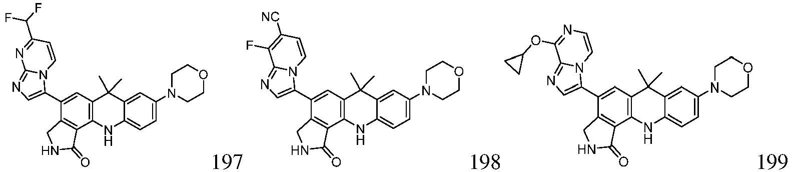

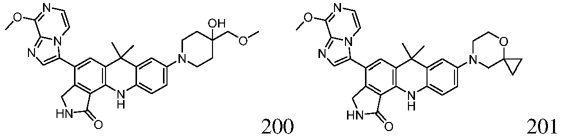

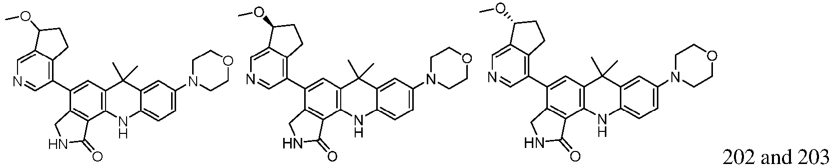

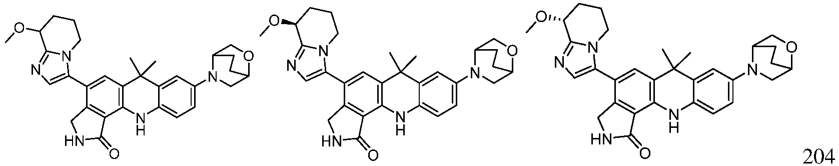

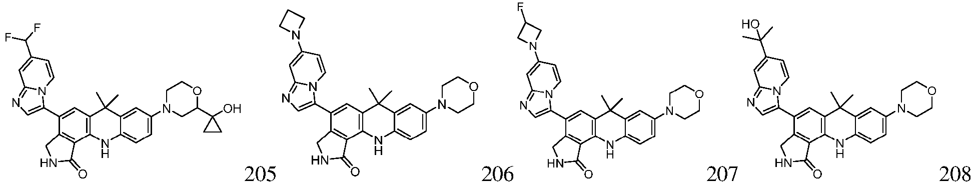

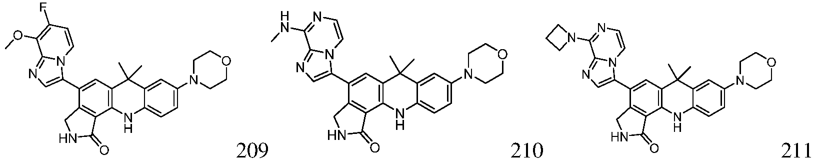

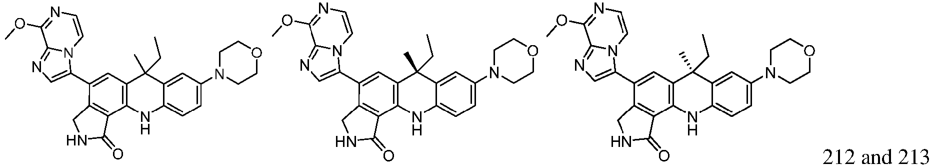

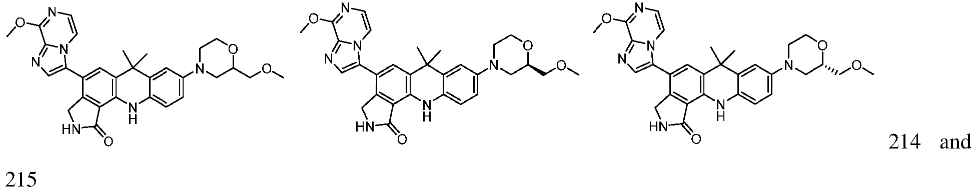

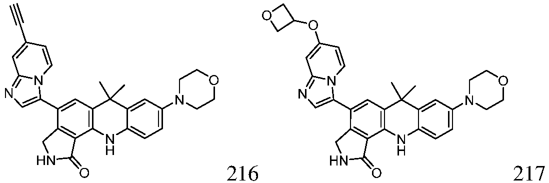

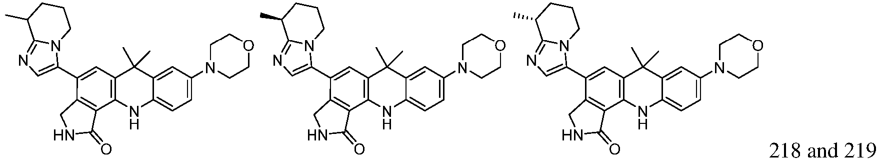

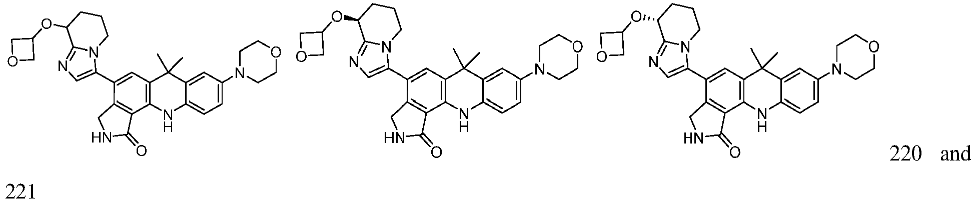

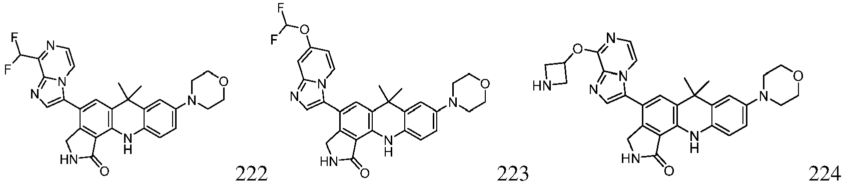

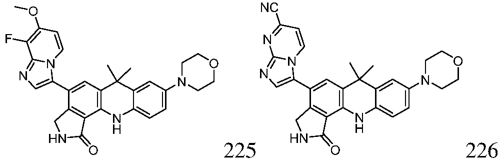

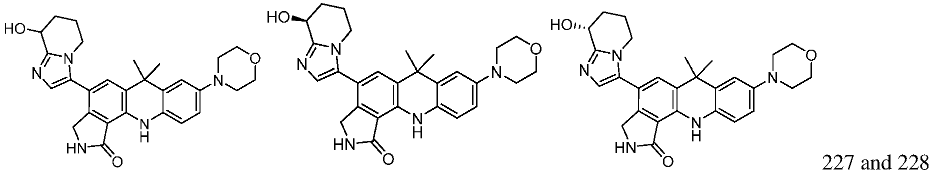

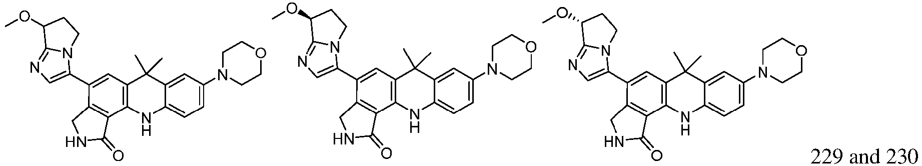

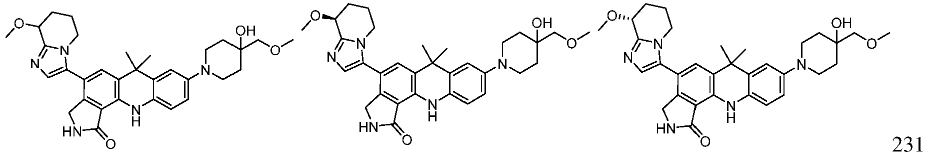

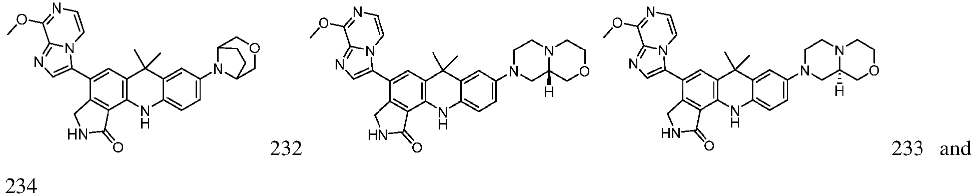

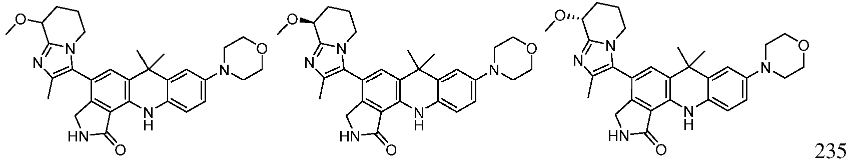

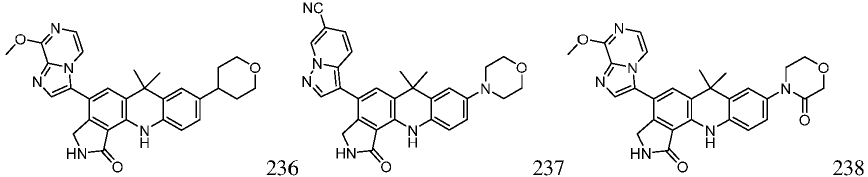

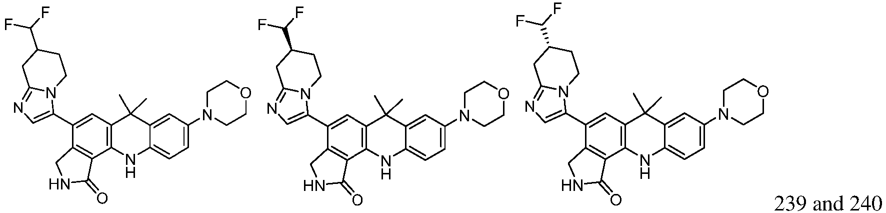

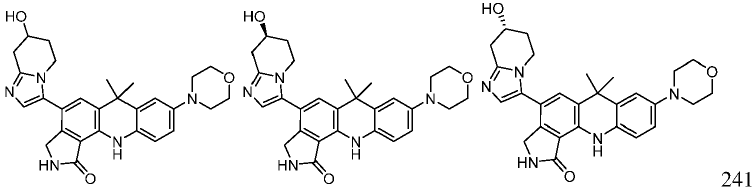

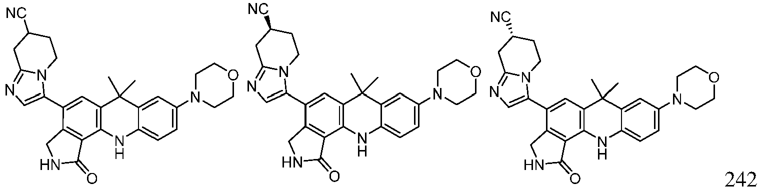

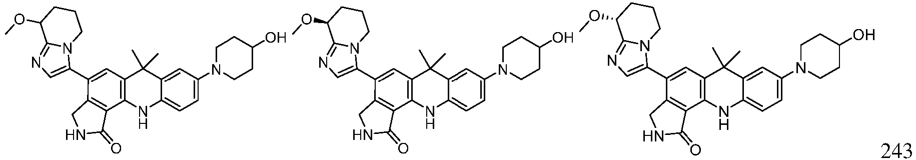

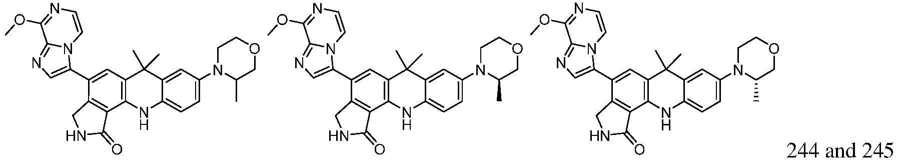

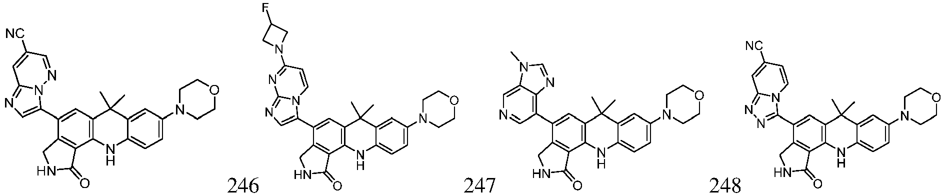

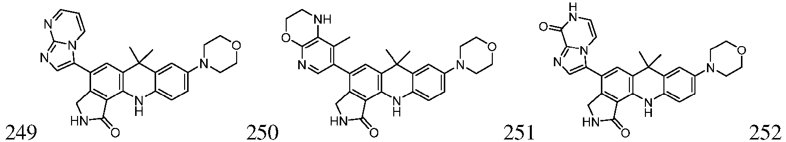

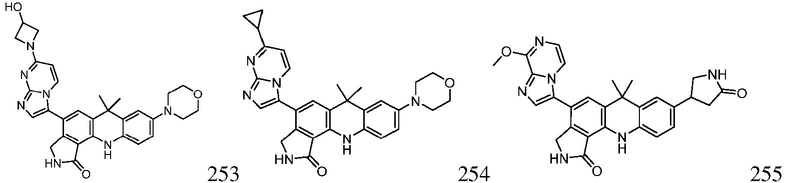

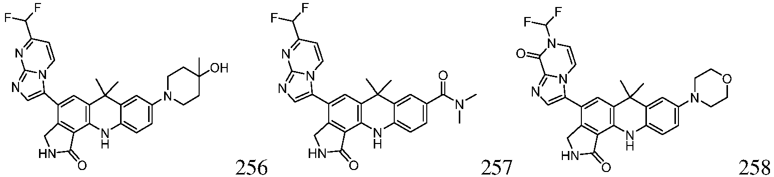

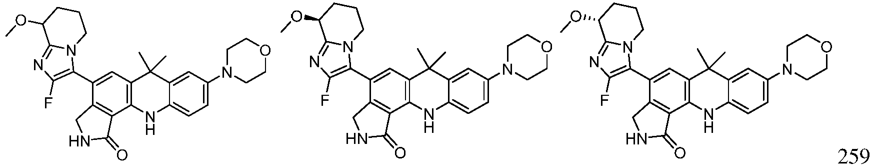

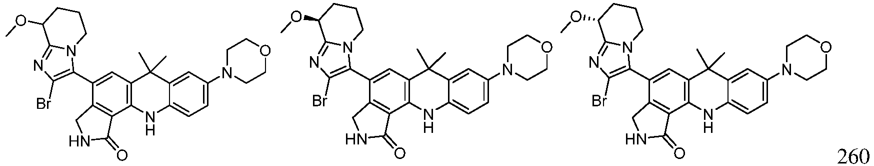

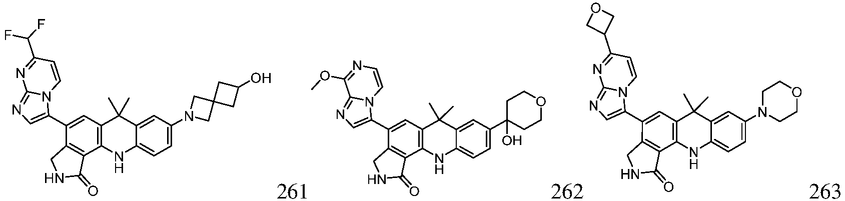

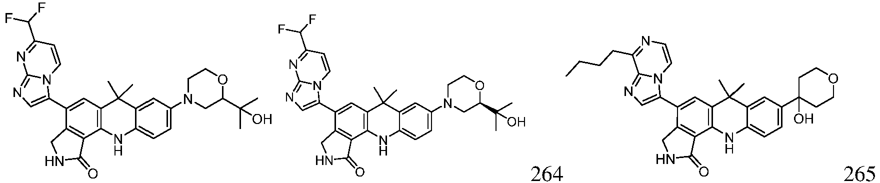

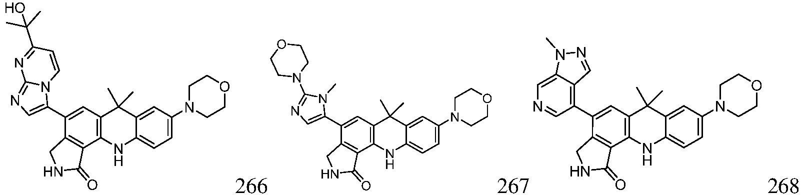

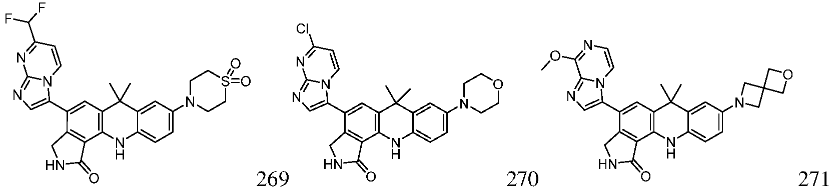

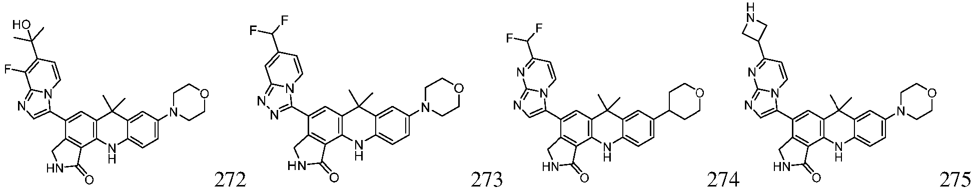

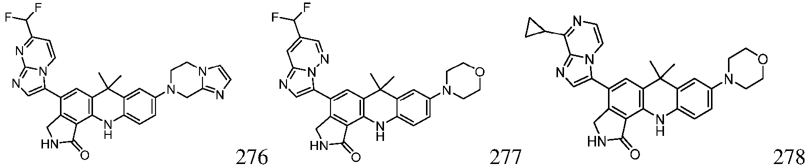

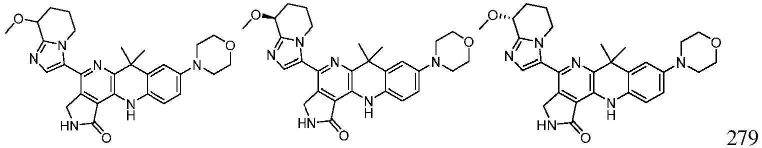

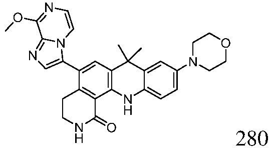

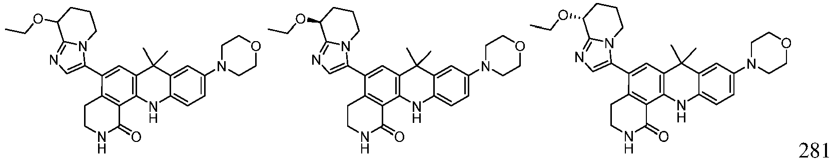

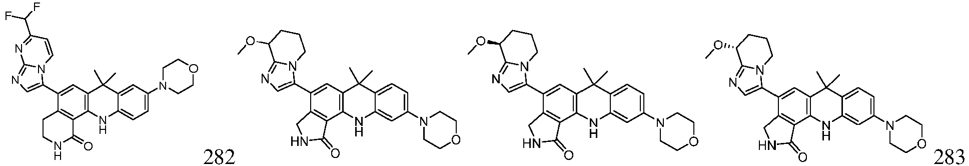

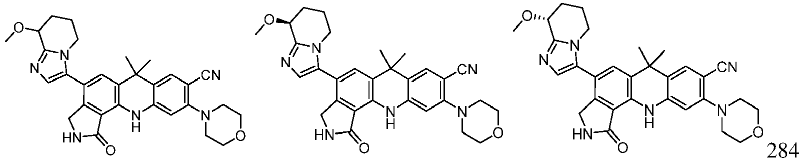

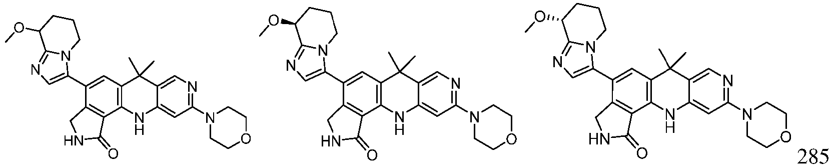

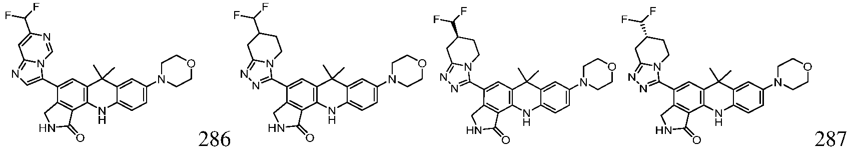

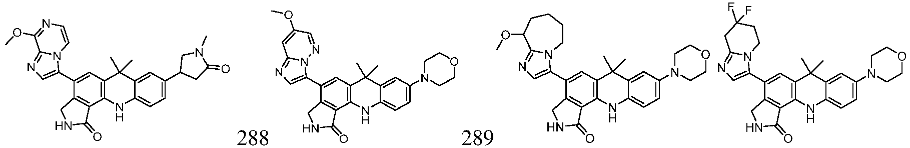

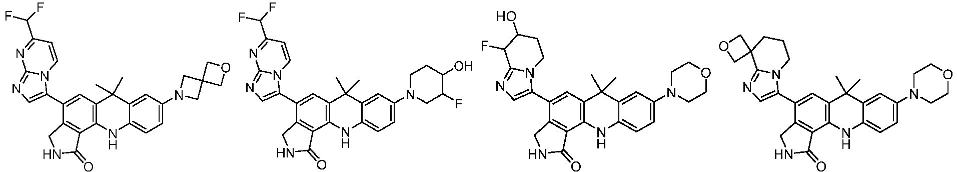

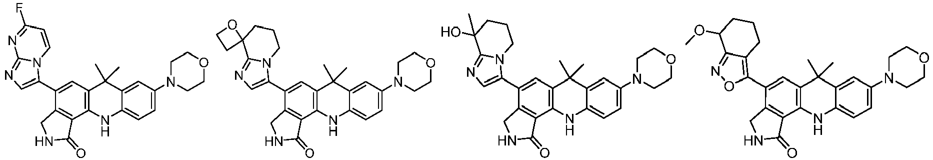

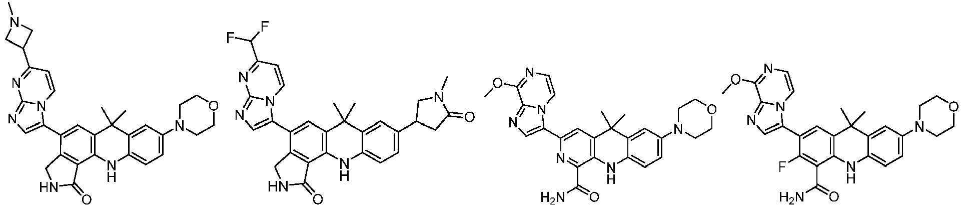

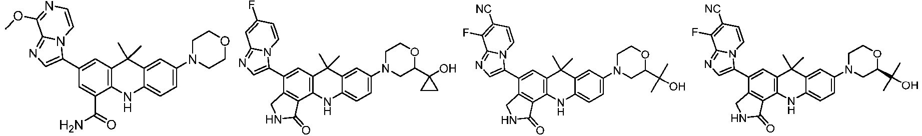

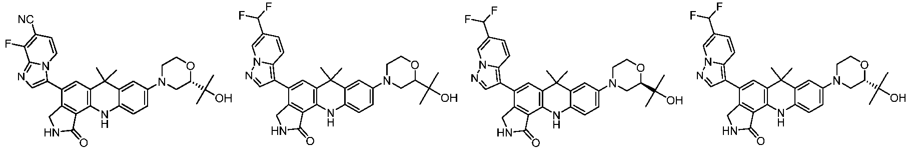

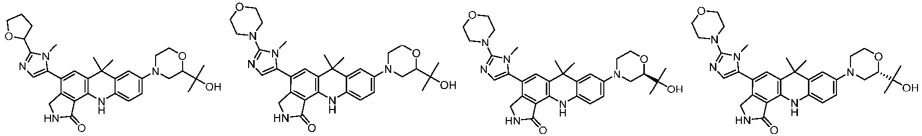

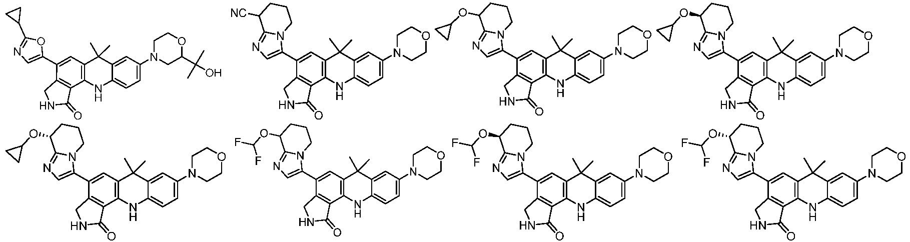

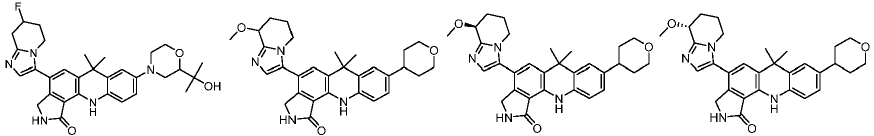

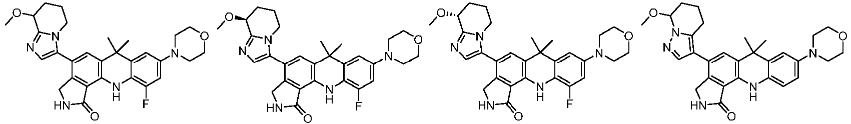

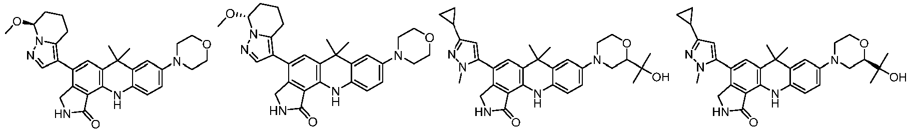

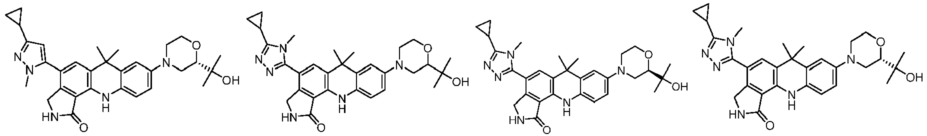

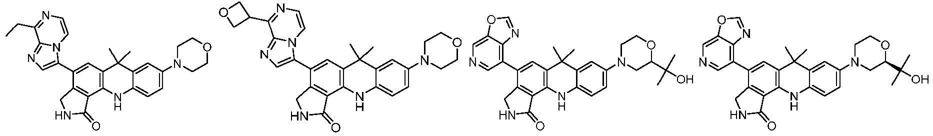

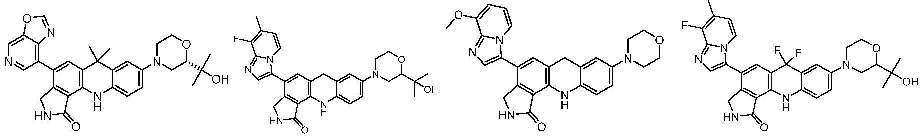

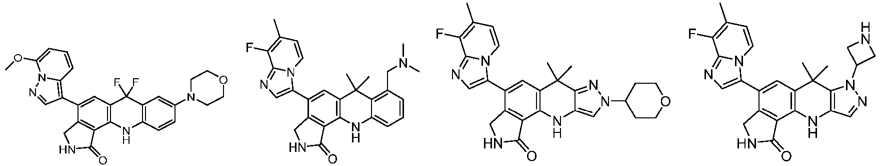

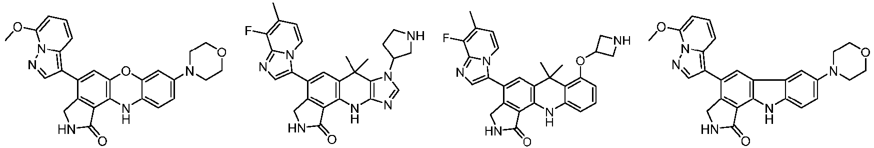

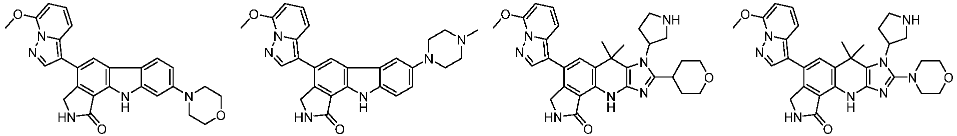

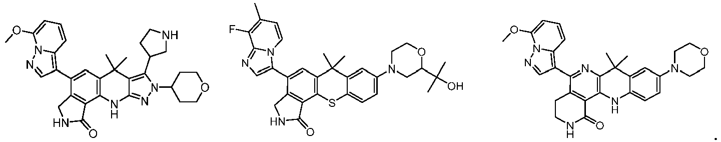

In a more specific embodiment, the present disclosure provides a compound of the above formula (I), or a stereoisomer, an N-oxide, a hydrate, a solvate, a metabolite, a pharmaceutically acceptable salt, a polymorph, or a prodrug thereof, and the compound is selected from:

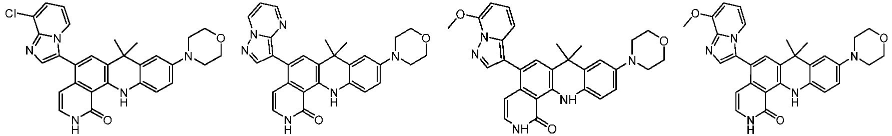

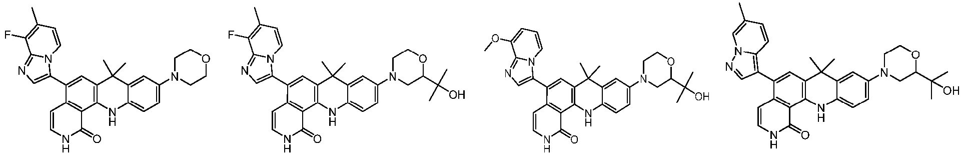

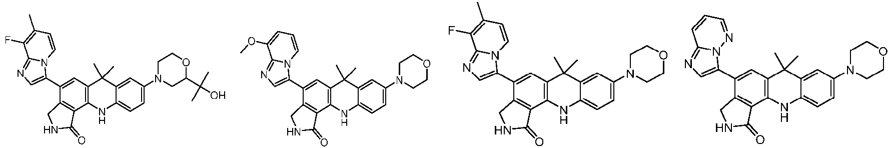

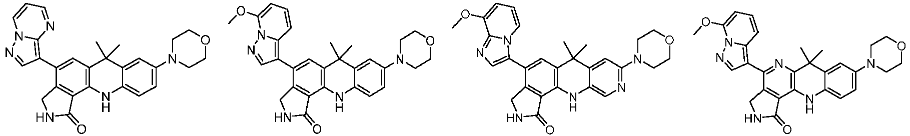

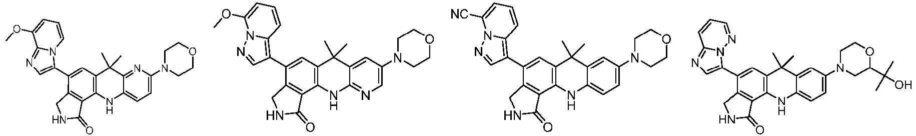

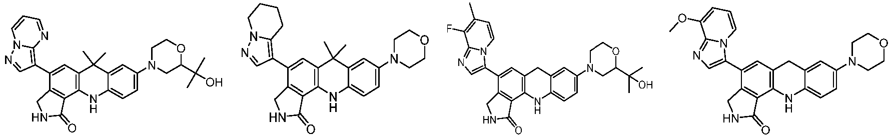

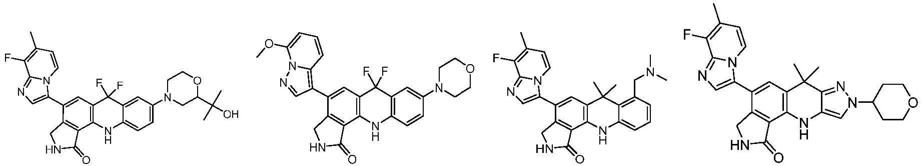

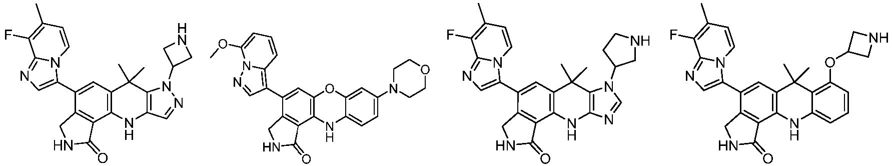

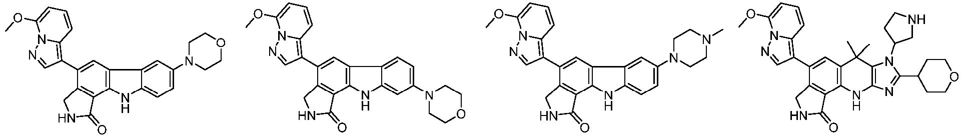

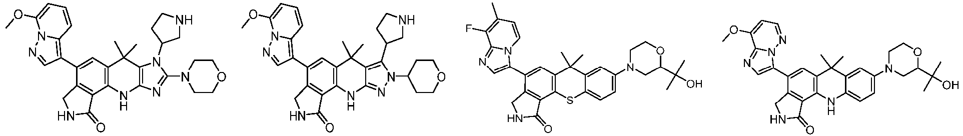

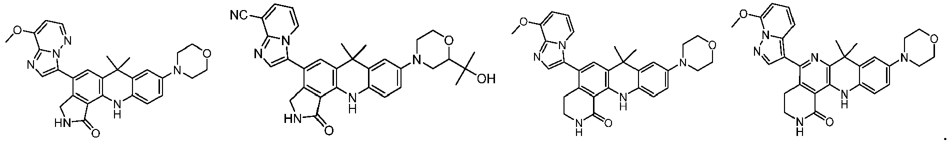

In one embodiment, the present disclosure relates to a pharmaceutical composition, comprising the compound of the present disclosure, or a stereoisomer, an N-oxide, a hydrate, a solvate, a metabolite, a pharmaceutically acceptable salt, a polymorph, or a prodrug thereof, and a pharmaceutically acceptable carrier, adjuvant or vehicle.

In one embodiment, the present disclosure relates to use of a compound of the present disclosure, or a stereoisomer, an N-oxide, a hydrate, a solvate, a metabolite, a pharmaceutically acceptable salt, a polymorph, or a prodrug thereof, or a pharmaceutical composition comprising the same, in the manufacture of a medicament for the prevention and/or treatment of an HPK1-mediated disorder, disease or condition.

In one embodiment, the present disclosure relates to a compound of the present disclosure, or a stereoisomer, an N-oxide, a hydrate, a solvate, a metabolite, a pharmaceutically acceptable salt, a polymorph, or a prodrug thereof, or a pharmaceutical composition comprising the same, for use in the prevention and/or treatment of an HPK1-mediated disorder, disease or condition.

In one embodiment, the present disclosure relates to a method of preventing and/or treating an HPK1-mediated disorder, disease or condition, comprising administering a compound of the present disclosure, or a stereoisomer, an N-oxide, a hydrate, a solvate, a metabolite, a pharmaceutically acceptable salt, a polymorph, or a prodrug thereof, or a pharmaceutical composition comprising the same to a subject.

Furthermore, the disorder, disease or condition is a proliferative disorder.

Furthermore, the proliferative disorder is a cancer.

Furthermore, the proliferative disorder is associated with one or more activating mutations in HPK1.

In one embodiment, the disorder, disease or condition is a chronic viral infection.

In one embodiment, the present disclosure relates to use of a compound of the present disclosure, or a stereoisomer, an N-oxide, a hydrate, a solvate, a metabolite, a pharmaceutically acceptable salt, a polymorph, or a prodrug thereof, or a pharmaceutical composition comprising the same, in the manufacture of a medicament for increasing the efficacy of a vaccination.

In one embodiment, the present disclosure relates to a compound, or a stereoisomer, an N-oxide, a hydrate, a solvate, a metabolite, a pharmaceutically acceptable salt, a polymorph, or a prodrug thereof, or a pharmaceutical composition comprising the same, for use in increasing the efficacy of a vaccination.

In one embodiment, the present disclosure relates to a method of increasing the efficacy of a vaccination, comprising administering a compound of the present disclosure, or a stereoisomer, an N-oxide, a hydrate, a solvate, a metabolite, a pharmaceutically acceptable salt, a polymorph, or a prodrug thereof, or a pharmaceutical composition comprising the same to a subject.

### Pharmaceutical compositions and kits

In one aspect, the present disclosure provides a pharmaceutical composition, comprising a compound of the present disclosure (also referred to as the "active ingredient") and a pharmaceutically acceptable carrier, adjuvant, or vehicle. In certain embodiments, the pharmaceutical composition comprises an effective amount of the compound of the present disclosure. In some embodiments, the pharmaceutical composition comprises a therapeutically effective amount of the compound of the present disclosure. In certain embodiments, the pharmaceutical composition comprises a prophylactically effective amount of the compound of the present disclosure.

A pharmaceutically acceptable carrier, adjuvant, or vehicle for use in the present disclosure refers to a non-toxic carrier, adjuvant, or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

Suitable formulations for administering the compounds of the present disclosure will be apparent to those skilled in the art, and include, for example, tablets, pills, capsules, suppositories, lozenges, troches, solutions (particularly the solutions for injection (subcutaneous, intravenous or intramuscular) and infusion (injection)), elixirs, syrups, cachets, emulsions, inhalants or dispersible powders. If necessary, the indicated dose may be administered several times per day.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other suitable containers) containing the compound disclosed herein or other therapeutic agents. In some embodiments, kits provided can also optionally include a third container containing a pharmaceutically acceptable excipient for diluting or suspending the compound disclosed herein and/or other therapeutic agents. In some embodiments, the compound disclosed herein provided in the first container and the other therapeutic agents provided in the second container is combined to form a unit dosage form.

### Use

The compound and composition described herein is generally useful for inhibiting the kinase activity of one or more kinases. In some embodiments, the kinase inhibited by the compound and method of the present disclosure is HPK1.

The compound disclosed herein can be used to inhibit the activity of HPK1. HPK1 is a member of the germinal center kinase subfamily of Ste20-related serine/threonine kinases. HPK1 exerts the function of MAP4K by phosphorylating and activating MAP3K proteins (including MEKK1, MLK3 and TAK1), and causing the activation of MAPK Jnk.

In one embodiment, the subject matter disclosed herein relates to a method of inhibiting HPK1, comprising contacting HPK1 with an effective amount of a compound or a pharmaceutical composition of the present disclosure.

In some embodiments, the subject matter disclosed herein relates to a method of enhancing the immune response of a subject in need, wherein the method comprises administering an effective amount of a compound of the present disclosure or a pharmaceutical composition of the present disclosure to the subject. In some aspects of this embodiment, T cells of the subject have at least one of the following compared with those before the administration of the compound or pharmaceutical composition: enhanced stimulation, enhanced activation, enhanced migration, enhanced proliferation, enhanced survival rate, and enhanced cytolytic activity. In some aspects of this embodiment, T cell activation is characterized by an increase in the occurrence rate of γ-IFN+CD8 T cells or an enhanced production of IL-2 or granzyme B by T cells compared with that before the administration of the compound or pharmaceutical composition. In some aspects of this embodiment, the number of T cells increases compared with that before the administration of the compound or pharmaceutical composition. In some aspects of this embodiment, T cells are antigen-specific CD8 T cells. In some aspects of this embodiment, the antigen presenting cells of the subject have enhanced maturation and activation compared with those before the administration of the compound or pharmaceutical composition. In some aspects of this embodiment, the antigen presenting cells are dendritic cells. In some aspects of this embodiment, the maturation of the antigen presenting cells is characterized by an increase in the occurrence rate of CD83+ dendritic cells. In some aspects of this embodiment, the activation of the antigen presenting cells is characterized by an increased expression of CD80 and CD86 on dendritic cells.

The compound disclosed herein directly binds to HPK1 and inhibits the kinase activity of HPK1. In some embodiments, the compound disclosed herein reduces, inhibits or otherwise decreases the HPK1-mediated phosphorylation of SLP76 and/or Gads.

The compound disclosed herein may or may not be a specific HPK1 antagonist. A specific HPK1 antagonist reduces the biological activity of HPK1 by an amount that is statistically greater than the inhibitory effect of the antagonist on any other protein (e.g., other serine/threonine kinases). In some embodiments, the compound disclosed herein specifically inhibits the serine/threonine kinase activity of HPK1. In some of these embodiments, the IC₅₀ of the HPK1 antagonist against HPK1 is about 90%, 80%, 70%, 60%, 50%, 40%, 30%, 20%, 10%, 0.1%, 0.01%, 0.001%, or less of the IC₅₀ of the HPK1 antagonist against another serine/threonine kinase or another type of kinase (e.g., tyrosine kinase).

The compound disclosed herein can be used in a method of inhibiting HPK1. Such methods comprise contacting HPK1 with an effective amount of a compound of the present disclosure. "Contacting" means that the compound is sufficiently close to isolated HPK1 or cells expressing HPK1 (e.g., T cells, B cells, or dendritic cells) to enable the compound to bind to HPK1 and inhibit the activity of HPK1. The compound can be contacted with HPK1 in vitro or in vivo by administering the compound to a subject.

Any method known in the art for measuring the kinase activity of HPK can be used to determine whether HPK1 has been inhibited, including in vitro kinase assays, immunoblotting with antibodies specific for phosphorylation targets of HPK1 (such as SLP76 and Gads), or measurement of downstream biological effects of HPK1 kinase activity, such as recruitment of 14-3-3 protein to phosphorylated SLP7 and Gads, release of SLP76-Gads-14-3-3 complex from LAT-containing microclusters, or activation of T or B cells.

The compound disclosed herein can be used to treat an HPK1-dependent condition. As used herein, "HPK1-dependent condition" is a pathological condition in which HPK1 activity is required for the cause or maintenance of the pathological condition. In some embodiments, the HPK1-dependent condition is a cancer.

The compound disclosed herein can also be used to enhance the immune response of a subject in need. Such methods comprise administering an effective amount of a compound of the present disclosure.

As used herein, "enhancing the immune response" refers to an improvement of any immunogenic response to an antigen. Non-limiting examples of the improvement of immunogenic response to an antigen include enhanced maturation or migration of dendritic cells, enhanced activation of T cells (e.g., CD4 T cells, CD8 T cells), enhanced proliferation of T cells (e.g., CD4 T cells, CD8 T cells), enhanced proliferation of B cells, increased survival rate of T cells and/or B cells, improved antigen presentation via antigen presenting cells (e.g., dendritic cells), improved clearance rate of antigens, increased production of cytokines (e.g., interleukin-2) via T cells, increased resistance to the immunosuppression induced by prostaglandin E2, and enhanced activation and/or cytolytic activity of CD8 T cells.

In some embodiments, the antigen presenting cells of an individual have enhanced maturation and activation compared with those before the administration of a compound of the present disclosure or a pharmaceutically acceptable salt, a prodrug, or a metabolite thereof. In some embodiments, the antigen presenting cells are dendritic cells. In some embodiments, the maturation of the antigen presenting cell is characterized by an increased occurrence rate of CD83+dendritic cells. In some embodiments, the antigen presenting cell activation is characterized by an increased expression of CD80 and CD86 on dendritic cells.

The compound of the present disclosure, or a stereoisomer, an N-oxide, a hydrate, a solvate, a metabolite, a pharmaceutically acceptable salt, a polymorph, or a prodrug thereof, and a pharmaceutically acceptable are suitable for the treatment of a T cell dysfunctional condition. "T cell dysfunctional condition" is a T cell condition or disorder characterized by a reduced response to an antigenic stimulation. In a specific embodiment, the T cell dysfunctional condition is a condition specifically associated with increased kinase activity of HPK1. In another embodiment, the T cell dysfunction condition is a condition in which the T cell capacity is lost or the ability to secrete cytokines, proliferate or perform cytolytic activity is reduced. In a specific aspect, the reduced response may cause ineffective control of the pathogens or tumors that express immunogens. Examples of T cell dysfunction condition characterized by T cell dysfunction include acute infections of unknown origin, chronic infections and tumor immunity.

Therefore, the compound disclosed herein can be used to treat a disease wherein enhanced immunogenicity is desired, for example, improving tumor immunogenicity for the treatment of a cancer.

The term "dysfunction" in the context of immune dysfunction is a state of reduced immune response to an antigenic stimulation. The term includes a common component in which an exhaustion and/or anergy of antigen recognition may occur, but the subsequent immune response is ineffective in controlling infection or tumor growth.

As used herein, the term "dysfunctional" also includes being resistant or unresponsive to an antigen recognition, specifically, having a reduced ability to translate the antigen recognition into downstream T cell effector functions (such as proliferation, cytokine production (e.g., IL-2, γ-IFN) and/or target cell killing).

The term "anergy" refers to a state of being unresponsive to an antigenic stimulation caused by incomplete or insufficient signal delivery via the T cell receptors (e.g., elevated intracellular Ca⁺² in the absence of ras activation). T cell anergy can also be induced by an antigenic stimulation in the absence of co-stimulation, causing the cell to become resistant to the subsequent antigenic activation even in the presence of co-stimulation. The unresponsive state can usually be resolved by the presence of interleukin-2. Anergic T cells do not undergo clonal expansion and/or acquire effector functions.

The term "exhaustion" refers to T cell exhaustion, a state of T cell dysfunction caused by persistent TCR signaling, such as that occurs during a variety of chronic infections and cancers. It differs from anergy in that it is not caused by incomplete or insufficient signaling, but by persistent signaling. It is defined by adverse effector function, persistent expression of inhibitory receptors, and a transcriptional state that is different from functional effectors or memory T cells. Exhaustion prevents the optimal control of an infection and a tumor. Exhaustion can be caused by an exogenous negative regulatory pathway (e.g., immunoregulatory cytokines) as well as an endogenous negative regulatory (co-stimulatory) pathway (PD-1, B7-H3, B7-H4, etc.).

"Immunogenicity" refers to the ability of a particular substance to elicit an immune response. Tumors are immunogenic and tumor immunogenic adjuvants are enhanced in the elimination of tumor cells via an immune response.

In some embodiments, the subject matter disclosed herein relates to a method of treating an HPK1-dependent condition, comprising administering an effective amount of a compound or a pharmaceutical composition of the present disclosure to a subject in need. In some aspects of this embodiment, the HPK1-dependent condition is a cancer. In some aspects of this embodiment, the cancer comprises at least one cancer selected from: colorectal cancer, melanoma, non-small cell lung cancer, ovarian cancer, breast cancer, pancreatic cancer, hematologic malignancies, and renal cell carcinoma. In some aspects of this embodiment, the cancer has an increased amount of T cell infiltration. In some aspects of this embodiment, cancer cells of the subject selectively have increased expression of MHC class I antigens compared with those before the administration of the compound or composition.

In some embodiments, the subject matter disclosed herein relates to a method of treating a chronic viral infection. In some embodiments, the subject matter disclosed herein relates to use of an HPK1 inhibitor as an adjuvant therapy to increase the efficacy of a vaccination.

In some aspects, the present disclosure provides a method of treating a cell proliferative condition, including cancer, benign papilloma, gestational trophoblastic disease and benign neoplastic disease, such as skin papilloma (warts) and genital papilloma.

Examples of cancer that may be treated using the compound of the present disclosure include, but are not limited to, bone cancer, pancreatic cancer, skin cancer, head and neck cancer, skin or intraocular malignant melanoma, uterine cancer, ovarian cancer, rectal cancer, anal cancer, gastric cancer, testicular cancer, uterine cancer, fallopian tube cancer, endometrial cancer, cervical cancer, vaginal cancer, vulvar cancer, Hodgkin's disease, non-Hodgkin's lymphoma, esophageal cancer, small intestine cancer, endocrine cancer, thyroid cancer, parathyroid cancer, adrenal cancer, soft tissue sarcoma, urethral cancer, penile cancer, chronic or acute leukemia (including acute myeloid leukemia, chronic myeloid leukemia, acute lymphoblastic leukemia, and chronic lymphocytic leukemia), pediatric solid tumor, lymphocytic lymphoma, bladder cancer, kidney cancer or urethral cancer, renal pelvis cancer, central nervous system (CNS) tumor, primary CNS lymphoma, tumor angiogenesis, spinal tumor, brainstem glioma, pituitary adenoma, Kaposi's sarcoma, epidermoid carcinoma, squamous cell carcinoma, T-cell lymphoma, environmentally induced cancers (including asbestos-induced cancers), and combinations of such cancers.

In some embodiments, cancers that can be treated using the compound of the present disclosure include, but are not limited to, solid tumors (e.g., prostate cancer, colon cancer, esophageal cancer, endometrial cancer, ovarian cancer, uterine cancer, kidney cancer, liver cancer, pancreatic cancer, gastric cancer, breast cancer, lung cancer, head and neck cancer, thyroid cancer, glioblastoma, sarcoma, bladder cancer, etc.), blood cancers (e.g., lymphoma, leukemia such as acute lymphoblastic leukemia (ALL), acute myeloid leukemia (AML), chronic lymphocytic leukemia (CLL), chronic myeloid leukemia (CML), DLBCL, mantle cell lymphoma, non-Hodgkin's lymphoma (including relapsed or refractory NHL and relapsed follicular lymphoma), Hodgkin's lymphoma or multiple myeloma), and combinations of such cancers.

In certain embodiments, the cancer is brain cancer, leukemia, skin cancer, prostate cancer, thyroid cancer, colon cancer, lung cancer, or sarcoma. In another embodiment, the cancer is selected from: glioma, glioblastoma multiforme, paraganglioma, supratentorial primitive neuroectodermal tumor, acute myeloid leukemia, myelodysplastic syndrome, chronic myeloid leukemia, melanoma, breast cancer, prostate cancer, thyroid cancer, colon cancer, lung cancer, central chondrosarcoma, central and periosteal chondroma, fibrosarcoma, and cholangiocarcinoma.

In certain embodiments, the cancer is selected from brain cancer and spinal cord cancer, head and neck cancer, leukemia and blood cancer, skin cancer, reproductive system cancer, gastrointestinal cancer, liver cancer and cholangiocarcinoma, kidney cancer and bladder cancer, bone cancer, lung cancer, malignant mesothelioma, sarcoma, lymphoma, adenocarcinoma, thyroid cancer, cardiac tumor, germ cell tumor, malignant neuroendocrine (carcinoid) tumor, midline carcinoma, and carcinoma of unknown primary (a cancer in which metastases are found but the original cancer site is unknown). In a specific embodiment, the cancer is present in an adult patient; in other embodiments, the cancer is present in a pediatric patient. In a specific embodiment, the cancer is AIDS-related.

In another embodiment, the cancer is selected from brain cancer and spinal cord cancer. In a specific embodiment, the cancer is selected from: pleomorphic astrocytoma, glioblastoma, astrocytoma and sensitive neuroblastoma (olfactive neuroblastoma). In a specific embodiment, the brain cancer is selected from: astrocytoma (e.g., pilocytic astrocytoma, subependymal giant cell astrocytoma, diffuse astrocytoma, pleomorphic xanthoastrocytoma, anaplastic astrocytoma, astrocytoma, giant cell glioblastoma, glioblastoma, secondary glioblastoma, primary adult glioblastoma and primary pediatric glioblastoma), oligodendroglial tumor (e.g., oligodendroglioma and degenerative oligodendroglioma), oligoastrocytic tumor (e.g., oligoastrocytoma and degenerative oligoastrocytoma), ependymoma (e.g., myxopapillary ependymoma and degenerative ependymoma); medulloblastoma, primitive neuroectodermal tumor, schwannoma, meningioma, atypical meningioma, anaplastic meningioma, pituitary adenoma, brainstem glioma, cerebellar astrocytoma, cerebral astrocytoma/malignant glioma, optic pathway and hypothalamic glioma, and primary central nervous system lymphoma. In a specific example of these embodiments, brain cancer is selected from: glioma, glioblastoma multiforme, paraganglioma and supratentorial primitive neuroectodermal tumor (sPNET).

In a certain embodiment, the cancer is selected from head and neck cancers, including nasopharyngeal carcinoma, nasal cavity and paranasal sinus cancer, hypopharyngeal cancer, oral cancer (e.g., squamous cell carcinoma, lymphoma and sarcoma), lip cancer, oropharyngeal cancer, salivary gland tumor, laryngeal cancer (e.g., laryngeal squamous cell carcinoma, rhabdomyosarcoma) and eye cancer or ocular cancer. In a certain embodiment, the ocular cancer is selected from intraocular melanoma and retinoblastoma.

In a specific embodiment, the cancer is selected from leukemia and blood cancer. In a specific embodiment, the cancer is selected from: myeloproliferative neoplasm, myelodysplastic syndrome, myelodysplasia/myeloproliferative neoplasm, acute myeloid leukemia (AML), myelodysplastic syndrome (MDS), chronic myeloid leukemia (CML), myeloproliferative neoplasm (MPN), post-MPN AML, post-MDS AML, del(5q)-associated high-risk MDS or AML, blastocyst stage chronic myeloid leukemia, angioimmunoblastic lymphoma, acute lymphoblastic leukemia, Langerhans cell histiocytosis, hairy cell leukemia, and plasma cell neoplasms including plasmacytoma and multiple myeloma. The leukemia mentioned herein can be acute or chronic.

In a certain embodiment, the cancer is selected from skin cancers. In a certain embodiment, the skin cancer is selected from melanoma, squamous cell carcinoma, and basal cell carcinoma.

In a certain embodiment, the cancer is selected from reproductive system cancers. In a certain embodiment, the cancer is selected from: breast cancer, cervical cancer, vaginal cancer, ovarian cancer, prostate cancer, penile cancer, and testicular cancer. In a specific example of these embodiments, the cancer is a breast cancer selected from the group consisting of ductal carcinoma and phyllodes tumor. In a specific example of these embodiments, the breast cancer may be male breast cancer or female breast cancer. In a specific example of these embodiments, the cancer is a cervical cancer selected from squamous cell carcinoma and adenocarcinoma. In a specific example of these embodiments, the cancer is an ovarian cancer selected from epithelial cancers.

In a specific embodiment, the cancer is selected from gastrointestinal cancers. In a specific embodiment, the cancer is selected from: esophageal cancer, gastric cancer, gastrointestinal carcinoid, pancreatic cancer, gallbladder cancer, colorectal cancer, and anal cancer. In an example of these embodiments, the cancer is selected from: esophageal squamous cell carcinoma, esophageal adenocarcinoma, gastric adenocarcinoma, gastrointestinal carcinoid tumor, gastrointestinal stromal tumor, gastric lymphoma, gastrointestinal lymphoma, solid pseudopapillary tumor of the pancreas, pancreatoblastoma, islet cell tumor, pancreatic cancer (including acinar cell carcinoma and ductal adenocarcinoma), gallbladder adenocarcinoma, colorectal adenocarcinoma, and anal squamous cell carcinoma.

In a specific embodiment, the cancer is selected from liver cancer and cholangiocarcinoma. In a specific embodiment, the cancer is liver cancer (hepatocellular carcinoma). In a specific embodiment, the cancer is cholangiocarcinoma; in an example of these embodiments, the cholangiocarcinoma is selected from intrahepatic cholangiocarcinoma and extrahepatic cholangiocarcinoma.

In a specific embodiment, the cancer is selected from kidney cancer and bladder cancer. In a specific embodiment, the cancer is a kidney cancer selected from: renal cell carcinoma, Wilms tumor, and transitional cell carcinoma. In a specific embodiment, the cancer is a bladder cancer selected from: urothelial carcinoma (transitional cell carcinoma), squamous cell carcinoma, and adenocarcinoma.

In a specific embodiment, the cancer is selected from bone cancers. In a specific embodiment, the bone cancer is selected from: osteosarcoma, malignant fibrous histiocytoma of bone, Ewing sarcoma and chordoma.

In a specific embodiment, the cancer is selected from lung cancers. In a specific embodiment, the lung cancer is selected from: non-small cell lung cancer, small cell lung cancer, bronchial tumor, and pleuropulmonary blastoma.

In a specific embodiment, the cancer is selected from malignant mesothelioma. In a specific example, the cancer is selected from the group consisting of epithelial mesothelioma and sarcomatoid carcinoma.

In a specific embodiment, the cancer is selected from sarcomas. In a specific embodiment, the sarcoma is selected from: central chondrosarcoma, central and periosteal chondroma, fibrosarcoma, tenosynovial clear cell sarcoma, and Kaposi's sarcoma.

In a specific embodiment, the cancer is selected from lymphoma. In a specific embodiment, the cancer is selected from: Hodgkin's lymphoma (e.g., Reed-Sternberg cell), non-Hodgkin's lymphoma (e.g., diffuse large B-cell lymphoma, follicular lymphoma, mycosis fungoides, Sézary syndrome, primary central nervous system lymphoma), cutaneous T-cell lymphoma and primary central nervous system lymphoma.

In a specific embodiment, the cancer is selected from adenocarcinomas. In a specific embodiment, the cancer is selected from: adrenocortical carcinoma, pheochromocytoma, paraganglioma, pituitary tumor, thymoma and thymic carcinoma.

In a specific embodiment, the cancer is selected from thyroid cancers. In a particular example, the thyroid cancer is selected from: medullary thyroid cancer, papillary thyroid cancer, and follicular thyroid cancer.

In a specific embodiment, the cancer is selected from germ cell tumors. In a specific embodiment, the cancer is selected from: malignant extracranial germ cell tumor and malignant extragonadal germ cell tumor. In a specific example of these embodiments, the malignant extragonadal germ cell tumor is selected from non-seminoma and seminoma.

In a specific embodiment, the cancer is selected from cardiac tumors. In a particular example, the cardiac tumor is selected from: malignant teratoma, lymphoma, rhabdomyosarcoma, angiosarcoma, chondrosarcoma, infantile fibrosarcoma, and synovial sarcoma.

In a specific embodiment, the cell proliferative condition is selected from benign papilloma, benign neoplastic disease and gestational trophoblastic disease. In a specific embodiment, the benign neoplastic disease is selected from skin papilloma (warts) and genital papilloma. In a specific embodiment, the gestational trophoblastic disease is selected from the group consisting of: hydatidiform mole and gestational trophoblastic neoplasia (e.g., invasive mole, choriocarcinoma, placental site trophoblastic tumor and epithelioid trophoblastic tumor).

In some embodiments, the subject has melanoma. The melanoma may be in an early or late stage. In some embodiments, the subject has colorectal cancer. The colorectal cancer may be in an early or late stage. In some embodiments, the subject has non-small cell lung cancer. The non-small cell lung cancer may be in an early or late stage. In some embodiments, the subject has pancreatic cancer. The pancreatic cancer may be in an early or late stage. In some embodiments, the subject has hematologic malignancies. The hematologic malignancies may be in an early or late stage. In some embodiments, the subject has ovarian cancer. The ovarian cancer may be in an early or late stage. In some embodiments, the subject has breast cancer. The breast cancer may be in an early or late stage. In some embodiments, the subject has renal cell carcinoma. The renal cell carcinoma may be in an early or late stage. In some embodiments, the cancer has an increased amount of T cell infiltration.

In some embodiments, cancers that can be treated with the compound of the present disclosure include melanoma (e.g., metastatic melanoma), kidney cancer (e.g., clear cell carcinoma), prostate cancer (e.g., hormone-refractory prostate cancer), breast cancer, triple-negative breast cancer, colon cancer, and lung cancer (e.g., non-small cell lung cancer and small cell lung cancer). In addition, the present disclosure includes the refractory or relapsed malignancies whose growth can be inhibited using the compound of the present disclosure.

In some embodiments, diseases and indications that can be treated using the compound of the present disclosure include, but are not limited to, blood cancer, sarcoma, lung cancer, gastrointestinal cancer, urogenital cancer, liver cancer, bone cancer, nervous system cancer, gynecologic cancer, and skin cancer.

Exemplary blood cancers include lymphoma and leukemia, such as acute lymphoblastic leukemia (ALL), acute myeloid leukemia (AML), acute promyelocytic leukemia (APL), chronic lymphocytic leukemia (CLL), chronic myeloid leukemia (CML), diffuse large B-cell lymphoma (DLBCL), mantle cell lymphoma, non-Hodgkin's lymphoma (including relapsed or refractory NHL and relapsed follicular lymphoma), Hodgkin's lymphoma, myeloproliferative disorder (e.g., primary myelofibrosis (PMF), polycythemia vera (PV), essential thrombocythemia (ET)), myelodysplastic syndrome (MDS), T-cell acute lymphoblastic lymphoma (T-ALL), multiple myeloma, cutaneous T-cell lymphoma, Waldenstrom macroglobulinemia, hairy cell lymphoma, chronic myeloid lymphoma, and Burkitt lymphoma.

Exemplary sarcomas include chondrosarcoma, Ewing sarcoma, osteosarcoma, rhabdomyosarcoma, angiosarcoma, fibrosarcoma, liposarcoma, myxoma, rhabdomyoma, rhabdomyosarcoma, fibroma, lipoma, hamartoma, and teratoma.

Exemplary lung cancers include non-small cell lung cancer (NSCLC), small cell lung cancer, bronchogenic carcinoma (squamous cell carcinoma, small cell undifferentiated carcinoma, large cell undifferentiated carcinoma, adenocarcinoma), alveolar (bronchiolar) carcinoma, bronchial adenoma, chondroma, hamartoma, and mesothelioma.

Exemplary gastrointestinal cancers include the following cancers of: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid, VIPoma), small intestine (adenocarcinoma, lymphoma, carcinoid, Kaposi's sarcoma, leiomyoma, hemangioma, lipoma, neurofibroma, fibroma), large intestine (adenocarcinoma, tubular adenoma, villous adenoma, hamartoma, leiomyoma), and colorectum.

Exemplary urogenital cancers include the following cancers of: kidney (adenocarcinoma, Wilms tumor (nephroblastoma)), bladder and urethra (squamous cell carcinoma, transitional cell carcinoma, adenocarcinoma), prostate (adenocarcinoma, sarcoma), and testicle (seminoma, teratoma, embryonal carcinoma, teratocarcinoma, choriocarcinoma, sarcoma, stromal cell carcinoma, fibroma, fibroadenoma, adenomatoid tumor, lipoma).

Exemplary liver cancers include hepatoma (e.g., hepatocellular carcinoma), cholangiocarcinoma, hepatoblastoma, angiosarcoma, hepatocellular adenoma, and hemangioma.

Exemplary bone cancers include, e.g., osteogenic sarcoma (e.g., osteosarcoma), fibrosarcoma, malignant fibrous histiocytoma, chondrosarcoma, Ewing sarcoma, malignant lymphoma (reticulum cell sarcoma), multiple myeloma, malignant giant cell tumor, chordoma, osteochondroma (osteochondral exostosis), benign enchondroma, chondroblastoma, chondromyxoid fibroma, osteoid osteoma, and giant cell tumor.

Exemplary nervous system cancers include the following cancers of: skull (osteoma, hemangioma, granuloma, xanthoma, osteitis deformans), meninges (meningioma, meningeal sarcoma, gliomatosis), brain (astrocytoma, medulloblastoma, glioma, ependymoma, blastoma (pineal tumor), glioblastoma, glioblastoma multiforme, oligodendroglioma, schwannoma, retinoblastoma, congenital tumor), and spinal cord (neurofibroma, meningioma, glioma, sarcoma), as well as neuroblastoma and Lhermitte-Duclos disease.

Exemplary gynecologic cancers include the following cancers of: uterus (endometrial cancer), cervix (cervical cancer, preneoplastic cervical dysplasia), ovary (ovarian cancer (serous cystadenocarcinoma, mucinous cystadenocarcinoma, unspecified cancer), follicular cell tumor, Sertoli-Leydig cell tumor, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, sarcoma botryoides (embryonal rhabdomyosarcoma), and fallopian tube (carcinoma).

Exemplary skin cancers include melanoma, basal cell carcinoma, squamous cell carcinoma, Kaposi's sarcoma, Merkel cell carcinoma of the skin, dysplasia, lipoma, hemangioma, dermatofibroma and keloid. In some embodiments, diseases and indications that can be treated using the compound of the present disclosure include, but are not limited to, sickle cell disease (e.g., sickle cell anemia), triple negative breast cancer (TNBC), myelodysplastic syndrome, testicular cancer, cholangiocarcinoma, esophageal cancer and urothelial cancer.

Exemplary head and neck cancers include glioblastoma, melanoma, rhabdomyosarcoma, lymphosarcoma, osteosarcoma, squamous cell carcinoma, adenocarcinoma, oral cancer, laryngeal cancer, nasopharyngeal cancer, nasal and paranasal sinus cancer, thyroid cancer and parathyroid cancer.

In some embodiments, an HPK1 inhibitor can be used to treat a PGE2-producing tumor (e.g., a tumor that overexpresses Cox-2) and/or an adenosine-producing tumor (a tumor that overexpresses CD73 and CD39). Overexpression of Cox-2 has been detected in a variety of tumors, such as colorectal cancer, breast cancer, pancreatic cancer, and lung cancer, in which the overexpression of Cox-2 is associated with poor prognosis. Overexpression of COX-2 has been reported in blood cancer models such as RAJI (Burkitt lymphoma) and U937 (acute monocytic leukemia) and in a patient's germ cells. CD73 is upregulated in various human carcinomas, including colon cancer, lung cancer, pancreatic cancer, and ovarian cancer. Importantly, higher expression of CD73 is associated with tumor angiogenesis, invasiveness, and cancer metastasis, and is associated with a shorter survival time of a patient in breast cancer.

### Administration

The pharmaceutical composition provided by the present disclosure can be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compostions of the present disclosure may be further delivered using a variety of dosing methods. For example, in certain embodiments, the pharmaceutical composition may be given as a bolus, *e.g.*, in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (*e.g.*, through an IV drip) allows a much faster delivery which quickly raises the concentration of the active ingredient in the blood to an effective level. In other embodiments, the pharmaceutical composition may be administered as a continuous infusion, *e.g.*, by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions.

An HPK1 antagonist is administered to a subject at a dosage of about 0.001 µg/kg to about 1000 mg/kg, including but not limited to about 0.001 µg/kg, 0.01 µg/kg, 0.05 µg/kg, 0.1 µg/kg, 0.5 µg/kg, 1 µg/kg, 10 µg/kg, 25 µg/kg, 50 µg/kg, 100 µg/kg, 250 µg/kg, 500 µg/kg, 1 mg/kg, 5 mg/kg, 10 mg/kg, 25 mg/kg, 50 mg/kg, 100 mg/kg and 200 mg/kg.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art.

Transdermal compositions are typically formulated as a topical ointment or cream containing the active ingredient(s). When formulated as a ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds of the present disclosure can also be administered in sustained release forms or from sustained release drug delivery systems. A description of representative sustained release materials can be found in Remington's Pharmaceutical Sciences.

The present disclosure also relates to the pharmaceutically acceptable formulations of a compound of the present disclosure. In one embodiment, the formulation comprises water. In another embodiment, the formulation comprises a cyclodextrin derivative. The most common cyclodextrins are α-, β- and γ-cyclodextrins consisting of 6, 7 and 8 α-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, e.g., sulfobutyl ether β-cyclodextrin, also known as Captisol. In certain embodiments, the formulation comprises hexapropyl-β-cyclodextrin.

### Combination therapy

Depending on the specific disorder or disease to be treated, other therapeutic agents for treating the disease are usually used in combination with a compound and composition of the present disclosure. As used herein, additional therapeutic agents administered to treat a particular disease or disorder are typically referred to as "appropriate for the disease or disorder being treated".

Another therapeutic agent that can be administered in combination with the compound of the present disclosure includes, but is not limited to, anti-proliferative compounds including, but not limited to, aromatase inhibitors; antiestrogens; topoisomerase I inhibitors; topoisomerase II inhibitors; microtubule-active compounds; alkylating compounds, etc.; antiestrogens such as tamoxifen, etc.; antiandrogen drugs such as bicalutamide, etc.; "topoisomerase I inhibitors" such as topotecan, etc.; topoisomerase II inhibitors such as epirubicin, etc.; "microtubule-active agents" such as docetaxel, etc.; alkylating agents such as cyclophosphamide, etc.; HDAC inhibitors such as suberoylanilide hydroxamic acid (SAHA), etc.; "antineoplastic antimetabolites" such as capecitabine, etc.; "platinum compounds" such as cisplatinum, etc.; "compounds that target/reduce protein or lipid kinase activity or protein or lipid phosphatase activity or other anti-angiogenic compounds" such as protein tyrosine kinase and/or serine and/or threonine kinase inhibitors or lipid kinase inhibitors; PI3K inhibitors; BTK inhibitors; PD-1 inhibitors; SYK inhibitors; Bd-2 inhibitors; JAK inhibitors; anti-angiogenic compounds such as thalidomide, etc.; proteasome inhibitors such as bortezomib; etc.

### Chemical synthesis

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein. All solvents used in the present disclosure are commercially available and can be used without further purification. The initial compound raw materials used for synthesis in the present disclosure are commercially available, and can also be prepared by the methods of the prior art.

### Example 1

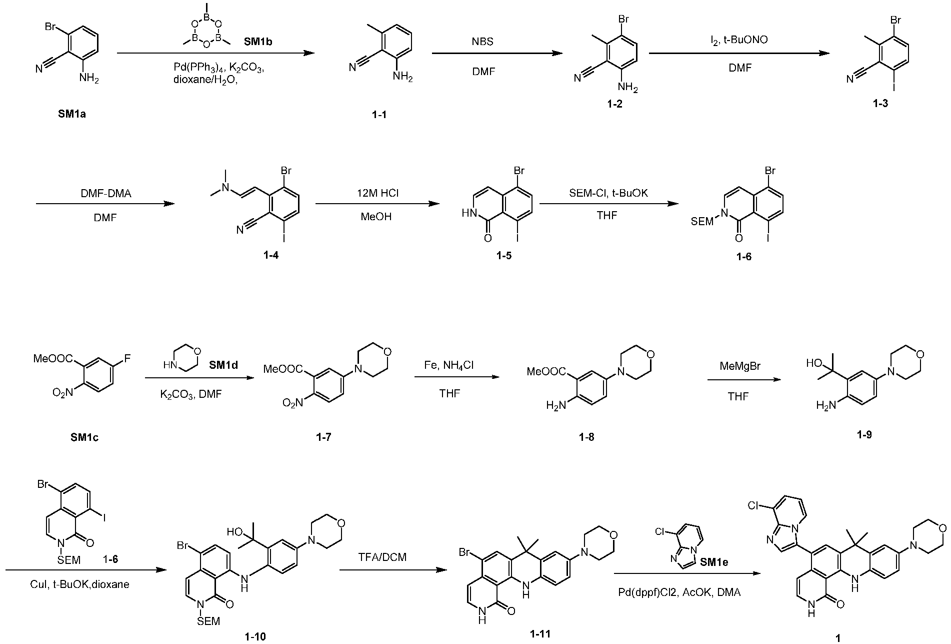

### Synthesis of intermediate 1-1

Compound SM1a (76 g) and compound SM1b (387 g) were dissolved in 1,4-dioxane (450 mL) and water (90 mL), and potassium carbonate (160 g) was added. The mixture was purged with nitrogen three times, and then tetrakis(triphenylphosphine)palladium(0) (2.23 g) was added into the mixture under nitrogen. The above reaction solution was reacted at 100 °C for 12 hours under nitrogen. The reaction solution was cooled to room temperature and poured into water (1000 mL). The mixture was extracted with ethyl acetate (500 mL x 3). The organic phases were combined, washed with saturated brine, dried and concentrated to give a crude product. The crude product was purified by column chromatography (silica, petroleum ether/ethyl acetate = 1/0-20/1) to give white solid compound 1-1 (45 g, yield: 87%). LCMS (ESI) m/z: 133. ¹H NMR (400 MHz, CDCl₃) δ ppm: 7.31 - 7.24 (m, 1H), 6.70 - 6.61 (m, 2H), 4.74 - 3.89 (m, 2H), 2.52 (s, 3H).

### Synthesis of intermediate 1-2

Compound **1-1** (45 g) was dissolved in N,N-dimethylformamide (300 mL), and N-bromosuccinimide (60.6 g) was added. The above reaction solution was reacted at 25 °C for 12 hours. The reaction solution was slowly poured into water (600 mL), and a solid was precipitated. After filtration, the filter cake was rotary evaporated to dryness to give yellow solid compound **1-2** (63 g) as a crude product, which was directly used in the next reaction. LCMS (ESI) m/z: 210/212. ¹H NMR (400 MHz, CDCl₃) δ ppm: 7.42 (d, *J =* 8.8 Hz, 1H), 6.49 (d, *J =* 8.8 Hz, 1H), 4.43 (s, 2H), 2.53 (s, 3H).

### Synthesis of intermediate 1-3

A solution of iodine (101 g) in N,N-dimethylformamide (120 mL) was slowly added dropwise to a solution of compound **1-2** (60 g) in N,N-dimethylformamide (240 mL) at 10 °C. After the addition was completed, the above reaction system was cooled to 0 °C, and tert-butyl nitrite (46.9 g) was slowly added dropwise to the above reaction solution. The above mixture was reacted at 20 °C for another 12 hours. The reaction solution was poured into ice water (500 mL), and the mixture was extracted with ethyl acetate (100 mL x 3). The organic phases were combined, washed with saturated brine, dried and concentrated to give a crude product. The crude product was purified by column chromatography (silica, petroleum ether/ethyl acetate = 1/10-1/1) to give brown solid compound **1-3** (40 g). LCMS (ESI) m/z: 322/324.

### Synthesis of intermediate 1-4

Compound **1-3** (40 g) was dissolved in N,N-dimethylformamide (200 mL), and N,N-dimethylformamide dimethyl acetal (49.9 g) was added to the reaction solution at room temperature. After the addition was completed, the reaction solution was heated to 135 °C and stirred for another 18 hours. The reaction solution was cooled to room temperature and rotary evaporated to dryness to give a crude product. The crude product was stirred with methyl tert-butyl ether (40 mL) at 25 °C for half an hour, and a solid was precipitated. The precipitated solid was filtered and dried to give brown solid compound **1-4** (24 g) as a crude product, which was directly used in the next reaction. LCMS (ESI) m/z: 377/379.

### Synthesis of intermediate 1-5

Compound **1-4** (24 g) was dissolved in methanol (240 mL), and concentrated hydrochloric acid (12M, 1.4 L) was added to the reaction solution. The above reaction solution was reacted at room temperature for 12 hours. The reaction solution was filtered, and the filter cake was collected and dried to give brown solid compound **1-5** (7 g) as a crude product, which was directly used in the next reaction. LCMS (ESI) m/z: 350/352.

### Synthesis of intermediate 1-6

Compound **1-5** (6.2 g) and potassium tert-butoxide (2.2 g) were dissolved in tetrahydrofuran (36 mL), and 2-(trimethylsilyl)ethoxymethyl chloride (2.95 g) was added to the reaction solution at 0 °C. The above reaction solution was stirred at 20 °C for another 12 hours. The reaction solution was poured into ice water (50 mL), and the mixture was extracted with ethyl acetate (50 mL x 3). The organic phases were combined, washed with saturated brine, dried and concentrated. The resulting crude product was purified by column chromatography (silica, petroleum ether/ethyl acetate = 1/10-1/1) to give brown solid compound **1-6** (6.0 g). LCMS (ESI) m/z: 480/482.

¹H NMR (400 MHz, CDCl₃) δ ppm: 8.00 (d, *J* = 8.4 Hz, 1H), 7.47 (d, *J* = 8.4 Hz, 1H), 7.36 (d, *J* = 7.6 Hz, 1H), 6.91 (d, *J* = 7.6 Hz, 1H), 5.41 (s, 2H), 3.68 - 3.61 (m, 2H), 1.00 - 0.93 (m, 2H), 0.02 - 0.01 (m, 9H).

### Synthesis of intermediate 1-7

Compound **SM1c** (2 g) was dissolved in N,N-dimethylformamide (40 mL), and compound **SM1d** (1.05 g) and potassium carbonate (2.78 g) were added to the reaction solution. The above mixture was stirred at 80 °C for 20 hours. The reaction solution was slowly poured into water (50 mL), and the mixture was extracted twice with ethyl acetate (50 mL). The organic phases were dried over anhydrous sodium sulfate, filtered and concentrated under vacuum to give yellow solid compound **1-7** (2.5 g) as a crude product, which was directly used in the next step. LCMS (ESI) m/z: 267.

### Synthesis of intermediate 1-8

Compound **1-7** (2.5 g) and ammonium chloride (2.51 g) were dissolved in tetrahydrofuran (30 mL) and water (6 mL). The above mixture was heated to 80 °C, and iron powder (2.62 g) was slowly added. The reaction solution was stirred at 80 °C for 20 hours. The reaction solution was filtered, and the filter cake was washed with tetrahydrofuran (30 mL). The filtrate was collected and rotary evaporated to dryness to give yellow solid compound **1-8** (1.8 g) as a crude product, which was directly used in the next step. LCMS (ESI) m/z: 237.

### Synthesis of intermediate 1-9

Compound **1-8** (1.8 g) was dissolved in tetrahydrofuran (30 mL) and cooled to 0 °C. Methylmagnesium bromide (15.2 mL) was slowly added dropwise to the above solution. After the addition was completed, the reaction solution was warmed to 20 °C and stirred for 12 hours. The reaction solution was slowly poured into ice water (30 mL), and the mixture was extracted twice with ethyl acetate (30 mL). The organic phases were collected, dried over anhydrous sodium sulfate, filtered and concentrated. The resulting crude product was purified by column chromatography (petroleum ether/ethyl acetate = 20/1 to 1/1) to give yellow solid compound **1-9** (0.58 g). LCMS (ESI) m/z: 237.

### Synthesis of intermediate 1-10

Compound **1-6** (500 mg), compound **1-9** (319 mg), potassium tert-butoxide (350 mg) and copper(I) iodide (1.98 mg) were dissolved in 1,4-dioxane (10 mL). The above mixture was stirred at 110 °C for 20 hours. The reaction solution was cooled to room temperature and filtered. The filtrate was rotary evaporated to dryness, and the resulting crude product was purified by column chromatography (petroleum ether/ethyl acetate = 30/1 to 2/1) to give yellow solid compound **1-10** (220 mg, yield: 41.5%). LCMS (ESI) m/z: 588/590.

### Synthesis of intermediate 1-11

Compound **1-10** (180 mg) was dissolved in dichloromethane (2 mL), and trifluoroacetic acid (2 mL) was added. The above mixture was stirred at 20 °C for 20 hours. The reaction solution was rotary evaporated to dryness, and the resulting crude product was purified by thin layer chromatography (dichloromethane/methanol = 20/1) to give yellow solid compound **1-11** (80 mg, yield: 59%). LCMS (ESI) m/z: 440/442.

### Synthesis of compound 1

Compound **1-11** (70 mg), compound **SM1e** (33.9 mg) and potassium acetate (21.8 mg) were dissolved in N,N-dimethylacetamide (1 mL), and [1,1'-bis(diphenylphosphino)ferrocene]palladium(II) dichloride (9.09 mg) was added under nitrogen. The above mixture was reacted at 125 °C for 5 hours. The reaction solution was cooled to room temperature and filtered. The filtrate was rotary evaporated to dryness, and the resulting crude product was purified by high performance liquid chromatography (chromatography column: Waters Xbridge BEH C18 100 * 30 mm * 10 um; mobile phase: [water (ammonium bicarbonate) - acetonitrile]; B%: 15% - 45%, 8 min) to give yellow solid compound **1** (3.35 mg, yield: 2.94%). LCMS (ESI) m/z: 512. ¹H NMR (400 MHz, CD₃OD) δ ppm: 7.77 (d, *J =* 6.8 Hz, 1H), 7.72 (d, *J =* 6.4 Hz, 2H), 7.47 (d, *J =* 7.2 Hz, 1H), 7.09 (br s, 1H), 7.01 (d, *J =* 7.2 Hz, 1H), 6.79 - 6.92 (m, 3H), 5.90 (d, *J =* 7.2 Hz, 1H), 3.81 - 3.88 (m, 4H), 3.08 (br s, 4H), 1.62 (br s, 6H).

The following examples were synthesized using the same methods as above:

| Exa mpl e | Structure | MS:M +H⁺ | ¹H NMR |
|---|---|---|---|
| 2 | 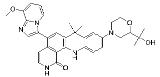 | 566 | ¹H NMR (400 MHz, DMSO-*d₆*) δ ppm: 11.83 (s, 1H), 11.40 (br d, *J* = 5.2 Hz, 1H), 7.68 (s, 1H), 7.57 (s, 1H), 7.40 (d, *J* = 6.4 Hz, 1H), 7.12 - 6.99 (m, 2H), 6.84 - 6.76 (m, 3H), 6.73 - 6.67 (m, 1H), 5.78 (d, *J* = 7.2 Hz, 1H), 4.47 (s, 1H), 4.03 - 3.93 (m, 4H), 3.70 - 3.61 (m, 1H), 3.58 - 3.51 (m, 1H), 3.41 (br d, *J* = 11.2 Hz, 2H), 2.68 - 2.56 (m, 2H), 1.59 (br s, 6H), 1.21 - 1.07 (m, 6H) |
| 3 | 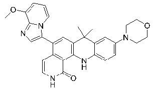 | 508 | ¹H NMR (400 MHz, DMSO-*d₆*) δ ppm: 11.83 (s, 1H), 11.39 (br d, *J* = 5.2 Hz, 1H), 7.67 (s, 1H), 7.58 (s, 1H), 7.41 (d, *J* = 6.4 Hz, 1H), 7.09 - 7.01 (m, 2H), 6.83 - 6.75 (m, 3H), 6.71 (br d, *J* = 7.2 Hz, 1H), 5.78 (d, *J* = 7.2 Hz, 1H), 3.98 (s, 3H), 3.76 - 3.69 (m, 4H), 3.09 - 3.02 (m, 4H), 1.58 (br s, 6H) |
| 4 | 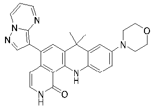 | 479 | ¹H NMR (400 MHz, CD₃OD) δ ppm: 11.59 (s, 1H), 8.99 (d, *J* = 7.0 Hz, 1H), 8.54 - 8.48 (m, 1H), 8.29 (s, 1H), 7.80 (s, 1H), 7.12 - 7.03 (m, 2H), 7.00 (d, *J* = 7.2 Hz, 1H), 6.91 - 6.78 (m, 2H), 6.50 (d, *J =* 7.2 Hz, 1H), 3.87 - 3.83 (m, 4H), 3.08 (br s, 4H), 1.63 (s, 6H) |
| 5 | 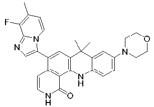 | 510 | ¹H NMR (400 MHz, DMSO-*d₆*) δ ppm: 11.84 (s, 1H), 11.41 (br d, *J* = 4.8 Hz, 1H), 7.69 (s, 1H), 7.63 (s, 1H), 7.59 (d, *J* = 7.2 Hz, 1H), 7.08 - 7.02 (m, 2H), 6.83 - 6.74 (m, 3H), 5.79 (d, *J* = 7.2 Hz, 1H), 3.76 - 3.72 (m, 4H), 3.06 - 3.02 (m, 4H), 2.31 (d, *J* = 2.0 Hz, 3H), 1.58 (s, 6H) |
| 6 | 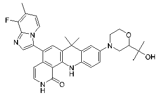 | 568 | ¹H NMR (400 MHz, CD₃OD) δ ppm: 7.72 (s, 1H), 7.60 (s, 1H), 7.54 (d, *J* = 7.2 Hz, 1H), 7.10 (br s, 1H), 7.02 (d, *J* = 7.2 Hz, 1H), 6.94 - 6.82 (m, 2H), 6.77 (t, *J* = 6.4 Hz, 1H), 5.93 (d, *J* = 7.2 Hz, 1H), 4.05 (br d, *J* = 11.2 Hz, 1H), 3.85 - 3.72 (m, 1H), 3.53 (br d, *J* = 10.4 Hz, 1H), 3.44 (br d, *J* = 10.4 Hz, 2H), 2.75 (br dd, *J* = 3.6, 12.8 Hz, 1H), 2.64 - 2.46 (m, 1H), 2.38 (d, *J =* 2.0 Hz, 3H), 1.63 (s, 6H), 1.25 (d, *J* = 12.4 Hz, 6H) |
| 7 | 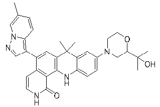 | 550 | ¹H NMR (400 MHz, CD₃OD) δ ppm: 8.39 (s, 1H), 7.96 (s, 1H), 7.67 (br s, 1H), 7.29 (d, *J* = 9.2 Hz, 1H), 7.12 (br d, *J* = 9.2 Hz, 2H), 7.00 (br d, *J* = 7.2 Hz, 1H), 6.96 - 6.64 (m, 2H), 6.42 (br d, *J* = 6.8 Hz, 1H), 4.04 (br d, *J* = 9.2 Hz, 1H), 3.78 (br t, *J* = 10.8 Hz, 1H), 3.63 - 3.34 (m, 4H), 2.80 - 2.50 (m, 1H), 2.38 (s, 3H), 1.62 (s, 6H), 1.24 (d, *J* = 12.2 Hz, 6H) |
| 8 | 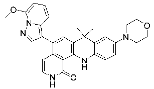 | 508 | ¹H NMR (400 MHz, CDCl₃) δ ppm: 11.85 - 11.57 (m, 1H), 9.45 (br s, 1H), 8.09 (s, 1H), 7.65 (br d, *J* = 1.8 Hz, 1H), 7.57 - 7.40 (m, 1H), 7.21 - 7.04 (m, 2H), 7.03 - 6.77 (m, 3H), 6.65 - 6.44 (m, 1H), 6.18 (d, *J* = 7.2 Hz, 1H), 4.22 (s, 3H), 4.09 (br t, *J =* 4.2 Hz, 4H), 3.42 (br s, 4H), 1.62 (s, 6H) |

### Example 10

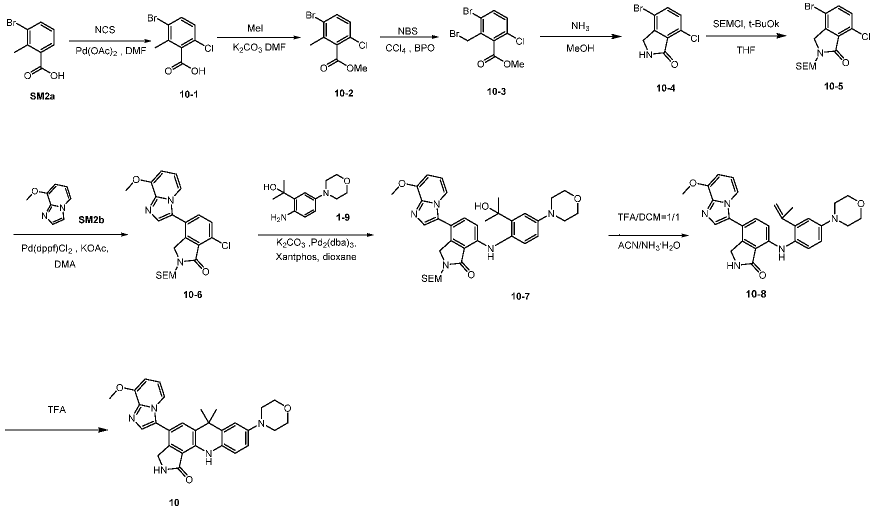

### Synthesis of intermediate 10-1

Compound SM**2a** (200 g) was dissolved in N,N-dimethylformamide (1 L), and NCS (150 g) and palladium(II) acetate (20.9 g) were added. The above mixture was reacted at 110 °C for 16 hours. The reaction solution was poured into water (2 L), and the mixture was extracted with ethyl acetate (2 L × 3). The organic phases were combined, dried over anhydrous sodium sulfate, filtered, and rotary evaporated to dryness. The resulting crude product was purified by column chromatography (silica, petroleum ether/ethyl acetate = 1/0 to 0/1) to give yellow solid compound **10-1** (118 g, yield: 50.9%). LCMS (ESI) m/z: 249/251.

### Synthesis of intermediate 10-2

Compound **10-1** (88 g) was dissolved in N,N-dimethylformamide (620 mL), and iodomethane (55 g) was added. The above solution was reacted at 110 °C for 16 hours. The reaction solution was cooled to room temperature and poured into water (1.8 L). The mixture was extracted with ethyl acetate (1.0 L × 3). The combined organic phase was dried over anhydrous sodium sulfate, filtered, and rotary evaporated to dryness to give yellow oily compound **10-2** (86 g) as a crude product, which was directly used in the next reaction. LCMS (ESI) m/z: 263/265.

### Synthesis of intermediate 10-3

Compound **10-2** (86 g) was dissolved in carbon tetrachloride (860 mL), and N-bromosuccinimide (63.8 g) and benzoyl peroxide (3.95 g) were added. The above mixture was reacted at 80 °C for 16 hours. The reaction solution was cooled to room temperature and filtered. The filtrate was rotary evaporated to dryness to give yellow oily compound **10-3** (106 g) as a crude product, which was directly used in the next reaction. LCMS (ESI) m/z: 341/343.

### Synthesis of intermediate 10-4

Compound **10-3** (106 g) was dissolved in methanol (530 mL), and a solution of ammonia in methanol (530 mL) was added under an ice-water bath. The above reaction solution was reacted at room temperature for 4 hours. The reaction solution was filtered, and the filter cake was dried to give off-white solid compound **10-4** (62 g) as a crude product, which was directly used in the next reaction. LCMS (ESI) m/z: 246/248.

### Synthesis of intermediate 10-5

Compound **10-4** (62 g) was dissolved in anhydrous tetrahydrofuran (1 L), and potassium tert-butoxide (56.5 g) and 2-(trimethylsilyl)ethoxymethyl chloride (125.8 g) were added under an ice-water bath. The above mixture was reacted at room temperature for 16 hours. The reaction solution was added to water (1 L), and the mixture was extracted with ethyl acetate (1 L x 3). The combined organic phase was dried over anhydrous sodium sulfate, filtered, and rotary evaporated to dryness. The resulting crude product was purified by column chromatography (silica, petroleum ether/ethyl acetate = 1/5 to 1/1) to give yellow oily compound **10-5** (43 g). LCMS (ESI) m/z: 376/378. ¹H NMR (400 MHz, CDCl₃) δ ppm: 7.61 (d, *J* = 8.4 Hz, 1H), 7.33 (d, *J =* 8.4 Hz, 1H), 5.06 (s, 2H), 4.38 (s, 2H), 3.63 - 3.56 (m, 2H), 1.01 - 0.91 (m, 2H), 0.01 (s, 9H).

### Synthesis of intermediate 10-6

Compound **10-5** (380 mg) and compound **SM2b** (179 mg) were dissolved in N,N-dimethylacetamide (2 mL), and potassium acetate (198 mg) was added. The reaction system was purged with nitrogen three times, and then [1,1'-bis(diphenylphosphino)ferrocene]palladium(II) dichloride (73.9 mg) was added. The above mixture was stirred at 110 °C for 3 hours under nitrogen. The reaction solution was cooled to room temperature and filtered, and the filtrate was rotary evaporated to dryness. The resulting crude product was purified by column chromatography (silica, petroleum ether/ethyl acetate = 1/5 to 0/1) to give brown solid compound **10-6** (260 mg, yield: 58%). LCMS (ESI) m/z: 445.

### Synthesis of intermediate 10-7

Compound **10-6** (120 mg) and compound **1-9** (63.8 mg) were dissolved in dioxane (5 mL). Potassium carbonate (114 mg) and 4,5-bis(diphenylphosphino)-9,9-dimethylxanthene (31.2 mg) were added. The reaction system was purged with nitrogen three times, and then tris(dibenzylideneacetone)dipalladium(0) (24.7 mg) was added. The above mixture was stirred at 100 °C for 12 hours under nitrogen. The reaction solution was cooled to room temperature and filtered, and the filtrate was rotary evaporated to dryness. The resulting crude product was purified by column chromatography (silica, petroleum ether/ethyl acetate = 1/3 to 0/1) to give brown solid compound **10-7** (85 mg, yield: 49%). LCMS (ESI) m/z: 644.

### Synthesis of intermediate 10-8

Compound **10-7** (85 mg) was dissolved in dichloromethane (1 mL), and trifluoroacetic acid (1 mL) was added dropwise. The above solution was stirred at room temperature for 2 hours. The reaction solution was concentrated under reduced pressure to give a brown oily compound. The brown oily compound was dissolved in acetonitrile (1 mL). Ammonia solution (1 mL) was added, and the mixture was stirred at room temperature for another 10 hours. The mixture was rotary evaporated to dryness and purified by high performance liquid chromatography (chromatography column: Waters Xbridge BEH C18 100*30mm*10um; mobile phase: [water (ammonium bicarbonate) - acetonitrile]; B%: 30% - 60%, 8 min) to give yellow solid compound **10-8** (36 mg, yield: 57%). LCMS (ESI) m/z: 496.

### Synthesis of compound 10

Compound **10-8** (34 mg) was added to trifluoroacetic acid (3 mL). The above solution was stirred at 80 °C for 12 hours. The reaction solution was rotary evaporated to dryness, and purified by high performance liquid chromatography (chromatography column: Phenomenex Luna C18 75*30mm*3um; mobile phase: [water (formic acid) - acetonitrile]; B%: 1% - 40%, 8 min) to give yellow solid compound **10** (4.4 mg, yield: 12.9%). LCMS (ESI) m/z: 496. ¹H NMR (400 MHz, DMSO-*d₆*) δ ppm: 8.91 (s, 1H), 8.57 (s, 1H), 7.84 (d, *J =* 6.8 Hz, 1H), 7.68 (d, *J =* 6.4 Hz, 2H), 7.05 (d, *J =* 2.4 Hz, 1H), 6.92 (d, *J =* 8.8 Hz, 1H), 6.85 (t, *J =* 7.2 Hz, 1H), 6.77 (dd, *J =* 2.4, 8.8 Hz, 1H), 6.71 (d, *J =* 7.2 Hz, 1H), 4.28 (s, 2H), 3.96 (s, 3H), 3.76 - 3.73 (m, 4H), 3.05 - 3.02 (m, 4H), 1.61 (s, 6H).

The following examples were synthesized using the same methods as above:

| Example | Structure | MS:M+ H⁺ | ¹H NMR |
|---|---|---|---|
| 9 | 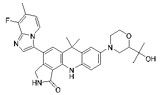 | 556 | ¹H NMR (400 MHz, CD₃OD) δ ppm: 7.91 (br d, *J* = 7.2 Hz, 1H), 7.73 - 7.59 (m, 2H), 7.12 (br s, 1H), 6.90 - 6.81 (m, 3H), 4.29 (s, 2H), 4.04 (br dd, *J =* 2.0, 11.2 Hz, 1H), 3.78 (dt, *J =* 2.0, 11.2 Hz, 1H), 3.52 (br d, *J* = 11.2 Hz, 1H), 3.48 - 3.43 (m, 1H), 2.74 (dt, *J* = 2.4, 11.6 Hz, 1H), 2.59 (s, 1H), 2.47 - 2.34 (m, 4H), 1.65 (s, 6H), 1.25 (d, *J =* 12.4 Hz, 6H) |
| 11 | 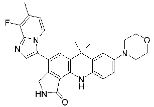 | 498 | ¹H NMR (400 MHz, CD₃OD) δ ppm: 7.91 (d, *J* = 7.2 Hz, 1H), 7.66 (d, *J* = 15.2 Hz, 2H), 7.12 (s, 1H), 6.96 - 6.71 (m, 3H), 4.30 (s, 2H), 3.87 - 3.83 (m, 4H), 3.16 - 2.99 (m, 4H), 2.39 (d, *J =* 2.4 Hz, 3H), 1.66 (s, 6H) |
| 12 | 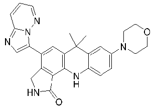 | 467 | ¹H NMR (400 MHz, DMSO-*d₆*) δ ppm: 8.95 (s, 1H), 8.68 - 8.60 (m, 2H), 8.22 - 8.19 (m, 2H), 8.07 (s, 1H), 7.28 (dd, *J* = 9.2, 4.4 Hz, 1H), 7.06 (d, *J* = 2.3 Hz, 1H), 6.91 (d, *J =* 8.6 Hz, 1H), 6.77 (dd, *J* = 8.7, 2.4 Hz, 1H), 4.47 (s, 2H), 3.80 - 3.68 (m, 4H), 3.09 - 2.98 (m, 4H), 1.63 (s, 6H) |
| 14 | 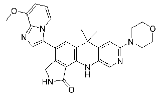 | 497 | ¹H NMR (400 MHz, CD₃OD) δ ppm: 8.08 -8.06 (m, 1H), 7.99 -7.98 (m, 1H), 7.92 (s, 1H), 7.77 (s, 1H), 7.32 -7.30 (m, 2H), 7.00 (s, 1H), 4.32 (s, 2H), 4.16 (s, 3H), 3.82 -3.80(m, 4H), 3.42 - 3.40 (m, 4H), 1.67 (s, 6H) |
| 16 | 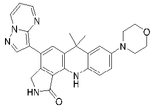 | 467 | ¹H NMR (400 MHz, DMSO-*d₆*) δ ppm: 9.18 (dd, *J* = 7.0, 1.6 Hz, 1H), 8.85 (s, 1H), 8.74 - 8.60 (m, 2H), 8.53 (s, 1H), 8.29 (s, 1H), 7.12 (dd, *J* = 6.9, 4.0 Hz, 1H), 7.05 (s, 1H), 6.87 (s, 1H), 6.77 (s, 1H), 4.58 (s, 2H), 3.75 (br s, 4H), 3.04 (br s, 4H), 1.63 (s, 6H) |
| 17 | 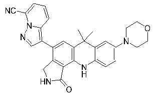 | 491 | ¹H NMR (400 MHz, DMSO-*d₆*) δ ppm: 8.85 (s, 1H), 8.62 (s, 1H), 8.52 (s, 1H), 8.22 (s, 1H), 8.10 - 8.03 (m, 1H), 7.88 (dd, *J* = 7.1, 1.1 Hz, 1H), 7.65 (s, 1H), 7.41 (dd, *J* = 9.1, 7.1 Hz, 1H), 7.06 (d, *J* = 2.6 Hz, 1H), 6.90 (d, *J* = 8.6 Hz, 1H), 6.77 (dd, *J* = 8.7, 2.6 Hz, 1H), 4.44 (s, 2H), 3.76 - 3.71 (m, 4H), 3.06 - 3.00 (m, 4H), 1.63 (s, 6H) |
| 18 | 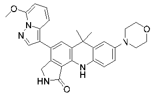 | 496 | ¹HNMR (400 MHz, CD₃OD) δ ppm: 8.14 (s, 1H), 7.65 (s, 1H), 7.33 (dd, *J* = 8.9, 7.3 Hz, 1H), 7.26 (dd, *J* = 8.9, 1.1 Hz, 1H), 7.09 (s, 1H), 6.83 - 6.81(m, 2H), 6.41 (dd, *J* = 7.3, 0.9 Hz, 1H), 4.41 (s, 2H), 4.18 (s, 3H), 3.84 - 3.80 (m, 4H), 3.08 - 3.04(m, 4H), 1.64 (s, 6H) |
| 19 | 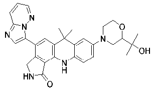 | 525 | ¹HNMR (400 MHz, CD₃OD) δ ppm: 8.56 - 8.48 (m, 1H), 8.16 (s, 1H), 8.07 (dd, *J =* 9.2, 1.6 Hz, 1H), 7.91 (s, 1H), 7.27 (dd, *J* = 9.2, 4.4 Hz, 1H), 7.10 (s, 1H), 6.84 - 6.80 (m, 2H), 4.47 (s, 2H), 4.02 (d, *J* = 10.7 Hz, 1H), 3.78 - 3.72 (m, 1H), 3.50 (d, *J* = 10.2 Hz, 1H), 3.42 (d, *J* = 10.4 Hz, 1H), 3.28 - 3.32 (m, 1H), 2.76 - 2.66 (m, 1H), 2.61 - 2.51 (m, 1H), 1.65 (s, 6H), 1.24 (s, 3H), 1.21 (s, 3H) |
| 20 | 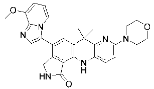 | 497 | ¹H NMR (400 MHz, DMSO-*d₆*) δ ppm: 8.83 (s, 1H), 8.59 (s, 1H), 7.84 (d, *J* = 6.6 Hz, 1H), 7.70 (s, 1H), 7.67 (s, 1H), 7.44 (d, *J* = 8.7 Hz, 1H), 6.87 (t, *J* = 7.2 Hz, 1H), 6.74 - 6.65 (m, 2H), 4.30 (s, 2H), 3.97 (s, 3H), 3.77 - 3.67 (m, 4H), 3.38 - 3.34 (m, 4H), 1.62 (s, 6H) |
| 21 | 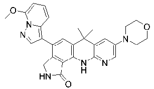 | 497 | ¹H NMR (400 MHz, DMSO-*d₆*) δ ppm: 9.09 (s, 1H), 8.62 (s, 1H), 8.27 (s, 1H), 7.72 (d, *J* = 2.6 Hz, 1H), 7.62 (s, 1H), 7.50 (d, *J* = 2.6 Hz, 1H), 7.32 - 7.17 (m, 2H), 6.52 - 6.35 (m, 1H), 4.40 (s, 2H), 4.09 (s, 3H), 3.77 - 3.67 (m, 4H), 3.09 - 2.98 (m, 4H), 1.61 (s, 6H) |
| 22 | 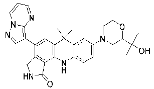 | 525 | ¹HNMR (400 MHz, CD₃OD) δ ppm: 8.91 (dd, *J* = 7.1, 1.7 Hz, 1H), 8.58 (dd, *J* = 4.0, 1.7 Hz, 1H), 8.34 (s, 1H), 8.23 (s, 1H), 7.10 (s, 1H), 7.03 (dd, *J* = 7.1, 4.0 Hz, 1H), 6.85 - 6.78 (m, 2H), 4.58 (s, 2H), 4.02 (d, *J =* 7.0 Hz, 1H), 3.86 - 3.67 (m, 1H), 3.52 - 3.43 (m, 1H), 3.42 (d, *J =* 9.8 Hz, 1H), 3.33 - 3.30 (m, 1H), 2.84 - 2.65 (m, 1H), 2.63 - 2.34 (m, 1H), 1.66 (s, 6H), 1.24 (s, 3H), 1.21 (s, 3H) |
| 23 | 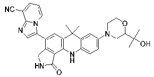 | 549 | ¹H NMR (400 MHz, DMSO-*d₆*) δ ppm: 8.94 (s, 1H), 8.61 (s, 1H), 8.57 - 8.50 (m, 1H), 8.04 - 8.00 (m, 1H), 7.97 (s, 1H), 7.77 (s, 1H), 7.10 (t, *J* = 7.0 Hz, 1H), 7.05 (d, *J =* 2.3 Hz, 1H), 6.94 (d, *J* = 8.6 Hz, 1H), 6.79 (dd, *J* = 8.7, 2.4 Hz, 1H), 4.48 (s, 1H), 4.31 (s, 2H), 3.97 (dd, *J* = 11.0, 2.3 Hz, 1H), 3.67 - 3.61 (m, 1H), 3.54 (d, *J =* 11.3 Hz, 1H), 3.41 (d, *J =* 11.6 Hz, 1H), 3.29 (dd, *J* = 10.5, 2.0 Hz, 1H), 2.63 - 2.56 (m, 1H), 2.48 - 2.42 (m, 1H), 1.62 (s, 6H), 1.17 (s, 3H), 1.11 (s, 3H) |
| 24 | 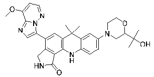 | 555 | ¹H NMR (400 MHz, DMSO-*d₆*) δ ppm: 8.94 (s, 1H), 8.62 (s, 1H), 8.46 (d, *J =* 5.5 Hz, 1H), 8.14 (s, 1H), 7.88 (s, 1H), 7.05 (d, *J* = 2.4 Hz, 1H), 6.92 (d, *J =* 8.6 Hz, 1H), 6.80 - 6.72 (m, 2H), 4.48 (s, 1H), 4.44 (s, 2H), 4.08 (s, 3H), 3.97 (dd, *J =* 11.0, 2.4 Hz, 1H), 3.67 - 3.61 (m, 1H), 3.54 (d, *J =* 11.5 Hz, 1H), 3.40 (d, *J* = 11.5 Hz, 1H), 3.30 (dd, *J* = 10.5, 2.1 Hz, 1H), 2.64 - 2.56 (m, 1H), 2.48 - 2.41 (m, 1H), 1.62 (s, 6H), 1.17 (s, 3H), 1.11 (s, 3H) |

### Example 13

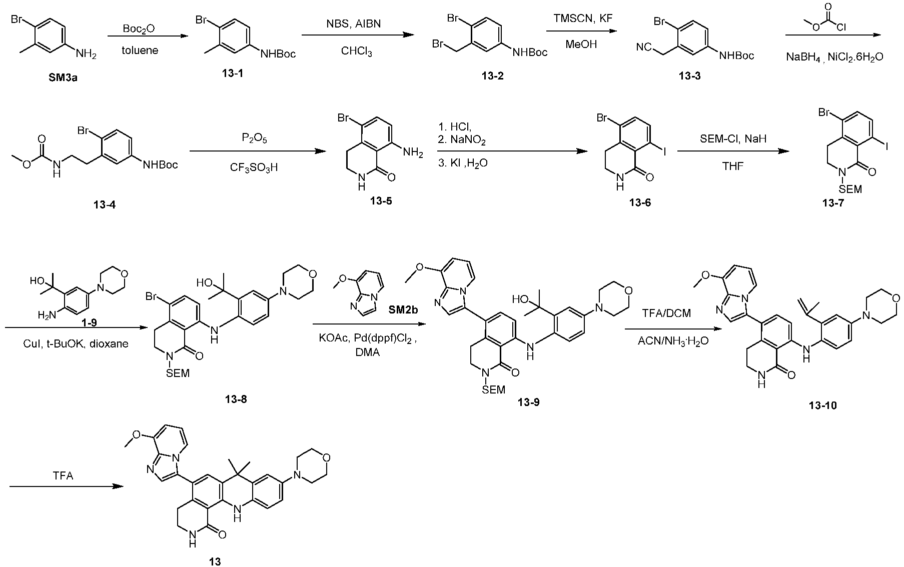

### Synthesis of intermediate 13-1

Compound **SM3a** (100 g) was dissolved in toluene (700 mL), and di-tert-butyl dicarbonate (117.3 g) was added. The above mixture was stirred at 100 °C for 20 hours. The reaction solution was cooled to room temperature and poured into ice water (1000 mL). The mixture was extracted twice with ethyl acetate (1000 mL). The organic phases were combined, washed with saturated brine, dried and concentrated to give a crude product. The crude product was purified by slurring (petroleum ether/ethyl acetate = 10/1, 200 mL) to give white solid compound **13-1** (120 g, yield: 78%). LCMS (ESI) m/z: 286/288.

### Synthesis of intermediate 13-2

Compound **16-1** (100 g) was dissolved in chloroform (700 mL), and N-bromosuccinimide (93.29 g) and azobisisobutyronitrile (11.48 g) were added. The above mixture was stirred at 80 °C for 20 hours. The reaction solution was cooled to room temperature and poured into ice water (400 mL). The mixture was extracted twice with dichloromethane (500 mL). The organic phases were combined, washed with saturated brine, dried and concentrated. The resulting crude product was purified by column chromatography (silica, petroleum ether/ethyl acetate = 100/1 to 20/1) to give yellow solid compound **13-2** (40 g, yield: 31.4%). LCMS (ESI) m/z: 364/366.

### Synthesis of intermediate 13-3

Compound **13-2** (40 g) was dissolved in methanol (200 mL), and trimethylsilyl cyanide (76.1 g) and potassium fluoride (2.81 g) were added. The above reaction solution was stirred at 20 °C for 20 hours. The reaction solution was poured into water (200 mL), and the mixture was extracted twice with ethyl acetate (100 mL). The organic phases were combined, washed with saturated brine, dried and concentrated. The resulting crude product was purified by column chromatography (silica, petroleum ether/ethyl acetate = 100/1 to 10/1) to give yellow solid compound **13-3** (22 g, yield: 64.5%). LCMS (ESI) m/z: 311/313.

### Synthesis of intermediate 13-4

Compound **13-3** (20 g), methanol (140 mL), nickel(II) chloride hexahydrate (1.5 g) and methyl chloroformate (18.8 g) were added to a 500-mL round-bottom flask at 0 °C. The above mixture was stirred at 0 °C for 15 minutes, and sodium borohydride (12.3 g) was added. The reaction solution was stirred at 20 °C for another 20 hours. The reaction solution was poured into an iced sodium bicarbonate aqueous solution (100 mL), and the mixture was extracted twice with ethyl acetate (100 mL). The organic phases were combined, washed with saturated brine, dried and concentrated. The resulting crude product was purified by column chromatography (silica, petroleum ether/ethyl acetate = 30/1 to 5/1) to give yellow oily compound **13-4** (16.1 g, yield: 68%). LCMS (ESI) m/z: 373/375.

### Synthesis of intermediate 13-5

Compound **13-4** (16 g) was dissolved in trifluoromethanesulfonic acid (100 mL), and phosphorus pentoxide (15 g) was added after the reaction solution was cooled to 0 °C. The above mixture was stirred at 100 °C for 20 hours. The reaction solution was cooled to room temperature and slowly poured into an iced sodium bicarbonate aqueous solution (200 mL). The mixture was extracted twice with ethyl acetate (150 mL). The organic phases were combined, washed with saturated brine, dried and concentrated to give yellow solid compound **13-5** (11 g) as a crude product, which was directly used in the next reaction. LCMS (ESI) m/z: 241/243.

### Synthesis of intermediate 13-6

Compound **13-5** (10 g) was dissolved in concentrated hydrochloric acid (12M, 50 mL), and the mixture was stirred at 0 °C for 15 minutes. A solution of sodium nitrite (3.72 g) in water (12 mL) was slowly added to the above solution, and the mixture was stirred at 0 °C for another 1.5 hours. A solution of potassium iodide (34.4 g) in water (50 mL) was slowly added to the above solution, and the mixture was stirred at 20 °C for 20 hours. The reaction solution was poured into water (40 mL), and the mixture was extracted twice with ethyl acetate (50 mL). The organic phases were combined, washed with saturated brine, dried and concentrated. The resulting crude product was purified by column chromatography (silica, petroleum ether/ethyl acetate = 10/1 to 1/1) to give yellow solid compound **13-6** (5 g). LCMS (ESI) m/z: 352/354.

### Synthesis of intermediate 13-7

Compound **13-6** (5 g), tetrahydrofuran (35 mL) and sodium hydride (1.7 g) were added to a 250 mL round-bottom flask at 0 °C. The reaction solution was stirred at 0 °C for 0.5 hours, and then 2-(trimethylsilyl)ethoxymethyl chloride (4.74 g) was added to the reaction solution. The above reaction solution was stirred at 20 °C for another 12 hours. The reaction solution was poured into ice water (50 mL), and the mixture was extracted twice with ethyl acetate (50 mL). The organic phases were combined, washed with saturated brine, dried and concentrated to give a crude product. The resulting crude product was purified by thin layer chromatography (petroleum ether/ethyl acetate = 20/1 to 3/1) to give yellow solid compound **13-7** (2.3 g, yield: 33.6%). LCMS (ESI) m/z: 482/484.

### Synthesis of intermediate 13-8

Compound **13-7** (500 mg) was dissolved in 1,4-dioxane (5 mL), and compound **1-9** (245 mg), potassium tert-butoxide (349 mg) and copper(I) iodide (1.97 g) were added. The above mixture was stirred at 110 °C for 16 hours under nitrogen. The reaction solution was cooled to room temperature and filtered. The filtrate was rotary evaporated to dryness, and the resulting crude product was purified by column chromatography (silica, dichloromethane/methanol = 1/0 to 10/1) to give yellow oily compound **13-8** (170 mg, yield: 28%). LCMS (ESI) m/z: 591.

### Synthesis of intermediate 13-9

Compound **13-8** (170 mg) was dissolved in N,N-dimethylacetamide (2 mL), and compound **SM2b** (170 mg) and potassium acetate (56.4 mg) were added. The reaction system was purged with nitrogen three times, and then [1,1'-bis(diphenylphosphino)ferrocene]palladium(II) dichloride (21.0 mg) was added. The above mixture was stirred at 110 °C for 2 hours. The reaction solution was rotary evaporated to dryness, and the resulting crude product was purified by column chromatography (silica, dichloromethane/methanol = 1/0 to 10/1) to give brown solid compound **13-9** (110 mg, yield: 58.1%). LCMS (ESI) m/z: 658.

### Synthesis of intermediate 13-10

Compound **13-9** (110 mg) was added to dichloromethane (1 mL), and trifluoroacetic acid (1 mL) was added dropwise. The above solution was stirred at room temperature for 2 hours. The reaction solution was concentrated under reduced pressure to give a brown oily compound. The brown oily compound was dissolved in acetonitrile (1 mL). Ammonia solution (1 mL) was added, and the mixture was stirred at room temperature for another 10 hours. The mixture was rotary evaporated to dryness, and the resulting crude product was purified by column chromatography (silica, dichloromethane/methanol = 1/0 to 10/1) to give brown solid compound **13-10** (75 mg, yield: 88%). LCMS (ESI) m/z: 510.

### Synthesis of compound 13

Compound **13-10** (75 mg) was added to trifluoroacetic acid (3 mL). The above solution was stirred at 80 °C for 12 hours. The reaction solution was rotary evaporated to dryness, and purified by high performance liquid chromatography (chromatography column: Phenomenex Luna C18 75*30mm*3um; mobile phase: [water (formic acid) - acetonitrile]; B%: 10% - 30%, 8 min) to give yellow solid compound **13** (10.8 mg, yield: 14%). LCMS (ESI) m/z: 510. ¹H NMR (400 MHz, DMSO-*d₆*) δ ppm: 11.28 (br s, 1H), 8.12 (br s, 1H), 7.68 - 7.45 (m, 3H), 7.01 (br s, 1H), 6.88 - 6.74 (m, 4H), 3.98 (s, 3H), 3.73 (br s, 4H), 3.44 (br s, 2H), 3.23 (br s, 2H), 3.02 (br s, 4H), 1.53 (s, 6H).

### Example 15

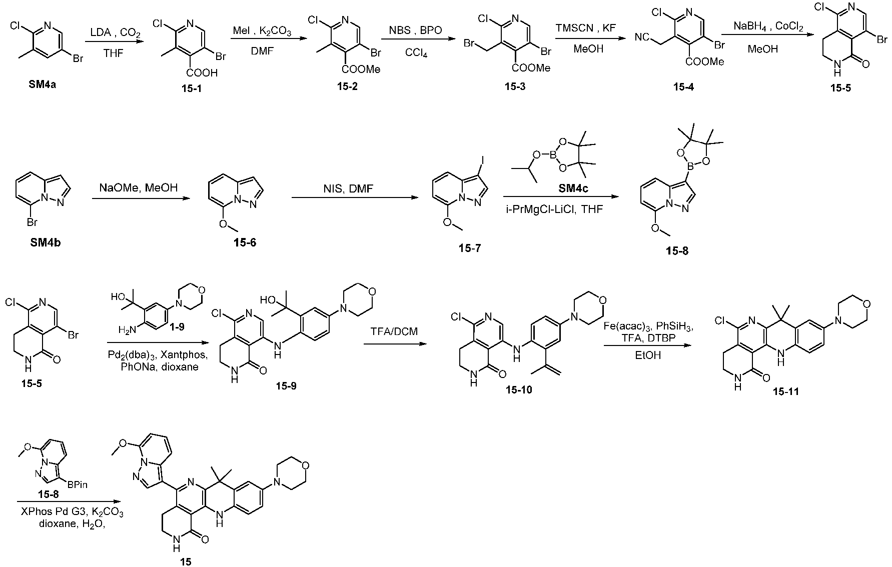

### Synthesis of intermediate 15-1

**SM4a** (2 g) was dissolved in tetrahydrofuran (15 mL), and the mixture was cooled to -78 °C under nitrogen. Lithium diisopropylamide (2.0 M, 5.33 mL) was slowly added to the above reaction system. The above reaction system was stirred at -78 °C for 0.5 hours. Then, CO₂ was blown into the reaction solution, and the reaction solution was stirred at -78 °C for another 0.5 hours. The reaction solution was slowly warmed to 20 °C and stirred for another 1 hour. Water (30 mL) was slowly added dropwise to the reaction solution to quench the reaction, and the mixture was back extracted with ethyl acetate (50 mL x 2). The aqueous phase was collected and rotary evaporated to dryness to give off-white solid compound **15-1** (2 g) as a crude product, which was directly used in the next reaction. LCMS (ESI) m/z: 250/252.

### Synthesis of intermediate 15-2

Compound **15-1** (2 g) was dissolved in N,N-dimethylformamide (20 mL), and potassium carbonate (2.21 g) and iodomethane (906 mg) were successively added to the solution. The above mixture was stirred at 20 °C for 10 hours. The reaction solution was slowly poured into ice water (100 mL), and the mixture was extracted with ethyl acetate (50 mL x 3). The organic phases were combined, washed with saturated brine, dried over anhydrous sodium sulfate, and concentrated to give a crude product. The crude product was purified by column chromatography (silica, petroleum ether/ethyl acetate = 50/1 - 10/1) to give yellow oily compound **15-2** (1.5 g). LCMS (ESI) m/z: 264/266.

### Synthesis of intermediate 15-3

Compound **15-2** (1.5 g) was dissolved in carbon tetrachloride (30 mL), and bromosuccinimide (1.1 g) and benzoyl peroxide (138 mg) were successively added. The above mixture was stirred at 90 °C for 10 hours. The reaction was cooled to room temperature, and the reaction solution was slowly poured into ice water (100 mL). The mixture was extracted with ethyl acetate (50 mL x 3). The organic phases were combined, washed with saturated brine, dried over anhydrous sodium sulfate, and concentrated to give a crude product. The crude product was purified by column chromatography (silica, petroleum ether/ethyl acetate = 50/1-1/1) to give yellow oily compound **15-3** (1 g, yield: 51.3%). LCMS (ESI) m/z: 342/344.

### Synthesis of intermediate 15-4

Compound **15-3** (400 mg) was dissolved in methanol (10 mL), and potassium fluoride (388 mg) and trimethylsilyl cyanide (577 mg) were successively added. The above mixture was stirred at 20 °C for 10 hours. The reaction solution was slowly poured into ice water (30 mL), and the mixture was extracted with ethyl acetate (30mL x 3). The organic phases were combined, washed with saturated brine, dried over anhydrous sodium sulfate, and concentrated to give a crude product. The crude product was purified by column chromatography (silica, petroleum ether/ethyl acetate = 20/1-5/1) to give yellow oily compound **15-4** (200 mg, yield: 59.3%). LCMS (ESI) m/z: 289/291.

### Synthesis of intermediate 15-5

Compound **15-4** (150 mg) was dissolved in methanol (10 mL), and the mixture was cooled to 0 °C. Cobalt chloride (403 mg) and sodium borohydride (118 mg) were successively added to the solution under nitrogen. The above mixture was stirred at 20 °C for 10 hours. The reaction solution was slowly poured into a saturated aqueous solution of ammonium chloride (20 mL), and the mixture was extracted with ethyl acetate (30 mL x 3). The combined organic phase was washed with saturated brine, dried over anhydrous sodium sulfate, and concentrated to give a crude product. The crude product was purified by column chromatography (silica, petroleum ether/ethyl acetate = 5/1-1/1) to give yellow solid compound **15-5** (100 mg, yield: 73.8%). LCMS (ESI) m/z: 261/263.

### Synthesis of intermediate 15-6

Compound **SM4b** (500 mg) and a solution of sodium methoxide in methanol (5 mL, 30% w/w) were added to a 100 mL single-neck flask. The above mixture was reacted at room temperature for 2 hours. Water (20 mL) was added to quench the reaction, and then the mixture was extracted with dichloromethane (50 mL × 3). The organic phases were combined, dried over anhydrous sodium sulfate and filtered. The filtrate was concentrated under reduced pressure to give a crude product. The resulting crude product was purified by silica gel column chromatography with an eluent system (petroleum ether/ethyl acetate = 1:1) to give yellow oily compound **15-6** (320 mg, yield: 85%). LCMS (ESI) m/z: 149.

### Synthesis of intermediate 15-7

Compound **15-6** (320 mg), N,N-dimethylformamide (10 mL) and N-iodosuccinimide (1.46 g, 6.48 mmol) were added to a 100 mL single-neck flask. The above mixture was reacted at 50 °C for 1.5 hours under nitrogen. The reaction was cooled to room temperature, and quenched with added water (10 mL). The mixture was extracted with ethyl acetate (50 mL × 3). The combined organic phase was washed with saturated sodium chloride solution (50 mL), dried over anhydrous sodium sulfate and filtered. The filtrate was concentrated under reduced pressure. The resulting residue was purified by silica gel column chromatography with an eluent system (petroleum ether/ethyl acetate = 2:1) to give yellow oily compound **15-7** (430 mg, yield: 73%). LCMS (ESI) m/z: 275.

### Synthesis of intermediate 15-8

Compound **15-7** (430 mg), tetrahydrofuran (10 mL) and **SM4c** (875 m) were added to a 100 mL three-neck flask. The above solution was cooled to 0 °C under nitrogen, and a 1.3M isopropylmagnesium chloride lithium chloride solution in tetrahydrofuran (2.4 mL) was added. The above mixture was reacted at 0 °C for 2 hours. The mixture was warmed to room temperature, and a saturated solution of ammonium chloride (5 mL) was added to the reaction mixture to quench the reaction. The mixture was extracted with ethyl acetate (30 mL × 3). The combined organic phase was washed with saturated sodium chloride solution (50 mL), dried over anhydrous sodium sulfate and filtered. The filtrate was concentrated under reduced pressure. The resulting residue was purified by silica gel column chromatography with an eluent system (petroleum ether/ethyl acetate = 1:1) to give yellow oily compound **15-8** (170 mg). LCMS (ESI) m/z: 275.

### Synthesis of intermediate 15-9

Compound **15-5** (600 mg) and compound **1-9** (542 mg) were dissolved in 1,4-dioxane (6 mL), and sodium phenoxide (532 mg), 4,5-bis(diphenylphosphino)-9,9-dimethylxanthene (132.7 mg) and tris(dibenzylideneacetone)dipalladium(0) (210 mg) were added. The above mixture was reacted at 90 °C for 12 hours under nitrogen. The reaction solution was cooled to room temperature and filtered. The filtrate was concentrated, and then the resulting crude product was purified by chromatography (silica, dichloromethane/methanol = 20/1) to give yellow oily compound **15-9** (200 mg, yield: 21%). LCMS (ESI) m/z: 417.

### Synthesis of intermediate 15-10

Compound **15-9** (200 mg) was dissolved in dichloromethane (5 mL), and trifluoroacetic acid (2 mL) was added. The above solution was reacted at room temperature for 20 hours. The reaction solution was rotary evaporated to dryness, and the resulting crude product was purified by chromatography (silica, dichloromethane/methanol = 20/1) to give yellow solid compound **15-10** (170 mg, yield: 89%). LCMS (ESI) m/z: 399.

### Synthesis of intermediate 15-11

Compound **15-10** (170 mg) was dissolved in anhydrous ethanol (5 mL), and phenylsilane (115 mg), trifluoroacetic acid (97.2 mg), iron(III) acetylacetonate (75.3 mg) and di-tert-butyl peroxide (187 mg) were added. The above mixture was reacted at 85 °C for 24 hours under oxygen. The reaction solution was concentrated to give a crude product. The resulting crude product was purified by chromatography (silica, dichloromethane/methanol = 20/1) to give yellow solid compound **15-11** (95 mg, yield: 56%).

LCMS (ESI) m/z: 399.

### Synthesis of compound 15

Compound **15-11** (80 mg) and compound **15-8** (82.4 mg) were dissolved in 1,4-dioxane (2 mL) and water (0.5 mL), and potassium carbonate (55.4 mg) and (2-dicyclohexylphosphino-2',4',6'-triisopropyl-1,1'-biphenyl)[2-(2'-amino-1,1'-biphenyl)]palladium(II) methanesulfonate (17 mg) were added. The above mixture was reacted at 100 °C for 16 hours under nitrogen. The reaction solution was cooled to room temperature and rotary evaporated to dryness. The resulting crude product was purified by high performance liquid chromatography (chromatography column: Phenomenex luna C18 75*30mm*3 um; mobile phase: [water (trifluoroacetic acid) - acetonitrile]; B%: 15% - 45%, 8 min) to give yellow solid compound **15** (24.3 mg, yield: 23%). LCMS (ESI) m/z: 511. ¹H NMR (400 MHz, CDCl₃) δ ppm: 11.22 - 9.78 (m, 1H), 8.24 - 7.99 (m, 1H), 7.76 (d, *J =* 8.8 Hz, 1H), 7.27 - 7.19 (m, 1H), 7.15 - 6.55 (m, 2H), 6.20 (br d, *J* = 7.2 Hz, 1H), 6.09 - 5.78 (m, 1H), 4.20 (s, 3H), 4.11 - 3.73 (m, 4H), 3.70 - 3.47 (m, 3H), 3.45 - 2.73 (m, 5H), 1.72 (s, 6H).

### Example 25

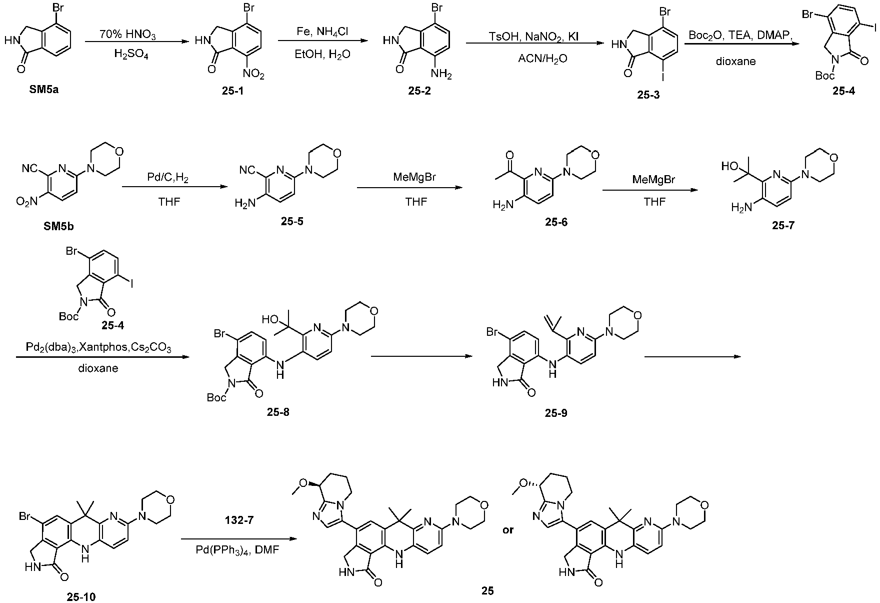

### Synthesis of intermediate 25-1

4-Bromoisoindolin-1-one (25 g, 117.9 mmol) and sulfuric acid (120 mL) were added to a three-neck flask and cooled to -10 °C. Nitric acid (15 mL) was added dropwise to the above solution, and the mixture was stirred at 25 °C for 4 hours. After the reaction was completed, the reaction solution was poured into ice water (200 mL) and filtered to collect the solid. The resulting filter cake was washed with water (100 mL) and then dried to give white solid compound **25-1** (27.8 g, yield 91.7%). LCMS (ESI) m/z: 257/259.

### Synthesis of intermediate 25-2

Compound **25-1** (20 g, 77.8 mmol) was dissolved in 240 mL of a mixed solvent of ethanol and water (V/V = 5:1). Iron (21.8 g, 389.1 mmol) and ammonium chloride (24.7 g, 466.9 mmol) were added. The above mixture was reacted at 85 °C for 2 hours. The reaction solution was cooled to room temperature and filtered. The filtrate was collected and concentrated to give yellow solid compound **25-2** (17.5 g) as a crude product, which was directly used in the next reaction. LCMS (ESI) m/z: 227/229.

### Synthesis of intermediate 25-3

Compound **25-2** (9.5 g, the crude product) was dissolved in 125 mL of a mixed solvent of acetonitrile and water (V/V = 5:1). The reaction system was cooled to -15 °C, and then p-toluenesulfonic acid (33.15 g, 192.51 mmol), sodium nitrite (8.66 g, 125.5 mmol) and potassium iodide (27.09 g, 163.22 mmol) were added. After the addition was completed, the mixture was heated to 40 °C and stirred overnight. The reaction solution was cooled to room temperature and then poured into water (300 mL). The mixture was extracted with ethyl acetate (300 mL × 3). The organic phases were combined, washed with saturated sodium chloride solution (150 mL), dried over anhydrous sodium sulfate, filtered, and concentrated under reduced pressure. The residue was purified by silica gel column chromatography (mobile phase: dichloromethane/methanol = 100:1) to give brown solid compound **25-3** (10 g). LCMS (ESI) m/z: 338/340.

### Synthesis of intermediate 25-4

Compound **25-3** (12 g, 35.5 mmol) was dissolved in 1,4-dioxane (20 mL), and triethylamine (7.19 g, 71.0 mmol), 4-dimethylaminopyridine (433 mg, 3.55 mmol) and di-tert-butyl dicarbonate (11.5 g, 53.25 mmol) were added. The above mixture was stirred at room temperature for 1 hour. The reaction solution was concentrated, and the resulting residue was purified by silica gel column chromatography (mobile phase: petroleum ether/ethyl acetate = 20:1) to give white solid compound **25-4** (12 g, yield 77.2%). LCMS (ESI) m/z: 438/440.

### Synthesis of intermediate 25-5

Compound **SM5b** (6 g, 25.64 mmol) and 10% palladium on carbon (2.72 g, 2.56 mmol) were added to tetrahydrofuran (100 mL) in air. After being purged with hydrogen three times, the above system was stirred at room temperature for 16 hours under hydrogen. The reaction solution was filtered, and the filter cake was washed with tetrahydrofuran. The filtrate was collected and concentrated under reduced pressure. The resulting residue was purified by silica gel column chromatography (mobile phase: petroleum ether/ethyl acetate = 1:1) to give yellow solid compound **25-5** (4.45 g, yield 85.1%). LCMS (ESI) m/z: 205.

### Synthesis of intermediate 25-6

Compound **25-5** (4.45 g, 21.81 mmol) was dissolved in tetrahydrofuran (50 mL). The above system was cooled to -15 °C under nitrogen, and a 3M solution of methylmagnesium bromide (36 mL, 109.07 mmol) in tetrahydrofuran was slowly added. After the addition was completed, the system was warmed to room temperature and stirred for another 16 hours. 1M hydrochloric acid (36 mL) was added to the above system, and the mixture was reacted at room temperature for another 3 hours. After the reaction was completed, ethyl acetate was added for extraction (50 mL × 3). The organic phases were combined, washed with saturated sodium chloride solution (50 mL), dried over anhydrous sodium sulfate, filtered, and concentrated under reduced pressure. The resulting residue was purified by silica gel column chromatography (mobile phase: petroleum ether/ethyl acetate = 1:1) to give yellow oily compound **25-6** (510 mg, yield 10.6%). LCMS (ESI) m/z: 222.

### Synthesis of intermediate 25-7

Compound **25-6** (510 mg, 2.31 mmol) was dissolved in tetrahydrofuran (10 mL). The above system was cooled to -15 °C under nitrogen, and a 3M solution of methylmagnesium bromide (3.85 mL, 11.55 mmol) in tetrahydrofuran was slowly added. After the addition was completed, the system was warmed to room temperature and stirred for another 2 hours. Saturated ammonium chloride solution was added to quench the reaction, and the mixture was extracted with ethyl acetate (50 mL × 3). The organic phases were combined, washed with saturated sodium chloride solution (30 mL × 3), dried over anhydrous sodium sulfate, filtered, and concentrated under reduced pressure. The resulting residue was purified by silica gel column chromatography (mobile phase: petroleum ether/ethyl acetate = 1:1) to give yellow solid compound **25-7** (250 mg, yield 45.6%). LCMS (ESI) m/z: 238.

### Synthesis of intermediate 25-8

Compound **25-7** (250 mg, 1.05 mmol), compound **25-4** (508 mg, 1.16 mmol), tris(dibenzylideneacetone)dipalladium(0) (192.1 mg, 0.21 mmol), 4,5-bis(diphenylphosphino)-9,9-dimethylxanthene (243 mg, 0.42 mmol), cesium carbonate (685 mg, 2.10 mmol) and 1,4-dioxane (3 mL) were added to a single-neck flask. The above system was stirred at 60 °C for 16 hours under nitrogen. The reaction was cooled to room temperature, and the reaction solution was concentrated. The resulting residue was purified by silica gel column chromatography (mobile phase: dichloromethane/methanol = 15:1) to give yellow solid compound **25-8** (300 mg, yield 52.2%). LCMS (ESI) m/z: 547/549.

### Synthesis of intermediate 25-9

Compound **25-8** (300 mg, 0.55 mmol) and Eaton's reagent (10 mL) were added to a single-neck flask, and the mixture was stirred at room temperature for 1 hour. The reaction solution was added to ice water (20 mL), and saturated sodium hydroxide solution was added dropwise until the pH of the reaction solution was 8 to 9. The mixture was extracted with ethyl acetate (30 mL × 3). The organic phases were combined, washed with saturated sodium chloride solution (30 mL), dried over anhydrous sodium sulfate, filtered, and concentrated under reduced pressure. The resulting residue was purified by silica gel column chromatography (mobile phase: petroleum ether/ethyl acetate = 1:1) to give yellow solid compound **25-9** (150 mg, yield 63.8%). LCMS (ESI) m/z: 429/431.

### Synthesis of intermediate 25-10

Compound **25-9** (150 mg, 0.35 mmol) and Eaton's reagent (50 mL) were added to a single-neck flask. The above mixture was stirred at 80 °C for 10 hours. The reaction solution was cooled to room temperature and added to ice water (20 mL). Saturated sodium hydroxide solution was added dropwise until the pH of the reaction solution was 8 to 9. The mixture was extracted with ethyl acetate (100 mL × 3). The organic phases were combined, washed with saturated sodium chloride solution (50 mL), dried over anhydrous sodium sulfate, filtered, and concentrated under reduced pressure. The residue was purified by silica gel column chromatography (mobile phase: petroleum ether/ethyl acetate = 3:1) to give yellow solid compound **25-10** (30 mg, yield 20%). LCMS (ESI) m/z: 429/431.

### Synthesis of compound 25

Compound **25-10** (30 mg, 0.07mmol), compound **132-7** (62 mg, 0.14 mmol), tetrakis(triphenylphosphine)palladium(0) (16.2 mg, 0.014 mmol) and N,N-dimethylformamide (1 mL) were added to a single-neck flask. The above system was stirred at 80 °C for 16 hours under nitrogen. The reaction solution was cooled to room temperature, and 10 mL of water was added to the reaction solution. The mixture was extracted with ethyl acetate (20 mL × 3). The organic phases were combined, washed with saturated sodium chloride solution (10 mL), dried over anhydrous sodium sulfate, filtered, and concentrated under reduced pressure. The resulting residue was purified by silica gel column chromatography (mobile phase: dichloromethane/methanol = 10:1) to give a yellow solid, which was then purified by high performance liquid chromatography (chromatography column: Gemini-C18 150 x 21.2 mm, 5 um mobile phase: ACN-H₂O (0.1%FA) gradient: 30-60%) to give yellow solid **compound 25** (10 mg, yield 28.6%). LCMS (ESI) m/z: 501. ¹H NMR (400 MHz, DMSO-*d₆*) δ ppm: 8.71 (s, 1H), 8.53 (s, 1H), 7.53 (s, 1H), 7.37 (d, *J =* 8.7 Hz, 1H), 7.16 (s, 1H), 6.63 (d, *J =* 8.8 Hz, 1H), 4.38 - 4.23 (m, 3H), 3.92 - 3.79 (m, 2H), 3.73 - 3.62 (m, 4H), 3.41 (s, 3H), 3.30 (br s, 4H), 2.08 - 1.88 (m, 3H), 1.87 - 1.76 (m, 1H), 1.56 (d, *J =* 7.3 Hz, 6H).

The following examples were synthesized using the same methods as above:

| Example | Structure | MS:M+ H⁺ | ¹H NMR |
|---|---|---|---|
| 26 | 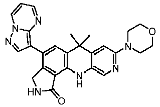 | 468 | ¹H NMR (400 MHz, DMSO-*d₆*) δ ppm: 9.18 (dd, *J =* 7.0, 1.6 Hz, 1H), 8.92 (s, 1H), 8.71 - 8.63 (m, 2H), 8.54 (s, 1H), 8.28 (s, 1H), 8.00 (s, 1H), 7.12 (dd, *J* = 7.0, 4.0 Hz, 1H), 6.94 (s, 1H), 4.59 (s, 2H), 3.77 - 3.69 (m, 4H), 3.46 - 3.41 (m, 4H), 1.64 (s, 6H). |
| 27 | 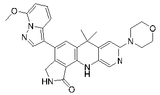 | 497 | ¹H NMR (400 MHz, CD₃OD) δ ppm: 8.15 (s, 1H), 7.87 (s, 1H), 7.65 (s, 1H), 7.36 - 7.30 (m, 1H), 7.27 - 7.24 (m, 1H), 6.92 (s, 1H), 6.42 (d, *J* = 7.3 Hz, 1H), 4.42 (s, 2H), 4.18(s, 3H), 3.83 - 3.79 (m, 4H), 3.38 - 3.31 (m, 4H), 1.66 (s, 6H). |
| 28 | 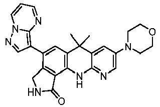 | 468 | ¹H NMR (400 MHz, DMSO-*d₆*) δ ppm: 9.22 - 9.15 (m, 2H), 8.75 - 8.67 (m, 2H), 8.56 (s, 1H), 8.31 (s, 1H), 7.76 (d, *J =* 2.5 Hz, 1H), 7.54 (d, *J* = 2.4 Hz, 1H), 7.15 - 7.12 (m, 1H), 4.61 (s, 2H), 3.81 - 3.70 (m, 4H), 3.16 - 3.04 (m, 4H), 1.65 (s, 6H). |
| 29 | 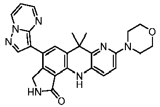 | 468 | ¹H NMR (400 MHz, DMSO-*d₆*) δ ppm: 9.18 (s, 1H), 9.00 - 8.47 (m, 4H), 8.27 (s, 1H), 7.39 (br s, 1H), 7.12 (br s, 1H), 6.69 (br s, 1H), 4.59 (s, 2H), 3.83 -3.70 (m, 4H), 3.30 - 3.25 (m, 4H), 1.64 (s, 6H). |

### Example 132

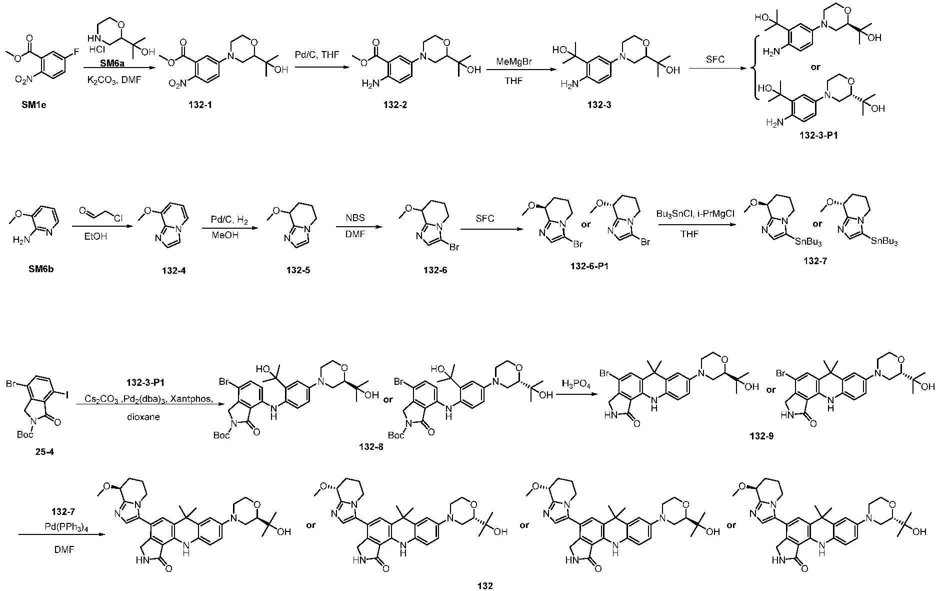

### Synthesis of intermediate 132-1

Compound **SM1e** (1 g), compound **SM6a** (1 g), potassium carbonate (2.08 g) and N,N-dimethylformamide (7 mL) were added to a single-neck flask. The above mixture was stirred at 75 °C for 20 hours. The reaction solution was cooled to room temperature and slowly poured into water (50 mL). The mixture was extracted with ethyl acetate (50 mL × 2). The organic phases were combined, dried over anhydrous sodium sulfate, filtered and concentrated under vacuum to give yellow solid compound **132-1** (1.25 g) as a crude product, which was directly used in the next reaction. LCMS (ESI) m/z: 325.

### Synthesis of intermediate 132-2

Compound **132-1** (1.25 g), tetrahydrofuran (50 mL), water (5 mL) and ammonium chloride (1.03 g) were added to a single-neck flask. The above mixture was heated to 80 °C, and iron powder (1.03 g) was slowly added. The reaction solution was stirred at 80 °C for 3 hours. The reaction solution was cooled to room temperature and filtered, and the filter cake was washed with THF (50 mL). The filtrates were combined, washed with saturated sodium chloride solution (30 mL × 2), dried over anhydrous sodium sulfate, filtered, and concentrated under reduced pressure to give yellow solid compound **132-2** (0.9 g) as a crude product, which was directly used in the next reaction. LCMS (ESI) m/z: 295.

### Synthesis of intermediate 132-3-P1

Compound **132-2** (0.9 g) was dissolved in tetrahydrofuran (10 mL), and methyllithium (30 mL) was slowly added dropwise at 0 °C under nitrogen. After the addition was completed, the mixture was warmed to room temperature and stirred for another 2 hours. The reaction solution was slowly poured into ice water (20 mL) to quench the reaction, and the mixture was extracted with ethyl acetate (50 mL × 2). The organic phases were combined, dried over anhydrous sodium sulfate, filtered, and concentrated. The resulting residue was purified by silica gel column chromatography (mobile phase: petroleum ether/ethyl acetate = 20/1 to 1/1) to give yellow solid compound **132-3** (0.7 g).

**Compound 132-3** was separated by SFC (chromatography column: CHIRALPAK AD-H, 250 mm × 20 mm, 5 µm, mobile phase A: CO₂, mobile phase B: 40% IPA (NH₄OH 0.2%), flow rate: 40 mL/min, column temperature: 40 °C) to give compound **132-3-P1** (RT1: 12.5 min), LCMS (ESI) m/z: 295; and compound **132-3-P2** (RT2: 15.8 min), LCMS (ESI) m/z: 295.

### Synthesis of intermediate 132-4

2-Amino-3-methoxypyridine (20 g, 0.16 mol), chloroacetaldehyde (100 mL) and ethanol (150 mL) were added to a single-neck flask, and the above mixture was stirred at 60 °C for 2 hours. The reaction solution was cooled to room temperature and concentrated. The residue after the concentration was dissolved in ethyl acetate (200 mL), washed with saturated potassium carbonate solution (50 mL × 2), dried over anhydrous sodium sulfate and filtered. The filtrate was concentrated under reduced pressure, and the residue was purified by silica gel column chromatography (mobile phase: petroleum ether/ethyl acetate = 1:1) to give brown oily compound **132-4** (12 g, yield 50.3%). LCMS (ESI) m/z: 149.

### Synthesis of intermediate 132-5

Compound **132-4** (1 g, 6.76 mmol) and 10% palladium on carbon (717 mg, 0.68 mmol) were added to methanol (50 mL) in air. The above system was purged with hydrogen three times and reacted at 60 °C for 24 hours under hydrogen. The reaction solution was cooled to room temperature, and the reaction mixture was filtered. The filtrate was collected and concentrated under reduced pressure to give white solid compound **132-5** (500 mg) as a crude product, which was directly used in the next step. LCMS (ESI) m/z: 153.

### Synthesis of intermediate 132-6-P1

Compound **132-5** (500 mg) and N,N-dimethylformamide (20 mL) were added to a single-neck flask, and N-bromosuccinimide (586 mg, 3.29 mmol) was added in batches. The above mixture was stirred at room temperature for 1 hour. The reaction was quenched by adding water (20 mL), and the mixture was extracted with ethyl acetate (100 mL × 2). The organic phases were combined, washed with saturated sodium chloride solution (50 mL × 2), dried over anhydrous sodium sulfate, filtered, and concentrated under reduced pressure. The residue was purified by silica gel column chromatography (mobile phase: petroleum ether/ethyl acetate = 2:1) to give white solid compound **132-6** (300 mg). LCMS (ESI) m/z: 231/233.

Compound **132-6** was separated by SFC (chromatography column: CHIRALPAK IC, 250 mm (20 mm, 5 µm), mobile phase A: CO₂, mobile phase B: 40% IPA (NH₄OH 0.2%), flow rate: 40 mL/min, column temperature: 40 °C) to give compound **132-6-P1** (RT1: 8.2 min), LCMS (ESI) m/z: 231/233; and compound **132-6-P2** (RT2: 12 min). LCMS (ESI) m/z: 231/233.

### Synthesis of intermediate 132-7

Compound **132-6-P1** (100 mg, 0.86 mmol) and tetrahydrofuran (10 mL) were added to a three-neck flask and cooled to -20 °C under nitrogen. 2 M solution of isopropylmagnesium chloride in tetrahydrofuran (1.3 mL, 2.6 mmol) was added dropwise. The above mixture was stirred at -20 °C for 0.5 hours, and then tributyltin chloride (847 mg, 2.6 mmol) was added dropwise. After the addition was completed, the system was warmed to room temperature and stirred for another 1 hour. Ammonium chloride solution (20 mL) was added to the reaction solution to quench the reaction, and the mixture was extracted with ethyl acetate (50 mL×2). The organic phases were combined, washed with saturated sodium chloride solution (30 mL×2), dried over anhydrous sodium sulfate, filtered, and concentrated under reduced pressure to give brown oily compound **132-7** (300 mg) as a crude product, which was directly used in the next reaction. LCMS (ESI) m/z: 443.

### Synthesis of intermediate 132-8

Compound **25-4** (300 mg, 0.68 mmol), compound **132-3-P1** (201 mg, 0.68 mmol), tris(dibenzylideneacetone)dipalladium(0) (125 mg, 0.14 mmol), 4,5-bis(diphenylphosphino)-9,9-dimethylxanthene (158 mg, 0.27 mmol), cesium carbonate (665 mg, 2.04 mmol) and 1,4-dioxane (10 mL) were added to a single-neck flask, and the mixture was stirred at 60 °C for 16 hours under nitrogen. The reaction was cooled to room temperature, and the reaction mixture was concentrated. The residue was purified by silica gel column chromatography (mobile phase: dichloromethane/methanol = 20:1) to give yellow solid compound **132-8** (200 mg, yield 48.7%). LCMS (ESI) m/z: 604.

### Synthesis of intermediate 132-9

Compound **132-8** (200 mg, 0.33 mmol) was added to phosphoric acid (20 mL), and the mixture was stirred at 35 °C for 3 hours. The reaction system was cooled to room temperature, and sodium hydroxide solution was added dropwise until the pH value of the reaction solution was 10 to 11. The mixture was extracted with ethyl acetate (100 mL × 2). The organic phases were combined, washed with water (50 mL × 2), dried over anhydrous sodium sulfate, filtered, and concentrated under reduced pressure. The residue was purified by silica gel column chromatography (mobile phase: dichloromethane/methanol = 10:1) to give yellow solid compound **132-9** (110 mg, yield 68.3%). LCMS (ESI) m/z: 486.

### Synthesis of compound 132

Compound **132-9** (50 mg, 0.10 mmol), compound **132-7** (88 mg), tetrakis(triphenylphosphine)palladium(0) (11.9 mg, 0.01 mmol) and N,N-dimethylformamide (3 mL) were added to a single-neck flask, and the mixture was stirred at 80 °C for 16 hours under nitrogen. The system was cooled to room temperature, and the reaction mixture was concentrated. The residue was purified by silica gel column chromatography (mobile phase: dichloromethane/methanol = 10:1) to give a crude product, which was purified by high performance liquid chromatography (chromatography column: Xbridge-C18 150 x 19 mm, 5 um mobile phase: ACN-H₂O (0.1%FA) gradient: 5 - 40%) to give yellow solid compound **132** (38 mg, yield 66.2%). LCMS (ESI) m/z: 558. ¹H NMR (400 MHz, DMSO-*d₆*) δ ppm: 8.79 (s, 1H), 8.52 (s, 1H), 8.18 (s, 1H), 7.52 (s, 1H), 7.07 (s, 1H), 6.99 (d, *J* = 2.3 Hz, 1H), 6.85 (d, *J =* 8.6 Hz, 1H), 6.74 - 6.64 (m, 1H), 4.29 - 4.25 (m, 3H), 3.93 (dd, *J =* 10.9, 2.4 Hz, 1H), 3.84 (dd, *J* = 9.0, 4.7 Hz, 2H), 3.60 (d, *J =* 11.3, 2.2 Hz, 1H), 3.48 (d, *J* = 11.5 Hz, 1H), 3.40 (s, 3H), 3.35 (d, *J =* 11.4 Hz, 1H), 3.26 - 3.22 (m, 1H), 2.55 (d, *J =* 11.7, 5.8 Hz, 1H), 2.40 (t, *J =* 11.1 Hz, 1H), 2.09 - 1.99 (m, 2H), 1.94 - 1.76 (m, 2H), 1.56 (d, *J =* 7.2 Hz, 6H), 1.12 (s, 3H), 1.06 (s, 3H).

The synthetic methods of the following examples refer to example **10** and example **132.**

Among them, example **90** (RT1: 7.5 min) and example **91** (RT2: 12.3 min) were obtained by SFC separation of their racemates (chromatography column: chiralpak-OJ-H, 250 mm x 20 mm, 5 µm, mobile phase A: CO₂, mobile phase B: 40% IPA (NH₄OH 0.2%), flow rate: 40 mL/min, column temperature: 40 °C).

Example **93** (RT1: 6.6 min) and example **94** (RT2: 7.48 min) were obtained by SFC separation of their racemates (chromatography column: chiralpak-OJ-H, 250 mm x 20 mm, 5 µm, mobile phase A: CO₂, mobile phase B: 40% MeOH (NH₄OH 0.2%), flow rate: 40 mL/min).

Example **95** (RT1: 7.5 min) and example **96** (RT2: 12.4 min) were obtained by SFC separation of their racemates (chromatography column: chiralpak-OJ-H, 250 mm x 20 mm, 5 µm, mobile phase A: CO₂, mobile phase B: 40% MeOH (NH₄OH 0.2%), flow rate: 40 mL/min).

Example **106** (RT1: 19.1 min) and example **107** (RT2: 26.2 min) were obtained by SFC separation of their racemates (chromatography column: chiralpak-AD-H, mobile phase A: CO₂, mobile phase B: 40% EtOH (NH₄OH 0.2%), flow rate: 40 mL/min).

Example **120** (RT1: 6.29 min) and example **121** (RT2: 7.9 min) were obtained by SFC separation of their racemates (chromatography column: CHIRALPAK AS-H, 250 mm ' 20 mm, 5 µm, mobile phase A: CO₂, mobile phase: 40% IPA (NH₄OH 0.2%), flow rate: 40 mL/min, column temperature: 40 °C).

Example **125** (RT1: 6.7 min) and example **126** (RT2: 7.59 min) were obtained by SFC separation of their racemates (chromatography column: chiralpak-OJ-H, 250 mm x 20 mm, 5 µm, mobile phase A: CO₂, mobile phase B: 40% MeOH (NH₄OH 0.2%), flow rate: 40 mL/min).

Example **127** (RT1: 6.81 min) and example **128** (RT2: 8.72 min) were obtained by SFC separation of their racemates (chromatography column: chiralpak-OJ-H, 250 mm x 20 mm, 5 µm, mobile phase A: CO₂, mobile phase B: 40% MeOH (NH₄OH 0.2%), flow rate: 40 mL/min).

Example **129** (RT1: 15.3 min) and example **130** (RT2: 18.4 min) were obtained by SFC separation of their racemates (chromatography column: chiralpak-AD-H, 250 mm x 20 mm, 5 µm, mobile phase A: CO₂, mobile phase B: 40% IPA (NH₄OH 0.2%), flow rate: 40 mL/min).

Example **136** (RT1: 3.08 min) and example **137** (RT2: 4.6 min) were obtained by SFC separation of their racemates (chromatography column: chiralpak-OJ-H, 250 mm x 20 mm, 5 µm, mobile phase A: CO₂, mobile phase B: 40% IPA (NH₄OH 0.2%), flow rate: 40 mL/min).

Example **138** (RT1: 18.64 min) and example **139** (RT2: 21.86 min) were obtained by SFC separation of their racemates (chromatography column: chiralpak-AD-H, 250 mm x 20 mm, 5 µm, mobile phase A: CO₂, mobile phase B: 40% MeOH (NH₄OH 0.2%), flow rate: 40 mL/min).

| Example | Structure | MS:M+ H⁺ | ¹H NMR |
|---|---|---|---|
| 31 | 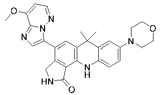 | 497 | ¹H NMR (400 MHz, DMSO-*d₆*) δ ppm: 8.93 (s, 1H), 8.63 (s, 1H), 8.46 (d, *J =* 5.3 Hz, 2H), 8.15 (s, 1H), 7.88 (s, 1H), 7.06 (s, 1H), 6.91 (d, *J* = 8.3 Hz, 1H), 6.77 (d, *J* = 6.0 Hz, 2H), 4.44 (s, 2H), 4.08 (s, 3H), 3.75 (s, 4H), 3.04 (s, 4H), 1.62 (s, 6H). |
| 32 | 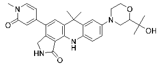 | 515 | ¹HNMR (400MHz, CD₃OD) δ ppm: 7.61 (d, *J* = 7.8 Hz, 2H), 7.02 (d, *J* = 2.3 Hz, 1H), 6.76 - 6.72 (m, 2H), 6.56 - 6.50 (m, 2H), 4.47 (s, 2H), 3.95 (dd, *J =* 11.1, 2.3 Hz, 1H), 3.68 (td, *J* = 11.4, 2.5 Hz, 1H), 3.51 (s, 3H), 3.43 (d, *J* = 11.4 Hz, 1H), 3.34 (dd, *J* = 10.5, 2.2 Hz, 1H), 3.24 - 3.23 (m, 1H), 2.64 (td, *J* = 11.6, 3.2 Hz, 1H), 2.48 (t, *J =* 11.0 Hz, 1H), 1.56 (s, 6H), 1.16 (s, 3H), 1.13 (s, 3H). |
| 33 | 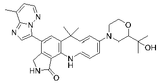 | 539 | ¹H NMR (400 MHz, DMSO-*d₆*) δ ppm: 8.91 (s, 1H), 8.59 (s, 1H), 8.44 (d, *J =* 4.6 Hz, 1H), 8.12 (s, 1H), 7.95 (s, 1H), 7.12 - 7.07 (m, 1H), 7.01 (s, 1H), 6.88 (d, *J =* 8.6 Hz, 1H), 6.75 (d, *J* = 8.4 Hz, 1H), 4.43 - 4.38 (m, 3H), 3.94 (d, *J =* 8.6 Hz, 1H), 3.60 (dd, *J* = 11.5, 9.1 Hz, 1H), 3.50 (d, *J* = 10.7 Hz, 1H), 3.36 (d, *J =* 11.6 Hz, 1H), 3.25 (s, 1H), 2.58 (s, 3H), 2.55 (d, *J* = 8.7 Hz, 1H), 2.40 (d, *J =* 10.7 Hz, 1H), 1.59 (s, 6H), 1.13 (s, 3H), 1.07 (s, 3H). |
| 34 | 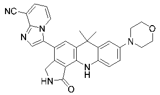 | 491 | ¹H NMR (400 MHz, DMSO-*d₆*) δ ppm: 8.94 (s, 1H), 8.61 (s, 1H), 8.54 (d, *J* = 6.8 Hz, 1H), 8.02 (d, *J* = 7.0 Hz, 1H), 7.97 (s, 1H), 7.77 (s, 1H), 7.10 (t, *J* = 7.0 Hz, 1H), 7.06 (s, 1H), 6.93 (d, *J* = 8.6 Hz, 1H), 6.78 (d, *J* = 8.6 Hz, 1H), 4.31 (s, 2H), 3.85 - 3.67 (m, 4H), 3.15 - 3.01 (m, 4H), 1.62 (s, 6H). |
| 35 | 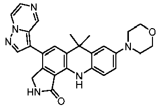 | 467 | ¹H NMR (400 MHz, DMSO-*d₆*) δ ppm: 9.20 (s, 1H), 8.83 (d, *J* = 4.6 Hz, 1H), 8.64 (s, 1H), 8.48 (s, 1H), 7.96 (d, *J* = 4.5 Hz, 1H), 7.81 (s, 1H), 7.26-6.91 (m, 4H), 4.49 (s, 2H), 3.76 (s, 4H), 3.05 (s, 4H), 1.65 (s, 6H). |
| 36 | 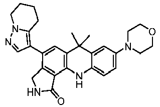 | 470 | ¹H NMR (400 MHz, DMSO-*d₆*) δ ppm: δ 8.71 (s, 1H), 8.50 (s, 1H), 7.62 (s, 1H), 7.38 (s, 1H), 6.99 (d, *J* = 2.5 Hz, 1H), 6.80 (d, *J* = 8.6 Hz, 1H), 6.71 (dd, *J* = 8.7, 2.6 Hz, 1H), 4.34 (s, 2H), 4.07 (t, *J* = 6.1 Hz, 2H), 3.73 - 3.65 (m, 4H), 3.00 - 2.95 (m, 4H), 2.82 (t, *J* = 6.3 Hz, 2H), 2.01 - 1.93 (m, 2H), 1.78 (dd, *J =* 7.5, 3.8 Hz, 2H), 1.54 (s, 6H). |
| 37 | 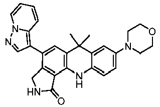 | 466 | ¹H NMR (400 MHz, DMSO-*d₆*) δ ppm: 8.82 (s, 1H), 8.74 (d, *J* = 7.0 Hz, 1H), 8.59 (s, 1H), 8.30 (s, 1H), 7.72 (d, *J* = 9.0 Hz, 1H), 7.65 (s, 1H), 7.35 - 7.27 (m, 1H), 7.06 (d, *J* = 2.4 Hz, 1H), 6.96 (t, *J* = 6.4 Hz, 1H), 6.88 (d, *J* = 8.6 Hz, 1H), 6.76 (dd, *J* = 8.6, 2.5 Hz, 1H), 4.44 (s, 2H), 3.79 - 3.69 (m, 4H), 3.09 - 2.94 (m, 4H), 1.63 (s, 6H). |
| 38 | 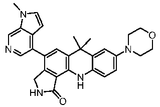 | 480 | ¹H NMR (400 MHz, DMSO-*d₆*) δ ppm: 8.86 (s, 1H), 8.79 (s, 1H), 8.51 (s, 1H), 8.33 (s, 1H), 8.22 (s, 1H), 7.64 (s, 1H), 7.59 (d, *J* = 2.9 Hz, 1H), 7.02 (d, *J* = 2.1 Hz, 1H), 6.86 (d, *J* = 8.6 Hz, 1H), 6.73 (dd, *J* = 8.7, 2.4 Hz, 1H), 6.36 (d, *J* = 2.7 Hz, 1H), 4.32 (s, 2H), 3.91 (s, 3H), 3.74 - 3.68 (m, 4H), 3.04 - 2.96 (m, 4H), 1.57 (s, 6H). |
| 39 | 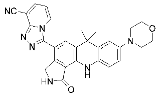 | 492 | ¹H NMR (400 MHz, CD₃OD) δ ppm: 8.50 (d, *J* = 6.7 Hz, 1H), 7.99 (d, *J* = 6.2 Hz, 1H), 7.87 (s, 1H), 7.10 - 7.07 (m, 2H), 6.79 - 6.81 (m, 2H), 4.46 (s, 2H), 3.85 - 3.69 (m, 4H), 3.08 - 2.94 (m, 4H), 1.60 (s, 6H). |
| 40 | 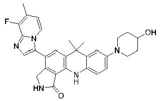 | 512 | ¹H NMR (400 MHz, CD₃OD) δ ppm: 7.93 (d, *J* = 6.9 Hz, 1H), 7.70 (s, 1H), 7.66 (s, 1H), 7.18 (s, 1H), 6.94 - 6.92 (m, 1H), 6.88 - 6.85 (m, 2H), 4.32 (s, 2H), 3.78 - 3.76 (m, 1H), 3.46 - 3.43 (m, 2H), 2.89 - 2.84 (m, 2H), 2.41 (d, *J* = 2.3 Hz, 3H), 2.06 - 1.98 (m, 2H), 1.78 - 1.75 (m, 2H), 1.67 (s, 6H). |
| 41 | 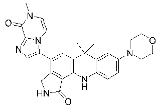 | 497 | ¹H NMR (400 MHz, DMSO-*d₆*) δ ppm: 8.89 (s, 1H), 8.59 (s, 1H), 7.65 (s, 1H), 7.59 (s, 1H), 7.18 (d, *J* = 5.9 Hz, 1H), 7.11 (d, *J* = 5.9 Hz, 1H), 7.01 (d, *J* = 2.5 Hz, 1H), 6.89 (d, *J* = 8.6 Hz, 1H), 6.73 (dd, *J* = 8.7, 2.5 Hz, 1H), 4.30 (s, 2H), 3.72 - 3.66 (m, 4H), 3.42 (d, *J* = 5.1 Hz, 3H), 3.03 - 2.94 (m, 4H), 1.57 (s, 6H). |
| 42 | 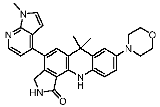 | 480 | ¹H NMR (400 MHz, DMSO-*d₆*) δ ppm: 8.97 (s, 1H), 8.60 (s, 1H), 8.30 (d, *J* = 4.9 Hz, 1H), 7.76 (s, 1H), 7.59 (d, *J* = 3.4 Hz, 1H), 7.25 (d, *J* = 4.9 Hz, 1H), 7.07 (d, *J* = 2.2 Hz, 1H), 6.91 (d, *J* = 8.6 Hz, 1H), 6.78 (dd, *J* = 8.6, 2.4 Hz, 1H), 6.42 (d, *J* = 3.5 Hz, 1H), 4.43 (s, 2H), 3.87 (s, 3H), 3.79 - 3.70 (m, 4H), 3.07 - 3.00 (m, 4H), 1.62 (s, 6H). |
| 43 | 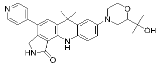 | 485 | ¹H NMR (400 MHz, DMSO-*d₆*) δ ppm: 9.06 (s, 1H), 8.71 (s, 1H), 8.65 - 8.59 (m, 2H), 7.80 (s, 1H), 7.75 - 7.65 (m, 2H), 7.06 (s, 1H), 6.92 (s, 1H), 6.80 (s, 1H), 4.60 (s, 2H), 4.48 (s, 1H), 3.98 (d, *J =* 9.9 Hz, 1H), 3.73 - 3.60 (m, 1H), 3.54 (d, *J =* 8.3 Hz, 1H), 3.41 (d, *J* = 7.2 Hz, 1H), 3.30 (d, *J* = 7.2 Hz, 1H), 2.61 (s, 1H), 2.47 - 2.39 (m, 1H), 1.65 (s, 6H), 1.14 (d, *J* = 24.2 Hz, 6H). |
| 44 | 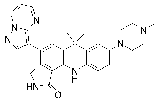 | 480 | ¹H NMR (400 MHz, DMSO-*d₆*) δ ppm: 9.18 (dd, *J =* 7.0, 1.7 Hz, 1H), 8.84 (s, 1H), 8.71 - 8.59 (m, 2H), 8.53 (s, 1H), 8.32 (s, 1H), 8.28 (s, 1H), 7.12 (dd, *J =* 7.0, 4.0 Hz, 1H), 7.04 (d, *J* = 2.3 Hz, 1H), 6.85 (d, *J =* 8.6 Hz, 1H), 6.75 (dd, *J =* 8.6, 2.4 Hz, 1H), 4.58 (s, 2H), 3.10 - 3.01 (m, 4H), 2.48 - 2.41 (m, 4H), 2.23 (s, 3H), 1.63 (s, 6H). |
| 45 | 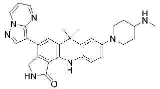 | 494 | ¹H NMR (400 MHz, DMSO-*d₆*) δ ppm: 9.14 (dd, *J =* 7.0, 1.8 Hz, 1H), 8.81 (s, 1H), 8.63 (dd, *J =* 4.0, 1.7 Hz, 1H), 8.60 (s, 1H), 8.49 (s, 1H), 8.24 (s, 1H), 7.08 (dd, *J* = 7.0, 4.0 Hz, 1H), 7.02 (d, *J* = 2.4 Hz, 1H), 6.83 (d, *J =* 8.6 Hz, 1H), 6.74 (dd, *J* = 8.6, 2.5 Hz, 1H), 4.54 (s, 2H), 3.55 (d, *J* = 12.5 Hz, 2H), 2.91 - 2.78 (m, 1H), 2.66 - 2.56 (m, 2H), 2.45 (s, 3H), 1.98 (d, *J =* 8.1 Hz, 2H), 1.59 (s, 6H), 1.55 - 1.46 (m, 2H). |
| 46 | 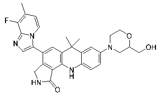 | 528 | ¹HNMR (400MHz, CD₃OD) δ ppm: 8.17 - 8.04 (m, 2H), 7.72 (s, 1H), 7.37 (s, 1H), 7.24 (t, *J* = 6.6 Hz, 1H), 7.11 (s, 1H), 6.95 (s, 1H), 4.26 (s, 2H), 4.04 (s, 1H), 3.87 - 3.77 (m, 2H), 3.65 - 3.29 (m, 5H), 3.07 - 2.91 (m, 1H), 2.45 (d, *J =* 2.2 Hz, 3H), 1.59 (s, 6H). |
| 47 | 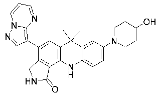 | 481 | ¹HNMR (400MHz, CD₃OD) δ ppm: 9.14 (dd, *J =* 7.0, 1.6 Hz, 1H), 8.79 (s, 1H), 8.63 (dd, *J =* 3.9, 1.6 Hz, 1H), 8.58 (s, 1H), 8.49 (s, 1H), 8.24 (s, 1H), 7.08 (dd, *J =* 7.0, 4.0 Hz, 1H), 7.00 (d, *J =* 2.2 Hz, 1H), 6.79 (d, *J =* 8.6 Hz, 1H), 6.72 (dd, *J =* 8.6, 2.3 Hz, 1H), 4.62 (s, 1H), 4.54 (s, 2H), 3.58 - 3.48 (m, 1H), 2.73 - 2.64 (m, 2H), 2.50 -(m, 2H), 1.85 - 1.76 (m, 2H), 1.58 (s, 6H), 1.53 - 1.43 (m, 2H). |
| 48 | 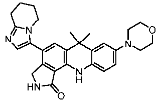 | 470 | ¹H NMR (400 MHz, DMSO-*d₆*) δ ppm: 8.80 (s, 1H), 8.54 (s, 1H), 7.52 (s, 1H), 7.04 (d, *J =* 2.4 Hz, 1H), 7.00 (s, 1H), 6.87 (d, *J* = 8.6 Hz, 1H), 6.75 (dd, *J* = 8.6, 2.5 Hz, 1H), 4.32 (s, 2H), 3.88 (s, 2H), 3.78 - 3.70 (m, 4H), 3.08 - 2.97 (m, 4H), 2.80 (s, 2H), 1.87 (s, 4H), 1.60 (s, 6H). |
| 49 | 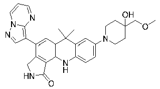 | 525 | ¹H NMR (400 MHz, DMSO-*d₆*) δ ppm: 9.14 (d, *J* = 6.9 Hz, 1H), 8.79 (s, 1H), 8.64 (s, 1H), 8.58 (s, 1H), 8.49 (s, 1H), 8.36 (s, 1H), 8.25 (s, 1H), 7.08 (s, 1H), 7.00 (s, 1H), 6.81 - 6.72 (m, 2H), 4.54 (s, 2H), 4.32 (s, 1H), 3.25 (s, 3H), 3.20 (d, *J =* 13.1 Hz, 2H), 3.15 (s, 2H), 2.92 (t, *J* = 10.2 Hz, 2H), 1.70 - 1.61 (m, 2H), 1.59 (s, 6H), 1.46 (d, *J =* 12.4 Hz, 2H). |
| 50 | 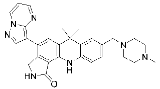 | 494 | ¹H NMR (400 MHz, DMSO-*d₆*) δ ppm: 9.18 (dd, *J* = 7.0, 1.7 Hz, 1H), 9.00 (s, 1H), 8.68 (d, *J* = 2.4 Hz, 2H), 8.54 (s, 1H), 8.33 (s, 1H), 8.32 (s, 1H), 7.33 (s, 1H), 7.12 (dd, *J* = 7.1, 4.0 Hz, 1H), 7.03 (d, *J* = 8.0 Hz, 1H), 6.90 (d, *J* = 8.2 Hz, 1H), 4.60 (s, 2H), 3.40 (s, 2H), 2.46 - 2.17 (m, 8H), 2.14 (s, 3H), 1.62 (s, 6H). |
| 51 | 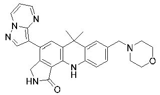 | 481 | ¹H NMR (400 MHz, DMSO-*d₆*) δ ppm: 9.18 (dd, *J* = 7.0, 1.7 Hz, 1H), 9.01 (s, 1H), 8.72 - 8.65 (m, 2H), 8.54 (s, 1H), 8.32 (s, 1H), 7.35 (s, 1H), 7.12 (dd, *J* = 7.0, 4.0Hz, 1H), 7.05 (d, *J* = 8.1 Hz, 1H), 6.91 (d, *J* = 8.1 Hz, 1H), 4.60 (s, 2H), 3.57 (s, 4H), 3.41 (s, 2H), 2.35 (s, 4H), 1.62 (s, 6H). |
| 52 | 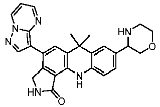 | 467 | ¹H NMR (400 MHz, DMSO-*d₆*) δ ppm: 9.19 (dd, *J* = 7.0, 1.7 Hz, 1H), 9.00 (s, 1H), 8.68 (dd, *J* = 4.1, 1.7 Hz, 2H), 8.54 (s, 1H), 8.32 (s, 1H), 8.29 (s, 1H), 7.49 (s, 1H), 7.19 - 7.09 (m, 2H), 6.91 (d, *J =* 8.1 Hz, 1H), 4.60 (s, 2H), 4.22 (t, *J =* 6.5 Hz, 1H), 3.73 - 3.76 (m, 4H), 2.89 (d, *J =* 5.4 Hz, 2H), 1.63 (d, *J* = 1.9 Hz, 6H). |
| 53 | 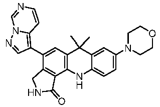 | 467 | ¹H NMR (400 MHz, DMSO-*d₆*) δ ppm: 9.55 (d, *J* = 1.4 Hz, 1H), 8.81 (s, 1H), 8.60 (s, 1H), 8.50 (s, 1H), 7.88 (d, *J* = 6.4 Hz, 1H), 7.69 (dd, *J* = 6.4, 1.4 Hz, 1H), 7.61 (s, 1H), 7.02 (d, *J =* 2.5 Hz, 1H), 6.85 (d, *J* = 8.6 Hz, 1H), 6.72 (dd, *J* = 8.7, 2.5 Hz, 1H), 4.42 (s, 2H), 3.72 - 3.69 (m, 4H), 3.01 - 2.97 (m, 4H), 1.59 (s, 6H). |
| 54 | 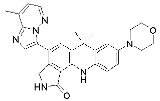 | 481 | ¹H NMR (400 MHz, DMSO-*d₆*) δ ppm: 8.94 (s, 1H), 8.63 (s, 1H), 8.48 (d, *J* = 4.6 Hz, 1H), 8.16 (s, 1H), 7.99 (s, 1H), 7.15 - 7.11 (m, 1H), 7.06 (d, *J* = 2.3 Hz, 1H), 6.91 (d, *J* = 8.6 Hz, 1H), 6.77 (dd, *J* = 8.6, 2.5 Hz, 1H), 4.46 (s, 2H), 3.79 - 3.70 (m, 4H), 3.09 - 2.99 (m, 4H), 2.62 (s, 3H), 1.62 (s, 6H). |
| 55 | 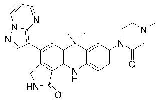 | 494 | ¹H NMR (400 MHz, DMSO-*d₆*) δ ppm: 9.20 (dd, *J* = 7.0, 1.6 Hz, 1H), 9.10 (s, 1H), 8.73 (s, 1H), 8.68 (dd, *J* = 4.0, 1.6 Hz, 1H), 8.55 (s, 1H), 8.34 (s, 1H), 7.38 (s, 1H), 7.13 (dd, *J* = 7.0, 4.0 Hz, 1H), 7.03 (s, 2H), 4.61 (s, 2H), 3.73 (s, 2H), 3.05 - 2.95 (m, 2H), 2.50 - 2.47 (m, 5H), 1.63 (s, 6H). |
| 56 | 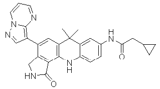 | 479 | ¹H NMR (400 MHz, DMSO-*d₆*) δ ppm: 9.67 (s, 1H), 9.20 - 9.18 (m, 1H), 8.97 (s, 1H), 8.69 - 8.67 (m, 2H), 8.54 (s, 1H), 8.33 (s, 1H), 7.72 (d, *J* = 1.9 Hz, 1H), 7.40 - 7.37 (m, 1H), 7.14 - 7.11 (m, 1H), 6.92 (d, *J* = 8.5 Hz, 1H), 4.60 (s, 2H), 2.18 (d, *J* = 7.0 Hz, 2H), 1.61 (s, 6H), 1.12 - 0.99 (m, 1H), 0.53 - 0.44 (m, 2H), 0.21 - 0.18 (m, 2H). |
| 57 | 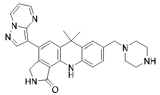 | 480 | ¹H NMR (400 MHz, CD₃OD) δ ppm: 8.85 (d, *J* = 7.2 Hz, 1H), 8.53 (s, 1H), 8.43 (s, 1H), 8.29 (s, 1H), 8.18 (s, 1H), 7.32 (s, 1H), 7.02 (d, *J* = 7.8 Hz, 1H), 6.97 (dd, *J* = 7.1, 3.9 Hz, 1H), 6.76 (d, *J* = 8.2 Hz, 1H), 4.53 (s, 2H), 3.49 (s, 2H), 3.05 (d, *J* = 4.9 Hz, 4H), 2.56 (s, 4H), 1.60 (s, 6H). |
| 58 | 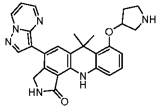 | 467 | ¹H NMR (400 MHz, DMSO-*d₆*) δ ppm: 10.42 (s, 1H), 9.17 (dd, *J* = 7.1, 1.7 Hz, 1H), 9.06 (s, 1H), 8.94 (s, 1H), 8.61 (dd, *J =* 4.0, 1.7 Hz, 1H), 8.50 (s, 1H), 8.40 (s, 1H), 7.94 (d, *J =* 8.6 Hz, 1H), 7.16 - 7.06 (m, 3H), 6.63 (d, *J* = 8.2 Hz, 1H), 5.62 (s, 1H), 5.13 (s, 1H), 4.55 (s, 2H), 3.58 - 3.45 (m, 2H), 3.33 - 3.27 (m, 2H), 2.23 - 2.18 (m, 2H), 1.59 (d, *J* = 3.3 Hz, 6H). |
| 59 | 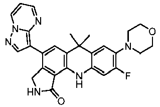 | 485 | ¹H NMR (400 MHz, DMSO-*d₆*) δ ppm: 9.18 (dd, *J* = 7.0, 1.7 Hz, 1H), 8.97 (s, 1H), 8.68 (dd, *J* = 4.0, 1.6 Hz, 2H), 8.54 (s, 1H), 8.29 (s, 1H), 7.12 (dd, *J* = 7.0, 4.0 Hz, 1H), 7.08 (d, *J =* 9.2 Hz, 1H), 6.99 (d, *J* = 13.6 Hz, 1H), 4.59 (s, 2H), 3.76 - 3.71 (m, 4H), 2.98 - 2.93 (m, 4H), 1.63 (s, 6H). |
| 60 | 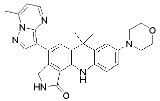 | 481 | ¹H NMR (400 MHz, DMSO-*d₆*) δ ppm: δ 8.96 (s, 1H), 8.67 (s, 1H), 8.59 (d, *J* = 4.2 Hz, 1H), 8.55 (s, 1H), 8.31 (s, 1H), 7.24 (s, 1H), 7.10 (d, *J* = 4.1 Hz, 1H), 6.96 (s, 2H), 4.59 (s, 2H), 3.81 (br s, 4H), 3.20 (br s, 4H), 2.78 (s, 3H), 1.64 (s, 6H). |
| 61 | 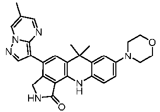 | 481 | ¹H NMR (400 MHz, DMSO-*d₆*) δ ppm: 8.98 (d, *J* = 0.6 Hz, 1H), 8.80 (s, 1H), 8.61 - 8.53 (m, 2H), 8.38 (s, 1H), 8.25 (s, 1H), 7.01 (d, *J* = 2.4 Hz, 1H), 6.83 (d, *J* = 8.6 Hz, 1H), 6.72 (dd, *J* = 8.6, 2.5 Hz, 1H), 4.53 (s, 2H), 3.74 - 3.68 (m, 4H), 3.03 - 2.94 (m, 4H), 2.32 (s, 3H), 1.59 (s, 6H). |
| 62 | 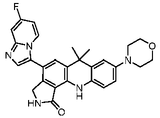 | 484 | ¹H NMR (400 MHz, DMSO-*d₆*) δ ppm: 8.87 (s, 1H), 8.56 (s, 1H), 8.25 (dd, *J =* 7.6, 5.8 Hz, 1H), 7.73 (s, 1H), 7.69 (s, 1H), 7.48 (dd, *J =* 10.1, 2.6 Hz, 1H), 7.02 (d, *J =* 2.5 Hz, 1H), 6.95 (dd, *J* = 7.5, 2.7 Hz, 1H), 6.88 (d, *J* = 8.6 Hz, 1H), 6.73 (dd, *J* = 8.7, 2.6 Hz, 1H), 4.26 (s, 2H), 3.72 - 3.67 (m, 4H), 3.02 - 2.97 (m, 4H), 1.58 (s, 6H). |
| 63 | 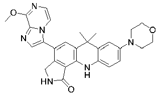 | 497 | ¹H NMR (400 MHz, DMSO-*d₆*) δ ppm: 8.90 (s, 1H), 8.58 (s, 1H), 8.10 (s, 1H), 7.88 - 7.82 (m, 2H), 7.68 (s, 1H), 7.42 (d, *J* = 4.8 Hz, 1H), 7.02 (d, *J* = 2.5 Hz, 1H), 6.89 (d, *J* = 8.6 Hz, 1H), 6.73 (dd, *J =* 8.7, 2.6 Hz, 1H), 4.29 (s, 2H), 4.03 (s, 3H), 3.72 - 3.68 (m, 4H), 3.02 - 2.98 (m, 4H), 1.58 (s, 6H). |
| 64 | 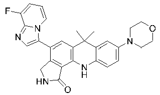 | 484 | ¹H NMR (400 MHz, DMSO-*d₆*) δ ppm: 8.93 (s, 1H), 8.61 (s, 1H), 8.11 (d, *J* = 6.8 Hz, 1H), 7.85 (s, 1H), 7.74 (s, 1H), 7.20 (dd, *J* = 11.0, 7.6 Hz, 1H), 7.05 (s, 1H), 6.97 - 6.88 (m, 2H), 6.80 - 6.75 (m, 1H), 4.31 (s, 2H), 3.83 - 3.70 (m, 4H), 3.10 - 2.98 (m, 4H), 1.62 (s, 6H). |
| 65 | 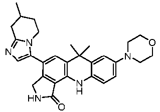 | 484 | ¹H NMR (400 MHz, DMSO-*d₆*) δ ppm: 8.81 (s, 1H), 8.55 (s, 1H), 8.14 (s, 1H), 7.54 (s, 1H), 7.04 (s, 2H), 6.87 (d, *J* = 8.6 Hz, 1H), 6.75 (dd, *J* = 8.6, 2.5 Hz, 1H), 4.43 (d, *J* = 17.9 Hz, 1H), 4.20 (d, *J* = 17.9 Hz, 1H), 3.99 (d, *J* = 4.3 Hz, 1H), 3.81 (d, *J* = 10.1 Hz, 1H), 3.76 - 3.68 (m, 4H), 3.07 - 2.99 (m, 4H), 2.94 (dd, *J* = 16.5, 4.9 Hz, 1H), 2.37 (dd, *J* = 16.6, 10.7 Hz, 1H), 2.05 - 1.95 (m, 2H), 1.60 - 1.50 (m, 7H), 1.09 (d, *J* = 6.5 Hz, 3H). |
| 66 | 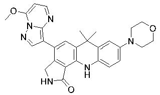 | 497 | ¹H NMR (400 MHz, DMSO-*d₆*) δ ppm: 8.84 (s, 1H), 8.62 (s, 1H), 8.58 (d, *J* = 5.0 Hz, 1H), 8.47 (s, 1H), 8.37 (s, 1H), 8.30 (s, 1H), 7.05 (d, *J* = 2.3 Hz, 1H), 6.87 (d, *J* = 8.6 Hz, 1H), 6.77 - 6.75 (m, 1H), 6.71 (d, *J* = 5.1 Hz, 1H), 4.57 (s, 2H), 4.24 (s, 3H), 3.76 - 3.73 (m, 4H), 3.05 - 3.02 (m, 4H), 1.63 (s, 6H). |
| 67 | 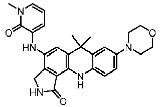 | 472 | ¹H NMR (400 MHz, DMSO-*d₆*) δ ppm: 8.60 (s, 1H), 8.42 (s, 1H), 7.37 (s, 1H), 7.20 (s, 1H), 7.01 - 6.96 (m, 2H), 6.82 (d, *J* = 8.6 Hz, 1H), 6.72 - 6.69 (m, 1H), 6.30 - 6.27 (m, 1H), 6.07 - 6.03(m, 1H), 4.14 (s, 2H), 3.71 - 3.68 (m, 4H), 3.47 (s, 3H), 2.99 - 2.96 (m, 4H), 1.50 (s, 6H). |
| 68 | 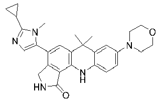 | 470 | ¹H NMR (400 MHz, DMSO-*d₆*) δ ppm: 8.82 (s, 1H), 8.55 (s, 1H), 8.17 (s, 1H), 7.49 (s, 1H), 7.04 (d, *J* = 2.3 Hz, 1H), 6.89 - 6.87 (m, 2H), 6.77 - 6.74 (m, 1H), 4.29 (s, 2H), 3.82 - 3.65 (m, 4H), 3.57 (s, 3H), 3.14 - 2.95 (m, 4H), 2.01 - 1.98 (m, 1H), 1.60 (s, 6H), 0.97 - 0.78 (m, 4H). |
| 69 | 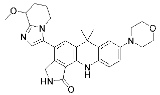 | 500 | ¹H NMR (400 MHz, DMSO-*d₆*) δ ppm: 8.83 (s, 1H), 8.56 (s, 1H), 8.14 (s, 1H), 7.57 (s, 1H), 7.14 (s, 1H), 7.04 (d, *J* = 2.4 Hz, 1H), 6.88 (d, *J* = 8.6 Hz, 1H), 6.75 (dd, *J* = 8.6, 2.5 Hz, 1H), 4.44 - 4.25 (m, 3H), 3.90 (t, *J* = 11.6 Hz, 2H), 3.79 - 3.69 (m, 4H), 3.45 (s, 3H), 3.06 - 2.99 (m, 4H), 2.14 - 2.01 (m, 2H), 1.97 - 1.81 (m, 2H), 1.60 (d, J = 8.3 Hz, 6H). |
| 70 | 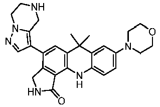 | 471 | ¹H NMR (400 MHz, DMSO-*d₆*) δ ppm: 8.78 (s, 1H), 8.62 (s, 1H), 7.80 (s, 1H), 7.32 (s, 1H), 7.05 (d, *J* = 1.7 Hz, 1H), 6.86 (d, *J* = 8.6 Hz, 1H), 6.75 (dd, *J* = 8.6, 2.0 Hz, 1H), 4.40 (s, 4H), 4.29 (s, 2H), 3.77 - 3.70 (m, 4H), 3.49 (s, 2H), 3.09 - 2.97 (m, 4H), 1.60 (s, 6H). |
| 71 | 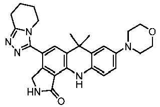 | 471 | ¹H NMR (400 MHz, DMSO-*d₆*) δ ppm: 8.93 (s, 1H), 8.59 (s, 1H), 7.75 (s, 1H), 7.06 (d, *J* = 2.3 Hz, 1H), 6.91 (d, *J* = 8.6 Hz, 1H), 6.77 (dd, *J =* 8.7, 2.5 Hz, 1H), 4.44 (s, 2H), 4.10 (t, *J* = 5.3 Hz, 2H), 3.82 - 3.67 (m, 4H), 3.09 - 2.99 (m, 4H), 2.91 (t, *J =* 5.9 Hz, 2H), 1.90 (br s, 4H), 1.62 (s, 6H). |
| 72 | 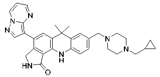 | 534 | ¹H NMR (400 MHz, CD₃OD) δ ppm: 8.92 (dd, *J* = 7.1, 1.6 Hz, 1H), 8.59 (dd, *J* = 4.0, 1.7 Hz, 1H), 8.36 (s, 1H), 8.26 (s, 1H), 7.41 (d, *J =* 1.5 Hz, 1H), 7.09 (dd, *J* = 8.1, 1.6 Hz, 1H), 7.04 (dd, *J* = 7.1, 4.0 Hz, 1H), 6.82 (d, *J* = 8.0 Hz, 1H), 4.60 (s, 2H), 3.55 (s, 2H), 3.14 - 2.42 (m, 8H), 2.38 (d, *J* = 6.7 Hz, 2H), 1.66 (s, 6H), 0.91 - 0.86 (m, 1H), 0.55 (q, *J* = 5.6 Hz, 2H), 0.16 (q, *J =* 5.6 Hz, 2H). |
| 73 | 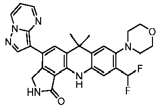 | 517 | ¹H NMR (400 MHz, CD₃OD) δ ppm: 8.94 - 8.91 (m, 1H), 8.61 - 8.59 (m, 1H), 8.37 (s, 1H), 8.27 (s, 1H), 7.42 (s, 1H), 7.10 (d, *J* = 6.9 Hz, 2H), 7.04 (dd, *J* = 7.2, 4.0 Hz, 1H), 4.61 (s, 2H), 3.83 - 3.80 (m, 4H), 2.92 - 2.89 (m, 4H), 1.68 (s, 6H). |
| 74 | 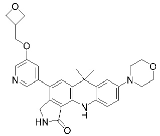 | 513 | ¹HNMR (400 MHz, CD₃OD) δ ppm: 8.28 (s, 1H), 8.23 (s, 1H), 7.62 - 7.60(m, 1H), 7.56 (s, 1H), 7.09 (s, 1H), 6.81 - 6.78 (m, 2H), 4.90 - 4.87 (m, 2H), 4.62 - 4.59(m, 2H), 4.52(s, 2H), 4.36 - 4.34 (m, 2H), 3.85 - 3.80 (m, 4H), 3.50 - 3.46 (m, 1H), 3.05 - 3.02(m, 4H), 1.64 (s, 6H). |
| 75 | 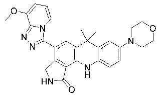 | 497 | ¹H NMR (400 MHz, DMSO-*d₆*) δ ppm: δ 9.01 (s, 1H), 8.63 (s, 1H), 8.14 (s, 1H), 7.97 (d, *J* = 6.9 Hz, 1H), 7.91 (s, 1H), 7.07 (d, *J* = 2.4 Hz, 1H), 7.00 - 6.93 (m, 2H), 6.84 - 6.75 (m, 2H), 4.44 (s, 2H), 4.02 (s, 3H), 3.79 - 3.68 (m, 4H), 3.09 - 2.97 (m, 4H), 1.64 (s, 6H). |
| 76 | 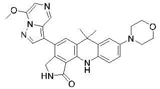 | 497 | ¹H NMR (400 MHz, DMSO-*d₆*) δ ppm: 8.99 (s, 1H), 8.59 (s, 1H), 8.45 - 8.35 (m, 2H), 7.70 (d, *J =* 5.1 Hz, 1H), 7.48 (d, *J =* 4.5 Hz, 1H), 7.08 (d, *J* = 45.8 Hz, 3H), 4.44 (s, 2H), 3.94 (s, 3H), 3.87 (s, 4H), 3.28 (s, 4H), 1.63 (s, 6H). |
| 77 | 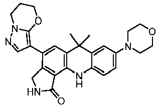 | 472 | ¹H NMR (400 MHz, DMSO-*d₆*) δ ppm: 8.75 (s, 1H), 8.56 (s, 1H), 7.64 (s, 1H), 7.55 (s, 1H), 7.02 (s, 1H), 6.84 (d, *J* = 8.6 Hz, 1H), 6.74 (dd, *J* = 8.6, 2.2 Hz, 1H), 4.45 - 4.40 (m, 2H), 4.38 (s, 2H), 4.14 (t, *J* = 6.0 Hz, 2H), 3.80 - 3.71 (m, 4H), 3.08 - 3.01 (m, 4H), 2.24 (dd, *J* = 10.7, 5.4 Hz, 2H), 1.56 (s, 6H). |
| 78 | 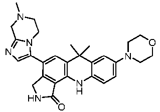 | 485 | ¹H NMR (400 MHz, DMSO-*d₆*) δ ppm: 8.82 (s, 1H), 8.57 (s, 1H), 7.57 (s, 1H), 7.10 (s, 1H), 7.04 (d, *J =* 2.2 Hz, 1H), 6.87 (d, *J* = 8.6 Hz, 1H), 6.75 (dd, *J* = 8.6, 2.4 Hz, 1H), 4.33 (s, 2H), 4.00 - 3.92 (m, 2H), 3.78 - 3.71 (m, 4H), 3.67 - 3.60 (m, 2H), 3.07 - 2.98 (m, 4H), 2.82 - 2.74 (m, 2H), 2.42 (s, 3H), 1.60 (s, 6H). |
| 79 | 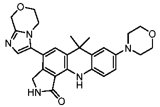 | 472 | ¹H NMR (400 MHz, DMSO-*d₆*) δ ppm: 8.83 (s, 1H), 8.59 (s, 1H), 7.60 (s, 1H), 7.21 (s, 1H), 7.05 (s, 1H), 6.88 (d, *J* = 7.4 Hz, 1H), 6.76 (s, 1H), 4.84 (s, 2H), 4.36 (s, 2H), 4.02 (s, 4H), 3.74 (s, 4H), 3.03 (s, 4H), 1.61 (s, 6H). |
| 80 | 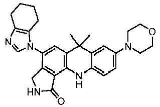 | 470 | ¹H NMR (400 MHz, DMSO-*d₆*) δ ppm: δ 8.79 (s, 1H), 8.59 (s, 1H), 7.70 (s, 1H), 7.43 (s, 1H), 6.99 (d, *J* = 2.2 Hz, 1H), 6.86 (d, *J* = 8.6 Hz, 1H), 6.74 - 6.64 (m, 1H), 4.26 (s, 2H), 3.75 - 3.64 (m, 4H), 3.05 - 2.93 (m, 4H), 2.49 (s, 2H), 2.38 (s, 2H), 1.78-1.63 (m, 4H), 1.54 (s, 6H). |
| 81 | 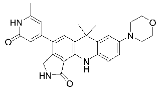 | 457 | ¹H NMR (400 MHz, DMSO-*d₆*) δ ppm: 11.52 (s, 1H), 8.99 (s, 1H), 8.64 (s, 1H), 7.62 (s, 1H), 7.05 (d, *J* = 2.4 Hz, 1H), 6.88 (d, *J* = 8.6 Hz, 1H), 6.76 (dd, *J* = 8.7, 2.5Hz, 1H), 6.26 (d, *J =* 9.7 Hz, 2H), 4.50 (s, 2H), 3.81 - 3.68 (m, 4H), 3.08 - 2.99 (m, 4H), 2.22 (s, 3H), 1.61 (s, 6H). |
| 82 | 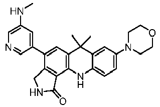 | 456 | ¹H NMR (400 MHz, CD₃OD) δ ppm: 8.52 (s, 1H), 7.90 (s, 1H), 7.62 (s, 1H), 7.13 (s, 1H), 7.10 (s, 1H), 6.87 (s, 1H), 6.85 (s, 1H), 4.64 (s, 2H), 3.90 - 3.84 (m, 4H), 3.10 (d, *J* = 4.9 Hz, 4H), 2.88 (s, 3H), 1.68 (s, 6H). |
| 83 | 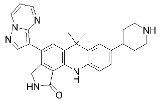 | 465 | ¹H NMR (400 MHz, DMSO-*d₆*) δ ppm: 9.19 (dd, *J* = 7.0, 1.7 Hz, 1H), 8.98 (s, 1H), 8.74 - 8.65 (m, 2H), 8.54 (s, 1H), 8.42 (s, 1H), 8.32 (s, 1H), 7.31 (s, 1H), 7.13 (dd, *J* = 7.0, 4.0 Hz, 1H), 6.98 (d, *J* = 8.2 Hz, 1H), 6.91 (d, *J* = 8.1 Hz, 1H), 4.60 (s, 2H), 3.24 (d, *J* = 11.8 Hz, 2H), 2.82 (t, *J =* 11.5 Hz, 2H), 2.70 (t, *J =* 11.6 Hz, 1H), 1.78 - 1.63 (m, 4H), 1.63 (s, 6H). |
| 84 | 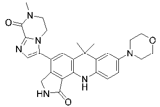 | 499 | ¹H NMR (400 MHz, DMSO-*d₆*) δ ppm: 8.97 (s, 1H), 8.64 (s, 1H), 7.78 (s, 1H), 7.64 - 7.52 (m, 1H), 7.26 - 6.67 (m, 3H), 4.36 - 4.30 (m, 4H), 3.81 - 3.74 (m, 6H), 3.29 - 2.79 (m, 7H), 1.58 (s, 6H). |
| 85 | 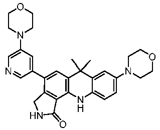 | 556 | ¹H NMR (400 MHz, DMSO-*d₆*) δ ppm: 8.90 (s, 1H), 8.60 (s, 1H), 8.27 (d, *J* = 2.6 Hz, 1H), 8.22 (d, *J =* 1.4 Hz, 1H), 7.63 (s, 1H), 7.42 (s, 1H), 7.05 (d, *J =* 2.3 Hz, 1H), 6.88 (d, *J =* 8.6 Hz, 1H), 6.76 (dd, *J* = 8.7, 2.5 Hz, 1H), 4.49 (s, 2H), 3.79 - 3.72 (m, 8H), 3.27 - 3.23 (m, 4H), 3.05 - 3.01 (m, 4H), 1.62 (s, 6H). |
| 86 | 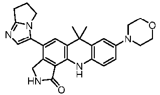 | 456 | ¹H NMR (400 MHz, DMSO-*d₆*) δ ppm: 9.00 (s, 1H), 8.76 (s, 1H), 7.79 (s, 1H), 7.71 (s, 1H), 7.18 (s, 1H), 6.98 (d, *J* = 8.5 Hz, 1H), 6.88 (s, 1H), 4.45 (s, 2H), 4.40 - 4.37 (m, 2H), 3.78 (brs, 4H), 3.24 - 3.20 (m, 2H), 3.13 (br s, 4H), 2.77- 2.71 (m, 2H), 1.63 (s, 6H). |
| 87 | 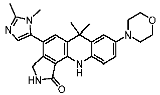 | 444 | ¹H NMR (400 MHz, DMSO-*d₆*) δ ppm: 8.82 (s, 1H), 8.56 (s, 1H), 8.17 (s, 1H), 7.46 (s, 1H), 7.04 (d, *J* = 2.4 Hz, 1H), 6.92 (s, 1H), 6.88 (d, *J* = 8.6 Hz, 1H), 6.75 (dd, *J* = 8.7, 2.5 Hz, 1H), 4.29 (s, 2H), 3.79 - 3.70 (m, 4H), 3.45 (s, 3H), 3.06 - 2.96 (m, 4H), 2.35 (s, 3H), 1.60 (s, 6H). |
| 88 | 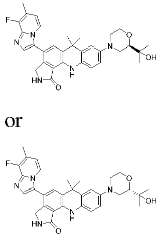 | 556 | ¹H NMR (400 MHz, DMSO-*d₆*) δ ppm: 8.92 (s, 1H), 8.62 (s, 1H), 8.02 (d, *J* = 7.0 Hz, 1H), 7.76 (s, 1H), 7.71 (s, 1H), 7.04 (d, *J* = 2.2 Hz, 1H), 6.94 (d, *J* = 8.6 Hz, 1H), 6.84 (t, *J* = 6.8 Hz, 1H), 6.79 (dd, *J* = 8.6, 2.4 Hz, 1H), 4.30 (s, 2H), 4.02 - 3.91 (m, 1H), 3.64 (dd, *J* = 11.3, 9.0 Hz, 1H), 3.54 (d, *J =* 11.1 Hz, 1H), 3.41 (d, *J* = 11.7 Hz, 1H), 3.35 - 3.24 (m, 1H), 2.63 - 2.55 (m, 1H), 2.49 - 2.40 (m, 1H), 2.33 (d, *J* = 2.0 Hz, 3H), 1.62 (s, 6H), 1.17 (s, 3H), 1.10 (s, 3H). |
| 89 | 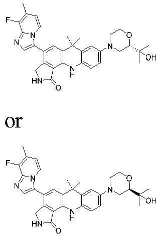 | 556 | ¹H NMR (400 MHz, DMSO-*d₆*) δ ppm: 8.92 (s, 1H), 8.61 (s, 1H), 8.02 (d, *J* = 7.0 Hz, 1H), 7.76 (s, 1H), 7.71 (s, 1H), 7.05 (d, *J* = 2.1 Hz, 1H), 6.94 (d, *J* = 8.6 Hz, 1H), 6.84 (t, *J* = 6.7 Hz, 1H), 6.79 (dd, *J* = 8.6, 2.4 Hz, 1H), 4.49 (s, 1H), 4.30 (s, 2H), 3.97 (d, *J* = 8.6 Hz, 1H), 3.64 (dd, *J* = 11.3, 9.0 Hz, 1H), 3.54 (d, *J =* 11.2 Hz, 1H), 3.41 (d, *J =* 11.9 Hz, 1H), 3.29 (dd, *J* = 10.5, 1.8 Hz, 1H), 2.64 - 2.55 (m, 1H), 2.48 - 2.41 (m, 1H), 2.33 (d, *J* = 1.9 Hz, 3H), 1.62 (s, 6H), 1.17 (s, 3H), 1.10 (s, 3H). |
| 90 | 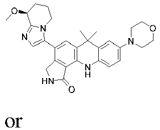 | 500 | ¹H NMR (400 MHz, DMSO-*d₆*) δ ppm: 8.82 (s, 1H), 8.56 (s, 1H), 7.56 (s, 1H), 7.11 (s, 1H), 7.04 (d, *J* = 2.4 Hz, 1H), 6.88 (d, *J =* 8.6 Hz, 1H), 6.75 (dd, *J =* 8.7, 2.5 Hz, 1H), 4.40 - 4.26 (m, 3H), 3.95 - 3.85 (m, 2H), 3.76 - 3.66 (m, 4H), 3.44 (s, 3H), 3.08 - 2.99 (m, 4H), 2.11 - 1.97 (m, 2H), 1.96 - 1.79 (m, 2H), 1.60 (d, *J =* 8.6 Hz, 6H). |
| | 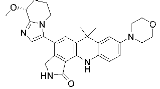 | | |
| 91 | 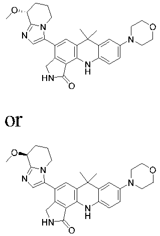 | 500 | ¹H NMR (400 MHz, DMSO-*d₆*) δ ppm: 8.82 (s, 1H), 8.56 (s, 1H), 7.56 (s, 1H), 7.11 (s, 1H), 7.04 (d, *J =* 2.4 Hz, 1H), 6.88 (d, *J =* 8.6 Hz, 1H), 6.75 (dd, *J =* 8.7, 2.5 Hz, 1H), 4.40 - 4.26 (m, 3H), 3.95 - 3.85 (m, 2H), 3.76 - 3.66 (m, 4H), 3.44 (s, 3H), 3.08 - 2.99 (m, 4H), 2.11 - 1.97 (m, 2H), 1.96 - 1.79 (m, 2H), 1.60 (d, *J =* 8.6 Hz, 6H). |
| 92 | 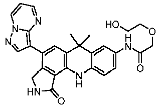 | 499 | ¹H NMR (400 MHz, DMSO-*d₆*) δ ppm: 9.61 (s, 1H), 9.20 - 9.17 (m, 1H), 9.00 (s, 1H), 8.69 - 8.67 (m, 2H), 8.54 (s, 1H), 8.32 (s, 1H), 7.72 (d, *J* = 2.1 Hz, 1H), 7.47 - 7.44 (m, 1H), 7.14 - 7.11 (m, 1H), 6.96 (d, *J* = 8.6 Hz, 1H), 5.08 (s, 1H), 4.60 (s, 2H), 4.05 (s, 2H), 3.60 (s, 4H), 1.62 (s, 6H). |
| 93 | 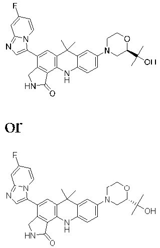 | 542 | ¹H NMR (400 MHz, DMSO-*d₆*) δ ppm: δ 8.92 (s, 1H), 8.61 (s, 1H), 8.33 - 8.26 (m, 1H), 7.78 (s, 1H), 7.73 (s, 1H), 7.52 (dd, *J* = 10.0, 2.6 Hz, 1H), 7.05 (d, *J* = 2.3 Hz, 1H), 7.00 (m, 1H), 6.93 (d, *J* = 8.6 Hz, 1H), 6.79 (dd, *J* = 8.7, 2.4 Hz, 1H), 4.49 (s, 1H), 4.31 (s, 2H), 3.97 (dd, *J* = 10.9, 2.3 Hz, 1H), 3.64 (dd, *J* = 11.4, 9.0 Hz, 1H), 3.54 (d, *J* = 11.1 Hz, 1H), 3.41 (d, *J* = 11.4 Hz, 1H), 3.29 (dd, *J* = 10.4, 1.9 Hz, 1H), 2.60 - 2.55 (m, 1H), 2.43 (d, *J* = 11.1 Hz, 1H), 1.62 (s, 6H), 1.14 (d, *J =* 24.5 Hz, 6H). |
| 94 | 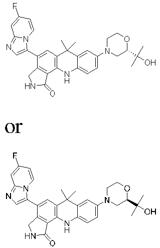 | 542 | ¹H NMR (400 MHz, DMSO-*d₆*) δ ppm: δ 8.92 (s, 1H), 8.61 (s, 1H), 8.33 - 8.26 (m, 1H), 7.78 (s, 1H), 7.73 (s, 1H), 7.52 (dd, *J* = 10.0, 2.6 Hz, 1H), 7.05 (d, *J* = 2.3 Hz, 1H), 7.00 (m, 1H), 6.93 (d, *J* = 8.6 Hz, 1H), 6.79 (dd, *J* = 8.7, 2.4 Hz, 1H), 4.49 (s, 1H), 4.31 (s, 2H), 3.97 (dd, *J* = 10.9, 2.3 Hz, 1H), 3.64 (dd, *J* = 11.4, 9.0 Hz, 1H), 3.54 (d, *J* = 11.1 Hz, 1H), 3.41 (d, *J* = 11.4 Hz, 1H), 3.29 (dd, *J* = 10.4, 1.9 Hz, 1H), 2.60 - 2.55 (m, 1H), 2.43 (d, *J* = 11.1 Hz, 1H), 1.62 (s, 6H), 1.14 (d, J = 24.5 Hz, 6H). |
| 95 | 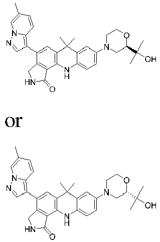 | 538 | ¹H NMR (400 MHz, DMSO-*d₆*) δ ppm: 8.82 (s, 1H), 8.59 (d, *J* = 7.0 Hz, 2H), 8.21 (s, 1H), 7.64 (d, *J* = 7.8 Hz, 2H), 7.23 - 7.15 (m, 1H), 7.04 (d, *J* = 2.3 Hz, 1H), 6.89 (d, *J* = 8.6 Hz, 1H), 6.78 (dd, *J* = 8.6, 2.4 Hz, 1H), 4.48 (s, 1H), 4.43 (s, 2H), 4.01 - 3.94 (m, 1H), 3.64 (dd, *J* = 11.5, 9.0 Hz, 1H), 3.53 (d, *J* = 11.5 Hz, 1H), 3.39 (d, *J* = 11.7 Hz, 1H), 3.31 - 3.28 (m, 1H), 2.62 - 2.56 (m, 1H), 2.47 - 2.42 (t, *J* = 20.0 Hz, 1H), 2.33 (s, 3H), 1.63 (s, 6H), 1.17 (s, 3H), 1.11 (s, 3H). |
| 96 | 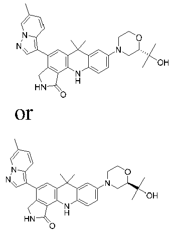 | 538 | ¹H NMR (400 MHz, DMSO-*d₆*) δ ppm: 8.82 (s, 1H), 8.59 (d, *J* = 7.1 Hz, 2H), 8.21 (s, 1H), 7.64 (d, *J* = 7.8 Hz, 2H), 7.23 - 7.14 (m, 1H), 7.04 (d, *J* = 2.3 Hz, 1H), 6.89 (d, *J* = 8.6 Hz, 1H), 6.78 (dd, *J =* 8.6, 2.4 Hz, 1H), 4.48 (s, 1H), 4.43 (s, 2H), 4.02 - 3.93 (m, 1H), 3.64 (dd, *J* = 11.5, 9.0 Hz, 1H), 3.53 (d, *J* = 11.4 Hz, 1H), 3.39 (d, *J* = 11.2 Hz, 1H), 3.32 - 3.27 (m, 2H), 2.63 - 2.56 (m, 1H), 2.47 - 2.42 (t, *J* = 20.0 Hz, 1H), 2.33 (s, 3H), 1.63 (s, 6H), 1.17 (s, 3H), 1.11 (s, 3H). |
| 97 | 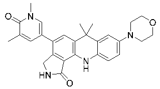 | 471 | ¹H NMR (400 MHz, DMSO-*d₆*) δ ppm: 8.78 (s, 1H), 8.58 (s, 1H), 7.72 (d, *J* = 1.8 Hz, 1H), 7.58 (s, 1H), 7.47 (s, 1H), 7.00 (d, *J* = 2.0 Hz, 1H), 6.81 (s, 1H), 6.71 (dd, *J* = 8.6, 2.2 Hz, 1H), 4.43 (s, 2H), 3.70 (s, 4H), 3.47 (s, 3H), 2.99 (s, 4H), 2.04 (s, 3H), 1.57 (s, 6H). |
| 98 | 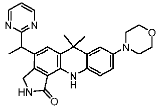 | 456 | ¹H NMR (400 MHz, DMSO-*d₆*) δ ppm: 8.71 (d, *J* = 4.9 Hz, 2H), 8.59 (s, 1H), 8.39 (s, 1H), 7.44 (s, 1H), 7.31 - 7.28 (m, 1H), 6.95 (d, *J* = 2.5 Hz, 1H), 6.77 (d, *J* = 8.6 Hz, 1H), 6.70 - 6.67 (m, 1H), 4.34 (d, *J* = 7.2 Hz, 1H), 4.19 (d, *J* = 5.3 Hz, 2H), 3.76 - 3.62 (m, 4H), 3.02 - 2.88 (m, 4H), 1.63 (d, *J* = 7.2 Hz, 3H), 1.50 (s, 3H), 1.44 (s, 3H). |
| 99 | 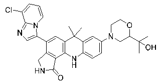 | 558 | ¹H NMR (400 MHz, DMSO-*d₆*) δ ppm: 8.93 (s, 1H), 8.61 (s, 1H), 8.23 (dd, *J* = 6.90, 0.80 Hz, 1H), 7.74 (s, 1H), 7.70 (s, 1H), 7.49 (dd, *J* = 7.3, 0.8 Hz, 1H), 7.04 (d, *J =* 2.5 Hz, 1H), 6.97 (t, *J* = 7.5 Hz, 2H), 6.79 (dd, *J* = 8.7, 2.5 Hz, 1H), 4.48 (s, 1H), 4.31 (s, 2H), 3.96 (dd, *J =* 11, 2.4 Hz, 1H), 3.67 (td, *J* = 11.3, 2.4 Hz, 1H), 3.42 (d, *J* = 11.5 Hz, 1H), 3.37 (d, *J =* 11.7 Hz, 1H), 3.26 (dd, *J =* 10.5, 2.1 Hz, 1H), 2.50 (td, *J =* 11.7, 3.2 Hz, 1H), 2.42 (dd, *J* = 18.2, 7.0 Hz, 1H), 1.62 (s, 6H), 1.23 (s, 3H), 1.11 (s, 3H). |
| 100 | 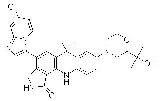 | 558 | ¹H NMR (400 MHz, DMSO-*d₆*) δ ppm: 8.92 (s, 1H), 8.61 (s, 1H), 8.26 (d, *J* = 7.3 Hz, 1H), 7.83 (s, 2H), 7.74 (s, 1H), 7.05 (d, *J* = 2.3 Hz, 1H), 7.01 (m, 1H), 6.93 (d, *J* = 8.6 Hz, 1H), 6.79 - 6.75 (m, 1H), 4.48 (s, 1H), 4.31 (s, 2H), 3.97 - 3.92 (m, 1H), 3.64 - 3.60 (m, 1H), 3.54 (d, *J* = 11.4 Hz, 1H), 3.41 (d, *J* = 11.5 Hz, 1H), 3.31 - 3.28 (m, 1H), 2.64 - 2.57 (m, 1H), 2.43 (d, *J* = 11.1 Hz, 1H), 1.62 (s, 6H), 1.17 (s, 3H), 1.11 (s, 3H). |
| 101 | 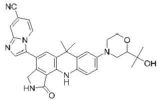 | 549 | ¹H NMR (400 MHz, DMSO-*d₆*) δ ppm: 8.96 (s, 1H), 8.63 (s, 1H), 8.44 (s, 1H), 8.40 (d, *J =* 7.2 Hz, 1H), 8.11 (s, 1H), 7.79 (s, 1H), 7.23 - 7.20 (m, 1H), 7.05 (d, *J =* 2.2 Hz, 1H), 6.94 (d, *J* = 8.6 Hz, 1H), 6.79 - 6.75 (m, 1H), 4.49 (s, 1H), 4.33 (s, 2H), 3.98 (d, *J* = 8.9 Hz, 1H), 3.64 - 3.61 (m, 1H), 3.54 (d,*J* = 11.3 Hz, 1H), 3.41 (d, *J =* 11.7 Hz, 1H), 3.28 (s, 1H), 2.64 - 2.56 (m, 1H), 2.43 (d, *J =* 11.1 Hz, 1H), 1.62 (s, 6H), 1.17 (s, 3H), 1.11 (s, 3H). |
| 102 | 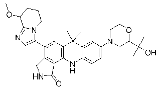 | 558 | ¹H NMR (400 MHz, DMSO-*d₆*) δ ppm: 8.79 (s, 1H), 8.52 (s, 1H), 8.11 (d, *J* = 1.4 Hz, 1H), 7.52 (s, 1H), 7.07 (s, 1H), 6.99 (d, *J* = 2.4 Hz, 1H), 6.85 (d, *J* = 8.6 Hz, 1H), 6.74 (d, *J =* 2.5 Hz, 1H), 4.35 (s, 1H), 4.32 - 4.19 (m, 3H), 3.99 - 3.90 (m, 1H), 3.88 - 3.85 (m, 2H), 3.65 - 3.56 (m, 1H), 3.47 (dd, *J =* 13.1, 6.1 Hz, 1H), 3.40 (s, 3H), 3.34 - 3.38 (m, 1H), 3.26 (d, *J* = 2.1 Hz, 1H), 2.63 - 2.53 (m, 1H), 2.49 - 2.45 (m, 1H), 2.10 - 1.97 (m, 2H), 1.91 - 1.75 (m, 2H), 1.56 (d, *J* = 8.1 Hz, 6H), 1.12 (s, 3H), 1.06 (s, 3H). |
| 103 | 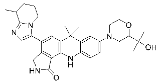 | 542 | ¹H NMR (400 MHz, DMSO-*d₆*) δ ppm: 8.77 (s, 1H), 8.50 (s, 1H), 8.16 (s, 1H), 7.49 (s, 1H), 7.02 - 6.95 (m, 2H), 6.84 (d, *J* = 8.6 Hz, 1H), 6.72 (dd, *J* = 8.7, 2.5 Hz, 1H), 4.43 (s, 1H), 4.38 (d, *J* = 17.9 Hz, 1H), 4.19 (d, *J* = 18.0 Hz, 1H), 3.98 - 3.85 (m, 2H), 3.81 - 3.70 (m, 1H), 3.60 (dd, *J =* 11.3, 2.4 Hz, 1H), 3.48 (d, *J =* 11.5 Hz, 1H), 3.35 (d, *J =* 11.8 Hz, 1H), 3.26 - 3.23 (m, 1H), 2.94 - 2.80 (m, 1H), 2.54 - 2.44 (m, 1H), 2.39 (dd, *J =* 9.7, 5.0 Hz, 1H), 2.06 - 1.91 (m, 2H), 1.83 - 1.68 (m, 1H), 1.58 (s, 3H), 1.54 (s, 3H), 1.47 (d, *J =* 11.1 Hz, 1H), 1.29 (d, *J =* 6.9 Hz, 3H), 1.12 (s, 3H), 1.06 (s, 3H). |
| 104 | 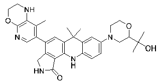 | 556 | ¹H NMR (400 MHz, DMSO-*d₆*) δ ppm: 8.78 (s, 1H), 8.48 (s, 1H), 7.35 (s, 1H), 7.30 (s, 1H), 7.02 (d, *J* = 2.2 Hz, 1H), 6.89 (d, *J* = 8.6 Hz, 1H), 6.78 - 6.75 (m, 1H), 5.61 (s, 1H), 4.48 (s, 1H), 4.26 (s, 2H), 4.12 (br s, 2H), 3.97 (d, *J* = 10.7 Hz, 1H), 3.66 - 3.61 (m, 1H), 3.52 (d, *J =* 11.3 Hz, 1H), 3.40 - 3.33 (m, 3H), 3.29 (d, *J* = 10.5 Hz, 1H), 2.61 - 2.56 (m,, 1H), 2.42 (d, *J =* 11.0 Hz, 1H), 1.89 (s, 3H), 1.57 (s, 6H), 1.13 (d, *J* = 24.5 Hz, 6H). |
| 105 | 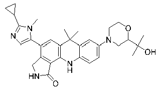 | 528 | ¹H NMR (400 MHz, DMSO-*d₆*) δ ppm: 8.82 (s, 1H), 8.55 (s, 1H), 8.19 (s, 1H), 7.50 (s, 1H), 7.04 (d, *J* = 2.3 Hz, 1H), 6.90 - 6.88 (m, 2H), 6.78 - 6.76 (m, 1H), 4.47 (s, 1H), 4.29 (s, 2H), 3.99 - 3.96 (m, 1H), 3.66 - 3.61 (m, 1H), 3.57 (s, 3H), 3.53 (d, *J* = 11.5 Hz, 1H), 3.39 (d, *J* = 11.5 Hz, 1H), 3.31 - 3.27 (m, 1H), 2.62 - 2.56 (m, 1H), 2.47 - 2.41 (m, 1H), 2.04 - 1.97 (m, 1H), 1.61 (s, 6H), 1.16 (s, 3H), 1.10 (s, 3H), 0.95 - 0.90 (m, 2H), 0.88 - 0.83 (m, 2H). |
| 106 | 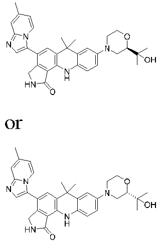 | 538 | ¹H NMR (400 MHz, DMSO-*d₆*) δ ppm: 8.86 (s, 1H), 8.55 (s, 1H), 8.11 (d, *J* = 7.0 Hz, 1H), 7.65 (s, 2H), 7.43 - 7.35 (m, 1H), 7.00 (d, *J* = 2.4 Hz, 1H), 6.89 (d, *J* = 8.6 Hz, 1H), 6.80 - 6.70 (m, 2H), 4.44 (s, 1H), 4.26 (s, 2H), 3.93 - 3.89 (m, 1H), 3.60 (dd, *J* = 11.3, 2.4 Hz, 1H), 3.50 (d, *J* = 11.5 Hz, 1H), 3.36 (d, *J* = 11.7 Hz, 1H), 3.25 (dd, *J* = 10.5, 2.1 Hz, 1H), 2.56 (dd, *J* = 11.8, 8.4 Hz, 1H), 2.43 - 2.38 (m, 1H), 2.34 (s, 3H), 1.58 (s, 6H), 1.13 (s, 3H), 1.07 (s, 3H). |
| 107 | 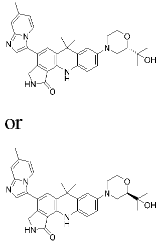 | 538 | ¹H NMR (400 MHz, DMSO-*d₆*) δ ppm: 8.86 (s, 1H), 8.55 (s, 1H), 8.11 (d, *J* = 7.0 Hz, 1H), 7.65 (s, 2H), 7.43 - 7.35 (m, 1H), 7.00 (d, *J* = 2.4 Hz, 1H), 6.89 (d, *J* = 8.6 Hz, 1H), 6.80 - 6.70 (m, 2H), 4.44 (s, 1H), 4.26 (s, 2H), 3.93 - 3.89 (m, 1H), 3.60 (dd, *J* = 11.3, 2.4 Hz, 1H), 3.50 (d, *J* = 11.5 Hz, 1H), 3.36 (d, *J* = 11.7 Hz, 1H), 3.25 (dd, *J* = 10.5, 2.1 Hz, 1H), 2.56 (dd, *J* = 11.8, 8.4 Hz, 1H), 2.43 - 2.38 (m, 1H), 2.34 (s, 3H), 1.58 (s, 6H), 1.13 (s, 3H), 1.07 (s, 3H). |
| 108 | 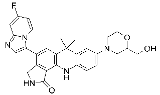 | 514 | ¹H NMR (400 MHz, DMSO-*d₆*) δ ppm: 8.91 (s, 1H), 8.61 (s, 1H), 8.38 (s, 1H), 8.34 - 8.26 (m, 1H), 7.75 (d, *J* = 17.7 Hz, 2H), 7.52 (dd, *J* = 10.0, 2.4 Hz, 1H), 7.05 (d, *J* = 2.3 Hz, 1H), 7.00 (m, 1H), 6.93 (d, *J* = 8.6 Hz, 1H), 6.77 (dd, *J* = 8.6, 2.3 Hz, 1H), 4.77 (s, 1H), 4.31 (s, 2H), 3.93 (d, *J* = 11.2 Hz, 1H), 3.65 (dd, *J* = 11.2, 9.1 Hz, 1H), 3.60 - 3.54 (m, 1H), 3.53 - 3.42 (m, 4H), 2.69 - 2.59 (m, 1H), 2.42 - 2.34 (m, 1H), 1.62 (s, 6H). |
| 109 | 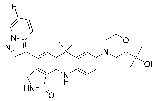 | 542 | ¹H NMR (400 MHz, DMSO-*d₆*) δ ppm: 9.07 (dd, J = 4.7, 2.0 Hz, 1H), 8.83 (s, 1H), 8.61 (s, 1H), 8.40 (s, 1H), 8.33 (s, 1H), 7.77 (dd, *J* = 9.8, 5.6 Hz, 1H), 7.64 (s, 1H), 7.46 - 7.38 (m, 1H), 7.05 (d, *J* = 2.2 Hz, 1H), 6.90 (d, *J* = 8.6 Hz, 1H), 6.78 (dd, *J* = 8.6, 2.4 Hz, 1H), 4.49 (s, 1H), 4.43 (s, 2H), 3.98 (d, *J* = 8.7 Hz, 1H), 3.64 (t, *J* = 11.4, 9.1 Hz, 1H), 3.53 (d, *J* = 11.6 Hz, 1H), 3.41 (s, 1H), 3.29 (s, 1H), 2.66 - 2.57 (m, 1H), 2.43 (d, *J* = 11.1 Hz, 1H), 1.63 (s, 6H), 1.17 (s, 3H), 1.11 (s, 3H). |
| 110 | 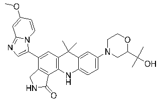 | 554 | ¹H NMR (400 MHz, DMSO-*d₆*) δ ppm: 8.89 (s, 1H), 8.59 (s, 1H), 8.18 (s, 1H), 8.10 (d, *J* = 7.5 Hz, 1H), 7.68 (s, 1H), 7.61 (s, 1H), 7.03 (dd, *J* = 7.9, 2.4 Hz, 2H), 6.92 (d, *J* = 8.6 Hz, 1H), 6.78 (dd, *J =* 8.7, 2.4 Hz, 1H), 6.66 (dd, *J =* 7.5, 2.5 Hz, 1H), 4.48 (s, 1H), 4.30 (s, 2H), 3.97 (dd, *J* = 10.9, 2.3 Hz, 1H), 3.86 (s, 3H), 3.67-3.61 (m, 1H), 3.53 (d, *J* = 11.5 Hz, 1H), 3.42 (s, 1H), 3.28 (d, *J =* 1.9 Hz, 1H), 2.58 (dd, *J =* 11.6, 3.1 Hz, 1H), 2.43 (d, *J =* 11.0 Hz, 1H), 1.62 (s, 6H), 1.17 (s, 3H), 1.10 (s, 3H). |
| 111 | 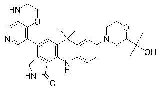 | 542 | ¹H NMR (400 MHz, DMSO-*d₆*) δ ppm: 8.79 (s, 1H), 8.46 (s, 1H), 8.19 (s, 1H), 7.79 (s, 1H), 7.64 (s, 1H), 7.41 (s, 1H), 6.99 (d, *J =* 2.4 Hz, 1H), 6.84 (d, *J =* 8.6 Hz, 1H), 6.74 (d, *J =* 2.5 Hz, 1H), 6.05 (s, 1H), 4.44 (s, 1H), 4.25 - 4.13 (m, 4H), 3.99 - 3.86 (m, 1H), 3.60 (dd, *J =* 11.4, 2.5 Hz, 1H), 3.48 (d, *J =*11.5 Hz, 1H), 3.35 - 3.33 (m, 2H), 3.25 - 3.20 (m, 2H), 2.64 - 2.53 (m, 1H), 2.47 - 2.42 (m, 1H), 1.55 (s, 6H), 1.12 (s, 3H), 1.06 (s, 3H). |
| 112 | 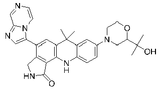 | 525 | ¹H NMR (400 MHz, DMSO-*d₆*) δ ppm: 9.14 (d, *J* = 1.3 Hz, 1H), 8.96 (s, 1H), 8.65 (s, 1H), 8.32 (dd, *J* = 4.7, 1.3 Hz, 1H), 8.08 (s, 1H), 7.93 (d, *J =* 4.7 Hz, 1H), 7.81 (s, 1H), 7.05 (d, *J* = 2.2 Hz, 1H), 6.95 (d, *J =* 8.6 Hz, 1H), 6.79 (dd, *J* = 8.7, 2.4 Hz, 1H), 4.49 (s, 1H), 4.37 (s, 2H), 3.98 (dd, *J =* 10.9, 2.2 Hz, 1H), 3.64 (dd, *J* = 11.4, 9.1 Hz, 1H), 3.54 (d, *J* = 11.4 Hz, 1H), 3.41 (d, *J* = 11.4 Hz, 1H), 3.29 (dd, *J* = 10.4, 1.9 Hz, 1H), 2.65 - 2.55 (m, 1H), 2.44 (d, *J* = 11.0 Hz, 1H), 1.64 (s, 6H), 1.17 (s, 3H), 1.11 (s, 3H). |
| 113 | 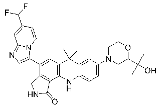 | 574 | ¹H NMR (400 MHz, DMSO-*d₆*) δ ppm: δ 8.94 (s, 1H), 8.62 (s, 1H), 8.38 (d, *J* = 7.2 Hz, 1H), 7.93 (d, *J* = 5.8 Hz, 2H), 7.77 (s, 1H), 7.20 (d, *J* = 55.5 Hz, 1H), 7.09 (d, *J* = 7.3 Hz, 1H), 7.07 - 6.98 (m, 1H), 6.94 (d, *J =* 8.6 Hz, 1H), 6.79 (dd, *J* = 8.7, 2.4 Hz, 1H), 4.49 (s, 1H), 4.33 (s, 2H), 3.98 (dd, *J =* 10.8, 2.5 Hz, 1H), 3.64 (dd, *J =* 11.5, 9.0 Hz, 1H), 3.54 (d, *J =* 11.5 Hz, 1H), 3.41 (d, *J =* 11.8 Hz, 1H), 3.30 - 3.26 (m, 1H), 2.64 - 2.56 (m, 1H), 2.44 (d, *J =* 11.0 Hz, 1H), 1.63 (s, 6H), 1.14 (d, *J =* 24.4 Hz, 6H). |
| 114 | 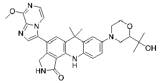 | 555 | ¹H NMR (400 MHz, DMSO-*d₆*) δ ppm: 8.94 (s, 1H), 8.63 (s, 1H), 7.96 - 7.86 (m, 2H), 7.73 (s, 1H), 7.46 (d, *J* = 4.7 Hz, 1H), 7.05 (d, *J =* 2.0 Hz, 1H), 6.94 (d, *J* = 8.6 Hz, 1H), 6.79 (dd, *J =* 8.7, 2.4 Hz, 1H), 4.49 (s, 1H), 4.34 (s, 2H), 4.07 (s, 3H), 3.98 (d, *J =* 8.6 Hz, 1H), 3.67 - 3.61 (m, 1H), 3.54 (d, *J =* 11.2 Hz, 1H), 3.41 (d, *J* = 11.3 Hz, 1H), 3.29 (dd, *J* = 10.4 Hz, 1H), 2.60 (dd, *J =* 11.8, 8.6 Hz, 1H), 2.43 (d, *J =* 11.1 Hz, 1H), 1.62 (s, 6H), 1.17 (s, 3H), 1.11 (s, 3H). |
| 115 | 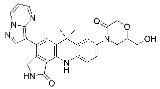 | 511 | ¹H NMR (400 MHz, DMSO-*d₆*) δ ppm: 9.19 (dd, *J* = 7.0, 1.6 Hz, 1H), 9.10 (s, 1H), 8.72 (s, 1H), 8.69 (dd, *J* = 4.0, 1.7 Hz, 1H), 8.55 (s, 1H), 8.46 (s, 1H), 8.34 (s, 1H), 7.44 (d, *J* = 2.0 Hz, 1H), 7.13 (dd, *J* = 7.0, 4.0 Hz, 1H), 7.09 (dd, *J* = 8.5, 2.1 Hz, 1H), 7.02 (d, *J* = 8.5 Hz, 1H), 4.98 (s, 1H), 4.61 (s, 2H), 4.23 (d, *J =* 1.7 Hz, 2H), 3.98 (dd, *J =* 10.0, 3.6 Hz, 1H), 3.72 - 3.63 (m, 1H), 3.60 - 3.50 (m, 3H), 1.63 (d, *J =* 3.0 Hz, 6H). |
| 116 | 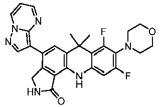 | 503 | ¹H NMR (400 MHz, DMSO-*d₆*) δ ppm: 9.15 - 9.04 (m, 2H), 8.64 (d, *J* = 2.5 Hz, 1H), 8.48 (s, 1H), 8.43 (s, 1H), 8.29 (s, 1H), 7.08 (dd, *J* = 6.9, 4.0 Hz, 1H), 6.74 (d, *J* = 12.8 Hz, 1H), 4.56 (s, 2H), 3.72 - 3.66 (m, 4H), 3.03 (s, 4H), 1.78 (s, 6H). |
| 117 | 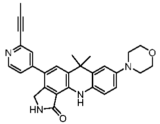 | 465 | ¹H NMR (400 MHz, DMSO-*d₆*) δ ppm: 8.98 (s, 1H), 8.63 (s, 1H), 8.48 (d, *J* = 5.3 Hz, 1H), 7.73 (s, 1H), 7.61 (d, *J* = 1.3 Hz, 1H), 7.51 (dd, *J =* 5.3, 1.8 Hz, 1H), 7.02 (d, *J =* 2.5 Hz, 1H), 6.85 (d, *J* = 8.6 Hz, 1H), 6.72 (dd, *J* = 8.7, 2.5 Hz, 1H), 4.53 (s, 2H), 3.74 - 3.66 (m, 4H), 3.02 - 2.96 (m, 4H), 2.06 (s, 3H), 1.60 (s, 6H). |
| 118 | 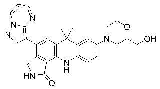 | 497 | ¹H NMR (400 MHz, DMSO-*d₆*) δ ppm: 9.13 (dd, *J* = 7.0, 1.7 Hz, 1H), 8.82 (s, 1H), 8.63 (dd, *J* = 4.0, 1.7 Hz, 1H), 8.58 (s, 1H), 8.49 (s, 1H), 8.24 (s, 1H), 7.07 (dd, *J* = 7.0, 4.0 Hz, 1H), 7.01 (d, *J* = 2.4 Hz, 1H), 6.84 (d, *J* = 8.6 Hz, 1H), 6.73 (dd, *J* = 8.7, 2.5 Hz, 1H), 4.71 (s, 1H), 4.54 (s, 2H), 3.89 (dd, *J =* 11.2, 1.7 Hz, 1H), 3.62 (td, *J* = 11.3, 2.4 Hz, 1H), 3.54 (m, 1H), 3.49 - 3.33 (m, 4H), 2.59 (d, *J* = 3.2 Hz, 1H), 2.37 - 2.31 (m, 1H), 1.60 (s, 6H). |
| 119 | 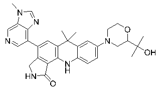 | 539 | ¹H NMR (400 MHz, CDCl₃) δ ppm: 8.95 - 8.88 (m, 2H), 8.52 (s, 1H), 8.05 (s, 1H), 7.75 (s, 1H), 7.06 (s, 1H), 6.83 (s, 2H), 6.16 (s, 1H), 4.55 (s, 2H), 4.08 (d, *J* = 10.4 Hz, 1H), 4.03 (s, 3H), 3.87 (t, *J =* 10.8 Hz, 1H), 3.52 (d, *J =* 10.3 Hz, 1H), 3.41 (d, *J* = 11.0 Hz, 1H), 3.29 (d, *J* = 11.2 Hz, 1H), 2.85 (d, *J =* 10.3 Hz, 1H), 2.68 (t, *J* = 10.7 Hz, 1H), 1.67 (s, 6H), 1.29 (s, 3H), 1.25 (s, 3H). |
| 120 | 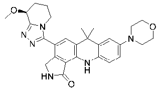 or | 501 | ¹H NMR (400 MHz, DMSO-*d₆*) δ ppm: 8.99 (s, 1H), 8.61 (s, 1H), 7.80 (s, 1H), 7.12 (s, 1H), 6.94 (d, *J* = 7.9 Hz, 1H), 6.83 (s, 1H), 4.57 (d, *J* = 3.9 Hz, 1H), 4.55 - 4.35 (m, 2H), 4.18 - 4.05 (m, 2H), 3.77 (s, 4H), 3.48 (s, 3H), 3.08 (s, 4H), 2.18 - 1.83 (m, 4H), 1.63 (d, *J =* 8.6 Hz, 6H). |
| | 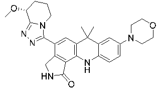 | | |
| 121 | 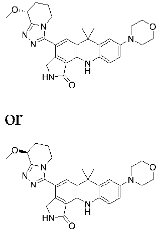 | 501 | ¹H NMR (400 MHz, DMSO-*d₆*) δ ppm: 8.99 (s, 1H), 8.61 (s, 1H), 7.80 (s, 1H), 7.12 (s, 1H), 6.94 (d, *J* = 7.9 Hz, 1H), 6.83 (s, 1H), 4.57 (d, *J* = 3.9 Hz, 1H), 4.55 - 4.35 (m, 2H), 4.18 - 4.05 (m, 2H), 3.77 (s, 4H), 3.48 (s, 3H), 3.08 (s, 4H), 2.18 - 1.83 (m, 4H), 1.63 (d, *J =* 8.6 Hz, 6H). |
| 122 | 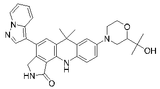 | 524 | ¹H NMR (400 MHz, CDCl₃) δ ppm: 8.95 (s, 1H), 8.52 (d, *J =* 6.8 Hz, 1H), 7.96 (s, 1H), 7.59 (s, 1H), 7.51 (d, *J* = 8.7 Hz, 1H), 7.22 - 7.16 (m, 1H), 7.00 (s, 1H), 6.85 (t, *J* = 6.6 Hz, 1H), 6.62 (s, 1H), 4.40 (s, 2H), 4.19 (d, *J =* 40.6 Hz, 2H), 3.90 (s, 1H), 3.52 (d, *J =* 49.5 Hz, 2H), 3.12 (s, 2H), 1.65 (s, 6H), 1.32 (s, 3H), 1.25 (s, 3H). |
| 123 | 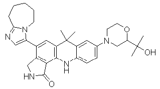 | 542 | ¹H NMR (400 MHz, DMSO-*d₆*) δ ppm: 8.83 (s, 1H), 8.55 (s, 1H), 7.29 (s, 1H), 7.03 (d, *J =* 2.0 Hz, 1H), 6.90 (d, *J =* 8.6 Hz, 1H), 6.85 (s, 1H), 6.77 (dd, *J =* 8.6, 2.2 Hz, 1H), 4.47 (s, 1H), 4.27 (s, 2H), 3.99 - 3.93 (m, 1H), 3.82 (d, *J* = 7.5 Hz, 2H), 3.64 (dd, *J* = 11.3, 9.3 Hz, 1H), 3.52 (d, *J* = 11.4 Hz, 1H), 3.37 (s, 1H), 3.28 (s, 1H), 2.90 - 2.83 (m, 2H), 2.61 - 2.56 (m, 1H), 2.43 (d, *J =* 10.9 Hz, 1H), 1.82 (s, 2H), 1.71 (s, 2H), 1.63 (s, 2H), 1.58 (s, 6H), 1.16 (s, 3H), 1.10 (s, 3H). |
| 124 | 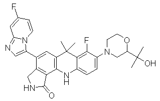 | 560 | ¹H NMR (400 MHz, DMSO-*d₆*) δ ppm: δ 9.08 (s, 1H), 8.69 (s, 1H), 8.52 (dd, *J =* 7.4, 5.3 Hz, 1H), 8.28 (s, 1H), 7.97 (dd, *J* = 8.4, 2.3 Hz, 1H), 7.88 (s, 1H), 7.49 (d, *J* = 2.3 Hz, 1H), 6.94 (d, *J* = 8.7 Hz, 1H), 6.84 (d, *J* = 8.6 Hz, 1H), 4.32 (s, 2H), 3.94 (d, *J* = 9.5 Hz, 1H), 3.67 (s, 1H), 3.33 (d, *J* = 8.7 Hz, 1H), 3.25 (d, *J =* 11.1 Hz, 1H), 3.01 (d, *J =* 11.0 Hz, 1H), 2.75 - 2.67 (m, 1H), 2.55 (s, 1H), 1.77 (s, 6H), 1.15 (s, 3H), 1.08 (s, 3H). |
| 125 | 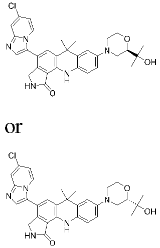 | 558 | ¹H NMR (400 MHz, DMSO-*d₆*) δ ppm: 8.92 (s, 1H), 8.62 (s, 1H), 8.26 (d, *J =* 7.4 Hz, 1H), 7.83 (s, 2H), 7.74 (s, 1H), 7.05 (d, *J* = 2.2 Hz, 1H), 7.01 (dd, *J =* 7.3, 2.1 Hz, 1H), 6.93 (d, *J =* 8.6 Hz, 1H), 6.79 (dd, *J =* 8.7, 2.3 Hz, 1H), 4.49 (s, 1H), 4.31 (s, 2H), 3.97 (d, *J* = 8.9 Hz, 1H), 3.64 (dd, *J =* 11.5, 9.2 Hz, 1H), 3.54 (d, *J =* 11.4 Hz, 1H), 3.41 (d, *J =* 11.7 Hz, 1H), 3.29 (dd, *J* = 10.5, 1.7 Hz, 1H), 2.64 - 2.55 (m, 1H), 2.49 - 2.42 (m, 1H), 1.62 (s, 6H), 1.17 (s, 3H), 1.10 (s, 3H). |
| 126 | 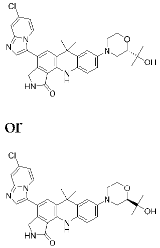 | 558 | ¹H NMR (400 MHz, DMSO-*d₆*) δ ppm: 8.92 (s, 1H), 8.61 (s, 1H), 8.26 (d, *J =* 7.4 Hz, 1H), 7.83 (s, 2H), 7.74 (s, 1H), 7.04 (s, 1H), 7.01 (dd, *J* = 7.3, 2.1 Hz, 1H), 6.93 (d, *J* = 8.6 Hz, 1H), 6.79 (dd, *J =* 8.7, 2.3 Hz, 1H), 4.49 (s, 1H), 4.31 (s, 2H), 3.97 (d, *J =* 8.9 Hz, 1H), 3.69 - 3.59 (m, 1H), 3.54 (d, *J =* 11.4 Hz, 1H), 3.41 (d, *J =* 11.7 Hz, 1H), 3.29 (dd, *J =* 10.5, 1.7 Hz, 1H), 2.64 - 2.55 (m, 1H), 2.49 - 2.42 (m, 1H), 1.62 (s, 6H), 1.17 (s, 3H), 1.10 (s, 3H). |
| 127 | 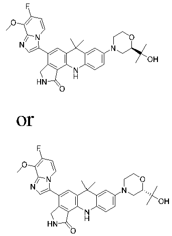 | 572 | ¹H NMR (400 MHz, DMSO-*d₆*) δ ppm: 8.92 (s, 1H), 8.61 (s, 1H), 7.98 (dd, *J =* 7.5, 4.7 Hz, 1H), 7.76 (s, 1H), 7.71 (s, 1H), 7.02 (dd, *J* = 9.6, 7.7 Hz, 2H), 6.93 (d, *J* = 8.6 Hz, 1H), 6.78 (dd, *J* = 8.6, 2.4 Hz, 1H), 4.49 (s, 1H), 4.30 (s, 2H), 4.23 (s, 3H), 3.98 (d, *J* = 10.7 Hz, 1H), 3.64 (t, *J =* 10.4 Hz, 1H), 3.53 (d, *J* = 11.8 Hz, 1H), 3.41 (d, *J* = 11.5 Hz, 1H), 3.29 (d, *J =* 10.4 Hz, 1H), 2.59 (dd, *J* = 11.8, 8.5 Hz, 1H), 2.43 (d, *J =* 11.0 Hz, 1H), 1.61 (s, 6H), 1.17 (s, 3H), 1.10 (s, 3H). |
| 128 | 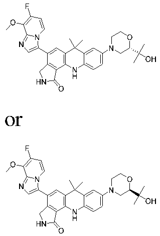 | 572 | ¹H NMR (400 MHz, DMSO-*d₆*) δ ppm: 8.92 (s, 1H), 8.61 (s, 1H), 7.98 (dd, *J =* 7.5, 4.7 Hz, 1H), 7.76 (s, 1H), 7.71 (s, 1H), 7.02 (dd, *J =* 9.6, 7.6 Hz, 2H), 6.93 (d, *J =* 8.6 Hz, 1H), 6.78 (dd, *J =* 8.7, 2.4 Hz, 1H), 4.49 (s, 1H), 4.30 (s, 2H), 4.23 (s, 3H), 3.97 (d, *J =* 8.6 Hz, 1H), 3.64 (dd, *J =* 11.4, 9.2 Hz, 1H), 3.53 (d, *J =* 11.4 Hz, 1H), 3.41 (d, *J =* 11.6 Hz, 1H), 3.30 (dd, *J* = 8.2, 6.4 Hz, 1H), 2.63 - 2.55 (m, 1H), 2.43 (d, *J =* 11.0 Hz, 1H), 1.61 (s, 6H), 1.17 (s, 3H), 1.10 (s, 3H). |
| 129 | 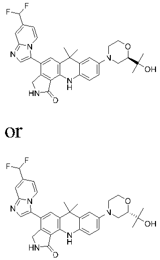 | 574 | ¹H NMR (400 MHz, DMSO-*d₆*) δ ppm: δ 8.94 (s, 1H), 8.62 (s, 1H), 8.38 (d, *J =* 7.2 Hz, 1H), 7.96 - 7.93 (m, 2H), 7.77 (s, 1H), 7.13 (t, *J =* 55.4 Hz, 1H), 7.09 (dd, *J =* 7.3, 1.5 Hz, 1H), 7.05 (d, *J =* 2.4 Hz, 1H), 6.94 (d, *J =* 8.6 Hz, 1H), 6.79 (dd, *J* = 8.7, 2.5 Hz, 1H), 4.49 (s, 1H), 4.33 (s, 2H), 3.98 (dd, *J =* 10.8, 2.5 Hz, 1H), 3.64 (dd, *J =* 11.5, 9.0 Hz, 1H), 3.54 (d, *J =* 11.5 Hz, 1H), 3.41 (d, *J =* 11.8 Hz, 1H), 3.30 - 3.26 (m, 1H), 2.64 - 2.56 (m, 1H), 2.44 (d, *J =* 11.0 Hz, 1H), 1.63 (s, 6H), 1.17 (s, 3H), 1.11 (s, 3H). |
| 130 | 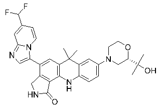 | 574 | ¹H NMR (400 MHz, DMSO-*d₆*) δ ppm: δ 8.94 (s, 1H), 8.62 (s, 1H), 8.38 (d, *J =* 7.2 Hz, 1H), 7.96 - 7.93 (m, 2H), 7.77 (s, 1H), 7.13 (t, *J =* 55.4 Hz, 1H), 7.09 (dd, *J =* 7.3, 1.5 Hz, 1H), |
| | or 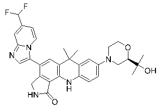 | | 7.05 (d, *J* = 2.4 Hz, 1H), 6.94 (d, *J* = 8.6 Hz, 1H), 6.79 (dd, *J* = 8.7, 2.5 Hz, 1H), 4.49 (s, 1H), 4.33 (s, 2H), 3.98 (dd, *J =* 10.8, 2.5 Hz, 1H), 3.64 (dd, *J =* 11.5, 9.0 Hz, 1H), 3.54 (d, *J =* 11.5 Hz, 1H), 3.41 (d, *J* = 11.8 Hz, 1H), 3.30 - 3.26 (m, 1H), 2.64 - 2.56 (m, 1H), 2.44 (d, *J* = 11.0 Hz, 1H), 1.63 (s, 6H), 1.17 (s, 3H), 1.11 (s, 3H). |
| 131 | 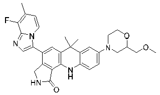 | 542 | ¹H NMR (400 MHz,DMSO-*d*₆) δ ppm: 8.88 (s, 1H), 8.57 (s, 1H), 7.98 (d, *J* = 7.0 Hz, 1H), 7.72 (s, 1H), 7.66 (s, 1H), 7.02 (d, *J* = 2.5 Hz, 1H), 6.88 (d, *J* = 8.6 Hz, 1H), 6.80 (t, *J* = 6.8 Hz, 1H), 6.73 (dd, *J* = 8.7, 2.5 Hz, 1H), 4.26 (s, 2H), 3.92 - 3.86 (m, 1H), 3.74 - 3.66 (m, 1H), 3.61 (m, 1H), 3.44 - 3.34 (m, 4H), 3.26 - 3.22 (m, 3H), 2.59 (m, 1H), 2.40 - 2.30 (m, 1H), 2.28 (d, *J =* 2.2 Hz, 3H), 1.57 (s, 6H). |
| 133 | 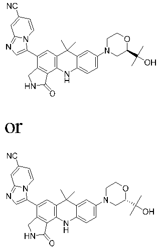 | 549 | ¹H NMR (400 MHz, DMSO-*d₆*) δ ppm: 9.06 (s, 1H), 8.66 (s, 1H), 8.48 (s, 1H), 8.43 (d, *J =* 6.4 Hz, 1H), 8.17 (s, 1H), 7.81 (s, 1H), 7.32 - 7.19 (m, 2H), 7.00 (d, *J* = 26.2 Hz, 2H), 4.34 (s, 2H), 4.02 (s, 1H), 3.71 (s, 1H), 3.56 (s, 1H), 3.42 (d, *J =* 24.7 Hz, 2H), 2.91 (s, 1H), 2.67 (s, 1H), 1.63 (s, 6H), 1.17 (s, 3H), 1.12 (s, 3H). |
| 134 | 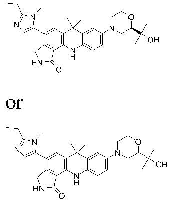 | 516 | ¹H NMR (400 MHz, DMSO-*d₆*) δ ppm: 8.82 (s, 1H), 8.54 (s, 1H), 7.47 (s, 1H), 7.03 (d, *J* = 2.1 Hz, 1H), 6.94 (s, 1H), 6.89 (d, *J* = 8.6 Hz, 1H), 6.77 (dd, *J* = 8.6, 2.3 Hz, 1H), 4.47 (s, 1H), 4.29 (s, 2H), 3.97 (d, *J* = 8.5 Hz, 1H), 3.64 (t, *J* = 10.3 Hz, 1H), 3.52 (d, *J* = 11.3 Hz, 1H), 3.46 (s, 3H), 3.39 (d, *J =* 11.6 Hz, 1H), 3.29 (d, *J* = 10.3 Hz, 1H), 2.73 - 2.69 (m, 2H), 2.59 (dd, *J =* 11.8, 8.6 Hz, 1H), 2.43 (d, *J =* 11.1 Hz, 1H), 1.60 (s, 6H), 1.26 (t, *J* = 7.5 Hz, 3H), 1.16 (s, 3H), 1.10 (s, 3H). |
| 135 | 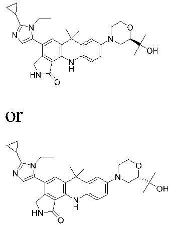 | 542 | ¹H NMR (400 MHz, DMSO-*d₆*) δ ppm: 8.79 (s, 1H), 8.50 (s, 1H), 7.42 (s, 1H), 6.99 (d, *J* = 2.3 Hz, 1H), 6.91 - 6.81 (m, 2H), 6.75 - 6.72 (m, 1H), 4.43 (s, 1H), 4.20 (s, 2H), 3.96 - 3.91 (m, 3H), 3.69 - 3.55 (m, 1H), 3.50 - 3.47 (m, 1H), 3.37 - 3.34 (m, 1H), 3.27 - 3.23 (m, 1H), 2.58 - 2.52 (m, 1H), 2.43 - 2.37 (m, 1H), 2.03 - 1.98 (m, 1H), 1.55 (s, 6H), 1.18 - 1.11 (m, 6H), 1.06 (s, 3H), 0.94 - 0.88 (m, 2H), 0.87 - 0.83 (m, 2H). |
| 136 | 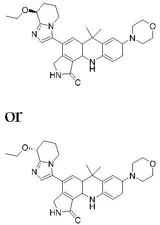 | 514 | ¹H NMR (400 MHz, DMSO-*d₆*) δ ppm: 8.78 (s, 1H), 8.51 (s, 1H), 7.52 (s, 1H), 7.06 (s, 1H), 7.00 (d, *J* = 2.4 Hz, 1H), 6.83 (d, *J* = 8.6 Hz, 1H), 6.72 (d, *J* = 2.5 Hz, 1H), 4.43 (s, 1H), 4.29 (d, *J* = 12.9 Hz, 2H), 3.92 - 3.76 (m, 3H), 3.73 - 3.67 (m, 4H), 3.61 (d, *J* = 7.0 Hz, 1H), 3.03 - 2.92 (m, 4H), 2.08 - 1.97 (m, 2H), 1.94 - 1.78 (m, 2H), 1.56 (d, *J =* 8.5 Hz, 6H), 1.09 (t, *J =* 7.0 Hz, 3H). |
| 137 | 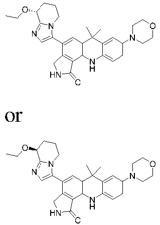 | 514 | ¹H NMR (400 MHz, DMSO-*d₆*) δ ppm: 8.78 (s, 1H), 8.51 (s, 1H), 7.52 (s, 1H), 7.06 (s, 1H), 7.00 (d, *J* = 2.4 Hz, 1H), 6.83 (d, *J* = 8.6 Hz, 1H), 6.72 (d, *J* = 2.5 Hz, 1H), 4.43 (s, 1H), 4.29 (d, *J* = 12.9 Hz, 2H), 3.92 - 3.76 (m, 3H), 3.73 - 3.67 (m, 4H), 3.61 (d, *J* = 7.0 Hz, 1H), 3.03 - 2.92 (m, 4H), 2.08 - 1.97 (m, 2H), 1.94 - 1.78 (m, 2H), 1.56 (d, *J =* 8.5 Hz, 6H), 1.09 (t, *J =* 7.0 Hz, 3H). |
| 138 | 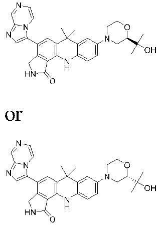 | 525 | ¹H NMR (400 MHz, DMSO-*d₆*) δ ppm: 9.13 (d, *J* = 1.1 Hz, 1H), 8.96 (s, 1H), 8.63 (s, 1H), 8.32 (dd, *J =* 4.7, 1.4 Hz, 1H), 8.08 (s, 1H), 7.93 (d, *J =* 4.7 Hz, 1H), 7.81 (s, 1H), 7.05 (d, *J* = 2.3 Hz, 1H), 6.94 (d, *J =* 8.6 Hz, 1H), 6.79 (dd, *J =* 8.7, 2.5 Hz, 1H), 4.48 (s, 1H), 4.36 (s, 2H), 3.98 (dd, *J =* 11.0, 2.3 Hz, 1H), 3.64 (dd, *J* = 11.5, 9.0 Hz, 1H), 3.54 (d, *J* = 11.5 Hz, 1H), 3.41 (d, *J* = 11.5 Hz, 1H), 3.31 - 3.27 (m, 1H), 2.65 - 2.55 (m, 1H), 2.44 (t, *J* = 7.4 Hz, 1H), 1.63 (s, 6H), 1.17 (s, 3H), 1.11 (s, 3H). |
| 139 | 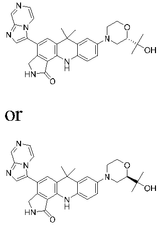 | 524 | ¹H NMR (400 MHz, DMSO-*d₆*) δ ppm: 9.09 (d, *J* = 1.3 Hz, 1H), 8.91 (s, 1H), 8.59 (s, 1H), 8.28 (dd, *J =* 4.7, 1.4 Hz, 1H), 8.04 (s, 1H), 7.89 (d, *J =* 4.7 Hz, 1H), 7.76 (s, 1H), 7.01 (d, *J* = 2.4 Hz, 1H), 6.90 (d, *J =* 8.6 Hz, 1H), 6.75 (dd, *J =* 8.7, 2.5 Hz, 1H), 4.44 (s, 1H), 4.32 (s, 2H), 3.93 (dd, *J =* 11.1, 2.3 Hz, 1H), 3.60 (dd, *J* = 11.3, 2.2 Hz, 1H), 3.50 (d, *J* = 11.7 Hz, 1H), 3.37 (d, *J* = 11.5 Hz, 1H), 3.25 (dd, *J* = 10.5, 2.0 Hz, 1H), 2.56 (dd, *J =* 11.8, 8.4 Hz, 1H), 2.41 (dd, *J =* 9.8, 5.8 Hz, 1H), 1.59 (s, 6H), 1.13 (s, 3H), 1.07 (s, 3H). |
| 140 | 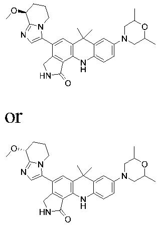 | 528 | ¹H NMR (400 MHz, DMSO-*d₆*) δ ppm: 8.83 (s, 1H), 8.57 (s, 1H), 7.58 (s, 1H), 7.21 (s, 1H), 7.04 (d, *J =* 2.0 Hz, 1H), 6.87 (d, *J* = 8.6 Hz, 1H), 6.75 (dd, *J =* 8.7, 2.2 Hz, 1H), 4.40 (d, *J =* 8.2 Hz, 1H), 4.38 - 4.26 (m, 2H), 3.96 - 3.86 (m, 2H), 3.74 - 3.66 (m, 2H), 3.50 - 3.44 (m, 5H), 2.19 (d, *J =* 11.0 Hz, 2H), 2.05 (dd, *J =* 15.6, 12.1 Hz, 2H), 1.99 - 1.83 (m, 2H), 1.60 (d, *J* = 6.8 Hz, 6H), 1.15 (d, *J =* 6.2 Hz, 6H). |
| 141 | 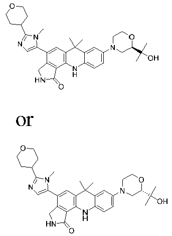 | 572 | ¹H NMR (400 MHz, DMSO-*d₆*) δ ppm: 8.79 (s, 1H), 8.51 (s, 1H), 7.46 (s, 1H), 6.99 (d, *J* = 2.2 Hz, 1H), 6.96 (s, 1H), 6.85 (d, *J* = 8.6 Hz, 1H), 6.73 (dd, *J* = 8.6, 2.4 Hz, 1H), 4.43 (s, 1H), 4.25 (s, 2H), 3.96 - 3.87 (m, 3H), 3.59 (dd, *J* = 11.3, 9.1 Hz, 1H), 3.51 - 3.41 (m, 6H), 3.35 (d, *J* = 12.2 Hz, 1H), 3.25 (dd, *J* = 10.4, 1.8 Hz, 1H), 3.08 - 3.00 (m, 1H), 2.58 - 2.51 (m, 1H), 2.40 (t, *J* = 11.0 Hz, 1H), 1.76 (d, *J* = 3.2 Hz, 4H), 1.56 (s, 6H), 1.12 (s, 3H), 1.06 (s, 3H). |
| 142 | 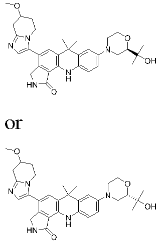 | 558 | ¹H NMR (400 MHz, DMSO-*d₆*) δ ppm: 8.78 (s, 1H), 8.51 (s, 1H), 8.13 (s, 1H), 7.48 (s, 1H), 7.02 - 6.94 (m, 2H), 6.84 (d, *J* = 8.6 Hz, 1H), 6.75 - 6.68 (m, 1H), 4.27 (s, 2H), 3.99 - 3.89 (m, 1H), 3.83 (d, *J* = 7.3 Hz, 3H), 3.65 - 3.56 (m, 1H), 3.51 - 3.45 (m, 1H), 3.35 (d, *J* = 10.3 Hz, 1H), 3.30 (s, 3H), 3.24 (s, 1H), 3.08 - 2.98 (m, 1H), 2.83 - 2.71 (m, 1H), 2.59 - 2.52 (m, 1H), 2.44 - 2.34 (m, 1H), 2.08 - 1.91 (m, 2H), 1.56 (d, *J* = 3.4 Hz, 6H), 1.13 (s, 3H), 1.06 (s, 3H). |
| 143 | 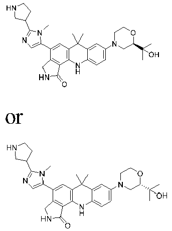 | 557 | ¹H NMR (400 MHz, DMSO-*d₆*) δ ppm: 9.38 (s, 1H), 9.30 (s, 1H), 8.99 (s, 1H), 8.69 (s, 1H), 7.78 (s, 1H), 7.60 (s, 1H), 7.15 (s, 1H), 7.00 (d, *J* = 8.6 Hz, 1H), 6.89 (d, *J* = 8.0 Hz, 1H), 4.34 (s, 2H), 4.07 - 3.99 (m, 2H), 3.77 (s, 1H), 3.72 - 3.63 (m, 4H), 3.57 - 3.41 (m, 4H), 3.34 (d, *J* = 9.9 Hz, 2H), 2.70 (d, *J* = 23.9 Hz, 1H), 2.56 (d, *J* = 13.2 Hz, 2H), 2.33 - 2.21 (m, 1H), 1.62 (s, 6H), 1.15 (s, 3H), 1.10 (s, 3H). |
| 144 | 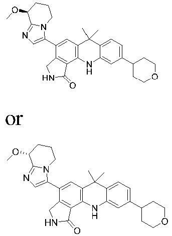 | 499 | ¹H NMR (400 MHz, DMSO-*d₆*) δ ppm: 8.96 (s, 1H), 8.61 (s, 1H), 7.59 (s, 1H), 7.36 (d, *J* = 8.0 Hz, 1H), 7.12 (s, 1H), 6.86 - 6.78 (m, 2H), 4.39 - 4.33 (m, 3H), 3.98 - 3.82 (m, 4H), 3.44 (s, 3H), 3.41 (dd, *J* = 10.7, 3.7 Hz, 2H), 2.74 - 2.65 (m, 1H), 2.12 - 1.98 (m, 2H), 1.98 - 1.80 (m, 2H), 1.73 - 1.64 (m, 4H), 1.59 (s, 3H), 1.57 (s, 3H). |
| 145 | 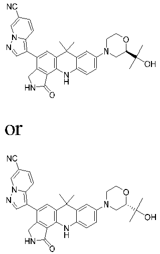 | 549 | ¹H NMR (400 MHz, DMSO-*d₆*) δ ppm: 9.61 (s, 1H), 8.82 (s, 1H), 8.59 (s, 1H), 8.55 (s, 1H), 7.79 (d, *J* = 9.3 Hz, 1H), 7.61 (s, 1H), 7.47 - 7.44 (m, 1H), 7.00 (d, *J* = 2.3 Hz, 1H), 6.87 (d, *J* = 8.6 Hz, 1H), 6.75 - 6.72 (m, 1H), 4.44 (s, 1H), 4.39 (s, 2H), 3.95 - 3.92 (m, 1H), 3.63 - 3.58 (m, 1H), 3.49 (d, *J =* 11.4 Hz, 1H), 3.36 (d, *J* = 11.5 Hz, 1H), 3.27 - 3.22 (m, 1H), 2.59 - 2.52 (m, 1H), 2.43 - 2.38 (m, 1H), 1.59 (s, 6H), 1.13 (s, 3H), 1.06 (s, 3H). |
| 146 | 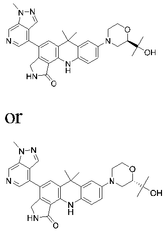 | 539 | ¹H NMR (400 MHz, DMSO-*d₆*) δ ppm: 9.16 (s, 1H), 8.97 (s, 1H), 8.61 (s, 1H), 8.40 (s, 1H), 8.12 (s, 1H), 7.74 (s, 1H), 7.06 (d, *J* = 2.3 Hz, 1H), 6.93 (d, *J =* 8.6 Hz, 1H), 6.79 (dd, *J =* 8.7, 2.4 Hz, 1H), 4.49 (s, 1H), 4.43 (s, 2H), 4.23 (s, 3H), 3.98 (dd, *J =* 10.9, 2.3 Hz, 1H), 3.64 (dd, *J =* 11.0, 5.6 Hz, 1H), 3.54 (d, *J* = 11.5 Hz, 1H), 3.41 (d, *J* = 11.6 Hz, 1H), 3.28 (d, *J* = 1.9 Hz, 1H), 2.60 (dd, *J =* 11.6, 3.1 Hz, 1H), 2.44 (d, *J =* 11.0 Hz, 1H), 1.64 (s, 6H), 1.17 (s, 3H), 1.11 (s, 3H). |
| 147 | 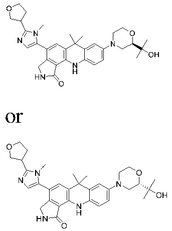 | 558 | ¹H NMR (400 MHz, DMSO-*d₆*) δ ppm: 8.55 (s, 1H), 8.26 (s, 1H), 7.50 (s, 1H), 7.03 (d, *J =* 2.0 Hz, 1H), 6.96 (s, 1H), 6.89 (d, *J* = 8.6 Hz, 1H), 6.77 (dd, *J* = 8.6, 2.3 Hz, 1H), 4.47 (s, 1H), 4.30 (s, 2H), 4.08 (t, *J =* 7.8 Hz, 1H), 3.97 (d, *J =* 8.6 Hz, 1H), 3.92 - 3.79 (m, 3H), 3.67 - 3.57 (m, 2H), 3.55 (d, *J =* 8.7 Hz, 1H), 3.50 (s, 3H), 3.39 (d, *J =* 11.3 Hz, 1H), 3.29 (d, *J =* 10.3 Hz, 1H), 2.64 - 2.55 (m, 1H), 2.43 (d, *J =* 11.0 Hz, 1H), 2.30 - 2.21 (m, 2H), 1.60 (s, 6H), 1.16 (s, 3H), 1.10 (s, 3H). |
| 148 | 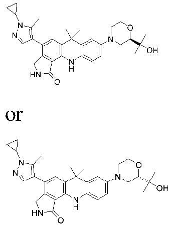 | 528 | ¹H NMR (400 MHz, DMSO-*d₆*) δ ppm: 8.72 (s, 1H), 8.49 (s, 1H), 7.52 (s, 1H), 7.34 (s, 1H), 6.99 (d, *J =* 2.2 Hz, 1H), 6.83 (d, *J* = 8.6 Hz, 1H), 6.72 (dd, *J* = 8.7, 2.4 Hz, 1H), 4.43 (s, 1H), 4.27 (s, 2H), 3.93 (dd, *J =* 11.0, 2.2 Hz, 1H), 3.66 - 3.55 (m, 2H), 3.49 (m, 1H), 3.34 (d, *J* = 11.5 Hz, 1H), 3.25 (dd, *J* = 10.5, 1.9 Hz, 1H), 2.55 - 2.50 (m, 1H), 2.40 (t, *J =* 11.0 Hz, 1H), 2.35 (s, 3H), 1.55 (s, 6H), 1.12 (s, 3H), 1.06 (s, 3H), 1.03 (dd, *J* = 5.9, 3.2 Hz, 2H), 1.01 - 0.96 (m, 2H). |
| 149 | 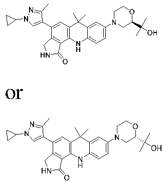 | 528 | ¹H NMR (400 MHz, DMSO-*d₆*) δ ppm: 8.71 (s, 1H), 8.52 (s, 1H), 7.88 (s, 1H), 7.40 (s, 1H), 6.98 (d, *J =* 2.3 Hz, 1H), 6.82 (d, *J* = 8.6 Hz, 1H), 6.72 (dd, *J* = 8.7, 2.4 Hz, 1H), 4.43 (s, 1H), 4.29 (s, 2H), 3.93 (dd, *J =* 11.0, 2.2 Hz, 1H), 3.64 - 3.55 (m, 2H), 3.47 (d, *J =* 11.4 Hz, 1H), 3.34 (d, *J =* 11.3 Hz, 1H), 3.25 (dd, *J =* 10.4, 1.9 Hz, 1H), 2.55 - 2.50 (m, 1H), 2.39 (t, *J* = 11.1 Hz, 1H), 2.18 (s, 3H), 1.53 (s, 6H), 1.12 (s, 3H), 1.06 (s, 3H), 1.00 - 0.95 (m, 2H), 0.90 - 0.85 (m, 2H). |
| 150 | 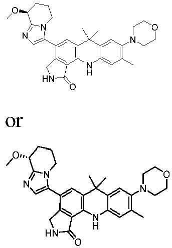 | 514 | ¹H NMR (400 MHz, DMSO-*d₆*) δ ppm: 8.83 (s, 1H), 8.57 (s, 1H), 8.17 (s, 1H), 7.56 (s, 1H), 7.11 (s, 2H), 6.78 (s, 1H), 4.41 - 4.25 (m, 3H), 3.89 (d, *J =* 2.7 Hz, 2H), 3.75 - 3.69 (m, 4H), 3.44 (s, 3H), 2.84 - 2.78 (m, 4H), 2.20 (s, 3H), 2.08 (t, *J =* 6.5 Hz, 2H), 1.93 (dd, *J =* 27.6, 17.3 Hz, 2H), 1.59 (d, *J =* 8.1 Hz, 6H). |
| 151 | 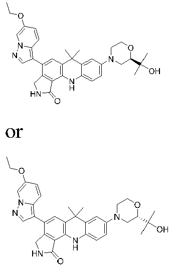 | 568 | ¹H NMR (400 MHz, DMSO-*d₆*) δ ppm: 8.82 (s, 1H), 8.60 (s, 1H), 8.43 (d, *J* = 1.7 Hz, 1H), 8.17 (s, 1H), 7.64 - 7.62 (m, 2H), 7.13 - 7.10 (m, 1H), 7.04 (d, *J =* 2.0 Hz, 1H), 6.89 (d, *J =* 8.6 Hz, 1H), 6.79 - 6.76 (m, 1H), 4.48 (s, 1H), 4.43 (s, 2H), 4.12 - 4.07 (m, 2H), 3.97 (d, *J =* 8.7 Hz, 1H), 3.67 - 3.62 (m, 1H), 3.53 (d, *J =* 11.1 Hz, 1H), 3.39 (d, *J =* 11.3 Hz, 1H), 3.30 (d, *J* = 10.4 Hz, 1H), 2.62 - 2.57 (m, 1H), 2.43 (d, *J* = 11.1 Hz, 1H), 1.63 (s, 6H), 1.39 - 1.36 (m, 3H), 1.15 (s, 3H), 1.10 (s, 3H). |
| 152 | 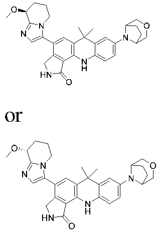 | 526 | ¹H NMR (400 MHz, DMSO-*d₆*) δ ppm: 8.74 (s, 1H), 8.50 (s, 1H), 8.10 (s, 1H), 7.51 (s, 1H), 7.08 (s, 1H), 6.88 (s, 1H), 6.80 (s, 1H), 6.64 (s, 1H), 4.41 - 4.25 (m, 3H), 4.13 - 4.00 (m, 2H), 3.89 - 3.80 (m, 2H), 3.72 - 3.63 (m, 2H), 3.45 - 3.35 (m, 5H), 2.07 - 1.77 (m, 8H), 1.55 (d, *J =* 8.0 Hz, 6H). |
| 153 | 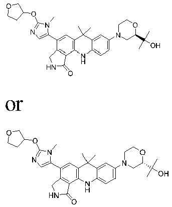 | 574 | ¹H NMR (400 MHz, DMSO-*d₆*) δ ppm: 8.81 (s, 1H), 8.56 (s, 1H), 7.49 (s, 1H), 7.03 (d, *J* = 2.1 Hz, 1H), 6.88 (d, *J* = 8.6 Hz, 1H), 6.76 (dd, *J* = 8.7, 2.3 Hz, 1H), 6.69 (s, 1H), 5.40 - 5.34 (m, 1H), 4.59 - 4.38 (m, 1H), 4.33 (s, 2H), 4.01 - 3.94 (m, 1H), 3.93 - 3.83 (m, 3H), 3.83 - 3.75 (m, 1H), 3.68 - 3.59 (m, 1H), 3.52 (d, *J* = 11.4 Hz, 1H), 3.40 - 3.37 (m, 1H), 3.30 (s, 3H), 3.28 - 3.25 (m, 1H), 2.64 - 2.54 (m, 1H), 2.47 - 2.40 (m, 1H), 2.30 - 2.19 (m, 1H), 2.14 - 2.05 (m, 1H), 1.60 (s, 6H), 1.16 (s, 3H), 1.10 (s, 3H). |
| 154 | 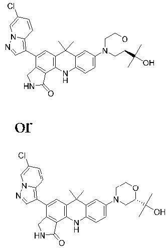 | 558 | ¹H NMR (400 MHz, DMSO-*d₆*) δ ppm: 9.05 (s, 1H), 8.80 (s, 1H), 8.57 (s, 1H), 8.32 (s, 1H), 7.70 (d, *J =* 9.5 Hz, 1H), 7.60 (s, 1H), 7.31 (dd, *J* = 9.5, 1.7 Hz, 1H), 7.00 (d, *J* = 2.2 Hz, 1H), 6.86 (d, *J =* 8.6 Hz, 1H), 6.74 (dd, *J =* 8.6, 2.4 Hz, 1H), 4.44 (s, 1H), 4.39 (s, 2H), 3.93 (dd, *J* = 11.0, 2.2 Hz, 1H), 3.60 (dd, *J* = 11.4, 9.1 Hz, 1H), 3.49 (d, *J* = 11.4 Hz, 1H), 3.36 (d, *J =* 11.3 Hz, 1H), 3.24 (d, *J* = 1.9 Hz, 1H), 2.65 - 2.55 (m, 1H), 2.41 (t, *J =* 11.0 Hz, 1H), 1.59 (s, 6H), 1.13 (s, 3H), 1.06 (s, 3H). |
| 155 | 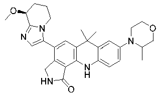 or | 514 | ¹H NMR (400 MHz, DMSO-*d₆*) δ ppm: 8.86 (s, 1H), 8.57 (s, 1H), 7.57 (s, 1H), 7.16 (s, 1H), 7.03 (s, 1H), 6.89 (d, *J* = 8.5 Hz, 1H), 6.77 (d, *J* = 8.5 Hz, 1H), 4.41 - 4.35 (m, 3H), 3.89 (d, *J* = 12.9 Hz, 2H), 3.84 - 3.78 (m, 1H), 3.75 (d, *J =* 8.6 Hz, 1H), 3.66 - 3.60 (m, 1H), 3.53 (dd, *J =* 18.5, 7.8 Hz, 2H), 3.45 (s, 3H), 2.99 (s, 2H), 2.10 - 1.83 (m, 4H), 1.59 (m, *J =* 8.2, 5.5 Hz, 6H), 0.88 (d, *J =* 6.3 Hz, 3H). |
| | 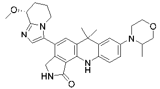 | | |
| 156 | 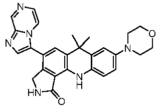 | 467 | ¹H NMR (400 MHz, DMSO-*d₆*) δ ppm: 9.13 (s, 1H), 8.95 (s, 1H), 8.63 (s, 1H), 8.32 (d, *J =* 4.5 Hz, 1H), 8.08 (s, 1H), 7.93 (d, *J* = 4.7 Hz, 1H), 7.80 (s, 1H), 7.06 (d, *J* = 1.8 Hz, 1H), 6.93 (d, *J* = 8.6 Hz, 1H), 6.79 - 6.76 (m, 1H), 4.36 (s, 2H), 3.77 - 3.72 (m, 4H), 3.06 - 3.01 (m, 4H), 1.63 (s, 6H). |
| 157 | 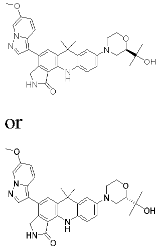 | 554 | ¹H NMR (400 MHz, DMSO-*d₆*) δ ppm: 8.82 (s, 1H), 8.59 (s, 1H), 8.45 (d, *J* = 2.0 Hz, 1H), 8.18 (s, 1H), 7.63 - 7.59 (m, 2H), 7.14 - 7.11 (m, 1H), 7.04 (d, *J =* 2.5 Hz, 1H), 6.89 (d, *J =* 8.6 Hz, 1H), 6.79 - 6.76 (m, 1H), 4.48 (s, 1H), 4.43 (s, 2H), 3.99 - 3.96 (m, 1H), 3.85 (s, 3H), 3.67 - 3.62 (m, 1H), 3.53 (d, *J* = 11.4 Hz, 1H), 3.39 (d, *J* = 11.8 Hz, 1H), 3.31 - 3.28 (m, 1H), 2.63 - 2.56 (m, 1H), 2.47 - 2.42 (m, 1H), 1.63 (s, 6H), 1.17 (s, 3H), 1.10 (s, 3H). |
| 158 | 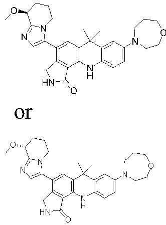 | 514 | ¹H NMR (400 MHz, DMSO-*d₆*) δ ppm: 8.72 (s, 1H), 8.52 (s, 1H), 7.54 (s, 1H), 7.09 (s, 1H), 6.84 - 6.79 (m, 2H), 6.58 (dd, *J =* 8.7, 2.5 Hz, 1H), 4.41 - 4.24 (m, 3H), 3.96 - 3.82 (m, 2H), 3.71 (t, *J* = 4.7 Hz, 2H), 3.55 - 3.49 (m, 6H), 3.44 (s, 3H), 2.06 (s, 2H), 2.14 - 1.98 (m, 2H), 1.97 - 1.81(m, 2H), 1.60 (d, *J* = 7.9 Hz, 6H). |
| 159 | 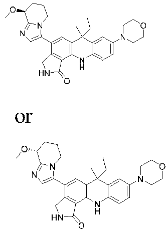 | 514 | ¹H NMR (400 MHz, DMSO-*d₆*) δ ppm: 8.76 (s, 1H), 8.53 (s, 1H), 7.48 (s, 1H), 7.11 (s, 1H), 6.97 (d, *J =* 1.9 Hz, 1H), 6.85 (d, *J* = 8.6 Hz, 1H), 6.74 (dd, *J =* 8.6, 2.2 Hz, 1H), 4.48 - 4.19 (m, 3H), 3.96 - 3.81 (m, 2H), 3.79 - 3.66 (m, 4H), 3.45 (s, 3H), 3.09 - 2.94 (m, 4H), 2.13 - 1.80 (m, 6H), 1.63 (d, *J =* 8.9 Hz, 3H), 0.55 - 0.37 (m, 3H). |
| 160 | 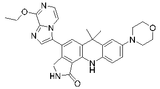 | 511 | ¹H NMR (400 MHz, DMSO-*d₆*) δ ppm: 8.94 (s, 1H), 8.60 (s, 1H), 7.91 - 7.84 (m, 2H), 7.71 (s, 1H), 7.44 (d, *J* = 4.7 Hz, 1H), 7.06 (d, *J* = 2.2 Hz, 1H), 6.93 (d, *J* = 8.6 Hz, 1H), 6.77 (dd, *J* = 8.6, 2.4 Hz, 1H), 4.55 - 4.48 (m, 2H), 4.33 (s, 2H), 3.78 - 3.71 (m, 4H), 3.08 - 3.01 (m, 4H), 1.62 (s, 6H), 1.43 (t, *J* = 7.0 Hz, 3H). |
| 161 | 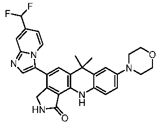 | 516 | ¹H NMR (400 MHz, DMSO-*d₆*) δ ppm: 8.89 (s, 1H), 8.57 (s, 1H), 8.34 (d, *J =* 7.2 Hz, 1H), 7.89 - 7.85 (m, 2H), 7.73 (s, 1H), 7.30 - 6.94 (m, 3H), 6.89 (d, *J =* 8.6 Hz, 1H), 6.76 - 6.71 (m, 1H), 4.28 (s, 2H), 3.75 - 3.67 (m, 4H), 3.04 - 2.96 (m, 4H), 1.58 (s, 6H). |
| 162 | 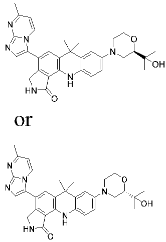 | 539 | ¹H NMR (400 MHz, DMSO-*d₆*) δ ppm: 8.91 (s, 1H), 8.60 - 8.54 (m, 2H), 7.82 (s, 1H), 7.73 (s, 1H), 7.04 (d, *J* = 2.3 Hz, 1H), 6.98 (d, *J* = 7.0 Hz, 1H), 6.93 (d, *J* = 8.6 Hz, 1H), 6.80 - 6.77 (m, 1H), 4.46 (s, 1H), 4.33 (s, 2H), 3.97 (d, *J =* 8.4 Hz, 1H), 3.67 - 3.62(m, 1H), 3.54 (d, *J* = 11.4 Hz, 1H), 3.40 (d, *J* = 11.3 Hz, 1H), 3.28 (s, 1H), 2.66 - 2.58 (m, 1H), 2.56 (s, 3H), 2.43 (d, *J =* 10.9 Hz, 1H), 1.62 (s, 6H), 1.15 (s, 3H), 1.10 (s, 3H). |

The synthetic methods of the following examples refer to example 10 and example 13:

| Example | Structure | MS:M+ H⁺ | ¹H NMR |
|---|---|---|---|
| 163 | 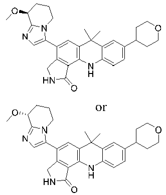 | 499 | ¹H NMR (400 MHz, DMSO-*d₆*) δ ppm: 8.88 (s, 1H), 8.56 (s, 1H), 7.54 (s, 1H), 7.27 (d, *J* = 1.4 Hz, 1H), 7.08 (s, 1H), 6.95 (dd, *J* = 8.2, 1.7 Hz, 1H), 6.85 (d, *J* = 8.1 Hz, 1H), 4.37 - 4.23 (m, 3H), 3.93 - 3.82 (m, 4H), 3.45 - 3.35 (m, 5H), 2.71 - 2.63 (m, 1H), 2.08 - 1.81 (m, 4H), 1.67 - 1.59 (m, 4H), 1.57 (s, 3H), 1.55(s, 3H). |
| 164 | 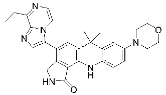 | 495 | ¹H NMR (400 MHz, DMSO-*d₆*) δ ppm: 8.94 (s, 1H), 8.63 (s, 1H), 8.16 (d, *J =* 4.7 Hz, 1H), 7.98 (s, 1H), 7.82 (d, *J =* 4.7 Hz, 1H), 7.77 (s, 1H), 7.06 (d, *J =* 2.4 Hz, 1H), 6.93 (d, *J =* 8.6 Hz, 1H), 6.77 (dd, *J =* 8.7, 2.5 Hz, 1H), 4.36 (s, 2H), 3.79 - 3.68 (m, 4H), 3.20 (q, *J =* 7.5 Hz, 2H), 3.08 - 2.98 (m, 4H), 1.62 (s, 6H), 1.37 (t, *J* = 7.5 Hz, 3H). |
| 165 | 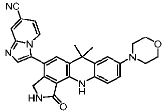 | 491 | ¹H NMR (400 MHz, DMSO-*d₆*) δ ppm: 8.93 (s, 1H), 8.59 (s, 1H), 8.41 (s, 1H), 8.36 (d, *J =* 7.2 Hz, 1H), 8.08 (s, 1H), 7.75 (s, 1H), 7.24 - 7.17 (m, 1H), 7.06 (s, 1H), 6.91 (d, *J* = 10.7 Hz, 1H), 6.78 (s, 1H), 4.29 (s, 2H), 3.78 - 3.69 (m, 4H), 3.15 - 3.02 (m, 4H), 1.58 (s, 6H). |
| 166 | 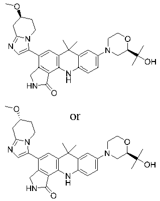 | 558 | ¹H NMR (400 MHz, DMSO-*d₆*) δ ppm: 8.82 (s, 1H), 8.55 (s, 1H), 7.52 (s, 1H), 7.07 - 6.98 (m, 2H), 6.88 (d, *J =* 8.6 Hz, 1H), 6.77 (dd, *J =* 8.7, 2.4 Hz, 1H), 4.47 (s, 1H), 4.31 (s, 2H), 4.03 - 3.80 (m, 4H), 3.68 - 3.51 (m, 2H), 3.42 - 3.36 (m, 1H), 3.34 (s, 3H), 3.32 - 3.26 (m, 1H), 3.06 (dd, *J* = 16.7, 4.6 Hz, 1H), 2.83 (dd, *J =* 16.7, 5.7 Hz, 1H), 2.65 - 2.55 (m, 1H), 2.50 - 2.40 (m, 1H), 2.12 - 1.98 (m, 2H), 1.60 (d, *J =* 4.4 Hz, 6H), 1.13 (d, *J* = 24.8 Hz, 6H). |
| 167 | 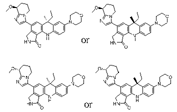 | 514 | ¹H NMR (400 MHz, DMSO-*d₆*) δ ppm: 8.75 (s, 1H), 8.54 (s, 1H), 7.48 (s, 1H), 7.12 (s, 1H), 6.97 (d, *J* = 2.3 Hz, 1H), 6.85 (d, *J* = 8.6 Hz, 1H), 6.74 (dd, *J* = 8.7, 2.4 Hz, 1H), 4.42 - 4.25 (m, 3H), 3.91 - 3.85 (m, 2H), 3.77 - 3.70 (m, 4H), 3.44 (s, 3H), 3.06 - 2.98 (m, 4H), 2.10 - 1.82 (m, 6H), 1.64 (s, 3H), 0.53 (t, *J* = 7.2 Hz, 3H). |
| 168 | 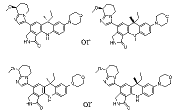 | 514 | ¹H NMR (400 MHz, DMSO-*d₆*) δ ppm: 8.75 (s, 1H), 8.54 (s, 1H), 7.48 (s, 1H), 7.11 (s, 1H), 6.97 (d, *J* = 2.3 Hz, 1H), 6.85 (d, *J* = 8.6 Hz, 1H), 6.74 (dd, *J* = 8.7, 2.4 Hz, 1H), 4.40 - 4.27 (m, 3H), 3.93 - 3.83 (m, 2H), 3.79 - 3.69 (m, 4H), 3.44 (s, 3H), 3.08 - 2.97 (m, 4H), 2.11 - 1.80 (m, 6H), 1.62 (s, 3H), 0.55 (t, *J* = 7.2 Hz, 3H). |
| 169 | 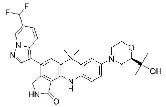 | 574 | ¹H NMR (400 MHz, DMSO-*d₆*) δ ppm: 9.12 (s, 1H), 8.85 (s, 1H), 8.62 (s, 1H), 8.46 (s, 1H), 7.85 (d, *J =* 9.3 Hz, 1H), 7.67 (s, 1H), 7.45 (d, *J* = 9.3 Hz, 1H), 7.30 - 6.98 (m, 2H), 6.91 (d, *J =* 8.6 Hz, 1H), 6.79 (dd, *J* = 8.7, 2.4 Hz, 1H), 4.48 (s, 1H), 4.45 (s, 2H), 3.98 (dd, *J* = 10.9, 2.3 Hz, 1H), 3.65 (dd, *J =* 11.5, 9.0 Hz, 1H), 3.54 (d, *J* = 11.3 Hz, 1H), 3.41 (d, *J* = 11.5 Hz, 1H), 3.30 (dd, *J* = 10.5, 2.1 Hz, 1H), 2.69 - 2.57 (m, 1H), 2.49 - 2.43 (m, 1H), 1.65 (s, 6H), 1.18 (s, 3H), 1.11 (s, 3H). |
| 170 | 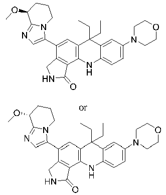 | 528 | ¹H NMR (400 MHz, DMSO-*d₆*) δ ppm: 8.69 (s, 1H), 8.54 (s, 1H), 7.42 (s, 1H), 7.13 (s, 1H), 6.92 (d, *J* = 2.2 Hz, 1H), 6.83 (d, *J* = 8.6 Hz, 1H), 6.73 (dd, *J* = 8.6, 2.4 Hz, 1H), 4.43 - 4.25 (m, 3H), 3.89 - 3.83 (m, 2H), 3.80 - 3.63 (m, 4H), 3.44 (s, 3H), 3.07 - 2.94 (m, 4H), 2.13 - 1.72 (m, 8H), 0.57 - 0.50 (m, 6H). |
| 171 | 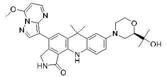 | 555 | ¹H NMR (400 MHz, DMSO-*d₆*) δ ppm: 8.85 (s, 1H), 8.62 (s, 1H), 8.57 (d, *J =* 4.9 Hz, 1H), 8.47 (s, 1H), 8.29 (s, 1H), 7.04 (s, 1H), 6.88 (d, *J* = 8.5 Hz, 1H), 6.78 (d, *J* = 8.4 Hz, 1H), 6.71 (d, *J* = 5.1 Hz, 1H), 4.57 (s, 2H), 4.53 - 4.45 (m, 1H), 4.24 (s, 3H), 3.99- 3.96 (m, 1H), 3.69 - 3.60 (m, 1H), 3.54 - 3.52 (m, 2H), 3.31 - 3.29 (m, 1H), 2.62 - 2.57 (m, 1H), 2.46 - 2.42 (m, 1H), 1.63 (s, 6H), 1.17 (s, 3H), 1.11 (s, 3H). |
| 172 | 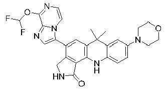 | 533 | ¹H NMR (400 MHz, DMSO-*d₆*) δ ppm: 8.92 (s, 1H), 8.59 (s, 1H), 8.11 (d, *J =* 4.8 Hz, 1H), 8.00 (s, 1H), 7.92 (t, *J =* 71.8 Hz, 1H), 7.73 (d, *J* = 4.2 Hz, 1H), 7.50 (d, *J* = 4.8 Hz, 1H), 7.02 (d, *J =* 2.3 Hz, 1H), 6.90 (d, *J* = 8.6 Hz, 1H), 6.74 (dd, *J* = 8.7, 2.5 Hz, 1H), 4.31 (s, 2H), 3.74 - 3.65 (m, 4H), 3.04 - 2.96 (m, 4H), 1.58 (s, 6H). |
| 173 | 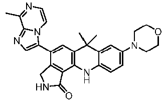 | 481 | ¹H NMR (400 MHz, DMSO-*d₆*) δ ppm: 8.94 (s, 1H), 8.62 (s, 1H), 8.16 (d, *J =* 4.7 Hz, 1H), 7.98 (s, 1H), 7.82 - 7.71 (m, 2H), 7.06 (d, *J =* 2.3 Hz, 1H), 6.93 (d, *J =* 8.6 Hz, 1H), 6.78 (dd, *J =* 8.7, 2.5 Hz, 1H), 4.35 (s, 2H), 3.84 - 3.69 (m, 4H), 3.12 - 2.98 (m, 4H), 2.79 (s, 3H), 1.62 (s, 6H). |
| 174 | 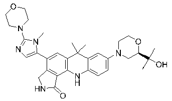 | 573 | ¹H NMR (400 MHz, DMSO-*d₆*) δ ppm: 8.81 (s, 1H), 8.56 (s, 1H), 7.52 (s, 1H), 7.03 (d, *J =* 2.2 Hz, 1H), 6.88 (d, *J =* 8.6 Hz, 1H), 6.83 (s, 1H), 6.76 (dd, *J =* 8.6, 2.4 Hz, 1H), 4.47 (s, 1H), 4.35 (s, 2H), 3.97 (d, *J =* 8.6 Hz, 1H), 3.87 - 3.69 (m, 4H), 3.68 - 3.60 (m, 1H), 3.52 (d, *J* = 11.3 Hz, 1H), 3.40 (s, 3H), 3.38 - 3.33 (m, 1H), 3.30 - 3.25 (m, 1H), 3.14 - 2.96 (m, 4H), 2.62 - 2.56 (m, 1H), 2.47 - 2.40 (m, 1H), 1.61 (s, 6H), 1.13 (d, *J =* 24.6 Hz, 6H). |
| 175 | 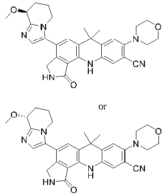 | 525 | ¹H NMR (400 MHz, DMSO-*d₆*) δ ppm: 9.08 (s, 1H), 8.58 (s, 1H), 7.56 (s, 1H), 7.52 (s, 1H), 7.21 (s, 1H), 7.09 (s, 1H), 4.38 - 4.24 (m, 3H), 3.88 - 3.78 (m, 2H), 3.76 - 3.67 (m, 4H), 3.40 (s, 3H), 3.05 - 3.00 (m, 4H), 2.02 - 1.78 (m, 4H), 1.59 (d, *J =* 8.3 Hz, 6H). |
| 176 | 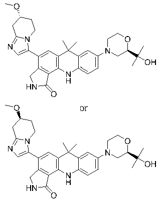 | 558 | ¹H NMR (400 MHz, DMSO-*d₆*) δ ppm:8.82 (s, 1H), 8.55 (s, 1H), 7.52 (s, 1H), 7.07 -- 6.98 (m, 2H), 6.89 (d, *J* = 8.6 Hz, 1H), 6.77 (dd, *J =* 8.7, 2.2 Hz, 1H), 4.48 (s, 1H), 4.31 (s, 2H), 4.01 - 3.82 (m, 4H), 3.64 (t, *J =* 10.3 Hz, 1H), 3.52 (d, *J =* 11.7 Hz, 1H), 3.42 - 3.36 (m, 1H), 3.34 (s, 3H), 3.32 - 3.26 (m, 1H), 3.07 (d, *J =* 12.9 Hz, 1H), 2.84 (dd, *J =* 16.6, 5.4 Hz, 1H), 2.65 - 2.55 (m, 1H), 2.50 - 2.40 (m, 1H), 2.12 - 1.98 (m, 2H), 1.60 (d, *J =* 4.4 Hz, 6H), 1.13 (d, *J =* 24.9 Hz, 6H). |
| 177 | 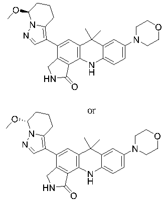 | 500 | ¹H NMR (400 MHz, DMSO-*d₆*) δ ppm: 8.77 (s, 1H), 8.55 (s, 1H), 7.75 (s, 1H), 7.46 (s, 1H), 7.03 (d, *J* = 2.3 Hz, 1H), 6.85 (d, *J =* 8.6 Hz, 1H), 6.75 (dd, *J* = 8.6, 2.5 Hz, 1H), 5.37 (t, *J =* 2.9 Hz, 1H), 4.47 (d, *J* = 17.8 Hz, 1H), 4.31 (d, *J* = 17.6 Hz, 1H), 3.81 - 3.67 (m, 4H), 3.52 (s, 3H), 3.08 - 2.96 (m, 4H), 2.89 (dd, *J* = 7.8, 4.6 Hz, 2H), 2.14 (d, *J* = 13.3 Hz, 1H), 2.06 - 1.79 (m, 3H), 1.59 (d, *J =* 15.8 Hz, 6H). |
| 178 | 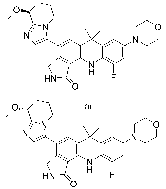 | 518 | ¹H NMR (400 MHz, DMSO-*d₆*) δ ppm: 8.93 (s, 1H), 8.65 (s, 1H), 7.61 (s, 1H), 7.13 (s, 1H), 6.90 (s, 1H), 6.80 (dd, *J =* 13.7, 2.1 Hz, 1H), 4.43 - 4.32 (m, 3H), 3.89 (d, *J* = 12.3 Hz, 2H), 3.79 -3.67 (m, 4H), 3.44 (s, 3H), 3.12 - 3.00 (m, 4H), 2.03 - 1.95 (m, 4H), 1.62 (d, *J =* 8.3 Hz, 6H). |
| 179 | 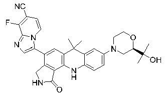 | 567 | ¹H NMR (400 MHz, DMSO-*d₆*) δ ppm: 8.93 (s, 1H), 8.59 (s, 1H), 8.20 (d, *J* = 7.2 Hz, 1H), 8.10 (s, 1H), 7.73 (s, 1H), 7.23 - 7.18 (m, 1H), 7.01 (d, *J* = 2.3 Hz, 1H), 6.91 (d, *J* = 8.6 Hz, 1H), 6.75 (dd, *J* = 8.7, 2.4 Hz, 1H), 4.43 (s, 1H), 4.29 (s, 2H), 3.94 (dd, *J* = 11.1, 2.2 Hz, 1H), 3.65 - 3.55 (m, 1H), 3.50 (d, *J* = 11.8 Hz, 1H), 3.37 (d, *J* = 11.1 Hz, 1H), 3.25 (dd, *J* = 10.6, 2.0 Hz, 1H), 2.56 - 2.53 (m, 1H), 2.49 - 2.37 (m, 1H), 1.58 (s, 6H), 1.13 (s, 3H), 1.07 (s, 3H). |
| 180 | 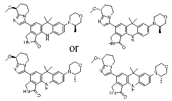 | 514 | ¹H NMR (400 MHz, DMSO-*d₆*) δ ppm: 8.86 (s, 1H), 8.57 (s, 1H), 7.57 (s, 1H), 7.17 (s, 1H), 7.04 (s, 1H), 6.89 (d, *J =* 8.4 Hz, 1H), 6.77 (d, *J* = 8.4 Hz, 1H), 4.45 - 4.25 (m, 3H), 4.01 - 3.70 (m, 4H), 3.69 - 3.48 (m, 3H), 3.45 (s, 3H), 3.05 - 2.90 (m, 2H), 2.20 - 1.80 (m, 4H), 1.64 - 1.54 (m, 6H), 0.88 (d, *J* = 6.3 Hz, 3H). |
| 181 | 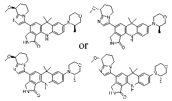 | 514 | ¹H NMR (400 MHz, DMSO-*d₆*) δ ppm: 8.85 (s, 1H), 8.57 (s, 1H), 7.57 (s, 1H), 7.17 (s, 1H), 7.03 (s, 1H), 6.89 (d, *J =* 8.5 Hz, 1H), 6.77 (d, *J* = 8.5 Hz, 1H), 4.44 - 4.24 (m, 3H), 3.96 - 3.71 (m, 4H), 3.69 - 3.48 (m, 3H), 3.45 (s, 3H), 3.05 - 2.90 (m, 2H), 2.20 - 1.83 (m, 4H), 1.64 - 1.54 (m, 6H), 0.88 (d, *J* = 6.2 Hz, 3H). |
| 182 | 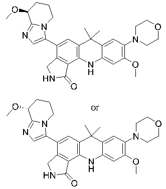 | 530 | ¹H NMR (400 MHz, DMSO-*d₆*) δ ppm: 8.90 (s, 1H), 8.60 (s, 1H), 7.65 (s, 1H), 7.63 (s, 1H), 6.89 (s, 1H), 6.70 (s, 1H), 4.64 (s, 1H), 4.32 (s, 2H), 4.03 - 3.85 (m, 2H), 3.74 (s, 3H), 3.70 - 3.63 (m, 4H), 3.44 (s, 3H), 2.95 - 2.85 (m, 4H), 2.08 - 1.85 (m, 4H), 1.54 (s, 6H). |
| 183 | 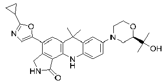 | 515 | ¹H NMR (400 MHz, DMSO-*d₆*) δ ppm: 8.92 (s, 1H), 8.70 (s, 1H), 7.79 (s, 1H), 7.33 (s, 1H), 7.04 (d, *J* = 2.3 Hz, 1H), 6.90 (d, *J =* 8.6 Hz, 1H), 6.77 (dd, *J* = 8.6, 2.4 Hz, 1H), 4.52 - 4.46 (m, 3H), 3.97 (d, *J* = 8.6 Hz, 1H), 3.64 (dd, *J* = 11.4, 9.1 Hz, 1H), 3.53 (d, *J* = 11.6 Hz, 1H), 3.40 (d, *J* = 11.5 Hz, 1H), 3.29 (dd, *J =* 10.5, 1.9 Hz, 1H), 2.62 - 2.55 (m, 1H), 2.43 (d, *J =* 11.0 Hz, 1H), 2.24 - 2.14 (m, 1H), 1.61 (s, 6H), 1.17 (s, 3H), 1.10 (s, 3H), 1.08 - 1.00 (m, 4H). |
| 184 | 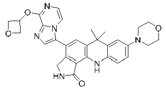 | 539 | ¹H NMR (400 MHz, DMSO-*d₆*) δ ppm: 8.94 (s, 1H), 8.62 (s, 1H), 7.97 - 7.90 (m, 2H), 7.73 (s, 1H), 7.40 (d, *J* = 4.8 Hz, 1H), 7.05 (d, *J* = 2.4 Hz, 1H), 6.93 (d, *J* = 8.6 Hz, 1H), 6.77 (dd, *J =* 8.7, 2.5 Hz, 1H), 5.83 - 5.75 (m, 1H), 4.98 (t, *J* = 7.0 Hz, 2H), 4.73 - 4.67 (m, 2H), 4.35 (s, 2H), 3.80 - 3.68 (m, 4H), 3.10 - 2.98 (m, 4H), 1.62 (s, 6H). |
| 185 | 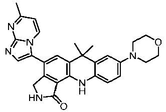 | 481 | ¹H NMR (400 MHz, DMSO-*d₆*) δ ppm: 8.91 (s, 1H), 8.68 - 8.52 (m, 2H), 7.86 (s, 1H), 7.73 (s, 1H), 7.13 - 6.99 (m, 2H), 6.92 (d, *J* = 8.6 Hz, 1H), 6.77 (d, *J =* 8.0 Hz, 1H), 4.33 (s, 2H), 3.87 - 3.64 (m, 4H), 3.15 - 2.85 (m, 4H), 2.57 (s, 3H), 1.61 (s, 6H). |
| 186 | 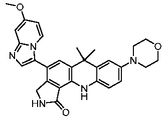 | 496 | ¹H NMR (400 MHz, DMSO-*d₆*) δ ppm: 8.85 (s, 1H), 8.55 (s, 1H), 8.06 (d, *J* = 7.5 Hz, 1H), 7.63 (s, 1H), 7.57 (s, 1H), 7.00 (dd, *J =* 12.3, 2.4 Hz, 2H), 6.87 (d, *J =* 8.6 Hz, 1H), 6.73 (dd, *J* = 8.7, 2.5 Hz, 1H), 6.62 (dd, *J =* 7.5, 2.5 Hz, 1H), 4.26 (s, 2H), 3.82 (s, 3H), 3.76 - 3.64 (m, 4H), 3.05 - 2.95 (m, 4H), 1.57 (s, 6H). |
| 187 | 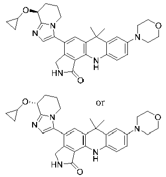 | 526 | ¹H NMR (400 MHz, DMSO-*d₆*) δ ppm: 8.92 (s, 1H), 8.64 (s, 1H), 7.84 (s, 1H), 7.69 (s, 1H), 7.06 (s, 1H), 6.93 (d, *J =* 8.6 Hz, 1H), 6.77 (d, *J =* 8.1 Hz, 1H), 4.93 (s, 1H), 4.36 (s, 2H), 4.08 - 3.93 (m, 2H), 3.81 - 3.64 (m, 5H), 3.13 - 2.96 (m, 4H), 2.18 - 1.95 (m, 4H), 1.60 (d, *J =* 1.7 Hz, 6H), 0.73 - 0.50 (m, 4H). |
| 188 | 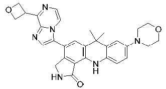 | 523 | ¹H NMR (400 MHz, DMSO-*d₆*) δ ppm: 8.95 (s, 1H), 8.63 (s, 1H), 8.24 (d, *J =* 4.7 Hz, 1H), 7.99 (s, 1H), 7.95 (d, *J =* 4.7 Hz, 1H), 7.77 (s, 1H), 7.06 (d, *J =* 2.3 Hz, 1H), 6.93 (d, *J =* 8.6 Hz, 1H), 6.78 (dd, *J =* 8.7, 2.4 Hz, 1H), 5.06 - 4.97 (m, 5H), 4.35 (s, 2H), 3.78 - 3.71 (m, 4H), 3.09 - 3.01 (m, 4H), 1.63 (s, 6H). |
| 189 | 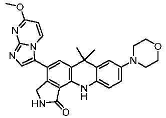 | 497 | ¹H NMR (400 MHz, DMSO-*d₆*) δ ppm: 8.90 (s, 1H), 8.61 (s, 1H), 8.48 (d, *J* = 7.4 Hz, 1H), 7.73 - 7.68 (m, 2H), 7.06 (s, 1H), 6.92 (d, *J =* 8.6 Hz, 1H), 6.77 (d, *J =* 6.5 Hz, 1H), 6.68 (d, *J =* 7.4 Hz, 1H), 4.33 (s, 2H), 3.98 (s, 3H), 3.81 - 3.64 (m, 4H), 3.12 - 2.91 (m, 4H), 1.61 (s, 6H). |
| 190 | 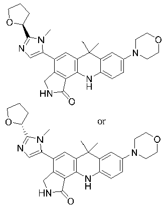 | 500 | ¹H NMR (400 MHz, DMSO-*d₆*) δ ppm: 8.85 (s, 1H), 8.57 (s, 1H), 7.52 (s, 1H), 7.12 (s, 1H), 7.05 (s, 1H), 6.88 (d, *J =* 8.6 Hz, 1H), 6.77 (s, 1H), 5.03 (t, *J* = 7.0 Hz, 1H), 4.31 (s, 2H), 3.82 (dd, *J* = 12.7, 6.3 Hz, 2H), 3.77 - 3.68 (m, 4H), 3.58 (s, 3H), 3.10 - 2.96 (m, 4H), 2.64 - 2.54 (m, 1H), 2.25 - 2.12 (m, 1H), 2.07 - 1.90 (m, 2H), 1.60 (s, 6H). |
| 191 | 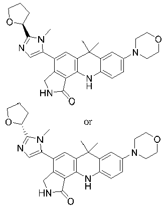 | 500 | ¹H NMR (400 MHz, DMSO-*d₆*) δ ppm: 8.83 (s, 1H), 8.56 (s, 1H), 7.49 (s, 1H), 7.04 (d, *J* = 2.2 Hz, 1H), 7.00 (s, 1H), 6.88 (d, *J* = 8.6 Hz, 1H), 6.75 (dd, *J* = 8.6, 2.4 Hz, 1H), 5.03 (t, *J =* 7.0 Hz, 1H), 4.30 (s, 2H), 3.79 (t, *J* = 6.7 Hz, 2H), 3.77 - 3.68 (m, 4H), 3.57 (s, 3H), 3.10 - 2.96 (m, 4H), 2.64 - 2.54 (m, 1H), 2.18 - 2.10 (m, 1H), 2.06 - 1.89 (m, 2H), 1.60 (s, 6H). |
| 192 | 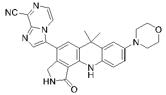 | 492 | ¹H NMR (400 MHz, DMSO-*d₆*) δ ppm: 8.94 (s, 1H), 8.62 (s, 1H), 8.59 (s, 1H), 8.27 (s, 1H), 8.09 (d, *J =* 4.6 Hz, 1H), 7.80 (s, 1H), 7.02 (d, *J* = 2.4 Hz, 1H), 6.91 (d, *J* = 8.6 Hz, 1H), 6.74 (dd, *J =* 8.7, 2.5 Hz, 1H), 4.32 (s, 2H), 3.76 - 3.66 (m, 4H), 3.04 - 2.97 (m, 4H), 1.59 (s, 6H). |
| 193 | 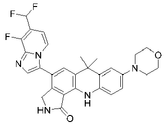 | 534 | ¹H NMR (400 MHz, DMSO-*d₆*) δ ppm: 8.95 (s, 1H), 8.62 (s, 1H), 8.21 (d, *J* = 7.2 Hz, 1H), 7.99 (s, 1H), 7.77 (s, 1H), 7.57 - 7.30 (m, 1H), 7.10 - 7.06 (m, 2H), 6.93 (d, *J* = 8.6 Hz, 1H), 6.78 - 6.76 (m, 1H), 4.33 (s, 2H), 3.82 - 3.67 (m, 4H), 3.14 - 2.96 (m, 4H), 1.62 (s, 6H). |
| 194 | 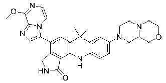 | 552 | ¹H NMR (400 MHz, DMSO-*d₆*) δ ppm: 8.89 (s, 1H), 8.57 (s, 1H), 7.86 (d, *J* = 4.7 Hz, 1H), 7.83 (s, 1H), 7.68 (s, 1H), 7.42 (d, *J* = 4.8 Hz, 1H), 7.01 (d, *J* = 2.3 Hz, 1H), 6.87 (d, *J* = 8.6 Hz, 1H), 6.72 (dd, *J* = 8.7, 2.5 Hz, 1H), 4.29 (s, 2H), 4.03 (s, 3H), 3.76 - 3.66 (m, 2H), 3.52 - 3.45 (m, 2H), 3.34 (d, *J* = 8.6 Hz, 1H), 3.13 (dd, *J* = 12.7, 7.5 Hz, 1H), 2.78 (s, 1H), 2.70 - 2.61 (m, 2H), 2.31 - 2.25 (m, 1H), 2.23 - 2.14 (m, 3H), 1.58 (s, 6H). |
| 195 | 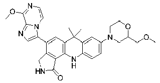 | 541 | ¹H NMR (400 MHz, DMSO-*d₆*) δ ppm: 8.90 (s, 1H), 8.58 (s, 1H), 7.89 - 7.82 (m, 2H), 7.68 (s, 1H), 7.42 (d, *J* = 4.8 Hz, 1H), 7.02 (d, *J* = 2.3 Hz, 1H), 6.89 (d, *J* = 8.6 Hz, 1H), 6.73 (dd, *J* = 8.7, 2.4 Hz, 1H), 4.29 (s, 2H), 4.03 (s, 3H), 3.92 - 3.86 (m, 1H), 3.69 - 3.63 (m, 2H), 3.46 - 3.33 (m, 4H), 3.25 (s, 3H), 2.63 - 2.56 (m, 1H), 2.40 - 2.32 (m, 1H), 1.58 (s, 6H). |
| 196 | 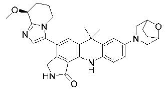 | 526 | ¹H NMR (400 MHz, DMSO-*d₆*) δ ppm: 8.78 (s, 1H), 8.55 (s, 1H), 7.55 (s, 1H), 7.10 (s, 1H), 6.92 (d, *J* = 2.3 Hz, 1H), 6.84 (d, *J* = 8.6 Hz, 1H), 6.67 (dd, *J* = 8.7, 2.5 Hz, 1H), 4.38 - 4.32 (m, 5H), 3.95 - 3.82 (m, 2H), 3.44 (s, 3H), 3.32 - 3.25 (m, 2H), 2.75 (d, *J* = 9.2 Hz, 2H), 2.37 - 1.70 (m, 8H), 1.59 (d, *J* = 8.1 Hz, 6H). |
| 197 | 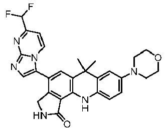 | 517 | ¹H NMR (400 MHz, DMSO-*d₆*) δ ppm: 8.94 (s, 1H), 8.87 (d, *J* = 7.1 Hz, 1H), 8.63 (s, 1H), 8.15 (s, 1H), 7.84 (s, 1H), 7.34 (d, *J* = 7.1 Hz, 1H), 7.19 - 6.94 (m, 1H), 7.05 (t, *J* = 54.4 Hz, 1H), 6.93 (s, 1H), 6.77 (dd, *J* = 8.7, 2.4 Hz, 1H), 4.36 (s, 2H), 3.76 - 3.73 (m, 4H), 3.06 - 3.03 (m, 4H), 1.63 (s, 6H). |
| 198 | 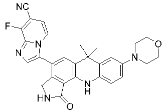 | 509 | ¹H NMR (400 MHz, DMSO-*d₆*) δ ppm: 9.06 (s, 1H), 8.67 (s, 1H), 8.32 - 8.17 (m, 2H), 7.79 (s, 1H), 7.27 - 7.23 (m, 2H), 7.10 - 6.85 (m, 2H), 4.34 (s, 2H), 3.88 - 3.78 (s, 4H), 3.18 - 3.10 (m, 4H), 1.62 (s, 6H). |
| 199 | 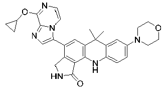 | 523 | ¹H NMR (400 MHz, DMSO-*d₆*) δ ppm: 8.94 (s, 1H), 8.61 (s, 1H), 7.93 (d, *J* = 4.7 Hz, 1H), 7.86 (s, 1H), 7.72 (s, 1H), 7.49 (d, *J* = 4.7 Hz, 1H), 7.06 (d, *J* = 2.3 Hz, 1H), 6.93 (d, *J* = 8.6 Hz, 1H), 6.77 (dd, *J* = 8.7, 2.4 Hz, 1H), 4.54 - 4.64 (m, 1H), 4.33 (s, 2H), 3.81 - 3.69 (m, 4H), 3.09 - 2.98 (m, 4H), 1.62 (s, 6H), 0.85 (dd, *J =* 13.9, 4.8 Hz, 4H). |
| 200 | 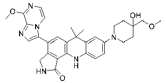 | 555 | ¹H NMR (400 MHz, DMSO-*d₆*) δ ppm: 8.91 (s, 1H), 8.61 (s, 1H), 7.95 - 7.85 (m, 2H), 7.71 (s, 1H), 7.46 (d, *J =* 4.8 Hz, 1H), 7.04 (d, *J* = 2.3 Hz, 1H), 6.88 (d, *J =* 8.6 Hz, 1H), 6.77 (dd, *J =* 8.7, 2.4 Hz, 1H), 4.38 - 4.31 (m, 3H), 4.07 (s, 3H), 3.32 - 3.22 (m, 5H), 3.18 (s, 2H), 2.96 (dd, *J =* 11.6, 9.3 Hz, 2H), 1.69 (dd, *J =* 12.2, 3.8 Hz, 2H), 1.61 (s, 6H), 1.50 (d, *J =* 12.5 Hz, 2H). |
| 201 | 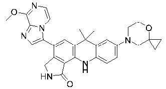 | 523 | ¹H NMR (400 MHz, DMSO-*d₆*) δ ppm: 8.93 (s, 1H), 8.62 (s, 1H), 7.90 (d, *J* = 4.8 Hz, 1H), 7.87 (s, 1H), 7.72 (s, 1H), 7.46 (d, *J* = 4.7 Hz, 1H), 7.04 (d, *J* = 2.2 Hz, 1H), 6.92 (d, *J* = 8.6 Hz, 1H), 6.76 (dd, *J* = 8.7, 2.4 Hz, 1H), 4.33 (s, 2H), 4.07 (s, 3H), 3.86 - 3.74 (m, 2H), 3.15 - 3.07 (m, 2H), 3.03 (s, 2H), 1.62 (s, 6H), 0.73 - 0.63 (m, 4H). |
| 202 | 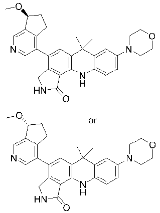 | 497 | ¹H NMR (400 MHz, DMSO-*d₆*) δ ppm: 8.87 (s, 1H), 8.61 - 8.57 (m, 2H), 8.54 (s, 1H), 7.55 (s, 1H), 7.04 (d, *J* = 2.0 Hz, 1H), 6.88 (d, *J* = 8.6 Hz, 1H), 6.76 (dd, *J* = 8.6, 2.3 Hz, 1H), 4.93 (dd, *J =* 6.2, 3.8 Hz, 1H), 4.33 (dd, *J =* 41.2, 17.7 Hz, 2H), 3.77 - 3.68 (m, 4H), 3.37 (s, 3H), 3.07 - 3.01 (m, 4H), 2.98 (d, *J* = 7.3 Hz, 1H), 2.83 - 2.71 (m, 1H), 2.29 - 2.25 (m, 1H), 2.06 - 1.96 (m, 1H), 1.60 (d, *J =* 6.0 Hz, 6H). |
| 203 | 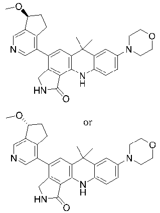 | 497 | ¹H NMR (400 MHz, DMSO-*d₆*) δ ppm: 8.87 (s, 1H), 8.61 - 8.57 (m, 2H), 8.54 (s, 1H), 7.55 (s, 1H), 7.04 (d, *J* = 2.0 Hz, 1H), 6.88 (d, *J* = 8.6 Hz, 1H), 6.76 (dd, *J* = 8.6, 2.3 Hz, 1H), 4.93 (dd, *J =* 6.2, 3.8 Hz, 1H), 4.33 (dd, *J =* 41.2, 17.7 Hz, 2H), 3.77 - 3.68 (m, 4H), 3.37 (s, 3H), 3.07 - 3.01 (m, 4H), 2.98 (d, *J* = 7.3 Hz, 1H), 2.83 - 2.71 (m, 1H), 2.29 (m, 1H), 2.06 - 1.96 (m, 1H), 1.60 (d, *J =* 6.0 Hz, 6H). |
| 204 | 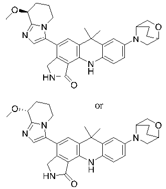 | 526 | ¹H NMR (400 MHz, DMSO-*d₆*) δ ppm: 8.73 (s, 1H), 8.52 (s, 1H), 7.54 (s, 1H), 7.09 (s, 1H), 6.84 (d, *J* = 8.6 Hz, 1H), 6.73 *(d, J* = 2.4 Hz, 1H), 6.52 (dd, *J* = 8.7, 2.5 Hz, 1H), 4.37 - 4.31 (m, 3H), 3.98 - 3.89 (m, 6H), 3.67 - 3.64 (m, 1H), 3.44 (s, 3H), 3.33 - 3.28 (m, 1H), 2.15 - 1.74 (m, 7H), 1.72 - 1.64 (m, 1H), 1.60 (d, *J =* 8.0 Hz, 6H). |
| 205 | 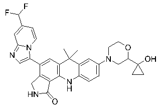 | 572 | ¹H NMR (400 MHz, DMSO-*d₆*) δ ppm: 8.94 (s, 1H), 8.61 (s, 1H), 8.38 (d, *J =* 7.0 Hz, 1H), 7.95 - 7.93 (m, 2H), 7.77 (s, 1H), 7.13 (t, *J =* 55.5 Hz, 1H), 7.12 - 7.03 (m, 2H), 6.94 (d, *J* = 8.6 Hz, 1H), 6.80 (d, *J* = 8.4 Hz, 1H), 5.42 (s, 1H), 4.32 (s, 2H), 3.93 (d, *J =* 12.0 Hz, 1H), 3.64 - 3.52 (m, 2H), 3.41 (d, *J =* 14.3 Hz, 2H), 2.63 - 2.55 (m, 2H), 1.63 (s, 6H), 0.69 - 0.34 (m, 4H). |
| 206 | 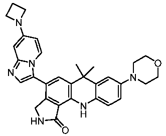 | 521 | ¹H NMR (400 MHz, DMSO-*d₆*) δ ppm: 8.88 (s, 1H), 8.57 (s, 1H), 8.04 (d, *J* = 7.4 Hz, 1H), 7.63 (s, 1H), 7.50 (s, 1H), 7.05 (d, *J =* 2.4 Hz, 1H), 6.90 (d, *J =* 8.6 Hz, 1H), 6.77 (dd, *J =* 8.7, 2.5 Hz, 1H), 6.38 (dd, *J =* 7.4, 2.0 Hz, 1H), 6.26 (d, *J =* 1.8 Hz, 1H), 4.29 (s, 2H), 3.91 (t, *J =* 7.2 Hz, 4H), 3.79 - 3.70 (m, 4H), 3.08 - 2.99 (m, 4H), 2.39 - 2.28 (m, 2H), 1.61 (s, 6H). |
| 207 | 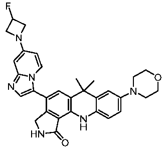 | 539 | ¹H NMR (400 MHz, DMSO-*d₆*) δ ppm: 8.88 (s, 1H), 8.57 (s, 1H), 8.06 (d, *J* = 7.3 Hz, 1H), 7.64 (s, 1H), 7.51 (s, 1H), 7.05 (d, *J* = 2.4 Hz, 1H), 6.91 (d, *J* = 8.6 Hz, 1H), 6.77 (dd, *J* = 8.7, 2.5 Hz, 1H), 6.47 - 6.37 (m, 2H), 5.64 - 5.45 (m, 1H), 4.29 (s, 2H), 4.28 - 4.20 (m, 2H), 4.02 (dd, *J* = 9.7, 2.1 Hz, 1H), 3.99 - 3.92 (m, 1H), 3.78 - 3.69 (m, 4H), 3.08 - 2.97 (m, 4H), 1.61 (s, 6H). |
| 208 | 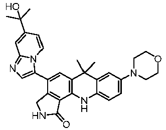 | 524 | ¹H NMR (400 MHz, DMSO-*d₆*) δ ppm: 8.90 (s, 1H), 8.58 (s, 1H), 8.18 (d, *J =* 7.2 Hz, 1H), 7.75 - 7.69 (m, 2H), 7.60 (s, 1H), 7.07 - 7.05 (m, 2H), 6.91 (d, *J* = 8.6 Hz, 1H), 6.79 - 6.76 (m, 1H), 5.25 (s, 1H), 4.31 (s, 2H), 3.78 - 3.71 (m, 4H), 3.06 - 2.97 (m, 4H), 1.62 (s, 6H), 1.48 (s, 6H). |
| 209 | 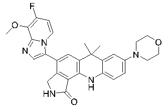 | 514 | ¹H NMR (400 MHz, DMSO-*d₆*) δ ppm: 8.91 (s, 1H), 8.60 (s, 1H), 7.98 (dd, *J* = 7.5, 4.7 Hz, 1H), 7.76 (s, 1H), 7.70 (s, 1H), 7.08 - 6.98 (m, 2H), 6.92 (d, *J =* 8.6 Hz, 1H), 6.77 (dd, *J =* 8.7, 2.5 Hz, 1H), 4.30 (s, 2H), 4.23 (s, 3H), 3.81 - 3.67 (m, 4H), 3.12 - 2.97 (m, 4H), 1.61 (s, 6H). |
| 210 | 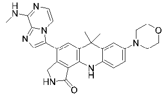 | 496 | ¹H NMR (400 MHz, DMSO-*d₆*) δ ppm: 8.92 (s, 1H), 8.61 (s, 1H), 7.71 (s, 1H), 7.66 (s, 1H), 7.54 (dd, *J* = 4.8 Hz, 1H), 7.45 (d, *J* = 4.7 Hz, 1H), 7.33 (d, *J* = 4.7 Hz, 1H), 7.06 (d, *J* = 2.4 Hz, 1H), 6.92 (d, *J* = 8.6 Hz, 1H), 6.77 (dd, *J* = 8.7, 2.5 Hz, 1H), 4.34 (s, 2H), 3.79 - 3.71 (m, 4H), 3.08 - 3.01 (m, 4H), 2.97 *(d, J* = 4.8 Hz, 3H), 1.62 (s, 6H). |
| 211 | 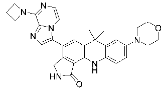 | 522 | ¹H NMR (400 MHz, DMSO-*d₆*) δ ppm: 8.92 (s, 1H), 8.61 (s, 1H), 7.75 (s, 1H), 7.65 (s, 1H), 7.50 (d, *J* = 4.7 Hz, 1H), 7.32 (d, *J* = 4.7 Hz, 1H), 7.05 (d, *J* = 2.4 Hz, 1H), 6.92 (d, *J* = 8.6 Hz, 1H), 6.77 (dd, *J* = 8.7, 2.5 Hz, 1H), 4.40 (s, 4H), 4.32 (s, 2H), 3.78 - 3.72 (m, 4H), 3.08 - 3.00 (m, 4H), 2.45 - 2.36 (m, 2H), 1.61 (s, 6H). |
| 212 | 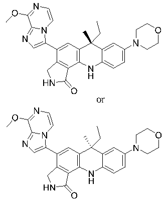 | 511 | ¹H NMR (400 MHz, DMSO-*d₆*) δ ppm: 8.87 (s, 1H), 8.61 (s, 1H), 7.93 - 7.77 (m, 2H), 7.65 (s, 1H), 7.48 (d, *J =* 4.7 Hz, 1H), 7.05 - 6.70 (m, 3H), 4.35 (s, 2H), 4.07 (s, 3H), 3.82 - 3.72 (m, 4H), 3.12 - 3.01(m, 4H), 2.02 - 1.74 (m, 2H), 1.67 (s, 3H), 0.58 (t, *J* = 7.2 Hz, 3H). |
| 213 | 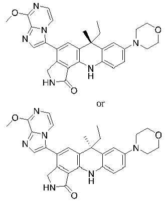 | 511 | ¹H NMR (400 MHz, DMSO-*d₆*) δ ppm: 8.87 (s, 1H), 8.61 (s, 1H), 7.95 - 7.79 (m, 2H), 7.64 (s, 1H), 7.48 (d, *J =* 4.7 Hz, 1H), 7.05 - 6.70 (m, 3H), 4.34 (d, *J* = 10.2 Hz, 2H), 4.07 (s, 3H), 3.82 - 3.72 (m, 4H), 3.12 - 3.01(m, 4H), 1.93 (d, *J* = 32.3 Hz, 2H), 1.67 (s, 3H), 0.58 (t, *J =* 7.2 Hz, 3H). |
| 214 | 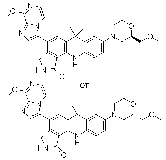 | 541 | ¹H NMR (400 MHz, DMSO-*d₆*) δ ppm: 8.94 (s, 1H), 8.61 (s, 1H), 7.92 - 7.85 (m, 2H), 7.72 (s, 1H), 7.46 (d, *J =* 4.7 Hz, 1H), 7.06 *(d, J* = 2.4 Hz, 1H), 6.93 *(d, J* = 8.6 Hz, 1H), 6.77 (dd, *J* = 8.7, 2.5 Hz, 1H), 4.33 (s, 2H), 4.07 (s, 3H), 3.92 - 3.86 (m, 1H), 3.77 - 3.69 (m, 1H), 3.68 - 3.61 (m, 1H), 3.45 - 3.39 (m, 4H), 3.29 (s, 3H), 2.67 - 2.61 (m, 1H), 2.43 - 2.36 (m, 1H), 1.62 (s, 6H). |
| 215 | 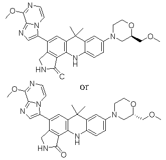 | 541 | ¹H NMR (400 MHz, DMSO-*d₆*) δ ppm: 8.93 (s, 1H), 8.58 (s, 1H), 7.90 - 7.84 (m, 2H), 7.69 (s, 1H), 7.44 (d, *J =* 4.8 Hz, 1H), 7.08 (d, *J* = 2.4 Hz, 1H), 6.92 *(d, J* = 8.4 Hz, 1H), 6.79 (dd, *J* = 8.7, 2.5 Hz, 1H), 4.30 (s, 2H), 4.04 (s, 3H), 3.92 - 3.86 (m, 1H), 3.78 - 3.59 (m, 2H), 3.43 - 3.36 (m, 4H), 3.25 (s, 3H), 2.67 - 2.59 (m, 1H), 2.43 - 2.36 (m, 1H), 1.59 (s, 6H). |
| 216 | 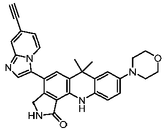 | 490 | ¹H NMR (400 MHz, DMSO-*d₆*) δ ppm: 8.92 (s, 1H), 8.59 (s, 1H), 8.23 (d, *J =* 7.0 Hz, 1H), 7.90 (s, 1H), 7.82 (s, 1H), 7.74 (s, 1H), 7.06 (d, *J =* 2.4 Hz, 1H), 6.98 - 6.89 (m, 2H), 6.77 (dd, *J =* 8.7, 2.5 Hz, 1H), 4.44 (s, 1H), 4.32 (s, 2H), 3.79 - 3.69 (m, 4H), 3.08 - 3.00 (m, 4H), 1.62 (s, 6H). |
| 217 | 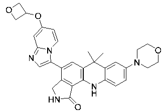 | 538 | ¹H NMR (400 MHz, DMSO-*d₆*) δ ppm: 8.88 (s, 1H), 8.56 (s, 1H), 8.23 - 8.17 (m, 1H), 7.78 (s, 1H), 7.68 (s, 1H), 7.02 (d, *J =* 2.3 Hz, 1H), 6.89 (d, *J* = 8.6 Hz, 1H), 6.82 (dd, *J* = 5.9, 2.2 Hz, 2H), 6.74 (dd, *J* = 8.7, 2.5 Hz, 1H), 5.44 (t, *J =* 5.1 Hz, 1H), 4.98 (t, *J* = 6.8 Hz, 2H), 4.56 (dd, *J* = 7.5, 4.8 Hz, 2H), 4.27 (s, 2H), 3.76 - 3.66 (m, 4H), 3.04 - 2.96 (m, 4H), 1.57 (s, 6H). |
| 218 | 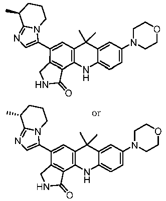 | 484 | ¹H NMR (400 MHz, DMSO-*d₆*) δ ppm: 8.97 (s, 1H), 8.66 (s, 1H), 7.85 (s, 1H), 7.68 (s, 1H), 7.06 (s, 1H), 6.93 (d, *J =* 8.6 Hz, 1H), 6.80 - 6.73 (m, 1H), 4.49 - 4.25 (m, 2H), 4.12 - 3.99 (m, 1H), 3.92 - 3.87 (m, 1H), 3.85 - 3.70 (m, 4H), 3.35 - 3.23 (m, 1H), 3.20 - 2.98 (m, 4H), 2.18 - 2.05 (m, 2H), 1.96 - 1.75 (m, 1H), 1.68 - 1.52 (m, 7H), 1.45 (d, *J =* 7.0 Hz, 3H). |
| 219 | 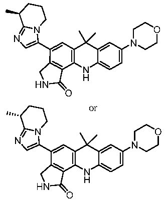 | 484 | ¹H NMR (400 MHz, DMSO-*d₆*) δ ppm: 8.93 (s, 1H), 8.65 (s, 1H), 7.84 (s, 1H), 7.67 (s, 1H), 7.06 (s, 1H), 6.93 (d, *J =* 8.6 Hz, 1H), 6.80 - 6.73 (m, 1H), 4.49 - 4.25 (m, 2H), 4.10 - 4.00 (m, 1H), 3.92 - 3.87 (m, 1H), 3.85 - 3.70 (m, 4H), 3.35 - 3.23 (m, 1H), 3.20 - 2.98 (m, 4H), 2.22 - 2.05 (m, 2H), 1.96 - 1.75 (m, 1H), 1.68 - 1.52 (m, 7H), 1.46 (d, *J =* 7.0 Hz, 3H). |
| 220 | 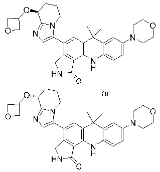 | 542 | ¹H NMR (400 MHz, DMSO-*d₆*) δ ppm: 8.82 (s, 1H), 8.54 (s, 1H), 7.56 (s, 1H), 7.09 (s, 1H), 7.04 (d, *J* = 2.2 Hz, 1H), 6.87 (d, *J* = 8.6 Hz, 1H), 6.75 (dd, *J* = 8.6, 2.4 Hz, 1H), 5.03 - 4.96 (m, 1H), 4.73 (t, *J* = 6.5 Hz, 1H), 4.65 - 4.43 (m, 3H), 4.37 - 4.25 (m, 2H), 4.23 - 4.17 (m, 1H), 3.96 - 3.81 (m, 2H), 3.78 - 3.69 (m, 4H), 3.09 - 2.93 (m, 4H), 2.10 - 1.83 (m, 4H), 1.60 (d, *J =* 8.6 Hz, 6H). |
| 221 | 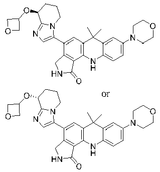 | 542 | ¹H NMR (400 MHz, DMSO-*d₆*) δ ppm: 8.82 (s, 1H), 8.54 (s, 1H), 7.56 (s, 1H), 7.09 (s, 1H), 7.04 (d, *J* = 2.4 Hz, 1H), 6.87 (d, *J* = 8.6 Hz, 1H), 6.75 (dd, *J* = 8.7, 2.5 Hz, 1H), 5.04 - 4.93 (m, 1H), 4.73 (t, *J* = 6.5 Hz, 1H), 4.60 (t, *J* = 6.6 Hz, 1H), 4.53 (t, *J* = 3.9 Hz, 1H), 4.47 (t, *J* = 6.1 Hz, 1H), 4.41 - 4.24 (m, 2H), 4.22 - 4.18 (m, 1H), 3.94 - 3.80 (m, 2H), 3.78 - 3.69 (m, 4H), 3.06 - 2.98 (m, 4H), 2.15 - 1.80 (m, 4H), 1.60 (d, *J* = 8.6 Hz, 6H). |
| 222 | 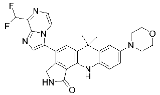 | 517 | ¹H NMR (400 MHz, DMSO-*d₆*) δ ppm: 8.96 (s, 1H), 8.63 (s, 1H), 8.47 (d, *J* = 4.6 Hz, 1H), 8.18 (s, 1H), 8.03 (d, *J* = 4.6 Hz, 1H), 7.83 (s, 1H), 7.60 - 7.29 (m, 1H), 7.06 (d, *J* = 2.3 Hz, 1H), 6.94 (d, *J =* 8.6 Hz, 1H), 6.78 (dd, *J =* 8.6, 2.4 Hz, 1H), 4.37 (s, 2H), 3.79 - 3.70 (m, 4H), 3.08 - 3.01 (m, 4H), 1.63 (s, 6H). |
| 223 | 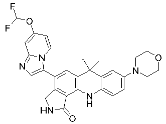 | 532 | ¹H NMR (400 MHz, DMSO-*d₆*) δ ppm: 8.91 (s, 1H), 8.60 (s, 1H), 8.28 (d, *J =* 7.5 Hz, 1H), 7.78 (s, 1H), 7.72 (s, 1H), 7.44 (t, *J* = 73.4 Hz, 1H), 7.41 (d, *J* = 2.3 Hz, 1H), 7.06 (d, *J* = 2.4 Hz, 1H), 6.92 (d, *J* = 8.6 Hz, 1H), 6.88 (dd, *J* = 7.5, 2.5 Hz, 1H), 6.77 (dd, *J =* 8.7, 2.5 Hz, 1H), 4.31 (s, 2H), 3.78 - 3.69 (m, 4H), 3.06 - 3.00 (m, 4H), 1.62 (s, 6H). |
| 224 | 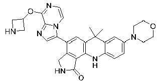 | 538 | ¹H NMR (400 MHz, DMSO-*d₆*) δ ppm: 8.94 (s, 1H), 8.62 (s, 1H), 7.94 (d, *J* = 4.8 Hz, 1H), 7.92 (s, 1H), 7.72 (s, 1H), 7.42 (d, *J* = 4.7 Hz, 1H), 7.06 (d, *J* = 2.3 Hz, 1H), 6.93 (d, *J* = 8.6 Hz, 1H), 6.78 (dd, *J* = 8.6, 2.4 Hz, 1H), 5.63 - 5.54 (m, 1H), 4.34 (s, 2H), 4.21 - 4.13 (m, 2H), 3.94 (dd, *J* = 10.8, 5.5 Hz, 2H), 3.77 - 3.72 (m, 4H), 3.06 - 3.01 (m, 4H), 1.62 (s, 6H). |
| 225 | 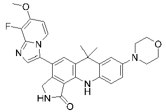 | 514 | ¹H NMR (400 MHz, DMSO-*d₆*) δ ppm: 8.87 (s, 1H), 8.56 (s, 1H), 8.04 (d, *J* = 7.7 Hz, 1H), 7.70 - 7.67 (m, 2H), 7.12 - 6.98 (m, 2H), 6.88 (d, *J* = 8.6 Hz, 1H), 6.73 (dd, *J* = 8.6, 2.5 Hz, 1H), 4.26 (s, 2H), 3.93 (s, 3H), 3.76 - 3.66 (m, 4H), 3.08 - 2.93 (m, 4H), 1.57 (s, 6H). |
| 226 | 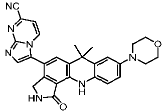 | 492 | ¹H NMR (400 MHz, DMSO-*d₆*) δ ppm: 8.99 - 8.90 (m, 2H), 8.64 (s, 1H), 8.37 (s, 1H), 7.83 (s, 1H), 7.60 (d, *J =* 7.0 Hz, 1H), 7.06 (d, *J* = 2.4 Hz, 1H), 6.94 (d, *J* = 8.6 Hz, 1H), 6.79 (d, *J =* 2.5 Hz, 1H), 4.36 (s, 2H), 3.78 - 3.72 (m, 4H), 3.06 - 3.02 (m, 4H), 1.62 (s, 6H). |
| 227 | 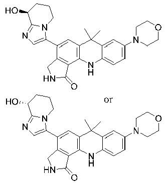 | 486 | ¹H NMR (400 MHz, DMSO-*d₆*) δ ppm: 8.78 (s, 1H), 8.51 (s, 1H), 7.49 (s, 1H), 7.05 (s, 1H), 7.00 (d, *J* = 2.4 Hz, 1H), 6.83 (d, *J =* 8.6 Hz, 1H), 6.71 (dd, *J =* 8.7, 2.5 Hz, 1H), 5.40 (s, 1H), 4.67 (s, 1H), 4.29 (dd, *J* = 39.2, 17.9 Hz, 2H), 3.92 - 3.78 (m, 2H), 3.73 - 3.68 (m, 4H), 3.09 - 2.89 (m, 4H), 2.07 - 1.72 (m, 4H), 1.56 (d, *J =* 8.0 Hz, 6H). |
| 228 | 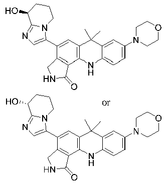 | 486 | ¹H NMR (400 MHz, DMSO-*d₆*) δ ppm: 8.78 (s, 1H), 8.51 (s, 1H), 7.49 (s, 1H), 7.04 (s, 1H), 7.00 (d, *J* = 2.4 Hz, 1H), 6.83 (d, *J =* 8.6 Hz, 1H), 6.71 (dd, *J =* 8.7, 2.5 Hz, 1H), 5.39 (d, *J =* 4.4 Hz, 1H), 4.67 (d, *J =* 4.0 Hz, 1H), 4.29 (dd, *J* = 39.3, 17.9 Hz, 2H), 3.82 (dd, *J* = 8.5, 4.5 Hz, 2H), 3.75 - 3.62 (m, 4H), 3.01 - 2.94 (m, 4H), 2.10 - 1.73 (m, 4H), 1.56 (d, *J* = 8.0 Hz, 6H). |
| 229 | 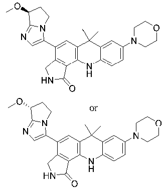 | | ¹H NMR (400 MHz, DMSO-*d₆*) δ ppm: 8.80 (s, 1H), 8.58 (s, 1H), 7.60 (s, 1H), 7.14 (s, 1H), 7.01 (d, *J* = 2.4 Hz, 1H), 6.83 (d, *J =* 8.6 Hz, 1H), 6.71 (dd, *J =* 8.7, 2.5 Hz, 1H), 4.59 (dd, *J =* 7.0, 2.0 Hz, 1H), 4.37 - 4.26 (m, 3H), 4.09 - 3.99 (m, 1H), 3.76 - 3.62 (m, 4H), 3.37 (s, 3H), 3.30 - 3.25 (m, 1H), 3.10 - 2.95 (m, 4H), 2.86 - 2.78 (m, 1H), 1.57 (d, *J =* 13.6 Hz, 6H). |
| 230 | 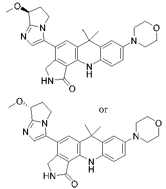 | 486 | ¹H NMR (400 MHz, DMSO-*d₆*) δ ppm: 8.80 (s, 1H), 8.59 (s, 1H), 7.60 (s, 1H), 7.14 (s, 1H), 7.00 (d, *J* = 2.1 Hz, 1H), 6.83 (d, *J =* 8.6 Hz, 1H), 6.71 (dd, *J =* 8.7, 2.3 Hz, 1H), 4.59 (d, *J =* 5.3 Hz, 1H), 4.37 - 4.26 (m, 3H), 4.08 - 4.00 (m, 1H), 3.72 - 3.68 (m, 4H), 3.37 (s, 3H), 3.30 - 3.25 (m, 1H), 3.10 - 2.96 (m, 4H), 2.88 - 2.77 (m, 1H), 1.57 (d, *J =* 13.9 Hz, 6H). |
| 231 | 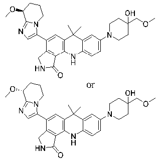 | 558 | ¹H NMR (400 MHz, DMSO-*d₆*) δ ppm: 8.80 (s, 1H), 8.52 (s, 1H), 7.55 (s, 1H), 7.09 (s, 1H), 7.02 (d, *J* = 2.4 Hz, 1H), 6.83 (d, *J* = 8.6 Hz, 1H), 6.77 - 6.74 (m, 1H), 4.39 - 4.24 (m, 4H), 3.94 - 3.81 (m, 2H), 3.44 (s, 3H), 3.29 (s, 3H), 3.24 (d, *J =* 12.1 Hz, 2H), 3.18 (s, 2H), 2.96 (t, *J =* 10.5 Hz, 2H), 2.10 - 1.81 (m, 4H), 1.74 - 1.67 (m, 2H), 1.59 (d, *J =* 7.8 Hz, 6H), 1.49 (d, *J* = 12.6 Hz, 2H). |
| 232 | 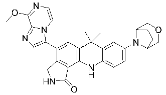 | 523 | ¹H NMR (400 MHz, DMSO-*d₆*) δ ppm: 8.85 (s, 1H), 8.55 (s, 1H), 7.89 - 7.81 (m, 2H), 7.66 (s, 1H), 7.42 (d, *J =* 4.7 Hz, 1H), 6.90 (s, 1H), 6.85 (d, *J =* 8.6 Hz, 1H), 6.66 (d, *J =* 8.6 Hz, 1H), 4.29 (s, 2H), 4.06 - 4.02 (m, 5H), 3.69 (d, *J* = 10.5 Hz, 2H), 3.40 (d, *J* = 10.7 Hz, 2H), 1.92 - 1.76 (m, 4H), 1.57 (s, 6H). |
| 233 | 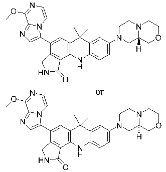 | 552 | ¹H NMR (400 MHz, DMSO-*d₆*) δ ppm: 8.93 (s, 1H), 8.62 (s, 1H), 7.90 (d, *J* = 4.7 Hz, 1H), 7.87 (s, 1H), 7.72 (s, 1H), 7.46 (d, *J* = 4.7 Hz, 1H), 7.05 (d, *J* = 2.1 Hz, 1H), 6.91 (d, *J* = 8.6 Hz, 1H), 6.76 (dd, *J* = 8.7, 2.4 Hz, 1H), 4.33 (s, 2H), 4.07 (s, 3H), 3.85 - 3.70 (m, 2H), 3.57 - 3.49 (m, 2H), 3.38 - 3.33 (m, 1H), 3.21 - 3.05 (m, 1H), 2.81 (d, *J =* 10.8 Hz, 1H), 2.73 - 2.65 (m, 2H), 2.38 - 2.14 (m, 4H), 1.62 (s, 6H). |
| 234 | 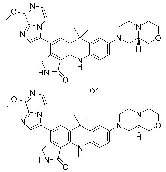 | 552 | ¹H NMR (400 MHz, DMSO-*d₆*) δ ppm: 8.93 (s, 1H), 8.62 (s, 1H), 7.90 (d, *J* = 4.8 Hz, 1H), 7.87 (s, 1H), 7.72 (s, 1H), 7.46 (d, *J* = 4.8 Hz, 1H), 7.05 (d, *J* = 2.3 Hz, 1H), 6.91 (d, *J* = 8.6 Hz, 1H), 6.76 (dd, *J* = 8.7, 2.4 Hz, 1H), 4.33 (s, 2H), 4.07 (s, 3H), 3.84 - 3.68 (m, 2H), 3.57 - 3.49 (m, 2H), 3.38 - 3.33 (m, 1H), 3.21 - 3.05 (m, 1H), 2.81 (d, *J* = 11.2 Hz, 1H), 2.73 - 2.65 (m, 2H), 2.37 - 2.15 (m, 4H), 1.62 (s, 6H). |
| 235 | 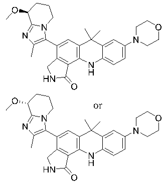 | 514 | ¹H NMR (400 MHz, DMSO-*d₆*) δ ppm: 8.93 (d, *J* = 6.8 Hz, 1H), 8.64 (d, *J* = 5.3 Hz, 1H), 7.59 (d, *J* = 45.3 Hz, 1H), 7.05 (s, 1H), 6.93 (d, *J* = 8.5 Hz, 1H), 6.77 (d, *J* = 8.5 Hz, 1H), 4.70 (s, 1H), 4.18 (d, *J* = 5.3 Hz, 1H), 4.14 (s, 1H), 3.90 (dd, *J =* 19.1, 10.5 Hz, 1H), 3.78 - 3.71 (m, 4H), 3.68 - 3.54 (m, 1H), 3.49 (d, *J* = 4.5 Hz, 3H), 3.07 - 3.01 (m, 4H), 2.16 (d, *J* = 2.3 Hz, 3H), 2.09 (dd, *J* = 19.9, 4.8 Hz, 2H), 1.97 (s, 2H), 1.58 (d, *J* = 4.1 Hz, 6H). |
| 236 | 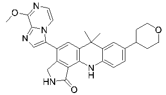 | 496 | ¹H NMR (400 MHz, DMSO-*d₆*) δ ppm: 9.04 (s, 1H), 8.67 (s, 1H), 7.95 - 7.87 (m, 2H), 7.75 (s, 1H), 7.46 (d, *J =* 4.8 Hz, 1H), 7.34 (s, 1H), 7.04 - 6.98 (m, 1H), 6.94 (d, *J =* 8.1 Hz, 1H), 4.35 (s, 2H), 4.07 (s, 3H), 3.95 (d, *J =* 10.8 Hz, 2H), 3.48 - 3.35 (m, 2H), 2.78 - 2.65 (m, 1H), 1.73 - 1.54 (m, 10H). |
| 237 | 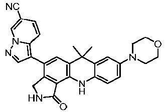 | 491 | ¹H NMR (400 MHz, DMSO-*d₆*) δ ppm: 9.64 (s, 1H), 8.85 (s, 1H), 8.61 (s, 1H), 8.58 (s, 1H), 7.82 (d, *J =* 9.3 Hz, 1H), 7.64 (s, 1H), 7.49 (dd, *J* = 9.3, 1.4 Hz, 1H), 7.05 (d, *J* = 2.4 Hz, 1H), 6.89 (d, *J =* 8.6 Hz, 1H), 6.77 (dd, *J =* 8.7, 2.5 Hz, 1H), 4.43 (s, 2H), 3.76 - 3.73 (m, 4H), 3.05 - 3.02 (m, 4H), 1.63 (s, 6H). |
| 238 | 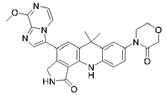 | 511 | ¹H NMR (400 MHz, DMSO-*d₆*) δ ppm: 9.13 (s, 1H), 8.66 (s, 1H), 7.90 - 7.84 (m, 2H), 7.72 (s, 1H), 7.45 - 7.40 (m, 2H), 7.09 - 7.02 (m, 2H), 4.32 (s, 2H), 4.15 (s, 2H), 4.03 (s, 3H), 3.95 - 3.91 (m, 2H), 3.72 - 3.65 (m, 2H), 1.57 (s, 6H). |
| 239 | 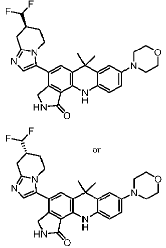 | 520 | ¹H NMR (400 MHz, DMSO-*d₆*) δ 8.83 (s, 1H), 8.58 (s, 1H), 7.53 (s, 1H), 7.16 (s, 1H), 7.05 (s, 1H), 6.89 (d, *J* = 8.5 Hz, 1H), 6.76 (d, *J* = 8.6 Hz, 1H), 6.33 - 6.04 (m, 1H), 4.45 (d, *J* = 17.9 Hz, 1H), 4.20 (d, *J* = 18.0 Hz, 1H), 4.12 - 4.02 (m, 1H), 3.90 (d, *J* = 10.3 Hz, 1H), 3.75 - 3.64 (m, 4H), 3.08 - 2.95 (m, 5H), 2.77 - 2.67 (m, 1H), 2.14 (d, *J* = 12.5 Hz, 1H), 1.79 - 1.66 (m, 1H), 1.60 (d, *J =* 18.1 Hz, 6H). |
| 240 | 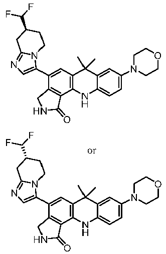 | 520 | ¹H NMR (400 MHz, DMSO-*d₆*) δ 8.85 (s, 1H), 8.60 (s, 1H), 7.55 (s, 1H), 7.31 (s, 1H), 7.05 (s, 1H), 6.90 (d, *J* = 8.5 Hz, 1H), 6.76 (d, *J* = 8.2 Hz, 1H), 6.33 - 6.04 (m, 1H), 4.45 (d, *J =* 17.9 Hz, 1H), 4.20 (d, *J* = 18.1 Hz, 1H), 4.15 - 4.03 (m, 1H), 3.92 (d, *J* = 10.7 Hz, 1H), 3.75 - 3.64 (m, 4H), 3.08 - 2.95 (m, 5H), 2.77 - 2.67 (m, 1H), 2.15 (d, *J =* 12.5 Hz, 1H), 1.77 (d, *J* = 7.4 Hz, 1H), 1.60 (d, *J* = 17.2 Hz, 6H). |
| 241 | 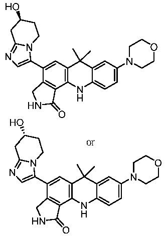 | 486 | ¹H NMR (400 MHz, DMSO-*d₆*) δ ppm: 8.81 (s, 1H), 8.55 (s, 1H), 7.51 (s, 1H), 7.04 (d, *J* = 2.4 Hz, 1H), 7.01 (s, 1H), 6.87 (d, *J =* 8.6 Hz, 1H), 6.75 (dd, *J =* 8.7, 2.5 Hz, 1H), 5.14 (s, 1H), 4.32 (s, 2H), 4.17 (s, 1H), 3.91 (dd, *J =* 12.0, 6.1 Hz, 2H), 3.78 - 3.70 (m, 4H), 3.06 - 3.01 (m, 4H), 2.99 (d, *J* = 4.6 Hz, 1H), 2.70 (dd, *J =* 16.6, 6.3 Hz, 1H), 2.04 - 1.88 (m, 2H), 1.60 (d, *J* = 2.4 Hz, 6H). |
| 242 | 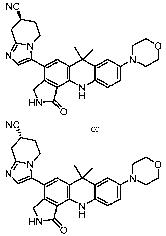 | 495 | ¹H NMR (400 MHz, DMSO-*d₆*) δ ppm: 8.82 (s, 1H), 8.57 (s, 1H), 7.50 (s, 1H), 7.09 - 7.02 (m, 2H), 6.88 (d, *J =* 8.6 Hz, 1H), 6.75 (dd, *J* = 8.6, 2.5 Hz, 1H), 4.40 - 4.20 (m, 2H), 4.06 - 3.88 (m, 2H), 3.78 - 3.72 (m, 4H), 3.50 - 3.44 (m, 1H), 3.25 (dd, *J =* 16.4, 5.6 Hz, 1H), 3.12 - 3.06 (m, 1H), 3.05 - 3.00 (m, 4H), 2.28 - 2.12 (m, 2H), 1.60 (d, *J =* 5.9 Hz, 6H). |
| 243 | 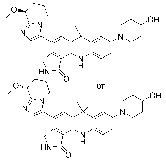 | 514 | ¹H NMR (400 MHz, DMSO-*d₆*) δ ppm: 8.80 (s, 1H), 8.55 (s, 1H), 7.55 (s, 1H), 7.11 (s, 1H), 7.02 (d, *J* = 2.3 Hz, 1H), 6.83 (d, *J =* 8.6 Hz, 1H), 6.75 (dd, *J =* 8.6, 2.4 Hz, 1H), 4.67 (s, 1H), 4.33 (q, *J =* 17.6 Hz, 3H), 3.88 (d, *J* = 9.9 Hz, 2H), 3.57 (dd, *J* = 8.6, 4.5 Hz, 1H), 3.44 (s, 3H), 3.40 (s, 2H), 2.71 (dd, *J =* 19.3, 9.4 Hz, 2H), 2.11 - 1.80 (m, 6H), 1.59 (d, *J =* 8.6 Hz, 6H), 1.49 (dd, *J =* 16.0, 6.7 Hz, 2H). |
| 244 | 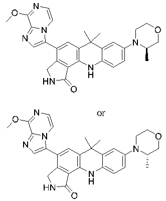 | 511 | ¹H NMR (400 MHz, DMSO-*d₆*) δ ppm: 8.97 (s, 1H), 8.64 (s, 1H), 8.05 - 7.88 (m, 2H), 7.78 - 7.68 (m, 1H), 7.47 (d, *J* = 4.7 Hz, 1H), 7.11 - 6.67 (m, 3H), 4.36 (s, 2H), 4.08 (s, 3H), 3.85 - 3.45 (m, 5H), 3.10 - 2.95 (m, 2H), 1.63 (s, 6H), 0.95 - 0.85 (m, 3H). |
| 245 | 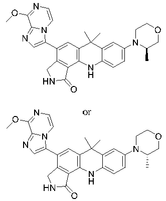 | 511 | ¹H NMR (400 MHz, DMSO-*d₆*) δ ppm: 8.97 (s, 1H), 8.63 (s, 1H), 8.05 - 7.88 (m, 2H), 7.78 - 7.68 (m, 1H), 7.46 (d, *J* = 4.7 Hz, 1H), 7.13 - 6.67 (m, 3H), 4.35 (s, 2H), 4.07 (s, 3H), 3.85 - 3.45 (m, 5H), 3.10 - 2.95 (m, 2H), 1.62 (d, *J* = 5.0 Hz, 6H), 0.90 (d, *J =* 5.0 Hz, 3H). |
| 246 | 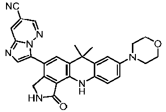 | 492 | ¹H NMR (400 MHz, DMSO-*d₆*) δ ppm: 9.07 - 8.97 (m, 3H), 8.70 (s, 1H), 8.40 (s, 1H), 8.20 (s, 1H), 7.07 (d, *J* = 2.1 Hz, 1H), 6.93 (d, *J* = 8.6 Hz, 1H), 6.78 (dd, *J* = 8.7, 2.4 Hz, 1H), 4.50 (s, 2H), 3.79 - 3.71 (m, 4H), 3.09 - 3.01 (m, 4H), 1.63 (s, 6H). |
| 247 | 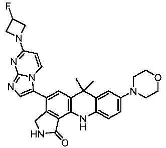 | 540 | ¹H NMR (400 MHz, DMSO-*d₆*) δ ppm: 8.83 (s, 1H), 8.55 (s, 1H), 8.26 (d, *J* = 7.4 Hz, 1H), 7.57 (s, 1H), 7.41 (s, 1H), 7.01 (d, *J* = 2.4 Hz, 1H), 6.86 (d, *J* = 8.6 Hz, 1H), 6.72 (dd, *J* = 8.7, 2.5 Hz, 1H), 6.34 (d, *J* = 7.5 Hz, 1H), 5.61 - 5.40 (m, 1H), 4.45 - 4.41 (m, 2H), 4.26 (s, 2H), 4.20 - 4.07 (m, 2H), 3.76 - 3.67 (m, 4H), 3.03 - 2.96 (m, 4H), 1.56 (s, 6H). |
| 248 | 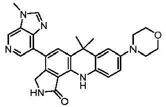 | 481 | ¹H NMR (400 MHz, DMSO-*d₆*) δ ppm: 8.92 (s, 1H), 8.89 (s, 1H), 8.49 (s, 1H), 8.42 (d, *J* = 8.0 Hz, 2H), 7.86 (s, 1H), 7.02 (d, *J* = 2.4 Hz, 1H), 6.86 (d, *J* = 8.6 Hz, 1H), 6.73 (dd, *J* = 8.7, 2.5 Hz, 1H), 4.44 (s, 2H), 3.95 (s, 3H), 3.75 - 3.67 (m, 4H), 3.05 - 2.97 (m, 4H), 1.59 (s, 6H). |
| 249 | 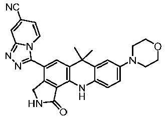 | 492 | ¹H NMR (400 MHz, DMSO-*d₆*) δ ppm: 9.05 (s, 1H), 8.75 (s, 1H), 8.65 (s, 1H), 8.52 (d, *J* = 7.3 Hz, 1H), 7.99 (s, 1H), 7.29 (dd, *J* = 7.3, 1.4 Hz, 1H), 7.07 (d, *J* = 2.3 Hz, 1H), 6.97 (d, *J =* 8.6 Hz, 1H), 6.80 (d, *J* = 2.4 Hz, 1H), 4.46 (s, 2H), 3.80 - 3.70 (m, 4H), 3.09 - 2.98 (m, 4H), 1.65 (s, 6H). |
| 250 | 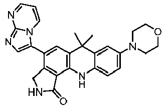 | 467 | ¹H NMR (400 MHz, DMSO-*d₆*) δ ppm: 8.92 (s, 1H), 8.70 - 8.69 (m, 1H), 8.62 (s, 1H), 8.59 - 8.57 (m, 1H), 7.97 (s, 1H), 7.78 (s, 1H), 7.12 - 7.09 (m, 1H), 7.06 (d, *J* = 2.3 Hz, 1H), 6.92 (d, *J* = 8.6 Hz, 1H), 6.79 - 6.76 (m, 1H), 4.34 (s, 2H), 3.77 - 3.69 (m, 4H), 3.06 - 2.99 (m, 4H), 1.62 (s, 6H). |
| 251 | 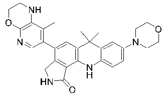 | 498 | ¹H NMR (400 MHz, DMSO-*d₆*) δ ppm: 8.78 (s, 1H), 8.45 (s, 1H), 7.35 (s, 1H), 7.29 (s, 1H), 7.03 (d, *J* = 2.4 Hz, 1H), 6.87 (d, *J* = 8.6 Hz, 1H), 6.77 - 6.74 (m, 1H), 5.60 (s, 1H), 4.30 - 4.24 (m, 2H), 4.11 (s, 2H), 3.77 - 3.71 (m, 4H), 3.34 (s, 2H), 3.04 - 3.00 (m, 4H), 1.89 (s, 3H), 1.56 (s, 6H). |
| 252 | 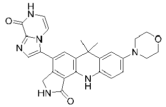 | 483 | ¹H NMR (400 MHz, DMSO-*d₆*) δ ppm: 11.23 (s, 1H), 8.93 (s, 1H), 8.63 (s, 1H), 7.69 (s, 1H), 7.64 (s, 1H), 7.14 (d, *J =* 5.7 Hz, 1H), 7.05 *(d, J* = 2.4 Hz, 1H), 6.94 - 6.87 (m, 2H), 6.77 (dd, *J* = 8.7, 2.5 Hz, 1H), 4.35 (s, 2H), 3.83 - 3.67 (m, 4H), 3.11 - 2.95 (m, 4H), 1.61 (s, 6H). |
| 253 | 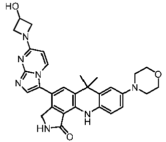 | 538 | ¹H NMR (400 MHz, DMSO-*d₆*) δ ppm: 8.87 (s, 1H), 8.59 (s, 1H), 8.24 (d, *J* = 7.5 Hz, 1H), 7.60 (s, 1H), 7.42 (s, 1H), 7.05 (d, *J* = 2.4 Hz, 1H), 6.90 (d, *J* = 8.6 Hz, 1H), 6.81 - 6.73 (m, 1H), 6.31 (d, *J* = 7.5 Hz, 1H), 5.82 (br s, 1H), 4.65 - 4.61 (m, 1H), 4.35 - 4.28 (m, 4H), 3.83 (dd, *J* = 9.4, 4.5 Hz, 2H), 3.79 - 3.71 (m, 4H), 3.08 - 3.00 (m, 4H), 1.60 (s, 6H). |
| 254 | 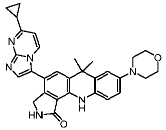 | 507 | ¹H NMR (400 MHz, DMSO-*d₆*) δ ppm: 9.04 (s, 1H), 8.79 (d, *J* = 7.1 Hz, 1H), 8.68 (s, 1H), 8.32 (s, 1H), 7.79 (s, 1H), 7.63 (d, *J* = 7.1 Hz, 1H), 7.15 (s, 1H), 7.01 (d, *J* = 8.5 Hz, 1H), 6.91 - 6.84 (m, 1H), 4.33 (s, 2H), 3.81 - 3.75 (m, 4H), 3.15 - 3.08 (m, 4H), 2.54 - 2.51 (m, 1H), 1.61 (s, 6H), 1.40 - 1.34 (m, 2H), 1.27 - 1.21 (m, 2H). |
| 255 | 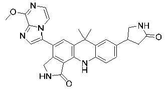 | 495 | ¹H NMR (400 MHz, DMSO-*d₆*) δ ppm: 9.07 (s, 1H), 8.67 (s, 1H), 7.95 - 7.88 (m, 2H), 7.82 - 7.58 (m, 2H), 7.56 - 7.26 (m, 2H), 7.25 - 6.89 (m, 1H), 4.35 (s, 2H), 4.08 (s, 3H), 3.58 - 3.35 (m, 3H), 2.65 - 2.60 (m, 1H), 2.45 - 2.35 (m, 1H), 1.62 (s, 6H). |
| 256 | 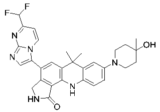 | 545 | ¹H NMR (400 MHz, DMSO-*d₆*) δ ppm: 8.91 (s, 1H), 8.86 (d, *J* = 7.1 Hz, 1H), 8.60 (s, 1H), 8.14 (s, 1H), 7.83 (s, 1H), 7.34 (d, *J* = 7.1 Hz, 1H), 7.05 (t, *J* = 54.5 Hz, 1H), 7.04 (d, *J* = 2.4 Hz, 1H), 6.88 (d, *J* = 8.6 Hz, 1H), 6.77 (dd, *J* = 8.7, 2.5 Hz, 1H), 4.36 (s, 2H), 4.24 (s, 1H), 3.18 - 2.98 (m, 4H), 1.64 - 1.57 (m, 10H), 1.16 (s, 3H). |
| 257 | 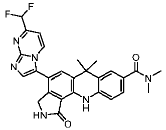 | 503 | ¹H NMR (400 MHz, DMSO-*d₆*) δ ppm: 9.23 (s, 1H), 8.85 (d, *J* = 7.1 Hz, 1H), 8.68 (s, 1H), 8.14 (s, 1H), 7.87 (s, 1H), 7.48 (d, *J* = 1.5 Hz, 1H), 7.31 (d, *J* = 7.1 Hz, 1H), 7.21 - 6.86 (m, 3H), 4.36 (s, 2H), 2.94 (s, 6H), 1.60 (s, 6H). |
| 258 | 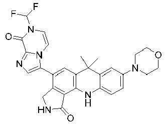 | 533 | ¹H NMR (400 MHz, DMSO-*d₆*) δ ppm: 8.95 (s, 1H), 8.63 (s, 1H), 7.98 (t, *J* = 59.4 Hz, 1H), 7.83 (d, *J* = 6.8 Hz, 1H), 7.67 (s, 1H), 7.31 - 7.25 (m, 2H), 7.06 (d, *J =* 2.5 Hz, 1H), 6.93 (d, *J* = 8.6 Hz, 1H), 6.77 (dd, *J =* 8.7, 2.5 Hz, 1H), 4.36 (s, 2H), 3.79 - 3.71 (m, 4H), 3.10 - 3.01 (m, 4H), 1.62 (s, 6H). |
| 259 | 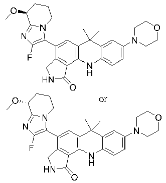 | 518 | ¹H NMR (400 MHz, DMSO-*d₆*) δ ppm: 8.85 (s, 1H), 8.52 (s, 1H), 7.59 (s, 1H), 7.04 (d, *J =* 2.4 Hz, 1H), 6.89 (d, *J =* 8.6 Hz, 1H), 6.75 (dd, *J* = 8.7, 2.5 Hz, 1H), 4.27 (t, *J* = 3.7 Hz, 1H), 4.18 (s, 2H), 3.79 (d, *J =* 7.3 Hz, 2H), 3.77 - 3.70 (m, 4H), 3.44 (d, *J* = 9.8 Hz, 3H), 3.07 - 2.97 (m, 4H), 2.10 - 1.83 (m, 4H), 1.60 (d, *J =* 4.9 Hz, 6H). |
| 260 | 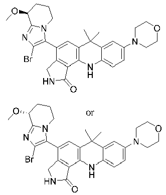 | 578 | ¹H NMR (400 MHz, DMSO-*d₆*) δ ppm: 8.88 (d, *J* = 5.2 Hz, 1H), 8.54 (d, *J* = 2.9 Hz, 1H), 7.55 (d, *J* = 20.4 Hz, 1H), 7.04 (d, *J* = 2.1 Hz, 1H), 6.89 (s, 1H), 6.79 - 6.74 (m, 1H), 4.37 - 4.25 (m, 2H), 4.18 - 4.11 (m, 1H), 3.91 - 3.83 (m, 1H), 3.79 - 3.72 (m, 4H), 3.57 - 3.51 (m, 1H), 3.43 (d, *J* = 7.5 Hz, 3H), 3.09 - 2.99 (m, 4H), 2.12 - 1.84 (m, 4H), 1.63 - 1.55 (m, 6H). |
| 261 | 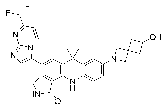 | 543 | ¹H NMR (400 MHz, DMSO-*d₆*) δ ppm: 8.89 - 8.82 (m, 2H), 8.13 (s, 1H), 7.80 (s, 1H), 7.33 (d, *J =* 7.1 Hz, 1H), 7.05 (t, *J =* 54.5 Hz, 1H), 6.86 (d, *J =* 8.4 Hz, 1H), 6.49 (d, *J =* 2.3 Hz, 1H), 6.27 - 6.25 (m, 1H), 5.03 (d, *J =* 6.2 Hz, 1H), 4.35 (s, 2H), 4.06 - 3.97 (m, 1H), 3.70 (d, *J =* 17.7 Hz, 4H), 2.46 - 2.40 (m, 2H), 2.06 - 1.94 (m, 2H), 1.59 (s, 6H). |
| 262 | 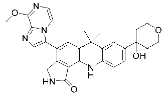 | 512 | ¹H NMR (400 MHz, DMSO-*d₆*) δ ppm: 9.03 (s, 1H), 8.62 (s, 1H), 7.89 - 7.82 (m, 2H), 7.71 (s, 1H), 7.53 (d, *J =* 1.7 Hz, 1H), 7.43 (d, *J =* 4.8 Hz, 1H), 7.20 (dd, *J =* 8.3, 1.9 Hz, 1H), 6.92 (d, *J* = 8.3 Hz, 1H), 4.89 (s, 1H), 4.31 (s, 2H), 4.04 (s, 3H), 3.78 - 3.65 (m, 4H), 1.99 - 1.90 (m, 2H), 1.60 (s, 6H), 1.51 (d, *J =* 12.4 Hz, 2H). |
| 263 | 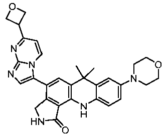 | 523 | ¹H NMR (400 MHz, DMSO-*d₆*) δ ppm: 8.94 (s, 1H), 8.70 (d, *J* = 7.1 Hz, 1H), 8.62 (s, 1H), 8.01 (s, 1H), 7.76 (s, 1H), 7.15 (d, *J* = 7.1 Hz, 1H), 7.06 (s, 1H), 6.93 (d, *J* = 8.6 Hz, 1H), 6.78 (d, *J* = 8.6 Hz, 1H), 5.05 - 4.93 (m, 2H), 4.85 (t, *J* = 6.1 Hz, 2H), 4.58 - 4.48 (m, 1H), 4.34 (s, 2H), 3.78 - 3.72 (m, 4H), 3.07 - 3.01 (m, 4H), 1.62 (s, 6H). |
| 264 | 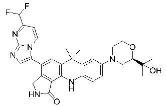 | 575 | ¹H NMR (400 MHz, DMSO-*d₆*) δ ppm: 8.94 (s, 1H), 8.87 (d, *J* = 7.1 Hz, 1H), 8.61 (s, 1H), 8.14 (s, 1H), 7.84 (s, 1H), 7.34 (d, *J* = 7.1 Hz, 1H), 7.19 - 6.92 (m, 3H), 6.79 (dd, *J* = 8.7, 2.4 Hz, 1H), 4.46 (s, 1H), 4.36 (s, 2H), 3.98 (dd, *J* = 11.0, 2.4 Hz, 1H), 3.70 - 3.59 (m, 1H), 3.54 (d, *J =* 11.5 Hz, 1H), 3.42 - 3.33 (m, 1H), 3.28 - 3.23 (m, 1H), 2.65 - 2.55 (m, 1H), 2.48 - 2.42 (m, 1H), 1.63 (s, 6H), 1.14 (d, *J =* 24.3 Hz, 6H). |
| 265 | 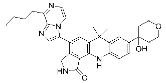 | 538 | ¹H NMR (400 MHz, DMSO-*d₆*) δ ppm: 9.07 (s, 1H), 8.67 (s, 1H), 8.16 (d, *J =* 4.7 Hz, 1H), 7.99 (s, 1H), 7.85 - 7.78 (m, 2H), 7.57 (d, *J =* 1.6 Hz, 1H), 7.23 (dd, *J =* 8.3, 1.8 Hz, 1H), 6.96 (d, *J =* 8.3 Hz, 1H), 4.94 (s, 1H), 4.37 (s, 2H), 3.82 - 3.68 (m, 4H), 3.21 - 3.14 (m, 2H), 2.02 - 1.92 (m, 2H), 1.85 (t, *J* = 7.6 Hz, 2H), 1.64 (s, 6H), 1.55 (d, *J =* 12.5 Hz, 2H), 1.42 (dd, *J =* 14.9, 7.4 Hz, 2H), 0.95 (t, *J =* 7.4 Hz, 3H). |
| 266 | 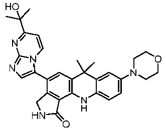 | 525 | ¹H NMR (400 MHz, DMSO-*d₆*) δ ppm: 8.91 (s, 1H), 8.64 (d, *J* = 7.2 Hz, 1H), 8.59 (s, 1H), 7.88 (s, 1H), 7.75 (s, 1H), 7.38 (d, *J* = 7.2 Hz, 1H), 7.05 (d, *J* = 2.4 Hz, 1H), 6.92 (d, *J* = 8.6 Hz, 1H), 6.77 (dd, *J* = 8.7, 2.5 Hz, 1H), 5.48 (s, 1H), 4.34 (s, 2H), 3.81 - 3.70 (m, 4H), 3.07 - 3.02 (m, 4H), 1.62 (s, 6H), 1.51 (s, 6H). |
| 267 | 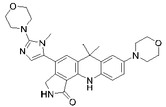 | 515 | ¹H NMR (400 MHz, DMSO-*d₆*) δ ppm: 8.77 (s, 1H), 8.51 (s, 1H), 7.47 (s, 1H), 7.01 (d, *J =* 2.4 Hz, 1H), 6.86 - 6.77 (m, 2H), 6.71 (dd, *J =* 8.7, 2.5 Hz, 1H), 4.31 (s, 2H), 3.78 - 3.71 (m, 8H), 3.36 (s, 3H), 3.04 - 2.97 (m, 8H), 1.56 (s, 6H). |
| 268 | 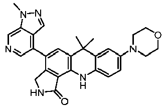 | 481 | ¹H NMR (400 MHz, DMSO-*d₆*) δ ppm: 9.15 (s, 1H), 8.96 (s, 1H), 8.60 (s, 1H), 8.40 (s, 1H), 8.12 (s, 1H), 7.73 (s, 1H), 7.07 (d, *J* = 2.4 Hz, 1H), 6.92 (d, *J =* 8.6 Hz, 1H), 6.78 (dd, *J =* 8.6, 2.5 Hz, 1H), 4.43 (s, 2H), 4.23 (s, 3H), 3.80 - 3.69 (m, 4H), 3.11 - 2.99 (m, 4H), 1.63 (s, 6H). |
| 269 | 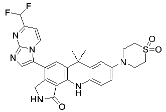 | 565 | ¹H NMR (400 MHz, DMSO-*d₆*) δ ppm: 8.97 (s, 1H), 8.86 (d, *J* = 7.1 Hz, 1H), 8.61 (s, 1H), 8.14 (s, 1H), 7.83 (s, 1H), 7.34 (d, *J* = 7.1 Hz, 1H), 7.21 - 7.12 (m, 1H), 7.05 (t, *J* = 54.5 Hz, 1H), 6.96 (d, *J =* 8.6 Hz, 1H), 6.89 - 6.83 (m, 1H), 4.36 (s, 2H), 3.71 - 3.60 (m, 4H), 3.20 - 3.11 (m, 4H), 1.64 (s, 6H). |
| 270 | 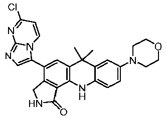 | 501 | ¹H NMR (400 MHz, DMSO-*d₆*) δ ppm: 8.92 (s, 1H), 8.69 (d, *J* = 7.2 Hz, 1H), 8.60 (s, 1H), 7.96 (s, 1H), 7.78 (s, 1H), 7.18 (d, *J* = 7.2 Hz, 1H), 7.05 (d, *J* = 2.5 Hz, 1H), 6.92 (d, *J* = 8.6 Hz, 1H), 6.77 (dd, *J =* 8.6, 2.5 Hz, 1H), 4.34 (s, 2H), 3.79 - 3.72 (m, 4H), 3.09 - 3.01 (m, 4H), 1.64 (s, 3H), 1.62(s, 3H). |
| 271 | 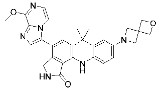 | 509 | ¹H NMR (400 MHz, DMSO-*d₆*) δ ppm: 8.83 (s, 1H), 8.56 (s, 1H), 7.86 (d, *J* = 4.8 Hz, 1H), 7.83 (s, 1H), 7.66 (s, 1H), 7.42 (d, *J =* 4.8 Hz, 1H), 6.84 (d, *J =* 8.4 Hz, 1H), 6.50 (s, 1H), 6.33 - 6.24 (m, 1H), 4.67 (s, 4H), 4.28 (s, 2H), 4.03 (s, 3H), 3.88 (s, 4H), 1.56 (s, 6H). |
| 272 | 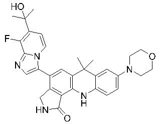 | 542 | ¹H NMR (400 MHz, DMSO-*d₆*) δ ppm: 8.92 (s, 1H), 8.60 (s, 1H), 8.07 (d, *J =* 7.2 Hz, 1H), 7.79 (s, 1H), 7.71 (s, 1H), 7.20 (t, *J* = 7.0 Hz, 1H), 7.06 (d, *J* = 2.1 Hz, 1H), 6.92 (d, *J* = 8.6 Hz, 1H), 6.77 (dd, *J* = 8.6, 2.4 Hz, 1H), 4.32 (s, 2H), 3.77 - 3.73 (m, 4H), 3.12 - 3.00 (m, 4H), 1.62 (s, 6H), 1.58 (s, 6H). |
| 273 | 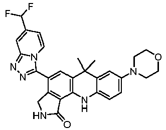 | 517 | ¹H NMR (400 MHz, DMSO-*d₆*) δ ppm: 9.03 (s, 1H), 8.62 (s, 1H), 8.48 (d, *J* = 7.2 Hz, 1H), 8.14 (s, 1H), 7.99 (s, 1H), 7.34 - 7.01 (m, 3H), 6.94 (s, 1H), 6.79 (dd, *J* = 8.7, 2.5 Hz, 1H), 4.46 (s, 2H), 3.80 - 3.70 (m, 4H), 3.11 - 3.00 (m, 4H), 1.65 (s, 6H). |
| 274 | 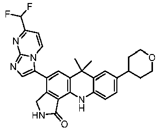 | 516 | ¹H NMR (400 MHz, DMSO-*d₆*) δ ppm: 9.04 (s, 1H), 8.87 (d, *J* = 7.1 Hz, 1H), 8.65 (s, 1H), 8.15 (s, 1H), 7.85 (s, 1H), 7.37 - 7.31 (m, 2H), 7.05 (t, *J* = 54.5 Hz, 1H), 7.02 (dd, *J* = 8.2, 1.6 Hz, 1H), 6.94 (d, *J* = 8.1 Hz, 1H), 4.37 (s, 2H), 3.98 - 3.92 (m, 2H), 3.45 - 3.40 (m, 2H), 2.77 - 2.64 (m, 1H), 1.72 - 1.58 (m, 10H). |
| 275 | 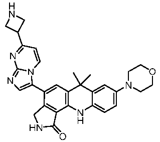 | 522 | ¹H NMR (400 MHz, DMSO-*d₆*) δ ppm: 8.92 (s, 1H), 8.68 - 8.56 (m, 2H), 7.92 (s, 1H), 7.73 (s, 1H), 7.07 (dd, *J* = 15.1, 4.6 Hz, 2H), 6.92 (d, *J* = 8.6 Hz, 1H), 6.77 (dd, *J* = 8.7, 2.4 Hz, 1H), 4.33 (s, 2H), 4.28 - 4.12 (m, 1H), 4.10 - 3.92 (m, 4H), 3.79 - 3.73 (m, 4H), 3.06 - 3.02 (m, 4H), 1.62 (s, 6H). |
| 276 | 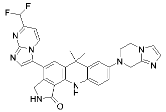 | 553 | ¹H NMR (400 MHz, DMSO-*d₆*) δ ppm: 8.97 (s, 1H), 8.87 (d, *J* = 7.1 Hz, 1H), 8.62 (s, 1H), 8.15 (s, 1H), 7.85 (s, 1H), 7.34 (d, *J* = 7.1 Hz, 1H), 7.20 (d, *J* = 2.6 Hz, 1H), 7.10 (d, *J* = 1.0 Hz, 1H), 7.05 (t, *J* = 55.6 Hz, 1H), 6.97 (d, *J* = 8.6 Hz, 1H), 6.90 (dd, *J* = 7.5, 1.8 Hz, 2H), 4.36 (s, 2H), 4.30 (s, 2H), 4.09 (t, *J =* 5.4 Hz, 2H), 3.61 (t, *J =* 5.4 Hz, 2H), 1.65 (s, 6H). |
| 277 | 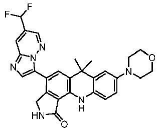 | 517 | ¹H NMR (400 MHz, DMSO-*d₆*) δ ppm: 8.94 (s, 1H), 8.81 (d, *J* = 1.9 Hz, 1H), 8.62 (s, 1H), 8.46 (s, 1H), 8.19 (s, 1H), 8.15 (s, 1H), 7.19 (t, *J =* 55.0 Hz, 1H), 7.03 (d, *J =* 2.6 Hz, 1H), 6.88 (d, *J* = 8.6 Hz, 1H), 6.74 (dd, *J* = 8.8, 2.4 Hz, 1H), 4.44 (s, 2H), 3.85 - 3.61 (m, 4H), 3.06 - 2.85 (m, 4H), 1.59 (s, 6H). |
| 278 | 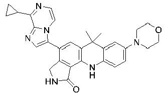 | 507 | ¹H NMR (400 MHz, DMSO-*d₆*) δ ppm: 9.04 (s, 1H), 8.65 (s, 1H), 8.18 - 8.01 (m, 2H), 7.80 - 7.77 (m, 2H), 7.21 (s, 1H), 7.09 - 6.84 (m, 2H), 4.40 - 4.32 (m, 2H), 3.85 - 3.74 (m, 4H), 3.25 - 3.08 (m, 4H), 2.99 - 2.91 (m, 1H), 1.63 (s, 6H), 1.26 - 1.16 (m, 4H). |
| 279 | 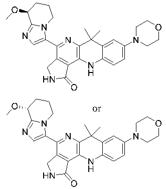 | 501 | ¹H NMR (400 MHz, DMSO-*d₆*) δ ppm: 8.93 (s, 1H), 8.51 (s, 1H), 7.31 (s, 1H), 7.08 - 7.03 (m, 2H), 6.77 (dd, *J =* 8.7, 2.4 Hz, 1H), 4.58 - 4.47 (m, 3H), 4.37 (t, *J* = 6.8 Hz, 1H), 4.17 (d, *J =* 10.7 Hz, 1H), 3.78 - 3.70 (m, 4H), 3.44 (s, 3H), 3.08 - 2.99 (m, 4H), 2.08 (s, 2H), 1.97 (m, 2H), 1.63 (d, *J =* 5.2 Hz, 6H). |
| 280 | 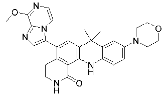 | 511 | ¹H NMR (400 MHz, DMSO-*d₆*) δ ppm: 11.31 (s, 1H), 8.16 (s, 1H), 7.73 - 7.68 (m, 2H), 7.52 (s, 1H), 7.41 (d, *J =* 4.7 Hz, 1H), 7.02 (s, 1H), 6.77 (s, 2H), 4.07 (s, 3H), 3.79 - 3.67 (m, 4H), 3.27 - 3.21 (m, 2H), 3.09 - 2.98 (m, 4H), 2.59 - 2.52 (m, 2H), 1.54 (s, 6H). |
| 281 | 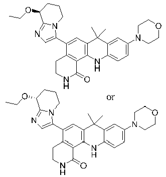 | 528 | ¹H NMR (400 MHz, DMSO-*d₆*) δ ppm: 11.27 (br s, 1H), 8.14 (s, 1H), 7.49 (s, 1H), 7.27 (s, 1H), 7.02 (s, 1H), 6.78 - 6.73 (m, 2H), 4.66 - 5.56 (m, 1H), 4.02 - 3.58 (m, 8H), 3.33 - 3.25 (m, 2H), 3.10 - 2.85 (m, 4H), 2.73 - 2.55 (m, 2H), 2.10 - 1.80 (m, 4H), 1.53 (s, 6H), 1.17 (t, *J =* 6.9 Hz, 3H). |
| 282 | 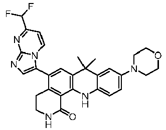 | 531 | ¹H NMR (400 MHz, DMSO-*d₆*) δ ppm:11.28 (s, 1H), 8.65 (d, *J* = 7.3 Hz, 1H), 7.93 (s, 1H), 7.57 (s, 1H), 7.25 (d, *J* = 6.9 Hz, 1H), 7.01 (t, *J* = 54.4 Hz, 1H), 6.99 (s, 1H), 6.76 - 6.69 (m, 2H), 3.78 - 3.65 (m, 4H), 3.24 - 3.15 (m, 2H), 3.04 - 2.95 (m, 4H), 2.58 - 2.50 (m, 2H), 1.51 (s, 6H). |
| 283 | 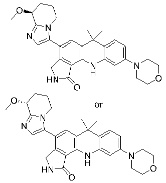 | 500 | ¹H NMR (400 MHz, DMSO-*d₆*) δ ppm: 8.92 (s, 1H), 8.59 (s, 1H), 7.57 (s, 1H), 7.27 (d, *J* = 9.3 Hz, 1H), 7.11 (s, 1H), 6.54 - 6.51 (m, 2H), 4.43 - 4.25 (m, 3H), 3.89 (s, 2H), 3.75 - 3.68 (m, 4H), 3.44 (s, 3H), 3.13 - 3.06 (m, 4H), 2.12 - 1.80 (m, 4H), 1.55 (d, *J =* 8.3 Hz, 6H). |
| 284 | 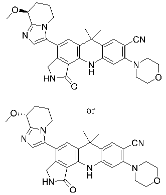 | 525 | ¹H NMR (400 MHz, DMSO-*d₆*) δ ppm: 9.37 (s, 1H), 8.71 (s, 1H), 7.77 (s, 1H), 7.64 (s, 1H), 7.15 (s, 1H), 6.86 (s, 1H), 4.47 - 4.28 (m, 3H), 3.96 - 3.87 (m, 2H), 3.80 - 3.70 (m, 4H), 3.45 (s, 3H), 3.16 - 3.07 (m, 4H), 2.05 - 1.95 (m, 4H), 1.59 (d, *J* = 8.2 Hz, 6H). |
| 285 | 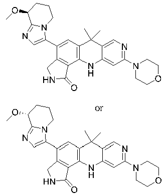 | 501 | ¹H NMR (400 MHz, DMSO-*d₆*) δ ppm: 10.08 (s, 1H), 8.97 (s, 1H), 8.05 (s, 1H), 7.99 (s, 1H), 7.90 (s, 1H), 7.20 (s, 1H), 4.83 (t, *J* = 4.6 Hz, 1H), 4.51 - 4.38 (m, 2H), 4.14 - 4.05 (m, 1H), 4.01 - 3.95 (m, 1H), 3.81 - 3.69 (m, 4H), 3.59 - 3.51 (m, 4H), 3.50 (s, 3H), 2.21 - 1.94 (m, 4H), 1.65 (s, 6H). |

The synthetic methods of the following examples refer to example 10:

| Example | Structure | MS:M+ H⁺ | ¹H NMR |
|---|---|---|---|
| **286** | 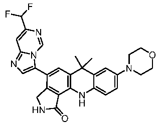 | 517 | ¹H NMR (400 MHz, DMSO-*d₆*) δ ppm: 9.48 (s, 1H), 8.99 (s, 1H), 8.70 (s, 1H), 8.55 (s, 1H), 8.25 (s, 1H), 7.93 (s, 1H), 7.18 - 6.86 (m, 3H), 6.77 (dd, *J* = 8.6, 2.4 Hz, 1H), 4.67 (s, 2H), 3.80 - 3.68 (m, 4H), 3.10 - 2.98 (m, 4H), 1.65 (s, 6H). |
| **287** | 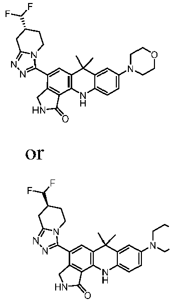 | 521 | ¹H NMR (400 MHz, CDCl₃) δ ppm: 8.94 (s, 1H), 8.59 (s, 1H), 7.74 (s, 1H), 7.07 (s, 1H), 6.92 (d, *J* = 8.6 Hz, 1H), 6.77 (d, *J* = 8.0 Hz, 1H), 6.39 - 6.05 (m, 1H), 4.57 (d, *J* = 18.9 Hz, 1H), 4.31 (d, *J* = 18.9 Hz, 1H), 4.29 - 4.08 (m, 2H), 3.78 - 3.72 (m, 4H), 3.18 - 3.10 (m, 2H), 3.09 - 3.01 (m, 4H), 2.84 - 2.73 (m, 1H), 2.18 (d, *J* = 13.1 Hz, 1H), 1.83 - 1.73 (m, 1H), 1.62 (d, *J* = 19.6 Hz, 6H). |
| **288** | 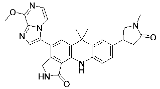 | 509 | ¹H NMR (400 MHz, DMSO-*d₆*) δ ppm: 9.07 (s, 1H), 8.66 (s, 1H), 7.91 (d, *J =* 4.8 Hz, 1H), 7.88 (s, 1H), 7.75 (s, 1H), 7.46 (d, *J =* 4.8 Hz, 1H), 7.41 (d, *J =* 1.2 Hz, 1H), 7.07 (dd, *J =* 8.2, 1.6 Hz, 1H), 7.00 (s, 1H), 4.35 (s, 2H), 4.07 (s, 3H), 3.72 - 3.64 (m, 1H), 3.57 - 3.46 (m, 1H), 2.78 (s, 3H), 2.66 - 2.56 (m, 2H), 2.42 - 2.37 (m, 1H), 1.62 (s, 6H). |
| **289** | 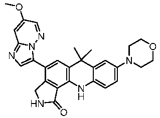 | 497 | ¹H NMR (400 MHz, DMSO-*d₆*) δ ppm: 8.93 (s, 1H), 8.64 (s, 1H), 8.49 (d, *J =* 2.8 Hz, 1H), 8.13 (s, 1H), 7.84 (s, 1H), 7.61 (d, *J =* 2.8 Hz, 1H), 7.07 (d, *J =* 2.4 Hz, 1H), 6.91 (d, *J =* 8.6 Hz, 1H), 6.78 (dd, *J =* 8.7, 2.5 Hz, 1H), 4.45 (s, 2H), 3.94 (s, 3H), 3.81 - 3.71 (m, 4H), 3.10 - 3.00 (m, 4H), 1.63 (s, 6H). |

### Biological activity assay

### Assay example 1 HPK1 ADP-Glo^{™} kinase assay

### 1. Preparation of compound

A compound to be assayed was serially diluted in DMSO, and 50 nL of the compound in 384 LDV plate (100, 33.33, 11.11, 3.70, 1.235, 0.412, 0.14, 0.05, 0.015, and 0.0051 µM) was transferred to an assay plate by liquid handling station ECHO. Final concentrations of the compound in the assay plate were 1000, 333.33, 111.11, 37.04, 12.35, 4.12, 1.37, 0.46, 0.15, and 0.05 nM.

### 2. Assay procedure

The assay was performed in a 384-well plate (corning, Cat#4512) with a total reaction system of 20 µL. 1×kinase buffer (40 mM Tris-HCl (pH 7.5), 20 mM MgCl₂, 0.1 mg/ml BSA, 0.05 mM DTT), 2 µM MBP protein and 40 µM ATP, 5 µL of ADP-Glo^{™} reagent, and 10 µL of kinase assay reagent were included in the reaction mixture. The reaction was initiated by adding 5 nM HPK1. After 60 minutes of reaction, the assay was terminated by adding 5 µL of ADP-Glo^{™} reagent. After 60 minutes, 10 µL of kinase assay reagent was added. After being incubated at room temperature for 60 minutes, the assay plate was read by EnVision multi-function microplate reader.

### 3. Data analysis

The luminescent signal value was converted to the percentage of inhibition. Inhibition % = (maximum value - sample value) / (maximum value - minimum value)×100. The minimum value refers to the value measured in a well without enzyme but with DMSO and substrate, and the maximum value refers to the value measured in a well with DMSO, enzyme and substrate. IC₅₀ values were calculated by XLFit (version 5.3.1.3) in Excel. Equation: Y = bottom + (top - bottom) / (1 + (IC₅₀/X) ^ HillSlope).

**Table 1. Results of in vitro enzymatic activity of the compounds (40 µM ATP)**

| Example | HPK1, IC₅₀ (nM) | Example | HPK1, IC₅₀ (nM) |
|---|---|---|---|
| 1 | 0.1 | 2 | 1.7 |
| 3 | 0.3 | 5 | 3.44 |

### Assay example 2 HPK1 HTRF kinase assay

### 1. Preparation of compound

A compound was serially diluted in DMSO, and 100 nL of the compound in 384 LDV plate (1000, 333.33, 111.11, 37.037, 12.35, 4.12, 1.4, 0.5, 0.15, and 0.051 µM) was transferred to an assay plate by liquid handling station ECHO. Final concentrations of the compound in the assay plate were 10000, 3333.33, 1111.11, 370.4, 123.5, 41.2, 13.7, 4.6, 1.5 and 0.51 nM.

### 2. Assay procedure

The assay was performed in a 384-well plate (Greier, Cat#784075) with a total reaction system of 20 µL. 1×kinase buffer (5*enzyme buffer, 5 mM MgCl₂, 0.1% BSA, 1 mM DTT), 1 µM STK1 substrate and 1000 µM ATP, and 10 µL of Detection Mix (0.25 nM STK antibody, 62.5 nM XL665) were included in the reaction mixture. The reaction was initiated by adding 50 nM HPK1. After 120 minutes of reaction, the assay was terminated by adding 10 µL of Detection Mix. After being incubated at room temperature for 60 minutes, the assay plate was read by EnVision multi-function microplate reader to read the FRET signal (the ratio of HTRF 665/615 was calculated from the readings at 665 nm and 615 nm).

### 3. Data analysis

The ratio of the signal at 665 nm to the signal at 615 nm was converted to the percentage of inhibition. Inhibition % = (maximum value - sample value) / (maximum value - minimum value)×100. The minimum value refers to the value measured in a well without enzyme but with DMSO and substrate, and the maximum value refers to the value measured in a well with DMSO, enzyme and substrate. IC₅₀ values were calculated by XLFit (version 5.3.1.3) in Excel. Equation: Y = Bottom + (Top - Bottom) / (1 + (IC₅₀/X) ^ HillSlope).

**Table 2. Results of in vitro enzymatic activity of the compounds (1000 µM ATP)**

| Example | HPK1, IC₅₀ (nM) | Example | HPK1, IC₅₀ (nM) |
|---|---|---|---|
| 9 | 20.38 | 10 | 8.39 |
| 11 | 7.38 | 12 | 6.99 |
| 13 | 9.32 | 14 | 30.33 |
| 16 | 2.85 | 17 | 6.31 |
| 18 | 11.75 | 19 | 6.31 |
| 20 | 5.76 | 23 | 3.65 |

**Table 3. Results of in vitro enzymatic activity of the compounds (1000 µM ATP)**

| Example | HPK1, IC₅₀ (nM) | Example | HPK1, IC₅₀ (nM) |
|---|---|---|---|
| 22 | 11.21 | 24 | 11.79 |
| 26 | 14.51 | 27 | 11.74 |
| 28 | 21.51 | 29 | 10.12 |
| 31 | 24.8 | 32 | 16.42 |
| 33 | 11.45 | 34 | 11.51 |
| 35 | 12.77 | 36 | 11.8 |
| 37 | 7.98 | 38 | 12.25 |
| 39 | 39.04 | 40 | 12.28 |
| 46 | 10.29 | 50 | 8.71 |
| 51 | 11.39 | 52 | 8.2 |
| 53 | 7.97 | 54 | 3.81 |
| 55 | 7.55 | 56 | 11.47 |
| 57 | 11.01 | 58 | 4148 |
| 59 | 7.78 | 60 | 14.42 |
| 61 | 8.55 | 62 | 6.08 |
| 63 | 5.48 | 64 | 5.28 |
| 65 | 8.59 | 66 | 11.07 |
| 67 | 3.93 | 68 | 12.02 |
| 69 | 6.86 | 70 | 12.71 |
| 71 | 11.04 | 72 | 2.13 |
| 73 | 7.87 | 74 | 49.68 |
| 75 | 4.55 | 76 | 8.73 |
| 77 | 4.56 | 78 | 35.05 |
| 79 | 6.36 | 80 | 9.18 |
| 81 | 6.31 | 82 | 6.62 |
| 83 | 5.80 | 84 | 8.63 |
| 85 | 36.23 | 86 | 7.95 |
| 87 | 7.88 | 88 | 11.62 |
| 89 | 8.13 | 90 | 6.72 |
| 91 | 4.24 | 92 | 7.64 |

### Assay example 3 Phospho-SLP-76 (Ser376) HTRF assay

### 1. Preparation of compound

A compound was dissolved in DMSO to 10 mM, and then diluted three-fold with DMSO to 10 dosages in a 96-well plate. Then, 3 µL of the diluted compound sample was transferred to a 96-well plate with 97 µL of cell culture medium, and the plate was shaken for 5 minutes. According to the experimental design diagram, 7.5 µL of the diluted compound sample was transferred to a cell plate. Final concentrations of the compound in this assay were 10000.00, 3333.33, 1111.11, 370.37, 123.46, 41.15, 13.72, 4.57, 1.52 and 0.51 nM.

### 2. Assay procedure

In this assay, the density of Jurkat E6-1 cells was maintained between 3*10⁵ to 3*10⁶ cells/mL. The cell culture was replaced with fresh culture medium every 2 to 3 days (depending on the cell density).

30 µL of Jurkat E6-1 (300,000 cells) was added to a 96-well plate and incubated in a 37 °C incubator for 1 hour. Then, according to the experimental design diagram, compounds (6×, 7.5 µL) were added to treat the cells for 4 hours, respectively. After 4 hours, purified anti-human anti-CD3 antibodies (6×, 7.5 µL) were added to treat the cells for 20 minutes. After 20 minutes, 15 µL of cell lysis buffer (4×) was added, and the cells were lysed at 25 °C with shaking (250 rpm) for 1 hour. Then, according to the experimental design diagram, 16 µL of cell lysate was transferred to a 384-well plate using a pipette. The experimental design diagram is shown in the following table. After the transfer, 4 µL of antibody detection mixture was added. After being placed at room temperature overnight, the plate was read by EnVision multi-function microplate reader to read the FRET signal (the ratio of HTRF 665/615 was calculated from the readings at 665 nm and 615 nm).

### 3. Data analysis

The ratio of the signal at 665 nm to the signal at 615 nm was converted to the percentage of inhibition. Inhibition % = (maximum value - sample value) / (maximum value - minimum value)×100. The minimum value refers to the signal value of a well without anti-CD3 antibody stimulation during the treatment of cells, and the maximum value refers to the signal value of a well with anti-CD3 antibody stimulation but without the compound during the treatment of cells. IC₅₀ values were calculated by XLFit (version 5.3.1.3) in excel. Equation: Y = Bottom + (Top-Bottom) / (1 + (IC₅₀/X) ^ HillSlope).

**Table 4. Results of cellular activity of the compounds**

| Example | pSLP76, IC₅₀ (nM) | Example | pSLP76, IC₅₀ (nM) |
|---|---|---|---|
| 1 | 120.88 | 2 | 64 |
| 3 | 57.52 | 4 | 38.09 |
| 6 | 163.57 | 7 | 487.23 |
| 8 | 103.35 | 9 | 57.82 |
| 10 | 42.73 | 11 | 73.35 |
| 12 | 25.88 | 13 | 24.35 |
| 14 | 196.58 | 15 | 72.2 |
| 16 | 31.6 | 17 | 56.87 |
| 18 | 65.96 | 20 | 83.58 |
| 21 | 37.04 | | |

**Table 5. Results of cellular activity of the compound**

| Example | pSLP76, IC₅₀ (nM) | Example | pSLP76, IC₅₀ (nM) |
|---|---|---|---|
| 31 | 58.81 | 41 | 13.53 |

### Assay example 4 Human Pan T cell IL-2 ELISA

### 1. Preparation of compound

A compound was dissolved in DMSO to 10 mM, and then diluted three-fold with DMSO to 9 dosages in a 96-well plate. Then, 10 µL of the diluted compound sample was transferred to a 96-well plate with 90 µL of cell culture medium, and the plate was shaken for 5 minutes. Then, 5 µL of the diluted compound sample was transferred to a 96-well plate with 120 µL of cell culture medium, and the plate was shaken for 5 minutes. According to the experimental design diagram, 50 µL of the diluted compound sample was transferred to a cell plate. Final concentrations of the compound in this assay were 10000.00, 5000, 2500, 1250, 625, 312.5, 156.25, 78.125 and 39.063 nM.

### 2. Assay procedure

The assay was conducted in a 96-well plate. On the first day of the assay, Anti-CD3 was added to the 96-well plate and incubated in a 4 °C refrigerator overnight. On the second day of the assay, human Pan T cells were recovered, centrifuged and counted. The cell density was adjusted to 1*10⁶ cells/ml. Then, 100 µL of cells were added to the 96-well plate washed with PBS and added with Anti-CD3; according to the experimental design diagram, 50 µL of diluted Anti-CD28 and 50 µL of the diluted compound were added to each well, centrifuged, and the cell plate was placed and incubated in an incubator for 48 hours; after 48 hours, the plate was centrifuged and the supernatant was collected for IL-2 ELISA.

### 3. Data analysis

The signal value of light absorption was converted into a multiple. Multiple = sample value/the average value of control wells. The average value of control wells refers to the value measured in wells with DMSO added. EC₅₀ values were calculated by XLFit (version 5.3.1.3) in Excel. Equation: Y=Bottom + (Top-Bottom) / (1 + (EC₅₀/X) ^ HillSlope).

**Table 6. Results of cellular activity of the compounds**

| Example | IL-2 release, EC₅₀ (nM) | Example | IL-2 release, EC₅₀ (nM) |
|---|---|---|---|
| 1 | 3.67 | 3 | 38 |
| 4 | 4.61 | 5 | 10.8 |
| 6 | 13 | 8 | 5.5 |
| 9 | 3.09 | 10 | 4.88 |
| 11 | 13.75 | 12 | 6.67 |
| 13 | 3.12 | 14 | 49.15 |
| 15 | 3.95 | 16 | 6.81 |
| 17 | 3.72 | 18 | 11.77 |
| 19 | 2.11 | 20 | 11.28 |

**Table 7. Results of cellular activity of the compounds**

| Example | IL-2 release, EC₅₀ (nM) | Example | IL-2 release, EC₅₀ (nM) |
|---|---|---|---|
| 21 | 8.32 | 22 | 2.2 |
| 23 | < 1.5 | 24 | 1.83 |
| 25 | 33.44 | 26 | 14.93 |
| 27 | 1.79 | 28 | 95.87 |
| 29 | 27.91 | 31 | 3.7 |
| 32 | 12.32 | 33 | 4.95 |
| 34 | 2.41 | 35 | 14.48 |
| 36 | 6.33 | 37 | 2.27 |
| 38 | 4.7 | 39 | 16.8 |
| 40 | 5.07 | 41 | 6.09 |
| 42 | 14.15 | 43 | 22.3 |
| 44 | 2.47 | 45 | 5.28 |
| 46 | 7.73 | 47 | 4.62 |
| 48 | 13.53 | 49 | 12.67 |
| 50 | 18.96 | 51 | 58.83 |
| 52 | 9.08 | 53 | 5.54 |
| 54 | 6.53 | 55 | 40.28 |
| 56 | 28.01 | 57 | 27.1 |
| 59 | 6.97 | 60 | 17.94 |
| 61 | 17.71 | 62 | 3.02 |
| 63 | 4.98 | 64 | 3.51 |
| 65 | 28.04 | 66 | 7.83 |
| 67 | 14.37 | 68 | 21.09 |
| 69 | 11.37 | 70 | 23.4 |
| 71 | 68.56 | 72 | 18.1 |
| 73 | 28.9 | 74 | 100.46 |
| 75 | 1.41 | 76 | 50.36 |
| 77 | 13.58 | 78 | 75.51 |
| 79 | 19.46 | 80 | 86.59 |
| 81 | 35.78 | 82 | 27.03 |
| 83 | 17.38 | 84 | 65.87 |
| 85 | 69.36 | 86 | 11.5 |
| 87 | 8.97 | 88 | 12.32 |
| 89 | 6.64 | 90 | 6.30 |
| 91 | 5.96 | 92 | 68.2 |
| 93 | 6.33 | 94 | 4.84 |
| 95 | 7.82 | 96 | 5.91 |
| 98 | NA | 99 | 5.52 |
| 100 | 13.05 | 101 | 5.58 |
| 102 | 10.77 | 103 | 10.41 |
| 104 | 6.44 | 105 | 11.58 |
| 106 | 18.26 | 107 | 11.93 |
| 108 | 5.58 | 109 | 9.18 |
| 110 | 15.95 | 111 | 30.76 |
| 112 | 4.67 | 113 | 13.96 |
| 114 | 10.90 | 115 | 91.47 |
| 116 | 65.25 | 118 | 7.32 |
| 119 | 8.52 | 120 | 66.69 |
| 122 | 4.3 | 123 | 20.29 |
| 124 | 15.65 | 125 | 7.23 |
| 127 | 12.42 | 129 | 18.17 |
| 131 | 8.27 | 132 | 10.06 |
| 133 | 22.15 | 134 | 19.46 |
| 136 | 24.54 | 137 | 26.77 |
| 140 | 35.75 | 141 | 87.35 |
| 142 | 25.15 | 143 | 591.79 |
| 144 | 133.77 | 145 | 6.50 |
| 146 | 5.70 | 147 | 42.68 |
| 148 | 21.79 | 149 | NA |
| 150 | 30.64 | 151 | 68.84 |
| 152 | 34.59 | 153 | 46.76 |
| 154 | 7.35 | 155 | 26.63 |
| 156 | 2.76 | 157 | 6.47 |
| 158 | 37.59 | 159 | 23.56 |
| 160 | 12.58 | 161 | 6.36 |
| 162 | 6.33 | | |

**Table 8. Results of cellular activity of the compounds**

| Example | IL-2 release, EC₅₀ (nM) | Example | IL-2 release, EC₅₀ (nM) |
|---|---|---|---|
| 163 | 25 | 164 | 17 |
| 165 | 4.8 | 166 | 15 |
| 167 | 38 | 168 | 17 |
| 169 | 6.4 | 171 | 6.3 |
| 172 | 8.1 | 173 | 3.8 |
| 174 | 39 | 175 | 14 |
| 176 | 31 | 177 | 25 |
| 178 | 19 | 179 | 6.9 |
| 180 | 27 | 181 | 26 |
| 182 | 20 | 184 | 14 |
| 185 | 2.2 | 186 | 7.9 |
| 187 | 15 | 188 | 73 |
| 189 | 5.7 | 190 | 53 |
| 191 | 28 | 192 | 19 |
| 193 | 9.5 | 194 | 4.3 |
| 195 | 3.7 | 196 | 27 |
| 197 | 11 | 198 | 5.3 |
| 199 | 24 | 200 | 1.4 |
| 201 | 2.7 | 202 | 8.7 |
| 203 | 95 | 204 | 15 |
| 205 | 6.6 | 206 | 99 |
| 207 | 23 | 208 | 16 |
| 209 | 3.3 | 211 | 55 |
| 212 | 11 | 213 | 19 |
| 216 | 5.4 | 217 | 19 |
| 218 | 19 | 219 | 8.1 |
| 220 | 50 | 221 | 98 |
| 222 | 6.4 | 224 | 33 |
| 225 | 8.1 | 227 | 6.4 |
| 228 | 11 | 229 | 14 |
| 230 | 7.6 | 231 | 10.5 |
| 232 | 11 | 237 | 8.7 |
| 238 | 58 | 239 | 7.5 |
| 240 | 26 | 241 | 10 |
| 242 | 8.5 | 243 | 13 |
| 244 | 9.8 | 246 | 9.2 |
| 247 | 9.2 | 248 | 1.9 |
| 249 | 4.7 | 250 | 3.1 |
| 251 | 6.4 | 253 | 54 |
| 254 | 28 | 255 | 5 |
| 256 | 16 | 257 | 154 |
| 258 | 6.1 | 279 | 170 |
| 280 | 13 | 281 | 18 |
| 282 | 44 | | |

## Claims

1. A compound of formula (I'), or a stereoisomer, an N-oxide, a hydrate, a solvate, a metabolite, a pharmaceutically acceptable salt, a polymorph, or a prodrug thereof: 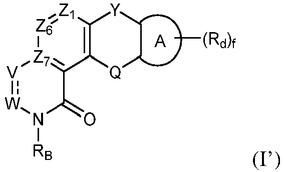
V is selected from covalent bond, O, S, NR', S(O), S(O)₂, C(O), CR_{C} and CR_{C}R_{C}';
W is selected from NR', S(O), S(O)₂, C(O), CR_{C} and CR_{C}R_{C}';
Z₇ is C;
or V is not connected to W, Z₇ is C or N, W is selected from H, C₁₋₆ alkyl and C₁₋₆ haloalkyl, alternatively selected from H and C₁₋₆ alkyl, V is selected from absent, H, D, halogen, C₁₋₈ alkyl and C₁₋₈ haloalkyl, alternatively selected from absent, H, D and halogen;
Q and Y are independently selected from covalent bond, -O-, -S-, -NR_{A}-, -S(O)-, -S(O)₂-, -C(O)-, -C(O)O-, -C(O)NR_{A}-, -OC(O)-, -OC(O)NR_{A}-, -N(R_{A})C(O)O-, -N(R_{A})C(O)- and -N(R_{A})S(O)₂-; and Q and Y are not covalent bonds at the same time;
or Q and Y are independently C₁₋₈ alkylene, in which one or more methylene units are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
R_{A} is selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl;
R_{B} is selected from H, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
R_{C}, R'_{C}, R₁ₐ and R_{1b} are independently selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
one of Z₁ and Z₆ is CR_{E}, and the other one is CR_{d1} or N;
R_{E} is selected from -L-C₆₋₁₀ aryl, -L-3- to 14-membered carbocyclyl, -L-3- to 14-membered heterocyclyl and -L-5- to 14-membered heteroaryl, wherein each of the C₆₋₁₀ aryl, 3- to 14-membered carbocyclyl, 3- to 14-membered heterocyclyl and 5- to 14-membered heteroaryl is optionally substituted with 1, 2, 3, 4, 5, 6, 7, 8, 9 or 10 Rₑ groups;
Rₑ is independently selected from H, D, halogen, -CN, oxo, C₁₋₈ alkyl, C₁₋₈ haloalkyl, C₂₋₈ alkenyl, C₂₋₈ alkynyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2 or 3 Rₑₛ;
or two geminal Rₑ are taken together with the atom to which they are jointly attached to form a 3- to 7-membered carbocyclyl or a 3- to 7-membered heterocyclyl, which is optionally substituted with 1, 2 or 3 Rₑₛ;
Rₑₛ is independently selected from H, D, halogen, CN, C₁₋₆ alkyl, C₁₋₆ haloalkyl, -L'-OR' and -L'-NR'R";
R_{d1} is selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
ring A is selected from C₆₋₁₀ aromatic ring, 3- to 14-membered carbocycle, 3- to 14-membered heterocycle and 5- to 14-membered heteroaromatic ring;
R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or R_{d} is selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₈ alkyl, C₂₋₈ alkenyl, C₂₋₈ alkynyl, C₁₋₈ haloalkyl, -L" -OR', -L"-NR'R", -L"-C(O)OR', -L"-C(O)NR'R", -L"-S(O)₀₋₂R', -L"-S(O)₂NR'R", -L" -S(O)NR'R", -L"-OC(O)R', -L"-OC(O)NR'R", -L"-C₆₋₁₀ aryl, -L''-3- to 14-membered carbocyclyl, -L"-3- to 14-membered heterocyclyl and -L"-5- to 14-membered heteroaryl;
or CR_{dd}R_{dd} forms a 3- to 7-membered carbocyclylene or a 3- to 7-membered heterocyclylene, which is optionally substituted with 1, 2 or 3 groups selected from: H, D, halogen, C₁₋₆ alkyl, C₁₋₆ haloalkyl, -OR' and -NR'R" ;
or two non-adjacent R_{dd} are taken together to form a C₁₋₈ alkylene, which is optionally substituted with one or more of the following substituents: H, D, halogen, CN, C₁₋₈ alkyl, C₁₋₈ alkoxy, C₁₋₈ haloalkyl, -L"-C₆₋₁₀ aryl, -L" -3- to 10-membered carbocyclyl, -L''-3- to 10-membered heterocyclyl and -L"-5- to 10-membered heteroaryl;
or two adjacent R_{dd} are taken together with the atoms to which they are attached to form a 3- to 7-membered carbocyclylene, a 3- to 7-membered heterocyclylene, a phenylene, or a 5- to 6-membered heteroarylene, which is optionally substituted with 1, 2, 3 or 4 substituents selected from: H, D, halogen, CN, C₁₋₆ alkyl, C₁₋₆ haloalkyl, -OR' and -NR'R";
L and L' are independently selected from covalent bond, -O-, -S-, -NR'-, -S(O)₂-, -C(O)-, -C(O)O-, -C(O)NR'-, -OC(O)-, -OC(O)NR'-, -N(R')C(O)O-, -N(R')C(O)- and -N(R*)S(O)₂-; or, L or L' is C₁₋₈ alkylene, in which one or more methylene units are optionally and independently replaced by -C(R')₂-, -N(R')-, -N(R')C(O)-, -C(O)N(R')-, -N(R')S(O)₂-, -S(O)₂N(R')-, -O-, -C(O)-, -OC(O)-, -C(O)O-, -S-, -S(O)-, or -S(O)₂-;
R' and R'' are independently selected from H, C₁₋₈ alkyl, C₁₋₈ alkoxy, C₁₋₈ haloalkyl, -L''-C₆₋₁₀ aryl, -L"-3- to 14-membered carbocyclyl, -L"-3- to 14-membered heterocyclyl and -L"-5- to 14-membered heteroaryl; or, R' and R" are taken together with the N atom to which they are attached to form a 3- to 14-membered heterocyclyl;
L" is selected from covalent bond, -O- and -S-; or, L" is C₁₋₈ alkylene, which is optionally substituted with one or more R, and one or more methylene units in the C₁₋₈ alkylene are optionally and independently replaced by -O-, -C(O)-, -OC(O)-, -C(O)O-, -S-, -S(O)-, or -S(O)₂-;
R is independently selected from H, D, C₁₋₈ alkyl, C₁₋₈ haloalkyl, -L_{d}-3- to 10-membered carbocyclyl and -L_{d}-3- to 10-membered heterocyclyl;
or two R are taken together with the C atom to which they are jointly attached to form a 3- to 10-membered carbocyclylene or a 3- to 10-membered heterocyclylene;
L_{d} is independently selected from covalent bond and C₁₋₈ alkylene;
f=0, 1, 2, 3, 4, 5, 6, 7, 8, 9 or 10;
wherein each of the above groups is optionally deuterated, up to completely deuterated.

2. A compound of formula (I), or a stereoisomer, an N-oxide, a hydrate, a solvate, a metabolite, a pharmaceutically acceptable salt, a polymorph, or a prodrug thereof: 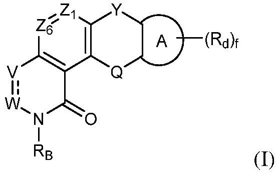
V is selected from covalent bond, O, S, NR', S(O), S(O)₂, C(O), CR_{C} and CR_{C}R_{C}';
W is selected from NR', S(O), S(O)₂, C(O), CR_{C} and CR_{C}R_{C}';
Q and Y are independently selected from covalent bond, -O-, -S-, -NR_{A}-, -S(O)-, -S(O)₂-, -C(O)-, -C(O)O-, -C(O)NR_{A}-, -OC(O)-, -OC(O)NR_{A}-, -N(R_{A})C(O)O-, -N(R_{A})C(O)- and -N(R_{A})S(O)₂-; and Q and Y are not covalent bonds at the same time;
or Q and Y are independently C₁₋₈ alkylene, in which one or more methylene units are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
R_{A} is selected from H, C₁₋₈ alkyl, C₁₋₈ alkoxy, C₁₋₈ haloalkyl, -L"-C₆₋₁₀ aryl, -L''-3- to 14-membered carbocyclyl, -L"-3- to 14-membered heterocyclyl and -L"-5- to 14-membered heteroaryl;
R_{B} is selected from H, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
R_{C}, R'_{C}, R₁ₐ and R_{1b} are independently selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
one of Z₁ and Z₆ is CR_{E}, and the other one is CR_{d1} or N;
R_{E} is selected from -L-C₆₋₁₀ aryl, -L-3- to 14-membered carbocyclyl, -L-3- to 14-membered heterocyclyl and -L-5- to 14-membered heteroaryl, wherein each of the C₆₋₁₀ aryl, 3- to 14-membered carbocyclyl, 3- to 14-membered heterocyclyl and 5- to 14-membered heteroaryl is optionally substituted with 1, 2, 3, 4, 5, 6, 7, 8, 9 or 10 Rₑ groups;
Rₑ is independently selected from H, D, halogen, -CN, oxo, C₁₋₈ alkyl, C₁₋₈ haloalkyl, C₂₋₈ alkenyl, C₂₋₈ alkynyl, -L'-OR', -L'-NR'R", -NO₂, -L'-SR', -L'-C(O)OR', -L'-C(O)NR'R", -L'-S(O)_{2R}', -L'-S(O)₂NR'R", -L'-OC(O)R', -L'-OC(O)NR'R", -L'-C₆₋₁₀ aryl, -L'-3- to 14-membered carbocyclyl, -L'-3- to 14-membered heterocyclyl and -L'-5- to 14-membered heteroaryl;
R_{d1} is selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
ring A is selected from C₆₋₁₀ aromatic ring, 3- to 14-membered carbocycle, 3- to 14-membered heterocycle and 5- to 14-membered heteroaromatic ring;
R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or, R_{d} is selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₈ alkyl, C₂₋₈ alkenyl, C₂₋₈ alkynyl, C₁₋₈ haloalkyl, -L" -OR', -L"-NR'R", -L"-C(O)OR', -L"-C(O)NR'R", -L"-S(O)₀₋₂R', -L"-S(O)₂NR'R", -L" -S(O)NR'R", -L"-OC(O)R', -L"-OC(O)NR'R", -L"-C₆₋₁₀ aryl, -L''-3- to 14-membered carbocyclyl, -L"-3- to 14-membered heterocyclyl and -L"-5- to 14-membered heteroaryl;
or two R_{dd} are taken together to form a C₁₋₈ alkylene, which is optionally substituted with one or more of the following substituents: H, D, halogen, CN, C₁₋₈ alkyl, C₁₋₈ alkoxy, C₁₋₈ haloalkyl, -L"-C₆₋₁₀ aryl, -L"-3-to 10-membered carbocyclyl, -L''-3- to 10-membered heterocyclyl and -L"-5- to 10-membered heteroaryl;
L and L' are independently selected from covalent bond, -O-, -S-, -NR'-, -S(O)₂-, -C(O)-, -C(O)O-, -C(O)NR'-, -OC(O)-, -OC(O)NR'-, -N(R')C(O)O-, -N(R')C(O)- and -N(R')S(O)₂-; or, L or L' is C₁₋₈ alkylene, in which one or more methylene units are optionally and independently replaced by -C(R')₂-, -N(R')-, -N(R')C(O)-, -C(O)N(R')-, -N(R')S(O)₂-, -S(O)₂N(R')-, -O-, -C(O)-, -OC(O)-, -C(O)O-, -S-, -S(O)-, or -S(O)₂-;
R' and R" are independently selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl; or, R' and R" are taken together with the N atom to which they are attached to form a 3- to 14-membered heterocyclyl;
L" is selected from covalent bond, -O- and -S-; or, L" is C₁₋₈ alkylene, which is optionally substituted with one or more R, and one or more methylene units in the C₁₋₈ alkylene are optionally and independently replaced by -O-, -C(O)-, -OC(O)-, -C(O)O-, -S-, -S(O)-, or -S(O)₂-;
R is independently selected from H, D, C₁₋₈ alkyl, C₁₋₈ haloalkyl, -L_{d}-3- to 10-membered carbocyclyl and -L₄-3- to 10-membered heterocyclyl;
or two R are taken together with the C atom to which they are jointly attached to form a 3- to 10-membered carbocyclylene or a 3- to 10-membered heterocyclylene;
L_{d} is independently selected from covalent bond and C₁₋₈ alkylene;
f=0, 1, 2, 3, 4, 5, 6, 7, 8, 9 or 10;
wherein each of the above groups is optionally deuterated, up to completely deuterated.

3. A compound of formula (I), or a stereoisomer, an N-oxide, a hydrate, a solvate, a metabolite, a pharmaceutically acceptable salt, a polymorph, or a prodrug thereof: 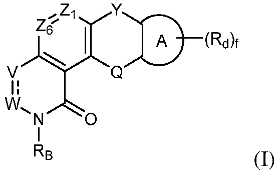
V is selected from covalent bond, O, S, NR', S(O), S(O)₂, C(O), CR_{C} and CR_{C}R_{C}';
W is selected from NR', S(O), S(O)₂, C(O), CR_{C} and CR_{C}R_{C}';
Q and Y are independently selected from covalent bond, -O-, -S-, -NR_{A}-, -S(O)-, -S(O)₂-, -C(O)-, -C(O)O-, -C(O)NR_{A}-, -OC(O)-, -OC(O)NR_{A}-, -N(R_{A})C(O)O-, -N(R_{A})C(O)- and -N(R_{A})S(O)₂-; and Q and Y are not covalent bonds at the same time;
or Q and Y are independently C₁₋₈ alkylene, in which one or more methylene units are optionally and independently replaced by -CR₁ₐR_{1b}-, -N(R_{A})-, -N(R_{A})C(O)-, -C(O)N(R_{A})-, -N(R_{A})S(O)₂-, -S(O)₂N(R_{A})-, -O-, -C(O)-, -OC(O)-, -C(O)O-, -S-, -S(O)-, or -S(O)₂-;
R_{A} is selected from H, C₁₋₈ alkyl, C₁₋₈ alkoxy, C₁₋₈ haloalkyl, -L"-C₆₋₁₀ aryl, -L''-3- to 14-membered carbocyclyl, -L" -3- to 14-membered heterocyclyl and -L"-5- to 14-membered heteroaryl;
R_{B} is selected from H, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
R_{C}, R'_{C}, R₁ₐ and R_{1b} are independently selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
one of Z₁ and Z₆ is CR_{E}, and the other one is CR_{d1} or N;
R_{E} is selected from -L-C₆₋₁₀ aryl, -L-3- to 14-membered carbocyclyl, -L-3- to 14-membered heterocyclyl and -L-5- to 14-membered heteroaryl, wherein each of the C₆₋₁₀ aryl, 3- to 14-membered carbocyclyl, 3- to 14-membered heterocyclyl and 5- to 14-membered heteroaryl is optionally substituted with 1, 2, 3, 4, 5, 6, 7, 8, 9 or 10 Rₑ groups;
Rₑ is independently selected from H, D, halogen, -CN, oxo, C₁₋₈ alkyl, C₁₋₈ haloalkyl, -L'-OR', -L'-NR'R", -NO₂, -L'-SR', -L'-C(O)OR', -L'-C(O)NR'R", -L'-S(O)_{2R}', -L'-S(O)₂NR'R", -L'-OC(O)R', -L'-OC(O)NR'R", -L'-C₆₋₁₀ aryl, -L'-3- to 14-membered carbocyclyl, -L'-3- to 14-membered heterocyclyl and -L'-5- to 14-membered heteroaryl;
R_{d1} is selected from H, D, halogen, -CN, C₁₋₈ alkyl, C₂₋₈ alkenyl, C₂₋₈ alkynyl, C₁₋₈ haloalkyl, -L-OR', -L-NR'R", -L-C(O)NR'R", -L-C₆₋₁₀ aryl, -L-3- to 14-membered carbocyclyl, -L-3- to 14-membered heterocyclyl and -L-5- to 14-membered heteroaryl;
ring A is selected from C₆₋₁₀ aromatic ring, 3- to 14-membered carbocycle, 3- to 14-membered heterocycle and 5- to 14-membered heteroaromatic ring;
R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or, R_{d} is selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₈ alkyl, C₂₋₈ alkenyl, C₂₋₈ alkynyl, C₁₋₈ haloalkyl, -L" -OR', -L"-NR'R", -L"-C(O)OR', -L"-C(O)NR'R", -L"-S(O)₀₋₂R', -L"-S(O)₂NR'R", -L" -S(O)NR'R", -L"-OC(O)R', -L"-OC(O)NR'R", -L"-C₆₋₁₀ aryl, -L''-3- to 14-membered carbocyclyl, -L"-3- to 14-membered heterocyclyl and -L"-5- to 14-membered heteroaryl;
L and L' are independently selected from covalent bond, -O-, -S-, -NR'-, -S(O)₂-, -C(O)-, -C(O)O-, -C(O)NR'-, -OC(O)-, -OC(O)NR'-, -N(R')C(O)O-, -N(R')C(O)- and -N(R')S(O)₂-; or, L or L' is C₁₋₈ alkylene, in which one or more methylene units are optionally and independently replaced by -C(R')₂-, -N(R')-, -N(R')C(O)-, -C(O)N(R')-, -N(R')S(O)₂-, -S(O)₂N(R')-, -O-, -C(O)-, -OC(O)-, -C(O)O-, -S-, -S(O)-, or -S(O)₂-;
R' and R" are independently selected from H, C₁₋₈ alkyl, C₁₋₈ alkoxy, C₁₋₈ haloalkyl, -L"-C₆₋₁₀ aryl, -L"-3- to 14-membered carbocyclyl, -L"-3- to 14-membered heterocyclyl and -L"-5- to 14-membered heteroaryl; or, R' and R" are taken together with the N atom to which they are attached to form a 3- to 14-membered heterocyclyl;
L" is selected from covalent bond, -O- and -S-; or, L" is C₁₋₈ alkylene, in which one or more methylene units are optionally and independently replaced by -O-, -C(O)-, -OC(O)-, -C(O)O-, -S-, -S(O)-, or -S(O)₂-;
f=0, 1, 2, 3, 4, 5, 6, 7, 8, 9 or 10;
wherein each of the above groups is optionally deuterated, up to completely deuterated.

4. The compound of formula (I') or formula (I) according to any one of claims 1 to 3, or a stereoisomer, an N-oxide, a hydrate, a solvate, a metabolite, a pharmaceutically acceptable salt, a polymorph, or a prodrug thereof, wherein V is selected from covalent bond, CR_{C} and CR_{C}R_{C}'; alternatively selected from covalent bond, CH, CH₂, CD, CHD and CD₂, yet alternatively covalent bond.

5. The compound of formula (I') or formula (I) according to any one of claims 1 to 4, or a stereoisomer, an N-oxide, a hydrate, a solvate, a metabolite, a pharmaceutically acceptable salt, a polymorph, or a prodrug thereof, wherein W is CR_{C} or CR_{C}R_{C}'; alternatively CR_{C}R_{C}', yet alternatively CH₂, CD, CHD or CD₂.

6. The compound of formula (I') or formula (I) according to any one of claims 1 to 5, or a stereoisomer, an N-oxide, a hydrate, a solvate, a metabolite, a pharmaceutically acceptable salt, a polymorph, or a prodrug thereof, wherein the compound of formula (I) is one of the following formulas:

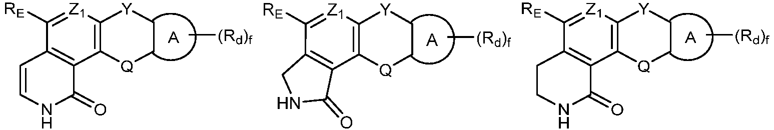

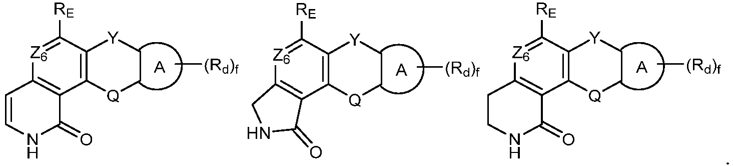

7. The compound of formula (I') or formula (I) according to any one of claims 1 to 6, or a stereoisomer, an N-oxide, a hydrate, a solvate, a metabolite, a pharmaceutically acceptable salt, a polymorph, or a prodrug thereof, wherein Q and Y are independently selected from covalent bond, O, S, NR_{A}, S(O), S(O)₂, C(O) and CR₁ₐR_{1b}; and Q and Y are not covalent bonds at the same time;
alternatively, Q is selected from O, S and NR_{A}, alternatively S or NR_{A}, alternatively NR_{A}, yet alternatively NH;
alternatively, Y is selected from covalent bond, O, S and CR₁ₐR_{1b}, alternatively selected from covalent bond, O and CR₁ₐR_{1b}, alternatively selected from covalent bond, O, CH₂, CF₂ and C(CH₃)₂, yet alternatively C(CH₃)₂.

8. The compound of formula (I') or formula (I) according to any one of claims 1 to 7, or a stereoisomer, an N-oxide, a hydrate, a solvate, a metabolite, a pharmaceutically acceptable salt, a polymorph, or a prodrug thereof, wherein R_{A} is selected from H, C₁₋₆ alkyl, C₁₋₆ haloalkyl, -L''-3- to 10-membered carbocyclyl and -L"-3- to 10-membered heterocyclyl; alternatively selected from H, C₁₋₆ alkyl and C₁₋₆ haloalkyl; alternatively H.

9. The compound of formula (I') or formula (I) according to any one of claims 1 to 8, or a stereoisomer, an N-oxide, a hydrate, a solvate, a metabolite, a pharmaceutically acceptable salt, a polymorph, or a prodrug thereof, wherein R_{B} is selected from H, C₁₋₆ alkyl, C₁₋₆ haloalkyl, -L-OR', -L-NR'R", -L-C(O)NR'R", -L-3-to 10-membered carbocyclyl and -L-3- to 10-membered heterocyclyl; alternatively selected from H, C₁₋₆ alkyl and C₁₋₆ haloalkyl; alternatively H.

10. The compound of formula (I') or formula (I) according to any one of claims 1 to 9, or a stereoisomer, an N-oxide, a hydrate, a solvate, a metabolite, a pharmaceutically acceptable salt, a polymorph, or a prodrug thereof, wherein R_{C} and R'_{C} are independently selected from H, D, halogen, -CN, C₁₋₆ alkyl, C₁₋₈ haloalkyl, -L-OR', -L-NR'R", -L-C(O)NR'R", -L-3- to 10-membered carbocyclyl and -L-3- to 10-membered heterocyclyl; alternatively independently selected from H, D, halogen, C₁₋₆ alkyl and C₁₋₆ haloalkyl; yet alternatively independently H or D.

11. The compound of formula (I') or formula (I) according to any one of claims 1 to 10, or a stereoisomer, an N-oxide, a hydrate, a solvate, a metabolite, a pharmaceutically acceptable salt, a polymorph, or a prodrug thereof, wherein R₁ₐ and R_{1b} are independently selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl; alternatively independently selected from H, D, halogen, CN, C₁₋₆ alkyl and C₁₋₆ haloalkyl; alternatively independently selected from H, D, halogen, C₁₋₆ alkyl and C₁₋₆ haloalkyl; alternatively independently selected from H, D, halogen, C₁₋₃ alkyl and C₁₋₃ haloalkyl; alternatively independently selected from H, D, F and Me; yet alternatively Me.

12. The compound of formula (I') or formula (I) according to any one of claims 1 to 11, or a stereoisomer, an N-oxide, a hydrate, a solvate, a metabolite, a pharmaceutically acceptable salt, a polymorph, or a prodrug thereof, wherein R_{E} is selected from phenyl, pyridyl, pyrimidinyl, pyrazinyl, pyrrolyl, pyrazolyl, imidazolyl, pyrrolopyridyl, pyrrolopyridazinyl, pyrrolopyrimidinyl, pyrrolopyrazinyl, imidazopyridyl, imidazopyridazinyl, imidazopyrimidinyl, imidazopyrazinyl, pyrazolopyridyl, pyrazolopyridazinyl, pyrazolopyrimidinyl, pyrazolopyrazinyl, pyridopyridyl, dihydropyridooxazinyl, indolyl, triazolopyridyl, tetrahydroimidazopyrazinyl, dihydropyrrolopyridyl, thienopyridyl, benzimidazolyl, dihydroimidazooxazinyl, tetrahydropyrazolopyridyl, tetrahydropyrrolopyridyl, dihydropyrazolooxazinyl, dihydropyrroloimidazolyl, oxazolopyridyl, pyrazoloazepanyl, pyrazolopiperidyl, imidazoazepanyl, imidazopiperidyl, pyrazolodiazepanyl, pyrazolodiazacyclohexyl and thienopyridyl, each of which is optionally substituted with 1, 2, 3, 4, 5, 6, 7, 8, 9 or 10 Rₑ groups;
alternatively, R_{E} and a substituent Rₑ thereon are taken together to form a group selected from: 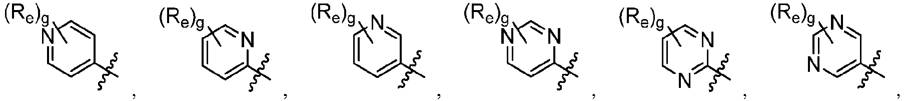 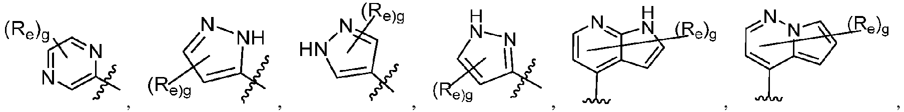 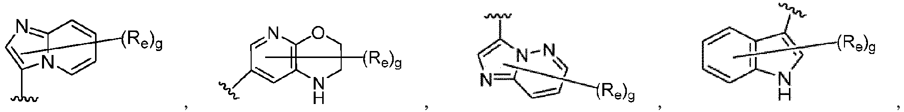 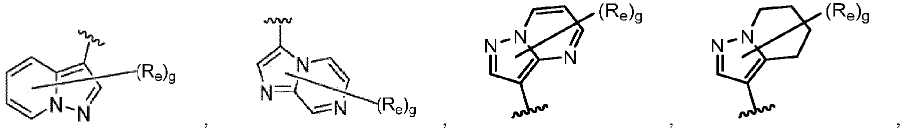 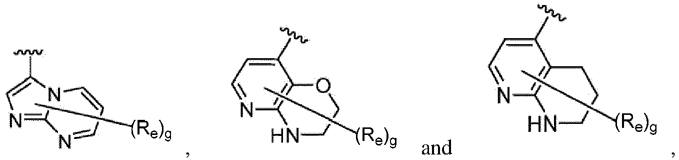 alternatively selected from 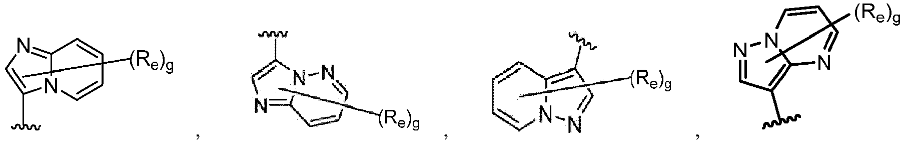 and 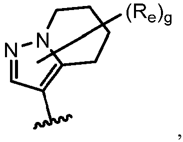 wherein g=0, 1, 2, 3, 4, 5, 6, 7, 8, 9 or 10;
alternatively, g=0, 1, 2, 3, 4, 5, or 6, alternatively, g=0, 1, 2, 3, or 4, alternatively, g=0, 1, or 2.

13. The compound of formula (I') or formula (I) according to any one of claims 1 to 12, or a stereoisomer, an N-oxide, a hydrate, a solvate, a metabolite, a pharmaceutically acceptable salt, a polymorph, or a prodrug thereof, wherein Rₑ is independently selected from H, D, halogen, -CN, oxo, C₁₋₆ alkyl, C₁₋₆ haloalkyl, -L'-OR', -L'-NR'R", -NO₂, -L'-SR', -L'-C(O)OR', -L'-C(O)NR'R'', -L'-S(O)₂R', -L'-S(O)₂NR'R", -L'-OC(O)R', -L'-OC(O)NR'R", -L'-3- to 10-membered carbocyclyl and -L'-3- to 10-membered heterocyclyl; alternatively independently selected from H, D, halogen, -CN, -OR', -SR', -NR'R", C₁₋₆ alkyl and C₁₋₆ haloalkyl; alternatively independently selected from H, D, halogen, -CN, -OR', C₁₋₆ alkyl and C₁₋₆ haloalkyl; alternatively independently selected from H, D, Me, F, Cl, -CN and -OCH₃;
alternatively, R_{E} and a substituent Rₑ thereon are taken together to form a group selected from: 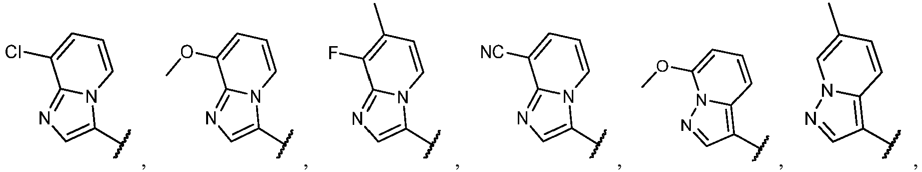 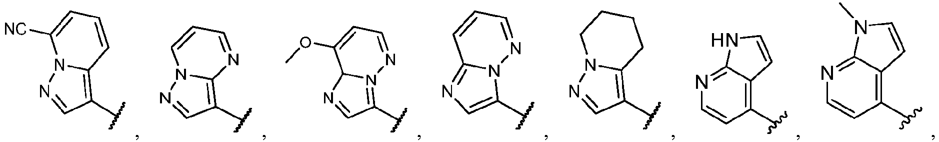 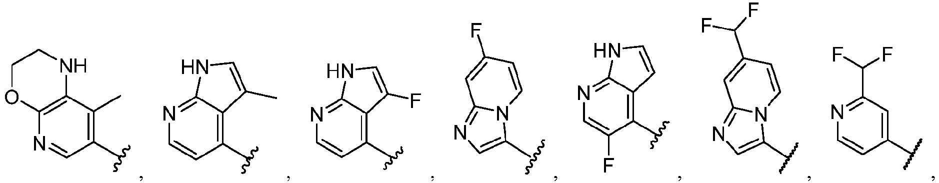 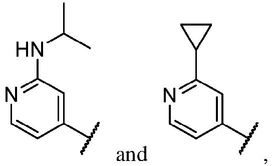 alternatively selected from: 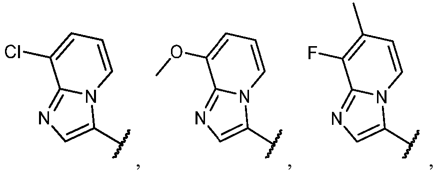 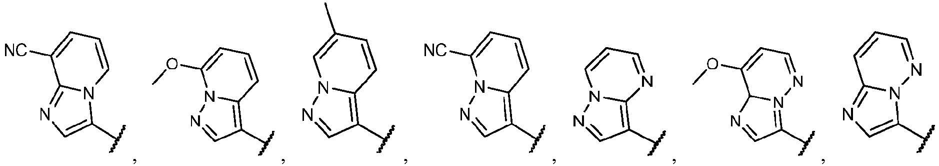 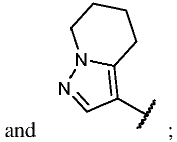 yet alternatively selected from:

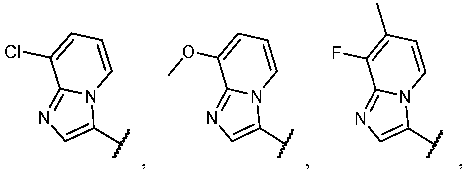

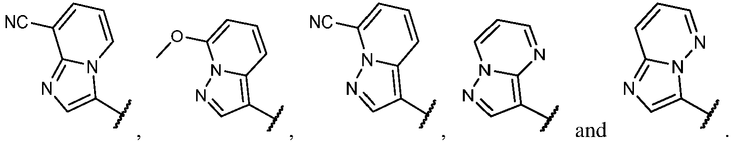

14. The compound of formula (I') or formula (I) according to any one of claims 1 to 13, or a stereoisomer, an N-oxide, a hydrate, a solvate, a metabolite, a pharmaceutically acceptable salt, a polymorph, or a prodrug thereof, wherein Z₆ is CR_{E}, and Z₁ is CR_{d1} or N, or Z₁ is CR_{E}, and Z₆ is N; alternatively Z₆ is CR_{E}, and Z₁ is CR_{d1} or N; alternatively Z₆ is CR_{E}, and Z₁ is CR_{d1}; yet alternatively, Z₆ is CR_{E}, and Z₁ is CH or CD.

15. The compound of formula (I') or formula (I) according to any one of claims 1 to 14, or a stereoisomer, an N-oxide, a hydrate, a solvate, a metabolite, a pharmaceutically acceptable salt, a polymorph, or a prodrug thereof, wherein R_{d1} is selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl; alternatively selected from H, D, halogen, C₁₋₆ alkyl and C₁₋₆ haloalkyl; yet alternatively H or D.

16. The compound of formula (I') or formula (I) according to any one of claims 1 to 15, or a stereoisomer, an N-oxide, a hydrate, a solvate, a metabolite, a pharmaceutically acceptable salt, a polymorph, or a prodrug thereof, wherein ring A is selected from benzene ring, furan ring, furazan ring, imidazolidine ring, imidazoline ring, imidazole ring, isothiazole ring, isoxazole ring, morpholine ring, oxadiazole ring, 1,2,3-oxadiazole ring, 1,2,4-oxadiazole ring, 1,2,5-oxadiazole ring, 1,3,4-oxadiazole ring, oxazolidine ring, oxazole ring, oxetane ring, azetidine ring, pyrimidine ring, piperazine ring, piperidine ring, pyran ring, pyrazine ring, pyrazolidine ring, pyrazoline ring, pyrazole ring, pyridazine ring, pyridine ring, pyrrolidine ring, pyrroline ring, 2H-pyrrole ring, pyrrole ring, tetrahydrofuran ring, tetrahydropyran ring, thiazole ring, thiophene ring, triazine ring, 1,2,3-triazole ring, 1,2,4-triazole ring, 1,2,5-triazole ring, 1,3,4-triazole ring, xanthene ring, tetrahydroquinoline ring, tetrahydroisoquinoline ring and hexahydropyrazinoquinoline ring; alternatively selected from benzene ring, pyridine ring, pyrazole ring and imidazole ring; yet alternatively benzene ring;
alternatively, ring A and a substituent R_{d} thereon are taken together to form a group selected from: 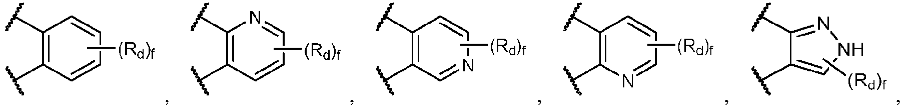 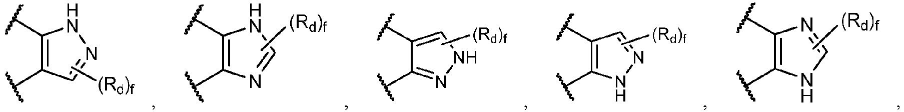 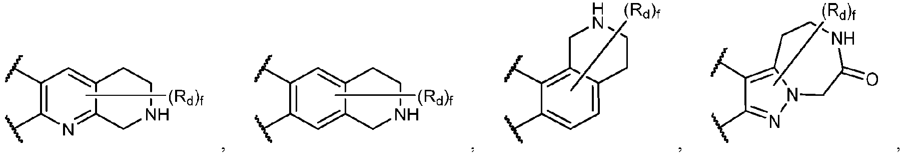 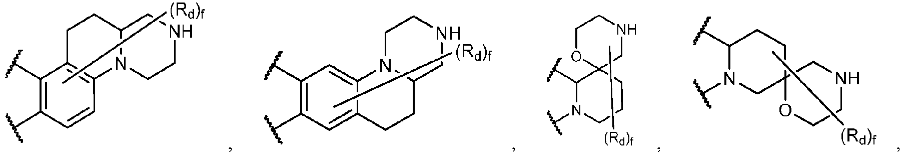 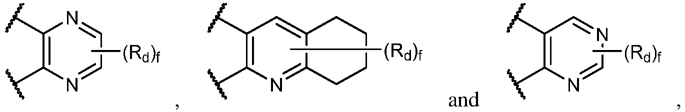 alternatively selected from: 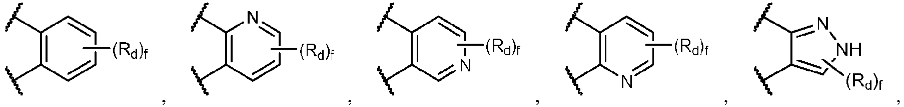 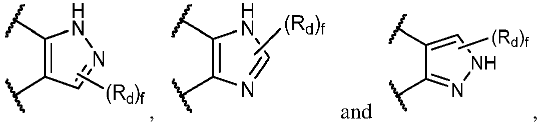 wherein f=0, 1, 2, 3, 4, 5, 6, 7, 8, 9 or 10;
alternatively, f=0, 1, 2, 3, 4, 5 or 6, alternatively f=0, 1, 2, 3 or 4, alternatively f=0, 1 or 2.

17. The compound of formula (I') or formula (I) according to any one of claims 1 to 16, or a stereoisomer, an N-oxide, a hydrate, a solvate, a metabolite, a pharmaceutically acceptable salt, a polymorph, or a prodrug thereof, wherein R_{d} is independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R"; or, R_{d} is independently selected from C₁₋₆ alkyl, C₁₋₆ haloalkyl, -L'-3- to 10-membered carbocyclyl and -L'-3- to 10-membered heterocyclyl, each of which is optionally substituted with 1, 2, 3, 4, 5, 6, 7 or 8 R_{dd};
or two adjacent R_{d} are taken together with the atoms to which they are attached to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl, or 6- to 10-membered fused bicyclic heterocyclyl is optionally substituted with 1, 2, 3, 4, 5, 6, 7 or 8 R_{dd};
alternatively, R_{d} is independently selected from H, D, halogen, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, and the C₁₋₆ alkyl, C₁₋₆ haloalkyl, 3- to 7-membered carbocyclyl or 3- to 7-membered heterocyclyl is optionally substituted with 1, 2, 3, 4, 5, 6, 7 or 8 R_{dd};
alternatively, R_{d} is independently selected from H, D, C₁₋₆ alkyl, C₁₋₆ haloalkyl, -L'-NR'R" and -L'-4-to 6-membered heterocyclyl, and the C₁₋₆ alkyl, C₁₋₆ haloalkyl or 4- to 6-membered heterocyclyl is optionally substituted with 1 or 2 R_{dd};
alternatively, R_{d} is independently selected from H, D, 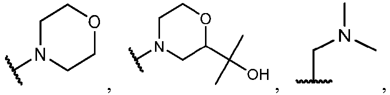 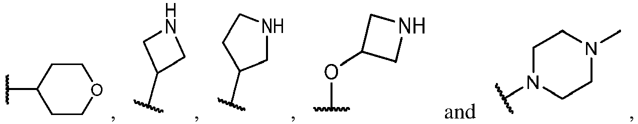 alternatively independently selected from H, D, 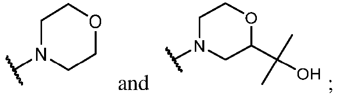
alternatively, ring A and a substituent R_{d} thereon are taken together to form a group selected from: 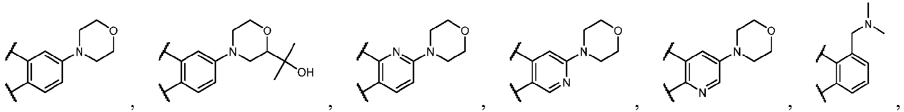 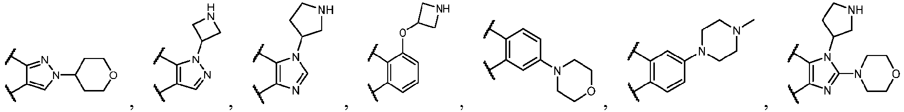 and 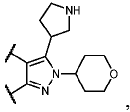 alternatively the group is 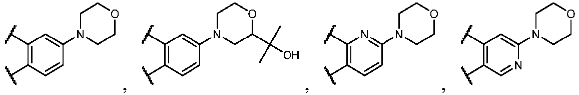 or 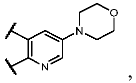 alternatively 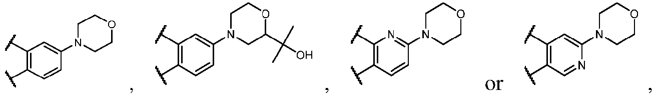 yet alternatively

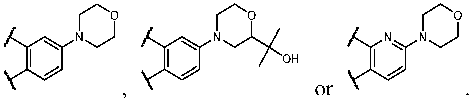

18. The compound of formula (I') or formula (I) according to any one of claims 1 to 17, or a stereoisomer, an N-oxide, a hydrate, a solvate, a metabolite, a pharmaceutically acceptable salt, a polymorph, or a prodrug thereof, wherein R_{d} is independently selected from H, F, -OH, -OCH₃,

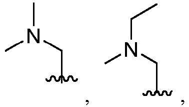

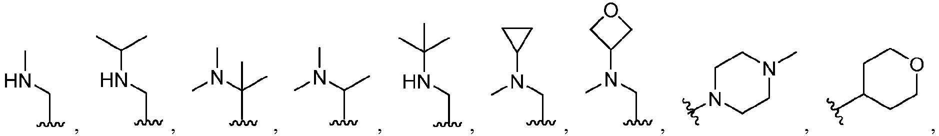

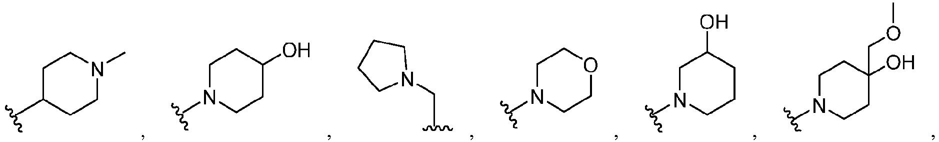

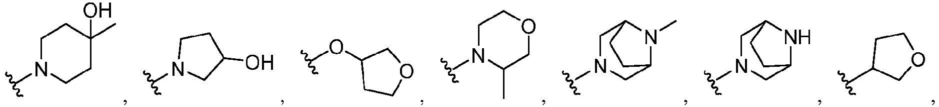

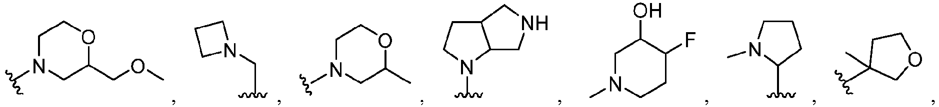

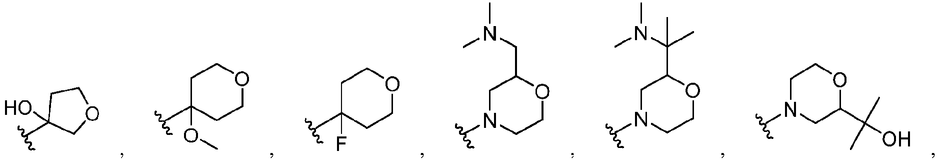

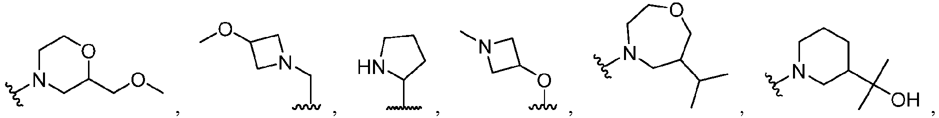

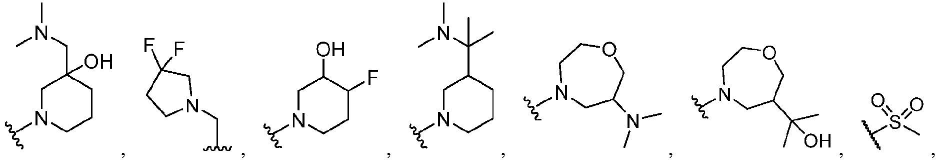

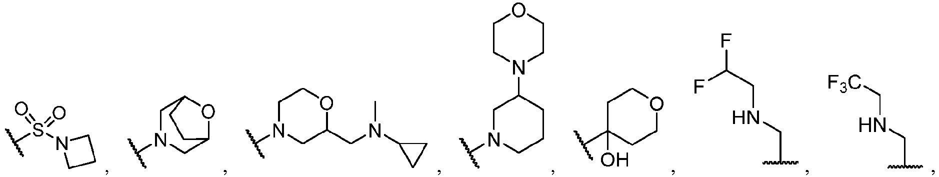

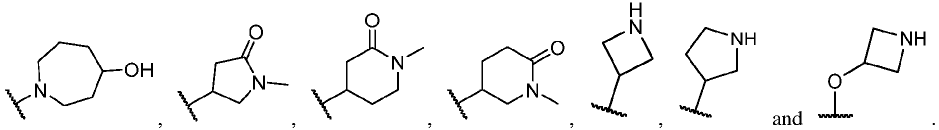

19. The compound of formula (I') or formula (I) according to any one of claims 1 to 17, or a stereoisomer, an N-oxide, a hydrate, a solvate, a metabolite, a pharmaceutically acceptable salt, a polymorph, or a prodrug thereof, wherein R_{dd} is independently selected from H, D, halogen, -CN, -NO₂, oxo, C₁₋₆ alkyl, C₁₋₆ haloalkyl, -L" -OR', -L"-NR'R", -L"-C(O)OR', -L"-C(O)NR'R", -L"-S(O)₀₋₂R', -L"-S(O)₂NR'R", -L" -S(O)NR'R", -L"-OC(O)R', -L"-OC(O)NR'R", -L''-3- to 10-membered carbocyclyl and -L''-3- to 10-membered heterocyclyl; alternatively independently selected from H, D, halogen, C₁₋₆ alkyl, C₁₋₆ haloalkyl, -L"-OR', -L" -SR' and -L''-NR'R''; alternatively independently selected from H, D, C₁₋₆ alkyl, C₁₋₆ haloalkyl, -L"-OR' and -L"-NR'R", alternatively independently selected from H, D, Me, N(CH₃)₂ and C(CH₃)₂OH.

20. The compound of formula (I') or formula (I) according to any one of claims 1 to 19, or a stereoisomer, an N-oxide, a hydrate, a solvate, a metabolite, a pharmaceutically acceptable salt, a polymorph, or a prodrug thereof,
wherein ring A and a substituent R_{d} thereon are taken together to form a group selected from:

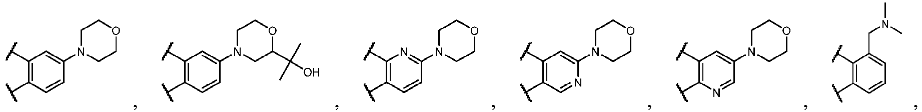

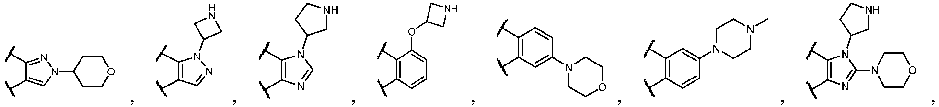

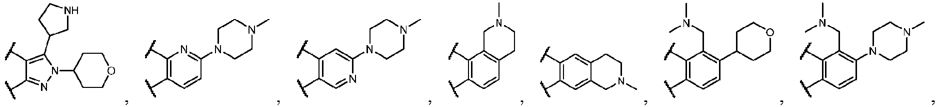

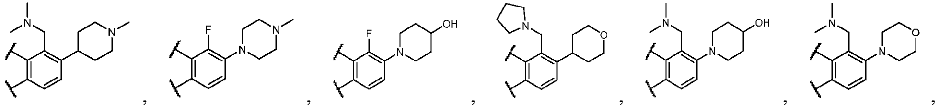

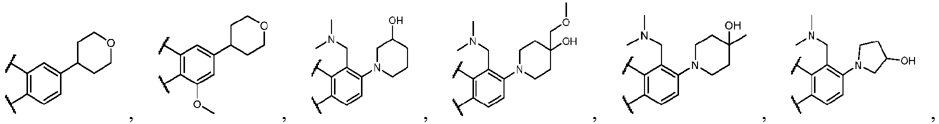

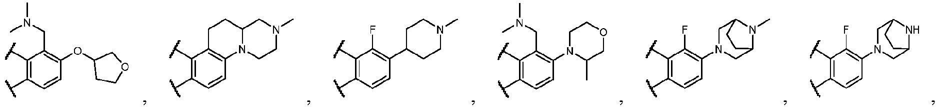

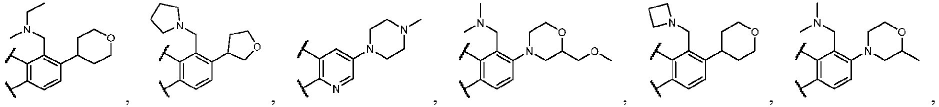

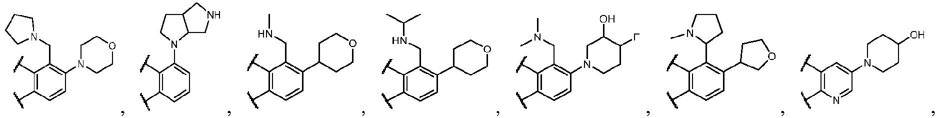

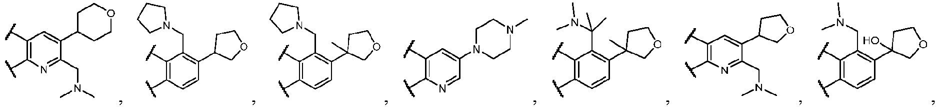

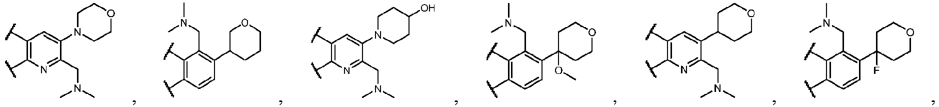

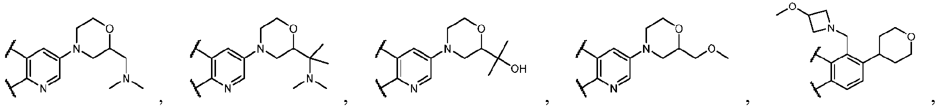

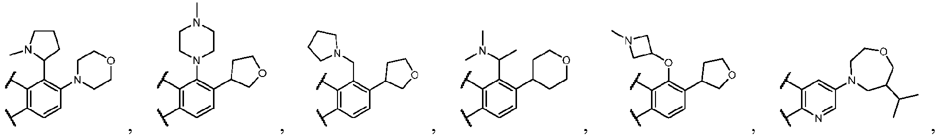

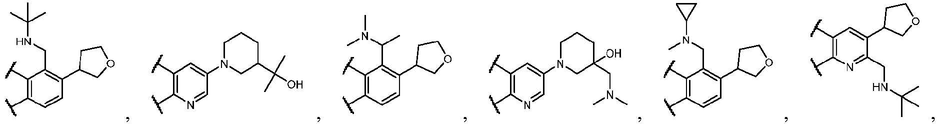

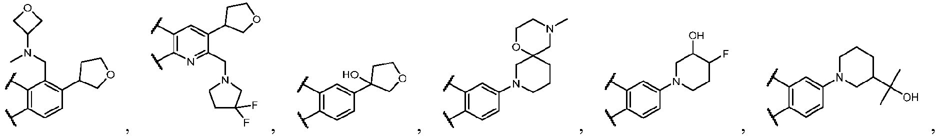

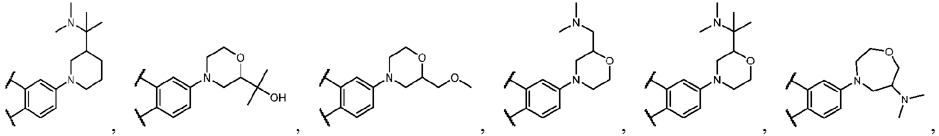

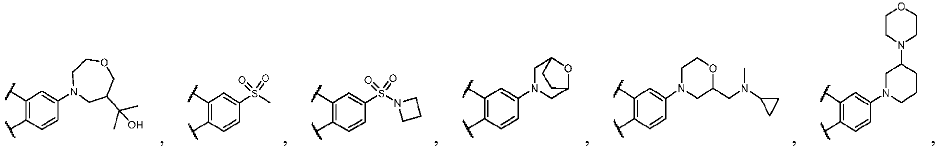

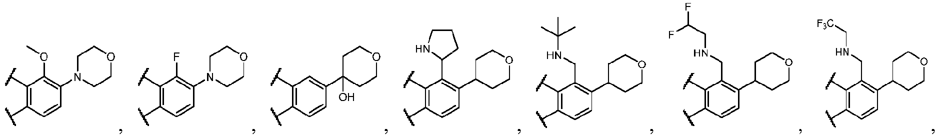

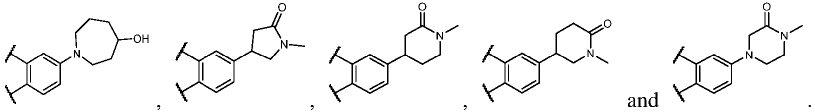

21. The compound of formula (I') or formula (I) according to any one of claims 1 to 20, or a stereoisomer, an N-oxide, a hydrate, a solvate, a metabolite, a pharmaceutically acceptable salt, a polymorph, or a prodrug thereof, wherein L and L' are independently selected from chemical bond, -O-, -S-, -NR'-, -S(O)₂- and -C(O)-; or, L or L' is C₁₋₆ alkylene, in which one or more methylene units are optionally and independently replaced by -C(R')₂-, -N(R')-, -O-, -C(O)-, -S-, -S(O)-, or -S(O)₂-;
alternatively, L or L' is independently selected from covalent bond, O and C₁₋₆ alkylene, alternatively independently selected from covalent bond, O and C₁₋₄ alkylene.

22. The compound of formula (I') or formula (I) according to any one of claims 1 to 21, or a stereoisomer, an N-oxide, a hydrate, a solvate, a metabolite, a pharmaceutically acceptable salt, a polymorph, or a prodrug thereof, wherein L" is selected from covalent bond, -O- and -S-; or, L" is C₁₋₆ alkylene, in which one or more methylene units are optionally and independently replaced by -O-, -C(O)-, -S-, -S(O)-, or -S(O)₂-; alternatively L" is selected from covalent bond and C₁₋₆ alkylene; yet alternatively covalent bond or C₁₋₄ alkylene.

23. The compound of formula (I') or formula (I) according to any one of claims 1 to 22, or a stereoisomer, an N-oxide, a hydrate, a solvate, a metabolite, a pharmaceutically acceptable salt, a polymorph, or a prodrug thereof, wherein the compound has the following structural formulas: 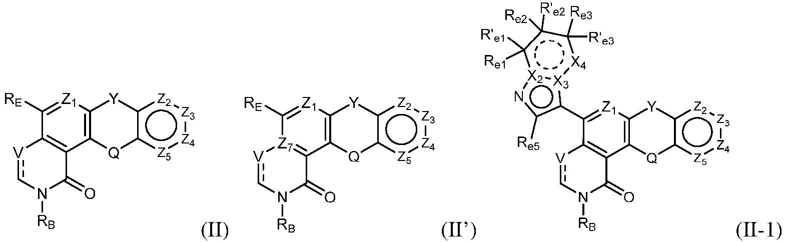 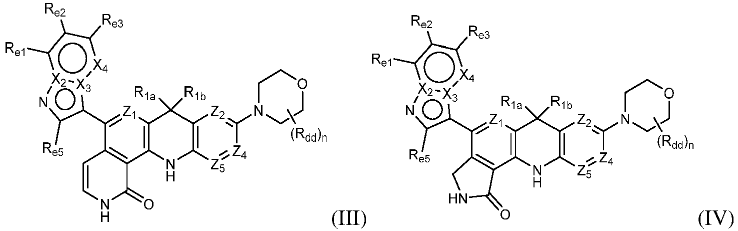 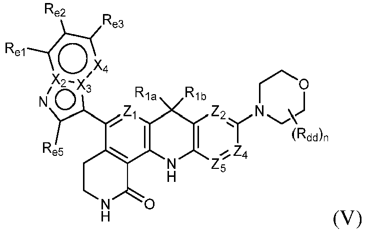 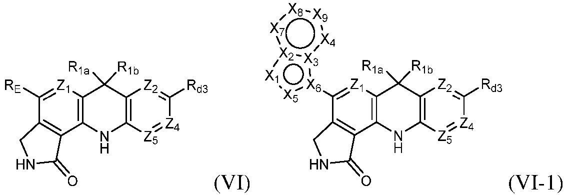 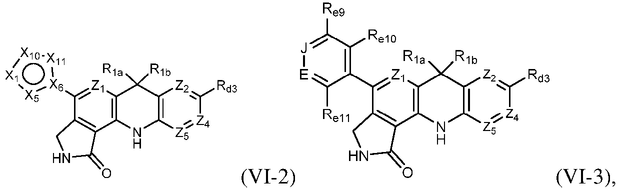 wherein
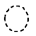 indicates that a double bond may be present or absent, when the double bond is present, it means that the ring where it is located is an aromatic ring, and R'ₑ₁, R'ₑ₂, R'ₑ₃ and R'ₑ₄ are absent, when the double bond is absent, it means that the ring where it is located is not aromatic;
X₁ is CRₑ₆, N, O, S or NR'ₑ₆;
X₂ is C or N;
X₃ is C or N;
X₄ is CRₑ₄, CRₑ₄R'ₑ₄ or N;
X₅ is CRₑ₅, N, O, S or NR'ₑ₅;
X₆ is C or N;
X₇ is CRₑ₁ or N;
X₈ is CRₑ₂ or N;
X₉ is CRₑ₃ or N;
X₁₀ is selected from CRₑ₇, O, S, N and NR'ₑ₇;
X₁₁ is selected from CRₑ₈, O, S, N and NR'ₑ₈;
Rₑ₁, Rₑ₂, Rₑ₃, Rₑ₄, Rₑ₅ and Rₑ₆ are independently selected from H, D, halogen, -CN, C₁₋₈ alkyl, C₁₋₈ haloalkyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-C₆₋₁₀ aryl, -L'-3- to 14-membered carbocyclyl, -L'-3-to 14-membered heterocyclyl and -L'-5- to 14-membered heteroaryl;
R'ₑ₁, R'ₑ₂, R'ₑ₃ and R'ₑ₄ are independently selected from absent, H, D, halogen, -CN, C₁₋₈ alkyl and C₁₋₈ haloalkyl; or CRₑ₁R'ₑ₁ forms -C(O)-, or CRₑ₂R'ₑ₂ forms -C(O)-, or CRₑ₃R'ₑ₃ forms -C(O)-, or CRₑ₄R'ₑ₄ forms -C(O)-;
R'ₑ₅ and R'ₑ₆ are independently selected from H, C₁₋₈ alkyl and C₁₋₈ haloalkyl;
Rₑ₇ and Rₑ₈ are independently selected from H, D, halogen, CN, C₁₋₈ alkyl, C₁₋₈ haloalkyl, -L'-OR', -L'-SR', -L'-NR'R", -L'-3- to 10-membered carbocyclyl and -L'-3- to 10-membered heterocyclyl;
R'ₑ₇ and R'ₑ₈ are independently selected from H, D, C₁₋₈ alkyl, C₁₋₈ haloalkyl, -L'-3- to 10-membered carbocyclyl and -L'-3- to 10-membered heterocyclyl;
or, the substituents on X₁₀ and X₁₁ are taken together to form a C₃₋₅ alkylene, which optionally contains one double bond, and the C₃₋₅ alkylene is optionally substituted with 1, 2, 3 or 4 Rₑ, and one or more methylene units in the C₃₋₅ alkylene are optionally and independently replaced by NR" -, -N(R")C(O)-, -C(O)N(R")-, -N(R")S(O)₂-, -S(O)₂N(R")-, -O-, -C(O)-, -OC(O)-, -C(O)O-, -S-, -S(O)-, or -S(O)₂-;
E is selected from CRₑ₁₂ and N;
J is selected from CRₑ₁₃ and N;
Rₑ₉, Rₑ₁₀, Rₑ₁₁, Rₑ₁₂ and Rₑ₁₃ are selected from H, D, halogen, CN, C₁₋₈ alkyl, C₁₋₈ haloalkyl, C₂₋₈ alkenyl, C₂₋₈ alkynyl, -L'-OR', -L'-SR', -L'-NR'R", -L'-3- to 10-membered carbocyclyl, -L'-3- to 10-membered heterocyclyl and -Y₅-C₀₋₈ alkylene-R₆;
Y₅ is selected from O, S and NR₅;
R₅ is selected from H, C₁₋₈ alkyl and C₁₋₈ haloalkyl;
R₆ is selected from 3- to 10-membered carbocyclyl and 3- to 10-membered heterocyclyl;
or Rₑ₉ and Rₑ₁₃ are taken together with the carbon atoms to which they are attached to form a 5- to 10-membered carbocyclyl, a 5- to 10-membered heterocyclyl, a C₆₋₁₀ aryl or a 5- to 10-membered heteroaryl, which is optionally substituted with 1, 2, 3 or 4 Rₑ;
or Rₑ₉ and Rₑ₁₀ are taken together with the carbon atoms to which they are attached to form a 5- to 10-membered carbocyclyl, a 5- to 10-membered heterocyclyl, a C₆₋₁₀ aryl or a 5- to 10-membered heteroaryl, which is optionally substituted with 1, 2, 3 or 4 Rₑ;
Z₂ is C atom or N atom, which is optionally substituted with R_{d2};
Z₃ is C atom or N atom, which is optionally substituted with R_{d3};
Z₄ is C atom or N atom, which is optionally substituted with R_{d4};
Z₅ is selected from covalent bond, CR_{d5} and N;
R_{d2}, R_{d3}, R_{d4} and R_{d5} are independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R", or, R_{d2}, R_{d3}, R_{d4} and R_{d5} are independently selected from C₁₋₈ alkyl, C₂₋₈ alkenyl, C₂₋₈ alkynyl, C₁₋₈ haloalkyl, -L'-C₆₋₁₀ aryl, -L'-3- to 14-membered carbocyclyl, -L'-3- to 14-membered heterocyclyl and -L'-5- to 14-membered heteroaryl, each of which is optionally substituted with 1, 2, 3, 4, 5, 6, 7 or 8 R_{dd};
or R_{d2} and R_{d3} are taken together with the atoms to which they are attached, or R_{d3} and R_{d4} are taken together with the atoms to which they are attached, or R_{d4} and R_{d5} are taken together with the atoms to which they are attached, to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl or 6- to 10-membered fused bicyclic heterocyclyl is optionally substituted with 1, 2, 3, 4 or 5 R_{dd};
n=0, 1, 2, 3, 4, 5, 6, 7 or 8;
other groups are as defined in claims 1 to 20;
each of the above groups is optionally deuterated, up to completely deuterated.

24. The compound of formula (II-1) according to claim 23, or a stereoisomer, an N-oxide, a hydrate, a solvate, a metabolite, a pharmaceutically acceptable salt, a polymorph, or a prodrug thereof, wherein
V is selected from covalent bond, CR_{C} and CR_{C}R_{C}';
Q and Y are independently selected from covalent bond, O, S, NR_{A}, S(O), S(O)₂, C(O) and CR₁ₐR_{1b}; and Q and Y are not covalent bonds at the same time;
R_{A} is selected from H, C₁₋₆ alkyl, C₁₋₆ haloalkyl, -L"-3- to 10-membered carbocyclyl and -L"-3- to 10-membered heterocyclyl;
R_{B} is selected from H, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl;
R_{C}, R'_{C}, R₁ₐ and R_{1b} are independently selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl;
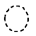 indicates that a double bond may be present or absent, when the double bond is present, it means that the ring where it is located is an aromatic ring, and R'ₑ₁, R'ₑ₂, R'ₑ₃ and R'ₑ₄ are absent, when the double bond is absent, it means that the ring where it is located is not aromatic;
X₂ is C or N;
X₃ is C or N;
X₄ is CRₑ₄, CRₑ₄R'ₑ₄ or N;
Rₑ₁, Rₑ₂, Rₑ₃, Rₑ₄ and Rₑ₅ are independently selected from H, D, halogen, -CN, C₁₋₆ alkyl, C₁₋₆ haloalkyl, -L'-OR', -L'-NR'R", -NO₂, -L'-SR', -L'-C(O)OR', -L'-C(O)NR'R", -L'-S(O)₂R', -L'-S(O)₂NR'R", -L'-OC(O)R', -L'-OC(O)NR'R", -L'-3- to 10-membered carbocyclyl and -L'-3- to 10-membered heterocyclyl;
R'ₑ₁, R'ₑ₂, R'ₑ₃ and R'ₑ₄ are independently selected from H, D, halogen, -CN, C₁₋₆ alkyl and C₁₋₆ haloalkyl; or CRₑ₁R'ₑ₁ forms -C(O)-, or CRₑ₂R'ₑ₂ forms -C(O)-, or CRₑ₃R'ₑ₃ forms -C(O)-, or CRₑ₄R'ₑ₄ forms -C(O)-;
Z₁ is CR_{d1} or N;
Z₂ is C atom or N atom, which is optionally substituted with R_{d2};
Z₃ is C atom or N atom, which is optionally substituted with R_{d3};
Z₄ is C atom or N atom, which is optionally substituted with R_{d4};
Z₅ is selected from covalent bond, CR_{d5} and N;
R_{d1} is selected from H, D, halogen, -CN, C₁₋₆ alkyl, C₁₋₆ haloalkyl, -L-OR', -L-NR'R", -L-C(O)NR'R", -L-3- to 10-membered carbocyclyl and -L-3- to 10-membered heterocyclyl;
R_{d2}, R_{d3}, R_{d4} and R_{d5} are independently selected from H, D, halogen, -CN, -L'-S(O)₀₋₂R', -L'-S(O)₂NR'R", -L'-S(O)NR'R", -L'-OR', -L'-NR'R", -L'-C(O)OR' and -L'-C(O)NR'R", or R_{d2}, R_{d3}, R_{d4} and R_{d5} are independently selected from C₁₋₆ alkyl, C₁₋₆ haloalkyl, -L'-3- to 10-membered carbocyclyl and -L'-3- to 10-membered heterocyclyl, each of which is optionally substituted with 1, 2, 3, 4, 5, 6, 7 or 8 R_{dd};
or R_{d2} and R_{d3} are taken together with the atoms to which they are attached, or R_{d3} and R_{d4} are taken together with the atoms to which they are attached, or R_{d4} and R_{d5} are taken together with the atoms to which they are attached, to form a 3- to 7-membered monocyclic heterocyclyl, a 6- to 10-membered bridged bicyclic heterocyclyl, a 6- to 10-membered spiro bicyclic heterocyclyl, or a 6- to 10-membered fused bicyclic heterocyclyl, and the 3- to 7-membered monocyclic heterocyclyl, 6- to 10-membered bridged bicyclic heterocyclyl, 6- to 10-membered spiro bicyclic heterocyclyl or 6- to 10-membered fused bicyclic heterocyclyl is optionally substituted with 1, 2, 3, 4, 5, 6, 7 or 8 R_{dd};
R_{dd} is independently selected from H, D, halogen, -CN, -NO₂, oxo, C₁₋₆ alkyl, C₁₋₆ haloalkyl, -L" -OR', -L"-NR'R", -L"-C(O)OR', -L"-C(O)NR'R", -L"-S(O)₀₋₂R', -L"-S(O)₂NR'R", -L" -S(O)NR'R", -L"-OC(O)R', -L"-OC(O)NR'R", -L''-3- to 10-membered carbocyclyl and -L''-3- to 10-membered heterocyclyl;
L and L' are independently selected from covalent bond, -O-, -S-, -NR'-, -S(O)₂- and -C(O)-; or, L or L' is C₁₋₆ alkylene, in which one or more methylene units are optionally and independently replaced by -C(R')₂-, -N(R')-, -O-, -C(O)-, -S-, -S(O)-, or -S(O)₂-;
R' and R" are independently selected from H, C₁₋₆ alkyl, C₁₋₆ haloalkyl, -L"-3- to 10-membered carbocyclyl and -L"-3- to 10-membered heterocyclyl; or, R' and R" are taken together with the N atom to which they are attached to form a 3- to 10-membered heterocyclyl;
L" is selected from covalent bond, -O- and -S-; or, L" is C₁₋₆ alkylene, in which one or more methylene units are optionally and independently replaced by -O-, -C(O)-, -S-, -S(O)-, or -S(O)₂-;
wherein each of the above groups is optionally deuterated, up to completely deuterated.

25. The compound of formula (II-1) according to claim 24, or a stereoisomer, an N-oxide, a hydrate, a solvate, a metabolite, a pharmaceutically acceptable salt, a polymorph, or a prodrug thereof, wherein
V is selected from covalent bond, CR_{C} and CR_{C}R'_{C};
Q is selected from O, S and NR_{A};
Y is selected from covalent bond, O, S and CR₁ₐR_{1b};
R_{A} and R_{B} are independently selected from H, C₁₋₆ alkyl and C₁₋₆ haloalkyl;
R_{C} and R'_{C} are independently selected from H, D, halogen, C₁₋₆ alkyl and C₁₋₆ haloalkyl;
R₁ₐ and R_{1b} are independently selected from H, D, halogen, CN, C₁₋₆ alkyl and C₁₋₆ haloalkyl;
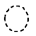 indicates that a double bond may be present or absent, when the double bond is present, it means that the ring where it is located is an aromatic ring, and R'ₑ₁, R'ₑ₂, R'ₑ₃ and R'ₑ₄ are absent, when the double bond is absent, it means that the ring where it is located is not aromatic;
X₂ is C or N;
X₃ is C or N;
X₄ is CRₑ₄, CRₑ₄R'ₑ₄ or N;
Rₑ₁ is selected from H, D, halogen, -CN, -OR', -SR', -NR'R", C₁₋₆ alkyl and C₁₋₆ haloalkyl;
Rₑ₂, Rₑ₃, Rₑ₄ and Rₑ₅ are independently selected from H, D, halogen, C₁₋₆ alkyl and C₁₋₆ haloalkyl;
R'ₑ₁, R'ₑ₂, R'ₑ₃ and R'ₑ₄ are independently selected from H, D, halogen, C₁₋₆ alkyl and C₁₋₆ haloalkyl; Z₁ is CR_{d1} or N;
Z₂ is C atom or N atom, which is optionally substituted with R_{d2};
Z₃ is C atom or N atom, which is optionally substituted with R_{d3};
Z₄ is C atom or N atom, which is optionally substituted with R_{d4};
Z₅ is selected from covalent bond, CR_{d5} and N;
R_{d1} and R_{d5} are independently selected from H, D, halogen, C₁₋₆ alkyl and C₁₋₆ haloalkyl;
R_{d2}, R_{d3} and R_{d4} are independently selected from H, D, halogen, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, and the C₁₋₆ alkyl, C₁₋₆ haloalkyl, 3- to 7-membered carbocyclyl or 3- to 7-membered heterocyclyl is optionally substituted with 1, 2, 3, 4, 5, 6, 7 or 8 R_{dd};
R_{dd} is independently selected from H, D, halogen, C₁₋₆ alkyl, C₁₋₆ haloalkyl, -L"-OR', -L"-SR' and -L"-NR'R";
R' and R" are independently selected from H, C₁₋₆ alkyl, C₁₋₆ haloalkyl, -L"-3- to 7-membered carbocyclyl and -L"-3- to 7-membered heterocyclyl; or, R' and R" are taken together with the N atom to which they are attached to form a 3- to 7-membered heterocyclyl;
L' is selected from covalent bond, O and C₁₋₆ alkylene;
L" is selected from covalent bond and C₁₋₆ alkylene;
each of the above groups is optionally deuterated, up to completely deuterated.

26. The compound of formula (II-1) according to claim 25, or a stereoisomer, an N-oxide, a hydrate, a solvate, a metabolite, a pharmaceutically acceptable salt, a polymorph, or a prodrug thereof, wherein
V is selected from covalent bond, CH, CH₂, CD, CHD and CD₂, alternatively covalent bond;
Q is selected from S and NR_{A}, alternatively NR_{A};
Y is selected from covalent bond, O and CR₁ₐR_{1b}, alternatively selected from covalent bond, O, CH₂, CF₂ and C(CH₃)₂, yet alternatively C(CH₃)₂;
R_{A} and R_{B} are H;
R₁ₐ and R_{1b} are independently selected from H, D, halogen, C₁₋₆ alkyl and C₁₋₆ haloalkyl, alternatively independently selected from H, D, F and Me;
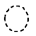 indicates that a double bond may be present or absent, when the double bond is present, it means that the ring where it is located is an aromatic ring, and R'ₑ₁, R'ₑ₂, R'ₑ₃ and R'ₑ₄ are absent, when the double bond is absent, it means that the ring where it is located is not aromatic;
X₂ is C or N;
X₃ is C or N; alternatively, one of X₂ and X₃ is C, and the other one is N;
X₄ is selected from CRₑ₄, CRₑ₄R'ₑ₄ and N;
Rₑ₁ is selected from H, D, halogen, -CN and -OR', alternatively H, D, F, Cl, -CN or -OCH₃;
Rₑ₂ is selected from H, D, C₁₋₆ alkyl and C₁₋₆ haloalkyl, alternatively H, D or Me;
Rₑ₃, Rₑ₄ and Rₑ₅ are independently selected from H and D;
R'ₑ₁, R'ₑ₂, R'ₑ₃ and R'ₑ₄ are independently selected from H and D;
Z₁ is CR_{d1} or N;
Z₂ is C atom or N atom, which is optionally substituted with R_{d2};
Z₃ is C atom or N atom, which is optionally substituted with R_{d3};
Z₄ is C atom or N atom, which is optionally substituted with R_{d4};
Z₅ is selected from covalent bond, CR_{d5} and N; alternatively, the ring where Z₂, Z₃, Z₄ and Z₅ are located is selected from benzene ring, pyridine ring, pyrazole ring and imidazole ring;
R_{d1} and R_{d5} are independently H or D;
R_{d2}, R_{d3} and R_{d4} are independently selected from H, D, C₁₋₆ alkyl, C₁₋₆ haloalkyl, -L'-NR'R" and -L'-4-to 6-membered heterocyclyl, and the C₁₋₆ alkyl, C₁₋₆ haloalkyl, or 4- to 6-membered heterocyclyl is optionally substituted with 1 or 2 R_{dd}; alternatively, R_{d2}, R_{d3} and R_{d4} are independently selected from H, D, 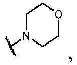 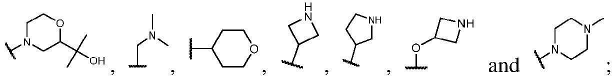 yet alternatively, R_{d2} is selected from H, D, 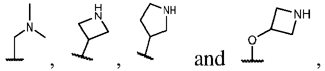 R_{d3} is selected from H, D, 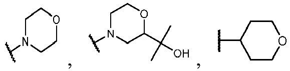 and 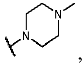 R_{d4} is selected from H, D and 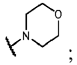
R_{dd} is independently selected from H, D, C₁₋₆ alkyl, C₁₋₆ haloalkyl, -L''-OR' and -L''-NR'R'', alternatively independently selected from H, D, Me, N(CH₃)₂ and C(CH₃)₂OH;
L' is selected from covalent bond, O and C₁₋₆ alkylene, alternatively covalent bond, O, or C₁₋₄ alkylene;
L'' is selected from covalent bond and C₁₋₆ alkylene, alternatively covalent bond or C₁₋₄ alkylene;
R' and R" are independently selected from H, C₁₋₆ alkyl and C₁₋₆ haloalkyl;
each of the above groups is optionally deuterated, up to completely deuterated.

27. The compound of formula (II-1) according to any one of claims 23 to 26, or a stereoisomer, an N-oxide, a hydrate, a solvate, a metabolite, a pharmaceutically acceptable salt, a polymorph, or a prodrug thereof, wherein 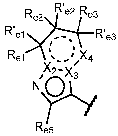 is selected from 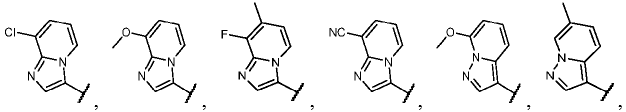 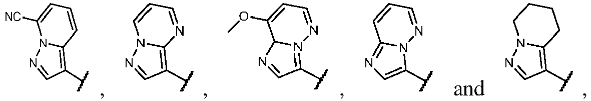 alternatively selected from 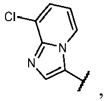 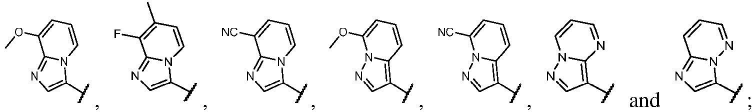 the ring where Z₂, Z₃, Z₄ and Z₅ are located and a substituent thereon are taken together to form the following groups: 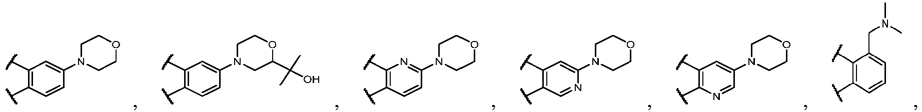 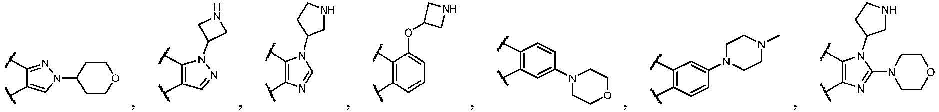 or 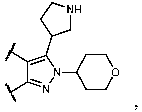 alternatively 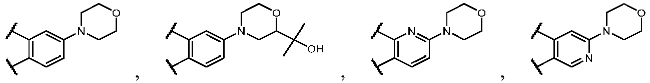 or 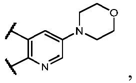 alternatively 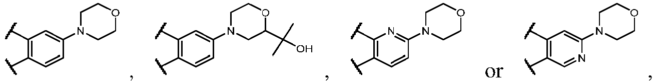 yet alternatively

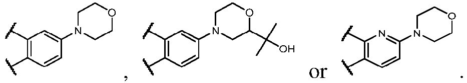

28. The compound of formula (III), formula (IV), or formula (V) according claim 23, or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N;
Rₑ₁ is selected from H, D, halogen, -CN, -OR', -SR', -NR'R", C₁₋₆ alkyl and C₁₋₆ haloalkyl;
Rₑ₂, Rₑ₃, Rₑ₄ and Rₑ₅ are independently selected from H, D, halogen, C₁₋₆ alkyl and C₁₋₆ haloalkyl;
R₁ₐ and R_{1b} are independently H, D, halogen, CN, C₁₋₆ alkyl or C₁₋₆ haloalkyl;
Z₁ is CR_{d1} or N, alternatively CR_{d1};
Z₂ is CR_{d2} or N, alternatively CR_{d2};
Z₄ is CR_{d4} or N, alternatively CR_{d4};
Z₅ is CR_{d5} or N, alternatively CR_{d5};
R_{d2} is selected from H, D, halogen, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, and the C₁₋₆ alkyl, C₁₋₆ haloalkyl, 3- to 7-membered carbocyclyl or 3- to 7-membered heterocyclyl is optionally substituted with 1, 2, 3, 4, 5, 6 or 7 R_{dd};
R_{d1}, R_{d4} or R_{d5} is independently selected from H, D, halogen, C₁₋₆ alkyl and C₁₋₆ haloalkyl;
R_{dd} is selected from H, D, halogen, C₁₋₆ alkyl, C₁₋₆ haloalkyl, -L''-OR', -L" -SR' and -L"-NR'R";
L' is selected from covalent bond, -O- and C₁₋₆ alkylene;
L" is selected from covalent bond and C₁₋₆ alkylene;
n=0, 1, 2, 3, 4, 5, 6, 7 or 8;
R' and R" are independently selected from H, C₁₋₆ alkyl, C₁₋₆ haloalkyl, -L"-3- to 7-membered carbocyclyl and -L"-3- to 7-membered heterocyclyl; or, R' and R" are taken together with the N atom to which they are attached to form a 3- to 7-membered heterocyclyl;
wherein each of the above groups is optionally deuterated, up to completely deuterated.

29. The compound of formula (III), formula (IV), or formula (V) according to claim 28, or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N;
Rₑ₁ is selected from H, D, halogen, -CN, -OR', C₁₋₆ alkyl and C₁₋₆ haloalkyl;
Rₑ₂, Rₑ₃, Rₑ₄ and Rₑ₅ are independently selected from H, D, halogen, C₁₋₆ alkyl and C₁₋₆ haloalkyl;
R₁ₐ and R_{1b} are independently C₁₋₆ alkyl or C₁₋₆ haloalkyl;
Z₁ is CR_{d1}; or N, alternatively CR_{d1};
Z₂ is CR_{d2} or N, alternatively CR_{d2};
Z₄ is CR_{d4} or N, alternatively CR_{d4};
Z₅ is CR_{d5} or N, alternatively CR_{d5}; alternatively, the ring where Z₂, Z₄ and Z₅ are located is selected from benzene ring and pyridine ring, alternatively benzene ring;
R_{d1}, R_{d2}, R_{d4} or R_{d5} is independently selected from H, D, halogen, C₁₋₆ alkyl and C₁₋₆ haloalkyl;
R_{dd} is selected from H, D, halogen, C₁₋₆ alkyl, C₁₋₆ haloalkyl, -L"-OR' and -L"-NR'R";
L" is selected from covalent bond and C₁₋₆ alkylene;
n=0, 1, 2, 3, 4, 5, 6, 7 or 8;
R' and R" are independently selected from H, C₁₋₆ alkyl and C₁₋₆ haloalkyl;
each of the above groups is optionally deuterated, up to completely deuterated.

30. The compound of formula (III) according to claim 29, or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N;
Rₑ₁ is selected from H, D, halogen and -OR', alternatively H, D or halogen;
Rₑ₂ is selected from H, D, C₁₋₆ alkyl and C₁₋₆ haloalkyl, alternatively H or D.
Rₑ₃, Rₑ₄ and Rₑ₅ are independently H or D;
R₁ₐ and R_{1b} are independently C₁₋₃ alkyl or C₁₋₃ haloalkyl, alternatively Me;
Z₁, Z₂, Z₄ and Z₅ are CH or CD;
R_{dd} is selected from H, D, C₁₋₆ alkyl, C₁₋₆ haloalkyl and -L"-OR';
L" is selected from covalent bond and C₁₋₆ alkylene;
n=0, 1, 2, 3, 4, 5, 6, 7 or 8;
R' is selected from H, C₁₋₆ alkyl and C₁₋₆ haloalkyl;
each of the above groups is optionally deuterated, up to completely deuterated.

31. The compound of formula (III) according to claim 30, or a stereoisomer, an N-oxide, a hydrate, a solvate, a metabolite, a pharmaceutically acceptable salt, a polymorph, or a prodrug thereof, wherein
X₂ is C or N;
X₃ is C or N; alternatively, one of X₂ and X₃ is C, and the other one is N;
X₄ is CRₑ₄ or N;
Rₑ₁ is selected from H, F, Cl and OCH₃, alternatively H, F or Cl;
Rₑ₂ is selected from H and Me, alternatively H;
Rₑ₃, Rₑ₄ and Rₑ₅ are H; alternatively, 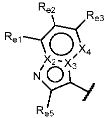 is selected from 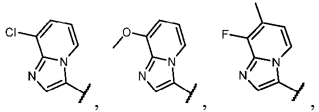 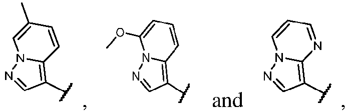 alternatively selected from 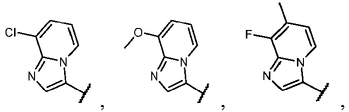 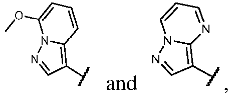 yet alternatively selected from 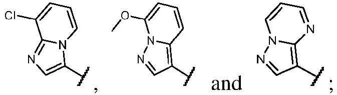
R₁ₐ and R_{1b} are Me;
Z₁, Z₂, Z₄ and Z₅ are CH;
R_{dd} is H or -L"-OH, alternatively H or C(CH₃)₂OH;
L" is selected from covalent bond and C₁₋₄ alkylene;
n=0 or 1; alternatively 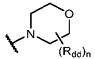 is

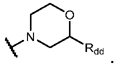

32. The compound of formula (IV) according to claim 29, or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N;
Rₑ₁ is selected from H, D, halogen, CN and -OR', alternatively selected from H, D, F, CN and OR';
Rₑ₂ is selected from H, D, C₁₋₆ alkyl and C₁₋₆ haloalkyl;
Rₑ₃, Rₑ₄ and Rₑ₅ are independently H or D;
R₁ₐ and R_{1b} are independently C₁₋₃ alkyl or C₁₋₃ haloalkyl, alternatively Me;
Z₁ is selected from CH, CD and N, alternatively CH or CD;
Z₂, Z₄ and Z₅ are independently selected from CH, CD and N;
R_{dd} is selected from H, D, C₁₋₆ alkyl, C₁₋₆ haloalkyl and -L"-OR';
L" is selected from covalent bond and C₁₋₆ alkylene;
n=0, 1, 2, 3, 4, 5, 6, 7 or 8;
R' is selected from H, C₁₋₆ alkyl and C₁₋₆ haloalkyl;
each of the above groups is optionally deuterated, up to completely deuterated.

33. The compound of formula (IV) according to claim 32, or a stereoisomer, an N-oxide, a hydrate, a solvate, a metabolite, a pharmaceutically acceptable salt, a polymorph, or a prodrug thereof, wherein
X₂ is C or N;
X₃ is C or N; alternatively, one of X₂ and X₃ is C, and the other one is N;
X₄ is CRₑ₄ or N;
Rₑ₁ is selected from H, F, Cl, CN and OCH₃, alternatively selected from H, F, CN and OCH₃;
Rₑ₂ is selected from H and Me;
Rₑ₃, Rₑ₄ and Rₑ₅ are H; alternatively, 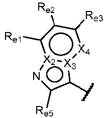 is selected from 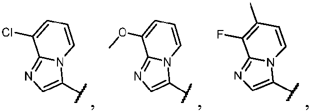 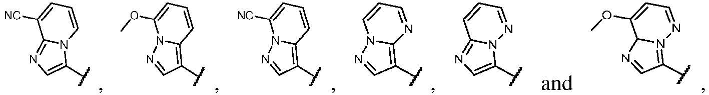 alternatively selected from 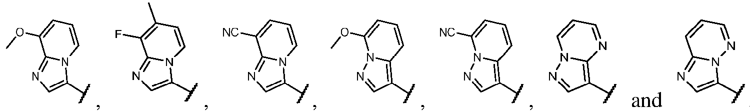 , yet alternatively selected from 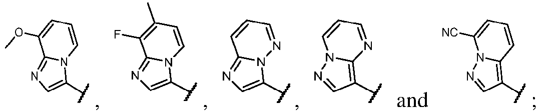 yet alternatively selected from 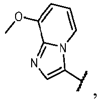 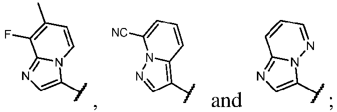
R₁ₐ and R_{1b} are Me;
Z₁ is CH;
Z₂, Z₄ and Z₅ are independently CH or N; alternatively Z₂ and Z₄ are independently CH or N, and Z₅ is CH; alternatively Z₂ is CH or N, and Z₄ and Z₅ are CH;
R_{dd} is H or -L"-OH, alternatively H or C(CH₃)₂OH;
L" is selected from covalent bond and C₁₋₄ alkylene;
n=0 or 1; alternatively

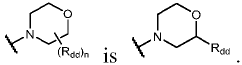

34. The compound of formula (V) according to claim 29, or a stereoisomer, an N-oxide, a hydrate, a solvate, a metabolite, a pharmaceutically acceptable salt, a polymorph, or a prodrug thereof, wherein
X₂ is C or N;
X₃ is C or N;
X₄ is CRₑ₄ or N, alternatively CRₑ₄;
Rₑ₁ is selected from H, D, halogen and -OR', alternatively OR';
Rₑ₂ is selected from H, D, C₁₋₆ alkyl and C₁₋₆ haloalkyl, alternatively H or D;
Rₑ₃, Rₑ₄ and Rₑ₅ are independently H or D;
R₁ₐ and R_{1b} are independently C₁₋₃ alkyl or C₁₋₃ haloalkyl, alternatively Me;
Z₁ is N, CH or CD, alternatively CH or CD;
Z₂, Z₄ and Z₅ are independently CH or CD;
R_{dd} is selected from H, D, C₁₋₆ alkyl, C₁₋₆ haloalkyl and -L"-OR', alternatively H or D;
L" is selected from covalent bond and C₁₋₆ alkylene;
n=0, 1, 2, 3, 4, 5, 6, 7 or 8;
R' is selected from H, C₁₋₆ alkyl and C₁₋₆ haloalkyl, alternatively C₁₋₆ alkyl or C₁₋₆ haloalkyl;
each of the above groups is optionally deuterated, up to completely deuterated.

35. The compound of formula (V) according to claim 34, or a stereoisomer, an N-oxide, a hydrate, a solvate, a metabolite, a pharmaceutically acceptable salt, a polymorph, or a prodrug thereof, wherein
X₂ is C or N;
X₃ is C or N; alternatively, one of X₂ and X₃ is C, and the other one is N;
X₄ is CRₑ₄ or N, alternatively CRₑ₄;
Rₑ₁ is selected from H, F, Cl and OCH₃, alternatively OCH₃;
Rₑ₂ is H;
Rₑ₃, Rₑ₄ and Rₑ₅ are H; alternatively, 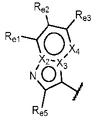 is selected from 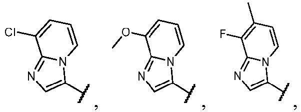 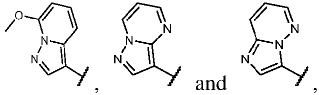 alternatively 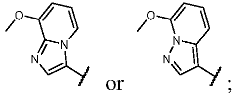
R₁ₐ and R_{1b} are Me;
Z₁ is CH or N;
Z₂, Z₄ and Z₅ are CH;
R_{dd} is H;
n=0.

36. The compound of formula (II) according to claim 23, or a stereoisomer, an N-oxide, a hydrate, a solvate, a metabolite, a pharmaceutically acceptable salt, a polymorph, or a prodrug thereof, 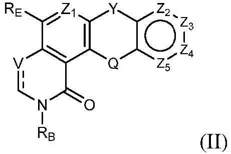 wherein
R_{E} is selected from -L-3- to 10-membered carbocyclyl, -L-3- to 10-membered heterocyclyl, -L-C₆₋₁₀ aryl and -L-5- to 10-membered heteroaryl, alternatively -L-C₆₋₁₀ aryl or -L-5- to 10-membered heteroaryl, each of which is optionally substituted with 1, 2, 3, 4 or 5 Rₑ;
Rₑ is independently selected from H, D, halogen, CN, oxo, C₁₋₆ alkyl, C₁₋₆ haloalkyl, C₂₋₆ alkenyl, C₂₋₆ alkynyl, -L'-OR', -L'-SR', -L'-NR'R", -L'-3- to 7-membered carbocyclyl and -L'-3- to 7-membered heterocyclyl;
Y is selected from O, S and CR₁ₐR_{1b};
R₁ₐ and R_{1b} are independently selected from H, D, halogen, C₁₋₆ alkyl and C₁₋₆ haloalkyl;
each of Q, V, R_{B} and Z₁ is as defined in any one of claims 23 to 25;
Z₂ is selected from CR_{d2} and N;
Z₃ is selected from CR_{d3} and N;
Z₄ is selected from CR_{d4} and N;
Z₅ is selected from covalent bond, CR_{d5} and N, alternatively CR_{d5} or N;
one of R_{d3} and R_{d4} is -NR'_{d6}C(O)R_{d6}, -L'-3- to 7-membered carbocyclyl or -L'-3- to 7-membered heterocyclyl, and the other one, R_{d2} and R_{d5} are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl;
R_{d2}, R_{d3}, R_{d4} and R_{d5} are optionally substituted with 1, 2, 3 or 4 R_{dd};
R_{dd} is independently selected from H, D, oxo, halogen, CN, C₁₋₆ alkyl, C₁₋₆ haloalkyl, -L"-OR', -L"-SR', -L"-NR'R", -L"-3- to 7-membered carbocyclyl and -L"-3- to 7-membered heterocyclyl;
or two non-adjacent R_{dd} are taken together to form a C₁₋₆ alkylene, alternatively form a C₁₋₄ alkylene, which is optionally substituted with 1, 2, 3 or 4 substituents selected from: H, D, halogen, CN, C₁₋₆ alkyl and C₁₋₆ haloalkyl;
R_{d6} is selected from H, C₁₋₆ alkyl, C₁₋₆ haloalkyl, -L"-OR', -L"-SR', -L"-NR'R", -L"-3- to 7-membered carbocyclyl, -L"-3- to 7-membered heterocyclyl and -L"-Y₃-C₁₋₆ alkylene-R₄;
Y₃ is selected from O, S and NR₃;
R'_{d6} and R₃ are independently selected from H, C₁₋₆ alkyl and C₁₋₆ haloalkyl;
R₄ is selected from -OR', -SR' and -NR'R";
L is independently selected from covalent bond, -O-, -S-, -NR'- and C₁₋₆ alkylene;
L' is independently selected from covalent bond and C₁₋₆ alkylene;
each of L and L' is optionally substituted with 1, 2, 3 or 4 groups selected from: C₁₋₆ alkyl, C₁₋₆ haloalkyl, -L_{d}-3- to 7-membered carbocyclyl and -L_{d}-3- to 7-membered heterocyclyl;
L" is independently selected from covalent bond and C₁₋₆ alkylene, each of which is optionally substituted with 1, 2, 3 or 4 R, and R is independently selected from H, D, C₁₋₆ alkyl, C₁₋₆ haloalkyl, -L_{d}-3- to 7-membered carbocyclyl and -L_{d}-3- to 7-membered heterocyclyl, or two R are taken together with the C atom to which they are jointly attached to form a 3- to 7-membered carbocyclylene or a 3- to 7-membered heterocyclylene;
L_{d} is independently selected from covalent bond and C₁₋₆ alkylene, alternatively selected from covalent bond and C₁₋₄ alkylene;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl;
wherein each of the above groups is optionally deuterated, up to completely deuterated.

37. The compound of formula (II) according to claim 36, or a stereoisomer, an N-oxide, a hydrate, a solvate, a metabolite, a pharmaceutically acceptable salt, a polymorph, or a prodrug thereof, wherein
R_{E} is -L-5- to 10-membered heteroaryl, alternatively 5- to 10-membered heteroaryl; alternatively, the heteroaryl in R_{E} is selected from pyridyl, pyridonyl, pyrazolyl, imidazolyl, oxazolyl, triazolyl, imidazopyridyl, imidazopyridazinyl, imidazopyrazinyl, imidazopyrimidinyl, pyrazolopyridyl, pyrazolopyrimidinyl, pyrazolopyrazinyl, triazolopyridyl, imidazopyrrolidinyl, imidazocyclohexyl, imidazopiperidyl, imidazoazepanyl, dihydroimidazooxazinyl, imidazopyrazinonyl, imidazodiazacyclohexyl, imidazopiperazinonyl, pyrazolopiperidyl, pyrazolodiazacyclohexyl, dihydropyrazolooxazinyl, triazolopiperidyl, dihydropyridooxazinyl, pyrrolopyridyl and oxazolopyridyl, each of which is optionally substituted with 1, 2, 3, 4 or 5 Rₑ;
Rₑ is independently selected from H, D, halogen, CN, oxo, C₁₋₆ alkyl, C₁₋₆ haloalkyl, C₂₋₆ alkynyl, -OR', -NR'R", 3- to 7-membered carbocyclyl and 3- to 7-membered heterocyclyl, alternatively selected from H, D, halogen, CN, oxo, C₁₋₆ alkyl, C₁₋₆ haloalkyl, C₂₋₆ alkynyl, -OR' and -NR'R";
Y is O or CR₁ₐR_{1b}, alternatively CR₁ₐR_{1b};
R₁ₐ and R_{1b} are independently selected from C₁₋₆ alkyl and C₁₋₆ haloalkyl, alternatively selected from Me and Et;
each of Q, V, R_{B} and Z₁ is as defined in any one of claims 23 to 25;
Z₂ is selected from CR_{d2} and N;
Z₃ is selected from CR_{d3} and N;
Z₄ is selected from CR_{d4} and N;
Z₅ is selected from CR_{d5} and N;
one of R_{d3} and R_{d4} is -NR'_{d6}C(O)R_{d6} or -L'-3- to 7-membered heterocyclyl, alternatively -L'-3- to 7-membered heterocyclyl, and the other one, R_{d2} and R_{d5} are independently selected from H, D, halogen, CN, C₁₋₆ alkyl and C₁₋₆ haloalkyl;
R_{d2}, R_{d3}, R_{d4} and R_{d5} are optionally substituted with 1, 2, 3 or 4 R_{dd};
R_{dd} is independently selected from H, D, oxo, C₁₋₆ alkyl, C₁₋₆ haloalkyl, -L"-OR', -L"-NR'R", -L"-3-to 7-membered carbocyclyl and -L"-3- to 7-membered heterocyclyl;
or two non-adjacent R_{dd} are taken together to form a C₁₋₃ alkylene, which is optionally substituted with 1, 2, 3 or 4 substituents selected from: H, D, halogen, CN, C₁₋₆ alkyl and C₁₋₆ haloalkyl;
R_{d6} is selected from C₁₋₆ alkyl, C₁₋₆ haloalkyl, -L"-3- to 7-membered carbocyclyl, -L"-3- to 7-membered heterocyclyl and -L"-Y₃-C₁₋₄ alkylene-R₄;
Y₃ is selected from O and NR₃, alternatively O;
R'_{d6} and R₃ are independently selected from H, C₁₋₆ alkyl and C₁₋₆ haloalkyl, alternatively H, C₁₋₃ alkyl or C₁₋₃ haloalkyl;
R₄ is selected from -OR' and -NR'R", alternatively -OR';
L is independently selected from covalent bond, -NR'- and C₁₋₄ alkylene, alternatively covalent bond or -NR'-; alternatively, L is covalent bond, -NH- or -CH(CH₃)-, alternatively covalent bond or -NH-;
L' is independently selected from covalent bond and C₁₋₄ alkylene;
each of L and L' is optionally substituted with 1, 2, 3 or 4 groups selected from: C₁₋₆ alkyl and C₁₋₆ haloalkyl;
L" is independently selected from covalent bond and C₁₋₆ alkylene, each of which is optionally substituted with 1, 2, 3 or 4 R, and R is independently selected from H, D, C₁₋₆ alkyl and C₁₋₆ haloalkyl, or two R are taken together with the C atom to which they are jointly attached to form a 3- to 7-membered carbocyclylene, alternatively form a 3- to 5-membered carbocyclylene;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl, alternatively selected from H, C₁₋₆ alkyl and C₁₋₆ haloalkyl.

38. The compound of formula (II) or formula (II') according to claim 23, or a stereoisomer, an N-oxide, a hydrate, a solvate, a metabolite, a pharmaceutically acceptable salt, a polymorph, or a prodrug thereof, 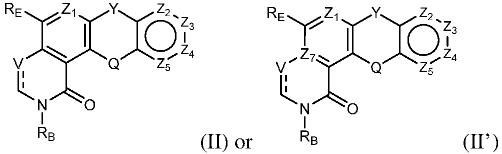
R_{E} is as defined in claim 36 or 37;
Rₑ is independently selected from H, D, halogen, CN, oxo, C₁₋₆ alkyl, C₁₋₆ haloalkyl, C₂₋₆ alkenyl, C₂₋₆ alkynyl, -L'-OR', -L'-SR', -L'-NR'R", -L'-3- to 7-membered carbocyclyl and -L'-3- to 7-membered heterocyclyl, alternatively selected from H, D, halogen, CN, oxo, C₁₋₆ alkyl, C₁₋₆ haloalkyl, C₂₋₆ alkynyl, -OR', -NR'R", 3- to 7-membered carbocyclyl and 3- to 7-membered heterocyclyl, alternatively selected from H, D, halogen, CN, oxo, C₁₋₆ alkyl, C₁₋₆ haloalkyl, C₂₋₆ alkynyl, -OR' and -NR'R", each of which is optionally substituted with 1, 2 or 3 Rₑₛ;
or two geminal Rₑ are taken together with the atom to which they are jointly attached to form a 3- to 7-membered carbocyclyl or a 3- to 7-membered heterocyclyl, alternatively form a 3- to 7-membered heterocyclyl, which is optionally substituted with 1, 2 or 3 Rₑₛ;
Rₑₛ is independently selected from H, D, halogen, C₁₋₆ alkyl, C₁₋₆ haloalkyl, -OR' and -NR'R";
each of Q, V, R_{B}, Z₁ and Z₇ is as defined in any one of claim 1 and claims 23 to 25;
Z₂ is selected from CR_{d2} and N;
Z₃ is selected from CR_{d3} and N;
Z₄ is selected from CR_{d4} and N;
Z₅ is selected from covalent bond, CR_{d5} and N, alternatively CR_{d5} or N;
one of R_{d3} and R_{d4} is -NR'_{d6}C(O)R_{d6}, -C(O)NR_{d7}R'_{d7}, -L'-3- to 7-membered carbocyclyl, or -L'-3- to 7-membered heterocyclyl, alternatively -NR'_{d6}C(O)R_{d6}, -C(O)NR_{d7}R'_{d7}, or -L'-3- to 7-membered heterocyclyl, and the other one, R_{d2} and R_{d5} are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl, alternatively selected from H, D, halogen, CN, C₁₋₆ alkyl, C₁₋₆ haloalkyl, OR' and NR'R";
R_{d2}, R_{d3}, R_{d4} and R_{d5} are optionally substituted with 1, 2, 3 or 4 R_{dd};
R_{dd} is independently selected from H, D, oxo, halogen, CN, C₁₋₆ alkyl, C₁₋₆ haloalkyl, -L"-OR', -L"-SR', -L"-NR'R", -L"-3- to 7-membered carbocyclyl and -L"-3- to 7-membered heterocyclyl; alternatively selected from H, D, oxo, C₁₋₆ alkyl, C₁₋₆ haloalkyl, -L"-OR', -L"-NR'R", -L"-3- to 7-membered carbocyclyl and -L"-3- to 7-membered heterocyclyl;
or CR_{dd}R_{dd} forms a 3- to 7-membered carbocyclylene or a 3- to 7-membered heterocyclylene, which is optionally substituted with 1, 2 or 3 groups selected from: H, D, halogen, C₁₋₆ alkyl, C₁₋₆ haloalkyl, -OR' and -NR'R", alternatively optionally substituted with one -OR';
or two non-adjacent R_{dd} are taken together to form a C₁₋₆ alkylene, alternatively form a C₁₋₄ alkylene, alternatively form a C₁₋₃ alkylene, which is optionally substituted with 1, 2, 3 or 4 substituents selected from: H, D, halogen, CN, C₁₋₆ alkyl and C₁₋₆ haloalkyl;
or two adjacent R_{dd} are taken together with the atoms to which they are attached to form a 3- to 7-membered carbocyclylene, a 3- to 7-membered heterocyclylene, a phenylene, or a 5- to 6-membered heteroarylene, alternatively form a 3- to 7-membered heterocyclylene or a 5- to 6-membered heteroarylene, alternatively form a 3- to 7-membered heterocyclylene, which is optionally substituted with 1, 2, 3 or 4 substituents selected from: H, D, halogen, CN, C₁₋₆ alkyl, C₁₋₆ haloalkyl, -OR' and -NR'R";
R_{d7} and R'_{d7} are independently selected from H, C₁₋₆ alkyl and C₁₋₆ haloalkyl;
the remaining variables are as defined in claim 36 or 37;
wherein each of the above groups is optionally deuterated, up to completely deuterated.

39. The compound of formula (VI) or formula (VI-1) according to claim 23, or a stereoisomer, an N-oxide, a hydrate, a solvate, a metabolite, a pharmaceutically acceptable salt, a polymorph, or a prodrug thereof, 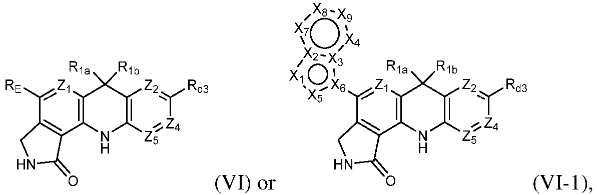 wherein
R_{E} is as defined in any one of claims 36 to 38;
X₁ is CRₑ₆, N, O, S or NR'ₑ₆;
X₂ is C or N;
X₃ is C or N;
X₄ is CRₑ₄ or N;
X₅ is CRₑ₅, N, O, S or NR'ₑ₅;
X₆ is C or N;
X₇ is CRₑ₁ or N;
X₈ is CRₑ₂ or N;
X₉ is CRₑ₃ or N;
Rₑ₁ and Rₑ₂ are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl;
Rₑ₃, Rₑ₄, Rₑ₅ and Rₑ₆ are independently selected from H, D, halogen, CN, C₁₋₆ alkyl and C₁₋₆ haloalkyl;
R'ₑ₅ and R'ₑ₆ are independently selected from H, C₁₋₆ alkyl and C₁₋₆ haloalkyl;
R₁ₐ and R_{1b} are independently selected from H, D, halogen, C₁₋₆ alkyl and C₁₋₆ haloalkyl;
Z₁ is selected from CR_{d1} and N;
Z₂ is selected from CR_{d2} and N;
Z₄ is selected from CR_{d4} and N;
Z₅ is selected from CR_{d5} and N;
R_{d1} is selected from H, D, halogen, CN, C₁₋₆ alkyl and C₁₋₆ haloalkyl;
R_{d2}, R_{d4} and R_{d5} are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl;
R_{d3} is selected from 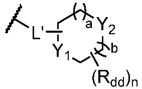 and -NR'_{d6}C(O)R_{d6};
a and b are independently 0, 1, 2 or 3, alternatively a+b≤5;
L' is independently selected from covalent bond and C₁₋₆ alkylene, each of which is optionally substituted with 1, 2, 3 or 4 groups selected from: C₁₋₆ alkyl and C₁₋₆ haloalkyl;
Y₁ and Y₂ are independently selected from CR_{dd}R_{dd}, O, S and NR'_{dd}, or when Y₁ is connected to L', Y₁ is CR_{dd} or N;
R_{dd} is independently selected from H, D, oxo, halogen, CN, C₁₋₆ alkyl, C₁₋₆ haloalkyl, -L"-OR', -L"-SR', -L"-NR'R", -L"-3- to 7-membered carbocyclyl and -L"-3- to 7-membered heterocyclyl;
or two non-adjacent R_{dd} are taken together to form a C₁₋₄ alkylene, or R'_{dd} and a non-adjacent R_{dd} are taken together to form a C₁₋₄ alkylene, or two R'_{dd} are taken together to form a C₁₋₄ alkylene, wherein the C₁₋₄ alkylene is optionally substituted with 1, 2, 3 or 4 substituents selected from: H, D, halogen, CN, C₁₋₆ alkyl and C₁₋₆ haloalkyl;
R'_{dd} is independently selected from H, C₁₋₆ alkyl, C₁₋₆ haloalkyl, -L"-OR', -L"-SR', -L"-NR'R", -L"-3- to 7-membered carbocyclyl and -L"-3- to 7-membered heterocyclyl;
n=0, 1, 2, 3 or 4;
R_{d6} is selected from H, C₁₋₆ alkyl, C₁₋₆ haloalkyl, -L"-OR', -L"-SR', -L"-NR'R", -L"-3- to 7-membered carbocyclyl, -L"-3- to 7-membered heterocyclyl and -L"-Y₃-C₁₋₆ alkylene-R₄;
L" is independently selected from covalent bond and C₁₋₆ alkylene, each of which is optionally substituted with 1, 2, 3 or 4 R, and R is independently selected from H, D, C₁₋₆ alkyl, C₁₋₆ haloalkyl, -L_{d}-3- to 7-membered carbocyclyl and -L_{d}-3- to 7-membered heterocyclyl, or two R are taken together with the C atom to which they are jointly attached to form a 3- to 7-membered carbocyclylene or a 3- to 7-membered heterocyclylene;
L_{d} is independently selected from covalent bond and C₁₋₆ alkylene, alternatively selected from covalent bond and C₁₋₄ alkylene;
Y₃ is selected from O, S and NR₃;
R'_{d6} and R₃ are independently selected from H, C₁₋₆ alkyl and C₁₋₆ haloalkyl;
R₄ is selected from -OR', -SR' and -NR'R";
each of R' and R" is independently selected from H, C₁₋₆ alkyl, C₁₋₆ haloalkyl, -L_{d}-3- to 7-membered carbocyclyl and -L_{d}-3- to 7-membered heterocyclyl;
wherein each of the above groups is optionally deuterated, up to completely deuterated.

40. The compound of formula (VI) or formula (VI-1) according to claim 39, or a stereoisomer, an N-oxide, a hydrate, a solvate, a metabolite, a pharmaceutically acceptable salt, a polymorph, or a prodrug thereof,
wherein R_{E} is as defined in any one of claims 36 to 38;
X₁ is CRₑ₆, N or NR'ₑ₆, alternatively N;
X₂ is C or N;
X₃ is C or N;
X₄ is CRₑ₄ or N;
X₅ is CRₑ₅, N or NR'ₑ₅, alternatively CRₑ₅ or N;
X₆ is C or N, alternatively C;
X₇ is CRₑ₁ or N;
X₈ is CRₑ₂ or N;
X₉ is CRₑ₃ or N;
Rₑ₁ and Rₑ₂ are independently selected from H, D, halogen, CN, C₁₋₆ alkyl, C₁₋₆ haloalkyl, -OR', -NR'R", 3- to 7-membered carbocyclyl and 3- to 7-membered heterocyclyl, alternatively selected from H, D, halogen, CN, C₁₋₆ alkyl, C₁₋₆ haloalkyl, -OR' and -NR'R";
Rₑ₃, Rₑ₄, Rₑ₅ and Rₑ₆ are independently H, D, halogen, C₁₋₆ alkyl or C₁₋₆ haloalkyl;
R'ₑ₅ and R'ₑ₆ are independently selected from H, C₁₋₆ alkyl and C₁₋₆ haloalkyl;
R₁ₐ and R_{1b} are independently C₁₋₆ alkyl or C₁₋₆ haloalkyl;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1}, R_{d2}, R_{d4} and R_{d5} are independently selected from H, D, halogen, CN, C₁₋₆ alkyl and C₁₋₆ haloalkyl;
R_{d3} is selected from 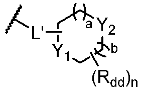 and -NR'_{d6}C(O)R_{d6};
a and b are independently 0, 1 or 2; alternatively a+b≤3;
L' is independently selected from covalent bond and C₁₋₄ alkylene;
Y₁ is CR_{dd}R_{dd} or NR'_{dd}, or when Y₁ is connected to L', Y₁ is CH, CD or N;
Y₂ is CR_{dd}R_{dd}, O or NR'_{dd}; alternatively, at least one of Y₁ and Y₂ is a heteroatom group;
R_{dd} is independently selected from H, D, oxo, C₁₋₆ alkyl, C₁₋₆ haloalkyl, -L"-OR', -L"-NR'R", -L"-3-to 7-membered carbocyclyl and -L"-3- to 7-membered heterocyclyl;
or two non-adjacent R_{dd} are taken together to form a C₁₋₃ alkylene, which is optionally substituted with 1, 2, 3 or 4 substituents selected from: H, D, halogen, CN, C₁₋₆ alkyl and C₁₋₆ haloalkyl;
R'_{dd} is independently selected from H, C₁₋₆ alkyl, C₁₋₆ haloalkyl, -C₁₋₆ alkylene-OR', -C₁₋₆ alkylene-NR'R", -L"-3- to 7-membered carbocyclyl and -L"-3- to 7-membered heterocyclyl, alternatively selected from H, C₁₋₆ alkyl, C₁₋₆ haloalkyl, -L"-3- to 7-membered carbocyclyl and -L"-3- to 7-membered heterocyclyl;
n=0, 1, 2, 3 or 4;
R_{d6} is selected from C₁₋₆ alkyl, C₁₋₆ haloalkyl, -L"-3- to 7-membered carbocyclyl, -L"-3- to 7-membered heterocyclyl and -L"-Y₃-C₁₋₄ alkylene-R₄, alternatively not -L"-Y₃-C₁₋₄ alkylene-R₄;
L" is independently selected from covalent bond and C₁₋₆ alkylene, each of which is optionally substituted with 1, 2, 3 or 4 R, and R is independently selected from H, D, C₁₋₆ alkyl and C₁₋₆ haloalkyl, or two R are taken together with the C atom to which they are jointly attached to form a 3- to 7-membered carbocyclylene;
Y₃ is selected from O and NR₃;
R'_{d6} and R₃ are independently selected from H, C₁₋₆ alkyl and C₁₋₆ haloalkyl;
R₄ is selected from -OR' and -NR'R";
each of R' and R" is independently selected from H, C₁₋₆ alkyl, C₁₋₆ haloalkyl, 3- to 7-membered carbocyclyl and 3- to 7-membered heterocyclyl;
alternatively, at most five of X₁-X₉ are heteroatoms, alternatively at most four are heteroatoms, and alternatively at most three are heteroatoms;
alternatively, one of X₂ and X₃ is N, and the other one is C;
alternatively, at most one of X₄ and X₇-X₉ is N;
alternatively, at most two of Z₂, Z₄ and Z₅ are N, alternatively at most one is N.

41. The compound of formula (VI) or formula (VI-1) according to claim 39 or 40, or a stereoisomer, an N-oxide, a hydrate, a solvate, a metabolite, a pharmaceutically acceptable salt, a polymorph, or a prodrug thereof, wherein R_{E} is as defined in any one of claims 36 to 38;
X₁ is N;
X₂ is C or N;
X₃ is C or N;
X₄ is CRₑ₄ or N, alternatively N;
X₅ is CRₑ₅ or N, alternatively CRₑ₅;
X₆ is C;
X₇ is CRₑ₁ or N, alternatively CRₑ₁;
X₈ is CRₑ₂ or N, alternatively N;
X₉ is CRₑ₃ or N, alternatively CRₑ₃;
Rₑ₁ and Rₑ₂ are independently selected from H, D, halogen, CN, C₁₋₆ alkyl, C₁₋₆ haloalkyl, -OR', -O-3-to 7-membered carbocyclyl and 3- to 7-membered heterocyclyl, alternatively selected from H, D, halogen, CN, C₁₋₆ alkyl, C₁₋₆ haloalkyl and -OR';
Rₑ₃, Rₑ₄ and Rₑ₅ are independently H or D;
R'ₑ₅ is selected from H, C₁₋₃ alkyl and C₁₋₃ haloalkyl;
R₁ₐ and R_{1b} are independently C₁₋₃ alkyl or C₁₋₃ haloalkyl;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is H or D;
R_{d2}, R_{d4} and R_{d5} are independently selected from H, D, halogen, C₁₋₃ alkyl and C₁₋₃ haloalkyl, alternatively R_{d2} and R_{d5} are independently selected from H, D and halogen, alternatively R_{d5} is H or D;
R_{d3} is selected from 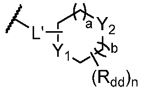 and -NR'_{d6}C(O)R_{d6}; alternatively, 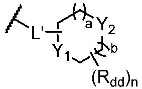 is 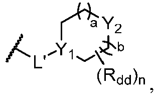 alternatively 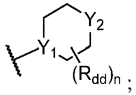
a and b are independently 0, 1 or 2, alternatively a and b are 1;
L' is independently selected from covalent bond and C₁₋₂ alkylene, alternatively selected from covalent bond and methylene;
Y₁ is NR'_{dd}, alternatively NH; or when Y₁ is connected to L', Y₁ is CH, CD or N;
Y₂ is CR_{dd}R_{dd}, O or NR'_{dd};
R_{dd} is independently selected from H, D, oxo, C₁₋₆ alkyl, C₁₋₆ haloalkyl, -L"-OR', -L"-NR'R" and -L"-3- to 7-membered carbocyclyl, alternatively selected from H, D, oxo, C₁₋₆ alkyl, C₁₋₆ haloalkyl, -L"-OR' and -L"-NR'R", alternatively selected from H, D, C₁₋₆ alkyl, C₁₋₆ haloalkyl and -L"-OR';
or two non-adjacent R_{dd} are taken together to form a C₁₋₂ alkylene;
R'_{dd} is independently selected from H, C₁₋₆ alkyl, C₁₋₆ haloalkyl, -C₁₋₄ alkylene-OR', -C₁₋₄ alkylene-NR'R" and -L"-3- to 7-membered carbocyclyl, alternatively selected from H, C₁₋₆ alkyl, C₁₋₆ haloalkyl and -L"-3- to 7-membered carbocyclyl;
n=0, 1, 2, 3 or 4;
R_{d6} is selected from C₁₋₆ alkyl, C₁₋₆ haloalkyl, -L"-3- to 7-membered carbocyclyl and -L"-O-C₁₋₄ alkylene-OR', alternatively not -L"-O-C₁₋₄ alkylene-OR';
R'_{d6} is selected from H, C₁₋₆ alkyl and C₁₋₆ haloalkyl;
L" is independently selected from covalent bond, C₁₋₄ alkylene and -C(R)₂-, alternatively selected from covalent bond and C₁₋₄ alkylene, wherein the two R in -C(R)₂- are taken together with the C atom to which they are jointly attached to form a 3- to 5-membered carbocyclylene, alternatively form 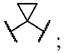
each of R' and R" is independently selected from H, C₁₋₆ alkyl and C₁₋₆ haloalkyl;
alternatively, when Y₁ is N and Y₂ is O, L' is a covalent bond and R_{dd} is not oxo;
alternatively, when Rₑ₂ is -O-C₁₋₆ alkyl, Rₑ₂ is -OMe, alternatively, when Rₑ₂ is -O-C₁₋₆ haloalkyl, Rₑ₂ is -O-halomethyl;
alternatively, at least one of R_{d2} and R_{d4} is H or D, alternatively at least one is H.

42. The compound of formula (VI) or formula (VI-1) according to any one of claims 39 to 41, or a stereoisomer, an N-oxide, a hydrate, a solvate, a metabolite, a pharmaceutically acceptable salt, a polymorph, or a prodrug thereof, wherein R_{E} is as defined in any one of claims 36 to 38;
X₁ is N;
X₂ is C or N;
X₃ is C or N;
X₄ is CRₑ₄ or N, alternatively N;
X₅ is CRₑ₅ or N, alternatively CRₑ₅;
X₆ is C;
X₇ is CRₑ₁ or N, alternatively CRₑ₁;
X₈ is CRₑ₂ or N, alternatively N;
X₉ is CRₑ₃ or N, alternatively CRₑ₃;
Rₑ₁ and Rₑ₂ are independently selected from H, F, Cl, CN, Me, Et, CHF₂, OCH₃, OEt, OCHF₂, -O-cyclopropyl and 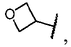 alternatively selected from H, F, Cl, CN, Me, CHF₂, OCH₃ and OEt, alternatively Rₑ₂ is not OEt;
Rₑ₃, Rₑ₄ and Rₑ₅ are H;
R'ₑ₅ is Me;
R₁ₐ and R_{1b} are independently Me or Et;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is H;
R_{d2}, R_{d4} and R_{d5} are independently selected from H, F, Me and CHF₂, alternatively R_{d2} and R_{d5} are independently selected from H and F, alternatively R_{d5} is H;
R_{d3} is selected from 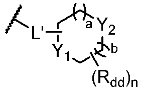
and -NR'_{d6}C(O)R_{d6}, alternatively 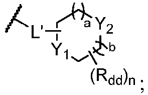
a is 1 or 2, b is 1;
L' is independently selected from covalent bond and methylene, alternatively covalent bond;
Y₁ is NH, CH or N, alternatively CH or N, alternatively N;
Y₂ is CR_{dd}R_{dd}, O or NR'_{dd}, alternatively O;
R_{dd} is independently selected from H, oxo, OH, Me, -C(CH₃)₂OH, -CH₂OH, -CH₂OMe, 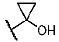
and -NHMe, alternatively H, oxo, OH, Me, -C(CH₃)₂OH, -CH₂OH, -CH₂OMe or -NHMe;
or two non-adjacent R_{dd} are taken together to form -CH₂CH₂-, alternatively make 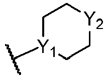 has the following structure: 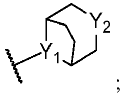
R'_{dd} is independently selected from H, Me and -CH₂-cyclopropyl;
n=0, 1 or 2;
R_{d6} is selected from -CH₂-cyclopropyl and -CH₂-O-CH₂CH₂-OH, alternatively -CH₂-cyclopropyl;
R'_{d6} is H;
alternatively,
R_{d3} is selected from: 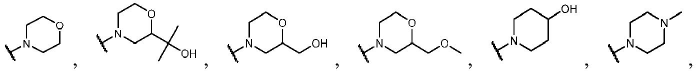 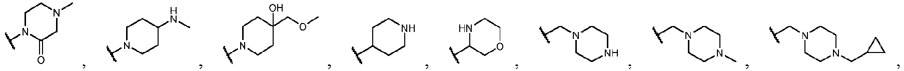 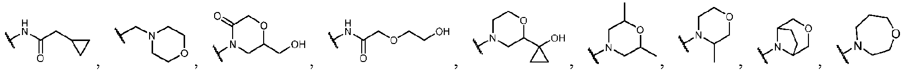 and 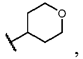 alternatively selected from: 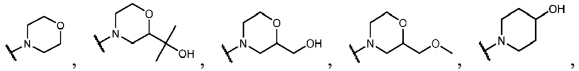 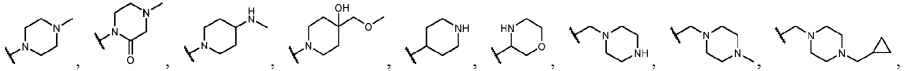 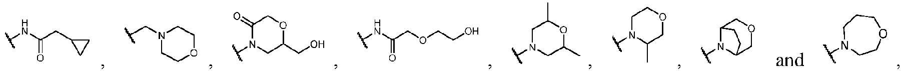 alternatively selected from: 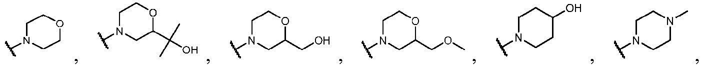 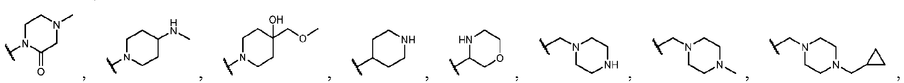 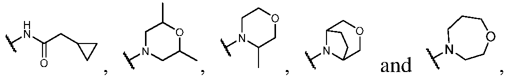 alternatively selected from: 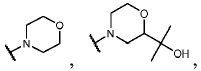 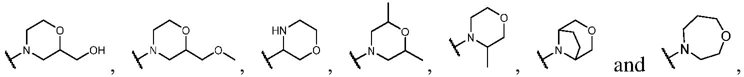 alternatively 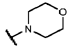 or 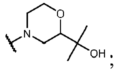
alternatively, R_{d3} is selected from: 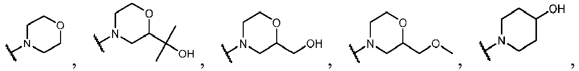 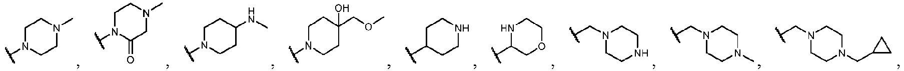 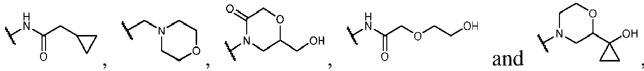 alternatively selected from: 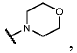 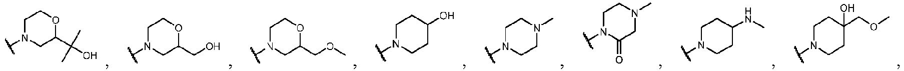 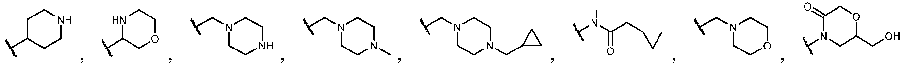 and 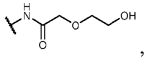 alternatively selected from: 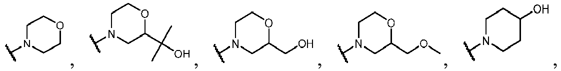 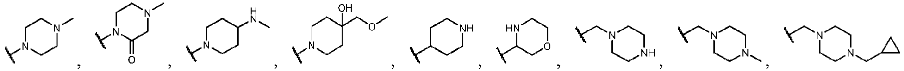 and 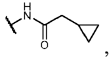 alternatively selected from: 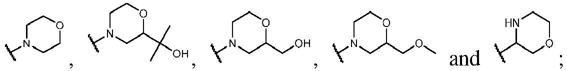
alternatively, 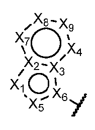 is selected from: 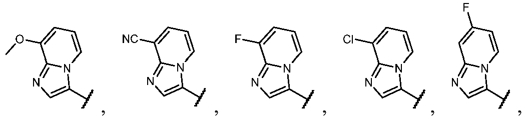 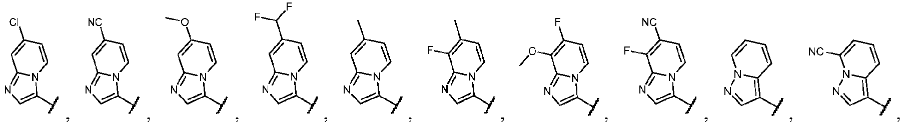 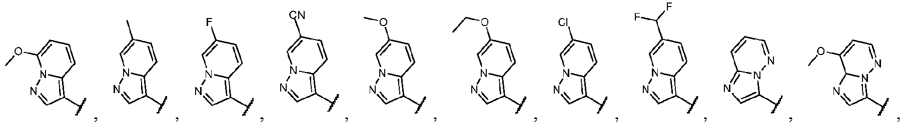 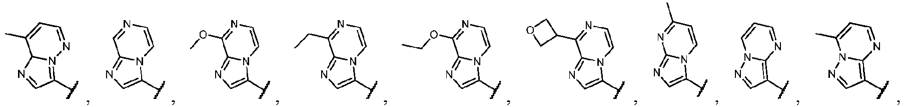 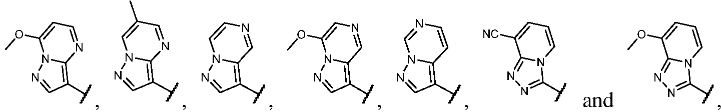 alternatively selected from: 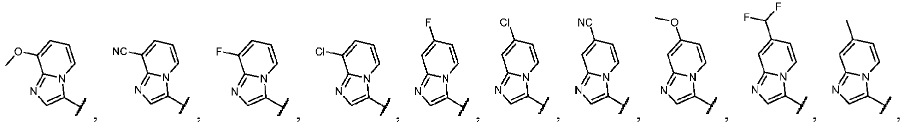 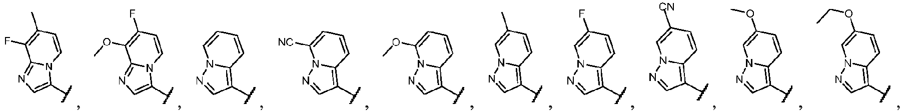 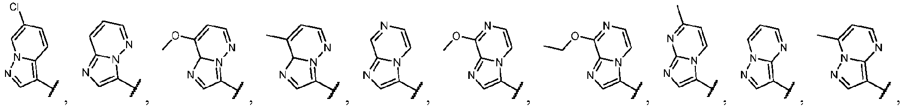 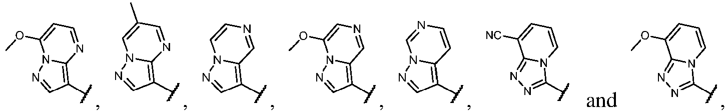 alternatively not 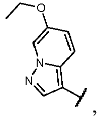 alternatively selected from:

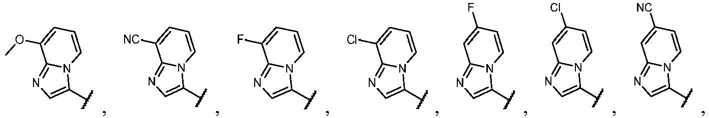

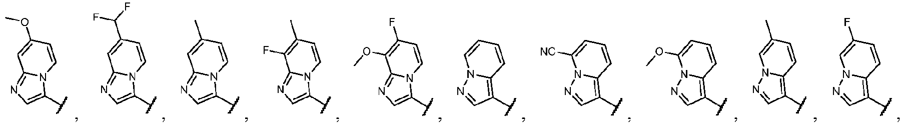

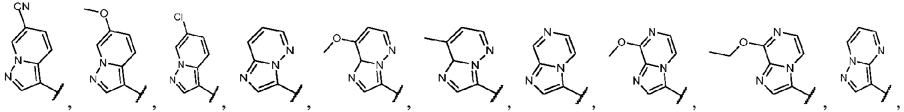

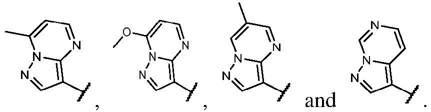

43. The compound of formula (VI) or formula (VI-1) according to claim 23, or a stereoisomer, an N-oxide, a hydrate, a solvate, a metabolite, a pharmaceutically acceptable salt, a polymorph, or a prodrug thereof, 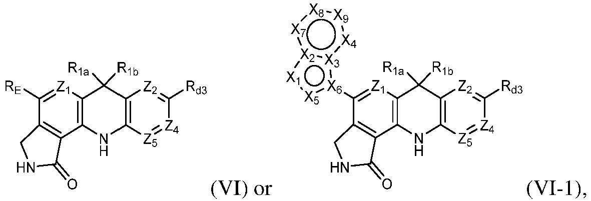 wherein
R_{E} is as defined in any one of claims 36 to 38;
X₅ is CRₑ₅, N, O, S or NR'ₑ₅, alternatively CRₑ₅, O, N or NR'ₑ₅, alternatively CRₑ₅ or N;
X₇ is CRₑ₁ or N;
X₈ is CRₑ₂ or N;
Rₑ₁ and Rₑ₂ are independently selected from H, D, halogen, CN, C₁₋₆ alkyl, C₁₋₆ haloalkyl, C₂₋₆ alkenyl, C₂₋₆ alkynyl, -L'-OR', -L'-SR', -L'-NR'R'', -L'-3- to 7-membered carbocyclyl and -L'-3- to 7-membered heterocyclyl, alternatively selected from H, D, halogen, CN, C₁₋₆ alkyl, C₁₋₆ haloalkyl, C₂₋₆ alkenyl, C₂₋₆ alkynyl, -OR', -NR'R", 3- to 7-membered carbocyclyl and 3- to 7-membered heterocyclyl, each of which is optionally substituted with 1, 2 or 3 Rₑₛ;
Rₑₛ is independently selected from H, D, halogen, C₁₋₆ alkyl, C₁₋₆ haloalkyl, -OR' and -NR'R";
Rₑ₅ is selected from H, D, halogen, CN, C₁₋₆ alkyl and C₁₋₆ haloalkyl, alternatively selected from H, D, halogen, C₁₋₆ alkyl and C₁₋₆ haloalkyl;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is selected from H, D, halogen, CN, C₁₋₆ alkyl and C₁₋₆ haloalkyl;
R_{d2}, R_{d4} and R_{d5} are independently selected from H, D, halogen, CN, C₁₋₆ alkyl, C₁₋₆ haloalkyl, -L'-OR', -L'-SR', -L'-NR'R'', -L'-3- to 7-membered carbocyclyl and -L'-3- to 7-membered heterocyclyl, alternatively selected from H, D, halogen, CN, C₁₋₆ alkyl, C₁₋₆ haloalkyl, -OR' and -NR'R"; alternatively R_{d2} and R_{d5} are independently selected from H, D, halogen, C₁₋₆ alkyl and C₁₋₆ haloalkyl;
R_{d3} is selected from 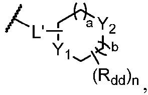 -NR'_{d6}C(O)R_{d6} and -C(O)NR_{d7}R'_{d7};
a and b are independently 0, 1, 2 or 3, alternatively 0, 1 or 2; alternatively a+b≤5, alternatively a+b≤3;
L' is independently selected from covalent bond and C₁₋₆ alkylene, alternatively selected from covalent bond and C₁₋₄ alkylene, each of which is optionally substituted with 1, 2, 3 or 4 groups selected from: C₁₋₆ alkyl and C₁₋₆ haloalkyl;
Y₁ is CR_{dd}R_{dd} or NR'_{dd}; or when Y₁ is connected to L', Y₁ is CR_{dd} or N;
Y₂ is selected from CR_{dd}R_{dd}, O, S, S(O), NR'_{dd} and S(O)₂, alternatively selected from CR_{dd}R_{dd}, O, NR'_{dd} and S(O)₂; alternatively, at least one of Y₁ and Y₂ is a heteroatom group;
R_{dd} is independently selected from H, D, oxo, halogen, CN, C₁₋₆ alkyl, C₁₋₆ haloalkyl, -L"-OR', -L"-SR', -L"-NR'R", -L"-3- to 7-membered carbocyclyl and -L"-3- to 7-membered heterocyclyl, alternatively selected from H, D, halogen, oxo, C₁₋₆ alkyl, C₁₋₆ haloalkyl, -L"-OR', -L"-NR'R", -L"-3- to 7-membered carbocyclyl and -L"-3- to 7-membered heterocyclyl;
or CR_{dd}R_{dd} forms a 3- to 7-membered carbocyclylene or a 3- to 7-membered heterocyclylene, which is optionally substituted with 1, 2 or 3 groups selected from: H, D, halogen, C₁₋₆ alkyl, C₁₋₆ haloalkyl, -OR' and -NR'R";
or two non-adjacent R_{dd} are taken together to form a C₁₋₃ alkylene, which is optionally substituted with 1, 2, 3 or 4 substituents selected from: H, D, halogen, CN, C₁₋₆ alkyl and C₁₋₆ haloalkyl;
R'_{dd} is independently selected from H, C₁₋₆ alkyl, C₁₋₆ haloalkyl, -L"-OR', -L"-NR'R", -L"-3- to 7-membered carbocyclyl and -L"-3- to 7-membered heterocyclyl, alternatively selected from H, C₁₋₆ alkyl, C₁₋₆ haloalkyl, -L"-3- to 7-membered carbocyclyl and -L"-3- to 7-membered heterocyclyl;
or R'_{dd} and an adjacent R_{dd} are taken together with the atoms to which they are attached to form a 3- to 7-membered heterocyclylene or a 5- to 6-membered heteroarylene, alternatively form a 3- to 7-membered heterocyclylene, which is optionally substituted with 1, 2, 3 or 4 substituents selected from: H, D, halogen, CN, C₁₋₆ alkyl, C₁₋₆ haloalkyl, -OR' and -NR'R";
R_{d7} and R'_{d7} are selected from H, C₁₋₆ alkyl and C₁₋₆ haloalkyl;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl, alternatively selected from H, C₁₋₆ alkyl, C₁₋₆ haloalkyl, 3-to 7-membered carbocyclyl and 3- to 7-membered heterocyclyl;
L_{d} is independently selected from covalent bond and C₁₋₆ alkylene, alternatively selected from covalent bond and C₁₋₄ alkylene;
the remaining variables are as defined in any one of claims 39 to 42;
wherein each of the above groups is optionally deuterated, up to completely deuterated.

44. The compound of formula (VI) or formula (VI-1) according to claim 43, or a stereoisomer, an N-oxide, a hydrate, a solvate, a metabolite, a pharmaceutically acceptable salt, a polymorph, or a prodrug thereof, wherein R_{E} is as defined in any one of claims 36 to 38;
X₅ is CRₑ₅ or N, alternatively CRₑ₅;
X₇ is CRₑ₁ or N, alternatively CRₑ₁;
X₈ is CRₑ₂ or N, alternatively N;
Rₑ₁ and Rₑ₂ are independently selected from H, D, halogen, CN, C₁₋₆ alkyl, C₁₋₆ haloalkyl, C₂₋₆ alkynyl, -OR', -NR'R", -O-3- to 7-membered carbocyclyl, -O-3- to 7-membered heterocyclyl, 3- to 7-membered carbocyclyl and 3- to 7-membered heterocyclyl, alternatively not -NR'R", alternatively selected from H, D, halogen, CN, C₁₋₆ alkyl, C₁₋₆ haloalkyl, C₂₋₆ alkynyl, -OR', -O-3- to 7-membered carbocyclyl, 3- to 7-membered carbocyclyl and 3- to 7-membered heterocyclyl, each of which is optionally substituted with 1, 2 or 3 Rₑₛ; alternatively Rₑ₁ is not heterocyclyl;
Rₑₛ is independently selected from H, D, halogen, C₁₋₆ alkyl, C₁₋₆ haloalkyl and -OR', alternatively selected from H, D, F, Me and OH;
Rₑ₅ is selected from H, D, halogen, C₁₋₄ alkyl and C₁₋₄ haloalkyl, alternatively H or D;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is H or D;
R_{d2}, R_{d4} and R_{d5} are independently selected from H, D, halogen, CN, C₁₋₄ alkyl, C₁₋₄ haloalkyl and -OR', alternatively R_{d2} and R_{d5} are independently selected from H, D and halogen; alternatively R_{d2}, R_{d4} and R_{d5} are H or D;
R_{d3} is selected from 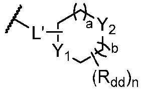 and -NR'_{d6}C(O)R_{d6}; alternatively, 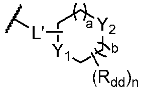 is 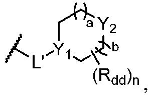 alternatively 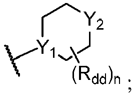
a and b are independently 0, 1 or 2, alternatively a and b are 1;
L' is independently selected from covalent bond and C₁₋₂ alkylene, alternatively selected from covalent bond and methylene;
Y₁ is NR'_{dd}; or when Y₁ is connected to L', Y₁ is CR_{dd} or N;
Y₂ is selected from CR_{dd}R_{dd}, O, NR'_{dd} and S(O)₂;
R_{dd} is independently selected from H, D, halogen, oxo, C₁₋₆ alkyl, C₁₋₆ haloalkyl, -L"-OR', -L"-NR'R" and -L"-3- to 7-membered carbocyclyl, alternatively selected from H, D, oxo, C₁₋₆ alkyl, C₁₋₆ haloalkyl, -L"-OR' and NR'R", alternatively selected from H, D, C₁₋₆ alkyl, C₁₋₆ haloalkyl and -L"-OR';
or CR_{dd}R_{dd} forms a 3- to 7-membered carbocyclylene or a 3- to 7-membered heterocyclylene, which is optionally substituted with one -OR', alternatively optionally substituted with one OH;
or two non-adjacent R_{dd} are taken together to form a C₁₋₂ alkylene;
R'_{dd} is independently selected from H, C₁₋₆ alkyl, C₁₋₆ haloalkyl and -L"-3- to 7-membered carbocyclyl;
or R'_{dd} and an adjacent R_{dd} are taken together with the atoms to which they are attached to form a 3- to 7-membered heterocyclylene or a 5- to 6-membered heteroarylene, alternatively form a 3- to 7-membered heterocyclylene, which is optionally substituted with 1, 2, 3 or 4 substituents selected from: H, D, halogen, C₁₋₆ alkyl and C₁₋₆ haloalkyl;
L" is independently selected from covalent bond and C₁₋₄ alkylene, each of which is optionally substituted with 1, 2, 3 or 4 R, and R is independently selected from H, D, C₁₋₆ alkyl and C₁₋₆ haloalkyl, or two R are taken together with the C atom to which they are jointly attached to form a 3- to 7-membered carbocyclylene, alternatively form a 3- to 5-membered carbocyclylene, alternatively form 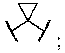
each of R' and R" is independently selected from H, C₁₋₆ alkyl and C₁₋₆ haloalkyl;
the remaining variables are as defined in any one of claims 39 to 42;
alternatively, when Y₁ is N and Y₂ is O, L' is a covalent bond and R_{dd} is not oxo;
alternatively, when Rₑ₂ is -O-C₁₋₆ alkyl, Rₑ₂ is -OMe, alternatively, when Rₑ₂ is -O-C₁₋₆ haloalkyl, Rₑ₂ is -O-halomethyl;
alternatively,
Rₑ₁ is selected from H, D, halogen, CN, C₁₋₆ alkyl, C₁₋₆ haloalkyl, -OR', -NR'R", -O-3- to 7-membered carbocyclyl, -O-3- to 7-membered heterocyclyl, 3- to 7-membered carbocyclyl and 3- to 7-membered heterocyclyl, alternatively not -NR'R" or 3- to 7-membered heterocyclyl, alternatively selected from H, D, halogen, CN, C₁₋₆ alkyl, C₁₋₆ haloalkyl, -OR', -O-3- to 7-membered carbocyclyl and 3- to 7-membered carbocyclyl, each of which is optionally substituted with 1, 2 or 3 Rₑₛ;
Rₑ₂ is selected from H, D, halogen, CN, C₁₋₆ alkyl, C₁₋₆ haloalkyl, C₂₋₆ alkynyl, -OR', -O-3- to 7-membered heterocyclyl, 3- to 7-membered carbocyclyl and 3- to 7-membered heterocyclyl, alternatively selected from H, D, halogen, CN, C₁₋₆ alkyl, C₁₋₆ haloalkyl, C₂₋₆ alkynyl, -OMe, -O-halomethyl, 3- to 7-membered carbocyclyl and 3- to 7-membered heterocyclyl, each of which is optionally substituted with 1, 2, or 3 Rₑₛ;
alternatively, when Rₑ₂ is heterocyclyl, Rₑₛ is independently selected from H, D, halogen (F), C₁₋₆ alkyl and C₁₋₆ haloalkyl, alternatively selected from H, D, F and Me, alternatively halogen (F).

45. The compound of formula (VI) or formula (VI-1) according to claim 43 or 44, or a stereoisomer, an N-oxide, a hydrate, a solvate, a metabolite, a pharmaceutically acceptable salt, a polymorph, or a prodrug thereof, wherein R_{E} is as defined in any one of claims 36 to 38;
X₅ is CRₑ₅ or N, alternatively CRₑ₅;
X₇ is CRₑ₁ or N, alternatively CRₑ₁;
X₈ is CRₑ₂ or N, alternatively N;
Rₑ₁ and Rₑ₂ are independently selected from H, F, Cl, CN, Me, Et, n-Bu, CHF₂, 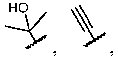 OH, OCH₃, OEt, OCHF₂, -NHCH₃, -O-cyclopropyl, 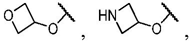 cyclopropyl, 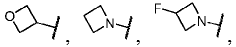 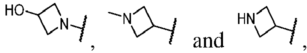 alternatively selected from H, F, Cl, CN, Me, Et, n-Bu, CHF₂, 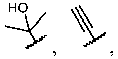 OCH₃, OEt, OCHF₂, -NHCH₃, -O-cyclopropyl, 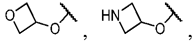 cyclopropyl, 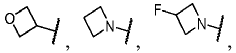 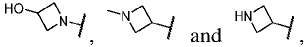 alternatively not 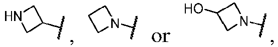 alternatively selected from H, F, Cl, CN, Me, Et, CHF₂, 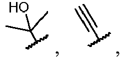 OCH₃, OEt, OCHF₂, -O-cyclopropyl, cyclopropyl, 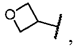 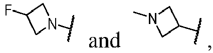 alternatively Rₑ₁ is not 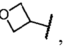 alternatively Rₑ₂ is not OEt;
Rₑ₅ is selected from H, D, F, Br and Me, alternatively H;
Z₁ is selected from CR_{d1} and N, alternatively CR_{d1};
Z₂ is selected from CR_{d2} and N;
Z₄ is selected from CR_{d4} and N;
Z₅ is selected from CR_{d5} and N, alternatively CR_{d5};
R_{d1} is H;
R_{d2}, R_{d4} and R_{d5} are independently selected from H, F, CN, Me, CHF₂ and OCH₃, alternatively R_{d2} and R_{d5} are independently selected from H and F; alternatively, R_{d2}, R_{d4} and R_{d5} are H;
R_{d3} is selected from 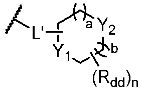
and -NR'_{d6}C(O)R_{d6}, alternatively 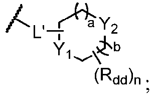
a is 0, 1 or 2, alternatively 0 or 1, b is 0 or 1;
L' is independently selected from covalent bond and methylene, alternatively covalent bond;
Y₁ is NH; or when Y₁ is connected to L', Y₁ is selected from CH, C(OH) and N, alternatively selected from CH and N, alternatively N;
Y₂ is selected from CR_{dd}R_{dd}, O, NR'_{dd} and S(O)₂, alternatively selected from CR_{dd}R_{dd}, O and NR'_{dd}, alternatively O;
R_{dd} is independently selected from H, F, oxo, OH, Me, -C(CH₃)₂OH, -CH₂OH, -CH₂OMe, 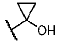 and -NHMe, alternatively selected from H, oxo, OH, Me, -C(CH₃)₂OH, -CH₂OH, -CH₂OMe, 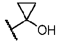 and -NHMe, alternatively selected from H, OH, Me, -C(CH₃)₂OH, -CH₂OMe and 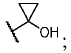
or CR_{dd}R_{dd} forms cyclopropylene, 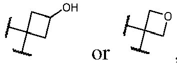
, alternatively forms cyclopropylene or 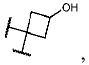 alternatively forms cyclopropylene;
or two non-adjacent R_{dd} are taken together to form -CH₂CH₂-;
R'_{dd} is independently selected from H, Me and -CH₂-cyclopropyl;
or R'_{dd} and an adjacent R_{dd} are taken together with the atoms to which they are attached to form 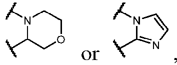
alternatively form 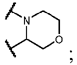
the remaining variables are as defined in any one of claims 39 to 42;
alternatively,
Rₑ₁ is selected from H, F, Cl, CN, Me, Et, n-Bu, CHF₂, OCH₃, OEt, OCHF₂, -NHCH₃, -O-cyclopropyl, 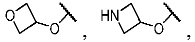 cyclopropyl, 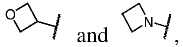 alternatively selected from H, F, Cl, CN, Me, Et, CHF₂, OCH₃, OEt, OCHF₂, -O-cyclopropyl, 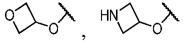 and cyclopropyl, alternatively selected from H, F, CN, Me, CHF₂, OCH₃, OEt, OCHF₂, -O-cyclopropyl and cyclopropyl;
Rₑ₂ is selected from H, F, Cl, CN, Me, CHF₂, 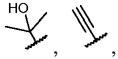 OCH₃, OEt, OCHF₂, 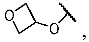 cyclopropyl, 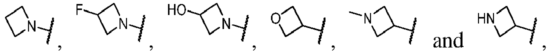 alternatively selected from H, F, Cl, CN, Me, CHF₂, 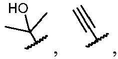 OCH₃, OCHF₂, cyclopropyl, 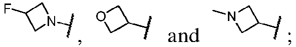
alternatively,
R_{d3} is selected from: 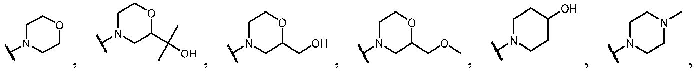 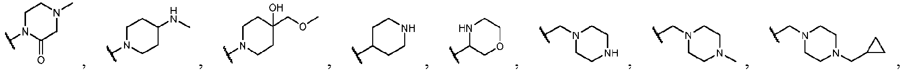 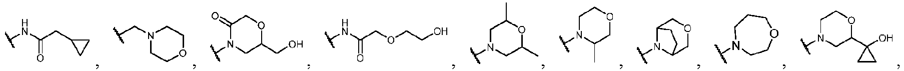 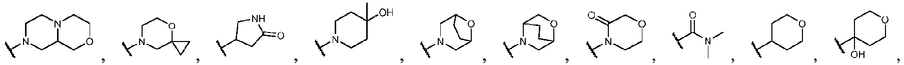 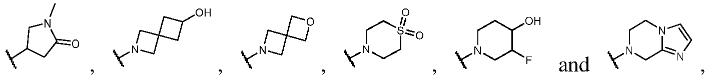 alternatively selected from: 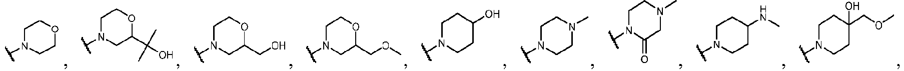 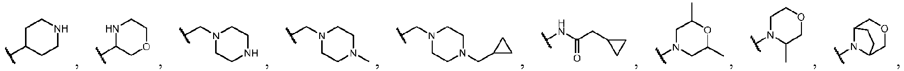 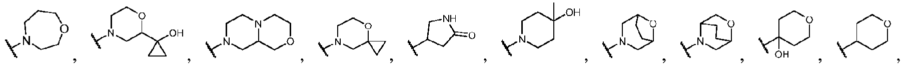 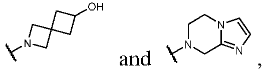 alternatively selected from: 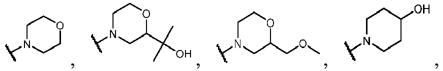 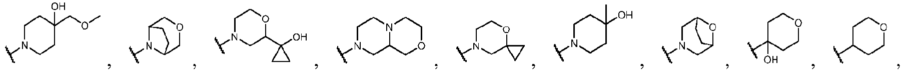 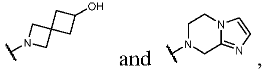 alternatively selected from: 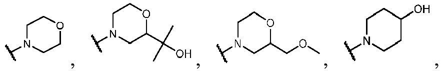 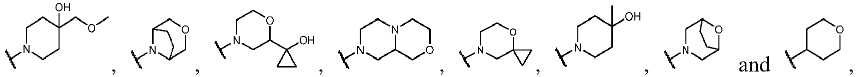 alternatively 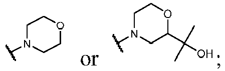
alternatively,
R_{d3} is selected from: 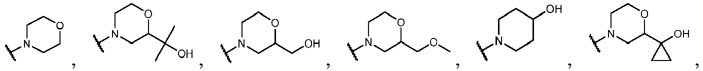 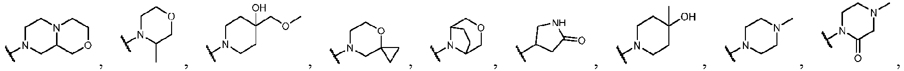 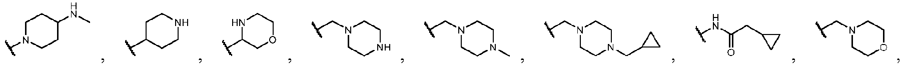 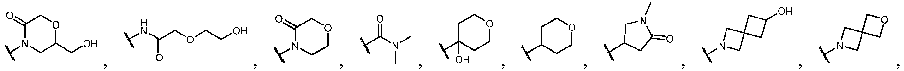 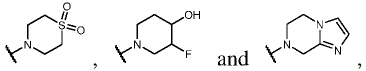 alternatively selected from: 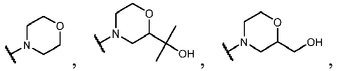 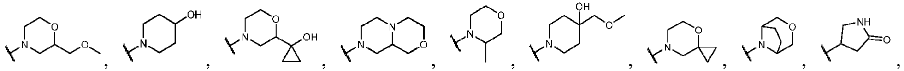 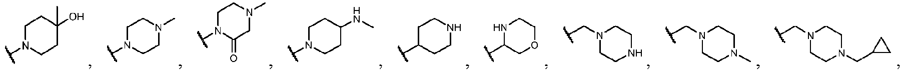 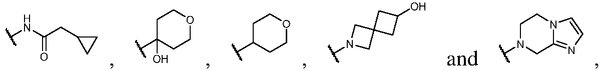 alternatively selected from: 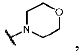 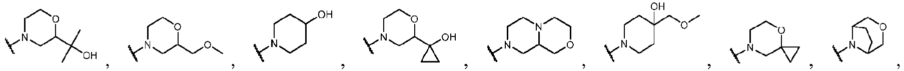 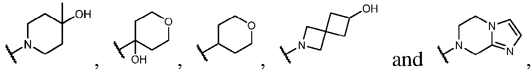 alternatively selected from: 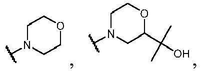 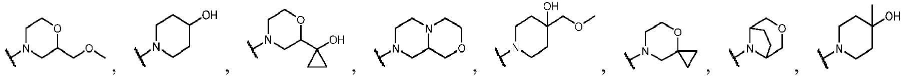 and 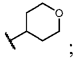
alternatively, 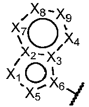
is selected from: 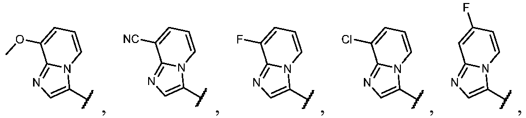 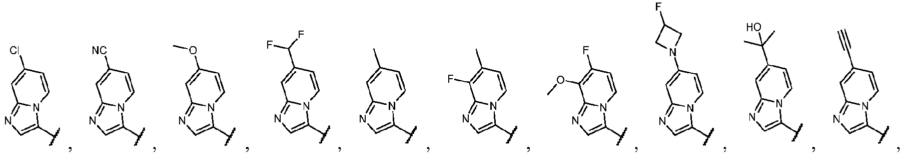 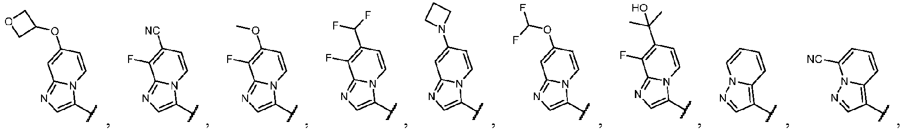 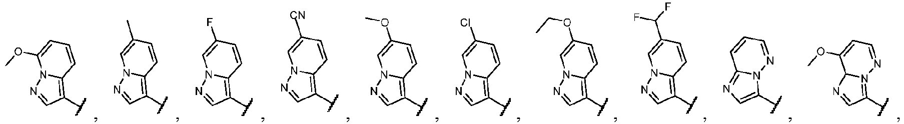 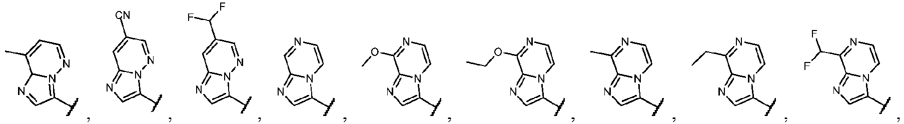 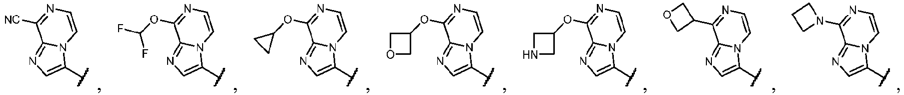 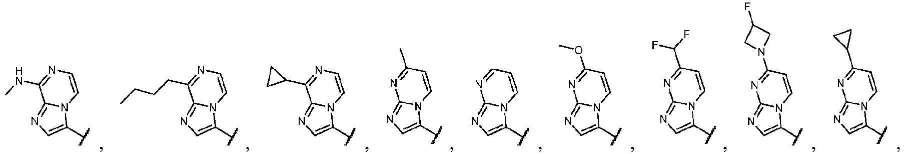 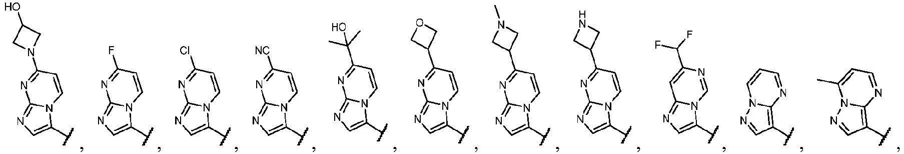 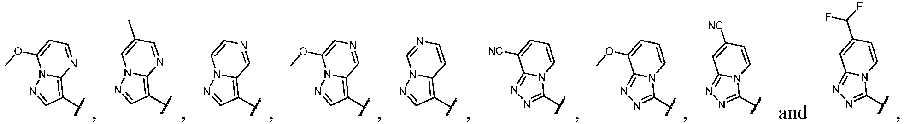 alternatively selected from: 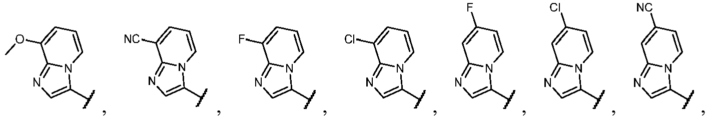 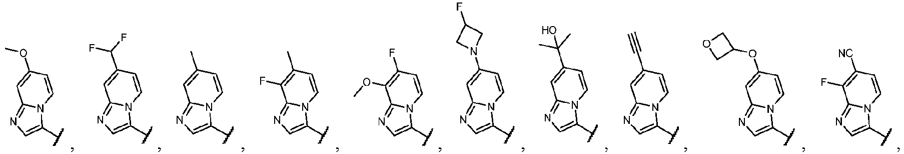 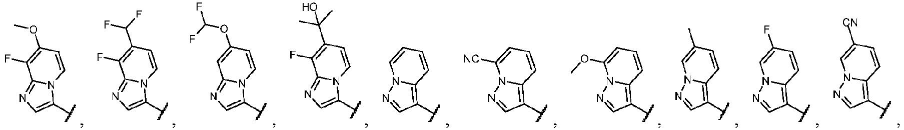 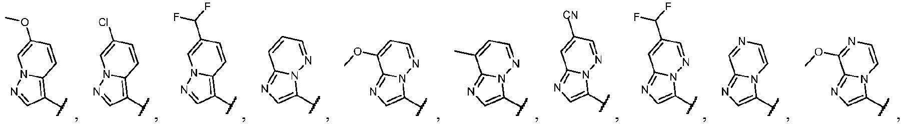 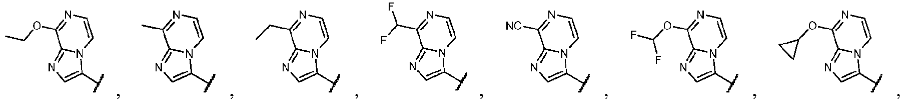 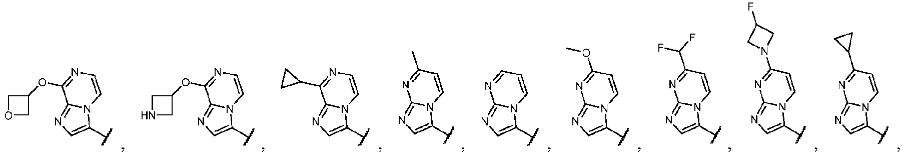 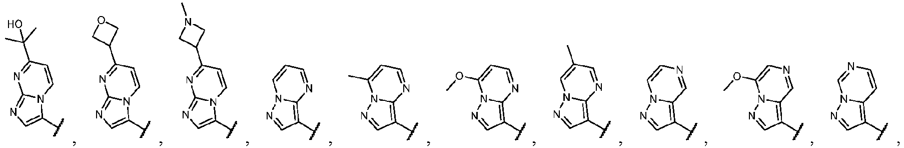 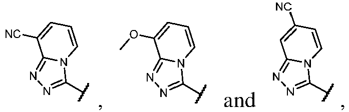 alternatively selected from:

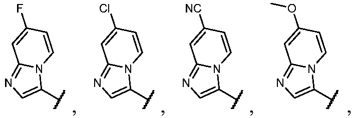

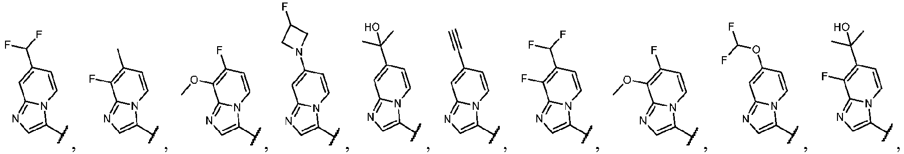

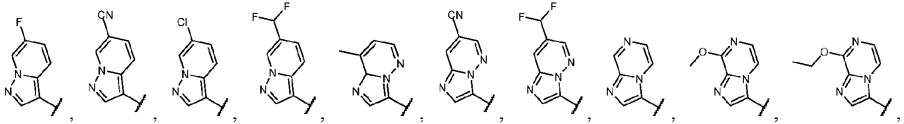

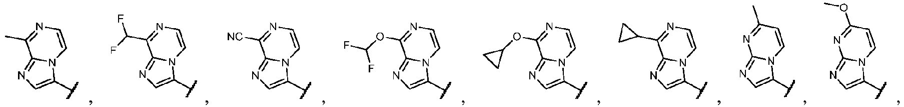

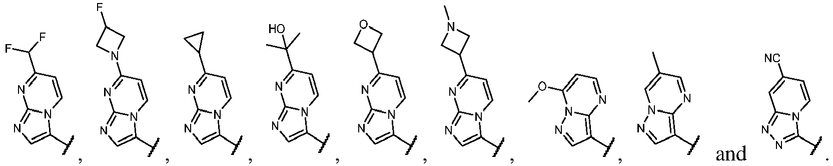

46. The compound of formula (VI-2) according to claim 23, or a stereoisomer, an N-oxide, a hydrate, a solvate, a metabolite, a pharmaceutically acceptable salt, a polymorph, or a prodrug thereof, 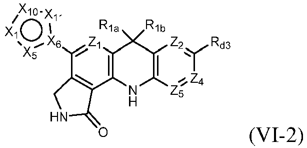 wherein
X₁ is selected from CRₑ₆, O, S, N and NR'ₑ₆;
X₅ is CRₑ₅, N, O, S or NR'ₑ₅;
X₆ is C or N;
X₁₀ is selected from CRₑ₇, O, S, N and NR'ₑ₇;
X₁₁ is selected from CRₑ₈, O, S, N and NR'ₑ₈;
Rₑ₇ and Rₑ₈ are independently selected from H, D, halogen, CN, C₁₋₆ alkyl, C₁₋₆ haloalkyl, -L'-OR', -L'-SR', -L'-NR'R", -L'-3- to 7-membered carbocyclyl and -L'-3- to 7-membered heterocyclyl;
R'ₑ₇ and R'ₑ₈ are independently selected from H, D, C₁₋₆ alkyl, C₁₋₆ haloalkyl, -L'-3- to 7-membered carbocyclyl and -L'-3- to 7-membered heterocyclyl;
or the substituents on X₁₀ and X₁₁ are taken together to form a C₃₋₅ alkylene, which optionally contains one double bond, and the C₃₋₅ alkylene is optionally substituted with 1, 2, 3 or 4 Rₑ, and 1, 2 or 3 methylene units in the C₃₋₅ alkylene are optionally and independently replaced by -NR"-, -N(R")C(O)-, -C(O)N(R")-, -O-, -C(O)-, -OC(O)-, -C(O)O-, -S-, or -S(O)-;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl;
L' is independently selected from covalent bond and C₁₋₆ alkylene, each of which is optionally substituted with 1, 2, 3 or 4 groups selected from: C₁₋₆ alkyl and C₁₋₆ haloalkyl;
L_{d} is independently selected from covalent bond and C₁₋₆ alkylene, alternatively selected from covalent bond and C₁₋₄ alkylene;
Rₑ is as defined in any one of claims 36 to 38, alternatively Rₑ is not alkenyl or alkynyl;
each of Rₑ₅, R'ₑ₅, Rₑ₆ and R'ₑ₆ is as defined in any one of claims 39 to 45;
each of R₁ₐ, R_{1b}, Z₁, Z₂, Z₄, Z₅ and R_{d3} is as defined in any one of claims 39 to 45;
wherein each of the above groups is optionally deuterated, up to completely deuterated.

47. The compound of formula (VI-2) according to claim 46, or a stereoisomer, an N-oxide, a hydrate, a solvate, a metabolite, a pharmaceutically acceptable salt, a polymorph, or a prodrug thereof, wherein
X₁ is selected from CRₑ₆, N and NR'ₑ₆, alternatively N;
X₅ is CRₑ₅, N or NR'ₑ₅;
X₆ is C or N, alternatively C;
X₁₀ is selected from CRₑ₇, N and NR'ₑ₇;
X₁₁ is selected from CRₑ₈, O, N and NR'ₑ₈;
Rₑ₇ is selected from H, D, C₁₋₆ alkyl, C₁₋₆ haloalkyl, -OR', -NR'R", 3- to 7-membered carbocyclyl and 3- to 7-membered heterocyclyl;
R'ₑ₇ is selected from H, D, C₁₋₆ alkyl, C₁₋₆ haloalkyl, 3- to 7-membered carbocyclyl and 3- to 7-membered heterocyclyl;
Rₑ₈ is selected from H, D, C₁₋₆ alkyl and C₁₋₆ haloalkyl;
R'ₑ₈ is selected from H, C₁₋₆ alkyl and C₁₋₆ haloalkyl;
or, the substituents on X₁₀ and X₁₁ are taken together to form a C₃₋₅ alkylene, alternatively form -(CH₂)₄-, which optionally contains one double bond, and the C₃₋₅ alkylene or -(CH₂)₄- is optionally substituted with 1, 2, 3 or 4 Rₑ, and 1, 2 or 3 methylene units in the C₃₋₅ alkylene or -(CH₂)₄- are optionally and independently replaced by -NR"-, -C(O)N(R")-, -C(O)-, or -O-, alternatively one methylene unit in the C₃₋₅ alkylene or -(CH₂)₄- is optionally and independently replaced by -O-;
each of R' and R" is independently selected from H, C₁₋₆ alkyl, C₁₋₆ haloalkyl, 3- to 7-membered carbocyclyl and 3- to 7-membered heterocyclyl;
Rₑ is as defined in any one of claims 36 to 38, alternatively Rₑ is not alkenyl or alkynyl;
each of Rₑ₅, R'ₑ₅, Rₑ₆ and R'ₑ₆ is as defined in any one of claims 39 to 45;
each of R₁ₐ, R_{1b}, Z₁, Z₂, Z₄, Z₅ and R_{d3} is as defined in any one of claims 39 to 45;
alternatively, at most three of X₁, X₅, X₆, X₁₀ and X₁₁ are heteroatoms.

48. The compound of formula (VI-2) according to claim 46 or 47, or a stereoisomer, an N-oxide, a hydrate, a solvate, a metabolite, a pharmaceutically acceptable salt, a polymorph, or a prodrug thereof, wherein
X₁ is N;
X₅ is CRₑ₅, N or NR'ₑ₅, alternatively CRₑ₅ or N, yet alternatively CRₑ₅;
X₆ is C or N, alternatively C;
X₁₀ is CRₑ₇ or NR'ₑ₇;
X₁₁ is selected from CRₑ₈, O and NR'ₑ₈, alternatively CRₑ₈ or NR'ₑ₈;
Rₑ₇ is selected from C₁₋₆ alkyl, C₁₋₆ haloalkyl, 3- to 7-membered carbocyclyl, 3- to 7-membered heterocyclyl and -O-3- to 7-membered heterocyclyl;
R'ₑ₇ is selected from C₁₋₆ alkyl, C₁₋₆ haloalkyl, 3- to 7-membered carbocyclyl and 3- to 7-membered heterocyclyl, alternatively 3- to 7-membered carbocyclyl;
alternatively, when Rₑ₇ or R'ₑ₇ is heterocyclyl, Rₑ₇ or R'ₑ₇ is 3- to 7-membered oxacyclyl, alternatively 4- to 6-membered oxacyclyl, alternatively 5-membered oxacyclyl;
Rₑ₈ is selected from H, D, C₁₋₆ alkyl and C₁₋₆ haloalkyl, alternatively C₁₋₆ alkyl or C₁₋₆ haloalkyl;
R'ₑ₈ is selected from C₁₋₆ alkyl and C₁₋₆ haloalkyl;
or, the substituents on X₁₀ and X₁₁ are taken together to form 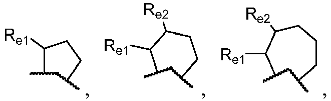 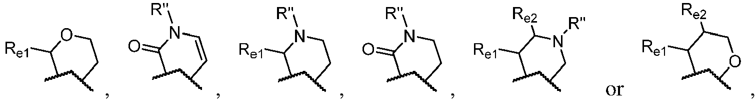 alternatively not form 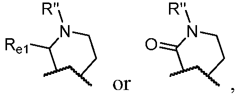 alternatively form a structure selected from: 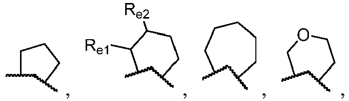 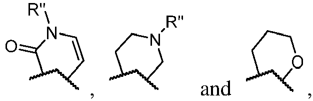 alternatively form 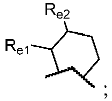
each of R' and R" is independently selected from H, C₁₋₆ alkyl and C₁₋₆ haloalkyl;
each of Rₑ₁, Rₑ₂, Rₑ₅ and R'ₑ₅ is as defined in any one of claims 39 to 45; alternatively, Rₑ₁ and Rₑ₂ are independently selected from H, D, halogen, CN, C₁₋₆ alkyl, C₁₋₆ haloalkyl, -OR' and -O-3- to 7-membered carbocyclyl, alternatively selected from H, D, C₁₋₆ alkyl, C₁₋₆ haloalkyl and -OR';
each of R₁ₐ, R_{1b}, Z₁, Z₂, Z₄, Z₅ and R_{d3} is as defined in any one of claims 39 to 45.

49. The compound of formula (VI-2) according to any one of claims 46 to 48, or a stereoisomer, an N-oxide, a hydrate, a solvate, a metabolite, a pharmaceutically acceptable salt, a polymorph, or a prodrug thereof, wherein
X₁ is N;
X₅ is CRₑ₅, N or NR'ₑ₅, alternatively CRₑ₅ or N, yet alternatively CRₑ₅;
X₆ is C or N, alternatively C;
X₁₀ is selected from CRₑ₇ and NR'ₑ₇;
X₁₁ is selected from CRₑ₈, O and NR'ₑ₈, alternatively CRₑ₈ or NR'ₑ₈;
Rₑ₇ is selected from Me, Et, cyclopropyl, tetrahydrofuranyl, tetrahydropyranyl, pyrrolidinyl, morpholinyl and -O-tetrahydrofuranyl, alternatively selected from Me, Et, cyclopropyl, tetrahydrofuranyl, tetrahydropyranyl and -O-tetrahydrofuranyl, alternatively selected from Me, Et, cyclopropyl, tetrahydrofuranyl and -O-tetrahydrofuranyl, alternatively selected from Me, Et, cyclopropyl, 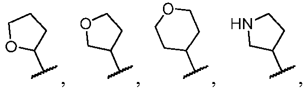 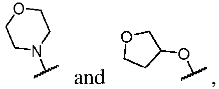 alternatively selected from Me, Et, cyclopropyl, 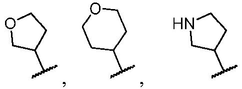 and 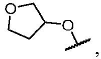 alternatively selected from Me, Et, cyclopropyl, 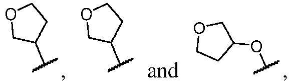 alternatively selected from Me, Et, cyclopropyl, 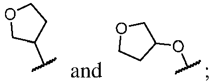
R'ₑ₇ is selected from Me, Et, cyclopropyl, tetrahydrofuranyl, tetrahydropyranyl, pyrrolidinyl and morpholinyl, alternatively selected from Me, Et, cyclopropyl, tetrahydrofuranyl and tetrahydropyranyl, alternatively selected from Me, Et, cyclopropyl and tetrahydrofuranyl, alternatively selected from Me, Et, cyclopropyl, 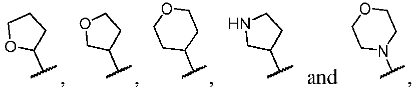 alternatively selected from Me, Et, cyclopropyl, 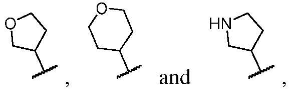 alternatively selected from Me, Et, cyclopropyl, 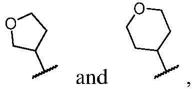 alternatively selected from Me, Et, cyclopropyl and 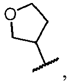 alternatively cyclopropyl;
Rₑ₈ is selected from H, Me and Et, alternatively H or Me, alternatively Me;
R'ₑ₈ is Me or Et, alternatively Me;
or, the substituents on X₁₀ and X₁₁ are taken together to form 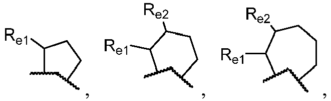 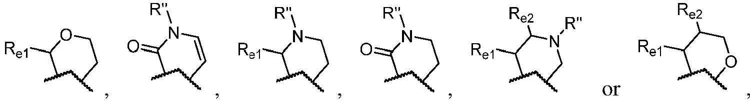 alternatively not form 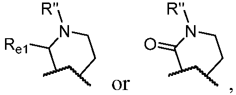 alternatively form a structure selected from: 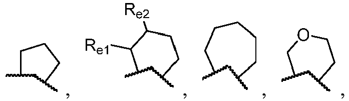 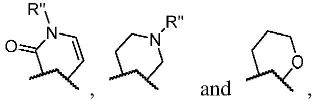 alternatively form 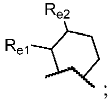
Rₑ₁ and Rₑ₂ are independently selected from H, F, Cl, CN, Me, Et, CHF₂, OCH₃, OEt, OCHF₂, -O-cyclopropyl and 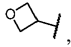 alternatively selected from H, F, CN, Me, OMe, OEt, OCHF₂ and -O-cyclopropyl, alternatively selected from H, F, CN, Me, OCH₃, OEt, OCHF₂ and -O-cyclopropyl, alternatively selected from H, Me, OCH₃ and OEt;
Rₑ₅ is H;
R'ₑ₅ is Me;
R" is H or methyl;
each of R₁ₐ, R_{1b}, Z₁, Z₂, Z₄, Z₅ and R_{d3} is as defined in any one of claims 39 to 45;
alternatively, the substituents on X₁₀ and X₁₁ are taken together to form a structure selected from: 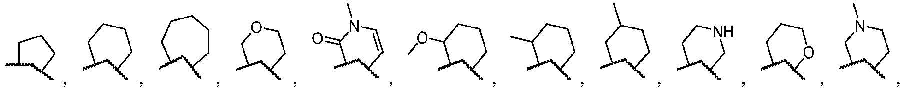 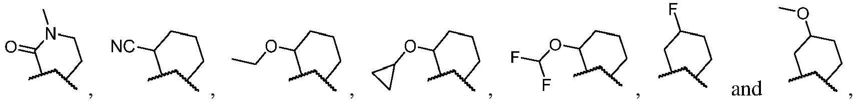 alternatively selected from: 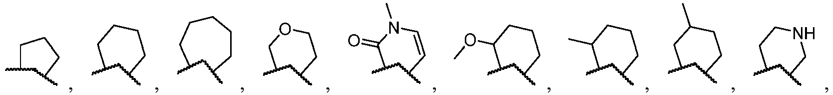 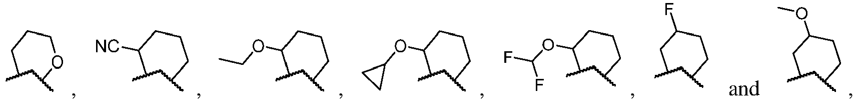 alternatively selected from: 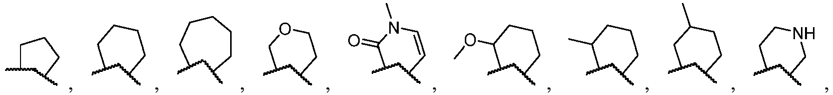 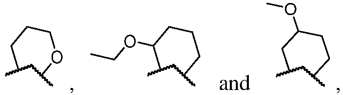 alternatively selected from: 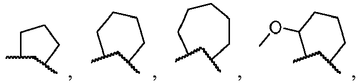 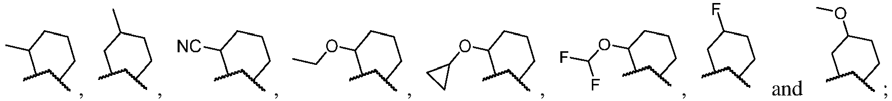
alternatively, 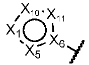 is selected from 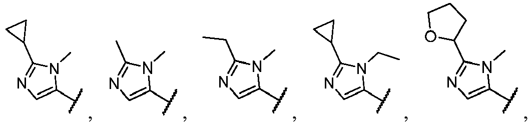 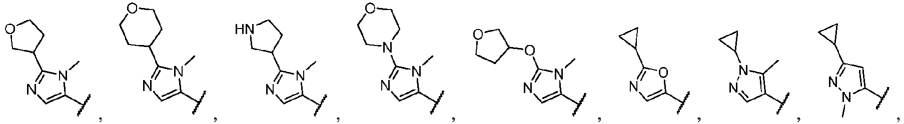 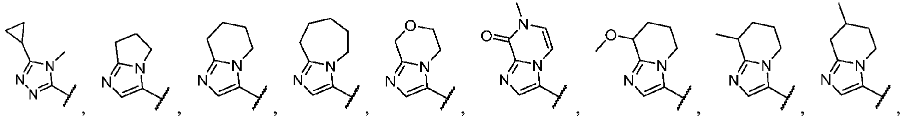 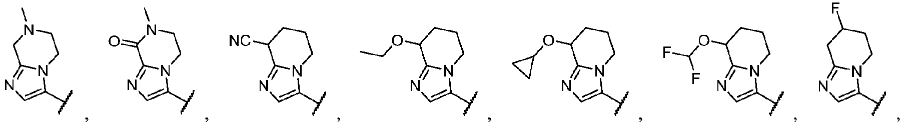 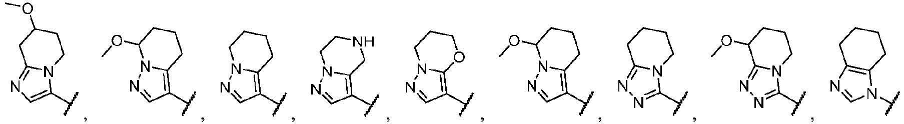 and 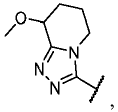 alternatively selected from: 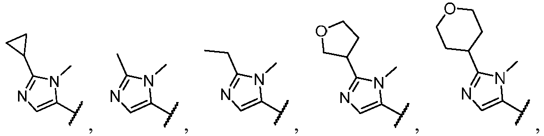 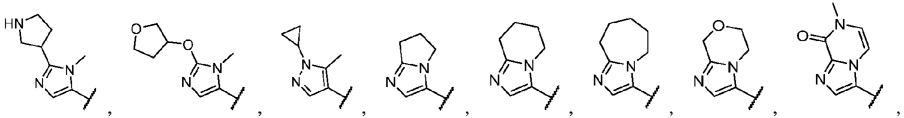 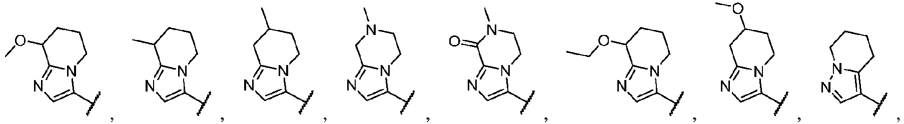 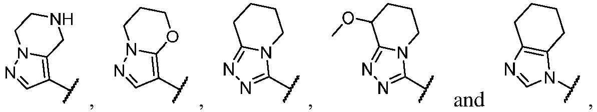 alternatively selected from: 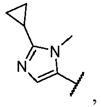 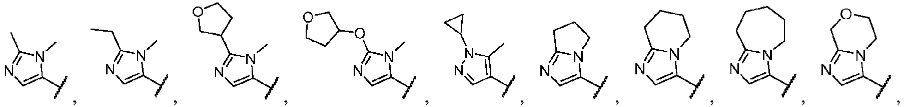 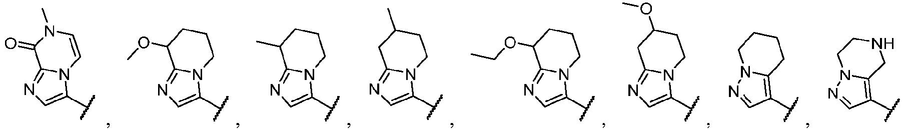 and 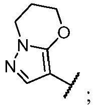
alternatively, R_{dd} is independently selected from H, Me and -C(CH₃)₂OH, or two non-adjacent R_{dd} are taken together to form -CH₂CH₂-, alternatively make 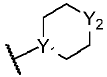 has the following structure: 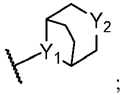
alternatively, R_{d3} is selected from: 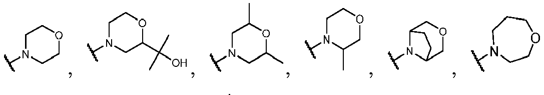 and 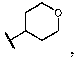 alternatively selected from: 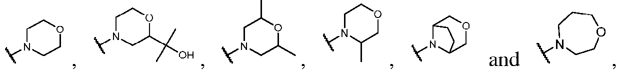 alternatively

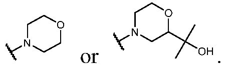

50. The compound of formula (VI-2) according to claim 23 or 46, or a stereoisomer, an N-oxide, a hydrate, a solvate, a metabolite, a pharmaceutically acceptable salt, a polymorph, or a prodrug thereof, 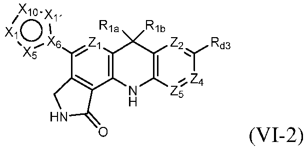 wherein
X₁₀ is CRₑ₇ or NR'ₑ₇;
X₁₁ is selected from CRₑ₈, O and NR'ₑ₈, alternatively CRₑ₈ or NR'ₑ₈;
Rₑ₇ is selected from C₁₋₆ alkyl, C₁₋₆ haloalkyl, 3- to 7-membered carbocyclyl, 3- to 7-membered heterocyclyl and -O-3- to 7-membered heterocyclyl; alternatively selected from C₁₋₆ alkyl, C₁₋₆ haloalkyl, 3- to 7-membered carbocyclyl, tetrahydrofuranyl, morpholinyl and -O-3- to 7-membered heterocyclyl; alternatively 3- to 7-membered carbocyclyl;
R'ₑ₇ is selected from C₁₋₆ alkyl, C₁₋₆ haloalkyl, 3- to 7-membered carbocyclyl and 3- to 7-membered heterocyclyl, alternatively 3- to 7-membered carbocyclyl;
Rₑ₈ is selected from H, D, C₁₋₆ alkyl and C₁₋₆ haloalkyl, alternatively C₁₋₆ alkyl or C₁₋₆ haloalkyl;
R'ₑ₈ is selected from C₁₋₆ alkyl and C₁₋₆ haloalkyl;
or, the substituents on X₁₀ and X₁₁ are taken together to form 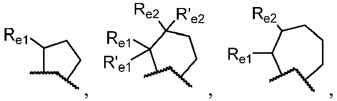 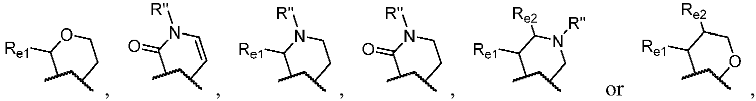 alternatively not form 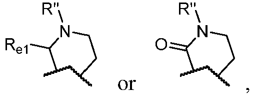 alternatively form a structure selected from: 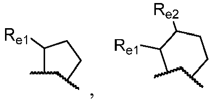 and 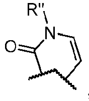 , alternatively form 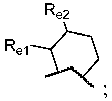
each of Rₑ₁ and Rₑ₂ is as defined in any one of claims 39 to 45, and each of R'ₑ₁ and R'ₑ₂ is as defined in any one of claims 23 to 26; alternatively, Rₑ₁ and Rₑ₂ are independently selected from H, D, halogen, CN, C₁₋₆ alkyl, C₁₋₆ haloalkyl, -OR', -O-3- to 7-membered carbocyclyl and -O-3- to 7-membered heterocyclyl, alternatively not halogen, alternatively selected from H, D, C₁₋₆ alkyl, C₁₋₆ haloalkyl, -OR' and -O-3- to 7-membered carbocyclyl; alternatively Rₑ₂ is selected from H, D, halogen, CN, C₁₋₆ alkyl, C₁₋₆ haloalkyl and -OR', alternatively Rₑ₂ is H, D or C₁₋₆ haloalkyl;
R'ₑ₁ and R'ₑ₂ are independently selected from H, D, halogen, C₁₋₆ alkyl and C₁₋₆ haloalkyl, alternatively selected from H, D, halogen and C₁₋₆ alkyl;
or CRₑ₁R'ₑ₁ forms a 3- to 7-membered carbocyclyl or a 3- to 7-membered heterocyclyl, alternatively forms a 3- to 7-membered heterocyclyl;
each of R' and R" is independently selected from H, C₁₋₆ alkyl and C₁₋₆ haloalkyl, alternatively R" is C₁₋₄ haloalkyl;
the remaining variables are as defined in any one of claims 46 to 49;
wherein each of the above groups is optionally deuterated, up to completely deuterated.

51. The compound of formula (VI-2) according to claim 50, or a stereoisomer, an N-oxide, a hydrate, a solvate, a metabolite, a pharmaceutically acceptable salt, a polymorph, or a prodrug thereof, wherein
X₁₀ is selected from CRₑ₇ and NR'ₑ₇;
X₁₁ is selected from CRₑ₈, O and NR'ₑ₈, alternatively CRₑ₈ or NR'ₑ₈;
Rₑ₇ is selected from Me, Et, cyclopropyl, 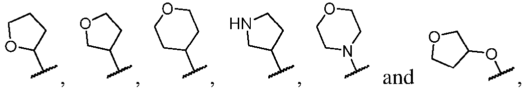 alternatively selected from Me, Et, cyclopropyl, 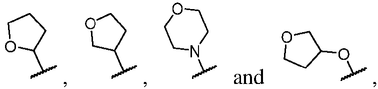 alternatively cyclopropyl;
R'ₑ₇ is selected from Me, Et and cyclopropyl, alternatively cyclopropyl;
Rₑ₈ is selected from H, Me and Et, alternatively H or Me, alternatively Me;
R'ₑ₈ is Me or Et, alternatively Me;
or, the substituents on X₁₀ and X₁₁ are taken together to form 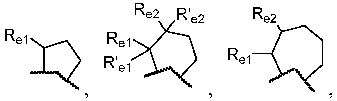 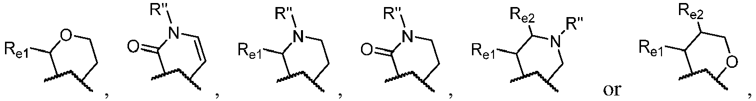 alternatively not form 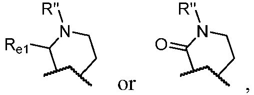 alternatively form a structure selected from: 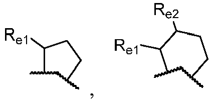 and 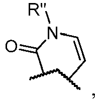 alternatively form 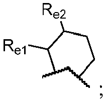
Rₑ₁ and Rₑ₂ are independently selected from H, F, CN, Me, CHF₂, OH, OMe, OEt, OCHF₂, -O-cyclopropyl and 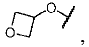 alternatively selected from H, CN, Me, CHF₂, OH, OCH₃, OEt, -O-cyclopropyl and 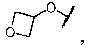 alternatively selected from H, Me, CHF₂, OCH₃, OEt and -O-cyclopropyl; alternatively Rₑ₂ is selected from H, F, CN, Me, CHF₂, OH and OMe, alternatively Rₑ₂ is H or CHF₂;
R'ₑ₁ and R'ₑ₂ are independently selected from H, F and Me;
or CRₑ₁R'ₑ₁ forms 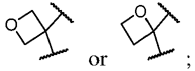
R" is selected from H, methyl and CHF₂, alternatively CHF₂;
the remaining variables are as defined in any one of claims 46 to 49;
alternatively,
the substituents on X₁₀ and X₁₁ are taken together to form a structure selected from: 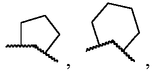 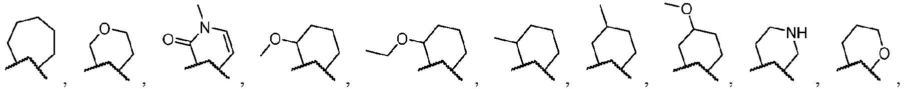 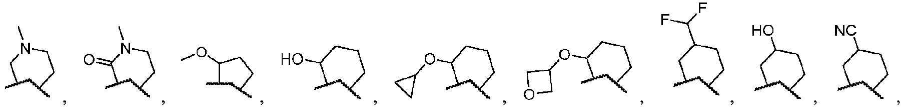 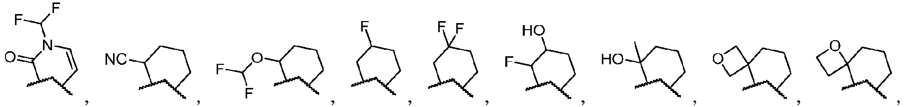 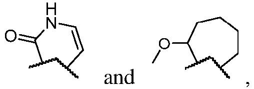 alternatively selected from: 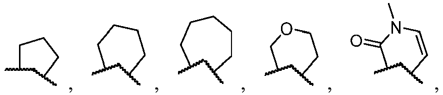 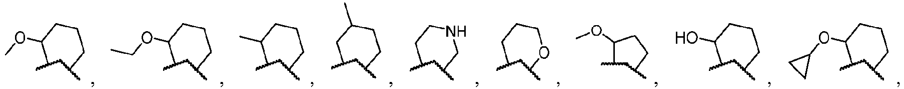 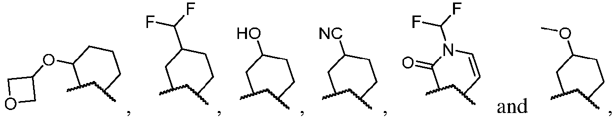 alternatively selected from: 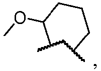 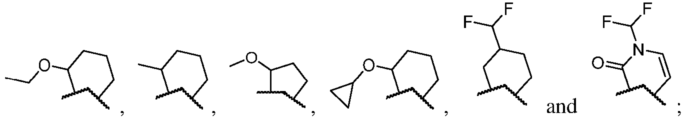
alternatively, 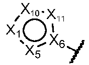 is selected from 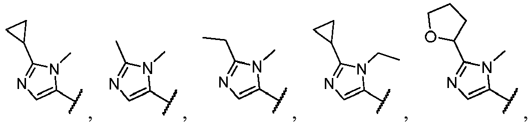 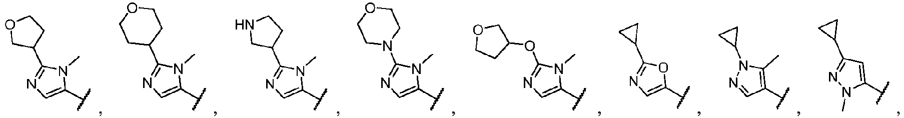 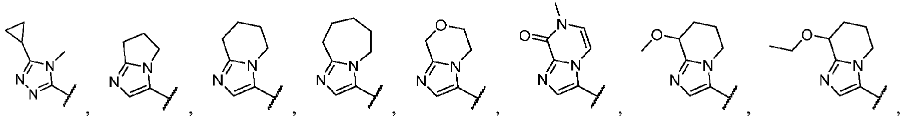 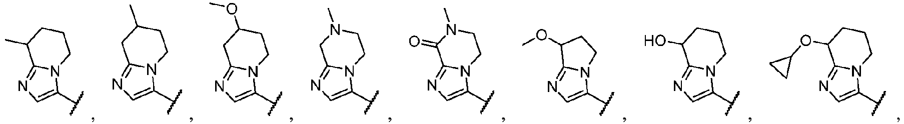 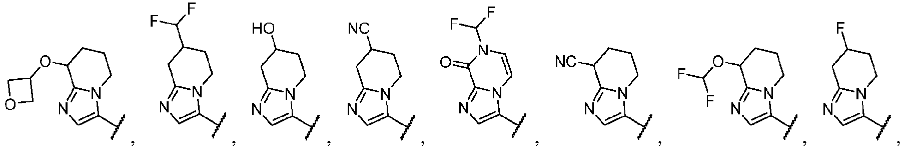 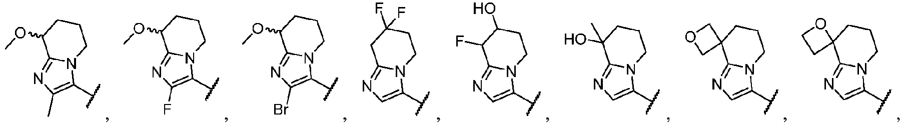 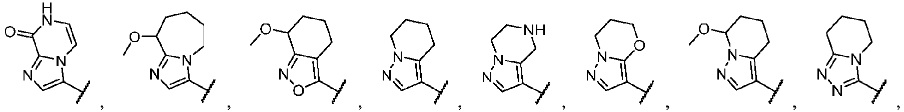 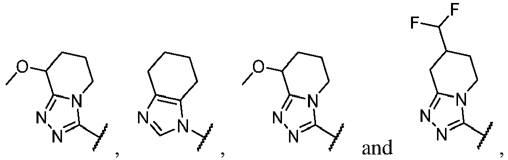 alternatively selected from: 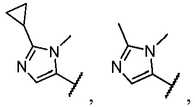 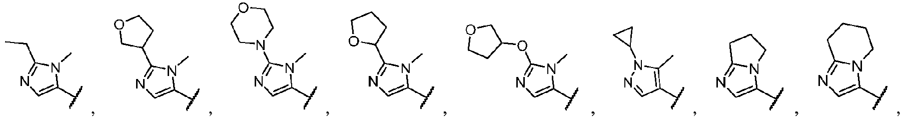 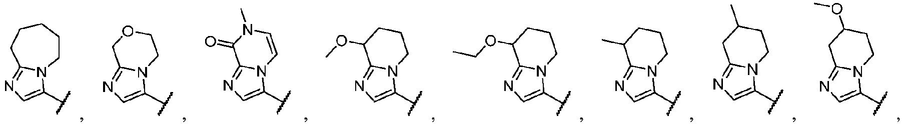 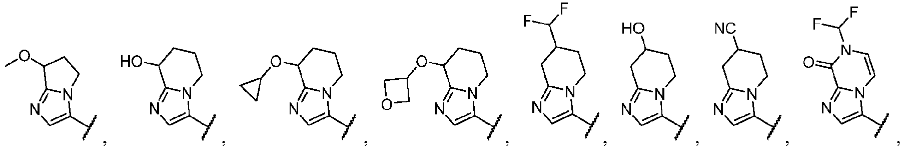 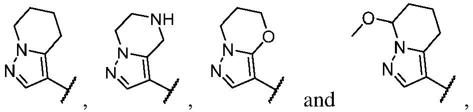 alternatively selected from: 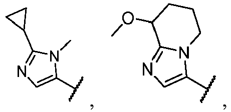 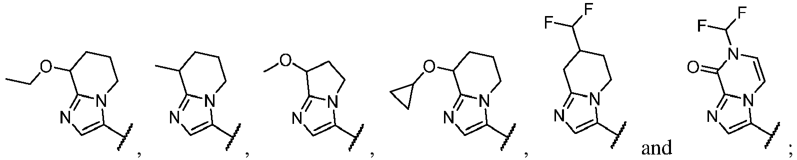
alternatively, Y₁ is CH or N, Y₂ is selected from CR_{dd}R_{dd} and O;
R_{dd} is independently selected from H, OH, Me, -CH₂OMe and -C(CH₃)₂OH, or two non-adjacent R_{dd} are taken together to form -CH₂CH₂-;
alternatively, R_{d3} is selected from: 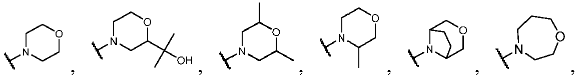 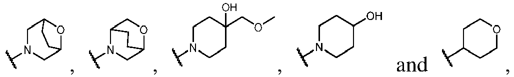 alternatively selected from: 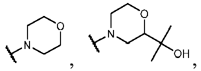 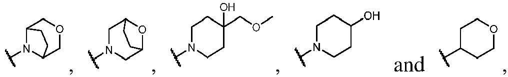
alternatively

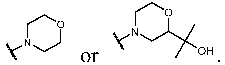

52. The compound of formula (VI-3) according to claim 23, or a stereoisomer, an N-oxide, a hydrate, a solvate, a metabolite, a pharmaceutically acceptable salt, a polymorph, or a prodrug thereof, 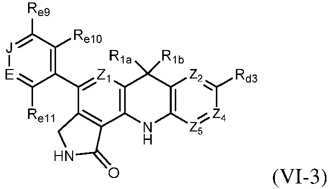 wherein
E is selected from CRₑ₁₂ and N;
J is selected from CRₑ₁₃ and N;
Rₑ₉, Rₑ₁₀, Rₑ₁₁, Rₑ₁₂ and Rₑ₁₃ are selected from H, D, halogen, CN, C₁₋₆ alkyl, C₁₋₆ haloalkyl, C₂₋₆ alkenyl, C₂₋₆ alkynyl, -L'-OR', -L'-SR', -L'-NR'R'', -L'-3- to 7-membered carbocyclyl, -L'-3- to 7-membered heterocyclyl and -Y₅-C₀₋₆ alkylene-R₆;
Y₅ is selected from O, S and NR₅;
R₅ is selected from H, C₁₋₆ alkyl and C₁₋₆ haloalkyl;
R₆ is selected from 3- to 7-membered carbocyclyl and 3- to 7-membered heterocyclyl;
or Rₑ₉ and Rₑ₁₃ are taken together with the carbon atoms to which they are attached to form a 5- to 7-membered carbocyclyl, a 5- to 7-membered heterocyclyl, a phenyl or a 5- to 6-membered heteroaryl, which is optionally substituted with 1, 2, 3 or 4 Rₑ;
or Rₑ₉ and Rₑ₁₀ are taken together with the carbon atoms to which they are attached to form a 5- to 7-membered carbocyclyl, a 5- to 7-membered heterocyclyl, a phenyl or a 5- to 6-membered heteroaryl, which is optionally substituted with 1, 2, 3 or 4 Rₑ;
Rₑ is as defined in any one of claims 36 to 38, alternatively Rₑ is not alkenyl or alkynyl;
L' is independently selected from covalent bond and C₁₋₆ alkylene, each of which is optionally substituted with 1, 2, 3 or 4 groups selected from: C₁₋₆ alkyl, C₁₋₆ haloalkyl, -L_{d}-3- to 7-membered carbocyclyl and -L_{d}-3- to 7-membered heterocyclyl;
L_{d} is independently selected from covalent bond and C₁₋₆ alkylene, alternatively selected from covalent bond and C₁₋₄ alkylene;
each of R' and R" is independently selected from H, C₁₋₆ alkyl, C₁₋₆ haloalkyl, -L_{d}-3- to 7-membered carbocyclyl and -L_{d}-3- to 7-membered heterocyclyl;
each of R₁ₐ, R_{1b}, Z₁, Z₂, Z₄, Z₅ and R_{d3} is as defined in any one of claims 39 to 45;
wherein each of the above groups is optionally deuterated, up to completely deuterated.

53. The compound of formula (VI-3) according to claim 52, or a stereoisomer, an N-oxide, a hydrate, a solvate, a metabolite, a pharmaceutically acceptable salt, a polymorph, or a prodrug thereof, wherein
E is selected from CRₑ₁₂ and N;
J is selected from CRₑ₁₃ and N;
Rₑ₉ is selected from H, D, halogen, C₁₋₆ alkyl, C₁₋₆ haloalkyl, C₂₋₆ alkenyl, C₂₋₆ alkynyl, -OR', -NR'R", 3- to 7-membered carbocyclyl, 3- to 7-membered heterocyclyl and -Y₅-C₀₋₆ alkylene-R₆, alternatively selected from H, D, halogen, C₁₋₆ alkyl, C₁₋₆ haloalkyl, C₂₋₆ alkenyl, C₂₋₆ alkynyl, -OR' and -NR'R";
Y₅ is selected from O and NR₅;
R₅ is selected from H, C₁₋₆ alkyl and C₁₋₆ haloalkyl;
R₆ is selected from 3- to 7-membered carbocyclyl and 3- to 7-membered heterocyclyl;
Rₑ₁₀ is selected from H, D, halogen, C₁₋₆ alkyl and C₁₋₆ haloalkyl;
Rₑ₁₁, Rₑ₁₂ and Rₑ₁₃ are H, D, C₁₋₆ alkyl or C₁₋₆ haloalkyl;
or Rₑ₉ and Rₑ₁₃ are taken together with the carbon atoms to which they are attached to form a 5- to 7-membered carbocyclyl or a 5- to 7-membered heterocyclyl, which is optionally substituted with 1, 2, 3 or 4 Rₑ;
or Rₑ₉ and Rₑ₁₀ are taken together with the carbon atoms to which they are attached to form a 5- to 7-membered carbocyclyl, a 5- to 7-membered heterocyclyl, a phenyl or a 5- to 6-membered heteroaryl, which is optionally substituted with 1, 2, 3 or 4 Rₑ;
Rₑ is as defined in any one of claims 36 to 38, alternatively Rₑ is not alkenyl or alkynyl;
each of R' and R" is independently selected from H, C₁₋₆ alkyl, C₁₋₆ haloalkyl, 3- to 7-membered carbocyclyl and 3- to 7-membered heterocyclyl;
each of R₁ₐ, R_{1b}, Z₁, Z₂, Z₄, Z₅ and R_{d3} is as defined in any one of claims 39 to 45;
alternatively, at least one of E and J is N; alternatively, only one of E and J is N.

54. The compound of formula (VI-3) according to claim 52 or 53, or a stereoisomer, an N-oxide, a hydrate, a solvate, a metabolite, a pharmaceutically acceptable salt, a polymorph, or a prodrug thereof, wherein
E is selected from CRₑ₁₂ and N;
J is selected from CRₑ₁₃ and N;
Rₑ₉ is selected from H, D, C₁₋₆ alkyl, C₁₋₆ haloalkyl, C₂₋₄ alkynyl, -NR'R", 3- to 7-membered heterocyclyl and -O-C₀₋₄ alkylene-3- to 7-membered heterocyclyl, alternatively selected from H, D, C₁₋₆ alkyl, C₁₋₆ haloalkyl, C₂₋₄ alkynyl and -NR'R";
Rₑ₁₀ is selected from H, D, C₁₋₆ alkyl and C₁₋₆ haloalkyl;
Rₑ₁₁, Rₑ₁₂ and Rₑ₁₃ are H or D;
or Rₑ₉ and Rₑ₁₃ are taken together with the carbon atoms to which they are attached to form a 5- to 7-membered heterocyclyl, alternatively form dihydrooxazinyl, which is optionally substituted with 1, 2, 3 or 4 Rₑ;
or Rₑ₉ and Rₑ₁₀ are taken together with the carbon atoms to which they are attached to form a 5- to 7-membered carbocyclyl, a 5- to 7-membered heterocyclyl, or a 5- to 6-membered heteroaryl, alternatively form a 5- to 7-membered heterocyclyl or a 5- to 6-membered heteroaryl, alternatively form dihydrooxazinyl or 5-membered azaaryl, which is optionally substituted with 1, 2, 3 or 4 Rₑ;
Rₑ is as defined in any one of claims 36 to 38, alternatively Rₑ is not alkenyl or alkynyl; alternatively Rₑ is independently selected from H, D, halogen, C₁₋₆ alkyl, C₁₋₆ haloalkyl, -OR' and -NR'R", alternatively selected from H, D, C₁₋₆ alkyl, C₁₋₆ haloalkyl and -OR', alternatively selected from Me and OMe;
each of R' and R" is independently selected from H, C₁₋₆ alkyl and C₁₋₆ haloalkyl;
each of R₁ₐ, R_{1b}, Z₁, Z₂, Z₄, Z₅ and R_{d3} is as defined in any one of claims 39 to 45.
alternatively, Rₑ is independently selected from H, D, C₁₋₆ alkyl and C₁₋₆ haloalkyl, alternatively H or Me.

55. The compound of formula (VI-3) according to any one of claims 52 to 54, or a stereoisomer, an N-oxide, a hydrate, a solvate, a metabolite, a pharmaceutically acceptable salt, a polymorph, or a prodrug thereof, wherein
E is selected from CRₑ₁₂ and N;
J is selected from CRₑ₁₃ and N;
Rₑ₉ is selected from H, -NHMe, 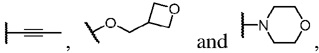 alternatively selected from H, -NHMe and 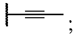
Rₑ₁₀ is selected from H and Me;
Rₑ₁₁, Rₑ₁₂ and Rₑ₁₃ are H;
or Rₑ₉ and Rₑ₁₃ are taken together with the carbon atoms to which they are attached to form 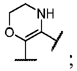
or Rₑ₉ and Rₑ₁₀ are taken together with the carbon atoms to which they are attached to form 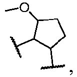 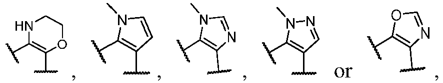 alternatively form 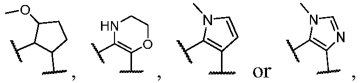 alternatively form 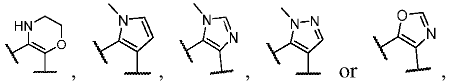 alternatively form 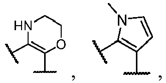 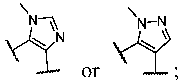
each of R₁ₐ, R_{1b}, Z₁, Z₂, Z₄, Z₅ and R_{d3} is as defined in any one of claims 39 to 45;
alternatively, 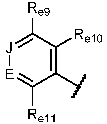 is selected from: 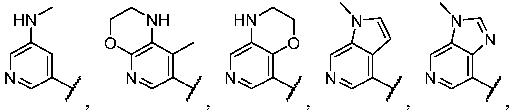 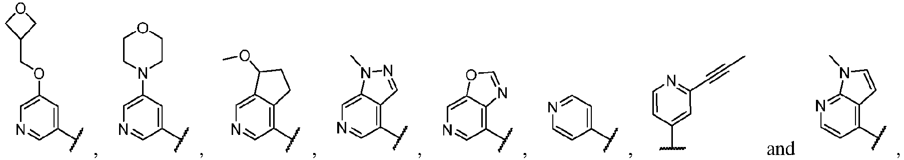 alternatively selected from: 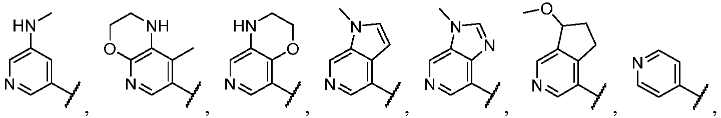 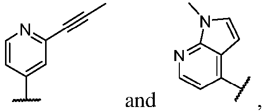 alternatively selected from: 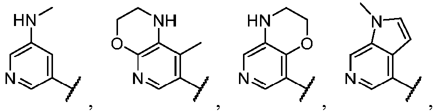 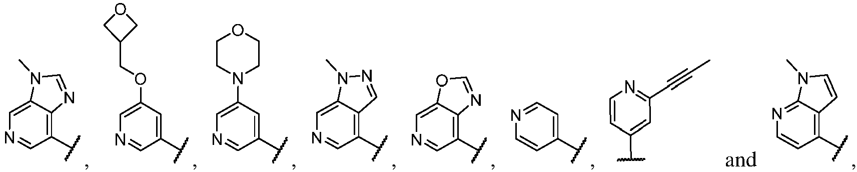 alternatively selected from: 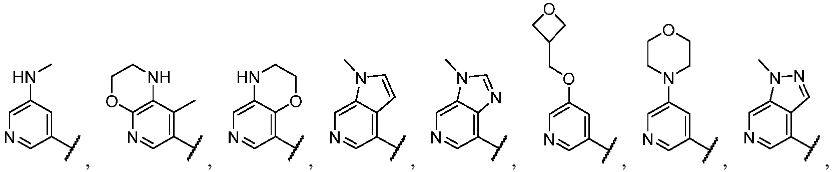 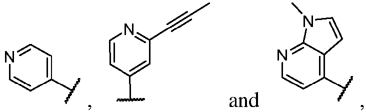 alternatively selected from: 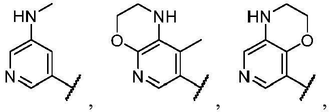 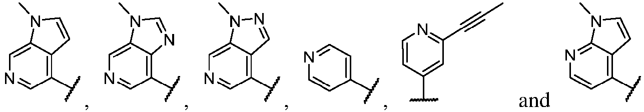 , alternatively

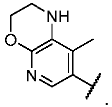

56. The compound of formula (I) according to claim 1, or a stereoisomer, an N-oxide, a hydrate, a solvate, a metabolite, a pharmaceutically acceptable salt, a polymorph, or a prodrug thereof, wherein the compound is selected from:

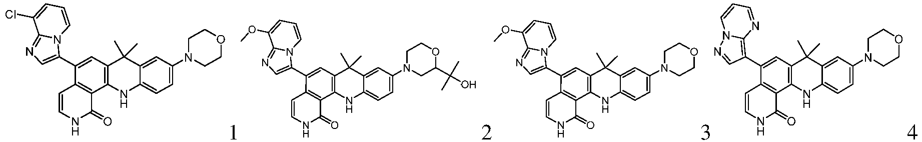

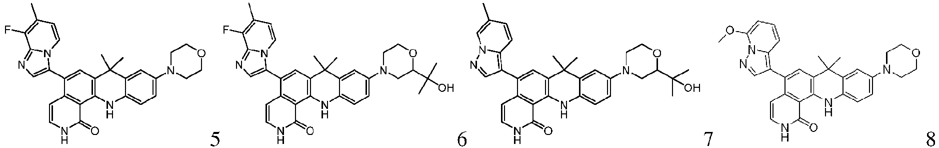

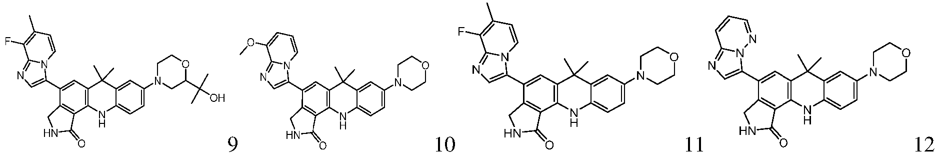

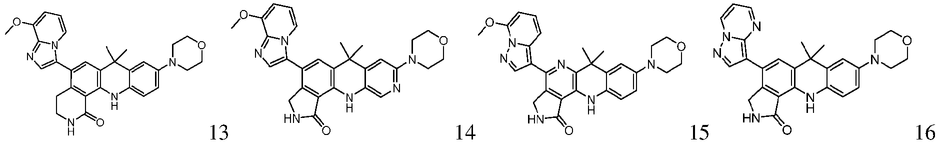

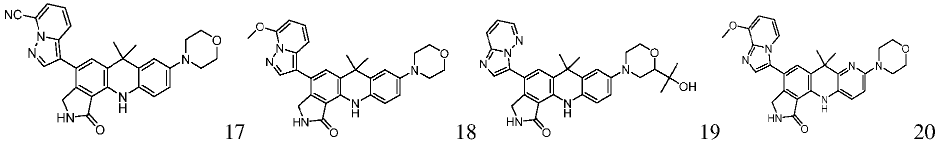

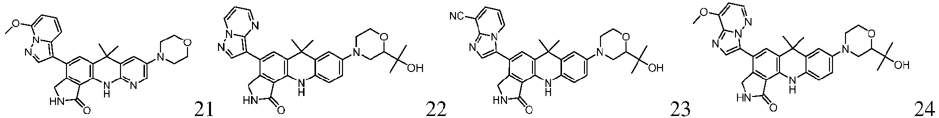

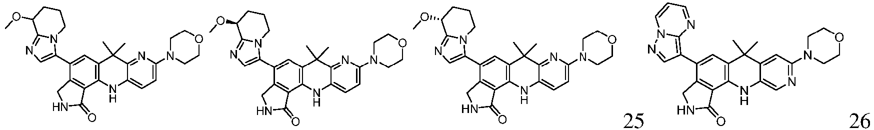

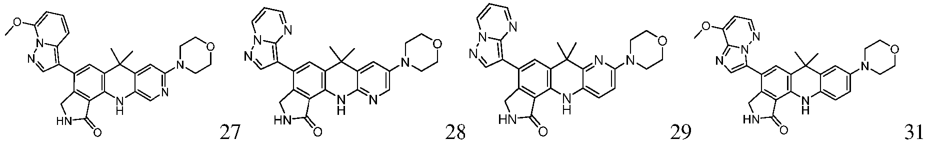

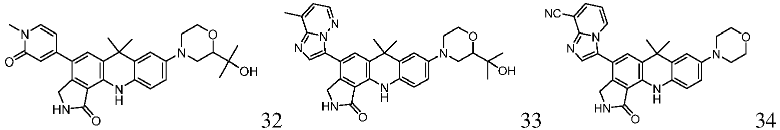

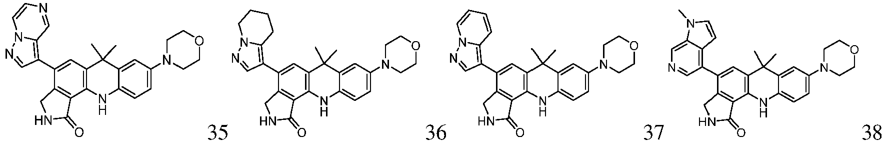

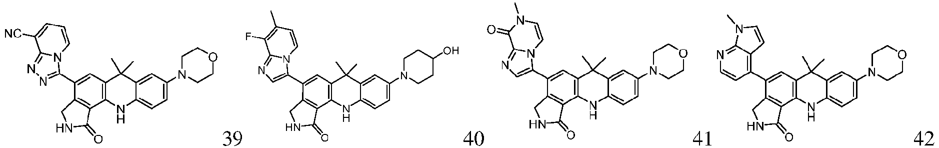

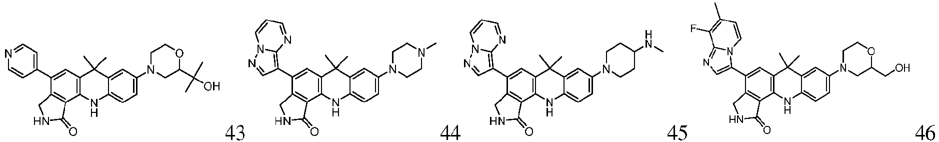

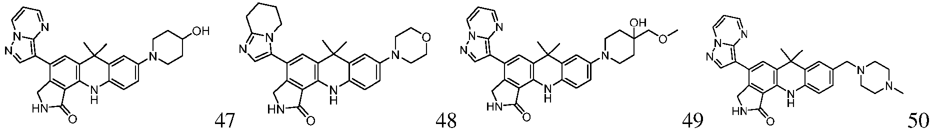

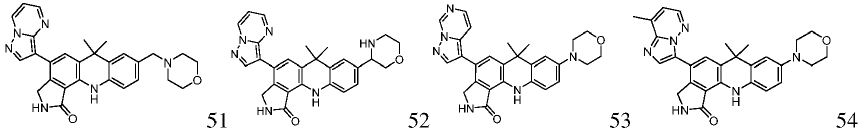

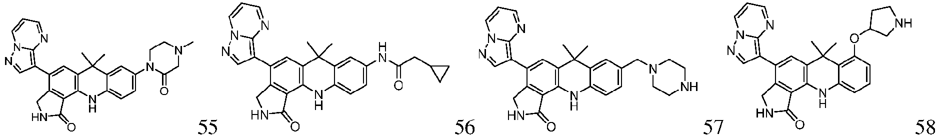

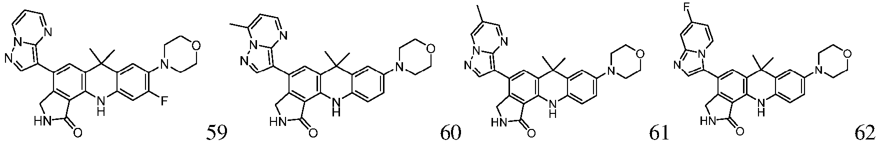

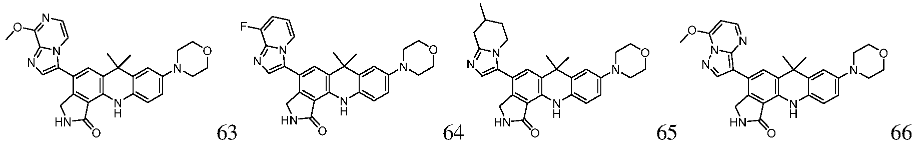

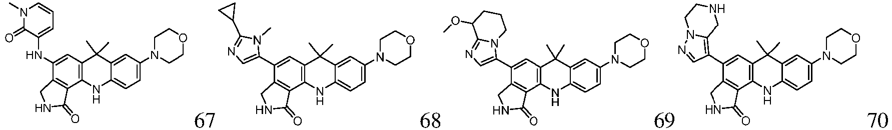

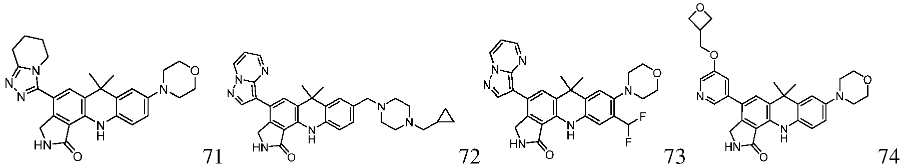

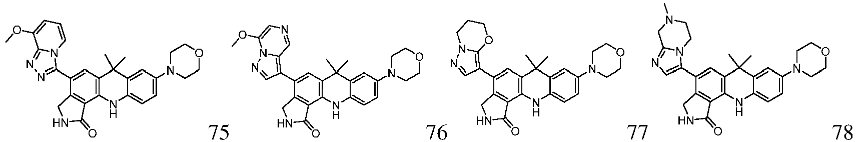

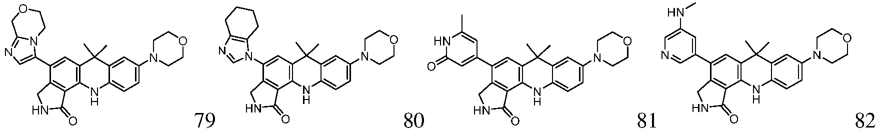

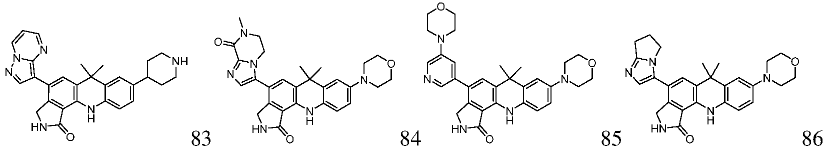

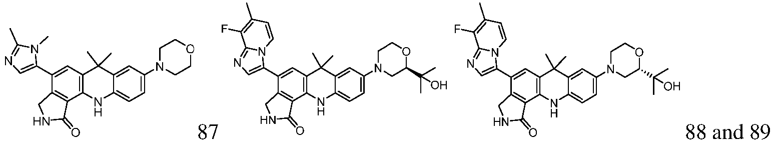

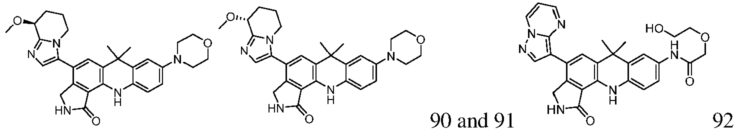

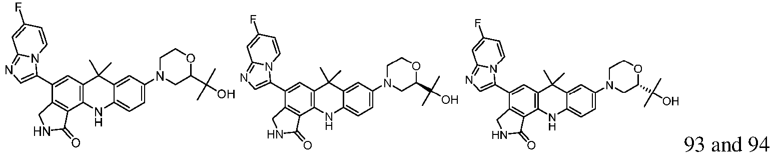

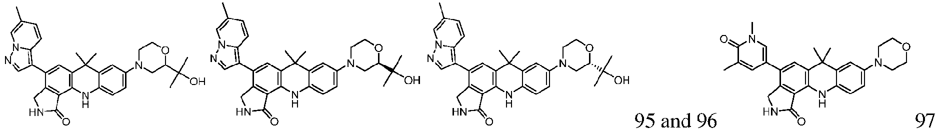

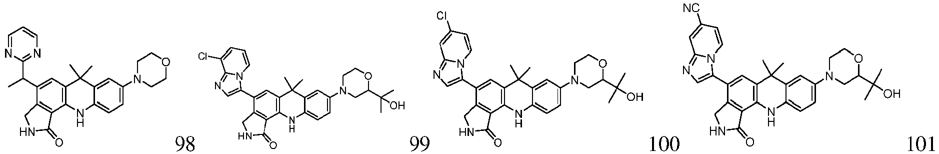

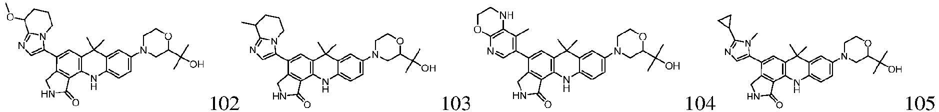

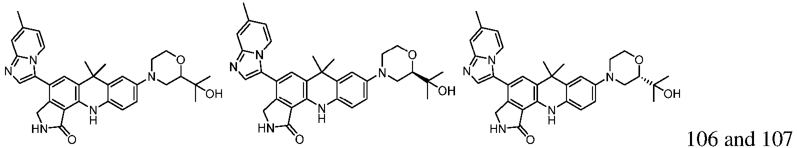

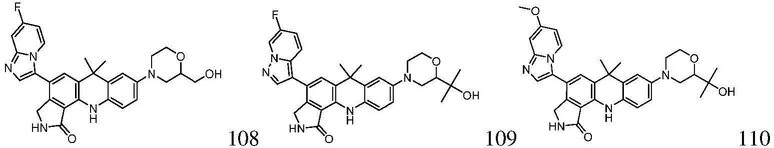

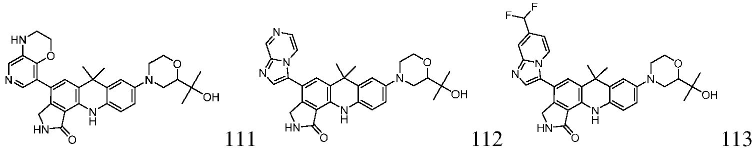

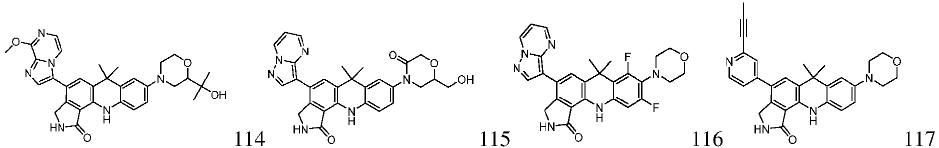

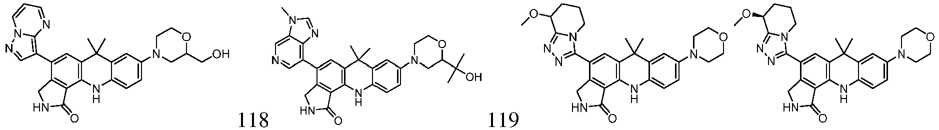

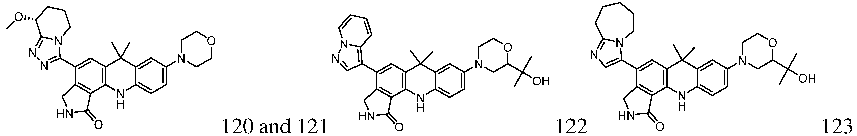

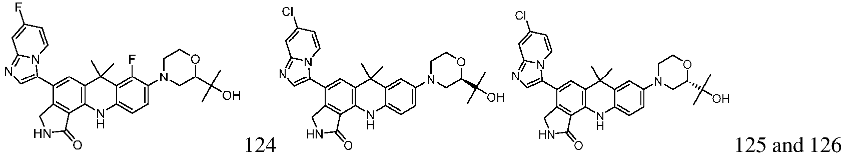

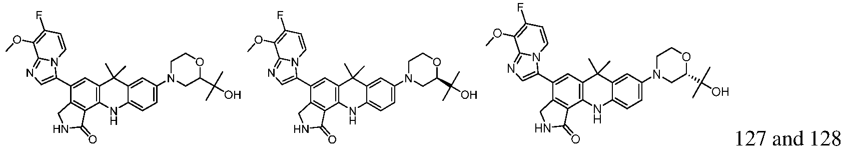

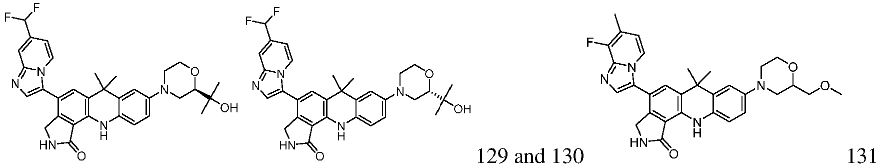

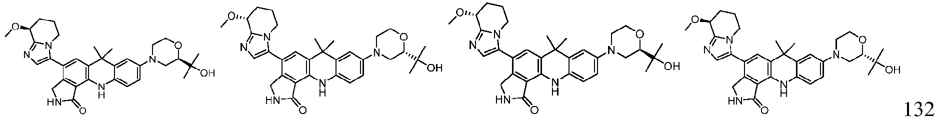

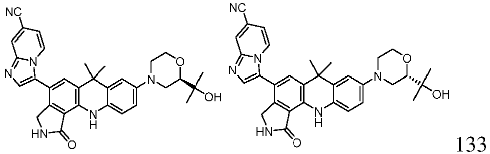

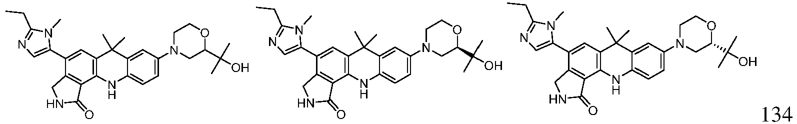

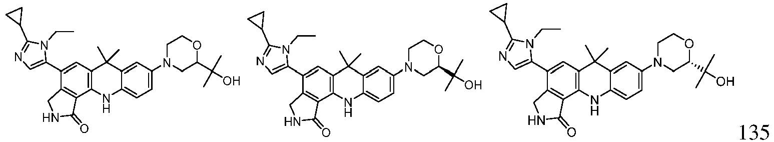

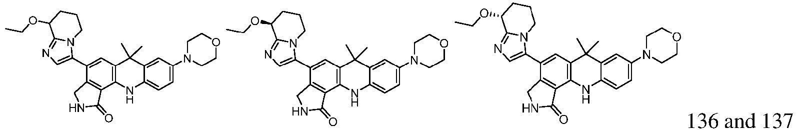

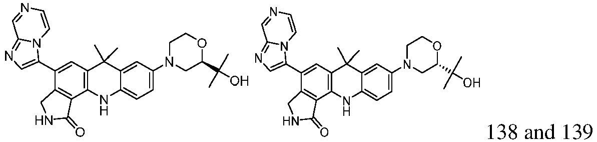

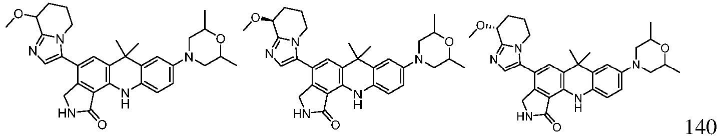

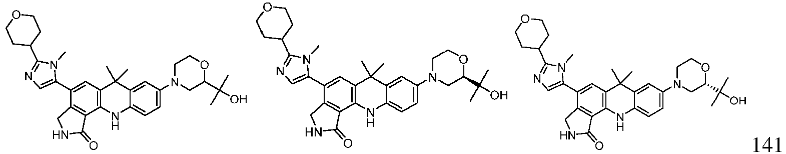

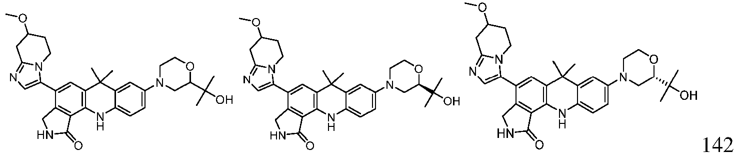

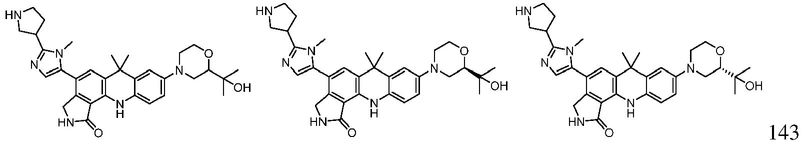

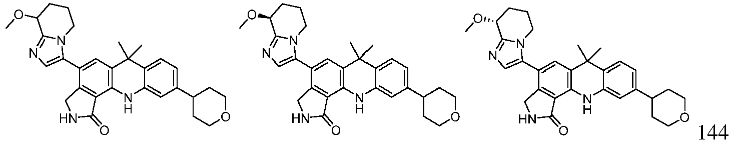

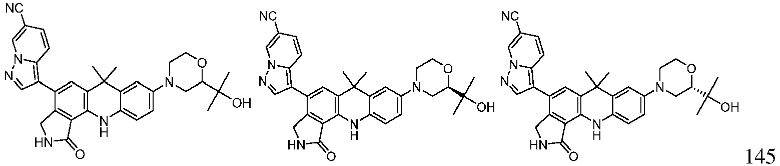

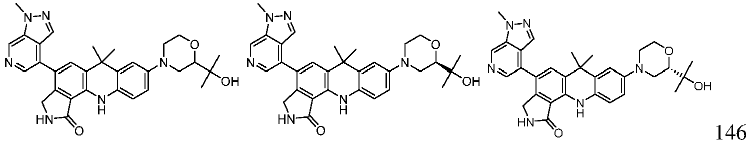

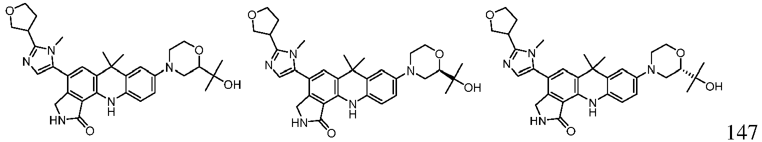

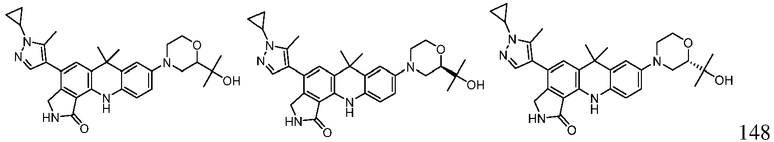

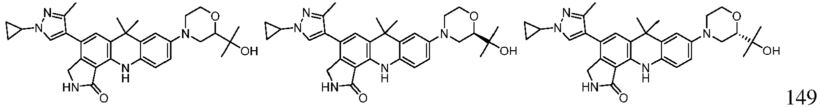

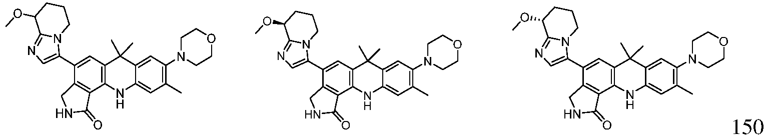

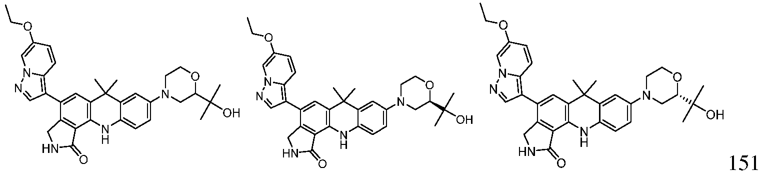

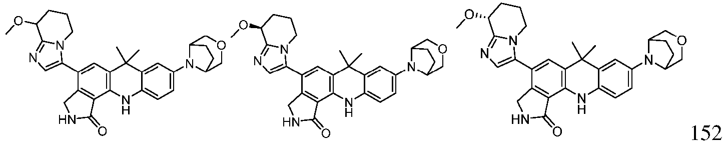

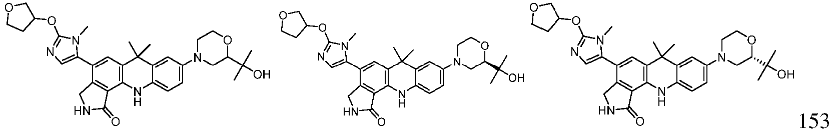

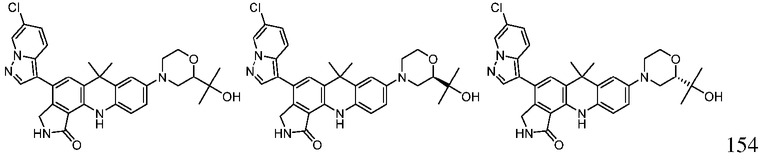

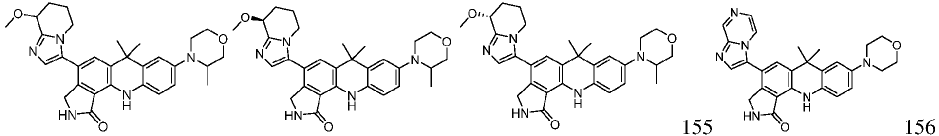

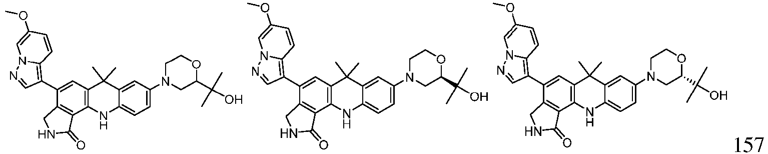

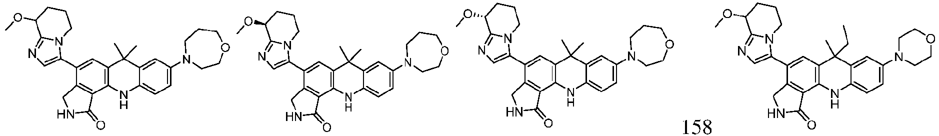

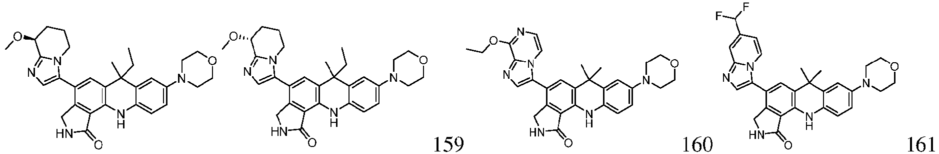

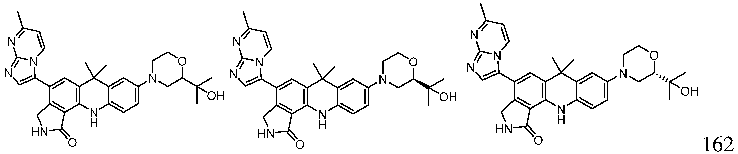

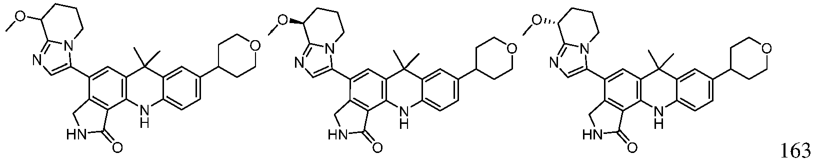

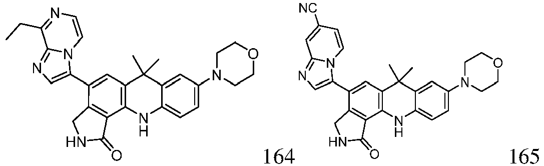

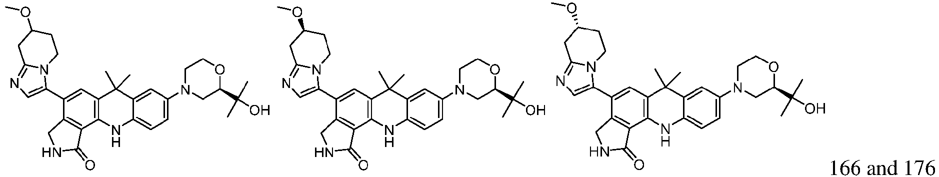

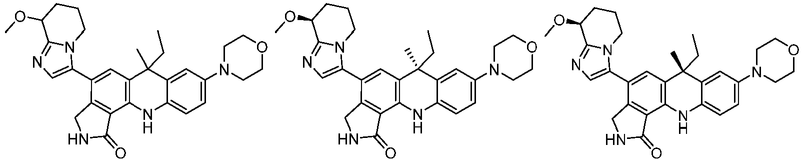

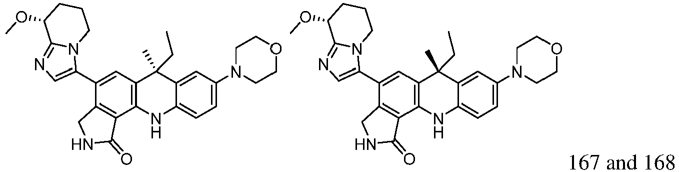

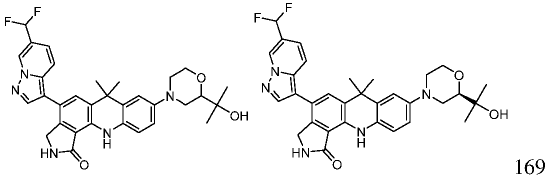

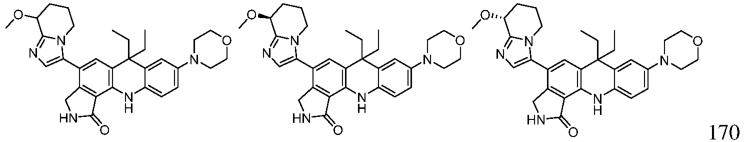

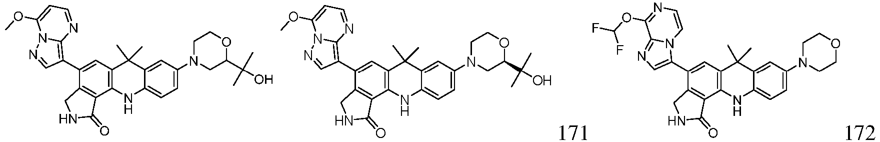

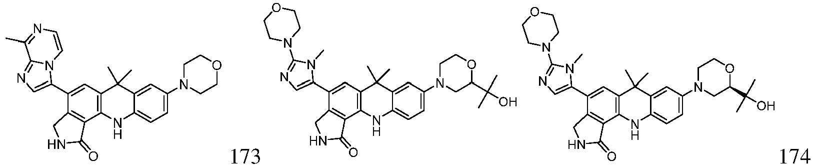

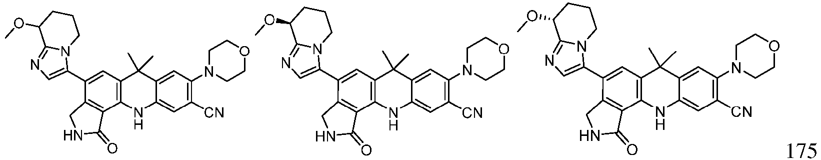

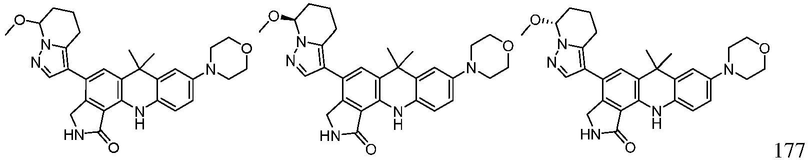

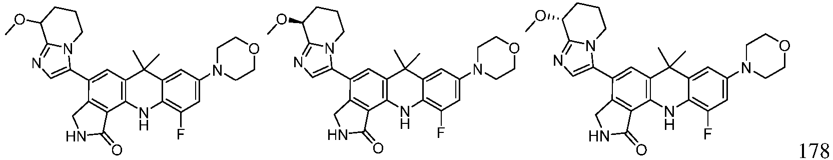

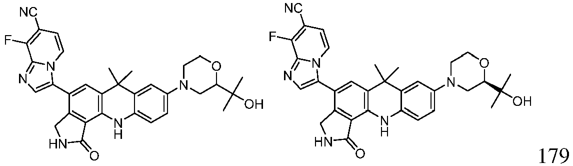

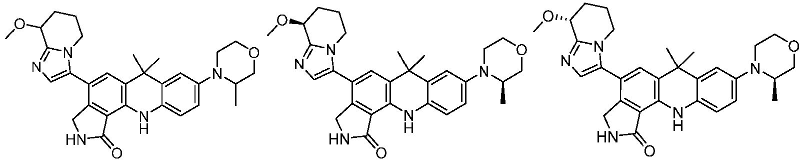

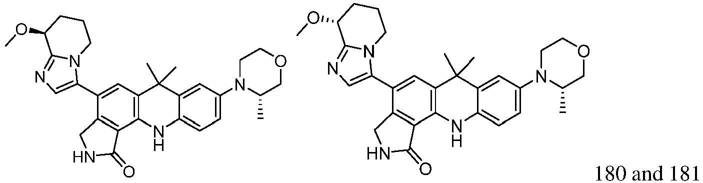

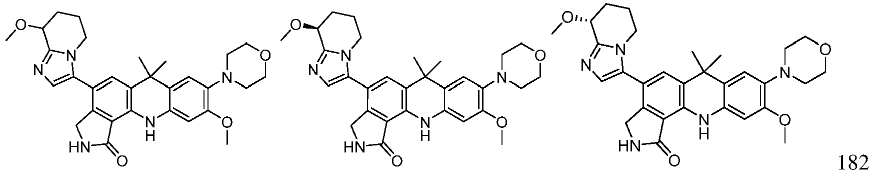

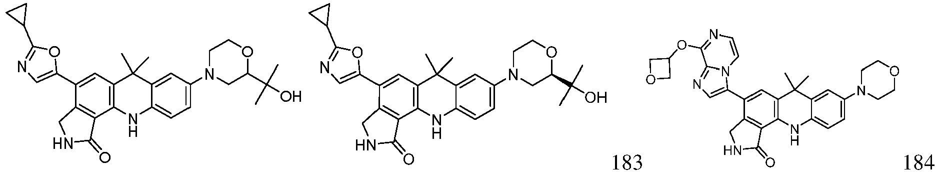

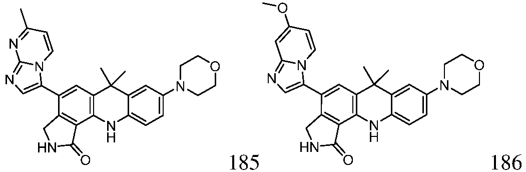

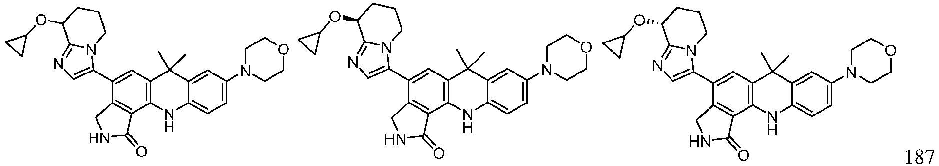

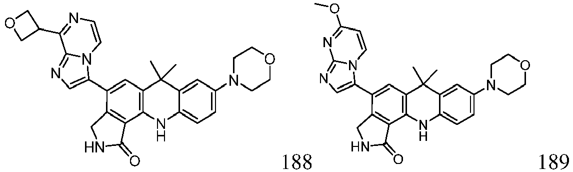

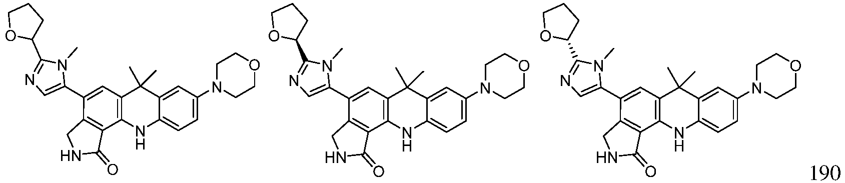

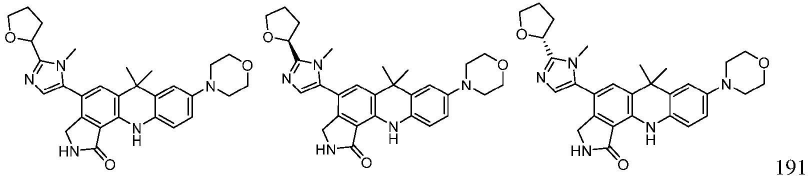

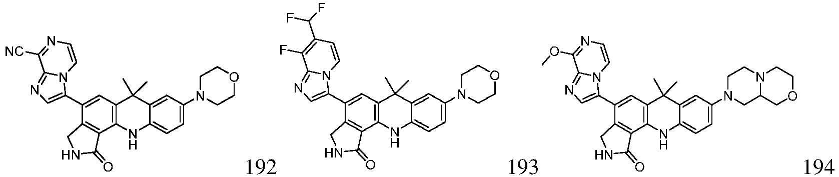

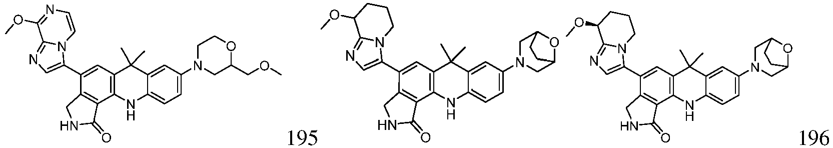

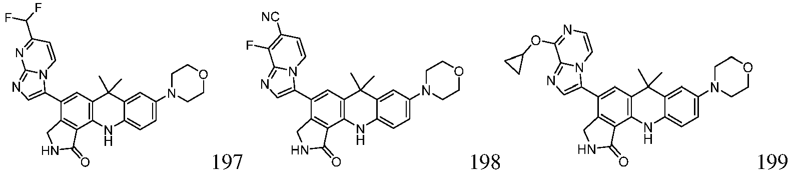

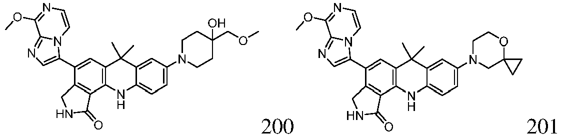

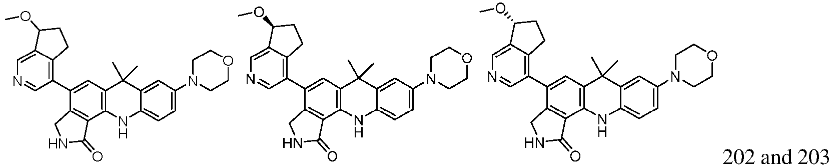

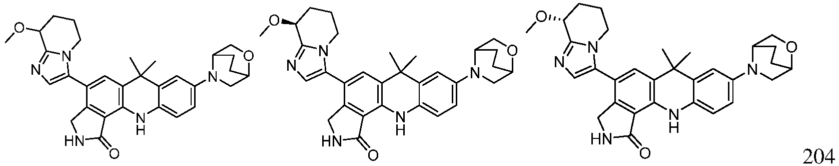

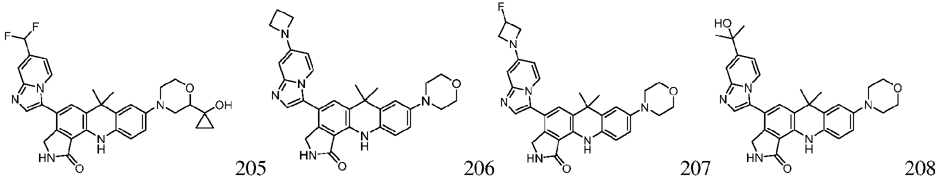

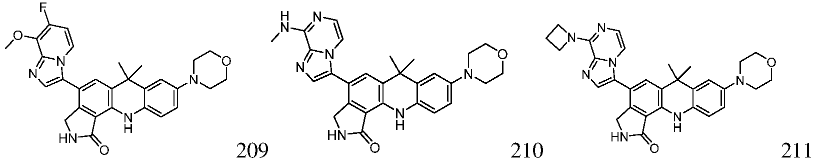

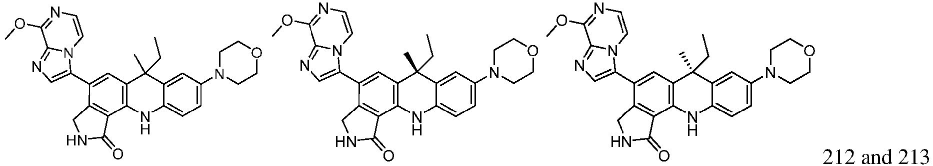

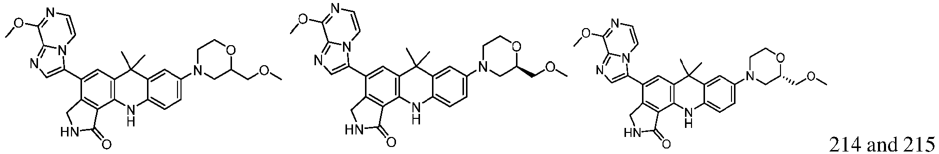

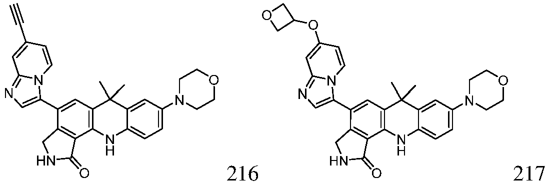

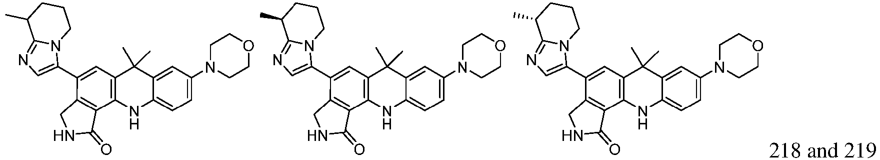

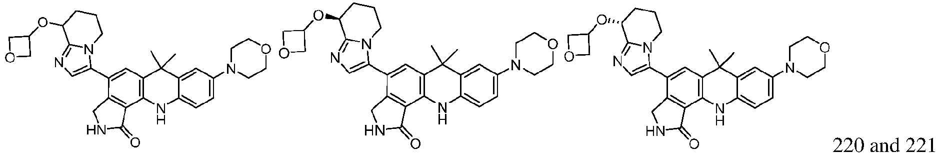

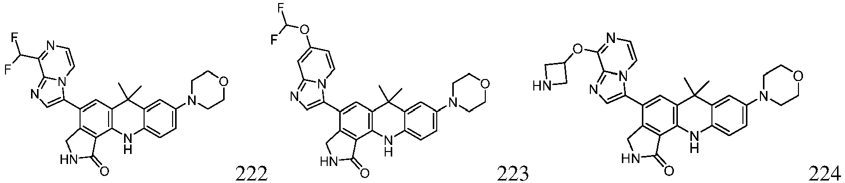

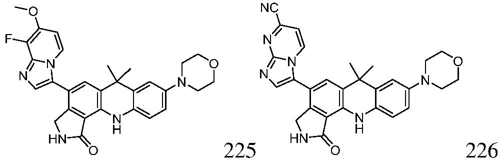

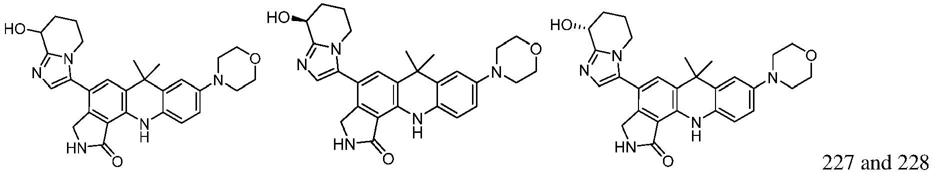

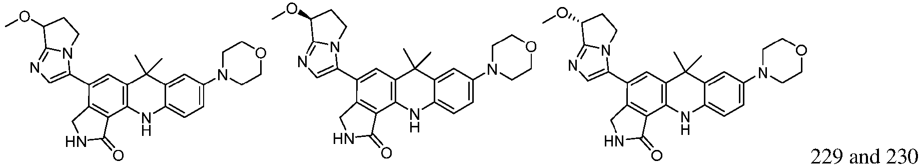

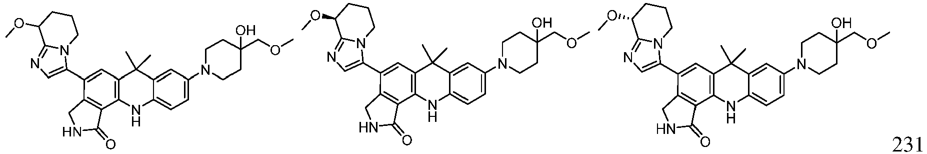

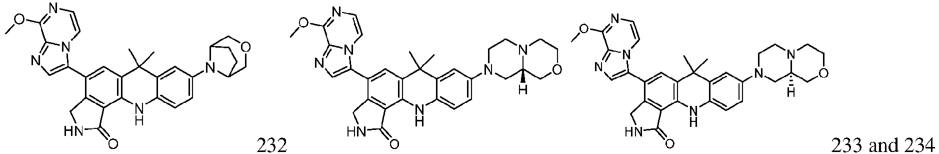

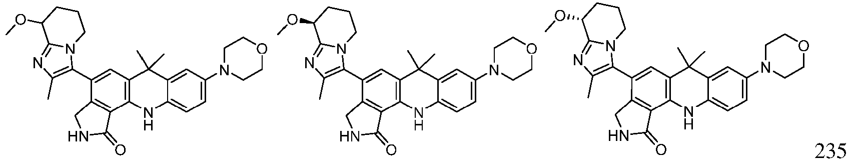

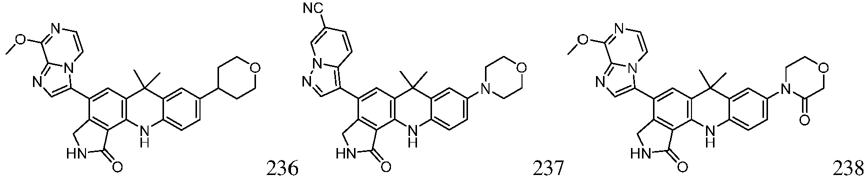

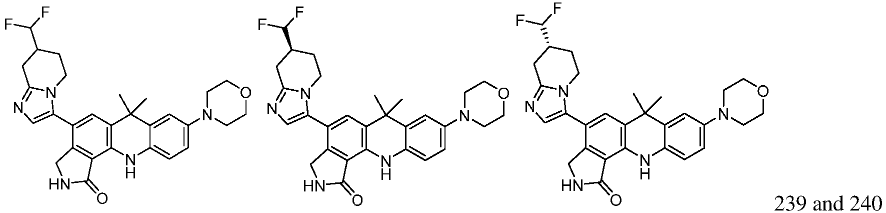

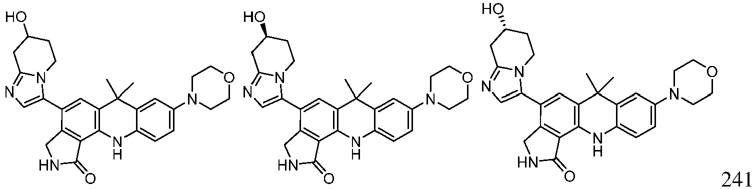

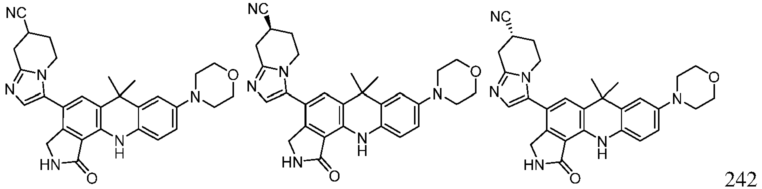

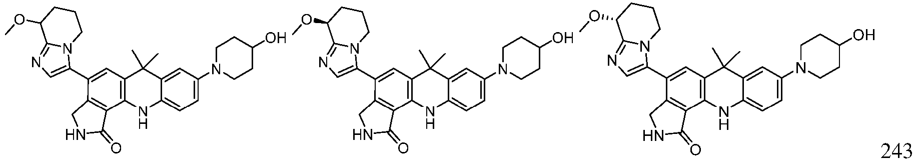

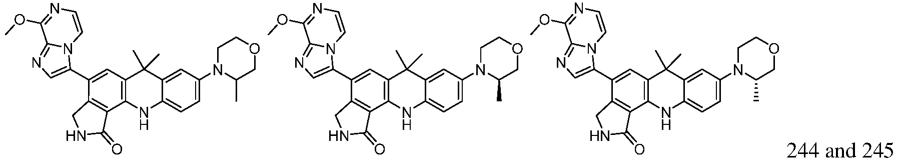

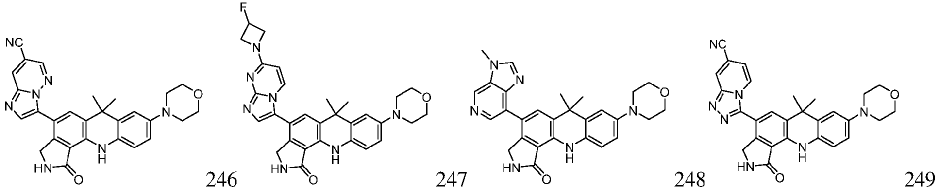

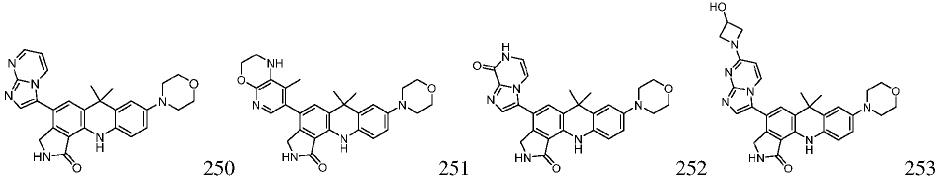

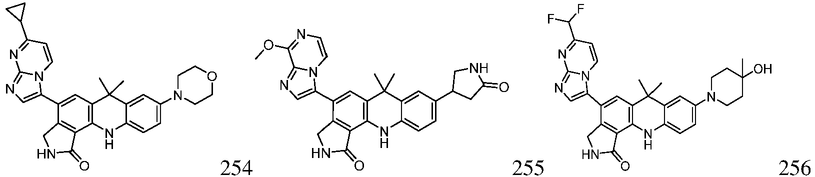

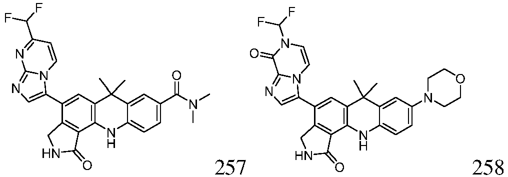

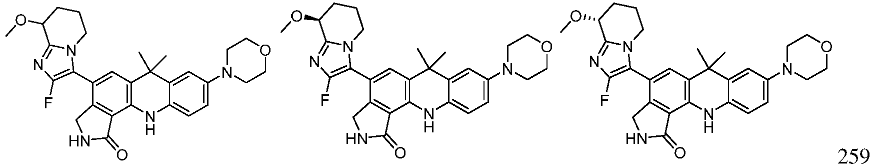

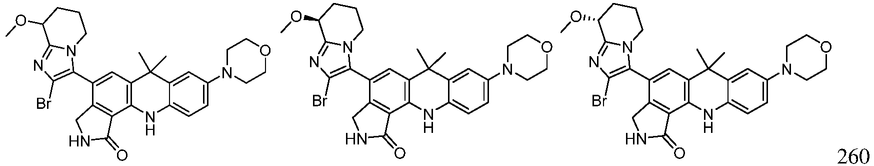

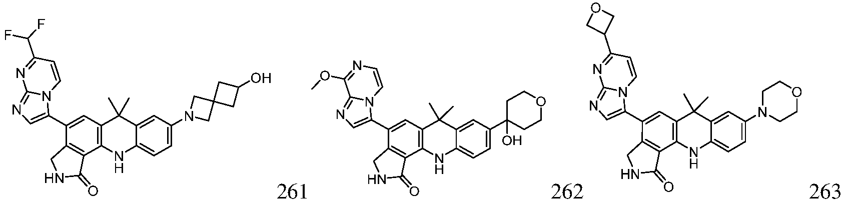

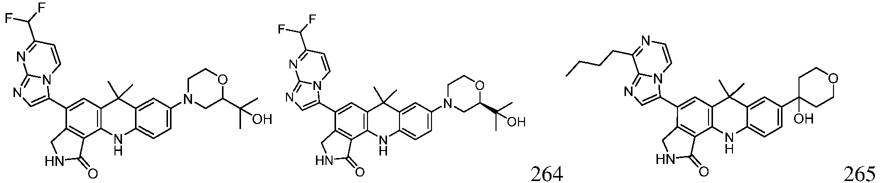

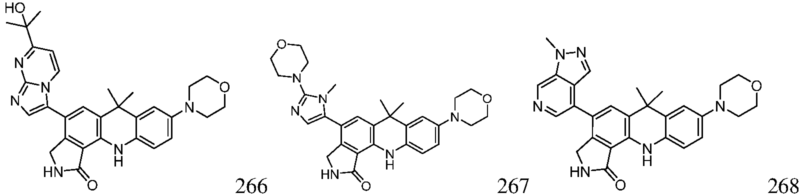

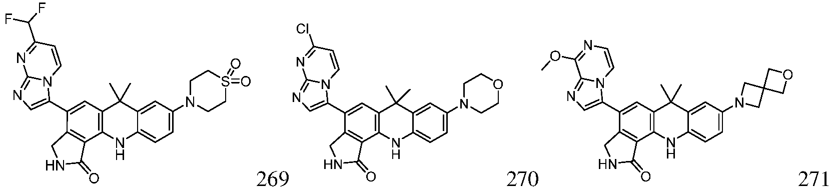

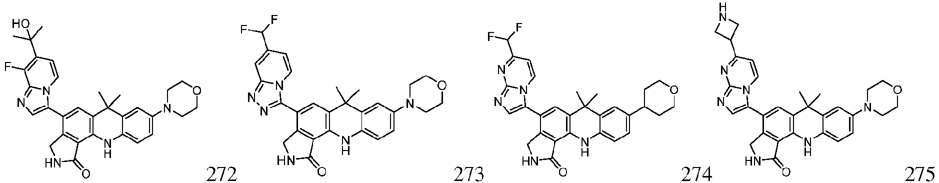

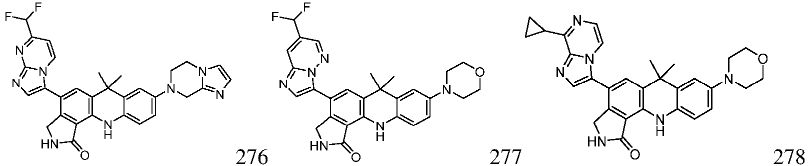

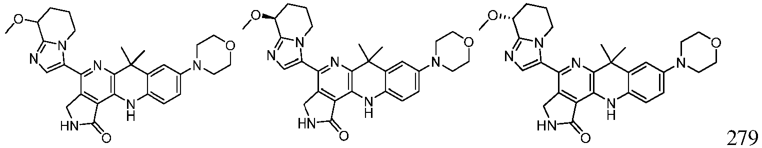

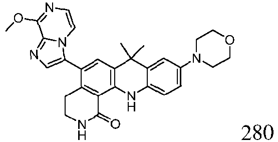

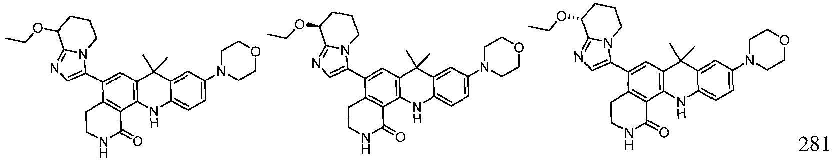

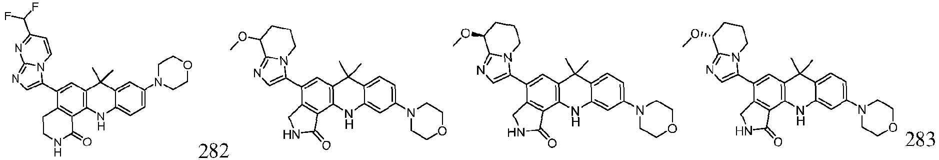

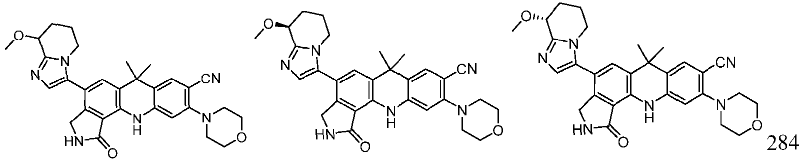

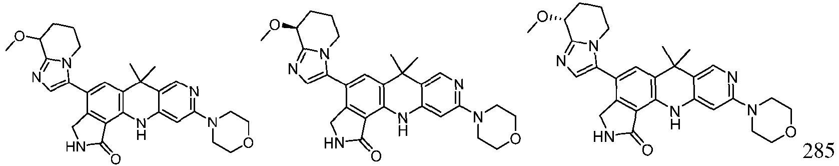

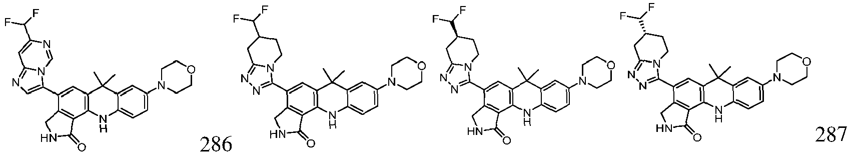

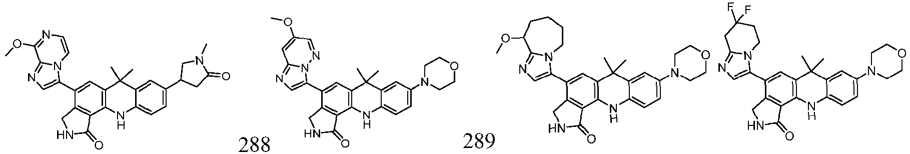

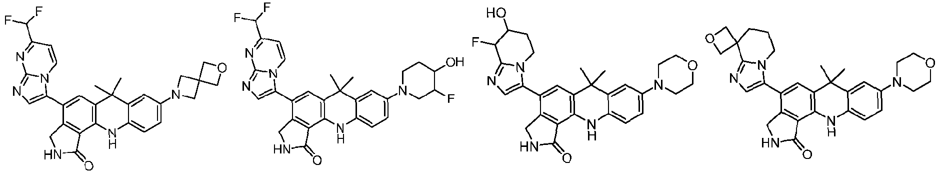

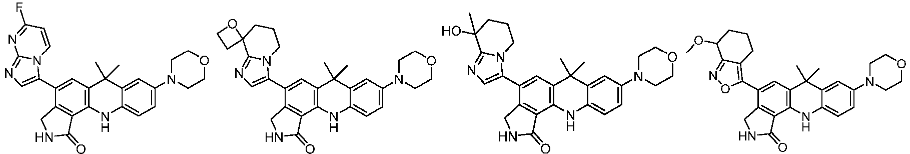

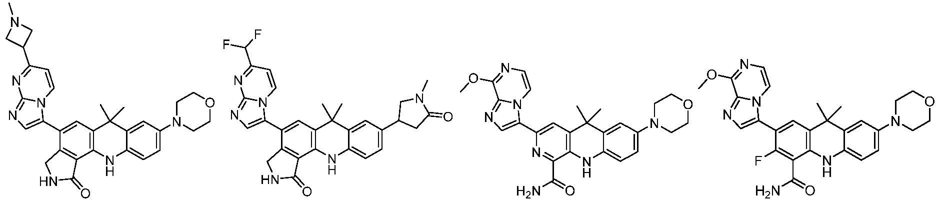

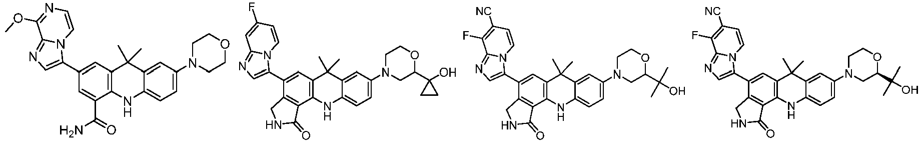

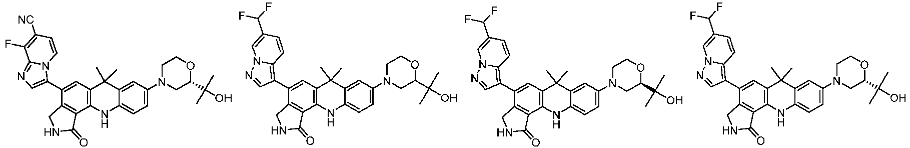

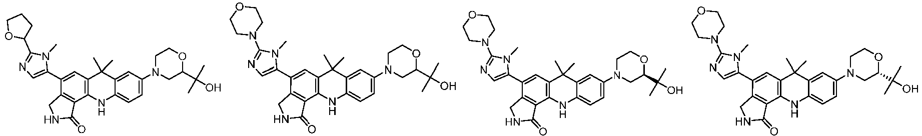

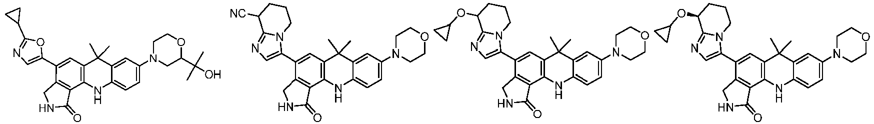

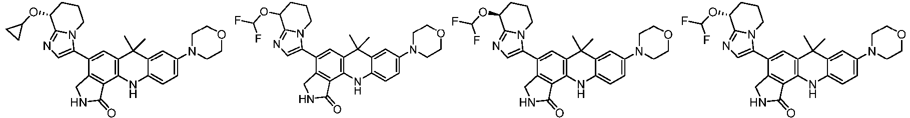

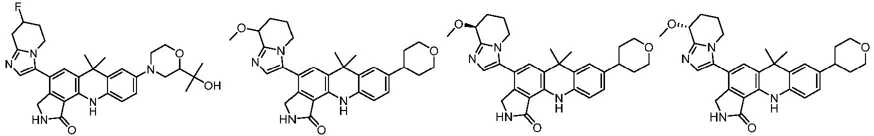

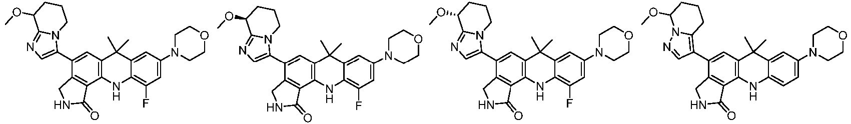

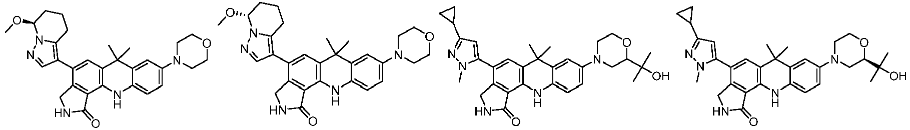

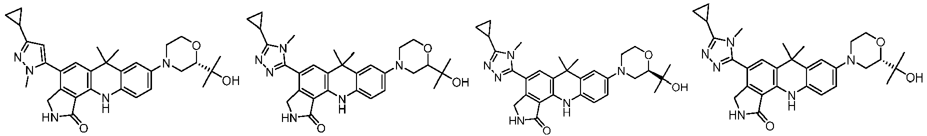

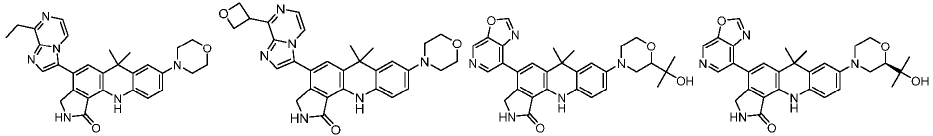

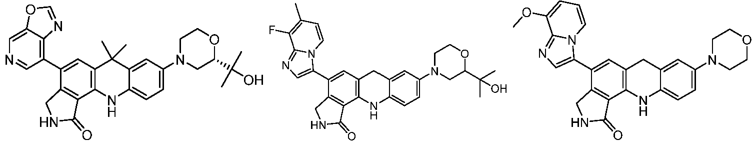

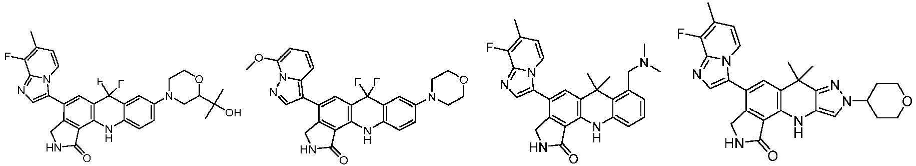

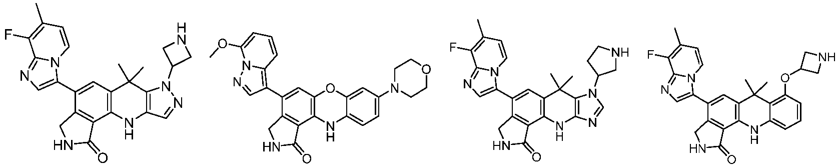

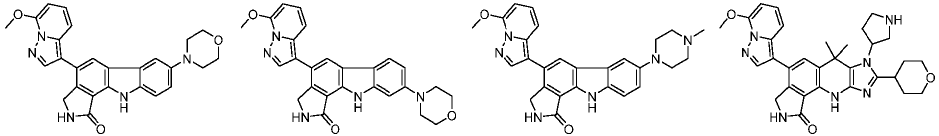

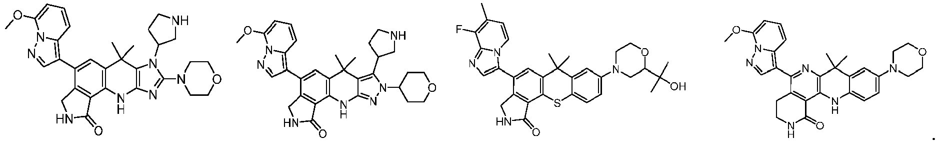

57. A pharmaceutical composition, comprising the compound according to any one of claims 1 to 56, or a stereoisomer, an N-oxide, a hydrate, a solvate, a metabolite, a pharmaceutically acceptable salt, a polymorph, or a prodrug thereof, and a pharmaceutically acceptable carrier, adjuvant or vehicle.

58. Use of the compound according to any one of claims 1 to 56, or a stereoisomer, an N-oxide, a hydrate, a solvate, a metabolite, a pharmaceutically acceptable salt, a polymorph, or a prodrug thereof, or the pharmaceutical composition according to claim 57 in the manufacture of a medicament for the prevention and/or treatment of an HPK1-mediated disorder, disease or condition.

59. The compound according to any one of claims 1 to 56, or a stereoisomer, an N-oxide, a hydrate, a solvate, a metabolite, a pharmaceutically acceptable salt, a polymorph, or a prodrug thereof, or the pharmaceutical composition according to claim 57, for use in the prevention and/or treatment of an HPK1-mediated disorder, disease or condition.

60. A method of preventing and/or treating an HPK1-mediated disorder, disease or condition, comprising administering the compound according to any one of claims 1 to 56, or a stereoisomer, an N-oxide, a hydrate, a solvate, a metabolite, a pharmaceutically acceptable salt, a polymorph, or a prodrug thereof, or the pharmaceutical composition according to claim 57 to a subject.

61. The use according to claim 58, the compound, or a stereoisomer, an N-oxide, a hydrate, a solvate, a metabolite, a pharmaceutically acceptable salt, a polymorph, or a prodrug thereof, or the pharmaceutical composition according to claim 59, or the method according to claim 60, wherein the disorder, disease or condition is a proliferative disorder.

62. The use, compound, pharmaceutical composition or method according to claim 61, wherein the proliferative disorder is a cancer.

63. The use, compound, pharmaceutical composition or method according to claim 61, wherein the proliferative disorder is associated with one or more activating mutations in HPK1.

64. The use according to claim 58, the compound, or a stereoisomer, an N-oxide, a hydrate, a solvate, a metabolite, a pharmaceutically acceptable salt, a polymorph, or a prodrug thereof, or the composition according to claim 59, or the method according to claim 60, wherein the disorder, disease or condition is a chronic viral infection.

65. Use of the compound according to any one of claims 1 to 56, or a stereoisomer, an N-oxide, a hydrate, a solvate, a metabolite, a pharmaceutically acceptable salt, a polymorph, or a prodrug thereof, or the pharmaceutical composition according to claim 57 in the manufacture of a medicament for increasing the efficacy of a vaccination.

66. The compound according to any one of claims 1 to 56, or a stereoisomer, an N-oxide, a hydrate, a solvate, a metabolite, a pharmaceutically acceptable salt, a polymorph, or a prodrug thereof, or the pharmaceutical composition according to claim 57, for use in increasing the efficacy of a vaccination.

67. A method of increasing the efficacy of a vaccination, comprising administering the compound according to any one of claims 1 to 56, or a stereoisomer, an N-oxide, a hydrate, a solvate, a metabolite, a pharmaceutically acceptable salt, a polymorph, or a prodrug thereof, or the pharmaceutical composition according to claim 57 to a subject.
